# EUROPEAN PATENT SPECIFICATION

(11) **EP 3 900 742 B1**
(45) Date of publication and mention of the grant of the patent: **15.05.2024**
(21) Application number: 21165610.3
(22) Date of filing: 10.09.2015
(51) Int. Cl.: A61K 47/68, A61K 47/54, A61K 47/64, C07H 15/26, C07K 7/02, A61K 45/06, A61P 35/00, A61P 37/00

(54) **TARGETED DELIVERY OF TERTIARY AMINE-CONTAINING DRUG SUBSTANCES**
GEZIELTE ABGABE VON TERTIÄRAMINHALTIGEN WIRKSTOFFEN
ADMINISTRATION CIBLÉE DE SUBSTANCES MÉDICAMENTEUSES CONTENANT UNE AMINE TERTIAIRE

(30) Priority: 11.09.2014 US 201462049206 P; 03.12.2014 US 201462087218 P; 04.12.2014 US 201462087755 P
(43) Date of publication of application: 27.10.2021
(62) Divisional of application: 15839347.0
(73) Proprietor: Seagen Inc., Bothell, WA 98021 (US)
(72) Inventor: Burke, Patrick, Bothell, WA Washington 98021 (US); Jeffrey, Scott, Bothell, WA Washington 98021 (US); Hamilton, Joseph Z., Bothell, WA Washington 98021 (US)
(74) Representative: Forresters IP LLP

(56) References cited:
- EP-A1- 2 461 830
- WO-A1-2014/193722
- WO-A1-2014/194247
- WO-A1-2016/090050
- WO-A2-2013/173337
- JEFFREY SCOTT C ET AL: "Development and properties of beta-glucuronide linkers for monoclonal antibody-drug conjugates", BIOCONJUGATE CHEMISTRY,, vol. 17, no. 3, 1 May 2006 (2006-05-01), pages 831-840, XP002423719, ISSN: 1043-1802, DOI: 10.1021/BC0600214
- DORONINA S O ET AL: "Development of potent monoclonal antibody auristatin conjugates for cancer therapy", NATURE BIOTECHNOLOGY (ADVANCE ONLINE PUBLICATION), GALE GROUP INC, vol. 21, no. 7, 1 July 2003 (2003-07-01), pages 778-784, XP002280966, ISSN: 1087-0156, DOI: 10.1038/NBT832
- DESBENE S ET AL: "APPLICATION OF THE ADEPT STRATEGY TO THE MDR RESISTANCE IN CANCER CHEMOTHERAPY", ANTI-CANCER DRUG DESIGN, OXFORD UNIVERSITY PRESS, BASINGSTOKE, vol. 14, no. 2, 1 April 1999 (1999-04-01), pages 93-106, XP000874376, ISSN: 0266-9536
- P. J. BURKE ET AL: "Development of Novel Quaternary Ammonium Linkers for Antibody-Drug Conjugates", MOLECULAR CANCER THERAPEUTICS, vol. 15, no. 5, 4 March 2016 (2016-03-04), pages 938-945, XP055456299, US ISSN: 1535-7163, DOI: 10.1158/1535-7163.MCT-16-0038

## Description

### BACKGROUND OF THE INVENTION

The invention relates to ligand-drug conjugates (LDCs) for targeted delivery of tertiary amine-containing drugs to abnormal cells associated with a given disease state or to the vicinity of such cells, as defined in the claims. The targeting ligand of such an LDC selectively exposes abnormal cells, in contrast to normal cells distant from the abnormal cells, to a tertiary amine-containing drug. That selective exposure is accomplished by concentrating the drug at the desired site of action by binding of the targeting ligand of the LDC on, or in the vicinity of, the abnormal cells. As a result, exposure of distant normal cells to the drug is reduced, thus reducing undesired side effects while reducing the contribution of abnormal cells to the disease state.

In general, the design of an LDC involves consideration of a variety of factors including the requirement that the drug has a site for attachment to a linker moiety that joins the drug to the targeting ligand and is capable of releasing the drug at the target site. A tertiary amine-containing compound may have no suitable site of attachment thereby necessitating modification of a parent drug for attachment to the linker moiety of an LDC. In those instances, the released drug is not the parent drug but is the modified drug. For example, a tertiary amine-containing drug may be modified by removing one of its amine substituents to provide a secondary amine, which could then be incorporated into a LDC through a carbonyl-containing functional group. However, oftentimes the modified drug will have significantly reduced biological activity, or undesired changes in other pharmacological properties, in comparison to the parent tertiary amine-containing drug.

Because of the difficulty in providing an alternative site of conjugation and the desire to retain the maximum biological activity of a tertiary amine-containing drug, there is a need in the art for LDCs that use the tertiary amine nitrogen as the site of conjugation in order to allow for release of fully active tertiary amine-containing drug at the targeted site of action. Even if reduced biological activity or changes in other pharmacological properties are tolerable through alteration of a tertiary amine-containing drug through removal of one of its nitrogen substituents, or when an alternative site of conjugation is available or can be introduced without serious consequences to desired biological activity, there remains a need in the art for the use of the tertiary amine nitrogen as the site of conjugation. That need exists since it is unpredictable as to which of the possible sites of conjugation that could be presented by a tertiary amine-containing drug will provide the most efficient release of the active drug and thus the most active LDC.
WO 2013/173337 discloses Ligand-Drug Conjugates, Drug-Linkers, Linkers, and Ligand-Linker Conjugates comprising a self-stabilizing linker assembly component. EP 2461830 discloses antiproliferative compounds for use in treating tumors. Jeffrey, S.C. et.al. (2006). Development and Properties of β-Glucuronide Linkers for Monoclonal Antibody-Drug Conjugates. Bioconjugate Chem,17, 831-840 discloses a β-glucuronide-based linker for attaching cytotoxic agents to monoclonal antibodies. Doronina, S.O. et. Al. (2003). Development of Potent Monoclonal Antibody Auristatin Conjugates for Cancer Therapy. Nature Biotechnology, 21(7), 778-784 discloses auristatin E and MMAE Antibody-Drug Conjugates.

### SUMMARY OF THE INVENTION

The invention is a Ligand Drug Conjugate (LDC) composition as defined in appended claims 1 to 24, wherein the LDC composition is represented by the structure of Formula 1 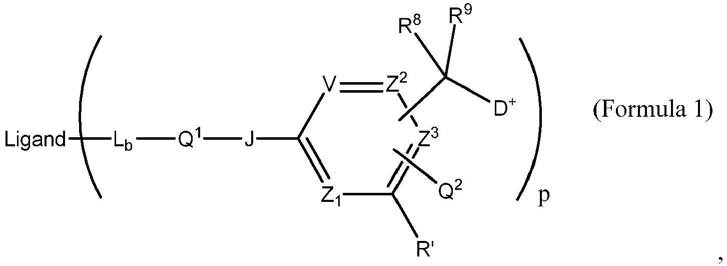
wherein: "Ligand" is a Ligand Unit (L), wherein L is capable of selectively binding to a targeting moiety, and L is an antibody or fragment thereof to define an antibody drug conjugate (ADC), wherein the target moiety is an antigen capable of selectively binding to the ADC, and wherein the antigen is an extracellularly accessible cell-surface protein, glycoprotein or carbohydrate displayed preferentially by abnormal cells in comparison to normal cells, wherein the abnormal and normal cells are cells in a mammal, wherein the abnormal cells are hyper-proliferating cells; L_{b} is a primary linker; Q¹ is Aₐ-W_{w}, wherein A is an optional Stretcher unit, so that the subscript a is 0 when A is absent or a is 1 when A is present and comprises two, three or four subunits; Q² is W'_{w'}-E-, wherein Q² when present is bonded to V, Z¹, Z² or Z³; W_{w} and W'_{w'} are Cleavable units; wherein W_{w} of Q¹ is capable of selective cleavage by a regulatory or intracellular protease in comparison to serum proteases, or is capable of cleavage by glutathione through disulfide exchange, or is more reactive to hydrolysis under lower pH conditions present in lysosomes in comparison to physiological pH of serum, and W'_{w'}-E of Q² provides a glycosidic bond cleavable by a glycosidase located intracellularly, wherein the subscript w is 0 or 1 so that W is absent when w is 0 or W is present when w is 1, and w' is 0 or 1, wherein W'_{w'}-E is absent when w' is 0 or W'_{w'}-E is present when w' is 1, and wherein w + w' is 1 (i.e., one and only one of W, W' is present); V, Z¹, Z² and Z³ are =N- or =C(R²⁴)-, wherein R²⁴ is hydrogen or alkyl, alkenyl or alkynyl, optionally substituted, or halogen, - NO₂, -CN or other electron withdrawing group, an electron donating group, -Q², or - C(R⁸)(R⁹)-D⁺, wherein at least one of V, Z¹, Z² and Z³ is =C(R²⁴)- when w is 1 and at least two of V, Z¹, Z² and Z³ are =C(R²⁴)- when w' is 1,
provided that when w is 1, Q² is absent and one and only one R²⁴ is - C(R⁸)(R⁹)-D⁺ so that -C(R⁸)(R⁹)-D⁺ is bonded to one of V, Z¹, Z², Z³ when that variable group is =C(R²⁴)- and the Q¹-J- and -C(R⁸)(R⁹)-D⁺ substituents are *ortho* or *para* to each other,
provided that when w' is 1, one any only one R²⁴ is -C(R⁸)(R⁹)-D⁺ so that - C(R⁸)(R⁹)-D⁺ is bonded to one of V, Z¹, Z², Z³ when that variable group is =C(R²⁴)- and one and only one other R²⁴ is Q² so that Q² is bonded to another one of V, Z¹, Z², Z³ when that variable group is =C(R²⁴)-, and the Q² and -C(R⁸)(R⁹)-D⁺ substituents are *ortho* or *para* to each other;
R⁸ and R⁹ independently are hydrogen, alkyl, alkenyl or alkynyl, optionally substituted, or aryl or heteroaryl, optionally substituted; E and J independently are -O-, -S-or -N(R³³)-, wherein R³³ is hydrogen or optionally substituted alkyl; R' is hydrogen or is halogen, -NO₂, -CN or other electron withdrawing group, or is an electron donating group; D⁺ represents a structure of a quaternized tertiary amine-containing drug D; and p is an average drug loading having a number ranging from 1 to 24; wherein said cleavage by an intracellular or regulatory protease, by glutathione through disulfide exchange, by acid hydrolysis, or by a glycosidase results in release of D from an LDC of the LDC composition.

In some aspects, W of Q¹ is comprised of a peptide moiety having a peptide bond to J that is selectively cleavable by an intracellular protease in comparison to serum proteases, wherein the intracellular protease may or may not be more specific to the targeted abnormal or other unwanted cells in comparison to normal cells, and wherein action of the intracellular protease on W will cause release of a tertiary amine-containing drug (D) from the LDC. In other aspects the peptide bond is cleavable by a protease that is excreted by abnormal cells to a greater extent than by normal cells.

In other aspects, W' of Q² is a glycoside-bonded carbohydrate, wherein the glycoside bond in W'_{w'}-E of Q² provides for a cleavage site for an intracellular glycosidase, wherein action of the glycosidase on W'_{w'}-E causes release of tertiary amine-containing drug (D) from the LDC, wherein the glycosidase may or may not be more specific to the targeted abnormal or other unwanted cells in comparison to normal cells, or is capable of selective cleavage by a glycosidase excreted in greater amounts by the targeted abnormal or other unwanted cells in comparison to normal cells.

### BRIEF DESCRIPTION OF THE DRAWINGS

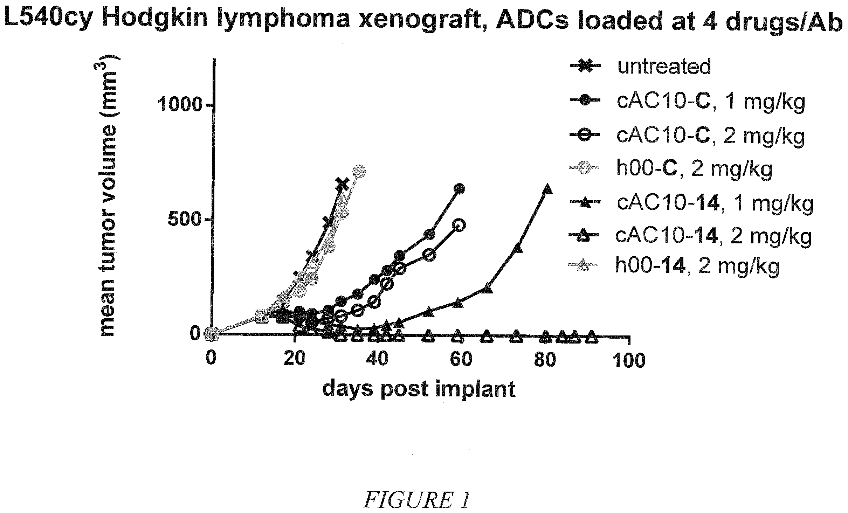

Figure 1. Treatment of L540cy Hodgkin lymphoma xenograft with a quaternized-linked auristatin antibody Ligand Drug Conjugate (cAC10-**14**) having a cathepsin cleavable unit that releases free tertiary amine-containing drug (auristatin E) in comparison to the corresponding carbamate-linked antibody Ligand Drug Conjugate (cAC10-**C**) that releases desmethyl drug (MMAE) vs. their respective non-targeting control conjugates h00-**14** and h00-**C**.

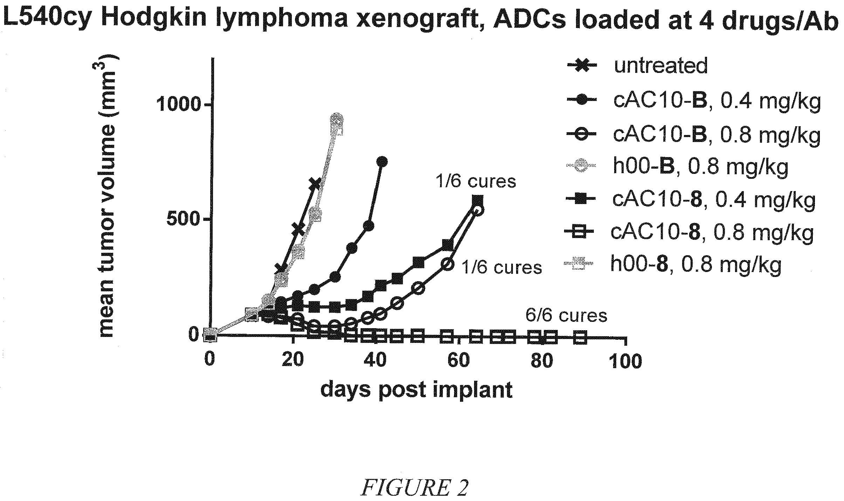

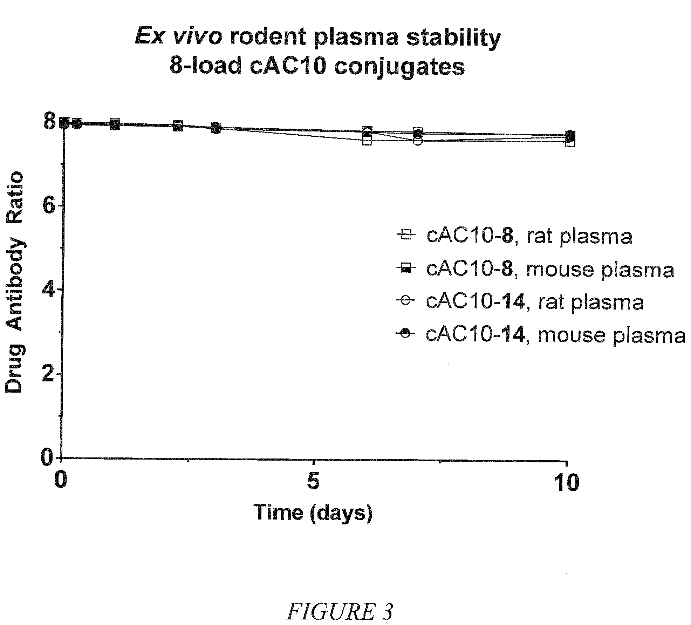

Figure 2. Treatment of L540cy Hodgkin's lymphoma xenograft with a quaternized-linked auristatin antibody Ligand Drug Conjugate (cAC10-**8**) having a glucuronidase cleavable unit that releases free tertiary amine-containing drug (auristatin E) in comparison to the corresponding carbamate-linked antibody Ligand Drug conjugate (cAC10-**B**) that releases desmethyl drug (MMAE) vs. their respective non-targeting control conjugates h00-**8** and h00-**B**.Figure 3. *Ex vivo* plasma stabilities for quaternized-linked auristatin LDCs having a cathepsin Cleavable unit or a glucuronidase Cleavable unit.

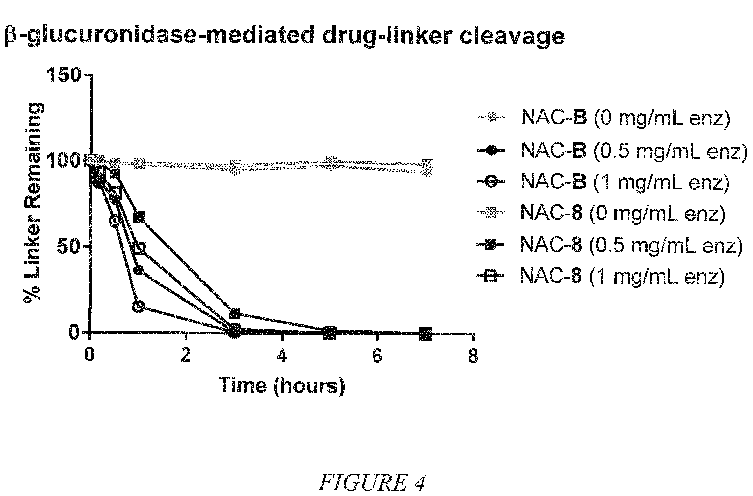

Figure 4. Kinetics of free tertiary amine-containing drug (auristatin E) release from a quaternized-linked LDC model system

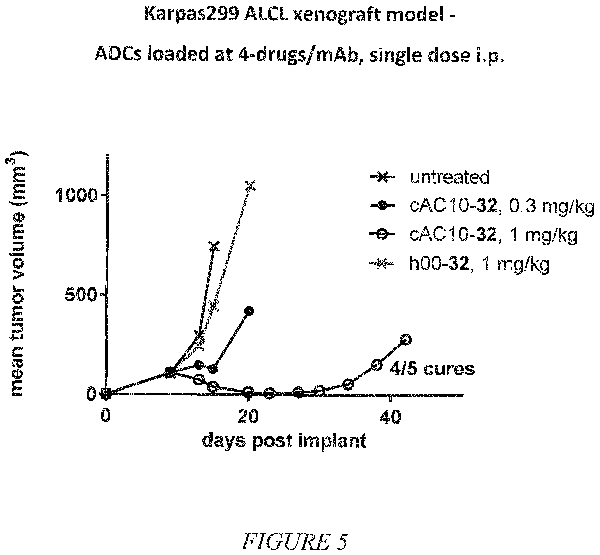

Figure 5. Treatment of a Karpas299 ALCL xenograft model with quaternized Tubulysin M Ligand Drug Conjugate, wherein the cAC10 antibody Ligand Unit targets CD30 antigen, at DAR of 4 D⁺ Drug Units/mAb, single dose i.p. at 0.3 mg/Kg and 1 mg/Kg vs. a control non-targeting conjugate of identical quaternized drug-linker and equivalent loading.

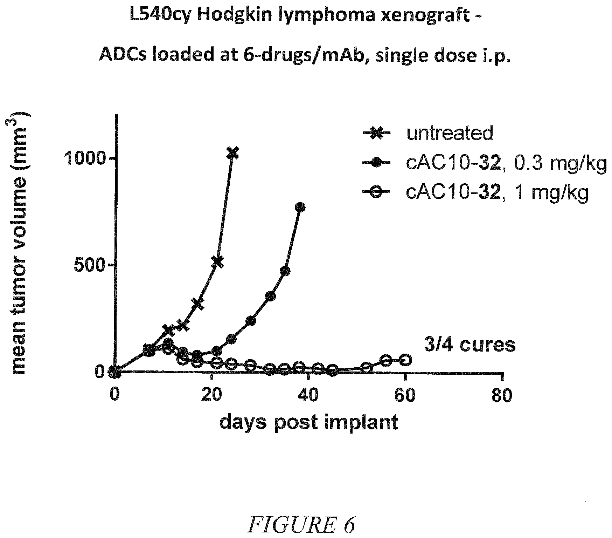

Figure 6. Treatment of L540cy Hodgkin lymphoma xenograft model with quaternized Tubulysin M Ligand Drug Conjugate prepared from Drug-Linker **32**, wherein the cAC10 antibody Ligand Unit targets CD30 antigen at DAR of 6 D⁺ Drug Units/mAb, single dose i.p. at 0.3 mg/Kg and 1 mg/Kg vs. sham treatment.

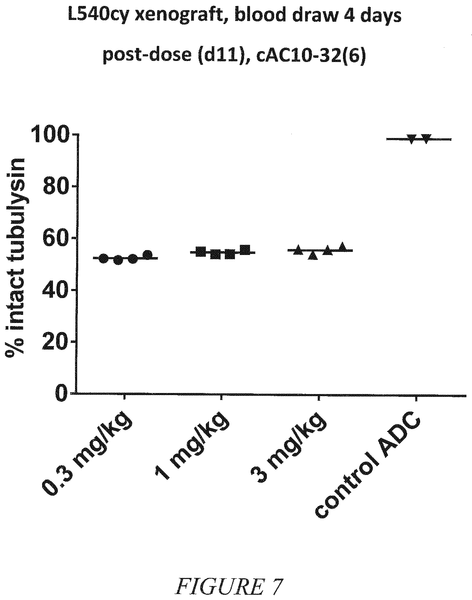

Figure 7. Assessment of acetate loss from the tubuvaline component of a Tubulysin M Ligand Drug Conjugate prepared from quaternized Drug-Linker **32**, which has the -Val-Ala- Cleavable Unit, at DAR of 6 D⁺ Drug Units/mAb administered single dose i.p. at 0.3 mg/Kg, 1 mg/Kg and 3.0 mg/Kg to a L540cy xenograft, wherein the cAC10 antibody Ligand Unit of the conjugate targets CD30 antigen, vs. a control conjugate from blood draw 4 days post dosing on day 17 of the study.

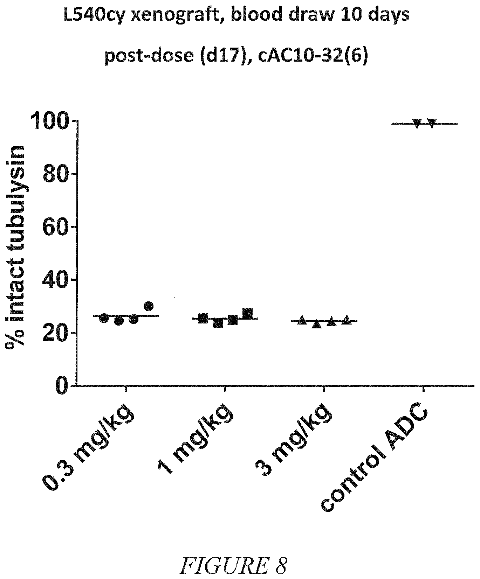

Figure 8. Assessment of acetate loss from the tubuvaline component of a Tubulysin M Ligand Drug Conjugate prepared from quaternized Drug-Linker **32** at DAR of 6 D⁺ Drug Units/mAb administered single dose i.p. at 0.3 mg/Kg, 1 mg/Kg and 3.0 mg/Kg to a L540cy xenograft, wherein the cAC10 antibody Ligand Unit of the conjugate targets CD30 antigen, vs. a control conjugate from blood draw 10 days post dosing on day 11 of the study.

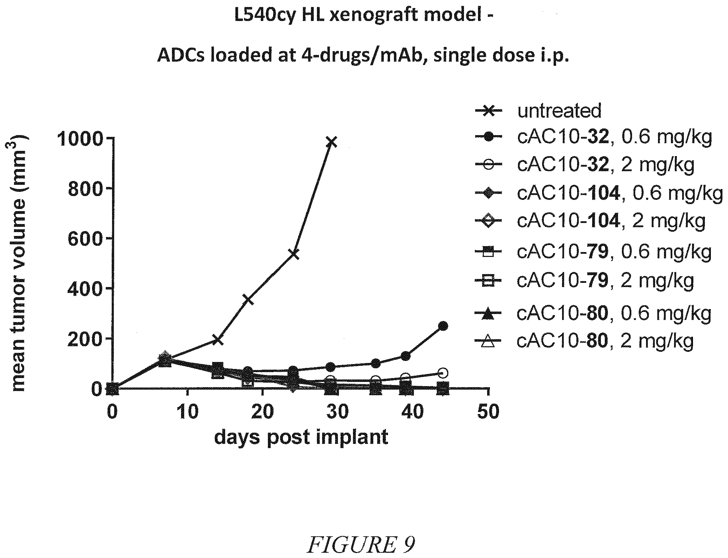

Figure 9. Comparisons of L540cy Hodgkin lymphoma xenograft model treatments at 0.6 mg/Kg and 2 mg/Kg, single dose i.p., with Ligand Drug Conjugates, wherein the cAC10 antibody Ligand Unit of the conjugate targets CD30 antigen, having 4 quaternized tubulysin Drug Units/ cAC10 antibody Ligand Unit wherein the conjugates were prepared from Drug Linker compound **32**, which has quaternized Tubulysin M with the -Val-Ala- Cleavable Unit, and from Drug Linker Compounds **79, 80** and **104,** which have quaternized Tubu(OEt), Tubu(O-Pr) and Tubulysin M Drug Units, respectively, and identical glucuronidase conditional release Linker Units.

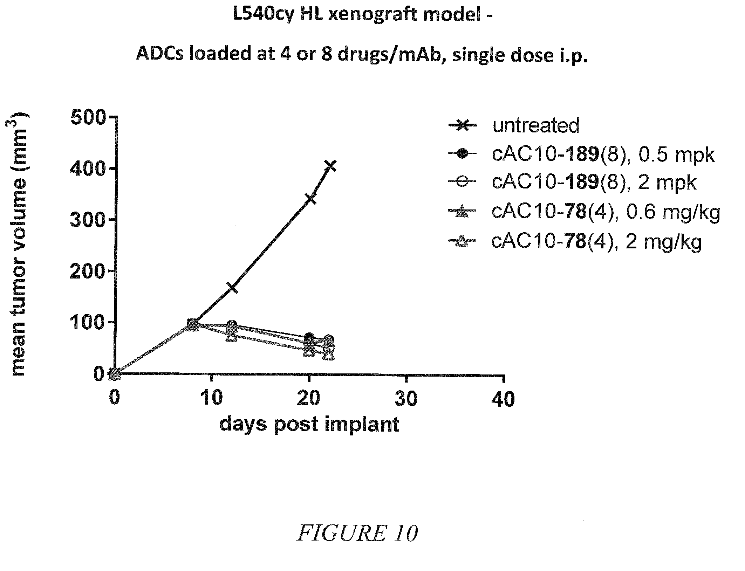

Figure 10. Comparisons of L540cy Hodgkin lymphoma xenograft model treatments at 0.5 mg/Kg or 0.6 mg/Kg and 2 mg/Kg, single dose i.p., with eAC10 Ligand Drug Conjugates, wherein the antibody Ligand Unit targets CD30 antigen, wherein one conjugate has 8 quaternized auristatin F Drug Units/ Ligand Unit and is prepared from Drug-Linker compound **189** vs. another conjugate that has 4 quaternized Tubu(O-CH₃) Drug Units per cAC10 antibody Ligand Unit prepared from Drug Linker compound **78,** with both conjugates having identical glucuronidase conditional release Linker Units.

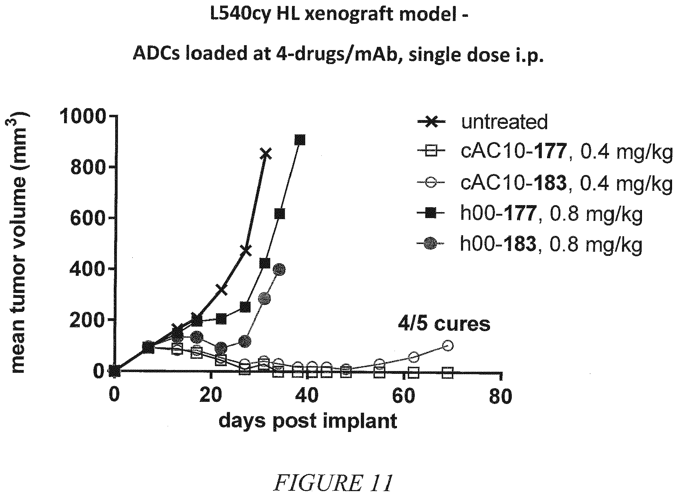

Figure 11. Comparisons of L540cy Hodgkin lymphoma xenograft model treatments at 0.4 mg/Kg and 0.8 mg/Kg, single dose i.p., with cAC10 Ligand Drug Conjugates having 4 drug-linker moieties, wherein the antibody Ligand Unit targets CD30 antigen, wherein one conjugate has quaternized Dolastatin 10 and is prepared from Drug-Linker **177** vs. a non-quaternized Dolastatin 10 conjugate prepared from Drug Linker **183,** in which both conjugates have glucuronidase conditional release Linker Units.

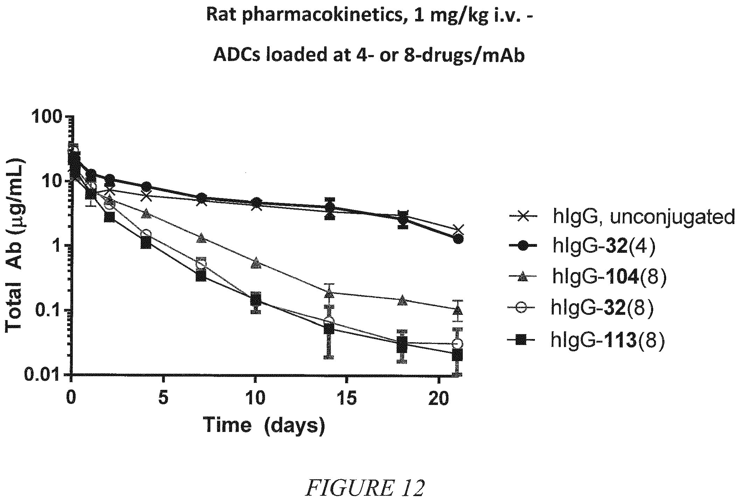

Figure 12. Pharmokinetic profile of Ligand Drug Conjugates of 4 vs. 8 quaternized Drug Unit loading having human IgG Ligand Units in comparison to unconjugated antibody dosed 1 mg/Kg i.v. in rat wherein the conjugates are prepared from Drug Linker **32** having quaternized Tubulysin M Drug Units and cathepsin conditional release Linker Unit and that prepared from Drug Linker **113** also having Tubulysin M Drug Units, but having glucuronidase conditional release Linker Units.

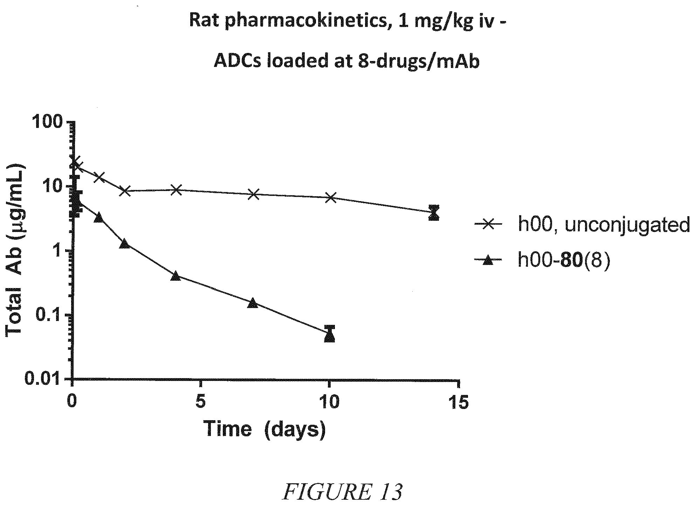

Figure 13. Pharmokinetic profile of a Ligand Drug Conjugate with 8 quaternized Drug Unit loading and having non-targeting antibody Ligand Units dosed 1 mg/Kg i.v. in rat, wherein the the Conjugate was prepared from Drug-Linker compound 80, whose quaternized Drug Unit is that of an ether varient of Tubulysin M in which the O-linked acetate moiety of the tubuvalene component has been replaced with propoxy and which has a glucuronidase conditional release Linker Unit.

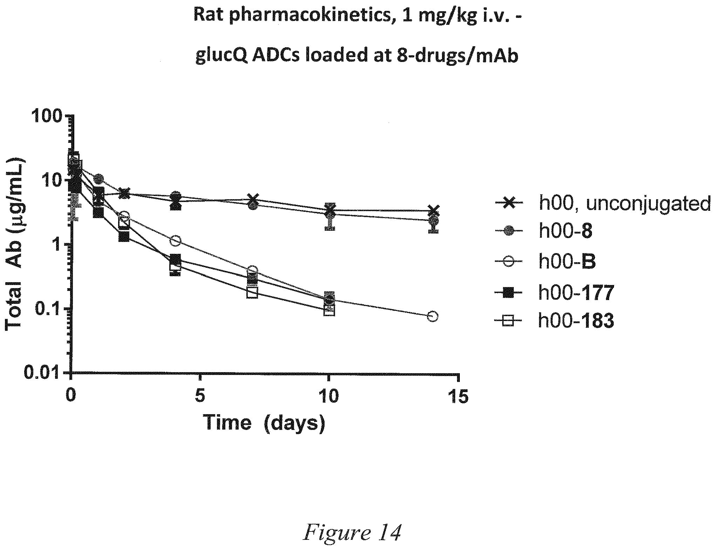

Figure 14 Pharmokinetic profile of Ligand Drug Conjugates with 8 quaternized Drug Unit loading wherein the Conjugates are prepared from Drug Linker compound **8** having quaternized Auristatin E Drug Units and cathepsin conditional release Linker Units, Drug-Linker **177** having quaternized Dolastatin 10 Drug Unit and glucuronidase conditional release Linker Unit and Drug Linker compound **183** having non-quaternized Dolastatin 10 conjugate and also having glucuronidase conditional release Linker Unit vs. un-conjugated non-targeting antibody.

### DETAILED DESCRIPTION OF THE INVENTION

### Definitions

As used herein and unless otherwise stated or implied by context, terms that are used herein have the meanings defined below. Unless otherwise contraindicated or implied, e.g., by including mutually exclusive elements or options, in these definitions and throughout this specification, the terms "a" and "an" mean one or more and the term "or" means and/or where permitted by context. Thus, as used in the specification and the appended claims, the singular forms "a," "an" and "the" include plural referents unless the context clearly dictates otherwise.

At various locations in the present disclosure, e.g., in any disclosed embodiments or in the claims, reference is made to compounds, compositions, or methods that "comprise" one or more specified components, elements or steps. Invention embodiments also specifically include those compounds, compositions, compositions or methods that are or that consist of or that consist essentially of those specified components, elements or steps. The term "comprised of" is used interchangeably with the term "comprising" and are stated as equivalent terms. For example, disclosed compositions, devices, articles of manufacture or methods that "comprise" a component or step are open and they include or read on those compositions or methods plus an additional component(s) or step(s). However, those terms do not encompass unrecited elements that would destroy the functionality of the disclosed compositions, devices, articles of manufacture or methods for its intended purpose. Similarly, disclosed compositions, devices, articles of manufacture or methods that "consist of" a component or step are closed and they would not include or read on those compositions or methods having appreciable amounts of an additional component(s) or an additional step(s). Furthermore, use of the term "including" as well as other forms, such as "include", "includes," and "included," is not limiting. Finally, the term "consisting essentially of" admits for the inclusion of unrecited elements that have no material effect on the functionality of the disclosed compositions, devices, articles of manufacture or methods for its intended purpose and is further defined herein. The section headings used herein are for organizational purposes only and are not to be construed as limiting the subject matter described. Unless otherwise indicated, conventional methods of mass spectroscopy, NMR, HPLC, protein chemistry, biochemistry, recombinant DNA techniques and pharmacology are employed.

"About" as used herein when used in connection with a numeric value or range of values provided to describe a particular property of a compound or composition indicate that the value or range of values may deviate to an extent deemed reasonable to one of ordinary skill in the art while still describing the particular property. Reasonable deviations include those that are within the accuracy or precision of the instrument(s) used in measuring, determining or deriving the particular property. Specifically, the term "about" when used in this context, indicate that the numeric value or range of values may vary by 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1% or 0.01% of the recited value or range of values, typically by 10% to 0.5 %, more typically by 5% to 1%, while still describing the particular property.

"Essentially retains", "essentially retaining and like terms as used herein refers to a property, characteristic or activity of a compound or composition or moiety thereof that has not detectably changed or is within experimental error of determination of that same activity, characteristic or property of another compound or composition or moiety from which it was derived.

"Negligibly" or "negligible" as used herein is an amount of an impurity below the level of quantification by HPLC analysis and if present represents from about 0.5% to about 0.1 w/w% of the composition that it contaminates. Depending on context those terms may also mean that no statistically significant difference is observed between measured values or outcomes or within experimental error of the instrumentation used to obtain those values. Negligible differences in values of a parameter determined experimentally do not imply that an impurity characterized by that parameter is present in negligible amount.

"Substantially retains" as used herein refers to a measured value of a physical property of a compound or composition or moiety thereof that is statistically different of the determination of that same physical property of another compound or composition or moiety from which it was derived, but which such difference does not translate it a statistically significant difference in biological activity in a suitable biological test system for evaluating that activity (i.e., biological activity is essentially retained). Thus the phrase "substantially retains" is made in reference to the effect that a physical property of a compound or composition has on a biological activity that is explicitly associated with that property.

"Predominately containing", "predominately having" and like terms refers to the major component of a mixture. When the mixture is of two components, then the major component represents more than 50% by weight of the mixture. With a mixture of three or more components the predominant component is the one present in greatest amount in the mixture and may or may not represent a majority of the mass of the mixture.

The term "electron-withdrawing group" refers to a functional group or electronegative atom that draws electron density away from an atom to which it is bonded either inductively and/or through resonance, whichever is more dominant (i.e. a functional group or atom may be electron withdrawing inductively but may overall be electron donating through resonance), and tends to stabilize anions or electron rich moieties. The electron withdrawing effect is typically transmitted inductively, albeit in attenuated form, to other atoms attached to the bonded atom that has been made electron deficient by the electron withdrawing group (EWG) thus affecting the electrophilicity of a more remote reactive center. Exemplary electron withdrawing groups include, but are not limited to, - C(=O), -CN, -NO₂, -CX₃, -X, -C(=O)OR, -C(=O)NR₂, -C(=O)R, -C(=O)X, -S(=O)₂R, - S(=O)₂OR, -S(=O)₂NHR, -S(=O)₂NR₂, -P(=O)(OR)₂, -P(=O)(CH₃)NHR, -NO, and -NR₃⁺, wherein X is -F, -Br, -Cl, or -I, and R is, at each occurrence, independently selected from the group consisting of hydrogen and C₁₋₆ alkyl. Exemplary EWGs can also include aryl groups (e.g., phenyl) depending on substitution and certain heteroaryl groups (e.g., pyridine). Thus, the term "electron withdrawing groups" also includes aryls or heteroaryls that are further substituted with electron withdrawing groups. Typically, electron withdrawing groups are -C(=O), -CN, -NO₂, -CX₃, and -X, wherein X is halogen. Depending on their substituents, an unsaturated alkyl moiety may also be an electron withdrawing group.

The term "electron donating group" refers to a functional group or electropositive atom that increases electron density of an atom to which it is bonded either inductively and/or through resonance whichever is more dominant (i.e., a functional group or atom may be electron donating through resonance but may overall be electron withdrawing inductively) and tends to stabilize cations or electron poor systems. The electron donating effect is typically transmitted through resonance to other atoms attached to the bonded atom that has been made electron rich by the electron donating group (EWG) thus affecting the nucleophilicity of a more remote reactive center. Exemplary electron donating groups include, but are not limited to -OH, and -NH₂. Depending on their substituents, an aryl, heteroaryl or unsaturated alkyl moiety may also be an electron donating group.

"Moiety" as used herein means a specified segment, fragment or functional group of a molecule or compound. Chemical moieties are sometimes indicated as chemical entities that are embedded in or appended (i.e., a substituent or variable group) to a molecule, compound or chemical formula.

For any substituent group or moiety described herein by a given range of carbon atoms, the designated range means that any individual number of carbon atoms is described. Thus, reference to, e.g., "optionally substituted C₁-C₄ alkyl", "optionally substituted alkenyl C₂₋₆ alkenyl", "optionally substituted C₃-C₈ heterocycle" specifically means that a 1, 2, 3 or 4 carbon optionally substituted alkyl moiety as defined herein is present, or a 2, 3, 4, 5 or 6 carbon alkenyl, or a 3, 4, 5, 6, 7 or 8 carbon moiety comprising a heterocycle or optionally substituted alkenyl moiety as defined herein is present. All such numerical designations are expressly intended to disclose all of the individual carbon atom groups; and thus "optionally substituted C₁-C₄ alkyl" includes, methyl, ethyl, 3 carbon alkyls, and 4 carbon alkyls, including all of their positional isomers, whether substituted or unsubstituted. Thus, when an alkyl moiety is substituted, the numerical designations refer to an unsubstituted base moiety and are not intended to include carbon atoms that may be present in the substituents of that base moiety. For esters, carbonates, carbamates and ureas as defined herein that are identified by a given range of carbon atoms, the designated range includes the carbonyl carbon of the respective functional group. Thus, an C₁ ester refers to a formate ester, a C₂ ester refers to an acetate ester and an unsubstituted C₁ urea refers to NH₂(C=O)NH₂.

The organic substituents, moieties and groups described herein, and for other any other moieties described herein, usually will exclude unstable moieties except where such unstable moieties are transient species that one can use to make a compound with sufficient chemical stability for the one or more of the uses described herein. Substituents, moieties or groups by operation of the definitions provided herein that results in those having a pentavalent carbon are specifically excluded.

"Alkyl" as used herein by itself or as part of another term refers to methyl or a collection of carbon atoms, wherein one or more of the carbon atoms is saturated (i.e., is comprised of one or more sp³ carbons) that are covalently linked together in normal, secondary, tertiary or cyclic arrangements, i.e., in a linear, branched, cyclic arrangement or some combination thereof. When the contiguous saturated carbon atoms are in a cyclic arrangement such alkyl moieties are sometimes referred to as cycloalkyl as defined herein. Saturated alkyl substituents contain saturated carbon atoms (i.e., sp³ carbons) and no aromatic, sp² or sp carbon atoms (i.e., is not substituted with unsaturated, aromatic and heteroaromatic moieties). Unsaturated alkyl substituents are alkyl moieties substituted with moieties as described herein for alkenyl, alkynyl, aryl and heteroaryl moieties.

Thus, unless otherwise indicated, the term "alkyl" will indicate a saturated non-cyclic hydrocarbon radical, optionally substituted with one or more cycloalkyl or unsaturated, aromatic or heteroaromatic moieties or some combination thereof, wherein the saturated hydrocarbon radical has the indicated number of covalently linked saturated carbon atoms (e.g., "C₁-C₆ alkyl" or "C1-C6 alkyl" means an alkyl moiety or group containing 1, 2, 3, 4, 5 or 6 contiguous non-cyclic saturated carbon atoms and "C₁-C₈ alkyl" refers to an alkyl moiety or group having 1, 2, 3, 4, 5, 6, 7 or 8 contiguous saturated non-cyclic carbon atoms). The number of saturated carbon atoms in an alkyl moiety or group can vary and typically is 1-50, 1-30 or 1-20, and more typically is 1-8 or 1-6. Typically, an alkyl substituent is a saturated C₁-C₈ alkyl moiety, or more typically is a C₁-C₆ or C₁-C₄ alkyl moiety with the latter sometimes referred to as lower alkyl. When the number of carbon atoms is not indicated, the alkyl group has from 1 to 8 carbon atoms.

When referring to an alkyl moiety or group as an alkyl substituent, that alkyl substituent to a Markush structure or another organic moiety with which it is associated is that chain of contiguous saturated carbon atoms covalently attached to the structure or moiety through a sp³ carbon of the alkyl substituent. An alkyl substituent, as used herein, therefore contains at least one saturated moiety and may also contain (i.e., be substituted with) cycloalkyl, unsaturated alkyl, aromatic or heteroaromatic moieties or groups. Thus, an alkyl substituent may additionally comprise one, two, three or more independently selected double bonds, triple bonds or cycloalkyl, aromatic or heteroaromatic moieties or some combination thereof, typically one double bond, one triple bond (i.e., is substituted one alkenyl or alkynyl moiety) or is substituted with one cycloalkyl, aromatic or heteroaromatic moiety. When an alkyl substituent, moiety or group is specified, species include those derived from removing a hydrogen atom from a parent alkane (i.e., is monovalent) and may include methyl, ethyl, 1-propyl (*n*-propyl), 2-propyl (*iso*-propyl, - CH(CH₃)₂), 1-butyl (*n*-butyl), 2-methyl-1-propyl (*iso*-butyl, -CH₂CH(CH₃)₂), 2-butyl (*sec-*butyl, -CH(CH₃)CH₂CH₃), 2-methyl-2-propyl (*t*-butyl, -C(CH₃)₃), amyl, isoamyl, *sec*-amyl and other linear, cyclic and branch chain alkyl moieties.

"Alkylene," as used herein by itself of as part of another term, refers to a saturated, branched, cyclic or straight chain hydrocarbon diradical, substituted or unsubstituted, wherein one or more of the carbon atoms is unsaturated (i.e., is comprised of one or more sp³ carbons), of the stated number of carbon atoms, typically 1-10 carbon atoms, and having two radical centers (i.e., is divalent) derived by the removal of two hydrogen atoms from the same or two different saturated (i.e., sp³) carbon atoms of a parent alkane. Alkylene moieties further include alkyl radicals as described herein in which a hydrogen atom has been removed from a saturated moiety or the radical carbon of an alkyl radical to form a diradical. Typically, alkylene moieties include divalent moieties derived from removing a hydrogen atom from saturated carbon atom of a parent alkyl moiety, but are not limited to: methylene (-CH₂-), 1,2-ethylene (-CH₂CH₂-), 1,3-propylene (-CH₂CH₂CH₂-), 1,4-butylene (-CH₂CH₂CH₂CH₂-), and like diradicals. Typically, an alkylene is a branched or straight chain hydrocarbon typically containing only sp³ carbons (i.e., is fully saturated notwithstanding the radical carbon atoms).

"Cycloalkyl" as used herein is a radical of a monocyclic, bicyclic or tricyclic ring system, wherein each of the atoms forming the ring system (i.e., skeletal atoms) is a carbon atom and wherein one or more of these carbon atoms in each ring of the cyclic ring system is saturated (i.e., is comprised of one or more sp³ carbons). Thus, a cycloalkyl is a cyclic arrangement of saturated carbons but may also contain unsaturated carbon atom(s) and therefore its carbocyclic ring may be saturated or partially unsaturated or may be fused with an aromatic ring, wherein the points of fusion to a cycloalkyl and aromatic ring are to adjacent unsaturated carbons of the cycloalkyl moiety or group or substituent and adjacent aromatic carbon of the aromatic ring.

Unless otherwise specified, a cycloalkyl moiety, group or substituent can be substituted with moieties described for alkyl, alkenyl, alkynyl, aryl, arylalkyl, alkylaryl and the like or can be substituted with another cycloalkyl moieties. Cycloalkyl moieties, groups or substituents include cyclopropyl, cyclopentyl, cyclohexyl, adamantly or other cyclic moieties having only carbon atoms. Cycloalkyls further include cyclobutyl, cyclopentenyl, cyclohexenyl, cycloheptyl and cyclooctyl. Depending on its structure, a cycloalkyl substituent can be a monoradical as described above for cycloalkyl moieties or groups or a diradical (i.e., an cycloalkylene, such as, but not limited to, cyclopropan-1,1-diyl, cyclobutan-1,1-diyl, cyclopentan-1,1-diyl, cyclohexan-1,1-diyl, cyclohexan-1,4-diyl, cycloheptan-1,1-diyl, and the like).

When cycloalkyl is used as a Markush group (i.e., a substituent) the cycloalkyl is attached to a Markush formula or another organic moiety with which it is associated through a carbon that is involved in the carbocyclic ring system of the cycloalkyl group provided that carbon is not an aromatic carbon. When an unsaturated carbon of an alkene moiety comprising the cycloalkyl substituent is attached to a Markush formula with which it is associated that cycloalkyl is sometimes referred to as a cycloalkenyl substituent. The number of carbon atoms in a cycloalkyl substituent is defined by the total number of skeletal atoms of the ring system. That number can vary and typically ranges from 3 to 50, 1-30 or 1-20, and more typically 3-8 or 3-6 unless otherwise specified, e.g., C₃₋₈ cycloalkyl means an cycloalkyl substituent, moiety or group containing 3, 4, 5, 6, 7 or 8 carbocyclic carbon atoms and C₃₋₆ cycloalkyl means an cycloalkyl substituent, moiety or group containing 3, 4, 5 or 6 carbocyclic carbon atoms. Therefore cycloalkyl substituents, moieties or groups usually have 3, 4, 5, 6, 7, 8 carbon atoms in its carbocyclic ring system and may contain *exo* or *endo-cyclic* double bonds or *endo-cyclic* triple bonds or a combination of both wherein the *endo-cyclic* double or triple bonds, or the combination of both, do not form a cyclic conjugated system of 4n + 2 electrons. A bicyclic ring system may share one (i.e., is a spiro ring system) or two carbon atoms and a tricyclic ring system may share a total of 2, 3 or 4 carbon atoms, typically 2 or 3.

"Alkenyl" as used herein means a substituent, moiety or group that comprises one or more double bond moieties (e.g., a -CH=CH- or =CH₂ functional group for *endo* and *exo* double bonds, respectively) or 1, 2, 3, 4, 5 or 6 or more, typically 1, 2 or 3 of such moieties and can be substituted with an aryl moiety or group such as benzene, or linked normal, secondary, tertiary or cyclic carbon atoms, i.e., linear, branched, cyclic or any combination thereof unless the alkenyl substituent, moiety or group is a vinyl moiety (e.g., a -CH=CH₂ functional group). An alkenyl moiety, group or substituent having multiple double bonds may have the double bonds arranged contiguously (i.e., a 1,3 butadienyl moiety) or non-contiguously with one or more intervening saturated carbon atoms or a combination thereof, provided that a cyclic, contiguous arrangement of double bonds do not form a cyclic conjugated system of 4n + 2 electrons (i.e., is not aromatic).

When an alkenyl moiety, group or substituent is specified, species include, by way of example and not limitation, any of the alkyl or cycloalkyl, groups moieties or substituents described herein that has an *exo* or one or more *endo* double bonds, including methylene (=CH₂), methylmethylene (=CH-CH₃), ethylmethylene (=CH-CH₂-CH₃), =CH-CH₂-CH₂-CH₃, and monovalent moieties derived from removal of a hydrogen atom from a sp2 carbon of a parent alkene compound. Such monovalent moieties typically include vinyl (-CH=CH₂), allyl, 1-methylvinyl, butenyl, iso-butenyl, 3-methyl-2-butenyl, 1-pentenyl, cyclopentenyl, 1-methyl-cyclopentenyl, 1-hexenyl, 3-hexenyl, cyclohexenyl, and other linear, cyclic and branched chained, all carbon-containing moieties containing at least one double bond. When alkenyl is used as a Markush group (i.e., is a substituent) the alkenyl is attached to a Markush formula or another organic moiety with which it is associated through a double-bonded carbon (i.e., an sp² carbon) of the alkenyl moiety or group. The number of carbon atoms in an alkenyl substituent is defined by the number of sp² carbon atoms of the alkene functional group that defines it as an alkenyl substituent and the total number of contiguous non-aromatic carbon atoms appended to each of these sp² carbons. That number can vary and unless otherwise specified ranges from 1 to 50, e.g., typically 1-30 or 1-20, more typically 1-8 or 1-6, when the double bond functional group is *exo* in a Markush structure, or can vary and ranges from 2 to 50, typically 2-30 or 2-20, more typically 2 to 8 or 2-6, when the double bond functional group is *endo* to the Markush structure. For example, C₂₋₈ alkenyl or C2-8 alkenyl means an alkenyl moiety containing 2, 3, 4, 5, 6, 7 or 8 carbon atoms in which at least two are sp² carbons in conjugation with each other and C₂₋₆ alkenyl or C2-6 alkenyl means an alkenyl moiety containing 2, 3, 4, 5 or 6 carbon atoms in which at least two are sp² carbons that are in conjugation with each other. Typically, an alkenyl substituent is a C₂-C₆ or C₂-C₄ alkenyl moiety having two sp² carbons that are in conjugation with each other.

"Alkenylene" as used herein by itself of as part of another term, refers to a substituent, moiety or group that comprises one or more double bond moieties, as previously described for alkenyl, of the stated number of carbon atoms, typically 1-10 carbon atoms when the double bond functional group is *exo* to a larger moiety or 2-10, when the double bond functional group is *endo* in the alkenylene moiety, and has two radical centers derived by the removal of two hydrogen atoms from the same or two different sp² carbon atoms of a double bond moiety in a parent alkene. Alkenylene moieties further include alkenyl radicals as described herein in which a hydrogen atom has been removed from the same or different sp² carbon atom of a double bond moiety of an alkenyl radical to form a diradical, or from a sp² carbon from a different double bonded moiety to provide another radical carbon. Typically, alkenylene moieties include diradicals having the structure of -C=C- or -C=C-X¹-C=C- wherein X¹ is absent or is an alkylene as defined herein.

"Alkynyl" as used herein means a substituent, moiety or group that comprises one or more triple bond moieties (i.e., a -C≡C- functional group), e.g., 1, 2, 3, 4, 5, 6 or more, typically 1 or 2 triple bonds, optionally comprising 1, 2, 3, 4, 5, 6 or more double bonds (i.e., optionally substituted with an alkenyl moiety), with the remaining bonds (if present) being single bonds and may be further comprised of linked normal, secondary, tertiary or cyclic carbon atoms, i.e., linear, branched, cyclic or any combination thereof, unless the alkynyl moiety is ethynyl.

When an alkynyl moiety or group is specified, species include, by way of example and not limitation, any of the alkyl moieties, groups or substituents described herein that has one or more double bonds, ethynyl, propynyl, butynyl, iso-butynyl, 3-methyl-2-butynyl, 1-pentynyl, cyclopentynyl, 1-methyl-cyclopentynyl, 1-hexynyl, 3-hexynyl, cyclohexynyl and other linear, cyclic and branched chained all carbon containing moieties containing at least one triple bond. When an alkynyl is used as a Markush group (i.e., a substituent) the alkynyl is attached to a Markush formula with which it is associated through one of the sp carbons of the alkynyl functional group. The number of carbon atoms in an alkynyl substituent is defined by the two sp carbon atoms of the alkyne functional group that defines it as an alkynyl substituent and the total number of contiguous non-cyclic, non-aromatic carbon atoms appended to the sp carbon not substituted with the Markush structure. That number can vary and ranges from 2 to about 50, typically 2-30 or 2-20 or more typically 2-8, unless otherwise specified, e.g., C₂₋₈ alkynyl means an alkynyl moiety containing 2, 3, 4, 5, 6, 7 or 8 carbon atoms. Alkynyl groups will typically have 2, 3, 4, 5, 6, 7, 8, 9, 10, 11, 12, 13, 14, 15, 16, 17, 18, 19 or 20 carbon atoms.

"Alkynylene" as used herein by itself of as part of another term, refers to a substituent, moiety or group that comprises one or more triple bond moieties, as previously described for alkynyl, of the stated number of carbon atoms, typically 2-10 carbon atoms, and having two radical centers derived by the removal of two hydrogen atoms from two different sp carbon atoms of a triple bond moiety in a parent alkyne. Alkynylene moieties further include alkynyl radicals as described herein in which two hydrogen atom have been removed acetylene or from two sp carbons atom of two triple bond moieties to form a diradical. Typically, alkynylene moieties include diradicals having the structure of -C≡C- or -C≡C-X-C≡C- wherein X is an alkylene, alkenylene or arylene moiety as defined herein.

"Aromatic", "aromatic ring system" or like terms as used herein refers to a planar ring having a delocalized pi-electron system containing 4n+2 pi electrons, where n is a positive integer. Aromatic rings can be formed from five, six, seven, eight, nine, ten, or more than ten atoms. Aromatics are optionally substituted. The term "aromatic" includes both carboxcylic aryl ("aryl", *e.g*., phenyl) and heterocyclic aryl (or "heteroaryl" or "heteroaromatic") groups (e.g., pyridine). The term includes monocyclic or fused-ring polycyclic (i.e., rings which share adjacent pairs of carbon atoms) groups.

"Aryl" as used here means an organic moiety, substituent or group defined by an aromatic ring system or a fused ring system with no ring heteroatoms comprising 1, 2, 3 or 4 to 6 rings, typically 1 to 3 rings, wherein the rings are composed of only carbon atoms that participate in a cyclically conjugated system of 4n + 2 electrons (Hückel rule), typically 6, 10 or 14 electrons some of which may additionally participate in exocyclic conjugation with a heteroatom (cross-conjugated (e.g., quinone). Aryl substituents, moieties or groups are typically formed by six, eight, ten or more aromatic carbon atoms. Aryl substituents, moieties or groups are optionally substituted. Exemplary aryls include C₆-C₁₀ aryls such as phenyl and naphthalenyl and phenanthryl. As aromaticity in a neutral aryl moiety requires an even number or elections it will be understood that a given range for that moiety will not encompass species with an odd number of aromatic carbons. When aryl is used as a Markush group (i.e., a substituent) the aryl is attached to a Markush formula or another organic moiety with which it is associated through an aromatic carbon of the aryl group.

Depending on the structure, an aryl group can be a monoradical (i.e., monovalent) or a diradical (i.e., an arylene group as described herein, which is divalent).

"Arylene," or "heteroarylene" as used herein by itself or as part of another term, is an aryl or heteroaryl moiety, group or substituent as defined herein that forms two covalent bonds (i.e., it is divalent) within a larger moiety, which can be in the ortho, meta, or para configurations or an aromatic diradical moiety. Exemplary arylenes include, but are not limited to, phenyl-1,2-ene, phenyl-1,3-ene, and phenyl-1,4-ene as shown in the following structures:

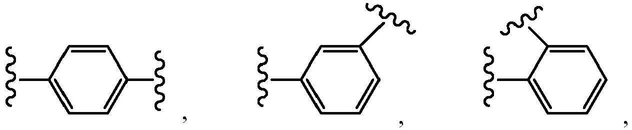

"Arylalkyl" as used herein means a substituent, moiety or group where an aryl moiety is bonded to an alkyl moiety, i.e., -alkyl-aryl, where alkyl and aryl groups are as described above, e.g., -CH₂-C₆H₅ or -CH₂CH(CH₃)-C₆H₅. When arylalkyl is used as a Markush group (i.e., a substituent) the alkyl moiety of the arylalkyl is attached to a Markush formula with which it is associated through a sp³ carbon of the alkyl moiety.

"Alkylaryl" as used herein means a substituent, moiety or group where an alkyl moiety is bonded to an aryl moiety, i.e., -aryl-alkyl, where aryl and alkyl groups are as described above, e.g., -C₆H₄-CH₃ or -C₆H₄-CH₂CH(CH₃). When alkylaryl is used as a Markush group (i.e., a substituent) the aryl moiety of the alkylaryl is attached to a Markush formula with which it is associated through a sp² carbon of the aryl moiety.

"Optionally substituted alkyl", "optionally substituted alkenyl", "optionally substituted alkynyl", "optionally substituted alkylaryl", "optionally substituted arylalkyl", "optionally substituted heterocycle", "optionally substituted aryl", "optionally substituted heteroaryl", "optionally substituted alkylheteroaryl", "optionally substituted heteroarylalkyl" and like terms refer to an alkyl, alkenyl, alkynyl, alkylaryl, arylalkyl heterocycle, aryl, heteroaryl, alkylheteroaryl, heteroarylalkyl, or other substituent, moiety or group as defined or disclosed herein wherein hydrogen atom(s) of that substituent, moiety or group has been optionally replaced with different moiety(ies) or group(s) or wherein an alicyclic carbon chain that comprise one of those substituents, moiety or group is interrupted by replacing carbon atom(s) of that chain with different moiety(ies) or group(s).

Optional substituents replacing hydrogen(s) in any one of the foregoing substituents, moieties or groups include those independently selected from the group consisting of halogen, -CN, -NH₂, -OH, -N(CH₃)₂, alkyl, fluoroalkyl, heteroalkyl, cycloalkyl, heterocycloalkyl, aryl, heteroaryl, alkoxy, aryloxy, alkylthio, arylthio, alkylsulfoxide, arylsulfoxide, alkylsulfone, and arylsulfone or those selected from the group consisting of halogen, -CN, -NH₂, -OH, -NH(CH₃), -N(CH₃)₂, -C(=O)OH (i.e., CO₂H), -C(=O)O-alkyl (i.e., CO₂-alkyl), -C(=O)NH₂, -C(=O)NH(alkyl), -C(=O)N(alkyl)₂, -S(=O)₂NH₂, -S(=O)zNH(alkyl), -S(=O)₂N(alkyl)₂, alkyl, cycloalkyl, fluoroalkyl, heteroalkyl, alkoxy, fluoroalkoxy, -S-alkyl and-S(=O)₂alkyl.

Typically, an optional substituent replacing hydrogen(s) in any one of the foregoing substituents, moieties or groups is independently selected from the group consisting of alkyl, cycloalkyl, aryl, heteroaryl, heteroalicyclic, hydroxy, alkoxy, aryloxy, cyano, halogen, nitro, haloalkyl, fluoroalkyl, fluoroalkoxy, and amino, including mono-, di- and tri-substituted amino groups, and the protected derivatives thereof, or is selected from the group consisting of halogen, -CN, -NH₂, -OH, -NH(CH₃), -N(CH₃)₂, -CH₃, - CH₂CH₃, -CF₃, -OCH₃, and -OCF₃ . Typically, any one of the foregoing substituents, moieties or groups that is optionally substituted by replacing one or more its hydrogens has its hydrogen(s) replaced with one or two of the preceding optional substituents, or more typically with one of the preceding optional substituents. An optional substituent on a saturated aliphatic carbon atom within an acyclic or cyclic ring system further includes oxo (=O). For a phenyl or a 6-membered heteroaryl moiety, the arrangement of any two substituents present on the aromatic or heteroaromatic ring can be ortho (*o*), meta (*m*), or para (*p*).

Typically, an optional substituent replacing carbon in an acyclic carbon chain is selected from the group consisting of -O-, -C(=O)-, -C(=O)O-, -S-, -S(=O)-, -S(=O)₂-, - NH-, -NHC(=O)-, -C(=O)NH-, S(=O)₂NH-, -NHS(=O)₂, -OC(=O)NH-, and -NHC(=O)O-.

Typically, any one of the foregoing substituents, moieties or groups that is optionally substituted by replacing one or more alicyclic carbon atoms has the carbon atom(s) replaced with one or two of the preceding optional substituents, or more typically with one of the preceding optional substituents.

"Heterocyclyl" as used herein means a parent monovalent carbocyclic moiety, substituent or group that includes cycloalkyl, cycloalkenyl, aryl, or a fused combination thereof, wherein one or more, but not all of the skeletal carbon atoms within a ring of the parent carbocyclic moiety are replaced independently by a heteroatom, optionally substituted where permitted, including N, O, S, Se, B, Si, P, wherein two or more heteroatoms may be adjacent to each other or separated by one or more carbon atoms within the same ring system, typically by 1-3 atoms. Those heteroatoms typically include N, O or S. Thus, heterocycles include those having a heteroaromatic ring (also known as a heteroaryl) or a heterocycloalkyl ring, either of which may be fused with an carbocyclic, aryl or heteroaryl moiety and includes phenyl- (i.e., benzo) fused heterocycloalkyl and heteroaryl moieties, provided that when a heteroaryl moiety is fused to a heterocycloalkyl or carbocyclic moiety (i.e., when the heterocyclic portion of the fused ring system is monovalent) the resulting fused ring system is classified as a heteroaryl and when a heterocycloalkyl moiety is fused to a carbocyclic moiety (i.e., when the carbocyclic portion of the fused ring system is monovalent) the resulting fused ring system is classified as a heterocycloalkyl.

Heterocycles typically contain a total one to four heteroatoms in the ring(s), provided that not all of the skeletal atoms of any one ring system in the heterocyclic moiety are heteroatoms, wherein each heteroatom in the ring(s), optionally substituted where permitted, is independently selected from O, S and N, wherein the heterocyclic group has a total of 4 to 10 atoms in its monocyclic or fused ring system, and with the proviso that any one ring does not contain two adjacent O or S atoms. Heterocycloalkyls have at least 3 atoms in their ring system, and heteroaryl groups have at least 5 atoms in their ring system. Heterocycles include, by way of example and not limitation, heterocycles and heteroaryls, which are aromatized heterocycles, as provided by Paquette, Leo A.; "Principles of Modern Heterocyclic Chemistry" (W. A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. 1960, 82:5545-5473 particularly 5566-5573).

Heteroaryls typically contain a total one to four heteroatoms in the ring(s) of the heteroaryl ring system, provided that not all of the skeletal atoms of any one ring system in the heterocyclic moiety are heteroatoms, optionally substituted where permitted, and have 0-3 N atoms, 1-3 N atoms or 0-3 N atoms with 0-1 O atoms or 0-1 S atoms, provided that at least one heteroatom is present. A heteroaryl may be monocyclic or bicyclic. The ring system of a heteroaryls ring typically contains 1-9 carbons (i.e., C₁-C₉ heteroaryl). Monocyclic heteroaryls include C₁-C₅ heteroaryls. Monocyclic heteroaryls include those having 5-membered or 6-membered ring systems. A 5-membered heteroaryl is a C₁-C₄ heteroaryl that contains 1 to 4 carbon atoms and the requisite number of heteroatoms within an heteroaromatic ring system. A 6-membered heteroaryl is a C₁-C₅ heteroaryl containing 1 to 5 carbon atoms and the requisite number of heteroatoms within an heteroaromatic ring system. Heteroaryls that are 5-membered include C₁, C₂, C₃ and C₄ heteroaryls having four, three, two or one aromatic heteroatom(s), respectively, and heteroaryls that are 6-membered include C₂, C₃, C₄ and C₅ heteroaryls having four, three, two or one aromatic heteroatom(s), respectively. C₁-C₄ heteroaryls that are 5-membered are exemplified by monovalent moieties derived from removing a hydrogen atom from an aromatic carbon or an electron from an aromatic heteroatom, where permitted, from the following parent heterocycle compounds: pyrrole, furan, thiophene, oxazole, isoxazole, thiazole, isothiazole, imidazole, pyrazole, triazole and tetrazole. C₂-C₄ heteroaryls that are 6-membered are exemplified by monovalent moieties derived from removing a hydrogen atom from an aromatic carbon or an electron from an aromatic heteroatom, where permitted, from the following parent heterocycle compounds: pyridine, pyridazine , pyrimidine, and triazine. Bicyclic heteroaryls (i.e., heteroaryls that have fused aromatic rings in which at least one is heteroaromatic) include C₆-C₉ heteroaryls (i.e., heteroaromatic moieties containing a total of 6-9 aromatic carbon atoms and at least one aromatic heteroatom). Some bicyclic heteroaryls are exemplified by monovalent moieties derived from removing a hydrogen atom from an aromatic carbon or an electron from an aromatic heteroatom, where permitted, of a heteroaromatic ring of the following parent 6,5-bicyclic heterocycle compounds: benzofuran (O1), isobenzofuran (O1), indole (N1), isoindole (N1), indolizine (N1), indoline (N1), isoindoline (N1), purine (N4), benzimidazole (N2), indazole (N2), benzoxazole (N1O1), benzisoxazole (N1O1), benzodioxole (02), benzofurazan (N2O1), benzotriazole (N3), benzothiofuran (S1), benzothiazole (NISI), benzothiadiazole (N2S). Other bicyclic heteroaryls are exemplified by monovalent moieties derived from removing a hydrogen atom from an aromatic carbon or an electron from an aromatic heteroatom, where permitted, of a heteroaromatic ring of the following parent 6,6-bicyclic heterocycle compounds: chromene (O1), isochromene (O1), chroman (O1), isochroman (O1), benzodioxan (02), quinoline (N1), isoquinoline (N1), quinolizine (N1), benzoxazine (N1O1), benzodiazine (N2), pyridopyridine (N2), quinoxaline (N2), quinazoline (N2), cinnoline (N2), phthalazine (N2), naphthyridine (N2), pteridine (N4). For the above 5,6- and 6,6-bicyclic heteroaromatic compounds the parenthetic expressions indicate the heteroatom composition of the fused aromatic ring system. Depending on the structure, a heteroaryl group can be a monoradical or a diradical (i.e., a heteroarylene group).

More typically a heteroaryl is an aryl moiety wherein one 1, 2 or 3 of the carbon atoms of the aromatic ring(s) of a parent aryl moiety are replaced by a heteroatom, optionally substituted where permitted, including N, O and S, provided that not all of the skeletal atoms of any one aromatic ring system in the aryl moiety are replaced by heteroatoms and more typically are replaced by oxygen (-O-), sulfur (-S-) nitrogen (=N-) or -NR- wherein R is -H, a protecting group or alkyl, aryl or is nitrogen substituted with another organic moiety in a manner which retains the cyclic conjugated system, wherein the nitrogen, sulfur or oxygen heteroatom participates in the conjugated system either through pi-bonding with an adjacent atom in the ring system or through a lone pair of electrons on the heteroatom.

Non-limiting examples of heteroaryls include pyridyl, thiazolyl, pyrimidinyl, furanyl, thienyl, pyrrolyl, pyrazolyl, purinyl, imidazolyl, benzofuranyl, indolyl, isoindoyl, quinolinyl, isoquinolinyl, benzimidazolyl, pyridazinyl, pyrazinyl, benzothiopyran, benzotriazine, isoxazolyl, pyrazolopyrimidinyl, quinoxalinyl, thiadiazolyl, triazolyl and the like. Monocyclic heteroaryls include, by way of example and not limitation, pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, pyridazinyl, triazinyl, oxadiazolyl, thiadiazolyl, and furazanyl.

Non-limiting examples of heterocycles that are not heteroaryls include tetrahydrothiophenyl, tetrahydrofuranyl, indolenyl, piperidinyl, pyrrolidinyl, 2-pyrrolidonyl, tetrahydroquinolinyl, tetrahydroisoquinolinyl, decahydroquinolinyl, octahydroisoquinolinyl, 2H-pyrrolyl, 3H-indolyl, 4H-quinolizinyl, imidazolidinyl, imidazolinyl, pyrazolidinyl, piperazinyl, quinuclidinyl, morpholinyl and oxazolidinyl.

Typically, a heterocycloalkyl is a cycloalkyl group, moiety or substituent wherein 1, 2 or 3 carbons of the cycloalkyl chain is replaced with a heteroatom selected from the group consisting of nitrogen, oxygen and sulfur and is a C₂-C₁₀ heterocycloalkyl, more typically a C₄-C₁₀ heterocycloalkyl. Non-limiting heterocycloalkyls may contain 0-2 N atoms, 0-2 O atoms or 0-1 S atoms or some combination thereof provided at least one of said heteroatoms is present in the cyclic ring system and may be substituted with one or two oxo (=O) moieties, as in pyrrolidin-2-one. More typically, heterocycloalkyls include pyrrolidinyl, tetrahydrofuranyl, tetrahydropyranyl, piperidinyl, morpholinyl, piperazinyl, indolinyl and monosaccharaides.

Heterocycloalkyls include, by way of example but not limitation, pyrrolidinyl, tetrahydrofuranyl, dihydrofuranyl, tetrahydrothienyl, oxazolidinonyl, tetrahydropyranyl, dihydropyranyl, tetrahydrothiopyranyl, piperidinyl, morpholinyl, thiomorpholinyl, thioxanyl, piperazinyl, aziridinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, pyrrolin-2-yl, pyrrolin-3-yl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3azabicyclo[4.1.0]heptanyl, 3H-indolyl and quinolizinyl. Heterocycloalkyls further include all ring forms of carbohydrates, including but not limited to monosaccharaides, disaccharides and oligosaccharides.

When heterocycle is used as a Markush group (i.e., a substituent) the heterocycle is attached to a Markush formula with which it is associated through a carbon or a heteroatom of the heterocycle, where such an attachment does not result in an unstable or disallowed formal oxidation state of that carbon or heteroatom. A heterocycle that is C-linked is bonded to a molecule through a carbon atom sometimes described as - C<heterocycle where C< represents a carbon atom in a heterocycle ring. A heterocycle that is N-linked is a nitrogen containing heterocycle that is bonded a heterocycle ring nitrogen sometimes described as -N<heterocycle where N< represents a nitrogen atom in a heterocyclic ring. Thus, nitrogen-containing heterocycles may be C-linked or N-linked and include pyrrole substituents, which may be pyrrol-1-yl (N-linked) or pyrrol-3-yl (C-linked), imidazole substituents, which may be imidazol-1-yl or imidazol-3-yl (both N-linked) or imidazol-2-yl, imidazol-4-yl or imidazol-5-yl (all of which are C-linked).

A "5-membered nitrogen heteroaryl" is a 5-membered heteroaromatic moiety containing at least one nitrogen atom it its aromatic ring system and is a monocyclic heteroaryl or is fused to an aryl or another heteroaryl ring system and may contain one or more other independently selected heteroatoms such as N, O or S. Exemplary 5-membered heteroaryls include thiazole, imidazole, oxazole, triazole, pyrrolopyrimidines, pyrazolopyrimidines, indole, and isoindole.

"Heteroarylalkyl" as used herein means a substituent, moiety or group where a heteroaryl moiety is bonded to an alkyl moiety, i.e., -alkyl-heteroaryl, where alkyl and heteroaryl groups are as described above. When heteroarylalkyl is used as a Markush group (i.e., a substituent) the alkyl moiety of the heteroarylalkyl is attached to a Markush formula with which it is associated through a sp³ carbon of the alkyl moiety.

"Alkylheteroaryl" as used herein means a substituent, moiety or group where a heteroaryl moiety is bonded to an alkyl moiety, i.e., -heteroaryl-alkyl, where heteroaryl and alkyl groups are as described above. When heteroarylalkyl is used as a Markush group (i.e., a substituent) the heteroaryl moiety of the heteroarylalkyl is attached to a Markush formula with which it is associated through a sp² carbon or heteroatom of the alkyl moiety.

"O-linked moiety", "O-linked substituent" and like terms as used herein refers to a group or substituent that is attached to a moiety directly through an oxygen atom of the group or substituent. An O-linked group may be monovalent including groups such as -OH, acetoxy (i.e., -OC(=O)CH₃), acyloxy (i.e., -OC(=O)R^{a}, wherein R^{a} is -H, optionally substituted alkyl, optionally substituted cycloalkyl, optionally substituted alkenyl, optionally substituted alkynyl, optionally substituted aryl, optionally substituted heteroaryl or optionally substituted heterocycle), and further include monovalent groups such as aryloxy (Aryl-O-), phenoxy (Ph-O-), heteroaryloxy (Heteroaryl-O-), silyloxy, (i.e., R₃SiO-, wherein R independently are alkyl or aryl, optionally substituted), and -OR^{PR}, wherein R^{PR} is a protecting group as previously defined, or an O-linked group may be divalent, i.e., =O or -X-(CH₂)ₙ-Y-, wherein X and Y independently are S and O and n is 2 to 3, to form a spiro ring system with the carbon to which X and Y are attached.

"Halogen" or "halo" as used herein means fluorine, chlorine, bromine or iodine and is typically -F or -Cl.

"Protecting group" as used here means a moiety that prevents or reduces the ability of the atom or functional group to which it is linked from participating in unwanted reactions. Typical protecting groups for atoms or functional groups are given in Greene (1999), "Protective groups in organic synthesis, 3rd ed.", Wiley Interscience. Protecting groups for heteroatoms such as oxygen, sulfur and nitrogen are sometime used to minimize or avoid unwanted their reactions with electrophilic compounds. Other times the protecting group is used to reduce or eliminate the nucleophilicity and/or basicity of the unprotected heteroatom. Non-limiting examples of protected oxygen are given by - OR^{PR}, wherein R^{PR} is a protecting group for hydroxyl, wherein hydroxyl is typically protected as an ester (e.g. acetate, propionate or benzoate). Other protecting groups for hydroxyl avoid interfering with the nucleophilicity of organometallic reagents or other highly basic reagents, where hydroxyl is typically protected as an ether, including alkyl or heterocycloalkyl ethers, (e.g., methyl or tetrahydropyranyl ethers), alkoxymethyl ethers (e.g., methoxymethyl or ethoxymethyl ethers), optionally substituted aryl ethers and silyl ethers (e.g., trimethylsilyl (TMS), triethylsilyl (TES), tert-butyldiphenylsilyl (TBDPS), tert-butyldimethylsilyl (TBS/TBDMS), triisopropylsilyl (TIPS) and [2-(trimethylsilyl)ethoxy]-methylsilyl (SEM)). Nitrogen protecting groups include those for primary or secondary amines as in -NHR^{PR} or -N(R^{PR})₂-, wherein least one of R^{PR} is a nitrogen atom protecting group or both R^{PR} together comprise a protecting group.

A protecting group is a suitable protecting when it is capable of preventing or avoiding unwanted side-reactions or premature loss of the protecting group under reaction conditions required to effect desired chemical transformation elsewhere in the molecule and during purification of the newly formed molecule when desired, and can be removed under conditions that do not adversely affect the structure or stereochemical integrity of that newly formed molecule. By way of example and not limitation, a suitable protecting group may include those previously described for protecting functional groups. A suitable protecting group is typically a protecting group used in peptide coupling reactions.

"Ester" as used herein means a substituent, moiety or group that contains a -C(=O)-O- structure (i.e., ester functional group) wherein the carbon atom of the structure is not directly connected to another heteroatom and is directly connected to -H or another carbon atom of an organic moiety, and the monovalent oxygen atom is attached to the same organic moiety to provide a lactone or a different organic moiety . Typically, esters comprise or consist organic moieties containing 1-50 carbon atoms, typically 1-20 carbon atoms or more typically 1-8 carbon atoms and 0 to 10 independently selected heteroatoms (e.g., O, S, N, P, Si, but usually O, S and N), typically 0-2 where the organic moieties are bonded through the -C(O)-O- structure (i.e., through the ester functional group). When an ester is a substituent or variable group of a Markush structure that substituent is bonded to the structure through the monovalent oxygen atom of the ester functional group. In those instances the organic moiety attached to the carbonyl carbon of the ester functional group comprises any one of the organic groups described herein, e.g., C₁₋₂₀ alkyl moieties, C₂₋₂₀ alkenyl moieties, C₂₋₂₀ alkynyl moieties, C₆-C₁₀ aryl moieties, C₄₋₈ heterocycles or substituted derivatives of any of these, e.g., comprising 1, 2, 3, 4 or more substituents, where each substituent is independently chosen. Exemplary esters include, by way of example and not limitation, acetate, propionate, isopropionate, isobutyrate, butyrate, vale rate, isovalerate, caproate, isocaproate, hexanoate, heptanoate, octanoate, phenylacetate esters or benzoate esters.

"Ether" as used herein means an organic moiety, group or substituent that comprises 1, 2, 3, 4 or more -O- (i.e., oxy) moieties that are not bonded to carbonyl moiety(ies) , usually 1 or 2, wherein no two -O- moieties are immediately adjacent (i.e., directly attached) to each other. Typically, an ether structure is comprised or consists of the formula -O-organic moiety wherein organic moiety is as described for an organic moiety bonded to an ester functional group. More typically, an ether moiety, group or substituent has the formula of -O-organic moiety wherein the organic moiety is as described herein for an optionally substituted alkyl group. When ether is used as a Markush group (i.e., an ether substituent) the oxygen of the ether functional group is attached to a Markush formula with which it is associated. When ether is a used as substituent in a Markush group it is sometimes designated as an "alkoxy" group. Alkoxy includes C₁-C₄ ether substituents such as, by way of example and not limitation, methoxy, ethoxy, propoxy, iso-propoxy and butoxy.

"Amide" or "carboxamide" as used here means an moiety that contains a - C(=O)N(R)₂ or R-C(=O)N(R)- structure (i.e., carboxamide or amide functional group, respectively) with no other heteroatom directly attached to the carbonyl carbon of the structure and where R, independently selected, is hydrogen, a protecting group or an organic moiety wherein the organic moiety is as described herein for an organic moiety bonded to an ester functional group and is typically an optionally substituted alkyl group. Typically, hydrogen or an organic moiety, independently selected from R, is bonded to the carboxamide or amide functional group, wherein the organic moiety is also as described herein for an organic moiety bonded to an ester functional group. When bonded to an organic moiety the resulting structure is represented by organic moiety-C(=O)N(R)₂, or R-C(=O)N(R)-organic moiety. When an amide is recited as a variable for a Markush structure, the amide nitrogen is bonded to that structure. For carboxamide substituents the carbonyl carbon of the amide functional group is bonded to the Markush structure. Amides and carboxamides are typically prepared by condensing an acid halide, such an acid chloride with a molecule containing a primary or secondary amine. Alternatively, amide coupling reactions well known in the art of peptide synthesis, which oftentimes proceed through an activated ester of a carboxylic acid-containing molecule, are used. Exemplary preparations of amide bonds through peptide coupling methods is provided in Benoiton (2006) Chemistry of peptide synthesis CRC Press, Bodansky "Peptide synthesis: A practical textbook" (1988) Springer-Verlag; Frinkin, M. et al. "Peptide Synthesis" Ann. Rev. Biochem. (1974) 43: 419-443. Reagents used in the preparation of activated carboxylic acids is provided in Han, et al. "Recent development of peptide coupling agents in organic synthesis" Tet. (2004) 60: 2447-2476.

"Carbonate" as used here means a substituent, moiety or group that contains a -O-C(=O)-O- structure (i.e., carbonate functional group). Typically, carbonate groups as used here comprise or consist of an organic moiety, wherein the organic moiety is as described herein for an organic moiety bonded to an ester functional group, bonded through the -O-C(=O)-O- structure, e.g., organic moiety-O-C(=O)-O-. When carbonate is used as a Markush group (i.e., a substituent) one of the singly bonded oxygen atoms of the carbonate functional group is attached to a Markush formula with which it is associated and the other is bonded to a carbon atom of an organic moiety as previously described for an organic moiety bonded to an ester functional group.

"Carbamate" or "urethane" as used here means a substituent, moiety or group that contains a structure represented by -O-C(=O)N(R^{a})- (i.e., carbamate functional group) or -O-C(=O)N(R^{a})₂, -O-C(=O)NH(optionally substituted alkyl) or -O-C(=O)N(optionally substituted alkyl)₂ (i.e., exemplary carbamate substituents) wherein R^{a} and optionally substituted alkyl are independently selected wherein R^{a}, independently selected, is hydrogen, a protecting group or an organic moiety, wherein the organic moiety is as described herein for an organic moiety bonded to an ester functional group and is typically an optionally substituted alkyl. Typically, carbamate groups as used herein comprise or consist of an organic moiety, independently selected from R^{a}, wherein the organic moiety is as described herein for an organic moiety bonded to an ester functional group, bonded through the -O-C(=O)-N(R^{a})- structure, wherein the resulting structure has the formula of organic moiety-O-C(=O)-N(R^{a})- or -O-C(=O)-N(R^{a})-organic moiety. When carbamate is used as a Markush group (i.e., a substituent), the singly bonded oxygen (O-linked) or nitrogen (N-linked) of the carbamate functional group is attached to a Markush formula with which it is associated. The linkage of the carbamate substituent is either explicitly stated (N- or O-linked) or implicit in the context to which this substituent is referred.

"Urea" as used here means a substituent, moiety or group that contains a structure represented by -N(R^{a})-C(=O)N(R^{a})- (i.e., urea functional group) and typically is represented by -NH-C(=O)NH(optionally substituted alkyl) or -NH-C(=O)N(optionally substituted alkyl)₂- (i.e., optionally substituted alkyl are exemplary urea substituents), wherein R^{a} and optionally substituted alkyl are independently selected with each R^{a} is independently -H, a protecting group or an organic moiety as described for an organic moiety bonded to an ester functional group. When an organic moiety is bonded through a urea functional group the resulting structure is represented by organic moiety-N(R^{a})-C(=O)-N(R^{a})- or -N(R^{a})-C(=O)-N(R^{a})-organic moiety. When urea is used as a Markush group (i.e., as a substituent), a singly- bonded nitrogen of the urea functional group is attached to a Markush formula with which it is associated while the other singly-bonded nitrogen is unsubstituted or is mono or disubstituted with one or two other independently selected organic moieties, wherein the organic moiety(ies) are as described herein for an organic moiety bonded to an ester functional group ester.

"Antibody" as used herein is used in the broadest sense and specifically covers intact monoclonal antibodies, polyclonal antibodies, monospecific antibodies, multispecific antibodies (e.g., bispecific antibodies), and antibody fragments that exhibit the desired biological activity provided that the antibody fragment have the requisite number of attachment sites for a drug-linker. The native form of an antibody is a tetramer and consists of two identical pairs of immunoglobulin chains, each pair having one light chain and one heavy chain. In each pair, the light and heavy chain variable regions (VL and VH) are together primarily responsible for binding to an antigen. The light chain and heavy chain variable domains consist of a framework region interrupted by three hypervariable regions, also called "complementarity determining regions" or "CDRs." The constant regions may be recognized by and interact with the immune system (*see, e.g.,* Janeway et al., 2001, Immunol. Biology, 5th Ed., Garland Publishing, New York). An antibody can be of any type (*e.g.,* IgG, IgE, IgM, IgD, and IgA), class (*e.g.,* IgG1, IgG2, IgG3, IgG4, IgA1 and IgA2) or subclass. The antibody can be derived from any suitable species. In some embodiments, the antibody is of human or murine origin. An antibody can be, for example, human, humanized or chimeric. An antibody or antibody fragment thereof, is an exemplary ligand targeting moiety that is incorporated into an LDC of the present invention.

In some aspects an antibody selectively and specifically binds to an epitope on hyper-proliferating cells or hyper-stimulated mammalian cells (i.e., abnormal cells), wherein the epitope is preferentially displayed by or is more characteristic the abnormal cells in contrast to normal cells, or is preferentially displayed by or is more characteristic of normal cells in the vicinity of abnormal cells in contrast to normal cells not localized to the abnormal cells. In those aspects the mammalian cells are typically human cells.

"Monoclonal antibody" as used herein refers to an antibody obtained from a population of substantially homogeneous antibodies, *i.e.,* the individual antibodies comprising the population are identical except for possible naturally-occurring mutations that may be present in minor amounts. Monoclonal antibodies are highly specific, being directed against a single antigenic site. The modifier "monoclonal" indicates the character of the antibody as being obtained from a substantially homogeneous population of antibodies, and is not to be construed as requiring production of the antibody by any particular method.

"Cytotoxic activity" as used herein refers to a cell-killing effect of a drug, Ligand-Drug Conjugate, or an intracellular metabolite of a Ligand- Drug Conjugate. Cytotoxic activity may be expressed as the IC₅₀ value, which is the concentration (molar or mass) per unit volume at which half the cells survive.

"Cytostatic activity" as used herein refers to an anti-proliferative effect of a drug, Ligand-Drug Conjugate, or an intracellular metabolite of a Ligand-Drug Conjugate that is not dependent on cell killing but whose effect is due to inhibition of cell division of hyper-proliferating cells, hyper-stimulated immune cells or other abnormal or unwanted cells.

The terms "specific binding" and "specifically binds" mean that an antibody or antibody in an LDC as the targeting moiety is capable of binding, in a highly selective manner, with its corresponding target antigen and not with a multitude of other antigens. Typically, the antibody or antibody derivative binds with an affinity of at least about 1 x 10⁻⁷ M, and preferably 10⁻⁸ M to 10⁻⁹ M, 10⁻¹⁰ M, 10⁻¹¹ M, or 10⁻¹² M and binds to the predetermined antigen with an affinity that is at least two-fold greater than its affinity for binding to a non-specific antigen (*e.g*., BSA, casein) other than for a closely-related antigen.

"Ligand-drug conjugate" or "LDC" as the term is used herein refers to a construct comprised of a Ligand Unit from a targeting moiety and a quaternized tertiary amine-containing Drug Unit (D⁺) corresponding in structure to a tertiary-amine containing drug that are bonded to each other through a Linker Unit, wherein the LDC selectively binds to a target moiety through its Ligand Unit. In some instances, the term LDC is a plurality (i.e., composition) of individual LDC compounds differing primarily by the number of D⁺ units bonded to each Ligand Unit or the location on the Ligand Unit at which the D+ units are bound. In other instances the term LDC applies to an individual member of the composition.

"Targeting moiety" as the term is used herein is a moiety incorporated as a Ligand Unit in a LDC that binds selectively to a target moiety typically present on, within, or in the vicinity of hyper-proliferating cells, hyper-stimulated immune cells or other abnormal or unwanted cells in comparison to other moieties present on, within, or in the vicinity of normal cells where these abnormal or unwanted cells are typically not present. Sometimes a target moiety is present on, within, or in the vicinity of abnormal in greater abundance in comparison to normal cells or the environment of normal cells where abnormal cells are typically not present. In some instances the targeting moiety is an antibody that specifically binds to an accessible antigen characteristic of an abnormal cell or is an accessible antigen that is particular to the surrounding environment in which these cells are found. In other instances the targeting moiety is a ligand that specifically binds to an accessible receptor characteristic of, or in greater abundance on, abnormal cells or other unwanted cells, or is an accessible receptor that is particular to cells of the surrounding environment in which abnormal cells are found. Typically a targeting moiety is an antibody as defined herein that binds selectively to a target moiety of an abnormal or unwanted mammalian cell, more typically a target moiety of an abnormal or unwanted a human cell.

"Target cells" as the term is used herein are the intended cells (i.e., abnormal or other unwanted cells) to which an LDC is designed to interact in order to inhibit the proliferation or other unwanted activity of the intended cells. In some instances the target cells are hyper-proliferating cells or hyper-activated immune cells, which are exemplary abnormal cells. Typically those abnormal cells are mammalian cells and more typically are human cells. In other instances the target cells are within the vicinity of abnormal or unwanted cells so that action of the LDC on the nearby cells has an intended effect on the abnormal or unwanted cells. For example, the nearby cells may be epithelial cells that are characteristic of the abnormal vasculature of a tumor. Targeting of those vascular cells by an LDC will either have a cytotoxic or cytostatic effect on these cells, which inhibits nutrient delivery to the abnormal cells of the tumor to indirectly have a cytotoxic or cytostatic effect on the abnormal cells, and/or have a direct a cytotoxic or cytostatic effect on the abnormal cells by releasing an active drug moiety in the vicinity of these cells.

"Target moiety" as the term is used herein is a moiety preferentially recognized by a targeting moiety or Ligand unit of a Ligand-Drug Conjugate from that targeting moiety (i.e., is selectively bound by the Ligand Unit) and is present on, within or in the vicinity of target cells. Sometimes the target moiety is an antigen accessible to selective binding by an antibody, which is an exemplary targeting moiety that is incorporated in a LDC as a Ligand Unit. In those instances, such an antigen is a cell-surface protein present on abnormal cells or other unwanted cells, or is present on cells that are particular to the surrounding environment in which the abnormal or unwanted cells are found, such as vascular cells that are characteristic of the environment of hyper-proliferating cells in a tumor. More typically, the antigen is a cell-surface protein of an abnormal cell or other unwanted cell that is capable of internalization upon binding with its cognate targeting ligand. In other instances the targeting moiety is a ligand for an extracellularly accessible cell membrane receptor that may be internalized upon binding of the targeting moiety or is capable of passive or facilitative transport of the LDC targeting the cell-surface receptor. In some aspects, the target moiety is present on abnormal mammalian cells or on mammalian cells characteristic of the environment of such abnormal cells.

"Antibody-drug conjugate" or "ADC" as the term is used herein refers to an LDC wherein the targeting moiety is an antibody, wherein the antibody is covalently attached to a quaternized drug unit (D⁺), typically through an intervening Linker unit. Oftentimes the term refers to a collection (i.e., population or plurality) of conjugates having the same antibody, Drug unit, and Linker unit, but having variable loading or distribution of the linker-drug moiety for each antibody (as for example when the number of D⁺ any two ADCs in a plurality of such constructs is the same but the location of their sites of attachment to the targeting moiety differ). In those instances an ADC is described by the averaged drug loading of the conjugates. An ADC obtained from the methods described herein have the general structure of Ab-L_{b}-Lₒ-D⁺ where L_{b}-Lₒ defines the Linker unit wherein L_{b} is a ligand covalent binding moiety sometimes referred to as a primary linker (L_{R}) linker, so named because that moiety is required to be present in a ligand unit of an ADC, Lₒ is an secondary linker susceptible to enzymatic (e.g., protease or glycosidase) or non-enzymatic (e.g., reductive or hydrolytic) cleavage. In some instances that cleavage is enhanced in the environment of abnormal or occurs subsequent to intracellular internalization of the ADC subsequent to binding of the ADC's targeting antibody to its cognate antigen. D⁺ is a quaternized tertiary amine containing drug unit D wherein D is released as a result of that enzymatic or non-enzymatic action on Lₒ.

The average number of Ligand Units per antibody, or fragment thereof, in an ADC composition (i.e., an averaged number for a population of ADC conjugates that differ by the number of conjugated drug units or their location in each of the ADCs that are present in that population) is designated as p or when the linkers are not branched, p is the average number of drug-linker moieties,. In that context p is a number ranging from about 2 to about 20 and is typically about 2, about 4, or about 8. In other contexts p represents the number of drug units, or Linker-drug units when the linkers are not branched, that are covalently bonded to a single antibody of an ADC within a population of antibody-drug conjugates that differ by the number or location of conjugated linker-drug units in each of the ADCs, and is designated as p'. In that content p' is an integer ranging from 1 to 20, typically 1 to 12, 1 to 10, and more typically from 1 to 8.

The average number of Drugs units per Ligand unit in a preparation from a conjugation reaction may be characterized by conventional means such as mass spectroscopy, ELISA assay, HIC and/or HPLC. The quantitative distribution of Drug-Linker-Ligand conjugates in terms of p may also be determined. In some instances, separation, purification, and characterization of homogeneous Ligand-Drug Conjugates, where p is a certain value from Ligand-Drug Conjugate with other drug loadings may be achieved by means such as reverse phase HPLC or electrophoresis.

"Antigen" is an entity that is capable of selective binding to an unconjugated antibody or a fragment thereof or to an ADC comprising a Ligand Unit from an antibody or fragment thereof. In some aspects, the antigen is an extracellularly-accessible cell-surface protein, glycoprotein, or carbohydrate preferentially displayed by abnormal or other unwanted cells in comparison to normal cells. In some instances the unwanted cells having the antigen are hyper-proliferating cells in a mammal. In other instances, the unwanted cells having the antigen are hyper-activated immune cells in a mammal. In other aspects, the specifically bound antigen is present in the particular environment of hyper-proliferating cells or hyper-activated immune cells in a mammal in contrast to the environment typically experienced by normal cells in the absence of such abnormal cells. In still other aspects the cell-surface antigen is capable of internalization upon selective binding of an ADC is associated with cells that are particular to the environment in which hyper-proliferating or hyper-stimulated immune cells are found in the absence of such abnormal cells. An antigen is an exemplary targeted moiety of an LDC wherein its Ligand Unit from the targeting moiety is an antibody that preferentially recognizes that antigen through selective binding.

Antigens associated with hyper-proliferating cells that are cell-surface accessible to an ADC include by way of example and not limitation CD19, CD70, CD30, CD33, NTB-A, αvβ6, and CD123.

"Ligand covalent binding moiety" is a moiety comprising a LDC that binds selectively to its cognate targeted moiety and is sometimes referred to as a targeting moiety. A Ligand covalent binding moiety includes without limitation receptor ligands, antibodies to cell-surface antigens and transporter substrates. Sometimes, the receptor, antigen or transporter to be bound by an LDC is present in greater abundance on abnormal cells in contrast to normal cells. Other times the receptor, antigen or transporter to be bound by an LDC is present in greater abundance on abnormal cells in contrast to normal cells.

"Ligand covalent binding moiety precursor" is a moiety of a linker unit, or substructure thereof used in the preparation of a linker unit, that is capable of covalent binding to a targeting moiety during the preparation of an LDC whereupon the ligand binding moiety precursor (L_{b}') is converted to a ligand covalent binding moiety (L_{b}). In some aspects a L_{b}' moiety typically has a functional group capable of reacting with a nucleophile or electrophile native to an antibody or fragment thereof or is introduced into an antibody by chemical transformation or genetic engineering. In some aspect the nucleophile is an N-terminal amino group of a peptide comprising an antibody or the epsilon amino group of a lysine residue. In other aspects the nucleophile is a sulfhydryl group of a cysteine residue introduced by genetic engineering or from chemical reduction of an interchain disulfide of an antibody. In some aspects the electrophile is an aldehyde introduced by selective oxidation of an antibody's carbohydrate moiety or is a ketone from an unnatural amino acid introduced into an antibody using a genetically engineered tRNA/tRNA synthetase pair. Those and other methods are reviewed by Behrens and Liu "Methods for site-specific drug conjugation to antibodies" mAB (2014) 6(1): 46-53.

"Ligand covalent binding moiety" is a moiety of a ligand unit in an LDC that covalently binds the remainder of the ligand unit that targets the abnormal or unwanted cells or their environment and is derived from reaction of the corresponding L_{b}' in a ligand unit precursor with the targeting moiety. For example, when L_{b}' is comprised of a maleimide moiety, reaction of that moiety with a reactive sulfhydryl group of a targeting moiety converts L_{b}' to L_{b} which is comprised of a thio substituted succinimide moiety. In another example, when L_{b}' is comprised of an activated carboxylic acid functional group, reaction of that functional group with an epsilon amino group of a lysine in a targeting moiety converts the functional group to an amide, wherein that amide comprises the L_{b} moiety of the attached ligand unit. Other L_{b} moieties and their conversion from L_{b}'-containing moieties are described in the embodiments of the invention. In some instances a targeting moiety is derivitized with a bi-functional molecule to provide an intermediate that is condensed with a ligand covalent binding precursor moiety. As a result of that condensation the L_{b} moiety so formed has atoms attributable to the bi-functional molecule and L_{b}'.

"Linker unit" as the term is used herein refers to an organic moiety in a ligand drug conjugate (LDC) intervening between and covalently attached to a quaternized drug unit (D⁺) and Ligand Unit from a targeting moiety. Typically, a linker unit (L) is comprised of a ligand covalent binding (L_{b}) moiety or a ligand covalent binding precursor (L_{b}') moiety and a secondary linker moiety as described herein. In some aspects the ligand covalent binding precursor contains a maleimide (M¹) moiety. Attachment of the targeting moiety through M¹ occurs through a cysteine sulfhydryl group of the targeting moiety by Michael addition of the sulfhydryl group to the maleimide ring system of M¹. As a result of that addition a succinimide (M²) moiety having a sulfur substituted succinimide ring system is obtained. Subsequent hydrolysis of that ring system, either spontaneously or under controlled conditions, as when that system is part of a self-stabilizing linker (L_{SS}) moiety, results in a succinic acid-amide (M³) moiety, which is an exemplary self-stabilized (L_{S}) moiety, as further described herein. Also covalently bonded to L_{b} or L_{b}', which are primary linker (L_{R} moieties), is a secondary linker (Lₒ) moiety, which further intervenes between the targeting moiety and quaternized drug unit in an LDC and is covalently bonded to L_{R} through intermediacy of an ether, ester, carbonate, urea, disulfide, amide or carbamate functional group, more typically through an ether, amide or carbamate functional group.

"Primary linker" as used is a ligand covalent binding moiety (L_{b}) or a ligand covalent binding moiety precursor (L_{b}') and is present as a component of a Linker unit of a LDC or as a component of a L_{b}'-containing moiety such as L_{b}'-Lₐ or L_{b}'-Lₒ-D⁺. A L_{b}' primary linker is comprised of a reactive functional group capable of reacting with an electrophilic or nucleophillic functional group of a targeting moiety. As a result of that reaction, the targeting moiety becomes covalently bonded as a Ligand Unit to a L_{b} primary linker through a functional group derived from the reactive functional group of L_{b}'.

"Secondary linker" moiety as used herein refers to an organic moiety in a Linker unit wherein the secondary linker (Lₒ) is covalently attached to a L_{b} or L_{b}' moiety (i.e., a primary linker unit) through intermediacy of a functional group between the L_{b} or L_{b}' moiety and the remainder of the linker unit to which a quaternized drug unit may be covalently attached. In a LDC, the secondary linker is also covalently attached to a quaternized drug unit (D⁺) through the benzylic position of a PAB or PAB-type self-immolating moiety that comprises a Spacer unit. In addition to a Spacer unit (Y), secondary linkers are comprised of a Cleavable (W) wherein W, Y and D⁺ are arranged either in a linear or orthogonal relationship and may be further comprised of a Stretcher unit (A). When present, A is sometimes comprised of a subunit A₁, which is scaffold moiety that interconnects the reactive functional group of L_{b}', or the functional group of L_{b} derived therefrom, with the remainder of the secondary linker.

In an LDC, Lₒ is comprised of a Spacer unit (Y) that contains a self-immolating moiety (SI) covalently attached to a Cleavable moiety unit (W) such that cleavage of W under conditions more likely experienced by or in the vicinity of abnormal cells in comparison to normal cells or their normal environment results in self-destruction of the self-emolliating moiety with concomitant release of D. Typically, that self-destruction occurs through a 1,6-elimination in an SI moiety as described herein. In those instances SI is attached to a tertiary amine-containing drug by quaternization of that drug's tertiary amine nitrogen.

A secondary linker (Lₒ) when bonded to D⁺ is typically represented by the structure of (1) or (2): 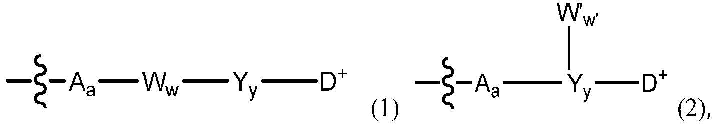 wherein Aₐ is a Stretcher unit; W_{w} and W'w' are Cleavable units; and Y_{y} is a Spacer unit, wherein a is 0 or 1, w or w' is 1 and y is 1. When a is 1 the wavy line before Aₐ indicates covalent bonding of that Lₒ subunit to L_{b}' or L_{b} (resulting from L_{b}' after its incorporation into an LDC). When a is 0 that wavy line indicates covalent binding of L_{b}' or L_{b} to a Cleavable unit W in structure (1) or Y in structure (2).

In some aspects of the invention Y in structure (1) is comprised or consists of a self-immolative (SI) moiety as described herein substituted with Wand D⁺. In other aspects of the invention Y in structure (2) is comprised or consists of a self-immolating moiety as described herein substituted with D⁺ through a quaternary amine nitrogen and is further substituted with Wand Ligand-L_{b}-A- or L_{b}'-A-, wherein A is optionally present (i.e., A is bonded to SI of Y when A is present or L_{b} or L_{b}' is bonded to SI of Y when A is absent).

Typically, secondary linkers with structure (1) are represented by 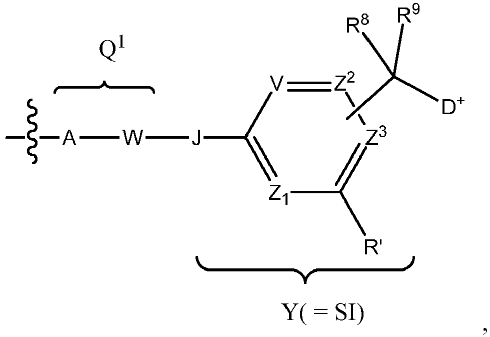 and secondary linkers with structure (2) are represented by 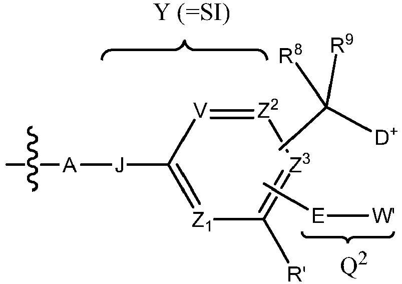 wherein Y consists of SI and E, J, V, Z¹, Z², Z³, R', R⁸ and R⁹ are as defined in embodiments for PAB or PAB-type self-immolative units.

"Maleimide moiety" as used herein is a ligand covalent binding precursor moiety having a maleimide ring system. A maleimide moiety (M¹) is capable of participating in Michael addition (i.e., 1,4-conjugate addition) of a targeting moiety-derived sulfhydryl group to provide a thio-substituted succinimide (M²) moiety, as described herein, which will be present in a Linker unit in an LDC. An M¹ moiety is attached to the remainder of the linker unit, through its imide nitrogen prior to its conversion to a thio-substituted succinimide moiety. Other than the imide nitrogen, an M¹ moiety is typically un-substituted, but may be asymmetrically substituted at the cyclic double bond of its maleimide ring system. Such substitution typically results in regiochemically preferred addition of a sulfhydryl group to the less hindered or more electronically deficient double bond carbon (dependent on the more dominant contribution) of the maleimide ring system. When present in a self-stabilizing linker (L_{SS}) moiety, controlled hydrolysis of the succinimide ring system of the thio-substituted succinimide moiety M² derived from such a substituted M¹ moiety is expected to provide regiochemical isomers of succinic acid-amide (M³) moieties in a self-stabilized linker (L_{S}) moiety that are due to differences in reactivity of the two carbonyl carbons of M² attributable to the substituent(s) that were present in the M¹ precursor.

"Succinimide moiety" as used herein is an organic moiety of which a Linker unit is comprised in an ADC and results from Michael addition of an antibody-derived sulfhydryl group to the maleimide ring system of a maleimide moiety (M¹). A succinimide (M²) moiety is therefore comprised of a thio-substituted succinimide ring system and has its imide nitrogen substituted with the remainder of the Linker unit and is optionally substituted with substituent(s) that were present on the M¹ precursor. Typically, when A is present the imide nitrogen is covalently attached to a stretcher moiety (A) or subunit thereof (i.e., A₁) as described herein. Sometimes M²-A (or M²-A₁) provides for a self-stabilizing linker (L_{SS}) moiety as described herein.

"Succinic acid-amide moiety" as used herein refers to succinic acid having an amide substituent that results from the thio-substituted succinimide ring system of a succinimide moiety M² having undergone breakage of one of its carbonyl-nitrogen bonds by hydrolysis. Hydrolysis resulting in a succinic acid-amide (M³) moiety provides a Linker unit less likely to suffer premature loss of an antibody in an LDC having that M³ moiety through elimination of the antibody-thio substituent. When present in a self-stabilizing linker (L_{SS}) moiety, controlled hydrolysis of the succinimide ring system of the thio-substituted succinimide moiety M² derived from such a substituted M¹ moiety is expected to provide regiochemical isomers of M³ moieties (individually referred to as M^{3A} and M^{3B}) that are due to differences in reactivity of the two carbonyl carbons of M² attributable to the substituent(s) that were present in the M¹ precursor.

"Self-stabilizing linker" as used herein is an L_{b}-containing moiety of Linker unit of an LDC, or precursor thereof (i.e., a L_{b}'-containing moiety) that under controlled conditions is capable of undergoing a chemical transformation to a self-stabilized linker moiety (L_{S}), such that an LDC initially comprised of a self-stabilizing (L_{SS}) moiety becomes more resistant to premature loss of targeting moiety from the LDC. Usually, a L_{SS} moiety in addition to a L_{b} or L_{b}' moiety is comprised of a stretcher unit or subunit thereof. In some those instances having L_{SS}, W and Y in a linear arrangement, L_{SS} is covalently attached through a stretcher unit or another subunit thereof to a cleavable unit (W). In other of those instances having W orthogonal to L_{SS}-Y, L_{SS} is covalently attached to Y through a stretcher unit or another subunit thereof. However, sometimes no intervening A is present and L_{SS} is covalently attached directly to W or Y depending on the relative arrangement of the L_{SS}, W and Y linker components. In some aspects, a L_{SS} prior to its incorporation into an LDC contains a maleimide (M¹) moiety as the L_{b}' moiety (through which a targeting moiety is to be attached) and a stretcher unit (A) or subunit thereof (i.e., A₁) and is represented by the formula of M¹-A or M¹-A₁. After incorporation into an LDC (i.e., after targeting moiety attachment through Michael addition to the maleimide moiety) the M¹-A (or M¹-A₁) moiety of an L_{SS} is converted to its corresponding thio-substituted succinimide moiety M²-A (or M₂-A₁). Usually, L_{SS} is also comprised of a Basic unit (BU) as described herein as a substituent of a stretcher unit bound to M² or its M¹ precursor. In those aspects, BU assists in the hydrolysis of the succinimide moiety of M² to its corresponding ring-opened form M³ [i.e., M²-A(BU)- or M²-A₁(BU)- is converted to M³-A(BU) or M³-A₁(BU)].

"Self-stabilized linker" is an organic moiety derived from an L_{SS} moiety, both of which are L_{b}-containing moieties, of an LDC that has undergone hydrolysis, typically under controlled condition, to provide a new L_{b}-containing moiety that is less likely to reverse the condensation reaction of a targeting moiety with a L_{b}'-containing moiety that provided the original L_{b}-containing moiety. Usually, a self-stabilized linker (L_{S}) is comprised of a stretcher unit or subunit thereof covalently attached to a moiety obtained from conversion of a succinimide moiety (M²), which has a thio-substituted succinimide ring system resulting from Michael addition of a sulfhydryl group of a targeting moiety to the maleimide ring system of M¹, to another moiety wherein that M²-derived moiety has reduced reactivity for elimination of its thio-substituent in comparison to the corresponding substituent in M². In those aspects, the M²-derived moiety has the structure of a succinic acid-amide (M³) moiety corresponding to M² wherein M² has undergone hydrolysis of one of its carbonyl-nitrogen bonds of its succinimide ring system. That hydrolysis may occur spontaneously or more typically is catalyzed by the basic functional group of BU that is covalently attached to a stretcher unit bound to M² and is in appropriate proximity as a result of that attachment to assist in the rupture of the carbonyl-nitrogen. The product of that hydrolysis therefore has a carboxylic acid functional group and an amide functional group substituted at its amide nitrogen, which corresponds to the imide nitrogen in the M²-containing L_{SS} precursor, by the structure of the aforementioned stretcher unit. Typically, that basic functional group is an amino group whose reactivity for base-catalyzed hydrolysis is controlled by pH. Thus, a self-stabilized linker (L_{S}) typically has the structure of M³ covalently bond to a scaffold unit or subunit thereof that in turn is covalently bonded to the remainder of the secondary linker Lₒ (Lₒ') in a linear arrangement and with the basic unit covalently attached to the M³-bound stretcher unit orthogonal to Lₒ. Ls with M³, A, BU and Lₒ arranged in the manner indicated is represented by the formula of M³-A(BU)-L₀' or M³-A₁(BU)-Lₒ'.

After hydrolysis, the resulting self-stabilized linker (L_{S}) typically has the structure of M³ covalently bond to the BU-substituted stretcher unit (M³-A(Bu)- or M³A₁(BU)-). That stretcher unit in turn is covalently bonded to the remainder of Lₒ (Lₒ') in a linear arrangement with the basic unit orthogonally arranged with respect to M³ and the other Lₒ component units. Exemplary structures of L_{SS} and L_{S} moieties with M² or M³, A(BU) [or A₁(BU)] and Lₒ' arranged in the manner indicated is shown by way of example but not limitation by: 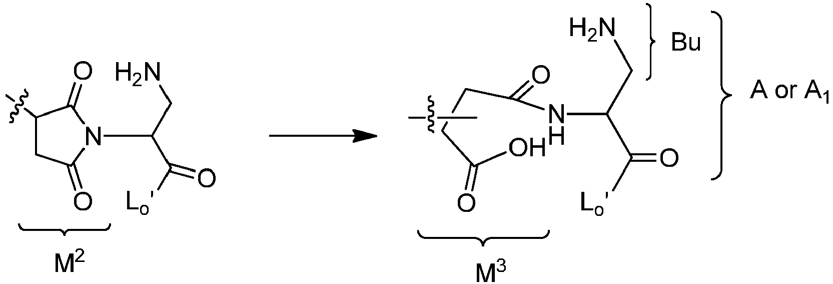 wherein the indicated CH(CH₂NH₂)C(=O) moiety is the structure of the stretcher unit, or subunit thereof, covalently bonded to the imide of amide nitrogen of M² or M³ where the -CH₂NH₂ moiety is the BU substituent of that stretcher unit. The remainder of the structures represents the succinimide moiety M² or succinic acid-amide moiety M³ from succinimide ring hydrolysis of M², which are substituted at the imide or the corresponding amide nitrogen with a sp³-carbon of the stretcher unit. The wavy line indicates attachment of a targeting moiety-derived sulfhydryl group resulting from Michael addition of that group to the maleimide ring system of M¹. Since the succinimide ring system of M² is asymmetrically substituted due to its targeting moiety-derived thio substituent, regiochemical isomers of succinic acid-amide (M³) moieties as defined herein differing in position relative to the liberated carboxylic acid group will typically result from M² hydrolysis. In the above structure, the carbonyl of the indicated stretcher unit exemplifies a hydrolysis enhancer (HE) as defined herein that is incorporated into the structure of such a unit.

M³-A(BU) represents exemplary structures of self-stabilized linker (L_{S}) moieties, since these structures are less likely to eliminate the thio substituent of the targeting moiety, and thus cause loss of that moiety, in comparison to the corresponding M²-A(BU) structure of an L_{SS} moiety. That increased stability results from the greater conformational flexibility in M³ in comparison to M², which no longer constrains the thio substituent in a conformation favorable for E2 elimination.

"Basic unit" as used herein is an organic moiety within a self-stabilizing linker (L_{SS}) moiety, as described herein, that can be carried forward into a corresponding L_{S} moiety by participating in base-assisted hydrolysis of the succinimide ring system within a M² moiety comprising L_{SS} (i.e., catalyzes water addition of a water molecule to one of the succinimide carbonyl-nitrogen bonds) and can be initiated under controlled conditions tolerable by the targeting moiety attached to L_{SS}. For that purpose, the basic functional group of the basic unit (BU) and its relative position in L_{SS} with respect to its M² component is selected for its ability to hydrogen bond to a carbonyl group of M² that effectively increase its electrophilicity and hence its susceptibility to water attack. Alternatively, those variables are selected so that a water molecule, whose nucleophilicity is increased by hydrogen bonding to the basic functional group of BU, is directed to an M² carbonyl group. Typically, BU, acting through either mechanism, is comprised of 1-6 contiguous carbon atoms that connect its basic amino group to the L_{SS} moiety to which it is attached. For increasing the electrophilicity of an M² carbonyl by hydrogen bonding, BU is required to have a primary or secondary amine as its basic functional group whereas increasing water nucleophilicity in the manner described above may be done with a primary, secondary or tertiary amine as the basic functional group. In order that a basic amine be in the required proximity to assist in the hydrolysis of a succinimide moiety M² to its corresponding ring opened carboxylic acid amide M³ by either mechanism, the amine-bearing carbon chain of BU is typically attached to a stretcher moiety of L_{SS} at the alpha carbon of that moiety relative to the point of attachment of A (or A₁) to the succinimide nitrogen of M² (and hence to the maleimide nitrogen of its corresponding M¹-A or M¹-A₁ structure). Typically, that alpha carbon has the (S) stereochemical configuration.

"Hydrolysis enhancer unit" as used herein is electron withdrawing group or moiety that is an optional substituent of a stretcher unit comprising an L_{SS} moiety. When present, a hydrolysis enhancer unit (HE) is usually incorporated into a stretcher unit bonded to the imide nitrogen of an M² moiety so that its electron withdrawing effects increases the electrophilicity of the succinimide carbonyl groups in that moiety. When the stretcher unit also has a BU substituent, the effect of HE on the carbonyl groups coupled with the effect of BU, which is dependent on the basicity of the BU basic functional group and the distance of that functional group in relation to those carbonyl, are balanced so that premature hydrolysis of M¹ or of M² to M³ does not occur or is negligible during preparation of an LDC from an L_{SS} precursor having the structure of M¹-A(BU)-, but allows for hydrolysis (i.e., conversion of an LDC-containing -M²-A(BU)- moiety to its corresponding -M³-A(BU)- moiety) under controlled conditions (as when pH is purposely increased) that are tolerable by the attached targeting moiety. Typically, the HE unit is a carbonyl moiety (i.e., ketone or -C(=O)-) or carbonyl-containing functional group located distal to the end of the stretcher unit that is bonded to M², or M³ derived therefrom, and that also covalently attaches that stretcher unit to the remainder of the secondary linker. Carbonyl-containing functional groups other than ketone include esters, carbamates, carbonates and ureas. When HE is a carbonyl-containing functional group other than ketone the carbonyl moiety of that functional group is typically bonded to A. In some aspects as when no BU substituent is present the HE unit may be sufficiently distant within a stretcher unit from the imide nitrogen to which the stretcher unit is covalently bonded so that no discernable effect on hydrolytic sensitivity of the succinimide carbonyl-nitrogen bonds of an M²-containing moiety is observable.

"Stretcher unit" as the term is used herein refers to an organic moiety in a secondary linker that physically separates the targeting moiety from other intervening components of a Linker unit that are distal to the stretcher unit, such as a cleavable unit and/or a spacer unit. Stretcher units are usually required when a L_{b} moiety of an LDC does not provide sufficient steric relief to allow for processing of the Linker unit at W so that a tertiary amine-containing drug incorporated into D⁺ can be released. A Stretcher unit (A) can comprise one or multiple stretcher groups or subunits as described herein. Prior to incorporation into an LDC, A has functional groups capable of covalently binding a L_{b}' to a Cleavable unit (W) in certain linker constructs (as when A, W and Y are in a linear arrangement) or to a Spacer unit (Y) in other linker constructs (as when W is orthogonal to A-Y). In some aspects of the invention a Stretcher unit is capable of attaching to more than one Cleavable unit, Spacer unit, and/or Drug unit as for example when A represents a dendrimer or other multifunctional branched structure. In some aspects of the invention, the secondary linker is attached to a L_{b} or L_{b}' moiety through one of its stretcher subunits while another or its subunits is covalently bonded to the remainder of the secondary linker.

A Stretcher unit (A), when present in an LDC, is an organic moiety covalently attached to a ligand covalent binding moiety or a ligand covalent binding moiety precursor and to another secondary linker unit and may comprise or consist of two, three or more subunits. Typically, A is one distinct unit or has two distinct subunits referred to as A₁ and Aₒ. In those aspects in which A has two subunits the subscript a is 2 in Drug Linker or Ligand Drug Conjugates that are comprised of moieties such as -Aₐ-W_{w}-Y_{y}- or Aₐ-Y_{y}(W_{w})- and Aₐ as -A₁-A₂- is sometimes referred to as -A₁-A_{O}-. The number of contiguous atoms in a stretcher unit is chosen to relieve steric interference from the ligand moiety not addressed by the ligand covalent binding moiety that would impede release of free tertiary amine-containing drug resulting from processing within or at the site of abnormal cells of W of an LDC comprised of that covalent ligand binding moiety, stretcher unit and cleavable unit. Typically, the stretcher unit or a subunit thereof has one to six contiguous carbon atoms that are between the ligand covalent binding moiety or the ligand covalent binding moiety precursor and the functional group that covalently attaches the stretcher unit A to a cleavable (W) or spacer (Y) unit of the secondary linker or to another subunit of A. In some aspects that functional group may also serve as a hydrolysis enhancing (HE) unit.

"Branching Unit" as used herein refers to an tri-functional organic moiety that is an optional subunit of a Stretcher unit (A). Sometimes A₁ or A_{O}, which are subunits of A, acts as a Branching Unit and other times the Branching Unit is an additional subunit of A. When A₁ Ao and B are present as subunits of A, B may be the proximal subunit to a ligand covalent binding moiety (L_{b}) or its precursor L_{b}' (i.e., is before -A₁-A_{O}-) or the distal subunit of A (i.e., is after -A₁-A_{O}-.). The Branching unit is trifunctional in order to be incorporated into a secondary linker unit (L_{O}) or to connect L_{O} to a primary linker unit (L_{R}) and for additionally connecting to a Solubilizing Unit (S) when that unit is present as a component of a Linker Unit (LU). In those aspects in which A has two subunits in which one subunit is a Branching Unit the subscript a is 2 in Drug Linker or Ligand Drug Conjugates that are comprised of moieties such as -Aₐ-W_{w}-Y_{y}- or Aₐ-Y_{y}(W_{w})- and in these instances Aₐ as -A₁-A₂- is sometimes referred to as -A-B- or -B-A-. In those aspects in which A three two subunits in which one subunit is a Branching Unit the subscript a is 3 in Drug Linker or Ligand Drug Conjugates that are comprised of moieties such as -Aₐ-W_{w}-Y_{y}- or Aₐ-Y_{y}(W_{w})- and in these instances Aₐ as -A₁-A₂-A₃- is sometimes referred to as -A₁-B-A_{O} or -B-A₁-A_{O}- or -A₁-A_{O}-B- depending on the relative ordering of A₁, Ao and B in the Linker unit of the Drug Linker or Ligand Drug Conjugate.

In some aspects a natural or un-natural amino acid or other amine-containing acid compound having a functionalized side chain serves as a Branching unit. Typically, when a Branching Unit (B) and a Solubilizing Unit (S) are components of a Linker unit the functionalized side chain of B connects S to the remainder of LU. Exemplary structures for B are those described herein for other subunits of A (e.g., A₁, A_{O}) provided the structure has the required aforementioned tri-functionality. In some aspects B is a lysine, glutamic acid or aspartic acid moiety in the L- or D-configuration in which the epsilon-amino, gamma-carboxylic acid or beta-carboxylic acid functional group, respectively, connects the Solubilizing unit to the remainder of LU.

"Solubilizing Unit" as used herein refers to an organic moiety that is hydrophilic. A Solubilizing unit (S), when present, is covalently attached to a Branching Unit and serves to counteract, at least in part, the hydrophobicity of a Drug Unit when the hydrophobicity of a Drug unit limits the drug average loading of its Ligand Drug Conjugate (LDC). That limitation in conjugation of hydrophobic drugs typically results from hydrophobic-mediated aggregation of individual ADC compounds in an ADC composition. Incorporation of an appropriate Solubilizing agent results in a detectable reduction of aggregate species as measured by size exclusion chromatography (SEC) in comparison to an appropriate control ADC lacking the Solubilizing Unit.

"Cleavable unit" as defined provides for a reactive site in which reactivity to that site is greater within or surrounding a hyper-proliferating cell or a hyper-stimulated immune cell (i.e., an abnormal cell) in comparison to a normal cell such that action upon that site results in preferential exposure to the abnormal cell of an tertiary amine-containing drug. That exposure results from eventual release of free drug from an LDC having that cleavable unit. In some aspects of the invention, W is comprised of a reactive site cleavable by an enzyme (i.e., W is comprised of an enzyme substrate) whose activity or abundance is greater within or surrounding the hyper-proliferating, immune-stimulating or other abnormal or unwanted cell. In other aspects, W is comprised of a reactive site cleavable by other mechanisms (i.e., non-enzymatic) more likely operable in the environment within or surrounding abnormal cells of the target site in comparison to the environment of normal cells in which abnormal cells are typically not present. In still other aspects of the invention, the reactive site is more likely operated upon subsequent to cellular internalization of the LDC into an abnormal cell. That internalization more likely occurs in those cells in comparison to normal cells due to greater presentation of the targeted moiety that is recognized by the targeting moiety of the LDC on the cellular membrane of the abnormal or unwanted cells. Therefore, the target cells will more likely be exposed intracellularly to active drug moiety liberated from its LDC. The Cleavable unit can comprise one or multiple sites susceptible to cleavage under those conditions of the target site, but typically has only one such site.

In some aspects of the invention, the cleavable unit is a substrate for a regulatory protease, hydrolase or glycosidase located intracellularly in the targeted cells (i.e., the reactive site of W is a peptide bond or glycoside bond cleavable by the regulatory protease, hydrolase or glycosidase) wherein is the peptide or glycoside bond is capable of selective cleavage by the regulatory protease, hydrolase or glycosidase in comparison to serum proteases, hydrolases, or glycosidases, wherein the regulatory protease, hydrolase or glycosidase may or may not be more specific to the targeted abnormal or other unwanted cells in comparison to normal cells, or is capable of selective cleavage by a protease, hydrolase or glycosidase excreted in greater amounts by the targeted abnormal or other unwanted cells in comparison to normal cells. Alternatively, W provides for a functional group that when incorporated into an LDC is susceptible to the acidic environment of lysozymes when the LDC is preferentially internalized into an abnormal cell, or to the greater reductive environment in or around these cells in comparison to the environment of normal cells where abnormal cells usually are not present such that release of free tertiary amine-containing drug preferentially exposes the abnormal cells to that drug in comparison to the normal cells.

The Cleavable unit (W), prior to its incorporation into an LDC, is capable of linking to the Spacer unit (Y). After incorporation into an LDC, W provides for a cleavable bond (i.e., a reactive site) that upon action by an enzyme present within a hyper-proliferating cell or hyper-activated immune cells or characteristic of the immediate environment of these abnormal or unwanted cells, or upon non-enzymatic action due to conditions more likely experienced by hyper-proliferating cells in comparison to normal cells, releases free tertiary amine-containing drug. Alternatively, W provides for a cleavable bond that is more likely acted upon intracellularly in a hyper-proliferating cell or hyper-activated immune cell due to preferential entry into such cells in comparison to normal cells. Typically, W in an ADC is covalently attached to a spacer unit (Y) that is comprised or consists of a self-immolating moiety such that enzymatic action on W triggers self-destruction of that moiety within Y-D⁺ to release free D.

Functional groups that provide for cleavable bonds include, by way of example and not limitation, (a) sulfhydryl groups that form a disulfide bond, which are susceptible to the greater reductive conditions of abnormal cells in comparison to normal cells or excess glutathione produced under hypoxic conditions experienced by such cells, (b) aldehyde, ketone, or hydrazine groups that form a Schiff base or hydrazone functional groups, which are susceptible to the acidic conditions of lysozymes upon selective internalization of an LDC having a linker unit with that cleavable bond into an abnormal cell in comparison to internalization into normal cells, (c) carboxylic or amino groups that form an amide bond, as in peptide bonds, that are susceptible to enzymatic cleavage by proteases produced or excreted preferentially by abnormal cells in comparison to normal cells or by a regulatory protease within a targeted cell (d) amino or hydroxyl groups that form certain urea or carbamate groups or carboxylic or hydroxy groups that form ester or carbonate groups that are susceptible to enzymatic cleavage by hydrolases or esterases that are produced or excreted preferentially by abnormal cells in comparison to normal cells.

Still other functional groups that provide for cleavable bonds are found in sugars or carbohydrates having a glycosidic linkage that are substrates for glycosides which sometimes may be produced preferentially by abnormal cells in comparison to normal cells. Alternatively, the protease, hydrolase or glycosidase enzyme required for processing of the Linker unit to release an active tertiary amine-containing drug need not be produced preferentially by abnormal cells in comparison to normal cells provided the processing enzyme is not excreted by normal cells to an entent that would cause undesired side effects from premature release of free drug. In other instances the required protease, hydrolase or glycosidase enzyme may be excreted but to avoid undesired premature release of drug, it is prefered that the processing enzyme be excreted in the vicinity of abnormal cells and remain localized to that enviroment, whether produced by abnormal cells or nearby normal cells in response to the abnormal enviroment caused by the abnormal cells. In that respect W is selected to be preferentially acted upon by a protease, hydrolase or glycosidase in or within the enviroment of abnormal cells in contrast to freely circulating enzymes. In those instances a LDC is less likely to release the tertiary amine-containing drug in the vicinity of normal cells nor would it be internalized into normal cells that do produce but not excrete the selected enzyme since such cells are less likely to display a targeted moiety required for entry by the LDC.

In some aspects, W is comprised of an amino acid or is comprised or consists of one or more sequences of amino acids that provide a substrate for a protease present within abnormal cells or localized to the environment of these abnormal cells. Thus, W may be comprised or consist of a dipeptide, tripeptide, tetrapeptide, pentapeptide, hexapeptide, heptapeptide, octapeptide, nonapeptide, decapeptide, undecapeptide or dodecapeptide moiety incorporated into a linker unit through an amide bond to SI of Y wherein that moiety is a recognition sequence for that protease. In other aspects, W is comprised or consists of a carbohydrate moiety attached to SI of Y by a glycosidic bond that is cleavable by a glycosidase preferentially produced by abnormal cells, or is found in such cells to which an LDC, which is comprised of that SI and carbohydrate moieties, has selective entry due to the presence of the targeted moiety on the abnormal cells.

"Spacer unit" as used herein is an organic moiety in a secondary linker (Lₒ) within a Linker unit that is covalently bonded to a cleavable unit (W) or stretcher unit (A) (or to L_{b} or L_{b}' when A is absent) depending on their configuration relative to each other. Typically, in one configuration, a quaternized drug unit (D⁺) and W are both covalently bonded to Y which in turn is bonded to A (or L_{b} or L_{b}' when A is absent) so that W is orthogonal to the remainder of Lₒ, whereas in another configuration W, Y, D are arranged in a linear configuration with D⁺ bonded to Y. In either arrangement, Y serves to separate the cleavage site W from D⁺ to avoid steric interactions from that unit that would interfere with cleavage of W whenever that cleavage is preformed through enzymatic action.

Typically, a spacer unit is comprised or consists of a self-immolating moiety (SI) as defined herein wherein that moiety is covalently bonded to a cleavage unit (W) so that *in vivo* processing of W activates SI to self-destruct thus releasing fee tertiary amine-containing drug. Oftentimes SI of Y is bonded to W through a amide (or anilide) functional group with Y also covalently bonded to the quaternary amine nitrogen of D⁺ through SI so that self-destruction of SI results in release of free tertiary amine-containing drug.

"Self-immolating moiety" as used herein refers to a bifunctional moiety within a spacer unit (Y) having an organic moiety that intervenes between the first and second functional group moieties and covalently incorporates these moieties into a normally stable tripartite molecule unless activated. On activation when the covalent bond to the first functional group moiety is cleaved, the second functional group moiety spontaneously separates from the tripartite molecule by self-destruction of the remainder of the SI moiety. That self-destruction on activation releases free tertiary amine-containing drug (D). In some aspects that self-destruction occurs after cellular internalization of an LDC comprising D⁺ and a Linker unit having a self-immolating (SI) in Y of the Linker unit. The intervening organic moiety in between the functional group moieties of a self-immolating (SI) moiety is sometimes an arylene or heteroarylene moiety capable of undergoing fragmentation to form a quinone methide or related structure by 1,4 or 1,6-elimination with concomitant release of free tertiary amine-containing drug. Such SI moieties are exemplified by optionally substituted p-aminobenzyl alcohol (PAB) moieties, ortho or para-aminobenzylacetals, or aromatic compounds that are electronically similar to the PAB group (i.e., PAB-type) such as 2-aminoimidazol-5-methanol derivatives (*see, e.g.,* Hay et al., 1999, Bioorg. Med. Chem. Lett. 9:2237) and other heteroaryls as described herein.

Typically, for one of the functional group moieties in a SI moiety, an aromatic carbon of an arylene or heteroarylene group of a PAB or PAB-type SI moiety incorporated into a Linker unit is substituted with an electron-donating (EDG) heteroatom attached to the cleavage site of W through a functional group comprising the heteroatom wherein that heteroatom is functionalized so that its electron-donating capacity is attenuated (i.e., the EDG is masked by incorporation of SI of Y into a Linker unit). The other substituent that provides the second functional group is a benzylic carbon having a quaternary amine substituent, wherein the quaternary amine, which comprises the tertiary amine-containing drug, bonded through the benzylic carbon attached to another aromatic carbon atom of the central arylene or heteroarylene group, wherein the aromatic carbon bearing the attenuated electron-donating heteroatom is adjacent to (i.e., 1,2-relationship), or two additional positions removed (i.e., 1,4-relationship) from that benzylic carbon atom. The EDG is chosen so that processing of the cleavage site of W restores the electron-donating capacity of the masked EDG thus triggering a 1,4- or 1,6-elimination to expel the tertiary amine containing-drug from the benzylic quaternary amine substituent.

Exemplary PAB or PAB-related SI moieties when present in a secondary linker-D+ moiety of structure (1) having an arylene or heteroarylene with the requisite substitution 1,2 or 1,4 substitution patterns that allow for 1,4- or 1,6-fragmentation to release D from a quaternized drug moiety are represented by 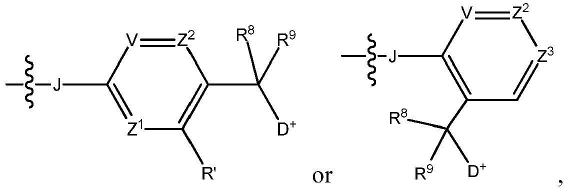 wherein D⁺ is attached in -C(R⁸)(R⁹)-D⁺ through a quaternary amine nitrogen covalently bonded to the aforementioned benzylic carbon and J is the masked EDG, wherein J, which is bonded to W through the attenuating functional group comprising J, is -O-, -N(R³³)-, or -S-, and R⁸, R⁹, R³³, R', V, Z¹, Z², Z³ are defined in embodiments of PAB and PAB-type SI units. Those variables are selected so that reactivity of J when released from processing of W at the targeted site is balanced with the reactivity of the tertiary amine eliminated from SI and the stability of the quinone-methide type intermediate resulting from that elimination.

In some aspects for a secondary linker-D⁺ moiety of structure (1), the PAB or PAB-type SI moiety bound to D⁺ has the structure of

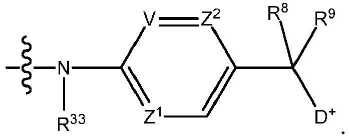

Other structures and their variable group definitions incorporating an SI moiety in a secondary linker-D⁺ moiety of structure (1) are provided by the embodiments.

In some aspects for a secondary linker of structure (2), a PAB or PAB-type SI moiety when present in a secondary linker of structure (2) has the structure of 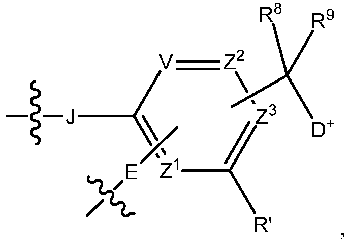 wherein the wavy line to J indicates covalent stable covalent bonding (i.e., not processed at the targeted site) to Ligand-L_{b}- or L_{b}'- when a is 0 or through A or subunit thereof when a is 1, wherein J is -O-, -N(R³³)-, or -S- bonded directly to L_{b}, L_{b}', A or a subunit of A or through a functional group comprising J, and wherein R', R⁸, R⁹, V, Z¹, Z² and Z³ are as defined in Formula 1 and E, independently selected from J, is an electron donating group such as -O-, -N(R³³)-, or -S-, wherein the electron donating ability of E is attenuated by it bonding to W', wherein W'-E provides for a cleavage site for a glycosidase, and E and the benzylic carbon of the -C(R⁸)(R⁹)-D⁺ moiety are bonded to the arylene or heteroarylene group at positions defined by V, Z¹, Z² or Z³, such that E and the -C(R⁸)(R⁹)-D⁺ moiety are in a 1,2 or 1,4 relationship so as to permit the 1,4- or 1,6-fragmention that results in release of tertiary amine-containing drug.

In some aspects for a secondary linker-D⁺ moiety of structure (2), the PAB or PAB-type SI moiety bound to D⁺ has the structure of

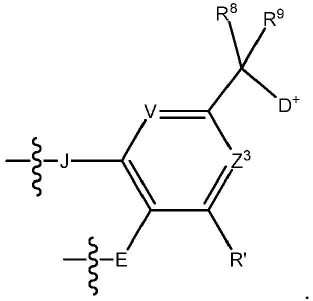

Other structures and their variable group definitions incorporating an SI moiety in a secondary linker-D⁺ moiety of structure (2) are provided by the embodiments.

The arylene or heteroarylene of an SI moiety may be further substituted to affect the kinetics of the 1,2- or 1,4-elimination in order to modulate the release of D or to improve the physiochemical properties of the Ligand Drug Conjugate (e.g., reduce hydrophobicity) into which it is incorporated.

Exemplary and non-limiting examples of SI structures that are modified to accommodate benzylic quaternary amine substituents are provided by Blencowe et al. "Self-immolative linkers in polymeric delivery systems" Polym. Chem. (2011) 2: 773-790; Greenwald et al. "Drug delivery systems employing 1,4- or 1,6-elimination: poly(ethylene glycol) prodrugs of amine-containing compounds" J. Med. Chem. (1999) 42: 3657-3667; and in US Pat. Nos. 7,091,186; 7,754,681; 7,553,816; and 7,989,434.

"Cytotoxic drug" as used herein refers to compound or a metabolite derived from an LDC that exerts an anti-survival effect on hyper-proliferating cells, hyper-activated immune cells or other abnormal or unwanted cells. In some aspects the cytotoxic drug acts directly upon those cells or indirectly by acting upon the abnormal vasculature that supports the survival and/or growth of the hyper-proliferating or other abnormal or unwanted cells, or the cytotoxic drug acts within sites of infiltrating hyper-activated immune cells. Typically, the abnormal or unwanted cells acted upon by the cytotoxic drug are mammalian cells, more typically human cells. Cytotoxic activity of a cytotoxic drug may be expressed as an IC₅₀ value, which is the effective concentration, typically molar amount per unit volume, at which half the cancer cells in an *in vitro* cell model system survive exposure to the cytotoxic agent. Thus, an IC₅₀ value is model-dependent. Typically, a cytotoxic agent incorporated into an LDC will have an IC₅₀ value in an *in vitro* cell model comprised of hyper-proliferating cells of between 100 nM to 0.1 pM or more typically about 10 nM to 1 pM. A highly toxic cytotoxic drug typically has an IC₅₀ value in such models of about 100 pM or lower. Although multiple drug resistant inhibitors that reverse resistance to cytotoxic drugs are not cytotoxic in their own right they are sometimes included as cytotoxic drugs.

"Cytostatic drug" as used herein refers to compound or a metabolite derived from an LDC that exerts an inhibitory effect on the growth and proliferation of hyper-proliferating cells, hyper-activated immune cells or other abnormal or unwanted cells. In some aspects the cytostatic drug acts directly upon those cells or indirectly by acting upon the abnormal vasculature that supports the survival and/or growth of the hyper-proliferating or other abnormal or unwanted cells, or the cytotoxic drug acts within sites of infiltrating hyper-activated immune cells. Typically, the abnormal or unwanted cells acted upon by the cytotoxic drug are mammalian cells, more typically human cells. Although multiple drug resistant inhibitors that reverse resistance to cytostatic drugs are not cytostatic in their own right they are sometimes included as cytostatic drugs.

"Hematological malignancy" as the term is used herein refers to a blood cell tumor that originates from cells of lymphoid or myeloid origin and is synonymous with the term "liquid tumor". Hematological malignancies may be categorized as indolent, moderately aggressive or highly aggressive.

"Lymphoma" as used herein is hematological malignancy that usually develops from hyper-proliferating cells of lymphoid origin. Lymphomas are sometimes classified into two major types: Hodgkin lymphoma (HL) and non-Hodgkin lymphoma (NHL). Lymphomas may also be classified according to the normal cell type that most resemble the cancer cells in accordance with phenotypic, molecular or cytogenic markers. Lymphoma subtypes under that classification include without limitation mature B-cell neoplasms, mature T cell and natural killer (NK) cell neoplasms, Hodgkin lymphoma and immunodeficiency-associated lympho-proliferative disorders. Lymphoma subtypes include precursor T-cell lymphoblastic lymphoma (sometimes referred to as a lymphoblastic leukemia since the T-cell lymphoblasts are produced in the bone marrow), follicular lymphoma, diffuse large B cell lymphoma, mantle cell lymphoma, B-cell chronic lymphocytic lymphoma (sometimes referred to as a leukemia due to peripheral blood involvement), MALT lymphoma, Burkitt's lymphoma, mycosis fungoides and its more aggressive variant Sézary's disease, peripheral T-cell lymphomas not otherwise specified, nodular sclerosis of Hodgkin lymphoma, and mixed-cellularity subtype of Hodgkin lymphoma.

"Leukemia" as the term is used herein is a hematological malignancy that usually develops from hyper-proliferating cells of myeloid origin, and include without limitation, acute lymphoblastic leukemia (ALL), acute myelogenous leukemia (AML), chronic lymphocytic leukemia (CLL), chronic myelogenous leukemia (CML) and acute monocyctic leukemia (AMoL). Other leukemias include hairy cell leukemia (HCL), T-cell lymphatic leukemia (T-PLL), large granular lymphocytic leukemia and adult T-cell leukemia.

"Quaternized drug unit" as used herein may be any tertiary amine-containing compound (D) having a cytotoxic, cytostatic, immunosuppressive or anti-inflammatory property, typically against mammalian cells, that has been incorporated into an LDC as its corresponding quaternary amine salt (D⁺). The term "drug" does not imply a compound that is only approved by a regulatory agency for use in treating a subject having a hyperproliferation or hyper-immune stimulated disease or condition or would be suitable for any such purpose outside the context of an LDC. Thus, D⁺ also encompasses quaternized compounds that in unconjugated (i.e., in tertiary amine-containing "free" form, and thus not present in an LDC) would be unsuitable for administration to a subject. For example, a tertiary amine-containing drug (D) when incorporated into an LDC as D⁺ may be a cytotoxic compound inappropriate for treating a cancer in a subject due to intolerable systemic toxicity at the required therapeutic dose. In some aspects, a quaternized drug is obtained by condensing the tertiary amine nitrogen of a tertiary amine-containing drug with a secondary linker Lₒ having a suitable leaving group. In some aspects the tertiary amine-containing drug incorporated into D⁺ will be suitable for use in treating cancer. In other aspects the tertiary amine-containing drug incorporated into D⁺ will be suitable for use in treating cancer. In some aspects, a tertiary amine-containing drug incorporated into D⁺ of an LDC will be a cytotoxic or cytostatic compound. In other aspects, a tertiary amine-containing drug incorporated into D⁺ of an LDC will be an anti-inflammatory or immune-suppressive compound. In still other aspects the tertiary amine-containing drug incorporated into D⁺ of an LDC will be a multi-drug resistance inhibitor. Other tertiary amine-containing drugs that may be incorporated into D⁺ are additionally provided by the definition of tertiary amine-containing drug.

"Tertiary amine-containing drug" as used herein are those compounds that have cytotoxic, cytostatic or anti-inflammatory activities and are characterized by an aliphatic tertiary amine moiety (D) that is capable of release from its corresponding quaternary amine (D⁺) moiety of an LDC subsequent to binding of the LDC's targeting moiety to its cognate targeted moiety. Oftentimes the tertiary amine-containing compound is converted to its quaternized form when it is first incorporated into a L_{b} or L_{b}'-containing moiety. However, sometimes a secondary amine- or primary amine-containing precursor is incorporated into L_{b} or L_{b}'-containing moiety, which is then quaternized to form the D⁺ moiety from which the tertiary amine-containing drug is capable of being released. Therefore, structures such as L-L_{b}-Lₒ-D⁺ and L_{b}'-Lₒ-D⁺ imply no particular method in which D⁺ was formed and does not require that a reactant used in its formation be a tertiary-amine containing drug.

Classes of tertiary-amine containing drugs released from an LDC of the present invention include, for example but without limitation compounds having a tertiary amine functional group that are useful in the treatment of cancer or an autoimmune disease. Those compounds include microtubule disrupting agents, DNA minor groove binding agents, DNA replication inhibitors, DNA alkylating agents, chemotherapy sensitizers and topoisomerase inhibitors such as tubulysins, auristatins, phenazine dimers, certain multiple drug resistant (MDR) inhibitors and nicotinamide phosphoribosyl-transferase (NAMPT) inhibitors.

"Hyper-proliferating cells" as used herein refers to cells that are characterized by unwanted cellular proliferation or an abnormally high rate or persistent state of cell division that is unrelated or uncoordinated with that of the surrounding normal tissues. Typically, the hyper-proliferating cells are mammalian cells. In some aspects the hyper-proliferating cells are hyper-stimulated immune cells as defined herein whose persistent state of cell division occurs after the cessation of the stimulus that may have initially evoked the change in their cell division. In other aspects the hyper-proliferating cells are transformed normal cells or cancer cells and their uncontrolled and progressive state of cell proliferation may result in a tumor that is benign, potentially malignant (premalignant) or frankly malignant. Hyperproliferation conditions resulting from transformed normal cells or cancer cells include but are not limited to those characterized as a precancer, hyperplasia, dysplasia, adenoma, sarcoma, blastoma, carcinoma, lymphoma, leukemia or papilloma. Precancers are usually defined as lesions that exhibit histological changes which are associated with an increased risk of cancer development and sometimes have some, but not all, of the molecular and phenotypic properties that characterize the cancer. Hormone associated or hormone sensitive precancers include, prostatic intraepithelial neoplasia (PIN), particularly high-grade PIN (HGPIN), atypical small acinar proliferation (ASAP), cervical dysplasia and ductal carcinoma in situ. Hyperplasias generally refers to the proliferation of cells within an organ or tissue beyond that which is ordinarily seen that may result in the gross enlargement of an organ or in the formation of a benign tumor or growth. Hyperplasias include, but are not limited to endometrial hyperplasia (endometriosis), benign prostatic hyperplasia and ductal hyperplasia.

"Normal cells" as used herein refers to cells undergoing coordinated cell division related to maintenance of cellular integrity of normal tissue or replenishment of circulating lymphatic or blood cells that is required by regulated cellular turnover, or tissue repair necessitated by injury, or to a regulated immune or inflammatory response resulting from pathogen exposure or other cellular insult, where the provoked cell division or immune response terminates on completion of the necessary maintenance, replenishment or pathogen clearance. Normal cells include normally proliferating cells, normal quiescent cells and normally activated immune cells.

"Normal quiescent cells" are noncancerous cells in their resting Gₒ state and have not been stimulated by stress or a mitogen or are immune cells that are normally inactive or have not been activated by pro-inflammatory cytokine exposure.

"Hyper-stimulated immune cells" as the term is used herein refers to cells involved in innate or adaptive immunity characterized by an abnormally persistent proliferation or inappropriate state of stimulation that occurs after the cessation of the stimulus that may have initially evoked the change in proliferation or stimulation or that occurs in the absence of any external insult. Oftentimes, the persistent proliferation or inappropriate state of stimulation results in a chronic state of inflammation characteristic of a disease state or condition. In some instance the stimulus that may have initially evoked the change in proliferation or stimulation is not attributable to an external insult but is internally derived as in an autoimmune disease. In some aspects a hyper-stimulated immune cells is a pro-inflammatory immune cell that has been hyper-activated through chronic pro-inflammatory cytokine exposure.

In some aspects of the invention an LDC binds to an antigen preferentially displayed by pro-inflammatory immune cells that are abnormally proliferating or are inappropriately activated. Those immune cells include classically activated macrophages or Type 1 T helper (Th1) cells, which produce interferon-gamma (INF-γ), interleukin-2 (IL-2), interleukin-10 (IL-10), and tumor necrosis factor-beta (TNF-β), which are cytokines that are involved in macrophage and CD8⁺ T cell activation.

"Glycosidase" as used herein refers to a protein capable of enzymatic cleavage of a glycosidic bond. Typically, the glycosidic bond to be cleaved is present in a Cleavable unit (W) of an LDC. Sometimes the glycosidase acting upon an LDC is present intracellularly in hyper-proliferating cells, hyper-activated immune cells or other abnormal or unwanted cells to which the LDC has preferential access in comparison to normal cells, which is attributable to the targeting capability of the ligand-binding component (i.e., the Ligand Unit). Sometimes the glycoside is more specific to the abnormal or unwanted cells or is preferentially excreted by abnormal or unwanted cells in comparison to normal cells or is present in greater amount in the vicinity of abnormal or unwanted in comparison to serum amounts. Oftentimes the glycosidic bond in W acted upon by a glycosidase attaches the anomeric carbon of a carbohydrate moiety (Su) to a phenolic oxygen of a self-immolating (SI) moiety that comprises a stretcher unit (Y) such that glycosidic cleavage of that bond triggers 1,4- or 1,6-elimination of an tertiary amine-containing drug from the quaternary amine moiety bonded to the benzylic position of SI.

In Drug Linker compounds or Ligand Drug Conjugates comprised of moieties such as -Aa-Y_{y}(W_{w})-D⁺ in which the subscripts w and y are each one the -Y(W)- is typically a Su-O'-SI moiety as defined herein with A, W and D attached to SI in a manner that permits self-immolative release of free tertiary amine containing drug upon action by a glycosidase. Such -Y(W)- moieties are sometimes referred to as a glucuronide unit in which Su is not limited to glucuronic acid.

Typically, a Su-O'-SI moiety (where -O'- represents the oxygen of the glycosidic bond and Su is a carbohydrate moiety) is represented by the structure described for self-emolliating moieties where E bonded to the aryl moiety of SI is oxygen with that heteroatom substituted with a carbohydrate moiety through that moiety's anomeric carbon atom. More typically Su-O'-SI has the structure of: 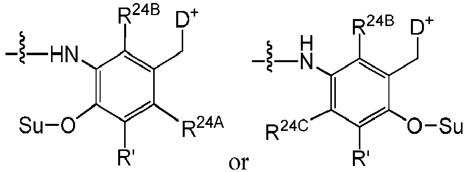 wherein R^{24A}, R^{24B} and R^{24C}, is as defined for R²⁴ in the summary of the invention and is selected so that the electron donating ability of the phenolic -OH released from the glycosidic bond, the sensitivity to selective cleavage by a desired glycosidase of the glycosidic bond to the carbohydrate moiety Su, and the stability of the quinone methide intermediate upon fragmentation is balanced with the leaving ability of the tertiary amine so that efficient release of D from D⁺ through 1,4- or 1,6-elimination occurs. Those Su-O'-SI structures are representative glucuronide units. When the glycosidic bond is to a glucuronic acid the glycosidase capable of enzymatic cleavage of that glycosidic bond is a glucuronidase. "Carbohydrate moiety" as used herein refers to a monosaccharide having the empirical formula of Cₘ(H₂O)ₙ, wherein n is equal to m, containing an aldehyde moiety in its hemiacetal form or a derivative thereof in which a CH₂OH moiety within that formula has been oxidized to a carboxylic acid (e.g., glucuronic acid from oxidation of the CH₂OH group in glucose). Typically, a carbohydrate moiety (Su) is a cyclic hexose, such as a pyranose, or a cyclic pentose, such as a furanose. Usually, the pyranose is a glucuronide or hexose in the β-D conformation. In some instances, the pyranose is a β-D-glucuronide moiety (*i.e.,* β-D-glucuronic acid linked to the self-immolative moiety -SI- via a glycosidic bond that is cleavable by β-glucuronidase). Oftentimes, the carbohydrate moiety is unsubstituted (*e*.*g*., is a naturally occurring cyclic hexose or cyclic pentose). Other times, the carbohydrate moiety can be a substituted β-D-glucuronide (*i.e.,* glucuronic acid substituted with one or more group, such hydrogen, hydroxyl, halogen, sulfur, nitrogen or lower alkyl.

"Amino acid" as used herein is an organic moiety containing an carboxylic acid and a aliphatic amine functional group, typically a primary or secondary aminne functional group capable of forming an amide bond with another organic moiety through standard peptide bond forming reactions well-known in the art of peptide synthesis. Amino acid includes natural, un-natural, and non-classical amino acids as defined herein. A hydrophobic amino acid contaims a hydrophobic substituent, typically a C₁-C₆ alkyl group attached to a carbon atom within the chain of atoms intervening between the carboylic acid and amine funtional groups and is typically on the carbon atom alpha to the amine or carboxylic acid functional group. For a hydrophobic natural amino acid the hydrophobic substiuent on the alpha carbon is that of valine, alanine, leucineor isoleucine.

"Natural amino acid" refers to the naturally occurring amino acids arginine, glutamine, phenylalanine, tyrosine, tryptophan, lysine, glycine, alanine, histidine, serine, proline, glutamic acid, aspartic acid, threonine, cysteine, methionine, leucine, asparagine, isoleucine, and valine, in the L or D configuration unless otherwise indicated by context.

"Un-natural amino acids" as used herein are amine-containing acid compounds that have the basic structure of natural amino acids (i.e., are alpha-amino-containing acids, but have R groups attached to the alpha carbon that are not present in natural amino acids.

"Non-classical amino acids" as used herein are amine-containing acid compounds that do not have its amine substituent bonded to the carbon alpha to the carboxylic acid (i.e., is not an alpha-amino acid). Non-classical amino acids include β-amino acids in which a methylene is inserted between the craboxylic acid and amino functional groups in a natural amino acid or an un-natural amino acid.

"Peptide" as used herein refers to a polymer of two or more amino acids wherein carboxylic acid group of one amino acid forms an amide bond with the alpha-amino group of the next amino acid in the peptide sequence. Methods for preparing amide bonds in polypeptides are additionally provided in the definition of amide.

Peptides may be comprised of naturally occurring amino acids in the L- or D-configuration or unnatural or non-classical amino acids, which include, but are not limited to, ornithine, citrulline, diaminobutyric acid, norleucine, pyrylalanine, thienylalanine, naphthylalanine and phenylglycine. Other examples of non-naturally occurring and non-classical amino acids are alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-F-phenylalanine*, L-allyl-glycine*, beta-alanine*, L-alpha-amino butyric acid*, L-gamma-amino butyric acid*, L-alpha-amino isobutyric acid*, L-epsilon-amino caproic acid#, 7-amino heptanoic acid*, L-methionine sulfone*, L-norleucine*, L-norvaline*, p-nitro- L-phenylalanine*, L-hydroxyproline#, L-thioproline*, methyl derivatives of phenylalanine (Phe) such as 4-methyl-Phe*, pentamethyl-Phe*, L-Phe (4-amino)#, L-Tyr (methyl)*, L-Phe (4-isopropyl)*, L-Tic (1,2,3,4-tetrahydroisoquinoline-3-carboxyl acid)*, L-diaminopropionic acid, L-Phe (4-benzyl)*, 2,4-diaminobutyric acid, 4-aminobutyric acid (gamma-Abu), 2-amino butyric acid (alpha-Abu), 6-amino hexanoic acid (epsilon-Ahx), 2-amino isobutyric acid (Aib), 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, fluoroamino acids, beta-methyl amino acids, Calpha-methyl amino acids, N-methyl amino acids, naphthyl alanine, and the like. The notation* indicates a derivative having hydrophobic characteristics, # indicates a derivative having hydrophilic characteristics, and #* indicates a derivative having amphipathic characteristics.

Variant amino acid sequences in a peptide may sometimes include suitable spacer groups inserted between any two amino acid residues of the sequence including alkyl groups such as methyl, ethyl or propyl groups in addition to amino acid spacers such as glycine or β-alanine residues. Also, a peptide may comprise or consist of peptoids. The term "peptoids" refers to variant amino acid structures where the alpha-carbon substituent group is on the backbone nitrogen atom rather than the alpha-carbon. Processes for preparing peptides in the peptoid form are known in the art (see, e.g., Simon et al., Proc. Nat'l. Acad. Sci. (USA) (1992) 89(20): 9367-9371; and Norwell, Trends Biotechnol. 13(4): 132-134 (1995)).

"Protease" as defined herein refers to a protein capable of enzymatic cleavage of a carbonyl-nitrogen bond such as an amide bond typically found in a peptide. Proteases are classified into major six classes: serine proteases, threonine proteases, cysteine proteases, glutamic acid proteases, aspartic acid proteases and metalloproteases so named for the catalytic residue in the active site that is primarily responsible for cleaving the carbonyl-nitrogen bond of its substrate. Proteases are characterized by various specificities, which are dependent of identities of the residues at the N-terminal and/or C-terminal side of the carbonyl-nitrogen bond and various distributions.

When W is comprised of amide or other carbonyl-nitrogen containing functional group cleavable by a protease that cleavage site is oftentimes limited to those recognized by proteases that are found in hyper-proliferating cells or hyper-stimulated immune cells or within cells particular to the environment in which hyper-proliferating cells or hyper-stimulated immune cells are present. In those instances, the protease is not necessarily required to be preferentially present or found in greater abundance in the cells targeted by the LDC since an LDC will have poorer access to those cells that do not preferential have the targeting moiety. Other times, the protease is preferentially excreted by abnormal cells or by cells in the environment in which those abnormal cells are found in comparison to normal cells or the typical environments in which those normal cells are found in the absence of abnormal cells. Thus, in those instances where the protease is excreted, the protease is necessarily required to be preferentially present or found in greater abundance in the vicinity of cells targeted by the LDC in comparison to that of normal cells.

When incorporated into an LDC, a peptide that comprises W will present a recognition sequence to a protease that cleaves a carbonyl-nitrogen bond in W resulting in fragmentation of the Linker unit to cause release of an tertiary amine-containing drug from D⁺. Sometimes, the recognition sequence is selectively recognized by an intracellular protease present in abnormal cells to which the LDC has preferred access in comparison to normal cells due to targeting of the abnormal cells, or is preferentially produced by abnormal cells in comparison to normal cells, for the purpose of appropriately delivering the drug to the desired site of action. Usually the peptide is resistant to circulating proteases in order to minimize premature expulsion of the tertiary amine-containing drug and thus minimize unwanted systemic exposure to the drug. Typically, the peptide will have one or more unnatural or non-classical amino acids in its sequence order to have that resistance. Oftentimes, the amide bond that is specifically cleaved by a protease produced by an abnormal cell is an anilide wherein the nitrogen of that anilide is a nascent electron-donating heteroatom (i.e., J) of an SI moiety having the previously defined structures. Thus, protease action on such a peptide sequence in W results in drug release from the linker fragment by 1,4- or 1,6-elimination through the arylene moiety of SI.

Regulatory proteases are typically located intracellularly and are required for the regulation of cellular activities that sometimes becomes aberrant or dysregulated in abnormal or other unwanted cells and are further. In some instances, when W is directed to a protease having preferential distribution intracellularly, that protease is a regulatory protease, which is involved in cellular maintenance or proliferation. In some instances, those proteases include cathepsins. Cathepsins include the serine proteases, Cathepsin A, Cathepsin G, aspartic acid proteases Cathepsin D, Cathepsin E and the cysteine proteases, Cathepsin B, Cathepsin C, Cathepsin F, Cathepsin H, Cathepsin K, Cathepsin L1, Cathepsin L2, Cathepsin O, Cathepsin S, Cathepsin W and Cathepsin Z.

In other instances, when W is directed to a protease that is preferentially distributed extracellularly in the vicinity of hyper-proliferating or hyper-stimulated immune cells due to preferential excretion by such cells or by neighboring cells whose excretion is peculiar to the environment of hyper-proliferating or hyper-stimulated immune cells, that protease is usually a metalloprotease. Typically, those proteases are involved in tissue remodeling, which aids in the invasiveness of hyper-proliferating cells or undesired accumulation of hyper-activated immune cells that results in further recruit of such cells.

"Tubulin disrupting agent" as use herein refers to a cytotoxic or cytostatic compound that binds to tubulin or a subunit thereof and as a result inhibits tubulin elogation or tubulin polymerization to negatively affect tubulin dynamics, particularly in hyperproliferting or hyper-stimulated immune cells. Examples of anti-tubulin agents (i.e., tubulin disrupting agents) include, but are not limited to, taxanes, vinca alkaloids, maytansine and maytansinoids, dolastatins and auristatins. A tertiary amine-containing tubulin disrubting agent is a tubulin disrupting agent as defined herein that conatins or can be modified to contain a teriary amine functional group.

"Dolastatin drug" as the term is used herein are pentapeptides isolated from marine sources having cytotoxic activity as a tubulin disrupting agents. Dolastatin 10 and Dolastatin 15 exemplify tertiary amine-containing dolastatins and have the following structures:

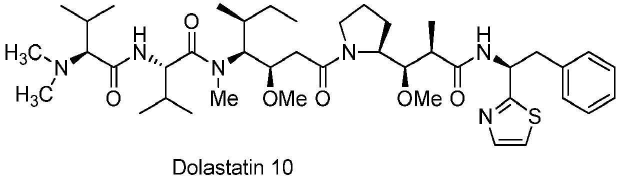

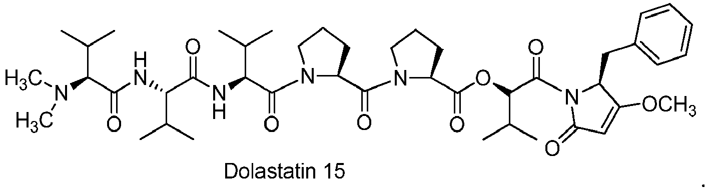

Some exemplary dolastatins are related to dolastatin 10 wherein the phenyl and thiazole substituents are replaced with independently selected aryl or heteroaryl moieties. Other exemplary dolastatins are related to dolastatin 15 wherein the C-terminal ester moiety is replaced by an amide wherein the amide nitrogen is substituted with an arylalkyl or hetereoarylalkyl moiety. Their structures and structures of other exemplary tertiary amine-containing dolastatins and manner of their incorporation into an LDC are provided in the embodiments of quaternized drug units.

"Auristatin drug" as the term is used herein is a peptidyl-based tubulin disrupting agent related to dolastatin 10. Some tertiary amine-containing auristatins have the structure of D_{E} or D_{F}: 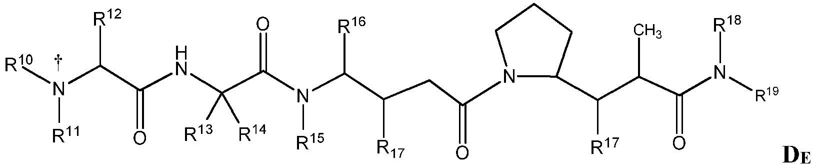 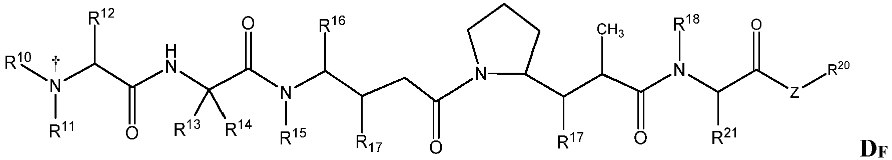 wherein Z is -O-, -S-, or -N(R¹⁹)-, and wherein R¹⁰-R²¹ are as defined in embodiments for quaternized tertiary amine-containing auristatin drugs. When incorporated into an LDC or precursor thereof the indicated (†) nitrogen of the tertiary amine moiety is quaternized by covalent binding to Lₒ or to Lₒ of an L_{b} or L_{b}'-containing moiety that is comprised of Lₒ. Typically, that quaternized moiety of D⁺ results from covalent of the tertiary amine moiety to the benzylic carbon of a PAB or PAB-type moiety of which a self-immoative Spacer Unit (Y) unit in Lₒ comprises or consists. Structures of other exemplary tertiary amine-containing auristatins and manner of their incorporation of such drugs into an LDC are provided in the embodiments of auristatin-based quaternized drug units and include Auristatin E and Auriststin F.

"Tubulysin drug" as used is a peptide-based tubulin disrupting agents having cytotoxic activity, and is comprised of one natural or un-natural amino acid component and three other un-natural amino acid components wherein one of those components is characterized by a central 5-membered or 6-membered heteroarylene moiety and another provides for a tertiary amine for incorporation into a quaternized drug unit.

Some tertiary amine-containing tubulysins have the structure of D_{G} or D_{H}: 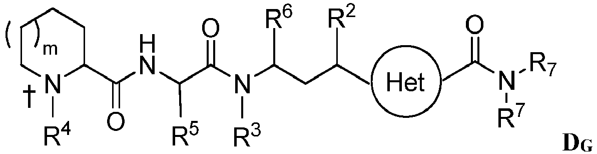 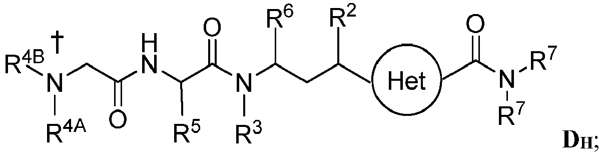 other tertiary amine-containing tubulysins have the structure of D_{G-1} or D_{H-1}: 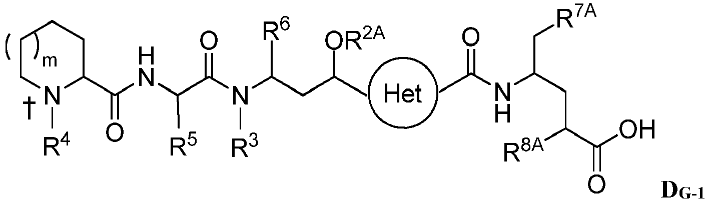 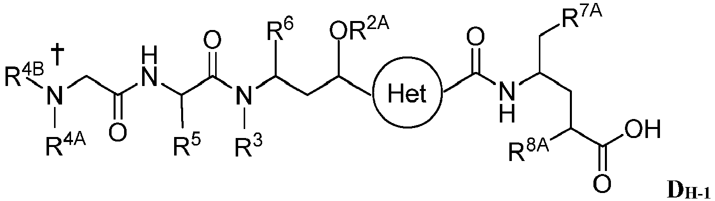 wherein the circle represents an 5-membered or 6-membered nitrogen-heteroaryl, wherein the indicated required substituents to that heteroaryl are in a 1,3- or meta-relationship to each other with optional substitution at the remaining positions; R⁴, R^{4A}, R^{4B}, R^{8A} are each an optionally substituted alkyl, independently selected; R⁷ is an optionally substituted arylalkyl or an optionally substituted heteroarylalkyl; R^{7A} is an optionally substituted aryl or an optionally heteroaryl; R² is a divalent O-linked substituent (i.e., the double bond is present or the carbon having that variable group is disubstituted by a single instance of R²), or the double bond is absent (i.e., R² is single-bonded to the carbon having that variable group) or R² is hydrogen, optionally substituted alkyl, or an monovalent O-linked substituent; m is 0 or 1; and R^{2A}, R³, R⁵ and R⁶ are as defined in embodiments for tubulysin-based quaternized tertiary amine-containing drugs.

Naturally occurring tubulysins have the structure of 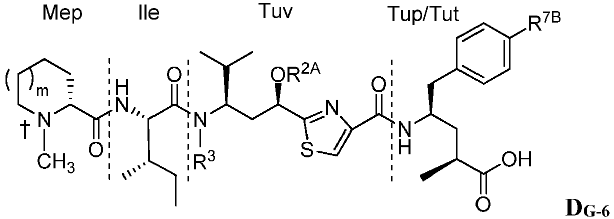 and are conveniently divided into four amino acid subunits, as indicated by the dashed vertical lines, named N-methyl-pipecolinic acid (Mep), isoleucine (Ile), tubuvaline (Tuv), and either tubuphenylalanine (Tup, when R^{7A} is hydrogen) or tubutyrosine (Tut, when R^{7A} is -OH). There are about a dozen naturally occurring tubulysins presently known named Tubulysin A-I, Tubulysin U, Tubulysin V and Tubulysin Z, whose structures are indicated by variable groups for structure D_{G-6} defined in in embodiments of tubulysin-based quaternized drug units.

Pretubulysins have the structure D_{G} or D_{H}, wherein R³ is -CH₃ and R² is hydrogen, and desmethyl tubulysins have the structure of D_{G}, D_{G-1}, D_{G-6}, D_{H}, D_{H-1} and other tubulysin structures given by the embodiments of tubulysin-based quaternized drug units, wherein R³ is hydrogen, and wherein the other variable groups as described for tubulysins. Pretubulysins and desmethyl tubulysins and are optionally included in the definition of tubulysins.

In structures D_{G}, D_{G-1}, D_{G-6}, D_{H}, D_{H-1} and other tubulysin structures described herein in embodiments of tubulysin-based quaternized drug units, the indicated (†) nitrogen is the site of quaternization when such structures are incorporated into an LDC or precursor thereof. When incorporated into an LDC or precursor thereof that nitrogen is quaternized by covalent binding to Lₒ or to Lₒ of an L_{b} or L_{b}'-containing moiety comprised of Lₒ. Typically, that quaternized moiety of D⁺ results from covalent attachment of the tertiary amine moiety to the benzylic carbon of a PAB or PAB-type moiety of which a Y unit in Lₒ comprises or consists. Structures of other exemplary tertiary amine-containing tubulysins and pretubulysins and manner of their incorporation into an LDC are provided in embodiments of tubulysin-based quaternized drug units.

Exemplary methods of preparing tubulysin drugs and structure-activity relationships are provided by Shankar et al. "Synthesis and structure-activity relationship studies of novel tubulysin U analogs-effect on cytotoxicity of structural variations in the tubuvaline fragment" Org. Biomol. Chem. (2013) 11: 2273-2287; Xiangming et al. "Recent advances in the synthesis of tubulysins" Mini-Rev. Med. Chem. (2013) 13: 1572-8; Shankar et al. "Synthesis and cytotoxic evaluation of diastereomers and N-terminal analogs of Tubulysin-U" Tet. Lett. (2013) 54: 6137-6141; Shankar et al. "Total synthesis and cytotoxicity evaluation of an oxazole analogue of Tubulysin U" Synlett (2011) 2011(12): 1673-6; Raghavan et al. J. Med. Chem. (2008) 51: 1530-3; Balasubramanian, R. et al. "Tubulysin analogs incorporating desmethyl and dimethyl tubuphenylalanine derivatives" Bioorg. Med. Chem. Lett. (2008) 18: 2996-9; and Raghavan et al. "Cytotoxic simplified tubulysin analogues" J. Med. Chem. (2008) 51: 1530-3.

"Phenazine dimer drug" as used herein is a tertiary amine-containing compound having two aryl fused phenazine ring systems linked together through carboxamide functional groups at their C-1 positions wherein the linking moiety is comprised of a tertiary amine-containing moiety that is quaternized when incorporated as D⁺ in an LDC of the present invention. Some tertiary amine-containing phenazine dimers have the structure of D_{I}: 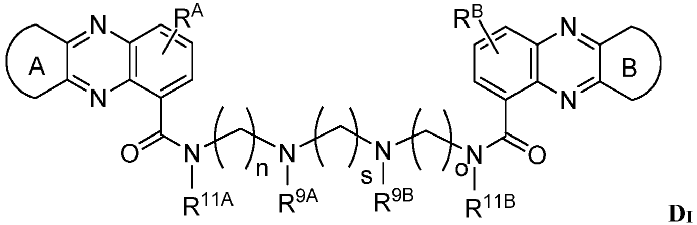 wherein Ring A and Ring B are independently selected optionally substituted aryl or optionally substituted heteroaryl fused to a phenazine ring system optional substituted with one two or three independently selected R^{A} and/or R^{B} substituents, R^{9A} and R^{9B} are independently selected optional substituted alkyl or taken together with the nitrogen atoms to which they are attached and the intervening carbon atoms between these nitrogen comprise a heterocycloalkyl ring system and wherein R^{A}, R^{B}, R^{11A}, R^{11B}, n, s and o are defined in embodiments for quaternized tertiary amine-containing drugs.

Some exemplary tertiary amine-containing phenazine dimers or phenazines having a fused aryl or heteroaryl that comprise a phenazine dimer of structure D_{I} are provided by Moorthy et al. "Fused aryl-phenazines: scaffold for the development of bioactive molecules" Curr. Drug Targets (2014) 15(7): 681-8; Zhuo et al. "Synthesis and biological evaluation of benzo[a]phenazine derivatives as a dual inhibitor of topoisomerase I and II" Org. Biomol. Chem. (2013) 11(24): 3989-4005; Kondratyuk et al. "Novel marine phenazines as potential cancer chemopreventive and anti-inflammatory agents" Marine Drugs (2012) 10(2):451-64; Gamage et al. "Phenazine-1-carboxamides: structure-cytotoxicity relationships for 9-substituents and changes in the H-bonding pattern of the cationic side chain" Bioorg. Med. Chem. (2006) 14(4):1160-8; Yang et al. "Novel synthetic isoquinolino[5,4-ab]phenazines: inhibition toward topoisomerase I, antitumor and DNA photo-cleaving activities " Bioorg. Med. Chem. (2005) 13(21): 5909-14. Gamage et al. "Structure-activity relationships for pyrido-, imidazo-, pyrazolo-, pyrazino-, and pyrrolophenazinecarboxamides as topoisomerase-targeted anticancer agents" J. Med. Chem. (2002) 45(3): 740-3; Vicker et al. "Novel angular benzophenazines: dual topoisomerase I and topoisomerase II inhibitors as potential anticancer agents" J. Med. Chem. (2002) 45(3):721-39; Mekapati et al. "QSAR of anticancer compounds: bis(11-oxo-11H-indeno[1,2-b]quinoline-6-carboxamides), bis(phenazine-1-carboxamides), and bis(naphthalimides)" Bioorg. Med. Chem. (2001) 9(11):2757-62; Gamange et al. "Dicationic bis(9-methylphenazine-1-carboxamides): relationship between biological activity and linker chain structure for a series of potent topoisomerase targeted anticancer agents" J. Med. Chem. (2001) 44: 1407-1415; and Spicer et al. "Bis(phenazine-1-carboxamides): structure-activity relationships for a new class of dual topoisomerase I/II-directed anticancer drugs" J. Med. Chem. (2000) 43(7): 1350-8.

"MDR inhibitor drugs" as used herein are tertiary amine-containing compounds that reverse or attenuate multi-drug resistance due to transporter-mediated efflux of hydrophobic cytotoxic, cytostatic or anti-inflammatory drugs, which confers resistance of hyper-proliferating cells or hyper-activated immune cells to the action of these compounds. Those transporters typically belong to the ATP binding cassette (ABC) transporter family and include P-glycoprotein, breast cancer resistant protein, and multidrug resistance-associated protein 1. Other MDR-conferring ABC transporters are described in Xia and Smith "Drug efflux and multidrug resistance in acute leukemia: therapeutic impact and novel approaches to mediation" Mol. Pharmacol. (2012) 82(6): 1008-1021. Exemplary tertiary amine-containing MDR inhibitor drugs are provided by Zinzi et al. "Small and innovative molecules as new strategy to revert MDR" Front. Oncol. (2014) doi: 10.3389/onc.2014.00002 and include Tariquidar^{™} and Elacridar^{™} and derivatives thereof as provided by Paleva et al. "Interactions of the multidrug resistance modulators Tariquidar and Elacridar and their analogues with p-glycoprotein" Chem. Med. Chem. (2013) doi: 10.1002/cmdc.201300233. Although tertiary amine-containing MDR inhibitor drugs are not cytotoxic or cytostatic in their own right they are classified as such for inclusion into tertiary amine-containing cytotoxic or cytostatic drugs that are released from an LDC of the present invention.

Exemplary MDR inhibitor drugs having a tertiary amine that are incorporated into a quaternized drug unit are provided in embodiments for quaternized tertiary amine-containing drugs. Typically, those drugs are characterized by an isoquinoline substructure whose nitrogen is the site of quaternization when incorporated into an LDC. Some exemplary MDR inhibitors thus have the structure of: 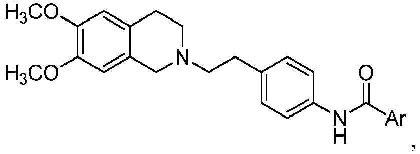 wherein Ar is an optionally substituted aryl and include Tariquidar^{™} and Elacridar^{™}.

"Prevent, "preventing" and like terms as used herein takes on its normal and customary meaning in the medical arts and therefore does not require that each instance to which the term refers be avoided with certainty.

"Intracellular metabolite" as used herein refers to a compound resulting from a metabolic process or reaction inside a cell on an LDC. The metabolic process or reaction may be an enzymatic process such as proteolytic cleavage of a peptide linker of an LDC. Intracellular metabolites include, but are not limited to, targeting moiety and free drug that have undergone intracellular cleavage after entry, diffusion, uptake or transport into a targeted cell.

"Intracellularly cleaved", "intracellular cleavage" and like terms used herein refer to a metabolic process or reaction inside a cell on an LDC or the like, whereby the covalent attachment, e.g., the linker, between the quaternary amine and the antibody is broken, resulting in the free tertiary amine-containing drug, or other metabolite of the conjugate dissociated from the targeting moiety inside the cell. The cleaved moieties of the LDC are thus intracellular metabolites.

"Bioavailability" as used herein refers to the systemic availability (i.e., blood/plasma levels) of a given amount of a drug administered to a patient. Bioavailability is an absolute term that indicates measurement of both the time (rate) and total amount (extent) of drug that reaches the general circulation from an administered dosage form.

"Subject" as used herein refers to a human, non-human primate or mammal having a hyper-proliferation, inflammatory or immune disorder or prone to such disorder that would benefit from administering an effective amount of an LDC. Non-limiting examples of a subject include human, rat, mouse, guinea pig, monkey, pig, goat, cow, horse, dog, cat, bird and fowl. Typically, the subject is a human, non-human primate, rat, mouse or dog.

The term "inhibit" or "inhibition of" means to reduce by a measurable amount, or to prevent entirely. Inhibition of proliferation of hyper-proliferating cells to an ADC is typically determined relative to untreated cells (sham treated with vehicle) in a suitable test system as in cell culture (*in vitro*) or in a xenograft model (*in vivo*)*.* Typically an LDC comprised of a targeting moiety to an antigen that is not present on the hyper-proliferating cells or activated immune-stimulating cells of interest is used as a negative control.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i.e.,* slow to some extent and preferably stop) cancer cell infiltration into peripheral organs; inhibit (*i.e.,* slow to some extent and preferably stop) tumor metastasis; inhibit, to some extent, tumor growth; and/or relieve to some extent one or more of the symptoms associated with the cancer. To the extent the drug may inhibit growth and/or kill existing cancer cells, it may be cytostatic and/or cytotoxic. For cancer therapy, efficacy can, for example, be measured by assessing the time to disease progression (TTP) and/or determining the response rate (RR).

In the case of immune disorders resulting from hyper-stimulated immune cells, a therapeutically effective amount of the drug may reduce the number of hyper-stimulated immune cells, the extent of their stimulation and/or infiltration into otherwise normal tissue and/or relieve to some extent one or more of the symptoms associated with a dysregulated immune system due to hyper-stimulated immune cells. For immune disorders due to hyper-stimulated immune cells, efficacy can, for example be measured by assessing one or more inflammatory surrogates, including one or more cytokines levels such as those for IL-1β, TNFα, INFγ and MCP-1, or numbers of classically activated macrophages.

In some aspects of the invention the Ligand-Drug Conjugate associates with an antigen on the surface of a target cell (i.e., a hyper-proliferating cell or a hyper-stimulated immune cell), and the ligand drug conjugate is then taken up inside a target cell through receptor-mediated endocytosis. Once inside the cell, one or more cleavage units within the Linker unit are cleaved, resulting in release of the tertiary amine-containing drug. The released tertiary amine-containing drug is then free to migrate in the cytosol and induce cytotoxic or cytostatic activities or in the case of hyper-stimulated immune cells may alternatively inhibit pro-inflammatory signal transduction. In another aspect of the invention, the Drug is cleaved from the Ligand-Drug Conjugate outside the target cell but within the vicinity of the target cell so that the released tertiary amine-containing unit subsequently penetrates the cell rather than being released at distal sites.

"Carrier" as the term is used herein refers to a diluent, adjuvant or excipient, with which a compound is administered. Such pharmaceutical carriers can be liquids, such as water and oils, including those of petroleum, animal, vegetable or synthetic origin, such as peanut oil, soybean oil, mineral oil, sesame oil. The carriers can be saline, gum acacia, gelatin, starch paste, talc, keratin, colloidal silica, urea,. In addition, auxiliary, stabilizing, thickening, lubricating and coloring agents can be used. In one embodiment, when administered to a patient, the compound or compositions and pharmaceutically acceptable carriers are sterile. Water is an exemplary carrier when the compounds are administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, and ethanol. The present compositions, if desired, can also contain minor amounts of wetting or emulsifying agents, or pH buffering agents.

"Treat", "treatment," and like terms, unless otherwise indicated by context, refer to therapeutic treatment and prophylactic measures to prevent relapse, wherein the object is to inhibit or slow down (lessen) an undesired physiological change or disorder, such as the development or spread of cancer or tissue damage from chronic inflammation. Typically, beneficial or desired clinical results of such therapeutic treatments include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival or quality of like as compared to expected survival or quality of life if not receiving treatment. Those in need of treatment include those already having the condition or disorder as well as those prone to have the condition or disorder.

In the context of cancer or a disease state related to chronic inflammation, the term "treating" includes any or all of inhibiting growth of tumor cells, cancer cells, or of a tumor; inhibiting replication of tumor cells or cancer cells, inhibiting dissemination of tumor cells or cancer cell, lessening of overall tumor burden or decreasing the number of cancerous cells, inhibiting replication or stimulation of pro-inflammatory immune cells, inhibiting or decreasing the chronic inflammatory state of a dysregulated immune system or decreasing the frequency and/or intensity of flares experienced by subjects having an autoimmune condition or disease or ameliorating one or more symptoms associated with cancer or a hyper-immune stimulated disease or condition.

"Pharmaceutically acceptable salt" as used herein, refers to pharmaceutically acceptable organic or inorganic salts of a compound. The compound typically contains at least one amino group, and accordingly acid addition salts can be formed with this amino group. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)) salts.

A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterions.

Typically, a pharmaceutically acceptable salt is selected from those described in P. H. Stahl and C. G. Wermuth, editors, Handbook of Pharmaceutical Salts: Properties, Selection and Use, Weinheim/Zürich:Wiley-VCH/VHCA, 2002. Salt selection is dependent on properties the drug product must exhibit, including adequate aqueous solubility at various pH values, depending upon the intended route(s) of administration, crystallinity with flow characteristics and low hygroscopicity (i.e., water absorption versus relative humidity) suitable for handling and required shelf life by determining chemical and solid-state stability under accelerated conditions (i.e., for determining degradation or solid-state changes when stored at 40 °C and 75% relative humidity).

"Loading", "drug loading", "payload loading" or like terms represent or refer to the average number of payloads ("payload" and "payloads" are used interchangeable herein with "drug" and "drugs") in an population of LDCs (i.e., a composition of LDC differing in number of attached D⁺ units having either the same or different attachment locations, but otherwise essentially identical in structure) . Drug loading may range from 1 to 24 drugs per targeting moiety. That is sometimes referred to as the DAR, or drug to targeting moiety ratio. Compositions of LDCs described herein typically have DAR's of from 1-24, and in some aspects from 1-8, from 2-8, from 2-6, from 2-5 and from 2-4. Typical DAR values are about 2, about 4, about 6 and about 8. The average number of drugs per antibody, or DAR value, may be characterized by conventional means such as UV/visible spectroscopy, mass spectrometry, ELISA assay, and HPLC. A quantitative DAR value may also be determined. In some instances, separation, purification, and characterization of homogeneous LDCs having a particular DAR value may be achieved by means such as reverse phase HPLC or electrophoresis. DAR may be limited by the number of attachment sites on the targeting moiety.

For example, when the targeting moiety is an antibody and the attachment site is a cysteine thiol, an antibody may have only one or several sufficiently reactive thiol groups that react with a L_{b}'-containing moiety. Sometimes, the cysteine thiol is a thiol group derived from of a cysteine residue that participated in an interchain disulfide bond. Other times, the cysteine thiol is a thiol group of a cysteine residue that did not participate in an interchain disulfide bond, but was introduced through genetic engineering. Typically, less than the theoretical maximum of D⁺ moieties is conjugated to an antibody during a conjugation reaction. For example, an antibody may contain many lysine residues that do not react with a L_{b}'-containing moiety, since only the most reactive lysine groups may react with that moiety.

"Antibiotic" as defined herein means a compound of natural, semi-synthetic or synthetic origin that primarily or selectively exerts is effect on prokaryotic cells (e.g., bacteria) as compared to mammalian cells. An antibiotic typically inhibits the growth of microorganisms without significant toxicity to the human or animal that is administered the antibiotic. Antibiotics include, for example, penicillins, cephalosporins, carbapenems, and ansamysins. Antibiotics are used chiefly in the treatment of infectious diseases. Drugs contemplated for the present invention are not antibiotics.

### I. Aspects

Provided herein are Ligand-drug conjugates (LDCs) capable of preferential delivery of a tertiary amine-containing drug to hyper-proliferating cells or hyper-activated immune cells or the vicinity of such abnormal cells in comparison to normal cells or the environment of normal cells where the abnormal cells are typically not present that are useful for treating diseases and conditions characterized by these abnormal cells.

### 1.1 General:

A LDC has three major components: (1) a Ligand Unit from an targeting moiety that selectively binds to a target moiety present on, within or in the vicinity of abnormal cells or other unwanted cells in comparison to other moieties present on, within, or in the vicinity of normal cells where these abnormal or unwanted cells are typically not present, or is present on, within, or in the vicinity of abnormal or other unwanted cells in greater abundance in comparison to normal cells or the environment of normal cells where abnormal or unwanted cells are typically not present, (2) a quaternized Drug Unit incorporating the structure corresponding to a tertiary amine-containing drug and (3) a Linker Unit that connects D⁺ to the Ligand Unit and is capable of conditionally releasing free tertiary amine-containing drug within or in the vicinity of abnormal or unwanted cells that are targeted by the Ligand Unit.

A tertiary amine-containing drug to be used in the present invention is one that primarily or selectively exerts its effect (e.g., cytotoxic, cytostatic effect) on mammalian cells as compared to prokaryotic cells. In some aspects the target moiety is an epitope of an extracellular displayed membrane protein that is preferentially found on abnormal or unwanted cells in comparison to normal cells.

In some aspects a cytotoxic, cytostatic, immune-suppressive or anti-inflammatory drug unit (D) having a tertiary amine group is incorporated into an LDC as a quaternized Drug Unit (D⁺) and has intracellular activity towards hyper-proliferating cells, hyper-activated immune cells or other abnormal or undesired cells that in "free" form (i.e., D is released from D⁺) may or may not be specific to these cells. Therefore, D may have selective or nonspecific cytotoxic, cytostatic, immune-suppressive or anti-inflammatory activity towards abnormal or unwanted cells in comparison to normal cells that are not associated with the abnormal or unwanted cells. Specificity towards the abnormal or unwanted cells (i.e., the targeted cells) in either situation results from the Ligand Unit (L) of the LDC into which D is incorporated. In addition to L and D⁺, an LDC that targets abnormal or unwanted cells typically has a Linker Unit that covalently attaches the quaternized Drug Unit to the Ligand Unit. In some aspects the Ligand Unit is from an antibody, which is an exemplary targeting moiety, which recognizes abnormal mammalian cells which are hyperproliferating cells.

In some aspects the target moiety recognized by the Ligand Unit is an epitope of an extracellular displayed membrane protein that is preferentially found on abnormal or unwanted cells in comparison to normal cells. In some of those aspects, as a further requirement, the membrane protein targeted by the Ligand Unit must have sufficient copy number and be internalized upon binding of the LDC in order to intracellularly deliver an effective amount of the bound cytotoxic, cytostatic, immune-suppressive or anti-inflammatory drug preferentially to the abnormal cells. Due to those restrictions the drug released from D⁺ is typically a highly active compound that as a standalone drug typically could not be administered to a subject because of intolerable side effects from destruction, damage or unwanted inhibition of normal cells distant or peripheral to the desired site of action.

Given that a tertiary-amine containing drug having adverse peripheral effects need to be selectively delivered by its LDC, the Linker unit of an LDC is not merely a passive structure that serves as a bridge between a targeting moiety and D⁺ from which the tertiary-amine containing drug is released, but must be carefully engineered to have stability from the site of administration of the LDC until its delivery to the targeted site and then must efficiently release an active drug moiety. To accomplish that task a targeting moiety is reacted with a L_{b}'-containing moiety to form a L_{b}-containing moiety. When the L_{b}-containing moiety so formed is an LDC such compounds are typically comprised of the targeting moiety in the form of a Ligand Unit, a ligand binding moiety (L_{b}) also referred to as a primary linker (L_{R}), a quaternized tertiary amine containing drug (D⁺) and a secondary linker (Lₒ) intervening between L_{b} and D⁺.

### 1.1 Primary Linker (L_{R}):

A primary linker (L_{R}) is a ligand binding moiety (L_{b}) or a ligand binding moiety precursor (L_{b}') and typically is present as a component of a Linker unit of a LDC or an L_{b}'-containing moiety such as L_{b}'-Lₒ or L_{b}'-Lₒ-D⁺. The primary linker of a L_{b}'-containing moiety is comprised of a functional group capable of reacting with an electrophilic or nucleophillic functional group of a targeting moiety. As a result of that reaction, the targeting moiety becomes covalently bonded to a primary linker through L_{b}, wherein the primary linker is now L_{b} having a functional group derived from L_{b}'.

In some embodiments L_{b}' in a L_{b}'-containing moiety has the structure of one of 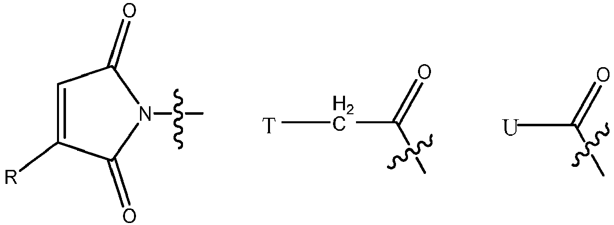 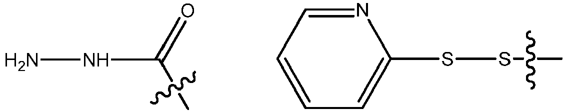 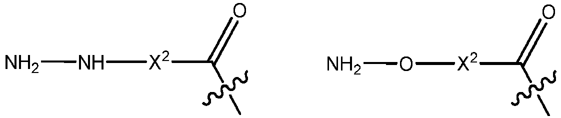 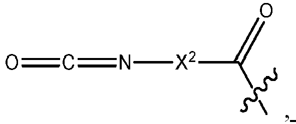 wherein R is hydrogen or C₁-C₆ optionally substituted alkyl; T is -Cl, -Br, -I, -O-mesyl, -O-tosyl or other sulfonate leaving group; U is -F, -Cl, -Br, -I, -O-N-succinimide, -O-(4-nitrophenyl), -O-pentafluorophenyl, tetrafluorophenyl or -O-C(=O)-OR⁵⁷; X² is C₁-₁₀ alkylene, C₃-C₈-carbocycle,-O-(C₁-C₆ alkyl), -arylene-, C₁-C₁₀ alkylene-arylene, -arylene-C₁-C₁₀ alkylene, -C₁-C₁₀ alkylene-(C₃-C₆-carbocycle)-, -(C₃-C₈ carbocycle)-C₁-C₁₀ alkylene-, C₃-C₈-heterocycle, -C₁-C₁₀ alkylene-(C₃-C₈ heterocyclo)-, -C₃-C₈-heterocyclo)-C₁-C₁₀ alkylene, -(CH₂CH₂O)ᵤ, or - CH₂CH₂O)ᵤ-CH₂-, wherein u is an integer ranging from 1 to 10 and R⁵⁷ is C₁-C₆ alkyl or aryl; and wherein the wavy line indicates covalent binding to a subunit of Lₒ.

On interaction with an electrophile or nucleophile of a targeting moiety L_{b}' is converted to Ligand-L_{b} moiety as exemplified below where one such interaction has taken place: 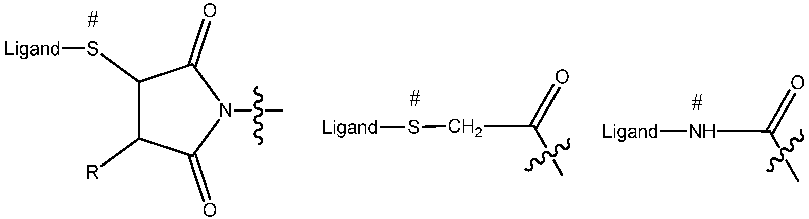 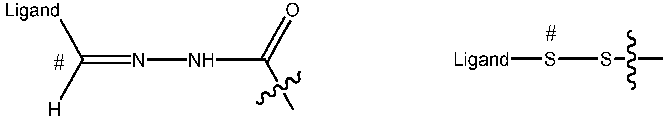 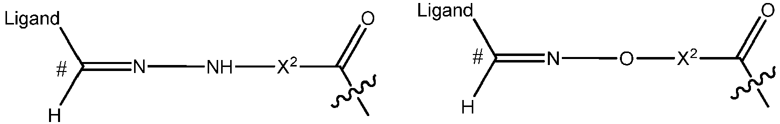 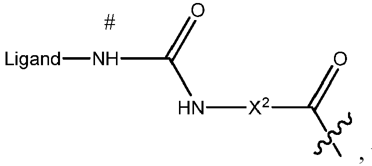 wherein the indicated (#) atom is derived from the reactive moiety of the ligand and X² is as defined.

### 1.2 Secondary Linkers (Lₒ):

Secondary linkers in a LDC or a L_{b}'-containing precursor thereof, is an organic moiety situated between the primary linker (L_{R}) and the quaternized drug unit (D⁺) that provides for processing of a cleavable unit within the LDC's Linker unit subsequent to selective binding of the LDC's targeting moiety to its cognate target moiety present on, within or in the vicinity of hyper-proliferating cells, hyper-activated immune cells or other abnormal or unwanted cells targeted by the LDC. In some embodiments W provides a substrate for a protease that is present within hyper-proliferating cells or hyper-activated immune cells. Preferred are those cleavage units that are not recognized by proteases excreted by normal cells that are not typically in the presence of hyper-proliferating cells or hyper-activated immune cells or are substrates for proteases having systemic circulation in order to minimize non-targeted drug release or systemic exposure to the tertiary amine-containing drug due its premature released from its LDC. More preferred are those proteases that are regulatory proteases or proteases found in lysosomes, which are cellular compartments to which an LDC is delivered upon internalization of a membrane-surface receptor to which the LDC has specifically bound. Regulatory and lysosomal proteases are exemplary intracellular proteases.

In one embodiment W within a secondary linker is comprised or consists of a dipeptide moiety having the structure of: 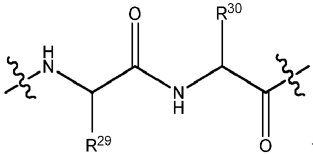 wherein R²⁹ is benzyl, methyl, isopropyl, isobutyl, sec-butyl, -CH(OH)CH₃ or has the structure of 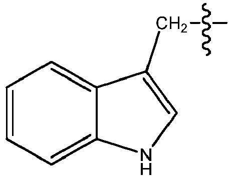 and R³⁰ is methyl, -(CH₂)₄-NH₂, -(CH₂)₃NH(C=O)NH₂, - (CH₂)₃NH(C=NH)NH₂, or , -(CH₂)₂CO₂H, wherein the dipeptide moiety provides for a recognition site for a regulatory or lysosomal protease.

In preferred embodiments the dipeptide is valine-alanine (val-ala). In another embodiment, W is comprised or consists of the dipeptide valine-citrulline (val-cit). In another embodiment W is comprised or consists of the dipeptide threonine-glutamic acid (thr-glu). In some of those embodiments the dipeptide moiety is covalently attached to a SI unit of Y through an amide bond formed between the alanine or citrulline carboxylic acid functional group or the alpha carboxylic acid functional group of glutamate and an aryl or heteroaryl amino group of SI. Thus, in those embodiments SI is comprised of an arylamine or heteroarylamine moiety and the aforementioned carboxylic acid functional group of a dipeptide moiety forms an anilide bond with the amino nitrogen that arylamine moiety.

In another embodiment, W within a secondary linker is comprised of a glycoside-bonded carbohydrate moiety having a recognition site for an intracellularly located glycosidase. In those embodiments W is a carbohydrate moiety bonded to E wherein W-E provides the recognition site for cleavage of W from E and the bond between W and E is a glycosidic bond. In those embodiments W-E typically has the structure of 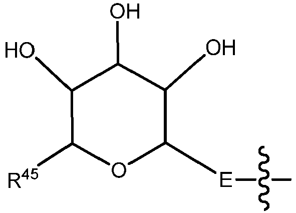 wherein R⁴⁵ is -CH₂OH or -CO₂H and E is a heteroatom moiety such as -O-, -S- or -NH- bonded to the carbohydrate moiety and to a self-immolative moiety of Y (as indicated by the wavy line) wherein the bond to the carbohydrate moiety provides for a recognition site for a glycosidase. Preferably that site is recognized by a lysosome glycosidase. In some embodiments the glycosidase is a glucuronidase as when R⁴⁵ is -CO₂H.

Secondary linkers in addition to W are also comprised of a spacer (Y) unit and may be additionally comprised of stretcher (A) unit arranged with respect to W in a linear or orthogonal relationship represent by 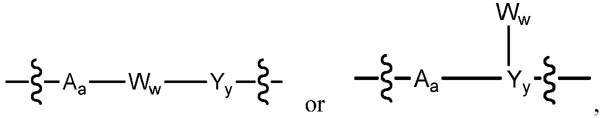
respectively, wherein the subscript w is 1, y is 1 and subscripts a is 0 or 1;
wherein the wavy line to Y indicates covalent binding of Y to D⁺ and the wavy line to A when a is 1 in both structures, or to W when in linear arrangement with Y-D⁺ or to Y when W is in orthogonal arrangement with Y-D⁺ when a is 0, indicates covalent bonding of A, W or Y, respectively, to Sc of L_{b} or L_{b}'. In preferred embodiments a, w and y are each one.

Structures of some exemplary A, W and Y moieties in Lₒ and their substituents are described in WO 2004/010957, WO 2007/038658, US Pat. Nos. 6,214,345, 7,498,298, 7,968,687 and 8,163,888, and US Pat. Publ. Nos. 2009-0111756, 2009-0018086 and 2009-0274713.

In some embodiments A moieties or it subunits (e.g., A₁, Aₒ) have the structure of 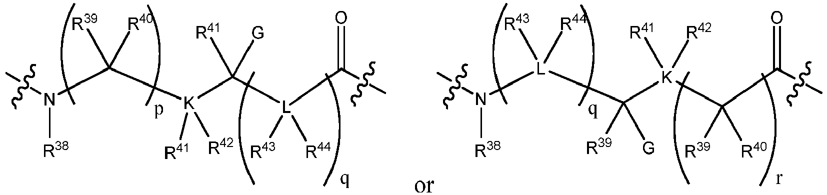
wherein the wavy line to the carbonyl moiety of either structure represents the point of attachment to the amino terminus of a dipeptide moiety comprising W arranged linearly with respect to A or to a self-immolating moiety of SI in Y described herein to which W is bonded to Y and is arranged orthogonal to A and wherein the wavy line to the amino moiety of either structures represents the point of attachment to a carbonyl-containing functional group of Sc in L_{b} or L_{b}';
wherein K and L independently are C, N, O or S, provided that when K or L is O or S, R⁴¹ and R⁴² to K or R⁴³ and R⁴⁴ to L are absent, and when K or L are N, one of R⁴¹, R⁴² to K or one of R⁴², R⁴³ to L are absent, and provided that no two adjacent L are independently selected as N, O, or S;
wherein q is an integer ranging from 0 to 12, and r is an integer ranging from 1 to 12:
wherein G is hydrogen, optionally substituted C₁-C₆ alkyl, -OH, -OR^{PR}, - CO₂H, CO₂R^{PR}, wherein R^{PR} is a suitable protecting, -N(R^{PR})(R^{PR}), wherein R^{PR} are independently a protecting group or R^{PR} together form a suitable protecting group, or - N(R⁴⁵)(R⁴⁶), wherein one of R⁴⁵, R⁴⁶ is hydrogen or R^{PR}, wherein R^{PR} is a suitable protecting group, and the other is hydrogen or optionally substituted C₁-C₆ alkyl;
wherein R³⁸ is hydrogen or optionally substituted C₁-C₆ alkyl; R³⁹-R⁴⁴ independently are hydrogen, optionally substituted C₁-C₆ alkyl, optionally substituted aryl, or optionally substituted heteroaryl, or both R³⁹, R⁴⁰ together with the carbon to which they are attached comprise a C₃-C₆ cycloalkyl, or R⁴¹, R⁴² together with K to which they are attached when K is C, or R⁴³, R⁴⁴ together with L to which they are attached when L is C comprise a C₃-C₆ cycloalkyl, or R⁴⁰ and R⁴¹, or R⁴⁰ and R⁴³, or R⁴¹ and R⁴³ to together with the carbon or heteroatom to which they are attached and the atoms intervening between those carbon and/or heteroatoms comprise a 5- or 6-membered cycloalkyl or heterocycloalkyl, provided that when K is O or S, R⁴¹ and R⁴² are absent, when K is N, one of R⁴¹, R⁴² is absent, when L is O or S, R⁴³ and R⁴⁴ are absent, and when L is N, one of R⁴³, R⁴⁴ is absent.

In some embodiments R³⁸ is hydrogen. In other embodiments -K(R⁴¹)(R⁴²) is -(CH₂)-. In other embodiments when p is not 0, R³⁹ and R⁴⁰ are hydrogen in each occurrence. In other embodiments when q is not 0, -L(R⁴³)(R⁴⁴)- is -CH₂- in each occurrence.

In preferred embodiments G is -CO₂H. In other preferred embodiments K and/or L are C. In other preferred embodiments q or p is 0. In still other preferred embodiments p + q is an integer ranging from 1 to 4.

In some embodiments A or a subunit thereof has the structure of -NH-C₁-C₁₀ alkylene-C(=O)-, -NH-C₁-C₁₀ alkylene-NH-C(=O)-C₁-C₁₀ alkylene-C(=O)-, -NH-C₁-C₁₀ alkylene-C(=O)-NH-C₁-C₁₀ alkylene (C=O)-, -NH-(CH₂CH₂O)ₛ-CH₂(C=O)-, -NH-(C₃-C₈ carbocyclo)(C=O)-, -NH-(arylene-)-C(=O)-, and -NH-(C₃-C₈ heterocyclo-)C(=O).

In other embodiments A or a subunit thereof has the structure of 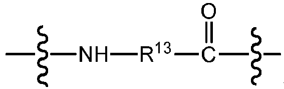 wherein R¹³ is -C₁-C₁₀ alkylene-, -C₃-C₈carbocyclo-, -arylene-, -C₁-C₃₀heteroalkylene-, -C₃-C₈heterocyclo-, -C₁-C₁₀ alkylene-arylene-, -arylene-C₁-C₁₀alkylene-, -C₁-C₁₀ alkylene-(C₃-C₈carbocyclo)-, -(C₃-C₈ carbocyclo)-C₁-C₁₀ alkylene-, - C₁-C₁₀ alkylene-(C₃-C₈ heterocyclo)-, -(C₃-C₈ heterocyclo)-C₁-C₁₀ alkylene-, - (CH₂CH₂O)₁₋₁₀(-CH₂)₁₋₃-, or -(CH₂CH₂NH)₁₋₁₀(-CH₂)₁₋₃-. In some embodiments, R¹³ is - C₁-C₁₀ alkylene- or -C₁-C₃₀heteroalkylene-. In some embodiments, R¹³ is -C₁-C₁₀ alkylene-, -(CH₂CH₂O)₁₋₁₀(-CH₂)₁₋₃-, or -(CH₂CH₂NH)₁₋₁₀(-CH₂)₁₋₃-. In some embodiments, R¹³ is -C₁-C₁₀ alkylene-polyethylene glycol, or polyethyleneimine.

In more preferred embodiments A or a subunit thereof corresponds in structure to an alpha-amino acid-, a beta-amino acid moiety, or other amine-containing acid. Other embodiments of A and subunits A₁ and Aₒ are described in embodiments for L_{R}-Lₒ as Linker units.

In some embodiments, Y moieties are capable of undergoing a 1,4- or 1,6-elimination reaction subsequent to enzymatic processing of W covalently bonded to Y (i.e., Y is comprised of an SI). In some embodiments SI of Y arranged linearly in Lₒ has the structure of: 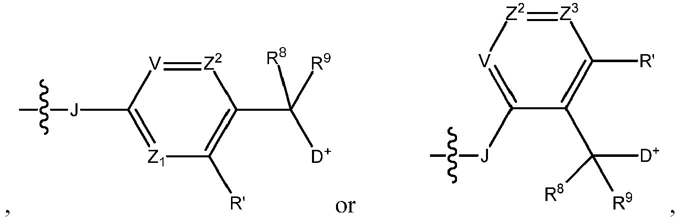
wherein V, Z¹, Z² and Z³ independently are -C(R²⁴)= or -N=; U is -O-, -S- or -N(R²⁵)-;
R²⁴ independently are hydrogen, halogen , -NO₂, -CN, -OR²⁵, -SR²⁶, - N(R²⁷)(R²⁸), optionally substituted C₁-C₆ alkyl, or -C(R²⁹)=C(R³⁰)-R³¹, wherein R²⁵ is hydrogen, optionally substituted C₁-C₆ alkyl, optionally substituted aryl or optionally substituted heteroaryl, R²⁶ is optionally substituted C₁-C₆ alkyl, optionally substituted aryl or optionally substituted heteroaryl, R²⁷ and R²⁸ independently are hydrogen, optionally substituted C₁-C₆ alkyl, optionally substituted aryl or optionally substituted heteroaryl or both R²⁷ and R²⁸ together with the nitrogen to which they are attached comprises a 5- or 6-membered heterocycle, R²⁹ and R³⁰ independently are hydrogen, or optionally substituted C₁-C₆ alkyl, and R³¹ is hydrogen, optionally substituted C₁-C₆ alkyl, optionally substituted aryl, optionally substituted heteroaryl, -C(=O)OR³² or -C(=O)NR³², wherein R³² is hydrogen, optionally substituted C₁-C₆ alkyl, optionally substituted aryl, or optionally substituted heteroaryl, R⁸ and R⁹ independently are hydrogen or optionally substituted C₁-C₆ alkyl; and R' is hydrogen or is halogen, -NO₂, -CN or other electron withdrawing group or is an electron donating group,
provided that no more than two of R²⁴ are other than hydrogen;
wherein J is -O-, S-, or -N(R³³)-, wherein R³³ is hydrogen or methyl;
wherein the wavy line to E represents covalent bonding of E to a functional group of W that inhibits the electron donating ability of E sufficiently to stabilizes the arylene moiety of SI to 1,4- or 1,6-elimination and wherein enzymatic processing of W results in dis-inhibition of that ability to trigger the elimination that releases an active drug moiety bonded to Y (e.g., when E is bonded to the carbonyl moiety of a carbonyl-containing functional group of W);
wherein upon 1,4- or 1,6-elimination a tertiary amine-containing compound within D⁺ is released.

In preferred embodiments when SI is arranged linearly in Lₒ with respect to W and D⁺ SI has the structure of 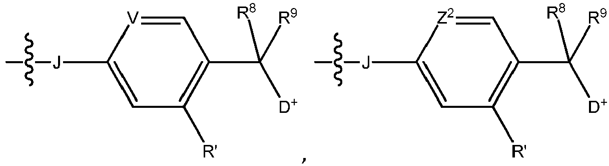 or

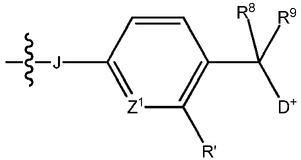

In preferred embodiments -J- is -O- or -NH- and V, Z¹ or Z² is =C(R²⁴), wherein R²⁴ is hydrogen or an electron donating group. In other preferred embodiments R⁸ and R⁹ are hydrogen and V, Z¹ or Z² is =CH-. In other preferred embodiments -J- is -NH, V, Z¹ or Z² is =CH- and R' is hydrogen or an electron donating group.

In other embodiments W-Y is arranged orthogonally in Lₒ (i.e., is -Y(W)-within the Linker unit) wherein SI of Y is bonded to a glycoside-bonded carbohydrate moiety having a recognition site for a glycosidase wherein the orthogonal arrangement involving SI is typically represented by the structure of 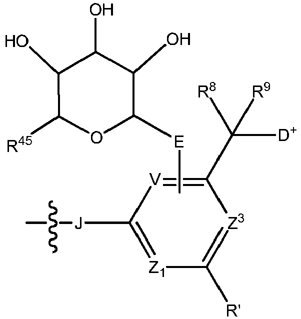 wherein E is bonded to one of V, Z¹, Z³, provided that the other V, Z¹, Z² (i.e., not bonded to E) is defined by =C(R²⁴)- or =N-, or SI is represented by the structure of 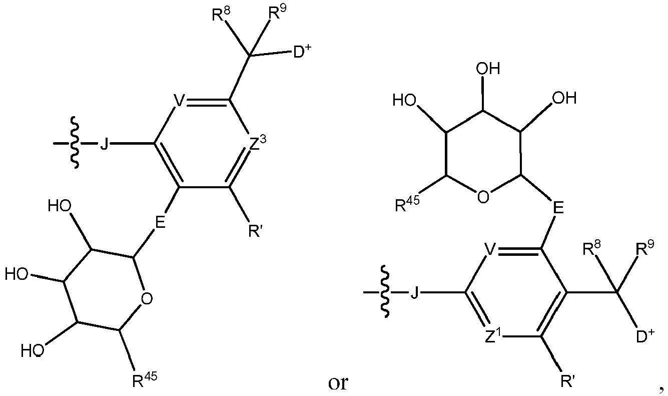
wherein V, Z¹ and Z³ independently are -C(R²⁴)= or -N=; R²⁴ independently are hydrogen, halogen, -NO₂, -CN, -OR²⁵, -SR²⁶, -N(R²⁷)(R²⁸), -C(R²⁹)=C(R³⁰)-R³¹ or optionally substituted C₁-C₆;
wherein R²⁵ is hydrogen, optionally substituted C₁-C₆ alkyl, optionally substituted aryl or optionally substituted heteroaryl; R²⁶ is optionally substituted C₁-C₆ alkyl, optionally substituted aryl or optionally substituted heteroaryl, and R²⁷ and R²⁸ independently are hydrogen, optionally substituted C₁-C₆ alkyl, optionally substituted aryl or optionally substituted heteroaryl or both R²⁷ and R²⁸ together with the nitrogen to which they are attached comprise a 5- or 6-membered heterocycle, R²⁹ and R³⁰ independently are hydrogen, or optionally substituted C₁-C₆ alkyl, and R³¹ is hydrogen, optionally substituted C₁-C₆ alkyl, optionally substituted aryl, optionally substituted heteroaryl, -CN, -C(=O)OR³² or -C(=O)NR³²; wherein R³² is hydrogen, optionally substituted C₁-C₆ alkyl, optionally substituted aryl, or optionally substituted heteroaryl;
R⁸ and R⁹ independently are hydrogen or optionally substituted C₁-C₆ alkyl; R' is hydrogen or is halogen, -NO₂, -CN or other electron withdrawing group, or is an electron withdrawing group; R⁴⁵ is -CH₂OH, -CO₂H; E is -O- or -NH-; J is -NH-; and D⁺ is as defined in embodiments described for quaternized drug units.

In preferred embodiments when W-Y is arranged orthogonally in Lₒ SI of Y has the structure of

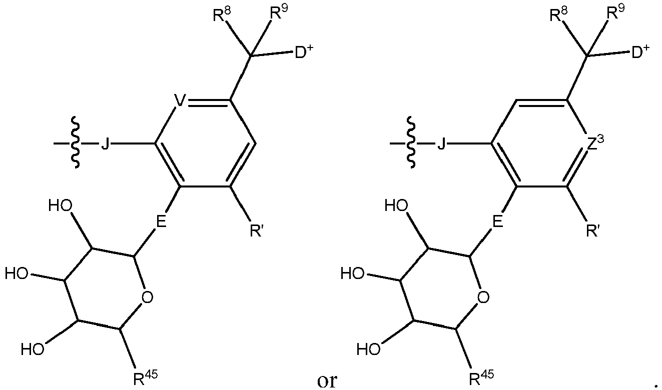

In preferred embodiments -E- is -O- or -NH- and V or Z³ is =C(R²⁴), wherein R²⁴ is hydrogen or an electron withdrawing group. In other preferred embodiments R⁸ and R⁹ are hydrogen and V, Z¹ or Z² is =CH-. In other preferred embodiments -J- is -NH, V, Z¹ or Z² is =CH- and R' is hydrogen or an electron withdrawing group.

### 1.3 L_{R}-Lₒ as Linker units

The quaternary drug unit (D⁺) attached to any of the above SI moieties disclosed herein can represent any quaternized cytotoxic, cytostatic, immunosuppressive or anti-inflammatory drug having a tertiary amine moiety (i.e., D⁺ is a quaternized tertiary amine-containing drug) whose quaternized nitrogen is attached to the benzylic position of an SI moiety.

In some embodiments -L_{b}-Lₒ-D⁺ or L_{b}'-Lₒ-D⁺ has the structure of 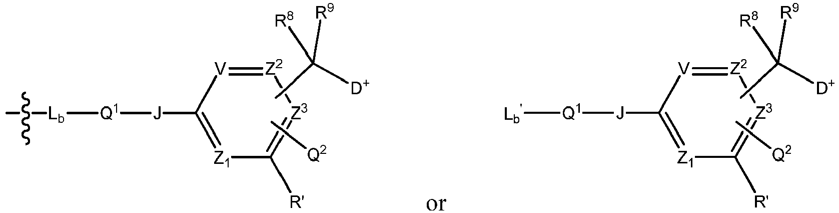
wherein the wavy line to L_{b} indicates covalent binding to a targeting moiety of a LDC, Q¹ is Aₐ-W_{w}, wherein A is an optional Stretcher unit, optionally comprised of two, three or four subunits and the subscript a is 0 or 1; Q² is W'_{w'}-E-, wherein Q², when present, is bonded to V, Z¹, Z² or Z³; W_{w} and W'_{w'} are Cleavable units; wherein W_{w} of Q¹ is capable of selective cleavage by a protease produced by abnormal cells targeted by the targeting moiety in comparison to circulating serum proteases, or by glutathione through disulfide exchange, or is more reactive to hydrolysis under lower pH conditions present in lysosomes in comparison to physiological pH of serum and W-E of Q² is cleavable by a glycosidase, wherein the subscript w is 0 or 1 and w' is 0 or 1, wherein w + w' is 1 (i.e., one and only one W is present); V, Z¹, Z² and Z³ are =N- or =C(R²⁴)-, wherein R²⁴ is hydrogen or alkyl, alkenyl or alkynyl, optionally substituted, or halogen, -NO₂, -CN, or other electron withdrawing group or is an electron donating group, -Q², or -C(R⁸)(R⁹)-D⁺, provided that when w' is 1 Q² is absent and one and only one R²⁴ is -C(R⁸)(R⁹)-D⁺ so that -C(R⁸)(R⁹)-D⁺ is bonded to one of V, Z¹, Z², Z³ and the Q¹-J- and -C(R⁸)(R⁹)-D⁺ substituents are *ortho* or *para* to each other,
provided that when w' is 1 one any only one R²⁴ is -C(R⁸)(R⁹)-D⁺ so that - C(R⁸)(R⁹)-D⁺ is bonded to one of V, Z¹, Z², Z³ and one and only one other R²⁴ is Q² so that Q² is bonded to another one of V, Z¹, Z², Z³, and the Q² and -C(R⁸)(R⁹)-D⁺ substituents are *ortho* or *para* to each other; R⁸ and R⁹ independently are hydrogen, alkyl, alkenyl or alkynyl, optionally substituted, or aryl or heteroaryl, optionally substituted; E and J independently are -O-, -S- or -N(R³³)-, wherein R³³ is hydrogen or optionally substituted alkyl; D⁺ represents a structure of a quaternized tertiary amine-containing drug D; and p is a number ranging from 1 to 24; wherein said protease cleavage, disulfide exchange, hydrolysis or glycosidase cleavage results in expulsion of D from D⁺.

In preferred embodiments, -L_{b}-Lₒ-D⁺ or L_{b}'-Lₒ-D⁺ has the structure of: 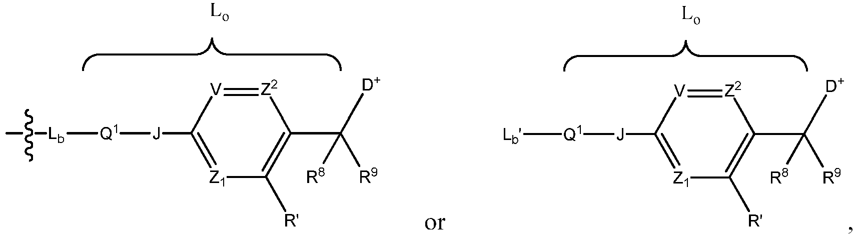 wherein V, Z¹ and Z² are independently =N- or =C(R²⁴)-, wherein R²⁴, independently selected, is hydrogen, optionally substituted alkyl or an electron donating group, and R⁸ and R⁹ independently are hydrogen or optionally substituted alkyl and J is - O- or -N(R³³), wherein R³³ is hydrogen or lower alkyl.

In other preferred embodiments, -L_{b}-Lₒ-D⁺ or L_{b}'-Lₒ-D⁺ has the structure of: 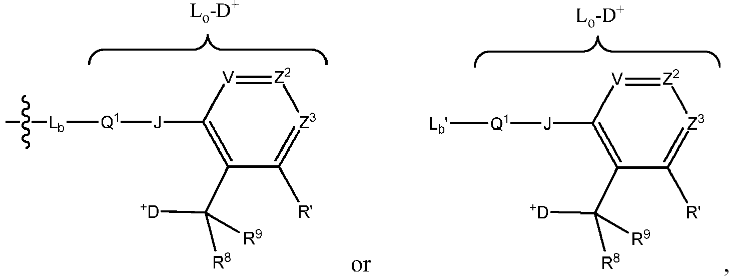 wherein V, Z² and Z³ are independently =N- or =C(R²⁴)-, wherein R²⁴, independently selected, is hydrogen, optionally substituted alkyl or an electron donating group, and R⁸ and R⁹ independently are hydrogen or optionally substituted alkyl and J is - O- or -N(R³³), wherein R³³ is hydrogen or lower alkyl.

In other preferred embodiments, -L_{b}-Lₒ-D⁺ or L_{b}'-Lₒ-D⁺ has the structure of: 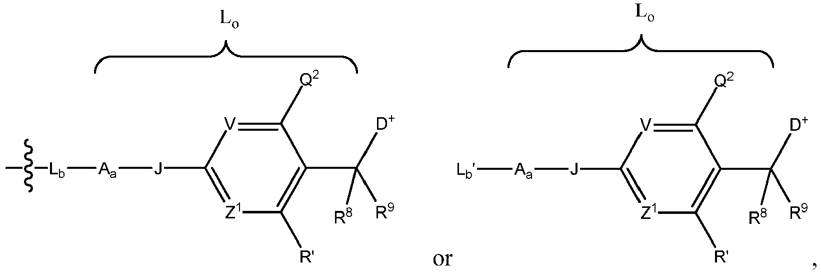 wherein V is =N- or =C(R²⁴)-, wherein R²⁴ is hydrogen, optionally substituted alkyl or an electron withdrawing group, Z¹ is =N- or =C(R²⁴)-, wherein R²⁴ is hydrogen, optionally substituted alkyl or an electron donating group and R⁸ and R⁹ independently are hydrogen or optionally substituted alkyl and J is -O- or -N(R³³), wherein R³³ is hydrogen or lower alkyl.

In more preferred embodiments of the above -L_{b}-Lₒ-D⁺ or L_{b}'-Lₒ-D⁺ structures comprised of Q², V is =C(R ²⁴)-, wherein R²⁴ is hydrogen, halogen or -NO₂.

In other preferred embodiments, -L_{b}-Lₒ-D⁺ or L_{b}'-Lₒ-D⁺ has the structure of: 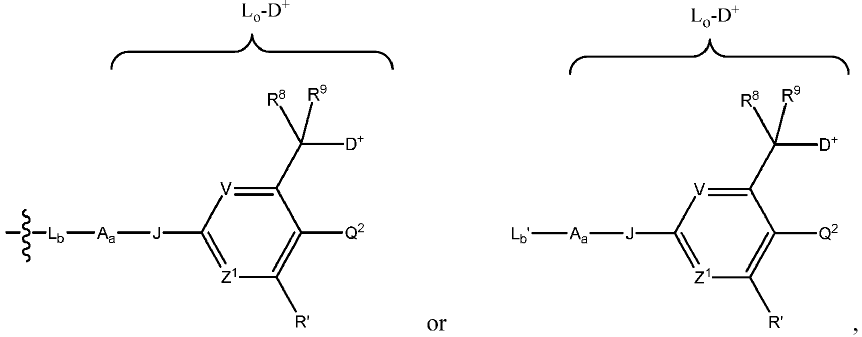 wherein V and Z¹ are independently =N- or =C(R²⁴)-, wherein R²⁴, independently selected, is hydrogen, optionally substituted alkyl or an electron donating group and R⁸ and R⁹ independently are hydrogen or optionally substituted alkyl, J is -O- or -N(R³³), wherein R³³ is hydrogen or lower alkyl and R' is hydrogen or an electron withdrawing group.

In other preferred embodiments, -L_{b}-Lₒ-D⁺ or L_{b}'-Lₒ-D⁺ has the structure of: 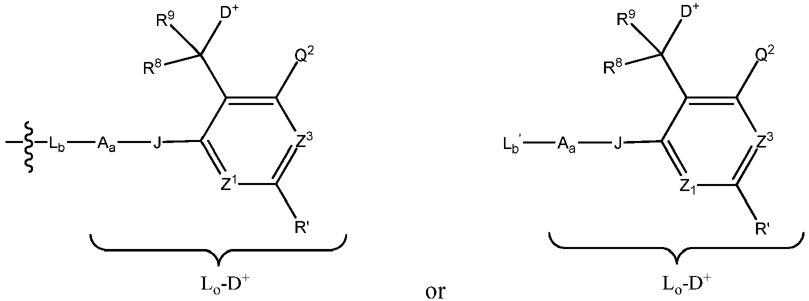 wherein Z¹ is =N- or =C(R²⁴)-, wherein R²⁴ is hydrogen, optionally substituted alkyl or an electron donating group, Z³ is =N- or =C(R²⁴)-, wherein R²⁴ is hydrogen, optionally substituted alkyl or an electron withdrawing group, and R⁸ and R⁹ independently are hydrogen or optionally substituted alkyl and J is -O- or -N(R³³), wherein R³³ is hydrogen or lower alkyl, and R' is hydrogen or an electron donating group.

In more preferred embodiments of the above -L_{b}-Lₒ-D⁺ or L_{b}'-Lₒ-D⁺ structures comprised of Q², Z³ is =C(R²⁴)-, wherein R²⁴ is hydrogen, halogen or -NO₂.

In other preferred embodiments, -L_{b}-Lₒ-D⁺ or L_{b}'-Lₒ-D⁺ has the structure of: 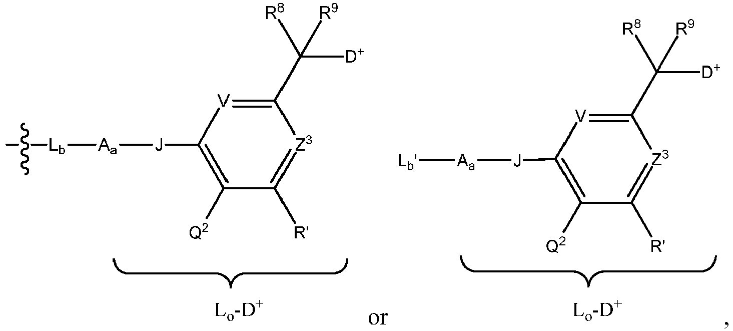 wherein V and Z³ independently are =N- or =C(R²⁴)-, wherein R²⁴, independently selected, is hydrogen, optionally substituted alkyl or an electron donating group, R⁸ and R⁹ independently are hydrogen or optionally substituted alkyl, and J is -O- or -N(R³³), wherein R³³ is hydrogen or lower alkyl, and R' is hydrogen or an electron withdrawing group.

In some embodiments L_{R}-Lₒ-D⁺ (i.e., -L_{b}-Lₒ-D⁺ or L_{b}'-Lₒ-D⁺) has the structure of 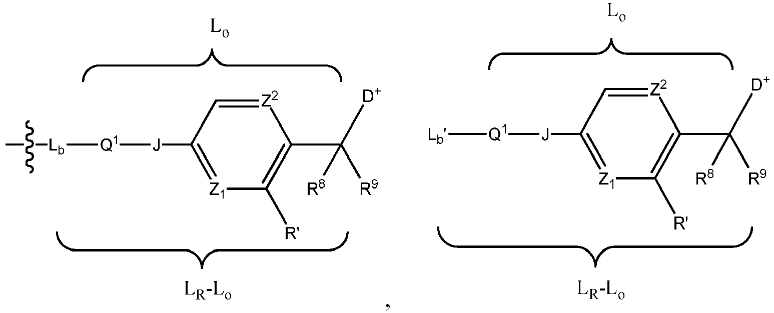 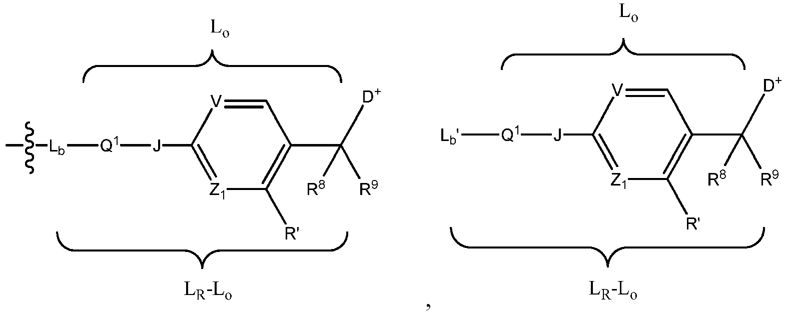 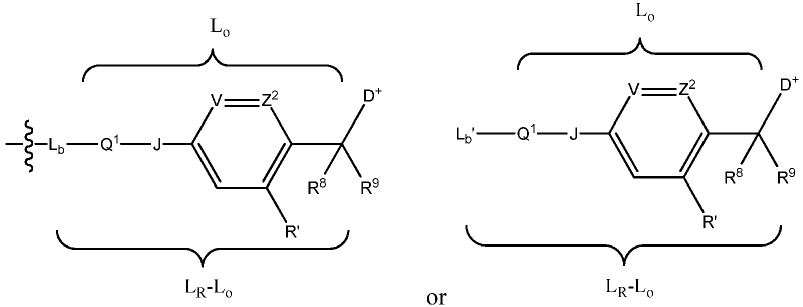 wherein V, Z¹ and Z² independently are =N- or =C(R²⁴)-, wherein R²⁴, independently selected, is hydrogen, optionally substituted alkyl or an electron donating group, R⁸ and R⁹ independently are hydrogen or optionally substituted alkyl, and J is -O- or -N(R³³), wherein R³³ is hydrogen or lower alkyl, and R' is hydrogen or an electron donating group.

In preferred embodiments, at least one of V, Z¹, Z², Z³ in any one of the above L_{R}-Lₒ-D⁺ (i.e., -L_{b}-Lₒ-D⁺ and L_{b}'-Lₒ-D⁺) structures is -CH= or is not =N-.

In more preferred embodiments L_{R}-Lₒ-D⁺ (i.e., -L_{b}-Lₒ-D⁺ or L_{b}'-Lₒ-D⁺) has the structure of 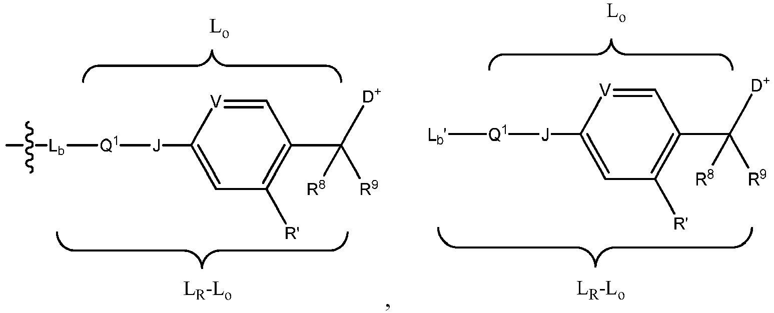 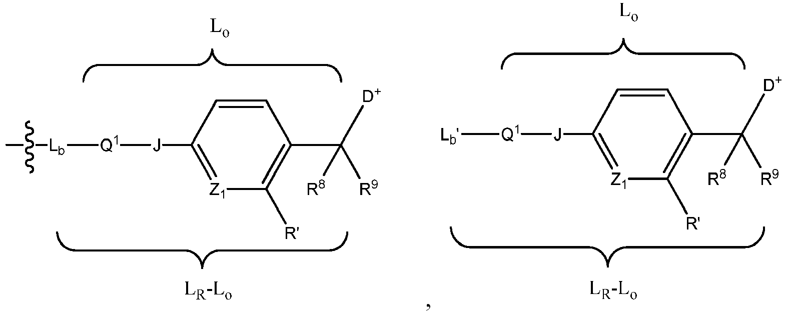 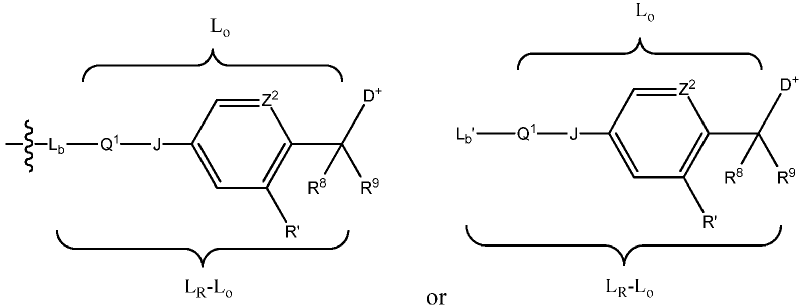 wherein V, Z¹ or Z³ is =N- or =C(R²⁴)-, wherein R²⁴, independently selected, is hydrogen, optionally substituted alkyl or an electron donating group, R⁸ and R⁹ independently are hydrogen or optionally substituted alkyl, and J is -O- or -N(R³³), wherein R³³ is hydrogen or lower alkyl, and R' is hydrogen or an electron withdrawing group.

In more preferred embodiments R⁸ and R⁹, in the any one of the above L_{R}-Lₒ structures comprised of Q¹, are hydrogen. In other more preferred embodiments in the any one of the above L_{R}-Lₒ structures comprised of Q¹, V, Z¹ or Z² is =C(R²⁴)-, wherein R²⁴ is hydrogen or an electron donating group. Thus, some of those more preferred embodiments have the structure of

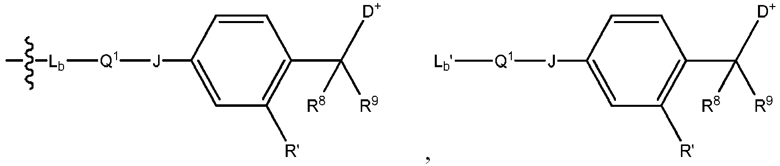

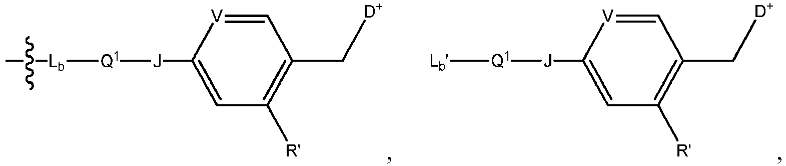

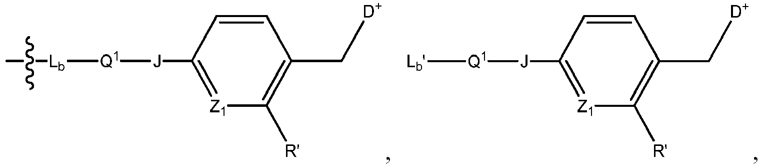

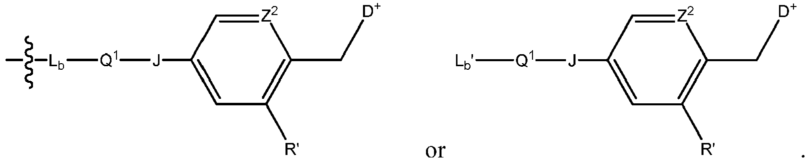

In other more preferred embodiments, A in Q¹, when A is present (i.e., a is 1) in any one of the above L_{R}-Lₒ structures comprised of Q¹, corresponds in structure to an amine-containing acid wherein the carboxylic acid terminus of the amine-containing acid is bonded to J as an ester or amide and its N-terminus is bonded to L_{b} or L_{b}' through a carbonyl-containing functional group.

In other embodiments L_{R}-Lₒ-D⁺ (i.e., -L_{b}-Lₒ-D⁺ or L_{b}'-Lₒ-D⁺) has the structure of 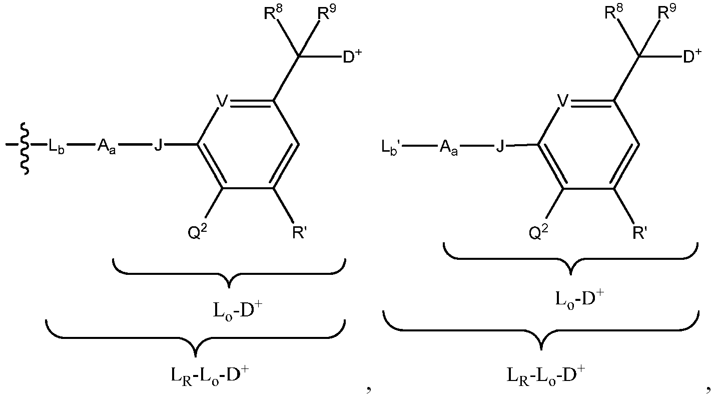 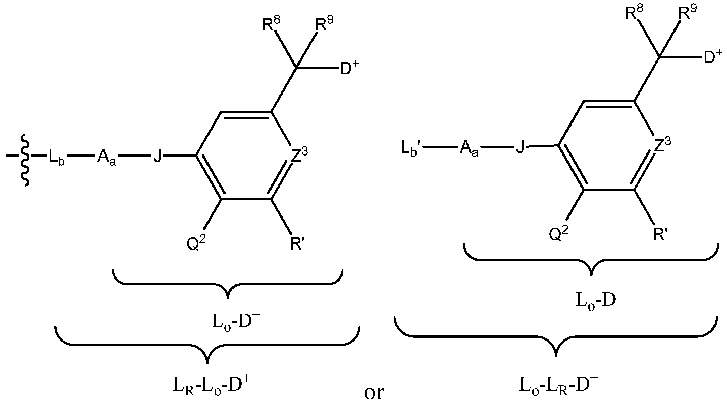 wherein V, Z¹ or Z³ is =N- or =C(R²⁴)-, wherein R²⁴, independently selected, is hydrogen, optionally substituted alkyl or an electron donating group, R⁸ and R⁹ independently are hydrogen or optionally substituted alkyl, and J is -O- or -N(R³³), wherein R³³ is hydrogen or lower alkyl, and R' is hydrogen or an electron withdrawing group.

In more preferred embodiments R⁸ and R⁹, in the any one of the above L_{R}-Lₒ structures comprised of Q², are hydrogen. In other more preferred embodiments V or Z³ is =C(R²⁴)-, wherein R²⁴ is hydrogen or an electron donating group. Thus, some of those more preferred embodiments have the structure of

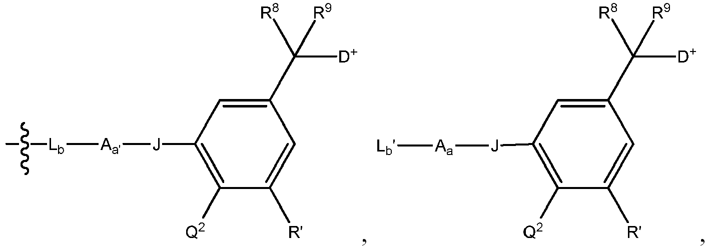

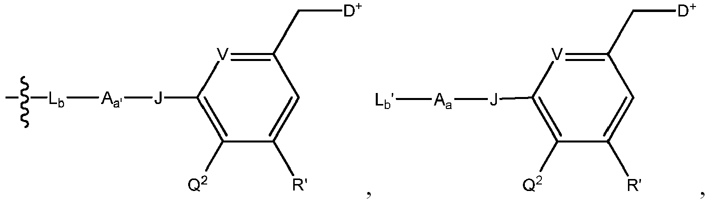

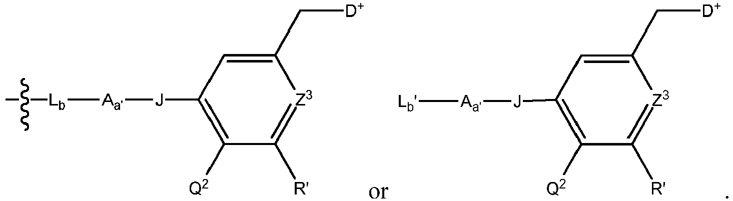

In other more preferred embodiments, A, when A is present in any one of the above L_{R}-Lₒ structures comprised of Q², corresponds in structure to an amine-containing acid wherein the carboxylic acid terminus of the amine-containing acid is bonded to J as an ester or amide and its N-terminus is bonded to L_{b} or L_{b}' through a carbonyl-containing functional group. In particularly preferred embodiments , -L_{b}-A- or L_{b}'-A in any one of the above -L_{b}-Lₒ-D⁺, L_{b}'-Lₒ-D⁺ or L_{R}-Lₒ-D⁺ embodiments has the structure of M¹-A, M¹-A₁-Aₒ-, M²-A or M²-A₁-Aₒ- represented by: 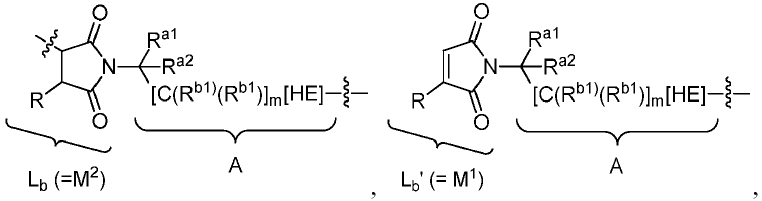 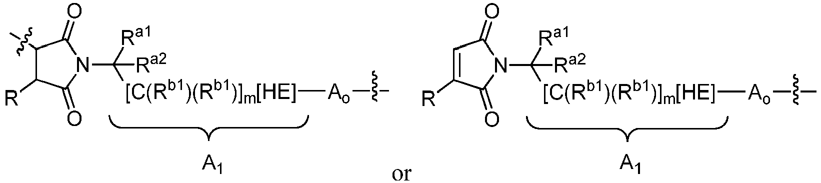 wherein A₁ and Aₒ are subunits of A, L_{b} is a succinimide (M²) moiety and L_{b}' is a maleimide moiety (M¹), wherein -[C(R^{b1})(R^{b1})]ₘ-[HE]- is A or a subunit (A₁) of A; R and R³² independently are hydrogen or optionally substituted alkyl; R^{a1} is hydrogen, lower alkyl or BU; HE is an optional hydrolysis enhancer (HE) unit; m is an integer ranging from 0 to 6; each R^{b1} independently is hydrogen, optionally substituted C₁-C₆ alkyl, optionally substituted aryl or optionally substituted heteroaryl, or two R^{b1} together with the carbon(s) to which they are attached comprise a C₃-C₆ cycloalkyl or one R^{b1} and HE together with the carbon to which they are attached comprise a 5 or 6-membered cycloalkyl or a 5- or 6-membered heterocycloalkyl and the other R^{b1} is hydrogen, optionally substituted C₁-C₆ alkyl, optionally substituted aryl or optionally substituted heteroaryl; BU is a basic unit having the structure of -[C(R¹)(R¹)]-[C(R²)(R²)]ₙ-N(R²²)(R²³), wherein n is 0, 1, 2 or 3, R¹ independently are hydrogen or lower alkyl or two R¹ together with the carbon to which they are attached comprise a C₃-C₆ cycloalkyl, R² independently are hydrogen, optionally substituted C₁-C₆ alkyl, optionally substituted aryl or optionally substituted heteroaryl, or two R² together with the carbon(s) to which they are attached and any intervening carbons define a C₃-C₆ cycloalkyl, or one R¹ and one R² together with the carbons to which they are attached and any intervening carbons comprise a 5- or 6-membered cycloalkyl and the remaining R¹ and R² are as defined; R²² and R²³ independently are hydrogen or optionally substituted C₁-C₆ alkyl or R²² and R²³ together with the nitrogen to which they are attached comprise a 5- or 6-membered heterocycloalkyl; and wherein a sulfhydryl moiety of an targeting moiety is bonded to M² as indicated by the wavy line to the succinimide moiety and wherein the wavy line to HE (or to [C(R^{b1})(R^{b1})]ₘ when HE is not present) indicates covalent binding to another subunit of A or to W in those embodiments where Q₁ is present or to V, Z¹, Z² or Z³ of an SI moiety in those embodiments where Q² is present.

In those embodiments -L_{b} and L_{b}' are referred to as succinimide (M²) moieties and -L_{b}-A-, L_{b}'-A, -L_{b}-A₁- or L_{b}'-A₁ corresponding to them are referred to as succinimide- containing moieties, which are representative L_{SS} moieties.

In other particularly preferred embodiments , -L_{b}-A- or -L_{b}-A₁-Aₒ in any one of the above L_{b}-containing embodiments has the structure of M³-A or M³-A₁-Aₒ represented by: 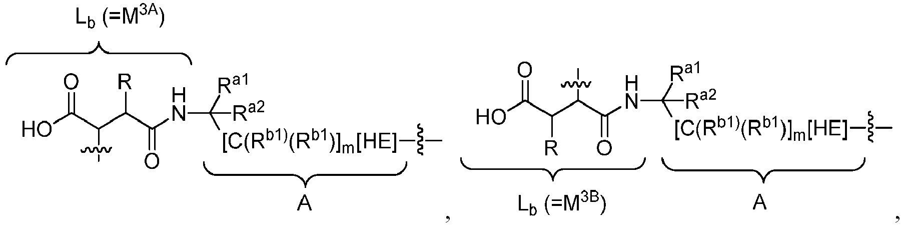 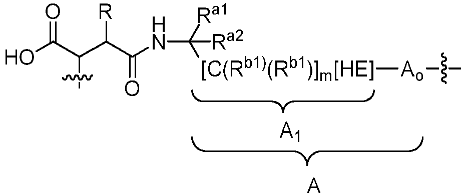 or 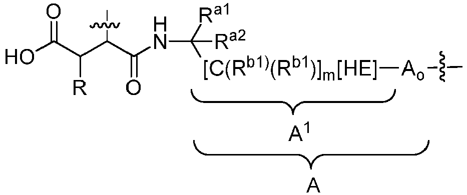 wherein A₁ and Aₒ are subunits of A, and M^{3A} and M^{3B} are regioisomers of M³ and wherein the variable groups and connectivity to a sulfhydryl group of a targeting moiety and HE (or [C(R^{b1})(R^{b1})]ₘ) are as defined for the corresponding succinimide-containing moieties shown immediately above. In the present embodiments L_{b} is referred to as a succinic acid-amide (M³) moiety and -L_{b}-A-, L_{b}'-A, -L_{b}-A₁- or L_{b}'-A₁ are referred to as succinic acid-amide-containing moieties, which are representative L_{S} moieties.

In any one of those -L_{b}-A-, L_{b}'-A, -L_{b}-A₁- and L_{b}'-A₁ or M¹-A, M¹-A₁-Aₒ-, M²-A, M²-A₁-Aₒ-, M³-A and M³-A₁-Aₒ embodiments each R^{b} independently is preferably hydrogen or lower alkyl and m is 0 or 1, R^{a1} is preferably hydrogen, lower alkyl or BU or R^{a2} is preferably hydrogen.

In any one of the above embodiments comprised of Q¹ in which Q¹ is comprised of A or A₁-Aₒ, preferred embodiments are those where W of Q¹ is bonded to A or Aₒ through an amide functional group. In those embodiments preferably A, A₁ and Aₒ have independently selected structures corresponding to amine-containing acids as described herein for stretcher unit embodiments. In any one of the above -L_{b}-A-, L_{b}'-A, - L_{b}-A₁- and L_{b}'-A₁ embodiments comprised of A or A₁, A and A₁ preferably have structures corresponding to amine-containing acids as described herein for stretcher unit embodiments wherein A is bonded to W or A₁ is bonded to Aₒ through an amide functional group. In any one of the above M¹-A, M¹-A₁-Aₒ-, M²-A, M^{Z}-A₁-Aₒ-, M³-A and M³-A₁-Aₒ embodiments, preferably A, A₁ and Aₒ have independently selected structures corresponding to amine-containing acids as described herein for stretcher unit A and subunit Aₒ embodiments. In any one of the above L_{SS} or L_{S} embodiments that are comprised of -A(BU)- or -A₁(BU)- moieties, A and A₁ preferably have structures corresponding to amine-containing acids substituted with BU, and are therefore diamino-containing acids as described herein for stretcher unit and basic unit embodiments. In M¹, M² and M³-containing moieties having A or A₁ moieties corresponding in structure to an amine-containing acid, the amine nitrogen of the amine-containing acid is incorporated as the imine nitrogen of the M¹ or M² ring system or the amide nitrogen of the M³ moiety. In L_{SS} or L_{S}-containing moieties the N-terminal amine nitrogen of the diamino-containing acid is incorporated as the imine nitrogen of the M¹ or M² ring system or the amide nitrogen of the M³ moiety. Preferably for any one of the above M¹, M² and M³-containing moieties or L_{SS} or L_{S}-containing moieties the carboxylic acid of the amine-containing acid or the diamino-containing acid is incorporated into an amide functional group to Aₒ for those moieties comprised of A₁-Aₒ- or to W for those moieties comprised of A when Aₒ is not present.

In other preferred embodiments W is comprised of a dipeptide recognized by a cathepsin protease, wherein the dipeptide is bonded to J of SI through another amide functional group, wherein the cathepsin is capable of cleaving the amide bond of W to SI to cause fragmentation of SI to release D from D⁺ bonded to SI.

In those and any one of the above embodiments comprised of HE, HE is preferably -C(=O)-. In any one of the above embodiments where W of Q¹ is comprised of a dipeptide that is recognized by a cathepsin protease, preferred dipeptides have the structure of 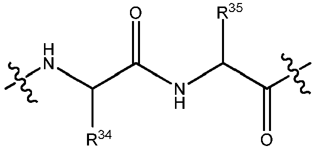 wherein R³⁴ is benzyl, methyl, isopropyl, isobutyl, sec-butyl, -CH(OH)CH₃ or has the structure of 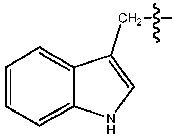 and R³⁵ is methyl, -(CH₂)₄-NH₂, -(CH₂)₃NH(C=O)NH₂, (CH₂)₃NH(C=NH)NH₂, or -(CH₂)₂CO₂H, wherein the wavy line at the dipeptide N-terminal indicates covalent binding to A or to L_{b} or L_{b}' and the wavy line at the dipeptide C-terminal indicates covalent binding to J of an SI moiety.

In preferred embodiments comprised of Q¹, -L_{b}-Lₒ-D⁺ or L_{b}'-Lₒ-D⁺ has the structure of: 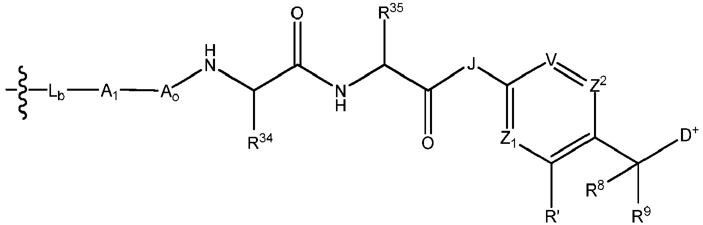 or 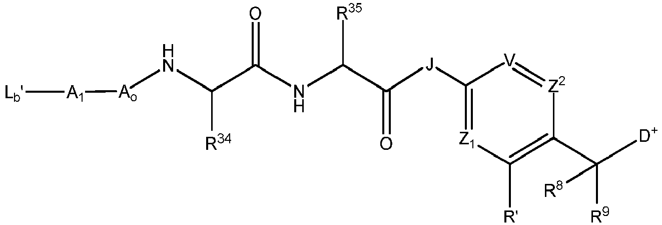 wherein A₁ and Aₒ are subunits of A, wherein Aₒ is an optional subunit (i.e., A₁ becomes A when Aₒ is absent), wherein R³⁴ is methyl, isopropyl or -CH(OH)CH₃ and R³⁵ is methyl, -(CH₂)₃NH(C=O)NH₂ or -(CH₂)₂CO₂H, wherein R', R⁸, R⁹, J, V, Z¹ and Z² are as previously defined for Formula I. In more preferred embodiments R⁸ and R⁹ are hydrogen. In other more preferred embodiments J is NH-. In still other more preferred embodiments J, V, Z¹ and Z² are each -CH=. Also more preferred are those embodiments wherein L_{b}' has the structure of a maleimide moiety (M¹) or wherein L_{b} has the structure of a succinimide moiety (M²) or an succinic acid-amide (M³) moiety.

In other preferred embodiments comprised of Q¹ a Ligand Drug Conjugate or a Drug Linker compound of formula L_{R}-L_{O}-D⁺ has the structure of 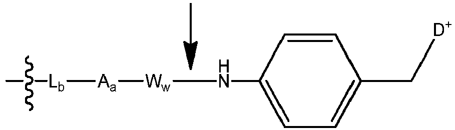 or, 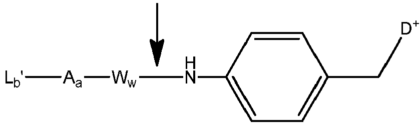 wherein the subscript w is 1, 2, 3 or more, preferably 2, and wherein W consists or is comprised of a dipeptide wherein the dipeptide are sequential Amino Acid subunits of W_{w} wherein the dipeptide subunit is at the distal end of W_{w} (i.e., the dipeptide has the formula of -W₂-W₁-) and the indicated bond is an amide bond specifically cleavable by an intracellular protease in comparison to freely circulating serum proteases. In some embodiments when the subscript a is 1 the Stretcher unit (A) is a Branching unit. In other embodiments when Aₐ is comprised of a Branching Unit (B) and the subscript a is 2 or more, B is preferably at the distal subunit of Aₐ. In other embodiments B is preferably connected to the proximal end of W_{w} and B is preferably at the distal end of Aₐ (e.g., B is A_{O} when Aₐ is -A₁-A_{O}- or Aₐ is -A₁-A_{O}-B-) For any one of those embodiments the Branching Unit is substituted by a Solubilizing Unit.

In more preferred embodiments A or A₁ and Aₒ in -A₁-A_{O}- are independently represented by structures (3) or (4): 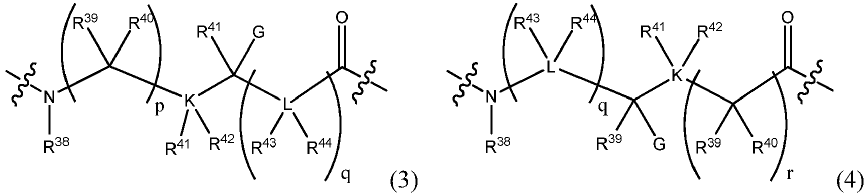 wherein the wavy line to the carbonyl moiety of either structure represents the point of attachment of A or Aₒ to W or A₁ to Aₒ preferably through an amide functional group and wherein the wavy line to the amino moiety of either structure represents the point of attachment of A or A₁ to L_{b} or L_{b}' or to a carbonyl-containing functional group of A₁ preferably forming an amide functional group; wherein the variable groups are as previously defined for structures representing A. In preferred embodiments L is absent (i.e., q is 0) and G is hydrogen, BU, -CO₂H or -NH₂ or the side chain of a naturally occurring amino acid such as aspartic acid, glutamic acid or lysine. In other preferred embodiments L and K are carbon and R⁴¹, R⁴², R⁴³ and R⁴⁴ in each occurrence is hydrogen. In other preferred embodiments R³⁸-R⁴⁴ in each occurrence is hydrogen. Other preferred embodiments have structure (3) wherein K is nitrogen and one of R⁴¹, R⁴² is absent and the other is hydrogen. Other preferred embodiments have structure (4) wherein r is 1, K is nitrogen and one of R⁴¹, R⁴² is absent and the other is hydrogen. In other preferred embodiments p and q of structure (3) are 0 or q and r of structure (4) are 0. Other preferred embodiments have structure (3) wherein p and q are both 0 and K together with R⁴¹ and R⁴² is -C(=O)-. Other preferred embodiments have structure (4) wherein q is 1 and L together with R⁴³ and R⁴⁴ is -C(=O)-.

Structure (3) and (4) also represent preferred embodiments for W₂ in -W₃-W₂-W₁- and a Branching unit provided in the latter case G is-CO₂H or -NH₂ or the side chain of a naturally occurring amino acid such as aspartic acid, glutamic acid or lysine.

In more preferred embodiments A, A₁ or Aₒ has the structure of (3a) or (4a) 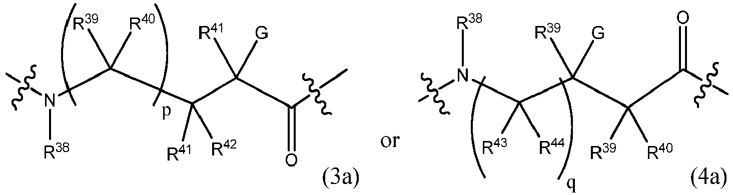 wherein p or q is 0 or 1.

When an L_{SS} or L_{S} moiety is comprised of A or A₁ preferred A or A₁ structures correspond to those shown for (3), (3a), (4) and (4a) wherein R³⁸ is absent, G is a basic unit (BU) and the N-terminal nitrogen is incorporated into a M¹ or M² moiety as the imine nitrogen of that moieties ring system or is incorporated into a M³ moiety as the amide nitrogen of the succinic acid amide.

In other more preferred embodiments A or A₁ and Aₒ of A correspond independently in structure to an alpha-amino, beta-amino or other amine-containing acid. When an L_{SS} or L_{S} moiety is comprised of A or A₁, preferred A or A₁ moieties correspond in structure to an alpha-amino, beta-amino or other amine-containing acid substituted with BU (i.e., is a diamino-containing acid), wherein the N-terminal nitrogen of the BU-substituted alpha-amino, beta-amino or other amine-containing acid, which is represented by A(BU) or A₁(BU), is incorporated into a M¹ or M² moiety as the imine nitrogen of that moiety's ring system or is incorporated into M³ as the amide nitrogen of the succinic acid amide moiety.

In those embodiments particularly preferred A(BU) or A₁(BU) have the structure of (3) or (3a) wherein p is 0 and G is BU or have the structure of (4) or (4a) wherein q is 1 and G is BU. In embodiments comprised of Q¹ wherein Aₒ is present, particularly preferred amine-containing acids that correspond to Aₒ have the structure of- NH₂-X¹-CO₂H wherein X¹ is an optionally substituted C₁-C₆-alkylene, including ε-amino-caproic acid and β-amino-propionic acid.

In preferred embodiments Linker Units of LDCs and their Drug-Linker precursors are comprised of Q² that provides a glycoside-bonded carbohydrate having a recognition site for a glycosidase. In other preferred embodiments comprised of Q², -L_{b}-Lₒ-D⁺ or L_{b}'-Lₒ-D⁺ has the structure of: 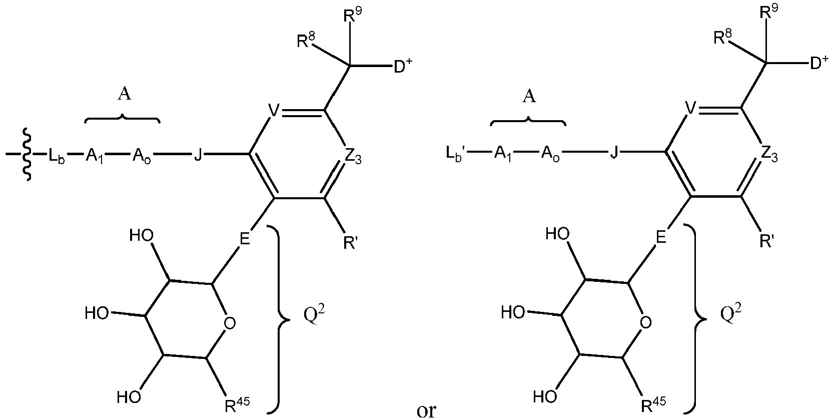 wherein A₁ and Aₒ are subunits of A wherein Aₒ is an optional subunit (i.e., when Aₒ is absent A₁ becomes A), R⁴⁵ is -CH₂OH or -CO₂H, and R⁸, R⁹, V, Z³, E and J are as previously defined. In more preferred embodiments, R⁸ and R⁹ are hydrogen. In other more preferred embodiments, E is -O-. In other more preferred embodiments J is - NH-. In other more preferred embodiments, one or both of V and Z³ are -CH=. Also more preferred are those embodiments wherein L_{b}' has the structure of a maleimide moiety (M¹) or wherein L_{b} has the structure of a succinimide moiety (M²) or an succinic acid-amide moiety (M³). More preferred are those embodiments in which L_{b}'-A₁ has the structure of M¹-A₁, or L_{b}-A₁ has the structure given above for M²-A₁ or M³-A₁. In any one of those embodiments

In preferred embodiments comprised of Q², Aₒ is present and has the structure previously defined for (3), (3a), (4) or (4a), wherein the wavy line to the carbonyl moiety of any one of the structure represents the point of attachment of Aₒ to J preferably through an amide functional group and wherein the wavy line to the amino moiety of either structure represents the point of attachment to a carbonyl-containing functional group of A₁, preferably forming an amide functional group; wherein the variable groups are as previously defined for structures representing A. In preferred embodiments L is absent (i.e., q is 0) and G is hydrogen, -CO₂H or -NH₂ or the side chain of a naturally occurring amino acid such as aspartic acid, glutamic acid or lysine. In other preferred embodiments, L and K are carbon and R⁴¹, R⁴², R⁴³ and R⁴⁴ in each occurrence is hydrogen. In other preferred embodiments R³⁸-R⁴⁴ in each occurrence is hydrogen. Other preferred embodiments have structure (3) wherein K is nitrogen and one of R⁴¹, R⁴² is absent and the other is hydrogen. Other preferred embodiments have structure (4) wherein r is 1, K is nitrogen and one of R⁴¹, R⁴² is absent and the other is hydrogen. In other preferred embodiments p and q of structure (3) are 0 or q and r of structure (4) are 0. Other preferred embodiments have structure (3) wherein p and q are both 0 and K together with R⁴¹ and R⁴² is -C(=O)-. Other preferred embodiments have structure (4) wherein q is 1 and L together with R⁴³ and R⁴⁴ is -C(=O)-.

In more preferred embodiments A or A₁ and Aₒ of A correspond independently in structure to an alpha-amino, beta-amino or other amine-containing acid. In other more preferred embodiments having an L_{SS} or L_{S} moiety that moiety is comprised of A or A₁ with preferred structures corresponding to those shown for (3), (3a), (4) and (4a) wherein R³⁸ is absent, G is a basic unit (BU) and the N-terminal nitrogen is incorporated into a M¹ or M² moiety as the imine nitrogen of that moieties ring system or is incorporated into a M³ moiety as the amide nitrogen of the succinic acid amide. Other preferred A or A₁ moieties for L_{SS} or L_{S} correspond in structure to an alpha-amino, beta-amino or other amine-containing acid substituted with BU (i.e., is a diamino-containing acid), wherein the N-terminal nitrogen of the BU-substituted alpha-amino, beta-amino or other amine-containing acid, which is represented by A(BU) or A₁(BU), is incorporated into a M¹ or M² moiety as the imine nitrogen of that moiety's ring system or is incorporated into M³ as the amide nitrogen of the succinic acid amide moiety.

In embodiments comprised of Q² wherein Aₒ is present, particularly preferred amine-containing acids that correspond to Aₒ have the structure of -NH₂-X¹-CO₂H wherein X¹ is an optionally substituted C₁-C₆-alkylene, including ε-aminocaproic acid and β-amino-propionic acid.

Particularly preferred are any one of the above L_{b}-containing embodiments wherein the targeting moiety bonded to L_{b} is an antibody.

### 1.4 Quaternized drug units

Useful classes of tertiary amine-containing drugs include, by way of example and not limitation those having cytostatic or cytotoxic activity on abnormal cells or normal cells in the vicinity of abnormal cells (i.e., tumor-associated cells). In some embodiments the tertiary amine-containing drug has cytostatic or cytotoxic activity on hyper-stimulated immune cells. In other embodiments the tertiary amine-containing drug has cytostatic or cytotoxic activity on tumor cells or tumor-associate cells. Such tertiary amine-containing drugs include tubulysins, dolastatins and auristatins having an N-terminal tertiary amine moiety, phenazine dimers, and tetrahydroquinoline-based MDR inhibitors.

### 1.4.1 Quaternized dolastatins and auristatins

In some embodiments, the anti-proliferative quaternized tertiary amine-containing drug is a tertiary amine-containing dolastatin, including dolastatin 10, dolastatin 15 and related compounds, wherein the indicated nitrogen (†) exemplified for dolastatin 10 and dolastatin 15 is quaternized by covalent binding to the benzylic position of a PAB or PAB-type self-immolating moiety in Lₒ.

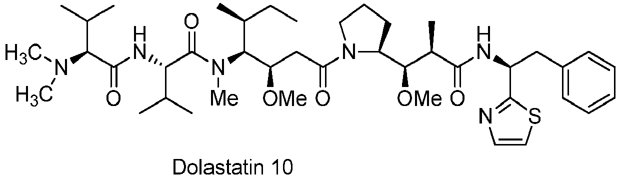

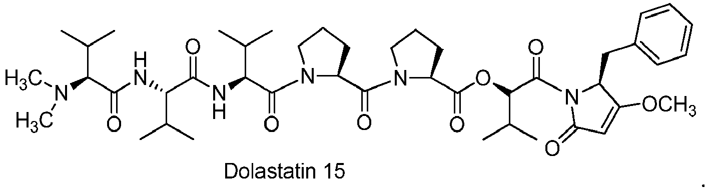

In those embodiments exemplary dolastatins, which are related to dolastatin 10, are those wherein the phenyl and thiazole substituents of dolastatin 10 are replaced with independently selected aryl or heteroaryl moieties. Other exemplary dolastatins are related to dolastatin 15 wherein the C-terminal ester moiety is replaced by an amide functional group wherein the amide nitrogen of that functional group is substituted with an arylalkyl or hetereoarylalkyl moiety.

Structures for other dolastatin-based tubulin disrupting agents are the following wherein the indicated nitrogen (†) is quaternized by covalent binding to the benzylic position of a PAB or PAB-type self-immolating moiety: 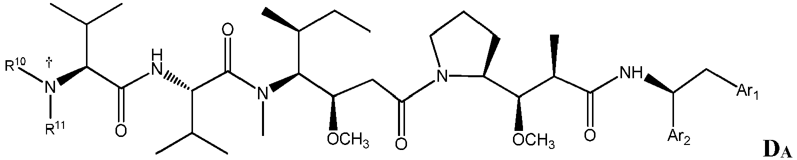 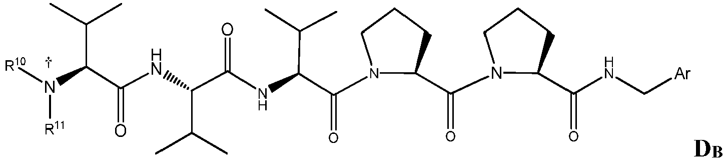 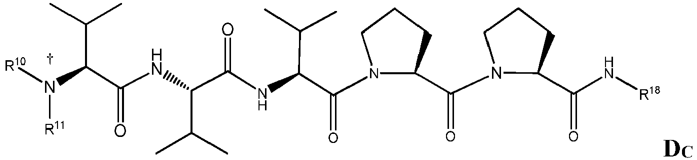 wherein each R¹⁰, R¹¹ and R¹⁸ is an independently selected optionally substituted C₁-C₆ alkyl and Ar or Ar₁ and Ar₂, independently selected, are optionally substituted aryl or optionally substituted heteroaryl. Other peptoid mimetics of dolastatins having a tertiary amine capable of quaternization are provided by Schmitt et al. "Synthesis of dolastatin 15 peptoids" Bioorg. Med. Chem. Lett. (1998) 8(4): 385-8. In preferred embodiments R¹⁰ and R¹¹ are methyl. In other preferred embodiment R¹⁸ is t-butyl. In other preferred embodiments Ar is an optionally substituted phenyl or Ar₁ and/or Ar₂, independently selected, are optionally substituted phenyl. In still other preferred embodiments Ar₁ is phenyl and Ar₂ is 2-thiazolyl.

In some embodiments, the quaternized drug is an auristatin having an N-terminal tertiary amine whose nitrogen is quaternized by its covalent binding to the benzylic position of a PAB or PAB-type self-immolating moiety. The syntheses and structures of auristatins having an N-terminal tertiary amine are described in U.S. Patent Application Publication Nos. 2003-0083263, 2005-0238649 2005-0009751, 2009-0111756, and 2011-0020343; International Patent Publication No. WO 04/010957, International Patent Publication No. WO 02/088172, and U.S. Patent Nos. 7,659,241 and 8,343,928.. Exemplary tertiary-amine containing auristatins released from LDCs of the present invention bind tubulin and exert a cytotoxic or cytostatic effect on the desired cells (i.e., target cells) as a result of that binding.

Exemplary auristatins having a N-terminal tertiary amine are represented by the following formula wherein the indicated nitrogen (†) is the site of quaternization when such compounds are incorporated into an LDC as a quaternized drug unit (D⁺): 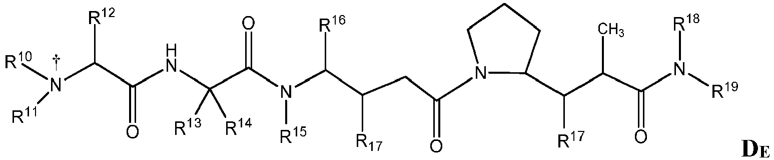 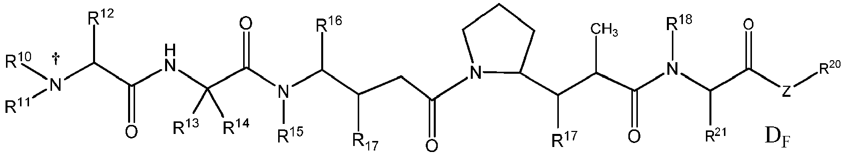
wherein R¹⁰ and R¹¹ are independently C₁-C₈ alkyl; R¹² is hydrogen, C₁-C₈ alkyl, C₃-C₈ cycloalkyl, aryl, -X¹-aryl, -X¹-(C₃-C₈ cycloalkyl), C₃-C₈ heterocycle or -X¹-(C₃-C₈ heterocycle); R¹³ is hydrogen, C₁-C₈ alkyl, C₃-C₈ cycloalkyl, aryl, -X¹-aryl, -X¹-(C₃-C₈ cycloalkyl), C₃-C₈ heterocycle and -X¹-(C₃-C₈ heterocycle); R¹⁴ is hydrogen or methyl, or R¹³ and R¹⁴ taken together with the carbon to which they are attached comprise a C₃-C₈ cycloalkyl; R¹⁵ is hydrogen or C₁-C₈ alkyl; R¹⁶ is hydrogen, C₁-C₈ alkyl, C₃-C₈ cycloalkyl, aryl, -X¹-aryl, -X¹-(C₃-C₈ cycloalkyl), C₃-C₈ heterocycle and -X¹-(C₃-C₈ heterocycle); R¹⁷ independently are hydrogen, -OH, C₁-C₈ alkyl, C₃-C₈ cycloalkyl and O-(C₁-C₈ alkyl); R¹⁸ is hydrogen or C₁-C₈ alkyl; R¹⁹ is -C(R^{19A})₂-C(R^{19A})₂-aryl, -C(R^{19A})₂-C(R^{19A})₂-(C₃-C₈ heterocycle) or -C(R^{19A})₂-C(R^{19A})₂-(C₃-C₈ cycloalkyl), wherein each R^{19A} independently is hydrogen, C₁-C₈ alkyl, -OH or -O-C₁-C₈ alkyl;
R²⁰ is hydrogen, C₁-C₂₀ alkyl, aryl, C₃-C₈ heterocycle, -(R⁴⁷O)ₘ-R⁴⁸, and - (R⁴⁷O)ₘ-CH(R⁴⁹)₂; R²¹ is arylalkyl, including -CH₂Ph, hydroxylalkyl, including - CH(CH₃)-OH or C₃-C₈ heterocycle; Z is O, S, NH, or NR⁴⁶, wherein R⁴⁶ is C₁-C₈ alkyl; m is an integer ranging from 1-1000; R⁴⁷ is C₂-C₈ alkyl; R⁴⁸ is hydrogen or C₁-C₈ alkyl; R⁴⁹ independently are -COOH, -(CH₂)ₙ-N(R⁵⁰)₂, -(CH₂)ₙ-SO₃H, or -(CH₂)ₙ-SO₃-C₁-C₈ alkyl; R⁵⁰ independently are C₁-C₈ alkyl, or -(CH₂)ₙ-COOH; n is an integer ranging from 0 to 6; and X¹ is C₁-C₁₀ alkylene.

Some preferred auristatins having an N-terminal tertiary amine are represented by the following formula wherein the indicated nitrogen (†) is the site of quaternization when such compounds are incorporated into an LDC as a quaternized drug unit (D⁺): 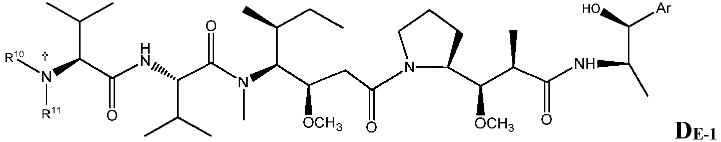 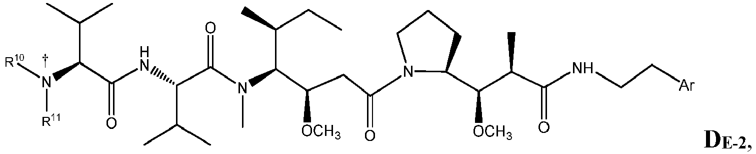 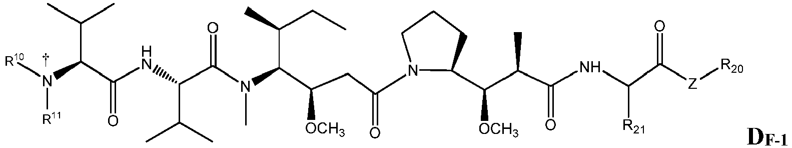 wherein R¹⁰ and R¹¹ are independently C₁-C₆ alkyl in structures D_{E-1}, D_{E-2} and D_{F-1}, Ar in D_{E-1} or D_{E-2} is optionally substituted aryl or optionally substituted heteroaryl, and in D_{F-1} Z is -O-, or -NH- R²⁰ is hydrogen, C₁-C₆ alkyl, optionally substituted aryl or optionally substituted heteroaryl, and R²¹ is optionally substituted C₁-C₆ alkyl, optionally substituted arylalkyl or optionally substituted heteroarylalkyl.

In more preferred embodiments R¹⁰ and R¹¹ is methyl. In other more preferred embodiments in D_{E-1} or D_{E-2} Ar is optionally substituted phenyl or optionally substituted 2-pyridyl. In other more preferred embodiments in D_{F-1}, R²¹ is X¹-S-R^{21a} or X¹-Ar, wherein X¹ is C₁-C₆ alkylene, R^{21a} is lower alkyl and Ar is optionally substituted phenyl or heteroaryl. In other preferred embodiments -Z- is -O- and R²⁰ is lower alkyl. In other preferred embodiments Z is -NH- and R²⁰ is optionally substituted phenyl or optionally substituted heteroaryl. In other embodiments R²¹ is -X¹-Ar, X is -NH- and R²⁰ is Ar, wherein X¹ and Ar independently selected are as previously defined for R²¹.

Some preferred auristatins having an N-terminal tertiary amine are represented by the following formula wherein the indicated nitrogen (†) is the site of quaternization when such compounds are incorporated into an LDC as a quaternized drug unit (D⁺): 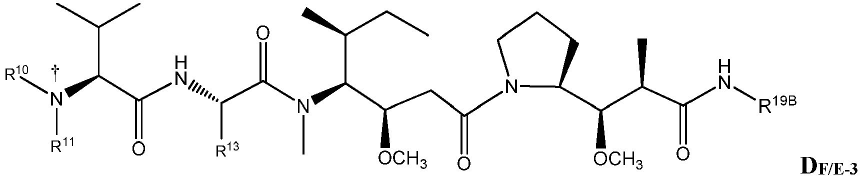 wherein R¹⁰ and R¹¹ are methyl, R¹³ is isopropyl or -CH₂-CH(CH₃)₂, and R^{19B} is -CH(CH₂Ph)-2-thiazole, -CH(CH₂Ph)-2-pyridyl, -CH(CH₂-p-Cl-Ph), -CH(CO₂Me)-CH₂Ph, -CH(CO₂Me)-CH₂CH₂SCH₃, CH(CH₂CH₂SCH₃)C(=O)NH-3-quinolyl, - CH(CH₂Ph)C(=O)NH-p-Cl-Ph or R^{19B} has the structure of

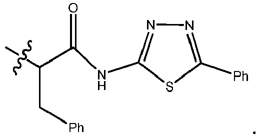

Particularly preferred tertiary amine-containing auristatins incorporated into a quaternized drug unit include but are not limited to, Auristatin E, Auristatin PE, Auristatin PHE, Auristatin PYE, Auristatin EFP, Auristatin EB and Auristatin EVB.

In some embodiments an L_{b}'-Lₒ-D⁺ moiety that incorporates a dolastatin or an auristatin having an N-terminal tertiary amine has the structure of 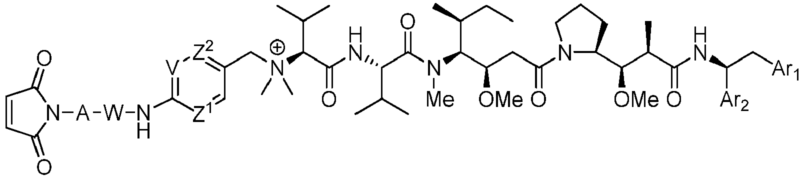 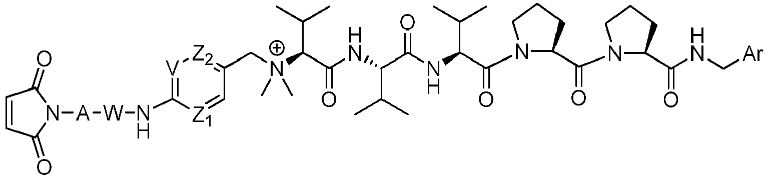 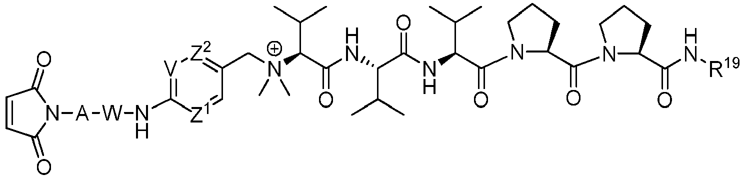 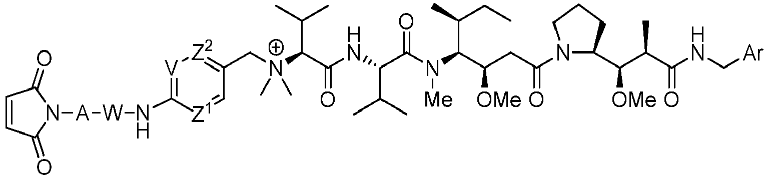 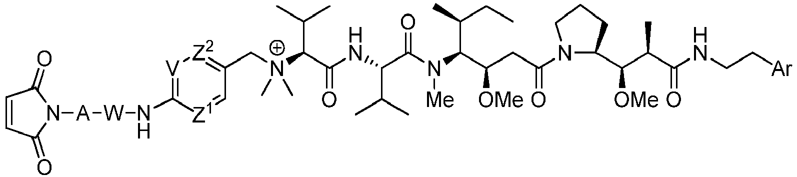 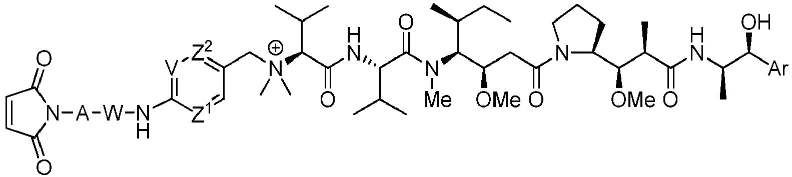 or 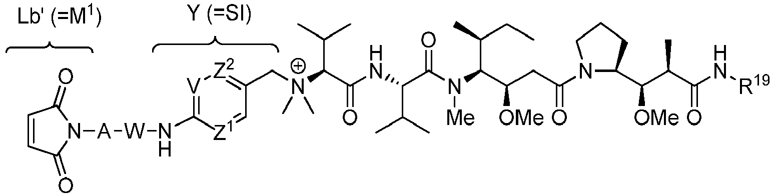 wherein L_{b}' is a maleimide moiety (M¹) and A, W, V, Z¹ and Z² are as previously defined for Formula 1 and Ar, Ar₁, Ar₂, and R¹⁹ are as previously defined herein for tertiary amine-containing dolastatins and auristatins, wherein the aniline nitrogen of SI (of which the Spacer unit Y consists) is bonded to W through a carbonyl of an anilide functional group. In preferred embodiments one, two or all of V, Z¹, Z² are =CH-. In other preferred embodiments Ar or Ar₁ and Ar₂ are independently optionally substituted phenyl. In more preferred embodiments Ar is phenyl or Ar₁ and Ar₂ are both phenyl and V, Z¹ and Z² are =CH-.

In other preferred embodiments a Drug-Linker compound of formula L_{b}'-Lₒ-D⁺ having a peptide cleavable Unit wherein D⁺ is a quaternized auristatin has the structure of one of:

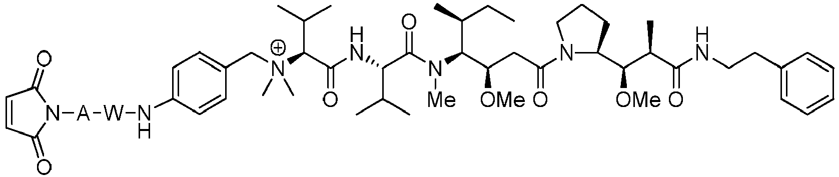

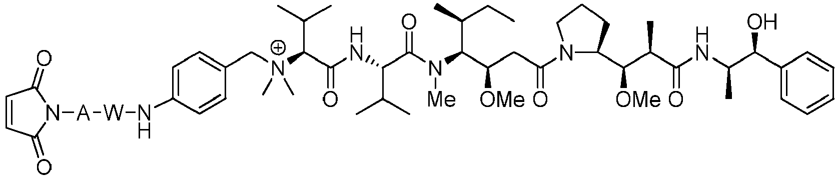

In any one of the above embodiments comprised of a quaternized auristatin drug unit preferred embodiments are those where L_{b}' (i.e., M¹) is replaced with a L_{b} moiety that is also bonded to a targeting moiety to provide an LDC wherein that L_{b} moiety is an M² or M³ moiety and/or A is replaced with -A₁-Aₒ, wherein A₁ and Aₒ are subunits of A.

In any one of the above L_{b} or L_{b}'-containing moieties comprised of a quaternized auristatin drug unit corresponding in structure to D_{E}, D_{F}, D_{F-1}, D_{E-1}, D_{E-2} or D_{F/E-3}, a preferred Cleavable unit (W) is comprised of a dipeptide recognized by a cathepsin protease capable of cleaving the anilide bond that covalently attaches W to SI. In more preferred embodiments W is comprised of a dipeptide having the structure of 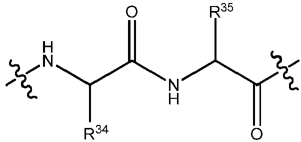 wherein R³⁴ and R³⁵ and attachments indicated by the wavy lines to A (or a subunit thereof) and Y units are as previously defined. In particularly preferred embodiments R³⁴ is -CH(CH₃)₂ and R³⁵ is -(CH₂)₃-NH(=O)NH₂, or R³⁴ is - CH(CH₃)₂ and R³⁵ is -CH₃; and wherein the alpha carbons to R³⁴ and R³⁵ are in the same absolute configuration of an L-amino acid.

In any one of the above L_{b} or L_{b}'-containing moieties comprised of a quaternized auristatin drug unit, a preferred Stretcher unit A or a subunit thereof (i.e., A₁) correspond in structure to an alpha amino acid, a beta amino acid or other amine-containing acid such as an amine-containing acid having the formula of NH₂-X¹-CO₂H wherein X¹ is a C₁-C₆ alkylene, wherein the N-terminal nitrogen of the amine-containing acid corresponds to the maleimide or succinimide imide nitrogen of an M¹ or M² moiety or the amide nitrogen of an succinic acid-amide (M³) moiety, and the carbonyl group corresponds to the C-terminus of the amine-containing acid that is bonded to W through an amide functional group. Thus, preferred M¹-A-, M²-A or M³-A moieties or preferred M¹-A₁-, M²-A₁ or M³-A₁ moieties have the structure of

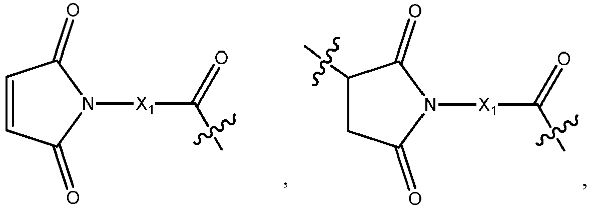

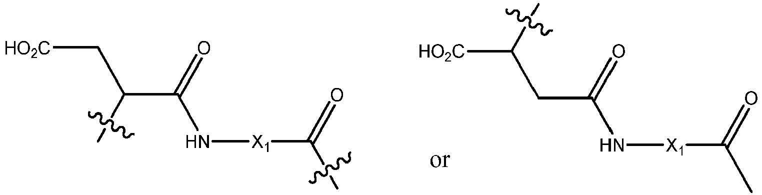

More preferred are those A units or subunits thereof corresponding to or derived from β-amino propionic acid, γ-amino butyric acid or ε-amino caproic acid. Other preferred A or A₁moieties correspond in structure to, or are derived from, diamino-containing acids having the formula of NH₂-C(R^{a1})(R^{a2})-[C(R^{b1})(R^{b1})]ₘ-C(=O)OH, wherein R^{a1} has the formula of -[C(R¹)(R¹)]-[C(R²)(R²)]ₙ-N(R²²)(R²³), wherein the variable groups are as previously defined for A(BU)- and A₁(BU)- moieties. More preferred are those A or A₁ moieties wherein R^{a1} is -CH₂NH₂. Thus, a preferred -A- or A₁ has the structure of -C(R^{a1})(R^{a2})-[C(R^{b2})(R^{b1})]ₘ-C(=O)-.

In more preferred embodiments of Drug-Linker compounds of formual L_{b}'-Lₒ-D⁺ having a peptide Cleavable Unit W wherein D+ is a quaternized auristatin have the structures of:

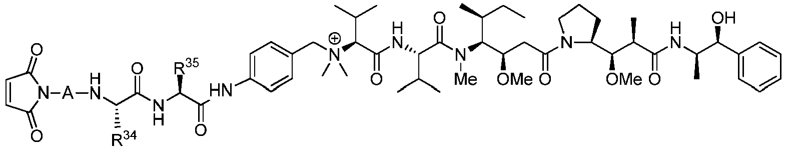

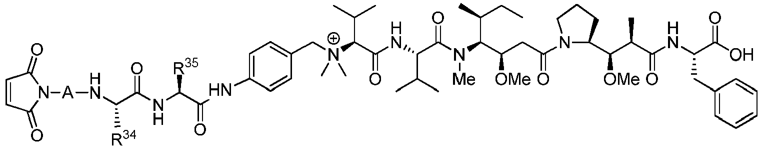

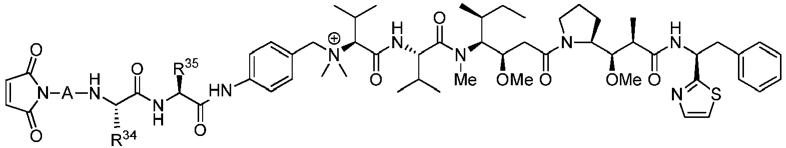

In some embodiments more preferred are Drug-Linker compounds of formula L_{b}'-Lₒ-D⁺ having a peptide cleavable Unit, wherein D⁺ is a quaternized auristatin and a Stretcher Unit comprised of M¹ and X¹, have the structures of

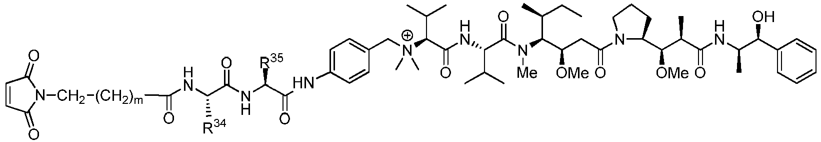

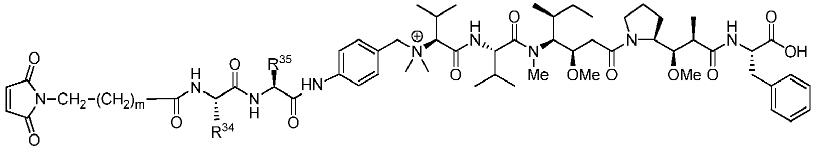

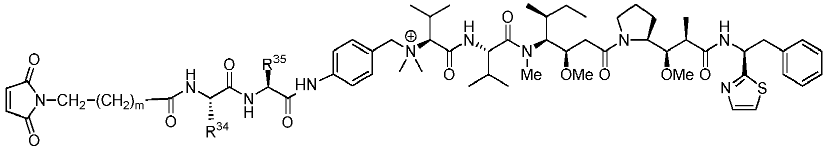

In any one of the above L_{b} or L_{b}'-containing moieties comprised of a quaternized auristatin drug unit corresponding in structure to D_{E}, D_{F}, D_{F-1}, D_{E-1}, D_{E-2} or D_{F/E-3}, A may contain 2 subunits wherein -A- in the above structures is replaced by -A₁-Aₒ-, wherein A₁ has the structure as defined above for A and wherein Aₒ corresponds in structure to another amine-containing acid. Preferred are -A₁-Aₒ- moieties that have the formula of -C(R^{a1})(R^{a2})-[C(R^{b1})(R^{b1})]ₘ-(C=O)-Aₒ-, wherein R^{a1} has the formula of - [C(R¹)(R¹)]-[C(R²)(R²)]ₙ-N(R²²)(R²³) wherein Aₒ is bonded to W and A₁ is bonded to Aₒ through amide functional groups and wherein the carbon having the R^{a1} and R^{a2} substituents is bonded to the imide nitrogen of an M¹ or M² moiety or to the amide nitrogen of an amide-acid M³ moiety. In more preferred embodiments -A₁-Aₒ- has the formula of -C(R^{a1})(R^{a2})-[C(R^{b1})(R^{b1})]ₘ-(C=O)-NH-(CH₂)_{m'}-C(=O)-, -C(R^{a1})(R^{a2})-[C(R^{b1})(R^{b1})]ₘ-(C=O)-NH-CH(CO₂H)-(CH₂)_{m'-1}-C(=O)-or-C(R^{a1})(R^{a2})-[C(R^{b1})(R^{b1})]ₘ-(C=O)-NH-(CH₂)_{m'-1}-CH(CO₂H)-C(=O)- wherein m is an integer ranging from 0 to 5 and m' is an integer ranging from 1 to 5 and wherein the other variable groups are as defined for A.In any one of the above L_{b}-A- or L_{b}'-A- containing moieties comprised of a quaternized auristatin drug unit corresponding in structure to D_{E}, D_{F}, D_{E-1}, D_{E-2} or D_{F}, D_{F1} or D_{F/E-3}, particularly preferred are those wherein L_{b}' is M¹, L_{b} is a M² or L_{b} is M^{3A} or M^{3B}, wherein M^{3A} and M^{3B} are regioisomers of the succinic acid-amide moiety M³ from hydrolytic ring opening of M², wherein M¹-A-, M²-A-, M^{3A}-A-, M^{3B}-A, M¹-A₁-Aₒ, M²-A₁-Aₒ, M^{3A}-A₁-Aₒ or M^{3B}-A₁-Aₒ has the structure of: 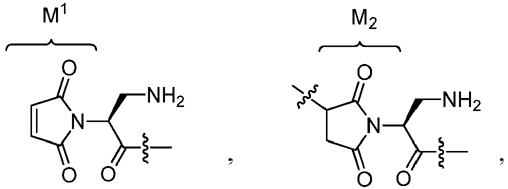 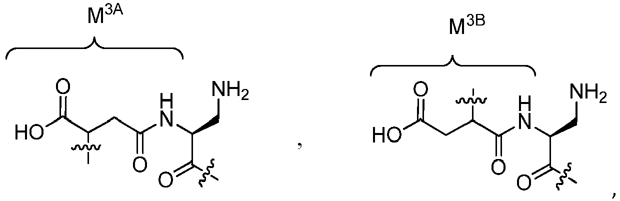 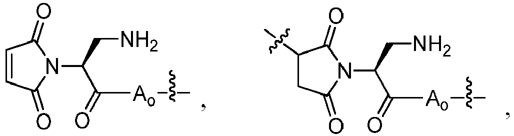 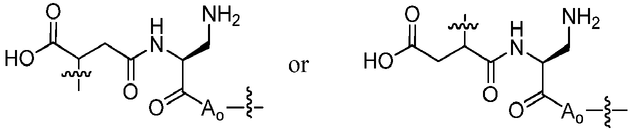 wherein the wavy lines to the carbonyl moieties indicate covalent bonding to W and the other wavy line indicates covalent bonding to a sulfhydryl group of a targeting moiety.

Preferred embodiments of Drug Linker compounds, wherein L_{b}' is M¹ and D⁺ is a quaternized auristatin and -A₁-A_{O}- has the structure of -C(R^{a1})(R^{a2})-[C(R^{b2})(R^{b1})]ₘ-(C=O)-NH-(CH₂)_{m'}-C(=O)-, have the structures of:

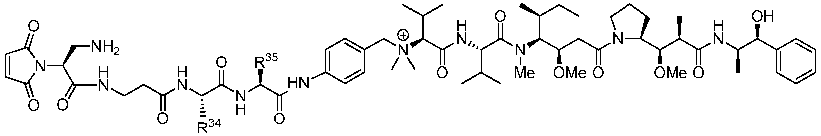

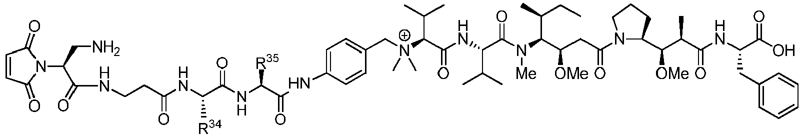

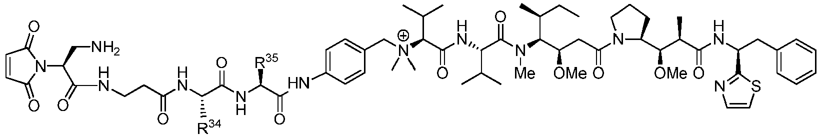

In other embodiments a Drug-Linker cpompound of formula L_{b}'-Lₒ-D⁺ is comprised of a quaternized dolastatin or an auristatin drug unit and a W' unit cleavable by a glycosidase wherein that L_{b}'-Lₒ-D⁺ moiety has the structure of: 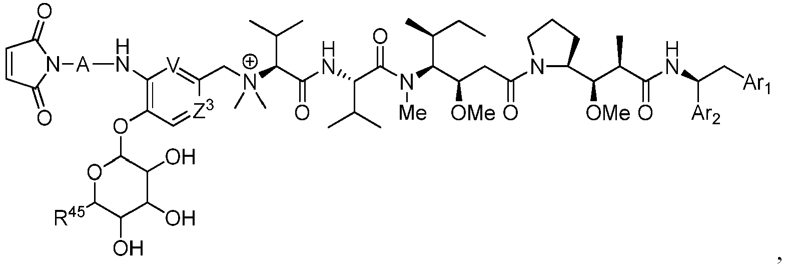 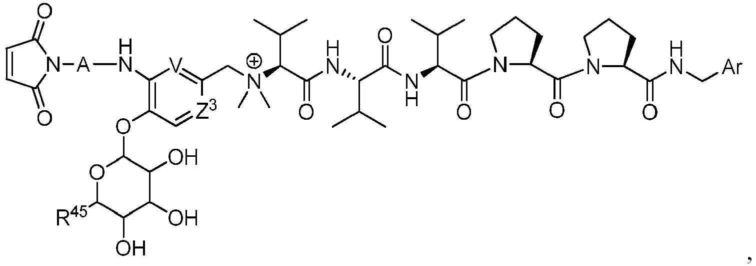 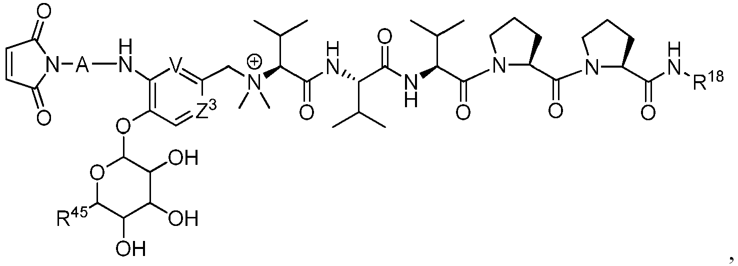 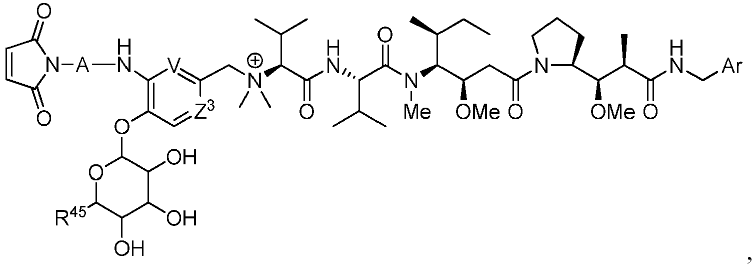 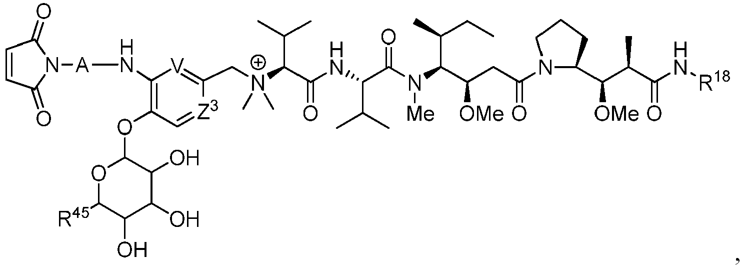 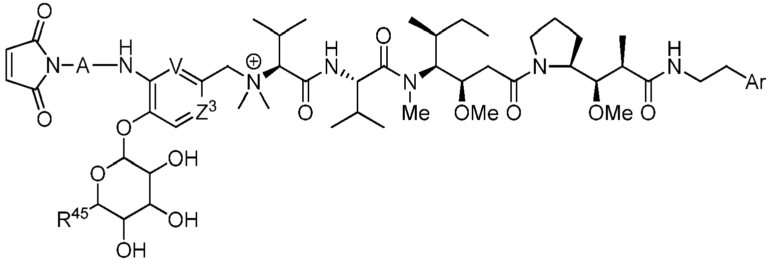 or 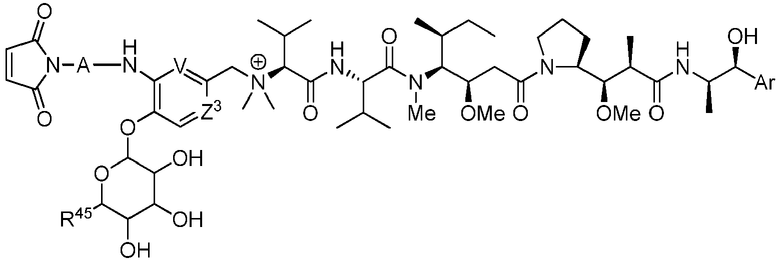 wherein L_{b}' is a maleimide moiety (M¹), A, V and Z³ are as previously defined for Formula 1 and SI unit embodiments, Ar, Ar₁, Ar₂ and R¹⁸ are as previously defined for auristatin-based quaternized drug units and R⁴⁵ is -CO₂H or CH₂OH, wherein the aniline nitrogen of SI as the Spacer unit Y is bonded to A through a carbonyl of an anilide functional group. In preferred embodiments one or both of V, Z³ is =CH-.

In any one of the above L_{b} or L_{b}'-containing moieties comprised of a quaternized auristatin drug and W' as a glycosidase cleavable unit (i.e., a Glucuronide Unit) A may contain 2 subunits wherein -A- in the above structures is replaced by -A₁-Aₒ-, wherein A₁ has the structure as defined above for A and wherein Aₒ corresponds in structure to another amine-containing acid. In more preferred embodiments a L_{b}'-Lₒ-D⁺ moiety comprised of a quaternized dolastatin or an auristatin drug unit has the structure of: 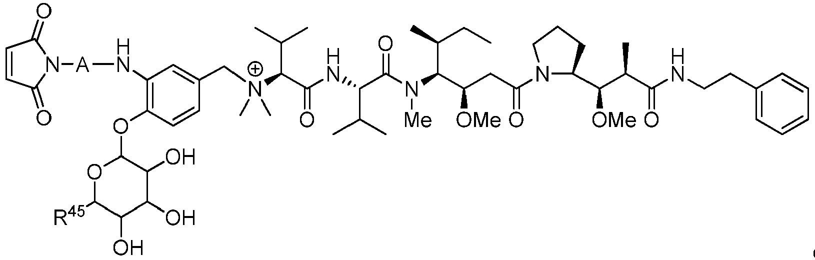 or

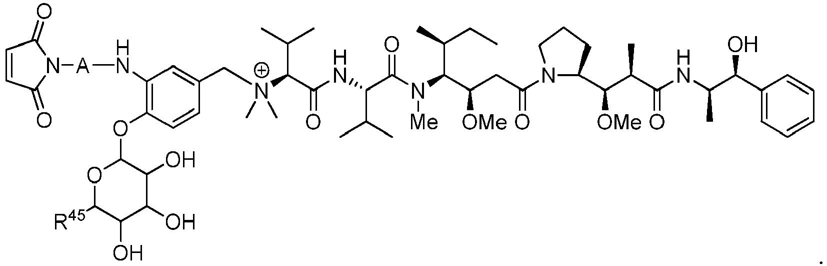

In other preferred embodiments L_{b}' (i.e., M¹) is replaced with a L_{b} moiety that is also bonded to a targeting moiety to provide an LDC wherein the L_{b} moiety is an M² or M³ moiety. In more preferred embodiments the carbohydrate moiety covalently attached to SI through a glycoside bond has its anomeric carbon in the β-configuration. In other more preferred embodiments R⁴⁵ is -CO₂H.

In embodiments having a Stretcher Unit and a Glucuronide Unit more preferred are those that the structures of :

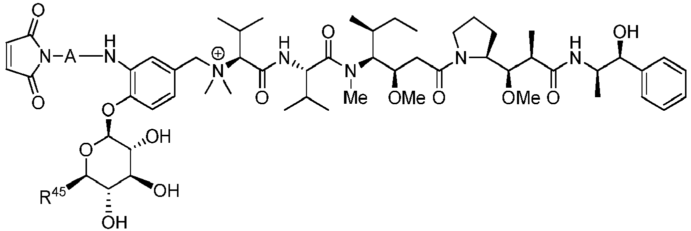

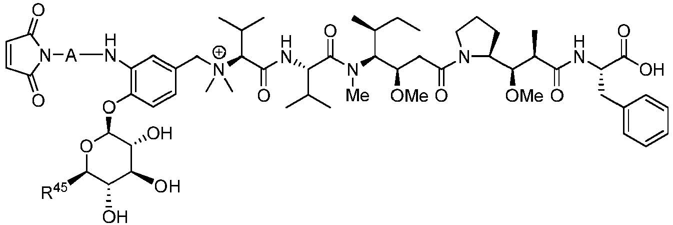

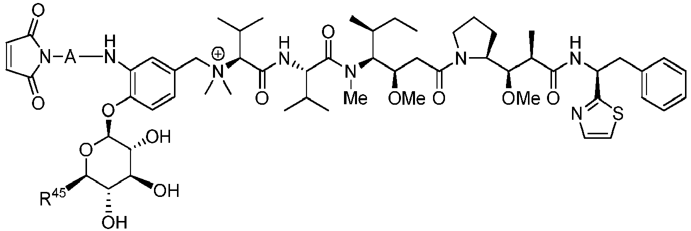

In embodiments having a Stretcher Unit comprised of M¹ and X¹ and a Glucuronide Unit more preferred are those that the structures of: 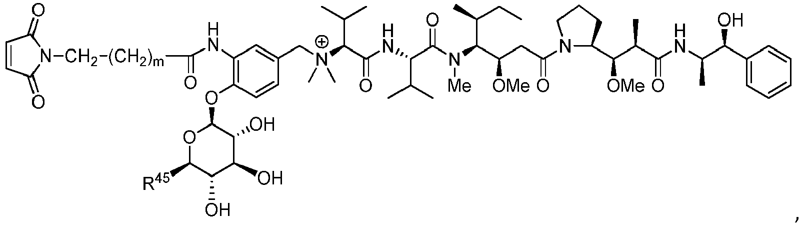 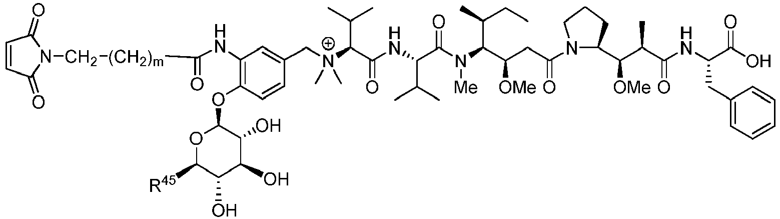 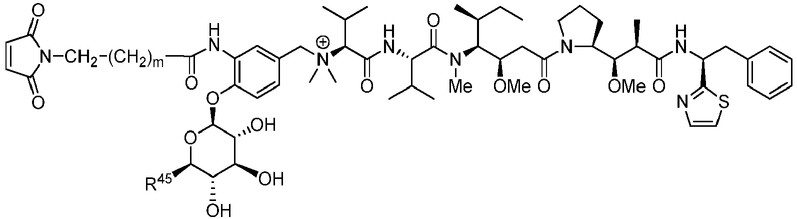 wherein R⁴⁵ is preferably -CO₂H and subscript m is 4.

In other preferred embodiments A in any one of the above embodiments is replaced by -A₁-Aₒ- as defined herein for L_{b} or L_{b}'-containing moieties comprised of a quaternized auristatin drug unit and a protease cleavable W unit with -A₁-Aₒ- having the formula of -C(R^{a1})(R^{a2})-[C(R^{b1})(R^{b1})]ₘ-(C=O)-NH-(CH₂)_{m'}-C(=O)-, -C(R^{a1})(R^{a2})-[C(R^{b})(R^{b})]ₘ-(C=O)-NH-CH(CO₂H)-(CH₂)_{m'-1}-C(=O)- or -C(R^{a1})(R^{a2})-[C(R^{b1})(R^{b1})]ₘ-(C=O)-NH-(CH₂)_{m'-1}-CH(CO₂H)-C(=O)-. Other preferred -A₁-Aₒ- moieties are as described for the corresponding quaternized tubulysin drug units.

In those embodiment preferred Drug Linker compounds have the structure of

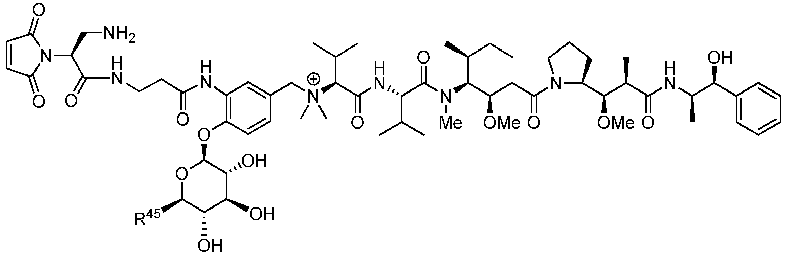

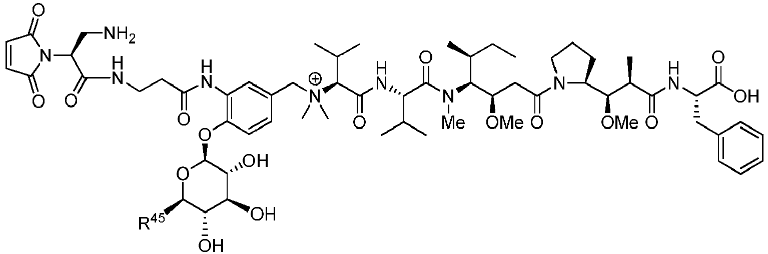

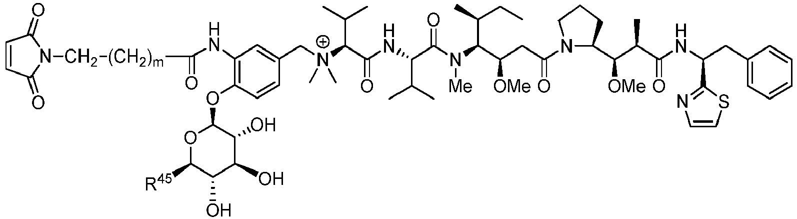

### 1.4.1 Quaternized tubulysins

In one group of embodiments, the quaternized drug corresponds in structure to a tubulysin having a tertiary amine at the N-terminus, wherein the nitrogen of that tertiary amine is incorporated into the quaternized drug unit.

In some embodiments, the quaternized drug is a tubulysin represented by structures of the following formula wherein the indicated nitrogen (†) is the site of quaternization when such compounds are incorporated into an LDC as a quaternized drug unit (D⁺): 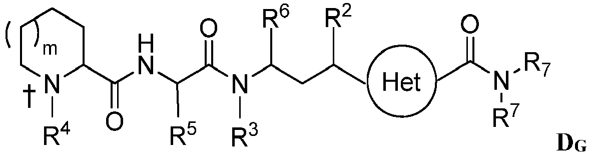 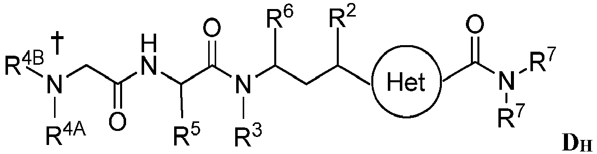 wherein the circle represents an 5-membered or 6-membered nitrogen heteroaryl wherein the indicated required substituents to that heteroaryl are in a 1,3- or meta-relationship to each other with optional substitution at the remaining positions; R² is X^{A}-R^{2A}, wherein X^{A} is -O-, -S-, -N(R^{2B})-, -CH₂-, -(C=O)N(R^{2B})- or -O(C=O)N(R^{2B})-wherein R^{2B} is hydrogen or optionally substituted alkyl, R^{2A} is hydrogen, optionally substituted alkyl, optionally substituted aryl, or -C(=O)R^{C}, wherein R^{C} is hydrogen, optionally substituted alkyl, or optionally substituted aryl or R² is an O-linked substituent; R³ is hydrogen or optionally substituted alkyl; R⁴, R^{4A}, R^{4B}, R⁵ and R⁶ are optionally substituted alkyl, independently selected, one R⁷ is hydrogen or optionally substituted alkyl and the other R⁷ is optionally substituted arylalkyl or optionally substituted heteroarylalkyl, and m is 0 or 1. In other embodiments the quaternized drug is a tubulysin represented by structure D_{G} wherein one R⁷ is hydrogen or optionally substituted alkyl, preferably lower alkyl, and the other R⁷ is an independently selected optionally substituted alkyl, preferably C₁-C₆ alkyl and m is 0 or 1, preferably 1, wherein the other variable group are as previously defined.

In some aspects, R² is X^{A}-R^{2A}, wherein X^{A} is -O-, -S-, -N(R^{2B})- -CH₂-, or - O(C=O)N(R^{2B})- wherein R^{2B} is hydrogen or optionally substituted alkyl, R^{2A} is hydrogen, optionally substituted alkyl, optionally substituted aryl, or -C(=O)R^{C}, wherein R^{C} is hydrogen, optionally substituted alkyl, or optionally substituted aryl or R² is an O-linked substituent.

In some aspects, R² is X^{A}-R^{2A}, wherein X^{A} is -O-, -S-, -N(R^{2B})- or -(C=O)N(R^{2B})- wherein R^{2A} and R^{2B} are independently hydrogen or optionally substituted alkyl, or R² is an O-linked substituent.

In other aspects -N(R⁷)(R⁷) in D_{G} or D_{H} is replaced by -N(R⁷)-CH(R¹⁰)(CH₂R¹¹) to define quaternized tubulysin drugs of formula D_{H}' and D_{G}': 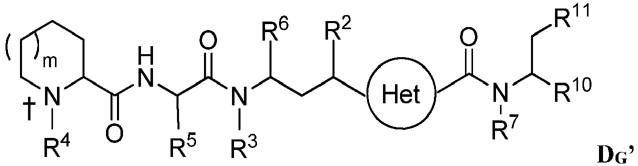 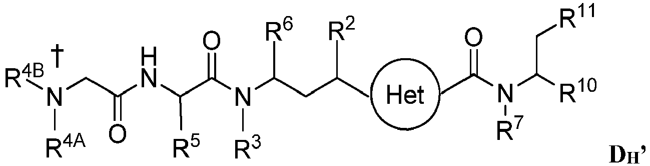 wherein R¹⁰ is C₁-C₆ alkyl substituted with -CO₂H, or ester thereof, and R⁷ is hydrogen or a C₁-C₆ alkyl independently selected from R¹⁰, or R⁷ and R¹⁰ together with the atoms to which they are attached define a 5 or 6-membered heterocycle; and R¹¹ is aryl or 5- or 6-membered heteroaryl, optionally substituted with one or more, preferably 1 or 2, more preferably 1, substituent(s) independently selected from the group consisting of halogen, lower alkyl, -OH and -O-C₁-C₆ alkyl, preferably -F, -CH₃, and -OCH₃; and the remaining variable groups are as defined for D_{G} and D_{H}.

In still other aspects one R⁷ in -N(R⁷)(R⁷) in D_{G} or D_{H} is hydrogen or C₁-C₆ alkyl, and the other R⁷ is an independently selected C₁-C₆ alkyl optionally substituted by - CO₂H or an ester thereof, or by an optionally substituted phenyl.

In some embodiments of structure D_{G} and D_{H}, one R⁷ is hydrogen and the other R⁷ is an optionally substituted arylalkyl having the structure of: 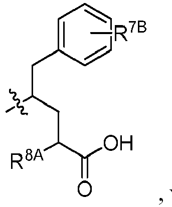 wherein R^{7B} is hydrogen or an O-linked substituent, preferably hydrogen or -OH in the *para* position, and R^{8A} is hydrogen or lower alkyl, preferably methyl; and wherein the wavy line indicates the point of attachment to the remainder of D_{G} or D_{H}.

In preferred embodiments of structure D_{G} or D_{H}, one R⁷ is hydrogen, and the other R⁷ is an optionally substituted arylalkyl having the structure of 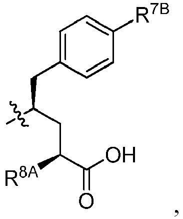 wherein R^{7B} is -H or -OH; and wherein the wavy line indicates the point of attachment to the remainder of D_{G} or D_{H}.

In other embodiments of structure D_{G} and D_{H}, one R⁷ is hydrogen or lower alkyl, preferably hydrogen or methyl, more preferably hydrogen, and the other R⁷ is optionally substituted arylalkyl having the structure of one of: 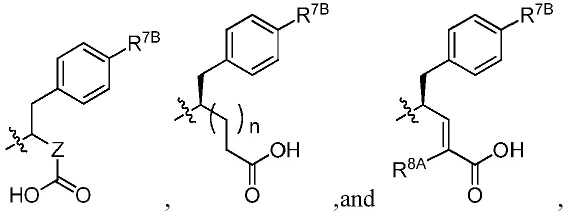 wherein Z is an optionally substituted alkylene or an optionally substituted alkenylene, R^{7B} is hydrogen or an O-linked substituent, preferably hydrogen or -OH in the *para* position, R^{8A} is hydrogen or lower alkyl, preferably methyl, and the subscript n is 0, 1 or 2, preferably 0 or 1; and wherein the wavy line indicates the point of attachment to the remainder of D_{G} or D_{H}.In still other embodiments of structure D_{G} and D_{H} -N(R⁷)(R⁷) is -NH(C₁-C₆ alkyl) wherein the C₁-C₆ alkyl is optionally substituted by -CO₂H or an ester thereof, or by an optionally substituted phenyl. In preferred embodiments -N(R⁷)(R⁷) is selected from the group consisting of -NH(CH₃), -CH₂CH₂Ph, and -CH₂-CO₂H, -CH₂CH₂CO₂H and - CH₂CH₂CH₂CO₂H.

In some embodiments of structure D_{G}' and D_{H}', R⁷ and R¹⁰ together with the atoms to which they are attached define an optionally substituted 5 or 6-membered heterocycle wherein -N(R⁷)-CH(R¹⁰)(CH₂R¹¹) has the structure of: 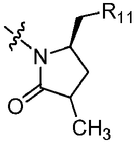 wherein the wavy line indicates the point of attachment to the remainder of D_{G}' or D_{H}'.

Some preferred tubulysins are represented by the following formula wherein the indicated nitrogen (†) is the site of quaternization when such compounds are incorporated into an LDC as a quaternized drug unit (D⁺): 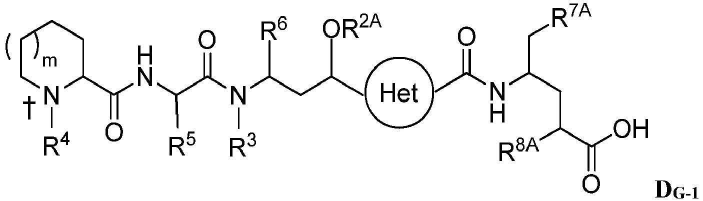 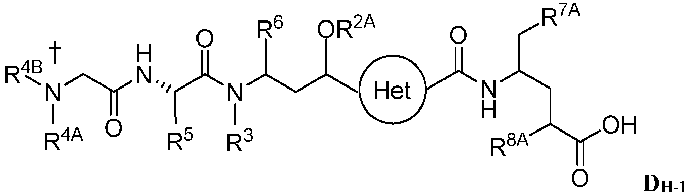 wherein the circle represents an 5-membered or 6-membered nitrogen-heteroaryl wherein the indicated required substituents to that heteroaryl are in a 1,3- or meta-relationship to each other with optional substitution at the remaining positions; R^{2A} is hydrogen or optionally substituted alkyl or R^{2A} along with the oxygen atom to which it is attached defines an O-linked substituent; R³ is hydrogen or optionally substituted alkyl; R⁴, R^{4A}, R^{4B}, R⁵ and R⁶ are optionally substituted alkyl, independently selected; R^{7A} is optionally substituted aryl or optionally substituted heteroaryl, R^{8A} is hydrogen or optionally substituted alkyl and m is 0 or 1.

In some preferred embodiments of structure D_{G}, D_{G-1}, D_{H}, or D_{H-1}, R⁴ is methyl or R^{4A} and R^{4B} are methyl. In other preferred embodiment of structure D_{G}' or D_{H}' R⁴ is methyl or R^{4A} and R^{4B} are methyl, In other preferred embodiments R^{7A} is optionally substituted phenyl. In other preferred embodiment R^{8A} is methyl in the (S)-configuration. In still other preferred embodiments R^{2A} along with the oxygen atom to which it is attached defines an O-linked substituent other than -OH, more preferably an ester, ether or an O-linked carbamate. In more preferred embodiments the circle represents a 5-membered nitrogen-heteroarylene with a divalent oxazole or thiazole moiety particularly preferred. In other preferred embodiments R⁴ is methyl or R^{4A} and R^{4B} are methyl. In other preferred embodiments R⁷ is optionally substituted arylalkyl, wherein aryl is phenyl and R^{7A} is optionally substituted phenyl.
In other embodiments of D_{G}, D_{G}', D_{G-1}, D_{H}, D_{H}' or D_{H-1} the circle represents a 5-membered nitrogen heteroarylene, preferably represented by the structure 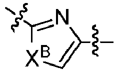 wherein X^{B} is O, S, or N-R^{B} wherein R^{B} is hydrogen or lower alkyl. Preferably the quaternized drug is a tubulysin represented by structure D_{G}, D_{G}' or D_{G-1}, wherein m is 1. More preferred are tubulysins represented by structure D_{G}, wherein m is 1 and the circle represents an optionally substituted divalent thiazole moiety.

Other preferred tubulysins are represented by the following formula wherein the indicated nitrogen (†) is the site of quaternization when such compounds are incorporated into an LDC as a quaternized drug unit (D⁺): 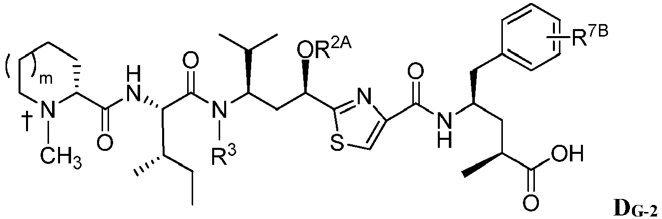 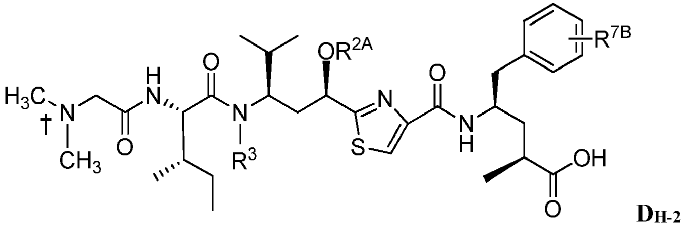
wherein R^{2A} along with the oxygen atom to which it is attached defines an O-linked substituent, preferably an ester, ether or O-linked carbamate, R³ is lower alkyl or - CH₂OC(=O)R^{3A} wherein R^{3A} is optionally substituted lower alkyl, and R^{7B} is hydrogen or an O-linked substituent, preferably in the *para* position. In more preferred embodiments, R³ is methyl or R^{3A} is methyl, ethyl, propyl, iso-propyl, iso-butyl or -CH₂C=(CH₃)₂. In other preferred embodiments R^{2A} is methyl, ethyl, propyl (i.e., -OR^{2A} is an ether) or is - C(=O)R^{2B} (i.e., -OR^{2A} is an ester) wherein R^{2B} is lower alkyl with R^{2B} as methyl (i.e., - OR^{2A} is acetate) more preferred. More preferred tubulysins having an N-terminal tertiary amine as the site of conjugation have the structure of one of the following formulae: 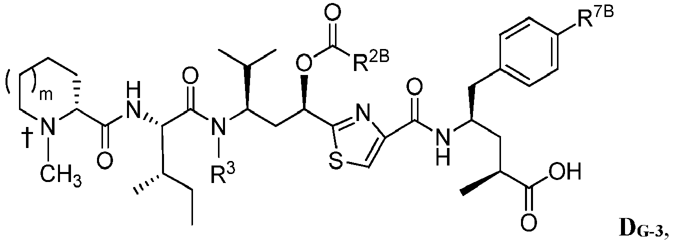 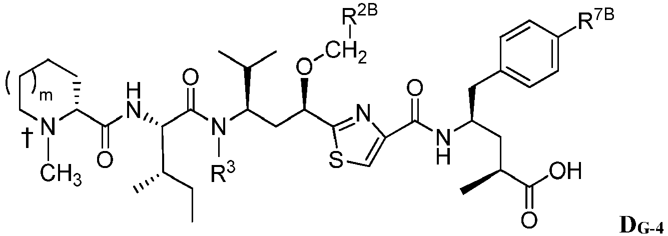 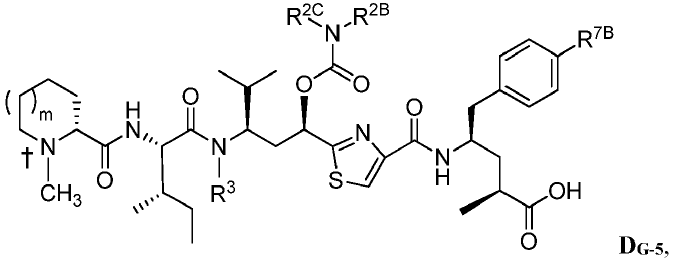
wherein R^{7B} is hydrogen or -OH, R³ is lower alkyl, preferably methyl or ethyl, and R^{2B} and R^{2C} are independently hydrogen or lower alkyl. In some preferred embodiments of any one of structures D_{G}, D_{G-1}, D_{G-2}, D_{G-3}, D_{G-4}, D_{G-5}, D_{H}, D_{H-1} and D_{H-2}, R³ is methyl or is -CH₂OC(=O)R^{3A}, wherein R^{3A} is optionally substituted alkyl. In other preferred embodiments of any one of structures D_{G}' and D_{H}', R³ is methyl or is - CH₂OC(=O)R^{3A}, wherein R^{3A} is optionally substituted alkyl. In other preferred embodiments of any one of those structures R³ is -C(R^{3A})(R^{3A})C(=O)-X^{C}, wherein X^{C} is - OR^{3B} or -N(R^{3C})(R^{3C}), wherein each R^{3A}, R^{3B} and R^{3C} independently is hydrogen, optionally substituted alkyl or optionally substituted cycloalkyl. Preferably R³ is - C(R^{3A})(R^{3A})C(=O)-N(R^{3C})(R^{3C}), with each R^{3A} hydrogen, one R^{3C} hydrogen and the other R^{3C} n-butyl or isopropyl as more preferred.

In other preferred embodiments of any one of structures D_{G}, D_{G}' D_{G-1}, D_{G-2}, D_{G-3}, D_{G-4}, D_{G-5}, D_{H}, D_{H}', D_{H-1} and D_{H-2}, R³ is ethyl or propyl
In other preferred embodiments of any one of structures D_{G-1}, D_{G-2}, D_{G-3}, D_{G-4}, D_{G-5}, D_{G-6}, D_{H-1} and D_{H-2}, the thiazole core heterocycle 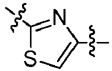 is replaced with

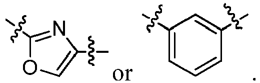

In some preferred embodiments of any one of structures D_{G}, D_{G-1}, D_{G-2}, D_{G-3}, D_{G-4}, D_{G-5}, D_{H}, D_{H-1}, D_{H-2}, D_{H-3} and D_{H-4}, R³ is methyl or is -CH₂OC(=O)R^{3A}, wherein R^{3A} is optionally substituted alkyl. In other preferred embodiments of any one of those structures R³ is -C(R^{3A})(R^{3A})C(=O)-X^{C}, wherein X^{C} is -OR^{3B} or -N(R^{3C})(R^{3C}), wherein each R^{3A}, R^{3B} and R^{3C} independently is hydrogen, optionally substituted alkyl or optionally substituted cycloalkyl. Preferably R³ is -C(R^{3A})(R^{3A})C(=O)-N(R^{3C})(R^{3C}), with each R^{3A} hydrogen, one R^{3C} hydrogen and the other R^{3C} n-butyl or isopropyl more preferred.

In other preferred embodiments of any one of structures D_{G-3}, D_{G-4}, D_{G-5}, D_{H-3} and D_{H-4}, the thiazole core heterocycle 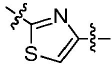 is replaced with

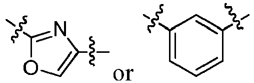

In more preferred embodiments the tubulysin has structure D_{G-3} or D_{G-4} wherein m is 1, R³ is optionally substituted methyl, ethyl or propyl, preferably methyl, ethyl or propyl.

In particularly preferred embodiments the tubulysin has structure D_{G-3}, wherein m is 1, R³ is methyl, ethyl or propyl, -OC(O)R^{2B} is -O-C(O)H, O-C(O)-C₁-C₆ alkyl or -OC₂-C₆ alkenyl, optionally substituted, preferably -OC(O)CH₃, -OC(O)CH₂CH₃, -OC(O)CH(CH₃)₂, -OC(O)C(CH₃)₃, or -OC(O)CH=CH₂.

In other particularly preferred embodiments the tubulysin has structure D_{G-4}, wherein m is 1, R³ is methyl, ethyl or propyl and -OCH₂R^{2B} is -OCH₃, -OCH₂CH₃, - OCH₂CH₂CH₃ or -OCH₂OCH₃.

Especially preferred tubulysins having an N-terminal tertiary amine as the site of conjugation have the structure of 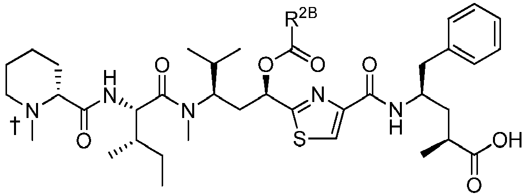 or 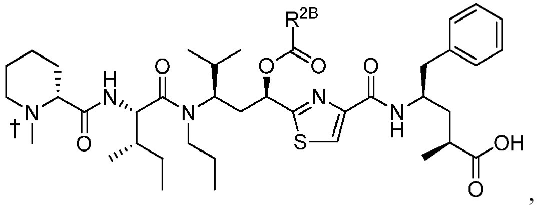 wherein R^{2B} is -CH₃, -CH₂CH₃, -CH₂CH₂CH₃, -CH(CH₃)₂, -CH₂CH(CH₃)₂, - CH₂C(CH₃)₃ and the indicated nitrogen (†) is the site of quaternization when such compounds are incorporated into an LDC as a quaternized drug unit (D⁺).

Other, especially preferred tubulysins having an N-terminal tertiary amine as the site of conjugation have the structure of 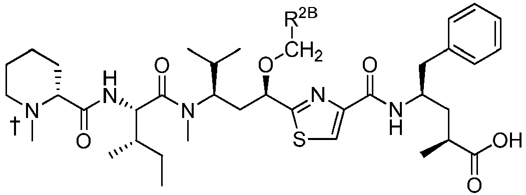 or 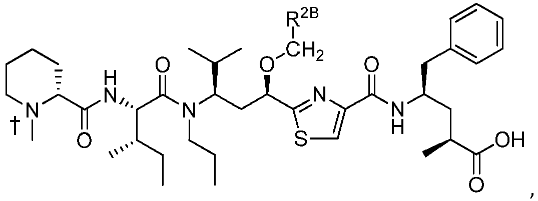 wherein R^{2B} is hydrogen, methyl or -OCH₃ (i.e., -OCH₂R^{2B} is a methyl ethyl, methoxymethyl ether substituent).

In other preferred embodiments the tubulysin incorporated as D⁺ in an LDC is a naturally occurring tubulysin including Tubulysin A, Tubulysin B, Tubulysin C, Tubulysin D, Tubulysin E, Tubulysin F, Tubulysin G, Tubulysin H, Tubulysin I, Tubulysin U, Tubulysin V, Tubulysin W, Tubulysin X or Tubulysin Z, whose structures are given by the following structure and variable group definitions wherein the indicated nitrogen (†) is the site of quaternization when such compounds are incorporated into an LDC as a quaternized drug unit (D⁺):

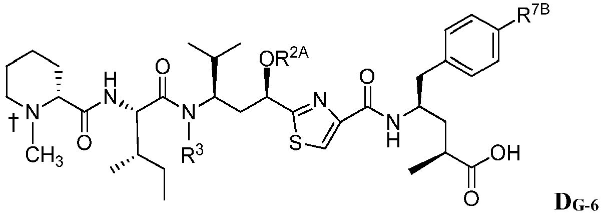

**TABLE 1. Some Naturally Occurring Tubulysins**

| **Tubulysin** | **R^{7B}** | **R^{2A}** | **R³** |
|---|---|---|---|
| **A** | OH | C(=O)CH₃ | CH₂OC=O)i-Bu |
| **B** | OH | C(=O)CH₃ | CH₂OC=O)n-Pr |
| **C** | OH | C(=O)CH₃ | CH₂OC=O)Et |
| **D** | H | C(=O)CH₃ | CH₂OC=O)i-Bu |
| **E** | H | C(=O)CH₃ | CH₂OC=O)n-Pr |
| **F** | H | C(=O)CH₃ | CH₂OC=O)Et |
| **G** | OH | C(=O)CH₃ | CH₂OC=O)CH=CH₂ |
| **H** | H | C(=O)CH₃ | CH₂OC=O)Me |
| **I** | OH | C(=O)CH₃ | CH₂OC=O)Me |
| **U** | H | C(=O)CH₃ | H |
| **V** | H | OH | H |
| **Z** | OH | OH | H |

In particularly preferred embodiments of structure D_{G-6} the quaternized tubulysin is Tubulysin M, wherein R³ is -CH₃, R² is C(=O)CH₃ and R^{7B} is hydrogen.

In some embodiments a L_{b}-, or its L_{b}'-containing precursor, comprised of Lₒ and D⁺ incorporates a tubulysin drug into the quaternary amine unit wherein L_{b}-, or its L_{b}'-containing precursor has a structure corresponding to any one of the -L_{R}-D⁺, L_{b}-Lₒ-D⁺ or L_{b}'-Lₒ-D⁺, L_{SS}-Lₒ-D⁺, L_{S}-D⁺, M¹-A, M²-A, M³-A, M¹-A₁, M²-A₁, M³-A₁ M¹-A(BU), M²-A(BU), M³-A(BU), M¹-A₁(BU), M²-A₁(BU) or M³-A₁(BU) moieties described herein wherein D⁺ quaternized auristatin drug. In preferred embodiments a tubulysin having the structure of D_{G}, D_{G-1}, D_{G-2}, D_{G-3}, D_{G-4}, D_{G-5}, D_{G-6}, D_{H}, D_{H-1}, D_{H-2}, D_{H-3} or D_{H-4} replaces D_{E}, D_{E-1}, D_{E-2}, D_{F}, D_{F-1} or D_{F/G-3} in in each embodiment having that auristatin structure at the indicated (†) tertiary amine nitrogen.

Thus, some preferred -L_{b}-Lₒ-D⁺ or L_{b}'-Lₒ-D⁺ embodiments have the structure of: 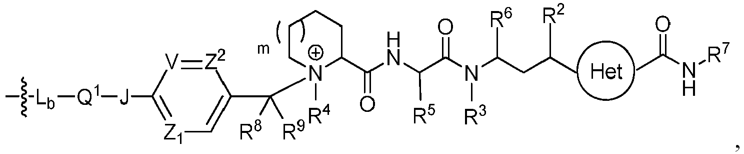 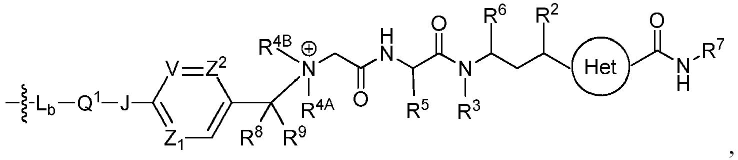 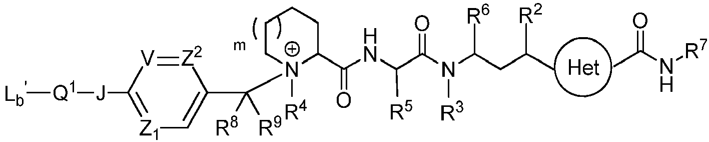 or 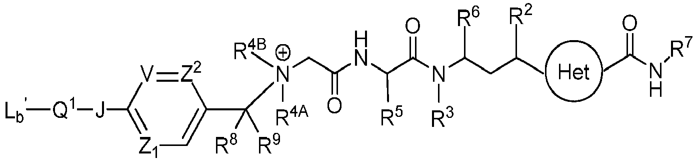 wherein R², R³, R⁴, R^{4A}, R^{4B}, R⁵, R⁶ and R⁷ are as described for tubulysin drugs in free form by D_{G} and D_{H}, wherein L_{b}, L_{b}', Q₁, V, Z¹ and Z² are as previously described for embodiments of L_{b} and L_{b}'-containing moieties comprised of a quaternized auristatin drug unit and Lₒ and Y units thereof comprised or consisting of a SI unit, and J is -O-, -S- or N(R³³)-, wherein R³³ is hydrogen or optionally substituted alkyl.

Other preferred -L_{b}-Lₒ-D⁺ or L_{b}'-Lₒ-D⁺ embodiments have the structure of: 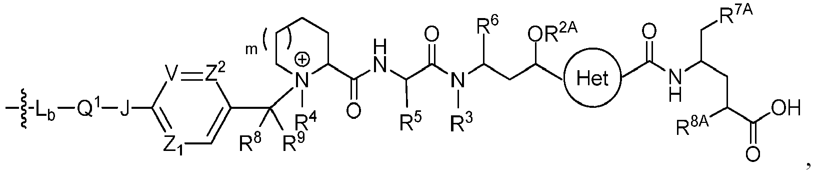 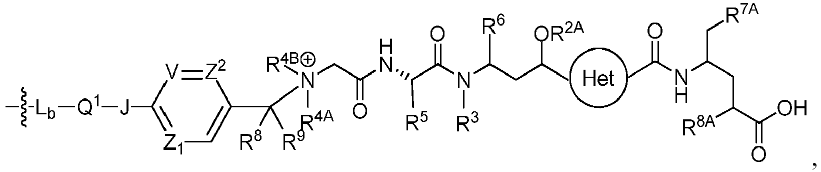 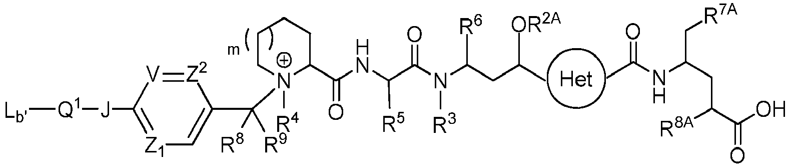 or 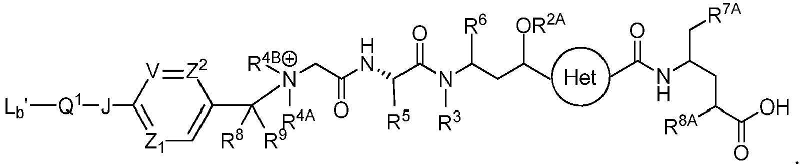 wherein R^{2A}, R³, R⁴, R^{4A}, R^{4B}, R⁵, R⁶, R^{7A} and R^{8A} are as described for tubulysin drugs in free form by D_{G-1} and D_{H-1}, wherein L_{b}, L_{b}', Q₁, V, Z¹ and Z² are as previously described for embodiments of L_{b} and L_{b}'-containing moieties comprised of a quaternized auristatin drug unit and Lₒ and Y units thereof comprised or consisting of a SI unit, and J is -O-, -S- or N(R³³)-, wherein R³³ is hydrogen or optionally substituted alkyl.

In more preferred embodiments R^{7A} is phenyl, optionally substituted with -OH or R^{8A} is methyl. In other more preferred embodiments R⁴ or R^{4A} and R^{4B} are methyl. In still other preferred embodiments J is -O-.

In other preferred embodiments -L_{b}-Lₒ-D⁺ or L_{b}'-Lₒ-D⁺ has the structure of: 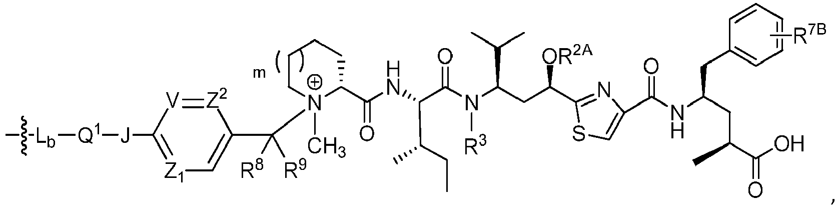 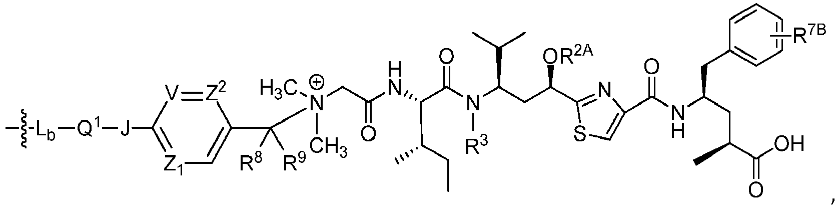 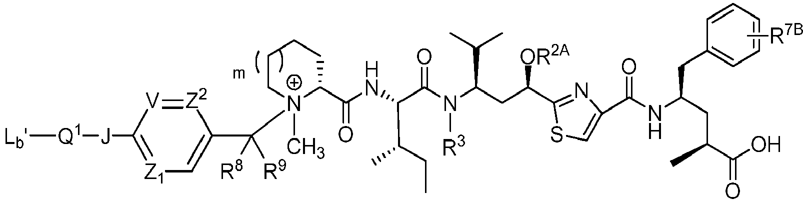 or

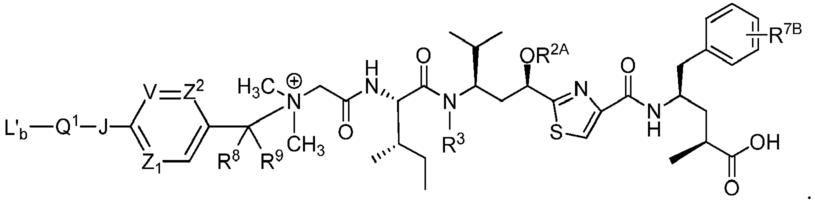

In more preferred embodiments R^{7A} is hydrogen or -OH in the *para* position.

In any one of the above L_{b} or L_{b}'-containing moieties comprised of a quaternized tubulysin drug unit, L_{b}' is a maleimide moiety (M¹) or L^{b} is a succinimide (M²) or succinic acid-amide (M³) moiety.

In preferred embodiments of any one of the above L_{b} or L_{b}'-containing moieties comprised of a quaternized tubulysin drug, R⁸ and R⁹ are hydrogen. In other preferred embodiments V, Z¹ and Z² are =CH-. In more preferred embodiments -OR² is an ester or R³ is lower alkyl. In other more preferred embodiments J is -NH-. In still more preferred embodiments Q¹ is comprised of a dipeptide covalently bound to J through an amide functional group that is cleavable by an intracellular or regulatory protease so proteolysis of that group releases an SI capable of 1,6-fragmentation.

In any one of the above embodiments comprised of a quaternized tubulysin drug unit more preferred embodiments are those where L_{b}' (i.e., M¹) is replaced with a L_{b} moiety that is also bonded to a targeting moiety to provide an LDC wherein that L_{b} moiety is an M² or M³ moiety.

Accordingly, more preferred embodiments of L_{b}-Lₒ-D⁺ or L_{b}'-Lₒ-D⁺ have the structure of one of: 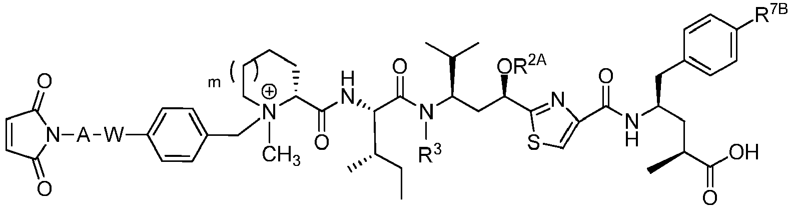 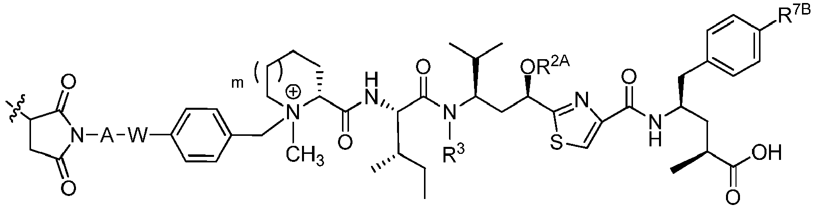 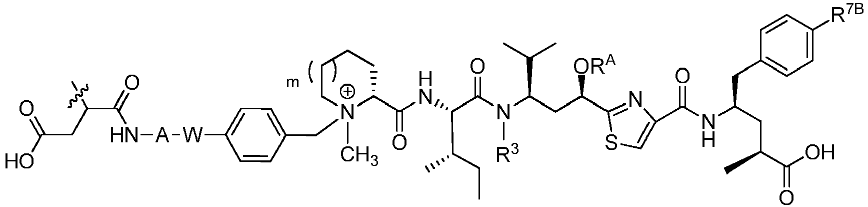 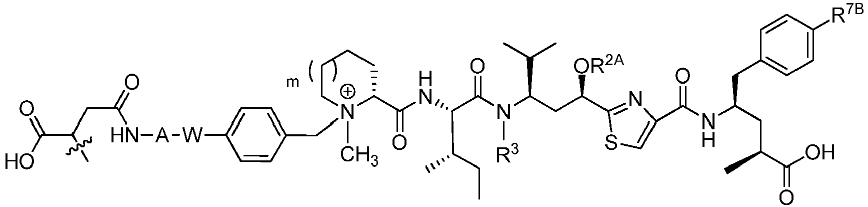 wherein the wavy line indicates covalent binding of a M² or M³ moiety to a sulfhydryl group of a targeting moiety. In preferred embodiments -OR^{2A} is a C₂-C₆ ester or R³ is lower alkyl and R^{7B} is hydrogen or -OH. In other more preferred embodiments - R^{2A} is optionally substituted lower alkyl, R³ is lower alkyl, independently selected from R^{2A}, and R^{7B} is hydrogen or -OH. In particularly preferred embodiments R³ is methyl, ethyl or propyl and R^{2A} is -C(O)CH₃, methyl, ethyl, propyl or -CH₂OCH₃.

In other preferred embodiments L_{b}'-Lₒ-D⁺ or L_{b}-Lₒ-D⁺ has the structure of: 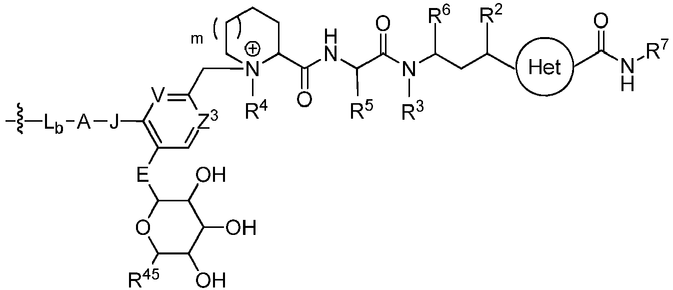 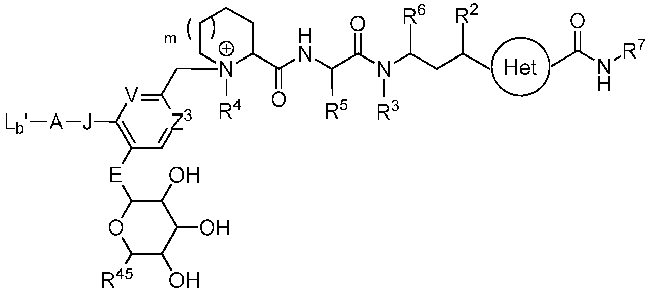 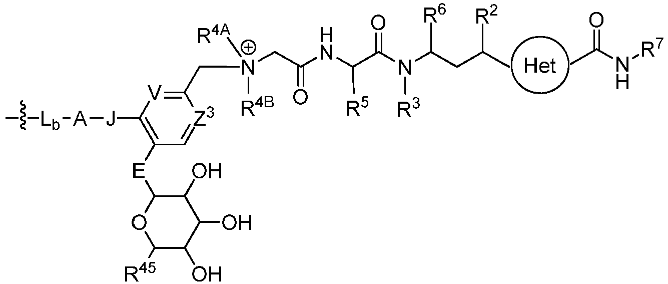 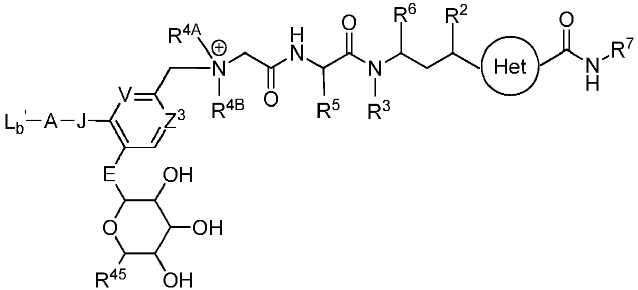 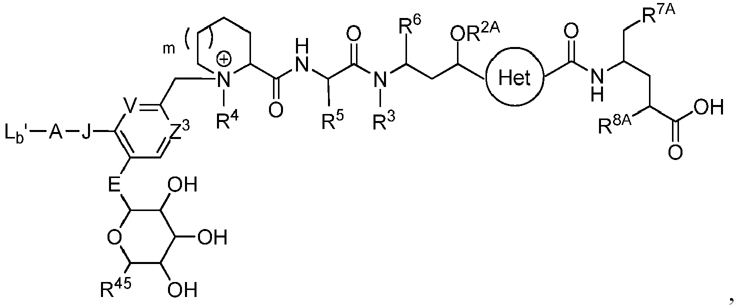 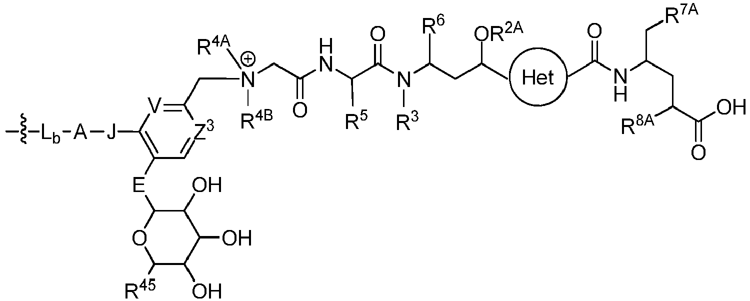 wherein R², R^{2A}, R³, R⁴, R^{4A}, R^{4B}, R⁵, R⁶, R⁷, R^{7A} an R^{8A} are as described for tubulysin drugs in free form in structure D_{G}, D_{G}', D_{G-1}, D_{H}, D_{H}' or D_{H-1}, L_{b}, L_{b}', A, V and Z³ are as previously described for the corresponding L_{b} and L_{b}'-containing moieties comprised of a quaternized auristatin drug unit and Lₒ and Y units thereof comprised or consisting of a SI unit; E and J are independently -O-, -S- or -N(R³³), wherein R³³ is hydrogen or optionally substituted alkyl; and R⁴⁵ is CO₂H or CH₂OH. In more preferred embodiments J is -NH-. In other preferred embodiments E is -O-.

More preferred are those embodiments where L_{b}' is a maleimide (M¹) moiety or L_{b} is a succinimide (M²) or amide-acid (M³) moiety.

In other more preferred embodiments L_{b}'-Lₒ-D⁺ has the structure of: 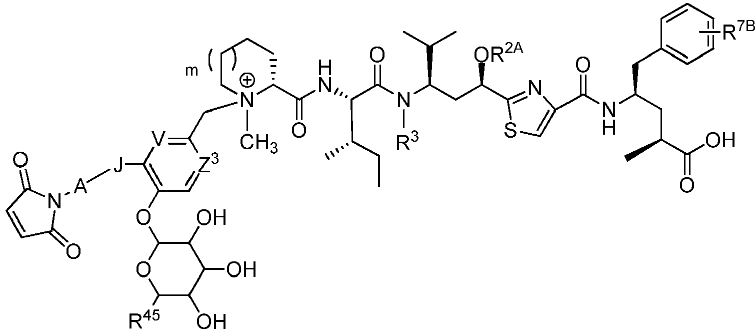 or 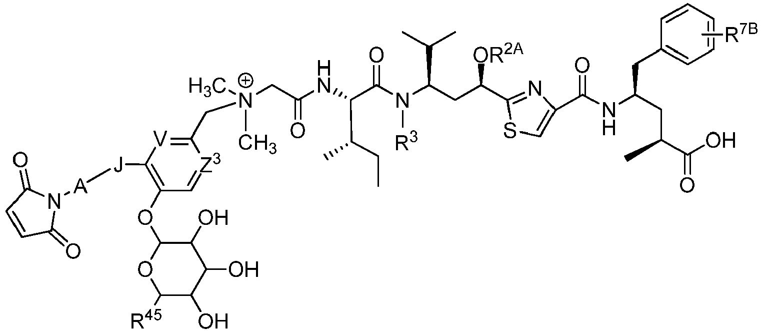 wherein A, R^{2A}, R³, R⁴⁵, R^{7B} and R⁴⁵ are as previously defined. In more preferred embodiments one or both of V, Z³ are =CH-.

In more preferred embodiments a L_{b}'-Lₒ-D⁺ or -L_{b}-Lₒ-D⁺ moiety comprised of a quaternized tubulysin drug unit has the structure of: 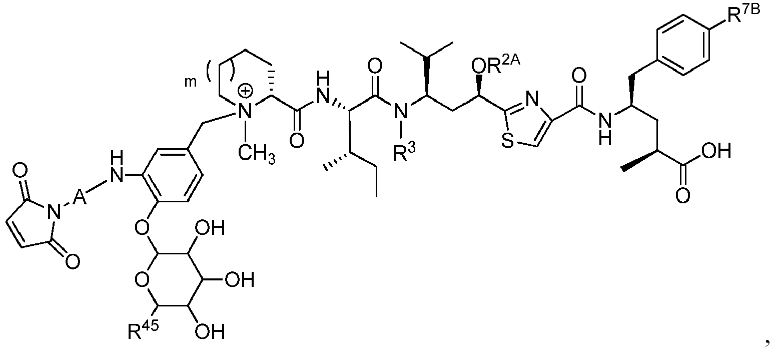 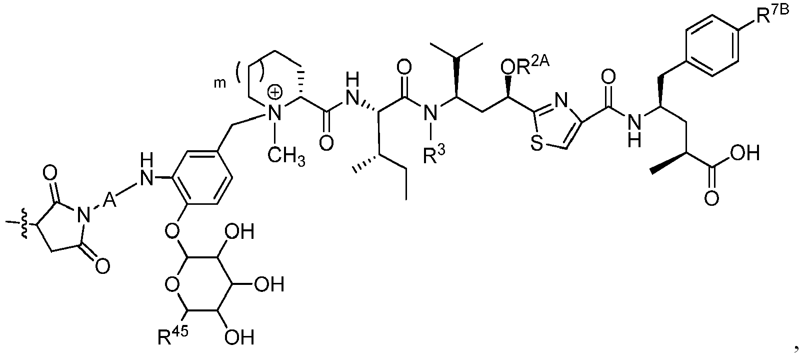 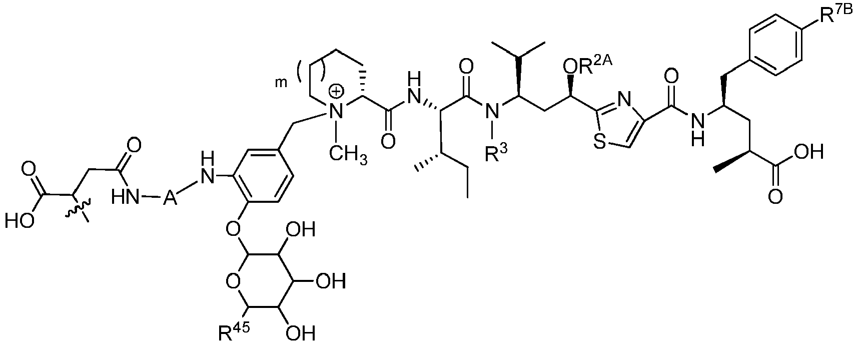 or 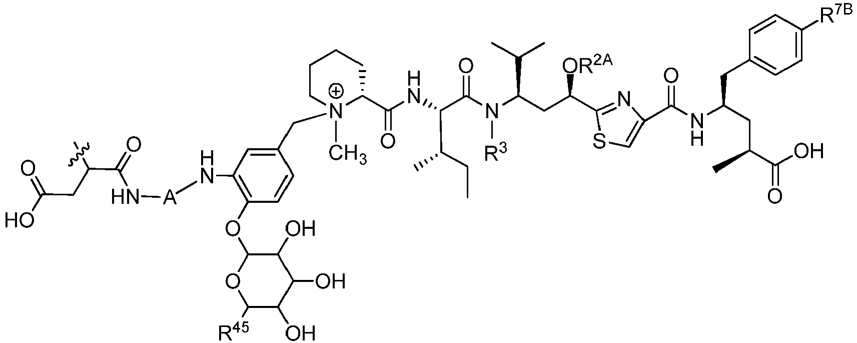 wherein the wavy line indicates covalent bonding to a sulfhydryl group of a targeting moiety. In more preferred embodiments the carbohydrate moiety covalently attached to SI through a glycoside bond has its anomeric carbon in the β-configuration. In other more preferred embodiments R⁴⁵ is -CH₂OH or -CO₂H.

In any one of the above embodiments for L_{b}-, L_{b}'-, M¹-, M²- or M³-containing moiety comprised of a quaternized tubulysin drug, R³ is preferably methyl or R² is preferably acetate or m is preferably 1. Also, preferred for such L_{b}-, L_{b}'-, M¹-, M²- or M³-containing moieties are those wherein R³ is methyl, ethyl or propyl and -OR^{2A} is - OC(O)CH₃, -OCH₃, -OCH₂CH₃ or -OCH₂CH₂CH₃.

Particularly preferred embodiments have the structure of: 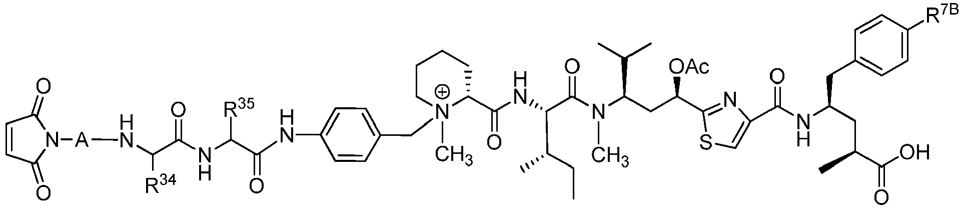 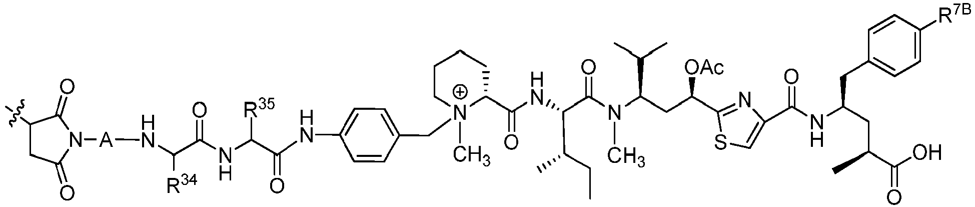 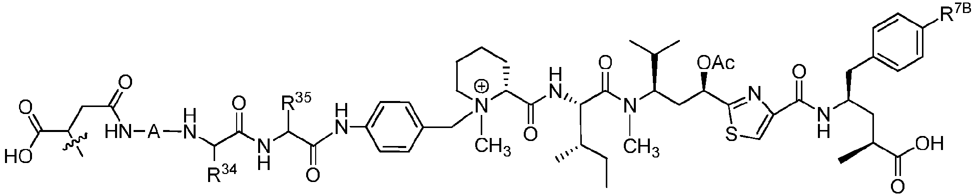 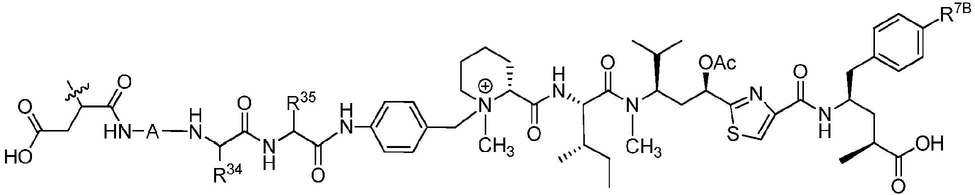 wherein the wavy line indicates covalent bonding to a sulfhydryl group of a targeting moiety and wherein R³⁴ is benzyl, methyl, isopropyl, isobutyl, sec-butyl, - CH(OH)CH₃ or has the structure of 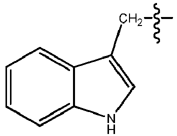 and R³⁵ is methyl, -(CH₂)₄-NH₂, - (CH₂)₃NH(C=O)NH₂, (CH₂)₃NH(C=NH)NH₂, or -(CH₂)₂CO₂H, and R^{7B} is hydrogen or - OH.

Other particularly preferred embodiments have the structure of: 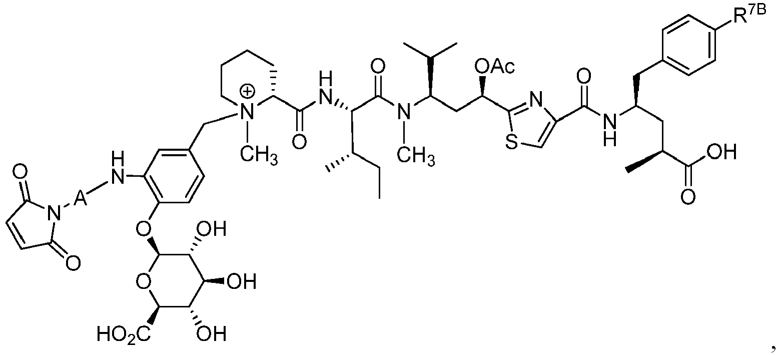 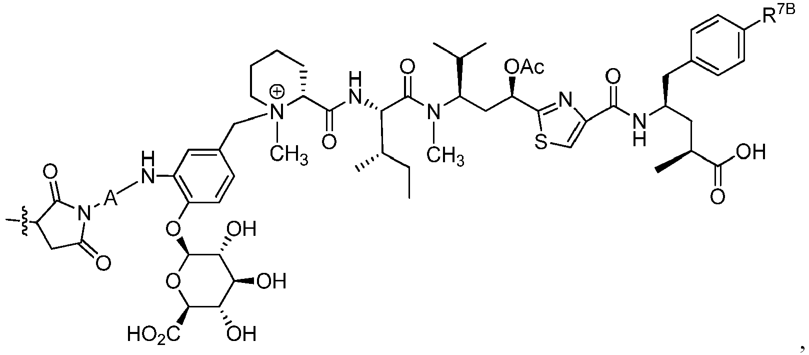 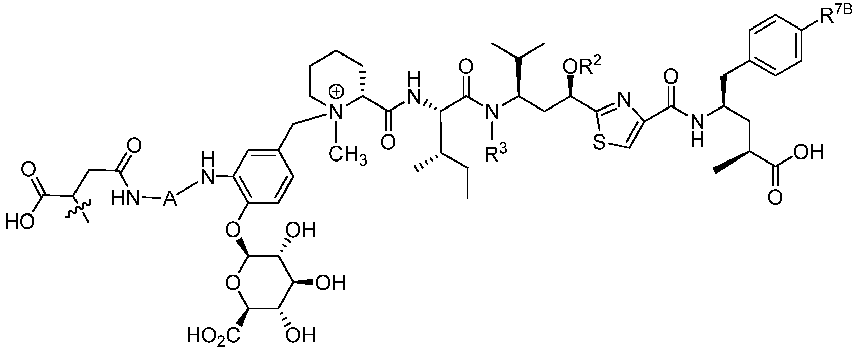 or 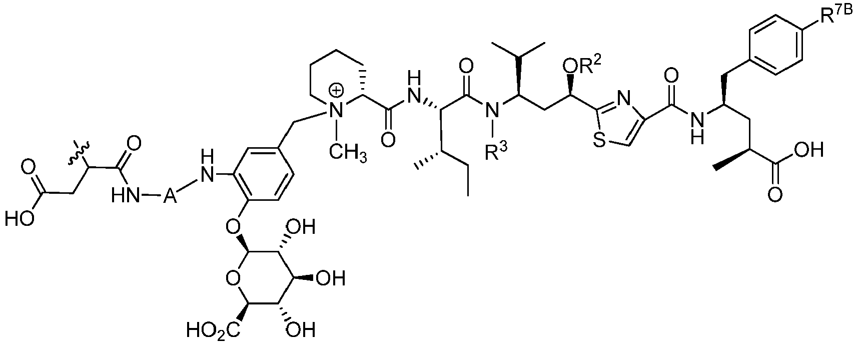 wherein R² and R³ independently are methyl, ethyl or propyl or R² is - C(O)CH₃ and R³ is methyl, ethyl or propyl.

In any one of the L_{b}-containing moieties comprised of a quaternized tubulysin drug, particularly preferred are those where the bonded targeting moiety is an antibody and in any one of the M²- or M³- containing moieties comprised of a quaternized tubulysin drug, particularly preferred are those where the bonded sulfhydryl group is from an antibody targeting moiety.

In any one of the structures for a an L_{b}- or L_{b}'-containing moiety comprised of A, A is replaced with -A₁-Aₒ-, wherein those subunits of A are as described for such moieties when present in corresponding L_{b}- or L_{b}'-containing moieties comprised of a quaternized auristatin drug unit and Lₒ having a stretcher unit. Other such moieties are further described below for stretcher units in M¹-A, M²-A, M^{3A}-A and M^{3B}.

For any of one of the L_{b}-, L_{b}', M¹, M² or M³-containing moieties comprised of a quaternized tubulysin drug, a preferred Stretcher group corresponds in structure to an amine-containing acid as defined herein with γ-aminocaproic acid more preferred. Thus, one preferred Stretcher unit has the structure of -(CH₂)₅-C(=O)-. Other preferred stretcher groups have the formula of -C(R^{a1})(R^{a2})-[C(R^{b1})(R^{b1})]m-(C=O)-NH-(CH2)_{m'}-C(=O)-, - C(R^{a1})(R^{a2})-[C(R^{b1})(R^{b1})]m-(C=O)-NH-CH(CO₂H)-(CH₂)_{m'-1}-C(=O)- or -C(R^{a1})(R^{a2})-[C(R^{b1})(R^{b1})]ₘ-(C=O)-NH-(CH₂)_{m'-1}-CH(CO₂H)-C(=O)- wherein m is an integer ranging from 0 to 5 and m' is an integer ranging from 1 to 5 wherein R^{a1} is hydrogen or has the formula of -[C(R¹)(R¹)]-[C(R²)(R²)]ₙ-N(R²²)(R²³) and wherein the other variable groups are as previously defined. For those stretcher units R^{a1} is -CH₂NH₂ is preferred.

More preferred are those stretcher units providing M¹-A, M²-A, M^{3A}-A and M^{3B} moieties having the structures of:

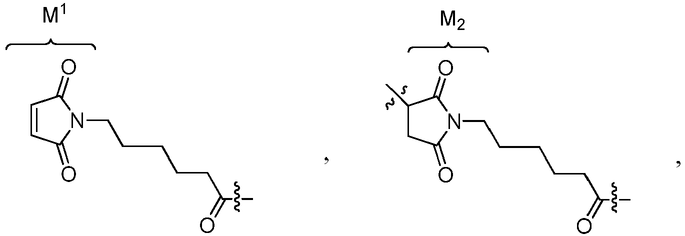

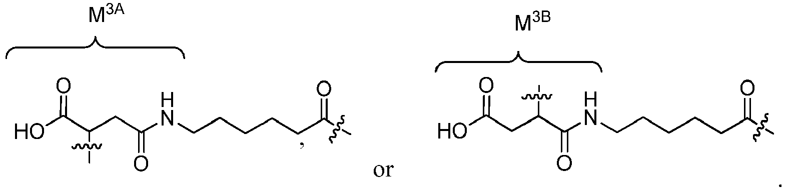

Other more preferred stretcher units are those providing M¹-A₁-Aₒ-, M²-A₁-Ao, M^{3A}-A₁-Aₒ- and M^{3B}-A₁-Aₒ- moieties having the structures of:

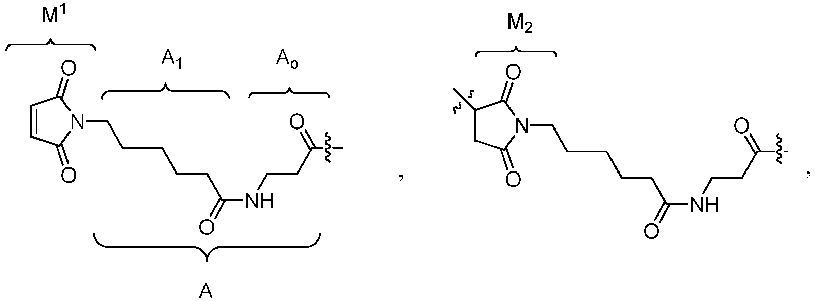

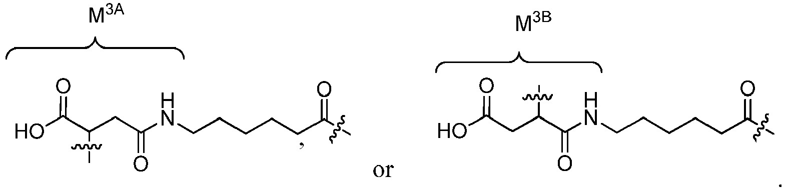

Still other more preferred stretcher units are those providing M¹-A₁-Aₒ-, M²-A₁-Aₒ, M^{3A}-A₁-Aₒ- and M^{3B}-A₁-Aₒ- moieties having the structures of:

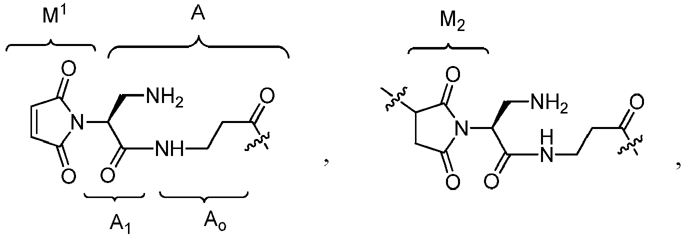

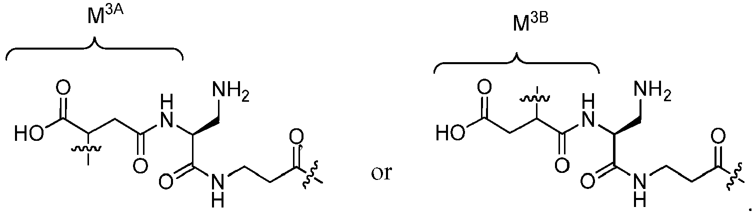

### 1.6 Treatment of hyper-proliferating conditions

The Ligand-Drug Conjugates are useful for inhibiting the multiplication of a tumor cell or cancer cell, causing apoptosis in a tumor or cancer cell, or for treating cancer in a patient. The Ligand-Drug Conjugates can be used accordingly in a variety of settings for the treatment of cancers. The Ligand-Drug Conjugates can be used to deliver a drug to a tumor cell or cancer cell. Without being bound by theory, the Ligand unit of a Ligand-Drug Conjugate may bind to or associate with a cell-surface cancer-cell or a tumor-cell-associated antigen, and upon binding the Ligand-Drug Conjugate can be taken up (internalized) inside a tumor cell or cancer cell through antigen-mediated endocytosis or other internalization mechanism. The antigen can be attached to a tumor cell or cancer cell or can be an extracellular matrix protein associated with the tumor cell or cancer cell. Once inside the cell, via a enzymatic or non-enzymatic cleavable mechanism, depending upon the components of the linker system, the drug is released within the cell. Alternatively, the Drug or Drug unit may be cleaved from the Ligand-Drug Conjugate within the vicinity of the tumor cell or cancer cell, and the Drug or Drug unit subsequently penetrates the cell.

The Ligand-Drug Conjugates can provide conjugation-specific tumor or cancer drug targeting, thus reducing general toxicity of the drug.

The Linker units may stabilize the Ligand-Drug Conjugates in blood, yet be capable of liberating drug once inside the cell.

The Ligand unit may bind to the tumor cell or cancer cell.

The Ligand unit may bind to a tumor cell or cancer cell antigen which is on the surface of the tumor cell or cancer cell.

The Ligand unit may bind to a tumor cell or cancer cell antigen which is an extracellular matrix protein associated with the tumor cell or cancer cell.

The specificity of the Ligand unit for a particular tumor cell or cancer cell can be important for determining those tumors or cancers that are most effectively treated. For example, a ligand drug conjugate having a BR96 Ligand unit can be useful for treating antigen positive carcinomas including those of the lung, breast, colon, ovaries, and pancreas. Ligand-Drug Conjugates having an anti-CD30 or an anti-CD70 binding Ligand unit can be useful for treating hematologic malignancies.

Other particular types of cancers that can be treated with a ligand drug conjugates include, but are not limited to the following solid tumors, blood-borne cancers, acute and chronic leukemias, and lymphomas.

Solid tumors include but are not limited to fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

Blood-borne cancers include but are not limited to acute lymphoblastic leukemia "ALL", acute lymphoblastic B-cell leukemia, acute lymphoblastic T-cell leukemia, acute myeloblastic leukemia "AML", acute promyelocytic leukemia "APL", acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia "CML", chronic lymphocytic leukemia "CLL", hairy cell leukemia, and multiple myeloma.

Acute and chronic leukemias include but are not limited to lymphoblastic, myelogenous, lymphocytic, and myelocytic leukemias.

Lymphomas include but are not limited to Hodgkin's disease, non-Hodgkin's Lymphoma, Multiple myeloma, Waldenström's macroglobulinemia, Heavy chain disease, and Polycythemia vera.

Cancers, including, but not limited to, a tumor, metastasis, or other diseases or disorders characterized by hyper-proliferating cells, can be treated or its progression inhibited by administration of an ADC composition.

Methods for treating cancer are disclosed, including administering to a patient in need thereof an effective amount of an LDC composition and a chemotherapeutic agent. The cancer to be treated with a chemotherapeutic in combination with an LDC may have not been found to be refractory to the chemotherapeutic agent. Alternatively, the cancer to be treated with a chemotherapeutic in combination with an ADC may be refractory to the chemotherapeutic agent. The LDC compositions can be administered to a patient that has also undergone surgery as treatment for the cancer.

The patient may also receive an additional treatment, such as radiation therapy. The Ligand-Drug Conjugate may be administered concurrently with the chemotherapeutic agent or with radiation therapy. The chemotherapeutic agent or radiation therapy may also be administered prior or subsequent to administration of a ligand drug conjugate.

A chemotherapeutic agent can be administered over a series of sessions. Any one or a combination of the chemotherapeutic agents, such a standard of care chemotherapeutic agent(s), can be administered.

Additionally, methods of treatment of cancer with a Ligand-Drug Conjugate are provided as an alternative to chemotherapy or radiation therapy where the chemotherapy or the radiation therapy has proven or can prove too toxic, e.g., results in unacceptable or unbearable side effects, for the subject being treated. The patient being treated can, optionally, be treated with another cancer treatment such as surgery, radiation therapy or chemotherapy, depending on which treatment is found to be acceptable or bearable.

### 1.7 Pharmaceutical compositions comprising an LDC

Pharmaceutical compositions comprising an LDC composition described herein and a pharmaceutically acceptable carrier are disclosed. The pharmaceutical compositions can be in any form that allows for an LDC to be administered to a patient for treatment of a disorder associated with expression of the antigen to which the antibody of the ADC binds. For example, the pharmaceutical compositions can be in the form of a liquid or a lyophilized solid. The preferred route of administration is parenteral. Parenteral administration includes subcutaneous injections, intravenous, intramuscular, and intrasternal injection or infusion techniques. Preferably, a pharmaceutical composition comprising an ADC is administered intravenously in the form of a liquid solution.

Pharmaceutical compositions can be formulated so as to allow a compound to be bioavailable upon administration of the composition to a patient. Such compositions can take the form of one or more dosage units, where for example, a lyophilized solid may provide a single dosage unit when reconstituted as a solution or suspension on addition of a suitable liquid carrier.

Materials used in preparing the pharmaceutical compositions are preferably non-toxic in the amounts used. It will be evident to those of ordinary skill in the art that the optimal dosage of the active ingredient(s) in the pharmaceutical composition will depend on a variety of factors. Relevant factors include, without limitation, the type of animal (*e.g.*, human), the particular form of the pharmaceutical composition, the manner of administration, and the LDC composition employed.

The pharmaceutical composition can be, for example, in the form of a liquid. The liquid can be useful for delivery by injection. In a composition for administration by injection, one or more of a surfactant, preservative, wetting agent, dispersing agent, suspending agent, buffer, stabilizer and isotonic agent can also be included.

The liquid compositions, whether they are solutions, suspensions or other like form, can also include one or more of the following: sterile diluents such as water for injection, saline solution, preferably physiological saline, Ringer's solution, isotonic sodium chloride, fixed oils such as synthetic mono or digylcerides which can serve as the solvent or suspending medium, polyethylene glycols, glycerin, cyclodextrin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as amino acids, acetates, citrates or phosphates; detergents, such as nonionic surfactants, polyols; and agents for the adjustment of tonicity such as sodium chloride or dextrose. A parenteral composition can be enclosed in ampoule, a disposable syringe or a multiple-dose vial made of glass, plastic or other material. Physiological saline is an exemplary adjuvant. An injectable pharmaceutical composition is preferably sterile.

The amount of the conjugate that is effective in the treatment of a particular disorder or condition will depend on the nature of the disorder or condition, and can be determined by standard clinical techniques. In addition, *in vitro* or *in vivo* assays can optionally be employed to help identify optimal dosage ranges. The precise dose to be employed in the compositions will also depend on the route of administration, and the seriousness of the disease or disorder, and should be decided according to the judgment of the practitioner and each patient's circumstances.

The pharmaceutical composition comprises an effective amount of an LDC composition such that a suitable dosage will be obtained for administration to a subject in need thereof. Typically, this amount is at least about 0.01% by weight of the pharmaceutical composition.

For intravenous administration, the pharmaceutical composition can comprise from about 0.01 to about 100 mg of an LDC composition per kg of the animal's body weight. In one aspect, the pharmaceutical composition can include from about 1 to about 100 mg of a ADC composition per kg of the animal's body weight. In another aspect, the amount administered will be in the range from about 0.1 to about 25 mg/kg of body weight of an ADC composition.

Generally, the dosage of an LDC composition administered to a patient is typically about 0.01 mg/kg to about 100 mg/kg of the subject's body weight. The dosage administered to a patient may be between about 0.01 mg/kg to about 15 mg/kg of the subject's body weight, or between about 0.1 mg/kg and about 15 mg/kg of the subject's body weight, or between about 0.1 mg/kg and about 20 mg/kg of the subject's body weight, or between about 0.1 mg/kg to about 5 mg/kg or about 0.1 mg/kg to about 10 mg/kg of the subject's body weight, or between about 1 mg/kg to about 15 mg/kg of the subject's body weight, or between about 1 mg/kg to about 10 mg/kg of the subject's body weight. The dosage administered may be between about 0.1 to 4 mg/kg, preferably 0.1 to 3.2 mg/kg, or more preferably 0.1 to 2.7 mg/kg of the subject's body weight over a treatment cycle.

An LDC can be administered by any convenient route, for example by infusion or bolus injection, by absorption through epithelial or mucocutaneous linings (e.g., oral mucosa, rectal and intestinal mucosa). Administration can be systemic or local. Various delivery systems are known, e.g., encapsulation in liposomes, microparticles, microcapsules, capsules, and can be used to administer a compound. More than one compounds or composition may be administered to a patient.

The conjugates may be formulated in accordance with routine procedures as a pharmaceutical composition adapted for intravenous administration to animals, particularly human beings. Typically, the carriers or vehicles for intravenous administration are sterile isotonic aqueous buffer solutions. Where necessary, the compositions can also include a solubilizing agent. Compositions for intravenous administration can optionally comprise a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachet indicating the quantity of active agent. Where a conjugate is to be administered by infusion, it can be dispensed, for example, with an infusion bottle containing sterile pharmaceutical grade water or saline. Where the conjugate is administered by injection, an ampoule of sterile water for injection or saline can be provided so that the ingredients can be mixed prior to administration.

The pharmaceutical compositions are generally formulated as sterile, substantially isotonic and in full compliance with all Good Manufacturing Practice (GMP) regulations of the U.S. Food and Drug Administration.

Pharmaceutical compositions described herein comprise LDC compositions of the present invention and a pharmaceutically acceptable carrier. Preferably, all, or substantially all, or more than 50% of the LDCs in the pharmaceutical composition comprises a hydrolyzed thio-substituted succinimide. Preferably, more than 55%, 60%, 65%, 70%, 75%, 80%, 85%, 90%, 91%, 92%, 93%, 94%, 95%, 96%, 97 %, 98%, or 99% of the Ligand Drug Conjugates present in the pharmaceutical composition comprises a hydrolyzed thio-substituted succinimide.

### 1.8 Preparation

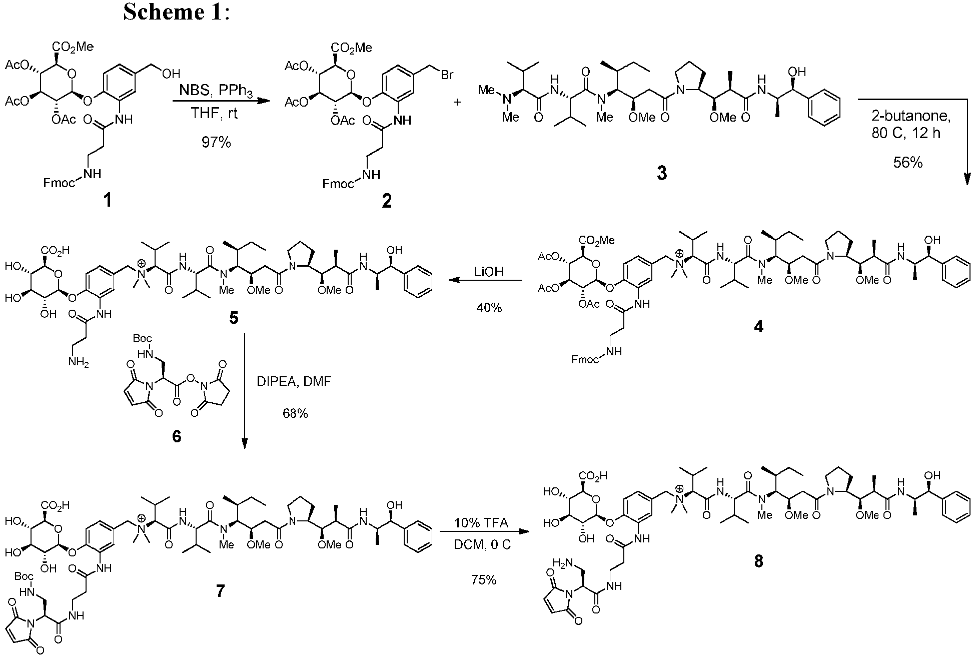

Scheme 1 is an exemplary preparation of a drug-linker compound of formula L_{b}'-A₁(BU)-Aₒ-Y(W)-D⁺ wherein L_{b}' is a M¹ moiety, Y is a PAB self-immolative (SI) moiety, W is a carbohydrate (Su) moiety bonded to SI through a glycosidic linkage wherein said linkage is cleavable by a glycosidase, and D⁺ is a quaternized auristatin. In that particular L_{b}'-Lₒ-D⁺ moiety the L_{b}'-A₁(BU)- structure represents an exemplary L_{SS} moiety.

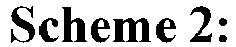

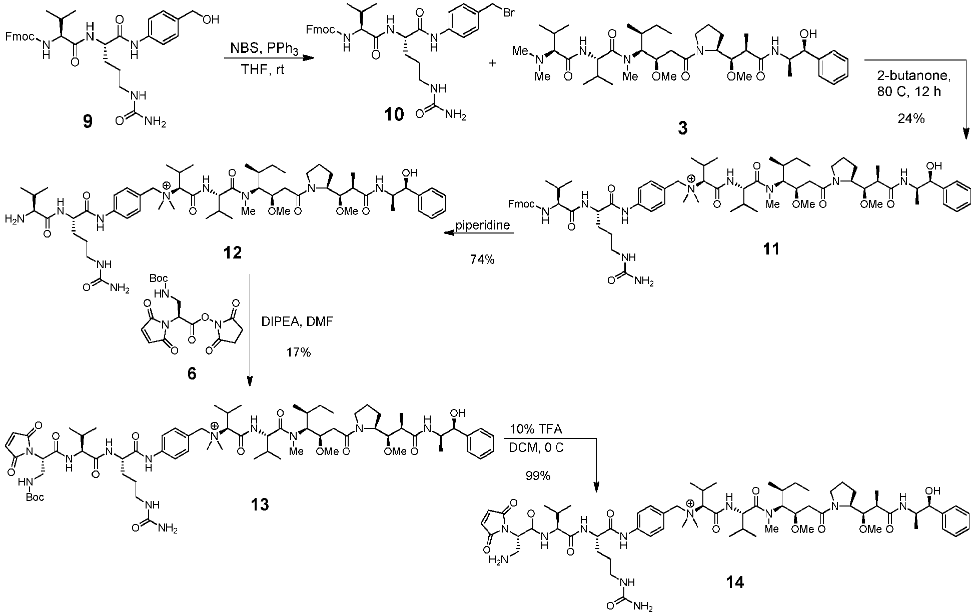

Scheme 2 is an exemplary preparation of a drug-linker compound of formula L_{b}'-A₁(BU)-Aₒ-Y-W-D⁺ wherein L_{b}' is a M¹ moiety, Y is a PAB self-immolative (SI) moiety, W is a dipeptide moiety bonded to SI through an anilide linkage wherein said linkage is cleavable by a regulatory protease and D⁺ is a quaternized auristatin. In that particular L_{b}'-Lₒ-D⁺ moiety the L_{b}'-A₁(BU)- structure represents an exemplary L_{SS} moiety covalently bonded to another stretcher subunit and the val-cit dipeptide moiety provides for recognition by said regulatory protease, which is in this case is Cathepsin B.

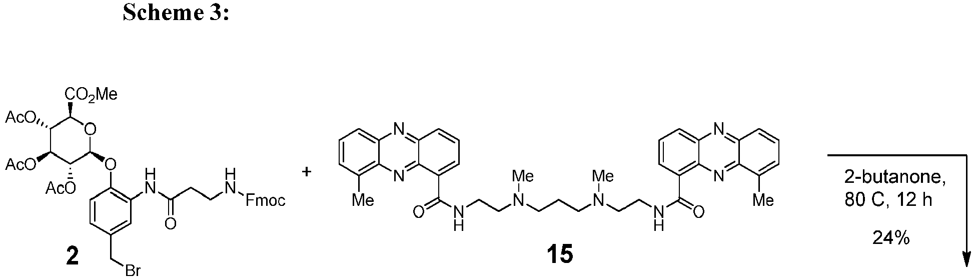

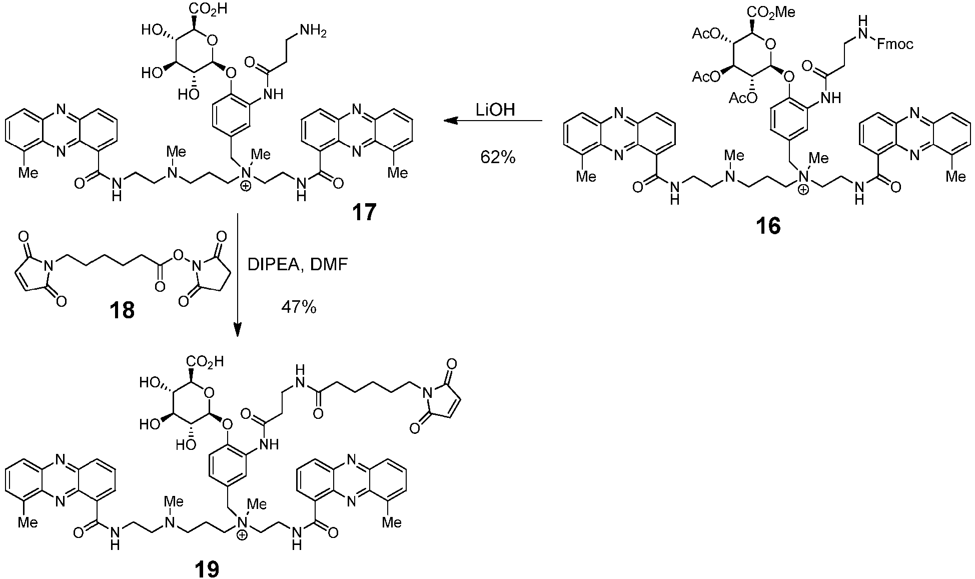

Scheme 3 is an exemplary preparation of a L_{b}'-A₁(BU)-Aₒ-Y(W)-D⁺ drug-linker compound wherein L_{b}' is a M¹ moiety, Y is a PAB self-immolative (SI) moiety, W is a carbohydrate (Su) moiety bonded to SI through a glycosidic linkage wherein said linkage is cleavable by a glycosidase, and D⁺ is a quaternized phenazine dimer. In that particular L_{b}'-Lₒ-D⁺ compound the L_{b}'-A₁-Aₒ- structure represents an M¹-A- moiety having two stretcher subunits (i.e., -A- is -A₁-A_{O}-).

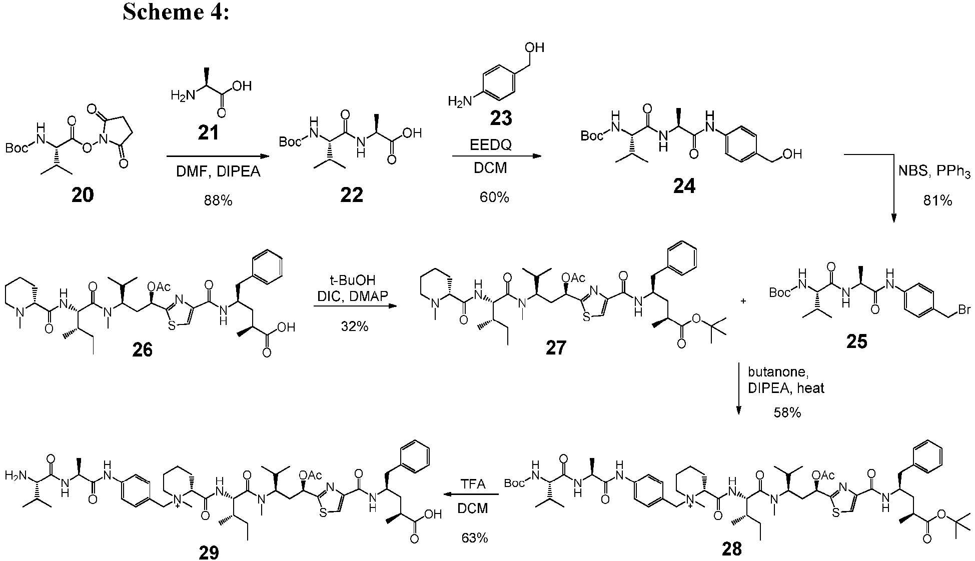

Scheme 4 is an exemplary preparation of a NH₂-W-Y-D⁺ compound as an intermediate for obtaining a drug-linker compound of formula L_{b}'-Lₒ-D⁺ wherein Y is a PAB self-immolative (SI) moiety, W is a dipeptide moiety bonded to SI through an anilide linkage wherein said linkage is cleavable by a regulatory protease and D⁺ is a quaternized tubulysin Drug Unit.

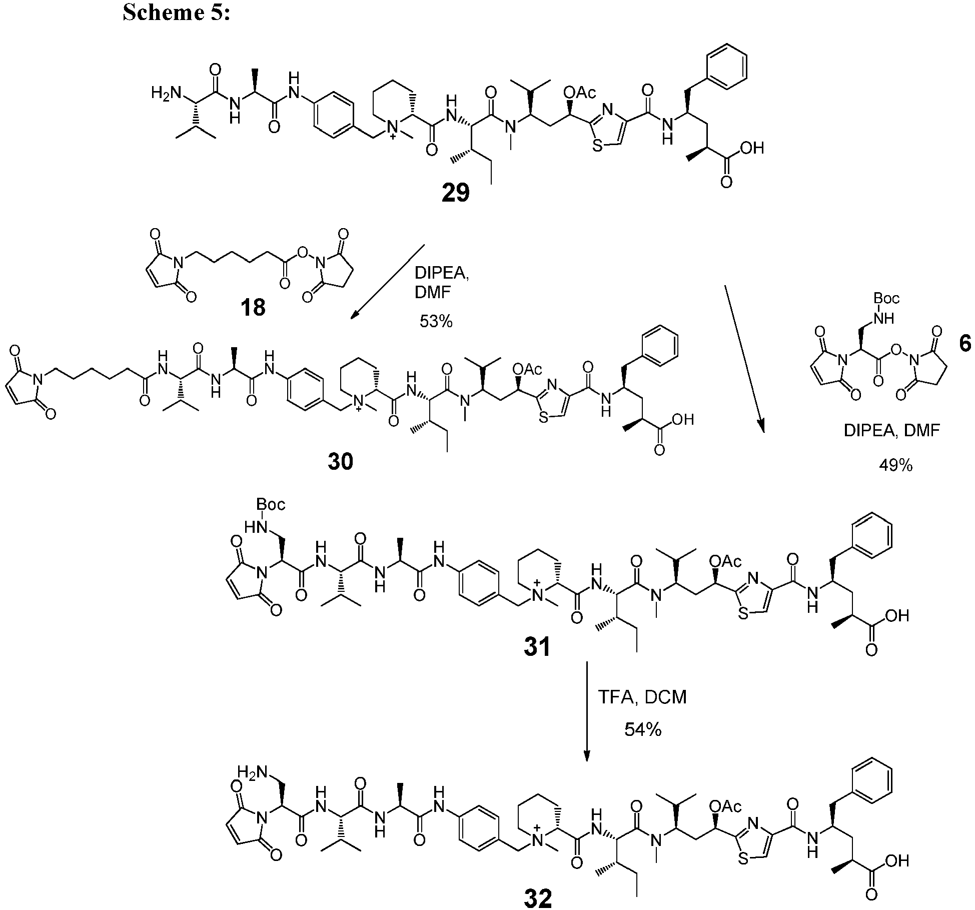

Scheme 5 is an exemplary preparation of a L_{b}'-A-W-Y-D⁺ drug-linker compound and a L_{b}'-A(BU)-W-Y-D+ drug-linker compound obtained from peptide coupling of the NH₂-W-Y-D⁺ intermediate of Scheme 4 with a L_{b}'-A-CO₂H or a L_{b}'-A(BU)-CO₂H intermediate wherein L_{b}' is a maleimide (M¹) moiety. The products so formed, M¹-A-W-Y-D⁺ and M¹-A(BU)-W-Y-D⁺ are an exemplary L_{b}'-Lₒ-D⁺ drug-linker compounds having a quaternized tubulysin Drug unit.

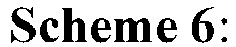

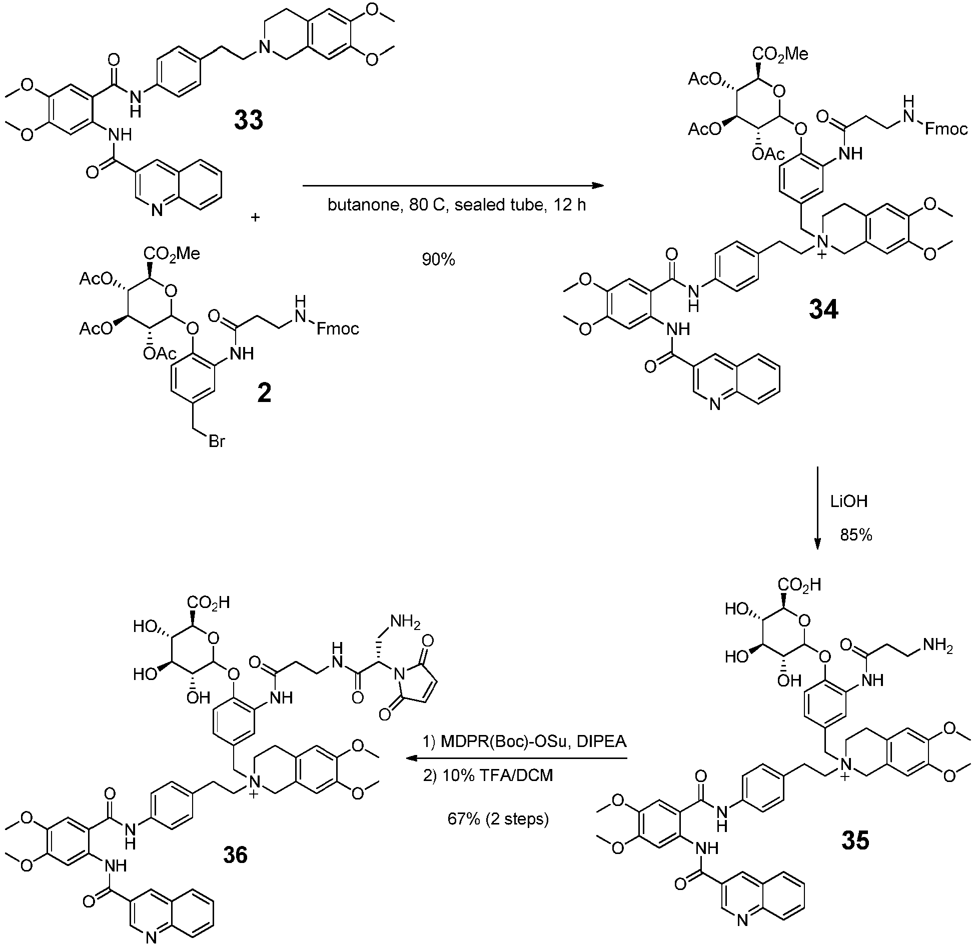

Scheme 6 is an exemplary preparation of an L_{b}'-A₁(BU)-Aₒ-Y(W)-D⁺ moiety wherein L_{b}' is a M¹ moiety, Y is a PAB self-immolative (SI) moiety, W is a carbohydrate (Su) moiety bonded to SI through a glycosidic linkage wherein said linkage is cleavable by a glycosidase, and D⁺ is a quaternized MDR inhibitor wherein the inhibitor is Tariquidar. In that particular L_{b}'-Lₒ-D⁺ moiety, the L_{b}'-A(BU)- structure represents an exemplary L_{SS} moiety.

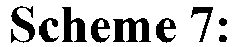

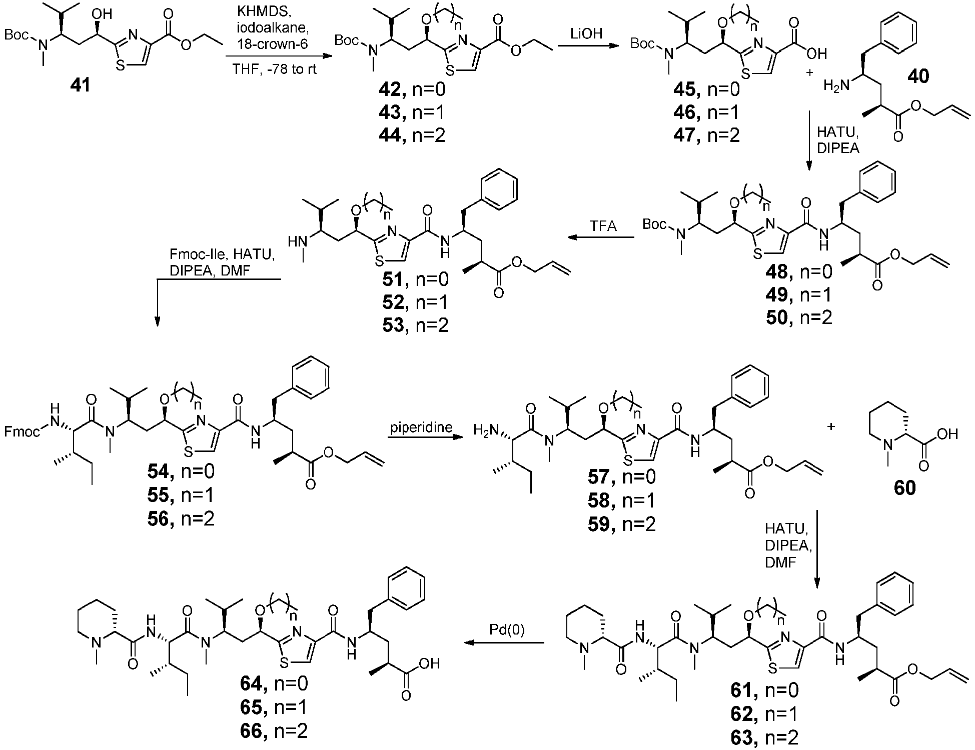

Scheme 7 represents exemplary preparations of tubulysins of formula D_{G-4}, wherein R^{7B} is hydrogen and R^{2B} is hydrogen, methyl or ethyl, which have an ether (i.e. methyl, ethyl or propyl ether) as the O-linked substituent replacing acetate in the tubuvaline moiety of Tubulysin M and which do not have the chemically unstable N,O-acetal functional group of such tubulysins. Compounds **64-66** are further stabilized in comparison to Tubulysin M by replacing the hydrolytically unstable tubuvaline acetate substituent with an ether moiety. 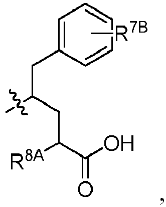 wherein R^{7B} is hydrogen or an O-linked substituent, preferably hydrogen or -OH in the *para* position, and R^{8A} is hydrogen or lower alkyl, preferably methyl; and wherein the wavy line indicates the point of attachment to the remainder of D_{G} or D_{H}.

In preferred embodiments of structure D_{G} or D_{H}, one R⁷ is hydrogen, and the other R⁷ is an optionally substituted arylalkyl having the structure of 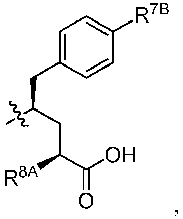 wherein R^{7B} is -H or -OH; and wherein the wavy line indicates the point of attachment to the remainder of D_{G} or D_{H}.

In other embodiments of structure D_{G} and D_{H}, one R⁷ is hydrogen or lower alkyl, preferably hydrogen or methyl, more preferably hydrogen, and the other R⁷ is optionally substituted arylalkyl having the structure of one of: 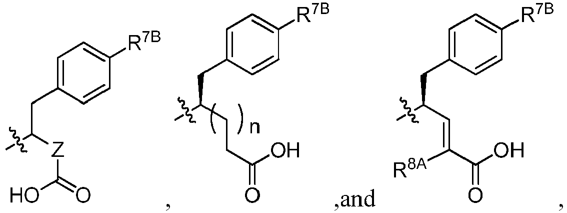 wherein Z is an optionally substituted alkylene or an optionally substituted alkenylene, R^{7B} is hydrogen or an O-linked substituent, preferably hydrogen or -OH in the *para* position, R^{8A} is hydrogen or lower alkyl, preferably methyl, and the subscript n is 0, 1 or 2, preferably 0 or 1; and wherein the wavy line indicates the point of attachment to the remainder of D_{G} or D_{H}.In still other embodiments of structure D_{G} and D_{H} -N(R⁷)(R⁷) is -NH(C₁-C₆ alkyl) wherein the C₁-C₆ alkyl is optionally substituted by -CO₂H or an ester thereof, or by an optionally substituted phenyl. In preferred embodiments -N(R⁷)(R⁷) is selected from the group consisting of -NH(CH₃), -CH₂CH₂Ph, and -CH₂-CO₂H, -CH₂CH₂CO₂H and - CH₂CH₂CH₂CO₂H.

In some embodiments of structure D_{G}' and D_{H}', R⁷ and R¹⁰ together with the atoms to which they are attached define an optionally substituted 5 or 6-membered heterocycle wherein -N(R⁷)-CH(R¹⁰)(CH₂R¹¹) has the structure of: 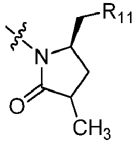 wherein the wavy line indicates the point of attachment to the remainder of D_{G}' or D_{H}'.

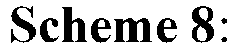

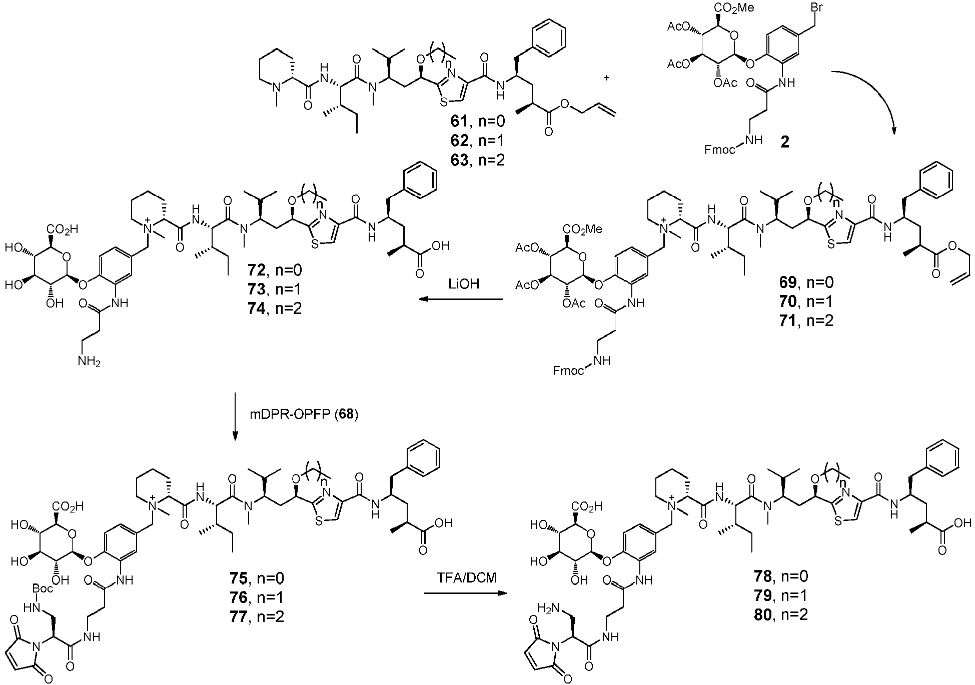

Scheme 8 represents an exemplary preparation of drug linker compounds of formula L_{b}'-L_{O}-D⁺ wherein D⁺ is a quaternized tubulysin compound that has an ether moiety as the O-linked substituent replacing acetate in the tubuvaline moiety of tubulysin M and which does not have the chemically unstable N,O-acetal functional group of such tubulysins. In the exemplified compounds -L_{O}-D⁺ has the structure of Aₐ-Y_{y}(W_{w})-D⁺ wherein subscripts a, y and w are each one. Compounds **78-80** therefore represent exemplary drug linker compounds wherein W is a carbohydrate moiety attached to a self-immolative Spacer unit (Y) having the structure of a PAB moiety through a glycosidic bond cleavable by a glycosidase to which is attached D⁺ wherein D⁺ is a quaternized tubulysin compound. Compounds **78-80** further represents drug linker compounds whose L_{b}' components are comprised of a M¹ moiety and a Basic unit so as to provide a self-stabilizing linker component (L_{SS}) of a Ligand Drug Conjugate from its condensation with a targeting moiety having a reactive thiol functional group as in a cysteine thiol of an antibody.

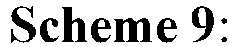

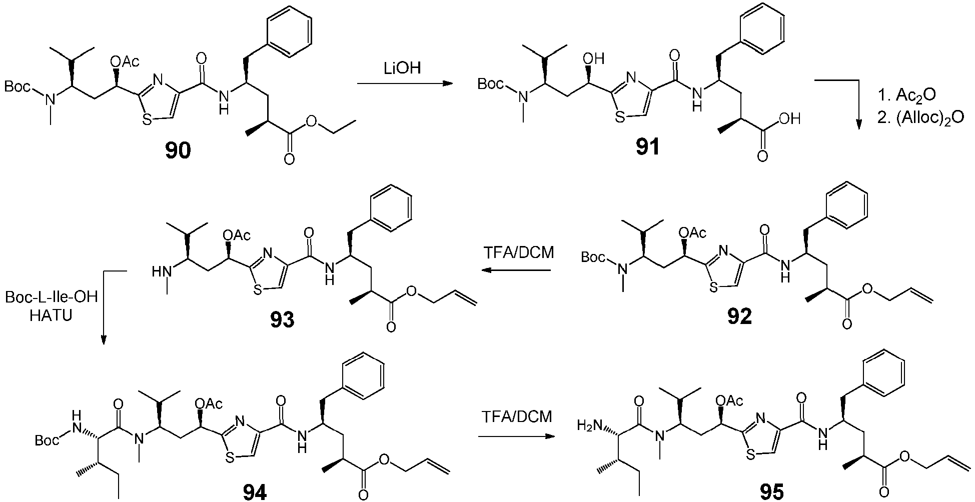

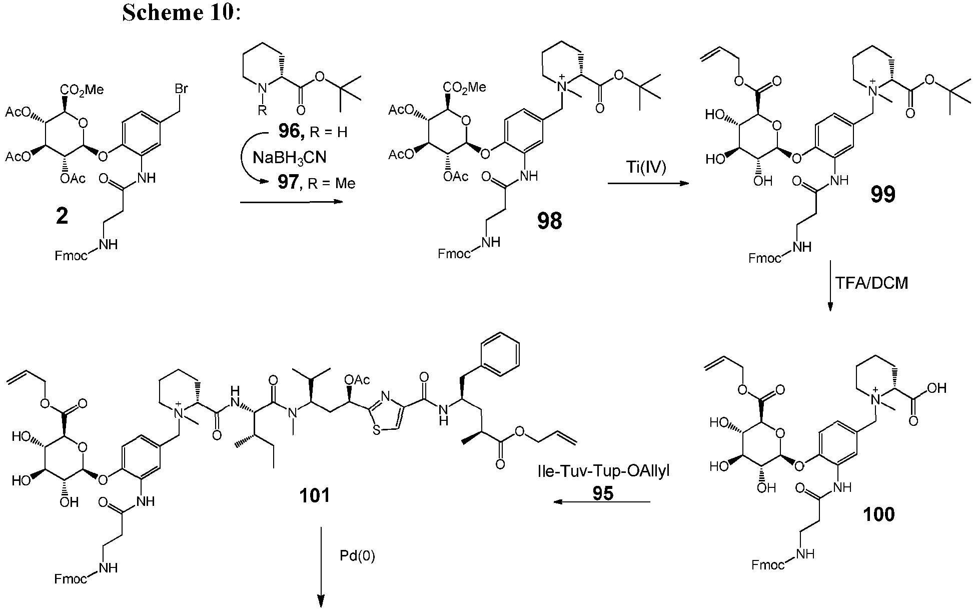

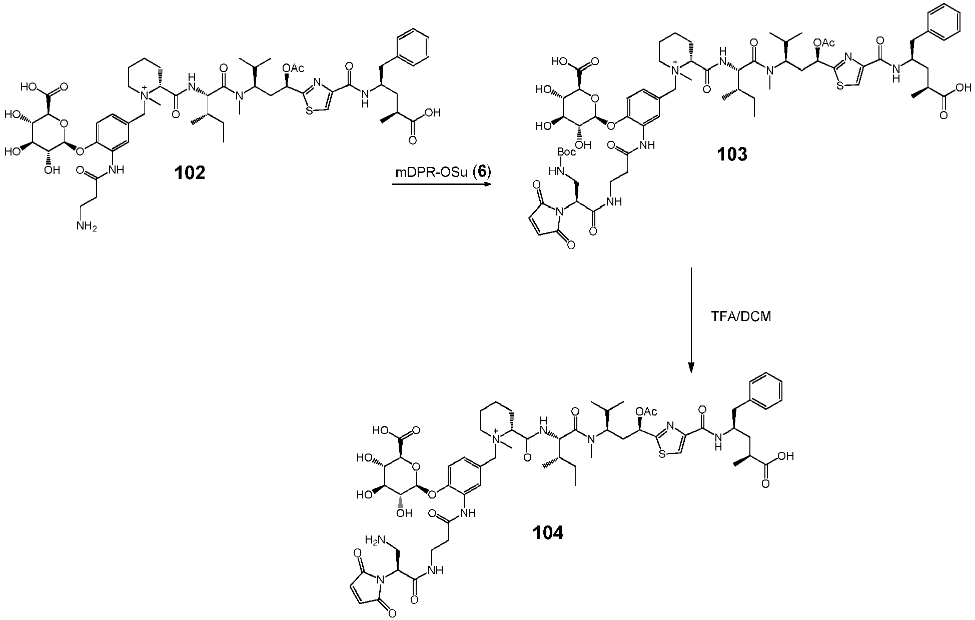

Schemes 9 and 10 together provide an alternative preparation of drug linker compounds of formula L_{b}'-L_{O}-D⁺ wherein -Lₒ-D⁺ has the structure of Aₐ-Y_{y}(W_{w})-D⁺ wherein subscripts a, y and w are each one, wherein -A- is -A₁-A_{O}-. Compound **104** therefore represents an exemplary drug linker compound of formula Lb-A₁(BU)-A_{O}-Y(W)-D⁺ wherein W is a carbohydrate moiety attached to a self-immolative Spacer unit (Y) having the structure of a PAB moiety through a glycosidic bond cleavable by a glycosidase to which is attached D+ wherein D⁺ is a quaternized tubulysin compound. For compound **104** the quaternized tubulysin is that of Tubulysin Mhas an ether moiety which retains the tubuvaline acetate component but does not have the chemically unstable N,O-acetal functional group of such tubulysins.

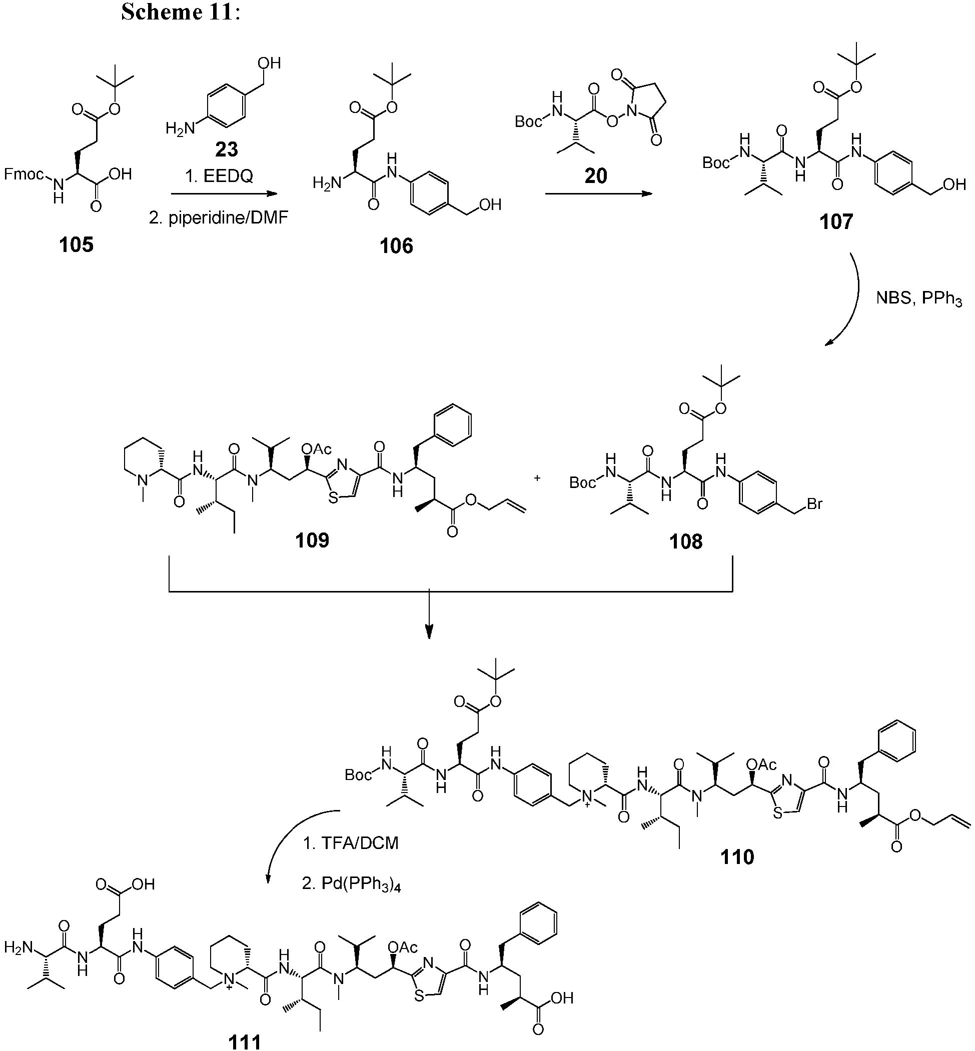

Scheme 11 represents an exemplary synthesis of a W"_{w}-Y_{y}-D⁺ intermediate for preparation of a drug-linker compound L_{b}'-L_{O}-D⁺ of formula D_{G-3}, wherein R^{7B} is -H and R^{2B} is methyl, and wherein subscripts w and y are each 1 and wherein W" is a precursor to W in a drug linker compound or Ligand Drug Conjugate prepared from the drug linker compound intermediate. Compound **111** further represents an drug linker intermediate compound wherein W is a dipeptide moiety and Y is a self-immolative Spacer Unit exemplified by a PAB moiety. In Compound **111** that dipeptide is exemplified by -valine-glutamate- and D⁺ is exemplified by quaternized Tubulysin M.

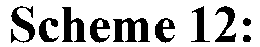

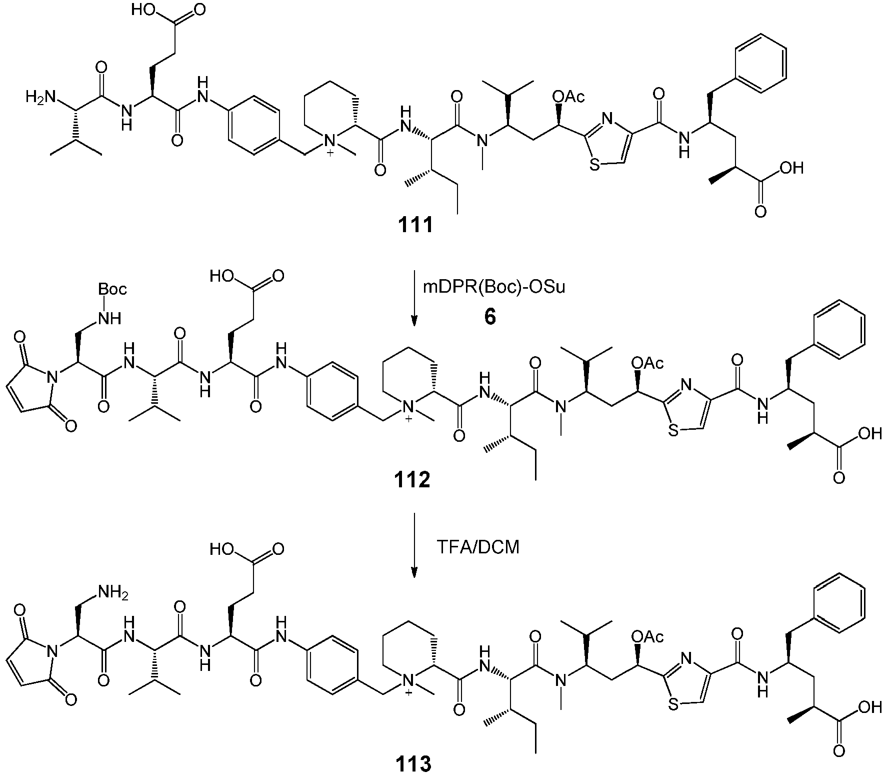

Scheme 12 exemplifies the preparation of a drug linker compound L_{b}'-L_{O}-D⁺ of formula D_{G-3}, wherein R^{7B} is -H and R^{2B} is methyl, from an intermediate of formula H-W_{w}-Y_{y}-D⁺, namely Compound **111**, wherein the subscripts w and y are each 1, and W, Y and D⁺ are exemplified by a dipeptide moiety (-valine-glutamate-), a self-immolative PAB moiety, and quaternized Tubulysin M, respectively. Compound **113** further represents the product having the formula of L_{b}'-A(BU)-W-Y-D⁺ from incorporation of a ligand covalent binding moiety precursor (L_{b}') into a drug linker compound, wherein L_{b}'-A(BU)- is comprised of a maleimide moiety (M¹), a Stretcher Unit (A) substituted by a Basic Unit (BU) by condensing Compound **6** having moieties M1 and a BU substituted Stretcher Unit precursor (Aₐ') substituted wherein the subscript a is 1, wherein that precursor on condensation with W_{w}-Y_{y}-D⁺ of compound **111** will complete formation of the L_{O} component of the Linker unit L_{b}'-L_{O}- after BOC deprotection **.Scheme 13:**

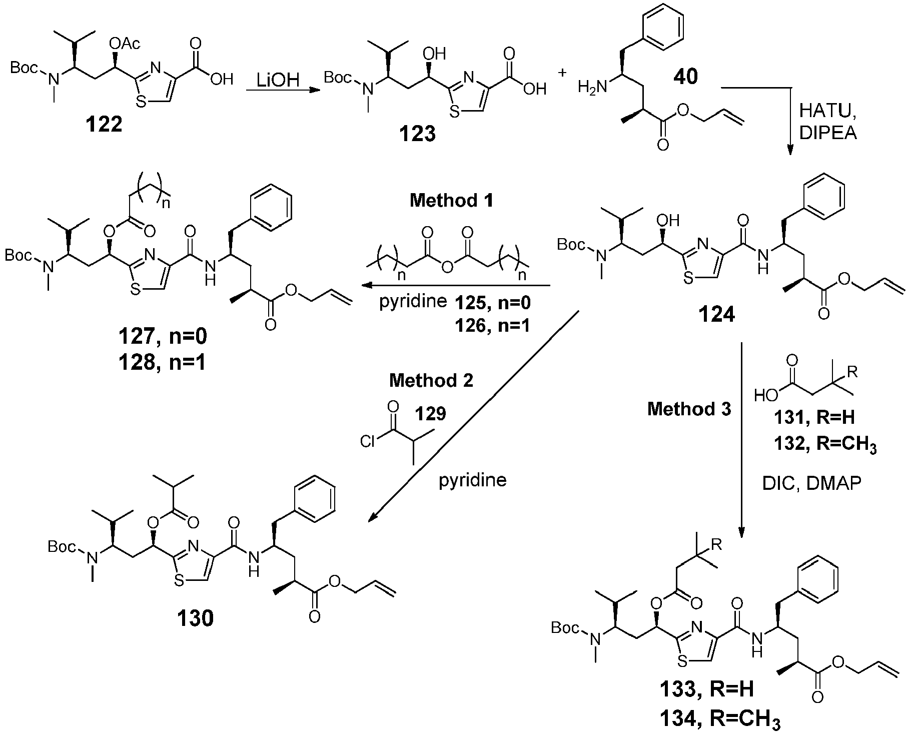

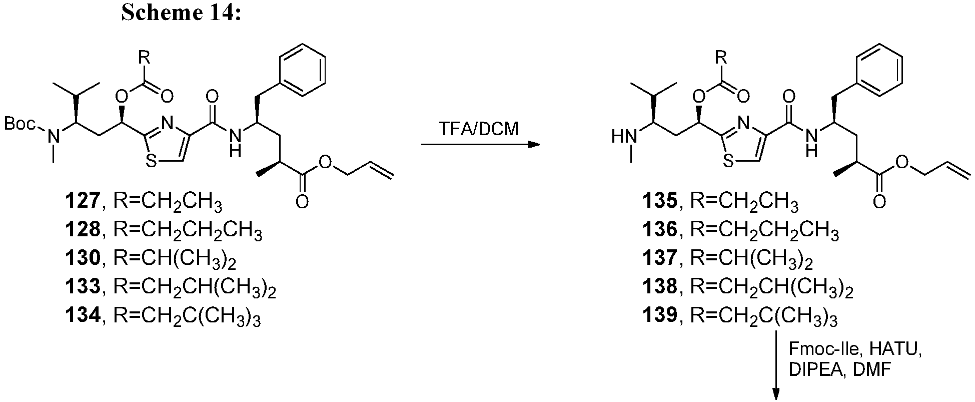

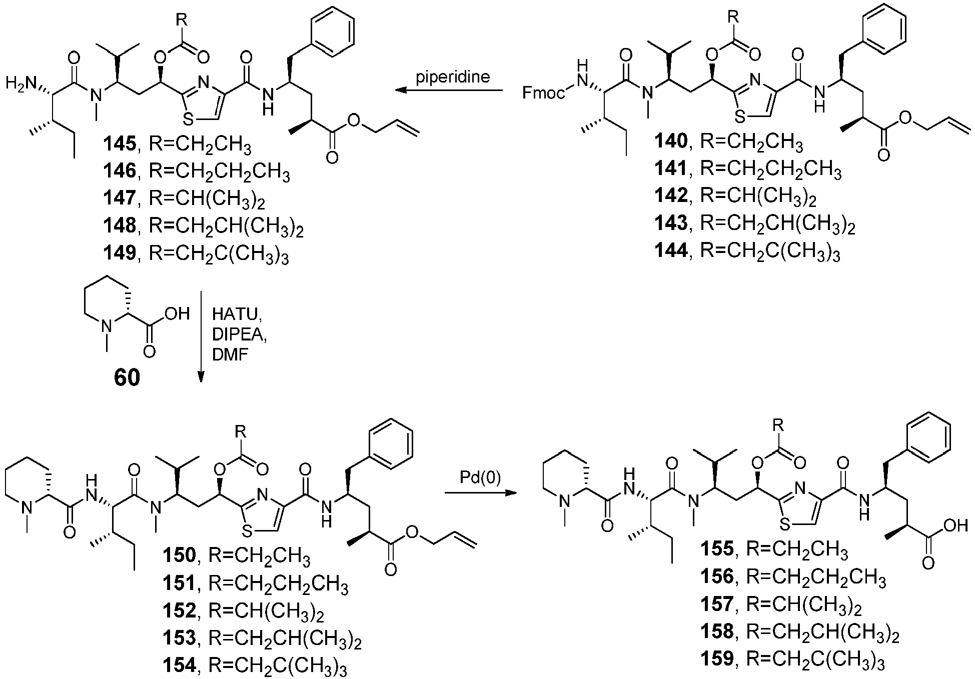

Schemes 13 and 14 together provide exemplary syntheses of tubulysins in which the acetate moiety of the tubuvaline component of Tubulysin M has been replaced with other O-linked moieties having the formula -OC(O)R^{2B} as shown in structure D_{G-3}.

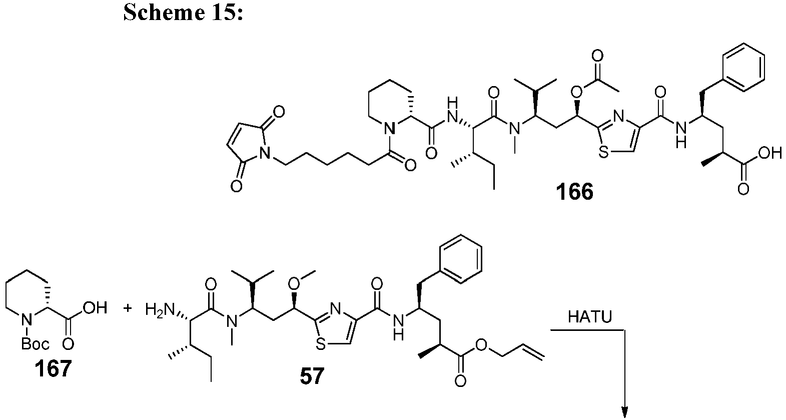

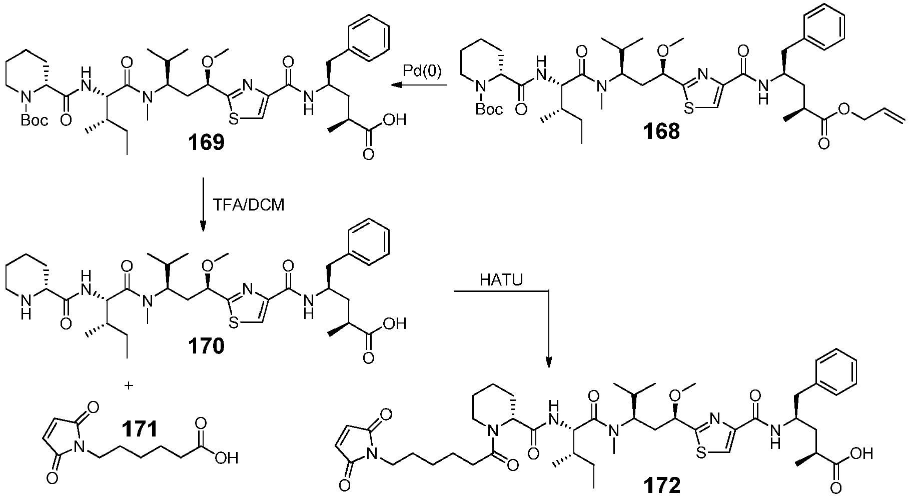

Scheme 15 shows an exemplary synthesis of a Drug Linker compound for preparation of Ligand Drug Conjugates having a non-quaternized tubulysin Drug Unit, wherein the acetate O-linked substituent of the tubuvaline component of des-methyl Tubulysin M has been replaced with an ether moiety as the O-linked substituent attached and wherein the Tub(OCH₃) Drug Unit is attached through is N-terminal component to a Linker Unit by the carbonyl functional group of the Stretcher Unit. A LDC from Compound **172** represents a non-cleavable conjugate (i.e., is resistant to protease-mediated release of free des-methyl Tubulysin M (OCH₃)-OH and is of the general formula M¹-A-D wherein M¹ is the maleimide moiety, the stretcher unit A is an alkylene and the non-quaternized Drug Unit D is des-MeTub(OCH₃)-OH. A Ligand Drug Conjugate from that Drug Linker compound is of the general formula L-S-M²-A-D, wherein M² is a succinimide moiety resulting from conjugate addition of a targeting moiety thiol to M¹ of the Drug Linker compound. An active moiety that is eventually released from non-specific proteolysis when the Ligand unit of the LDC is of an antibody targeting moiety (i.e., is an ADC) has the structure of Cys-S-M²-A-Tub(OCH₃)-OH wherein the Cys-S- moiety is from a cysteine amino acid the antibody Ligand Unit.

Drug-linker compounds replacing Tubulysin M with another tertiary amine-containing tubulysin retaining the acetate moiety in its tubuvaline component or replacing that moiety with an ether or a different acyloxy or O-linked substituent (e.g., an O-linked carbamate) of formula **D_{G}, D_{G-1}, D_{G-2}, D_{G-3}, D_{G-4}, D_{G-5}, D_{G-6}, D_{G-7}, D_{G-8}, D_{H}, D_{H-1}** or **D_{H-2}** are prepared in analogous fashion to that of Scheme 4, Scheme 7, Scheme 8, Schemes 9+10, Scheme 11, Scheme 12, or Schemes 13+14.

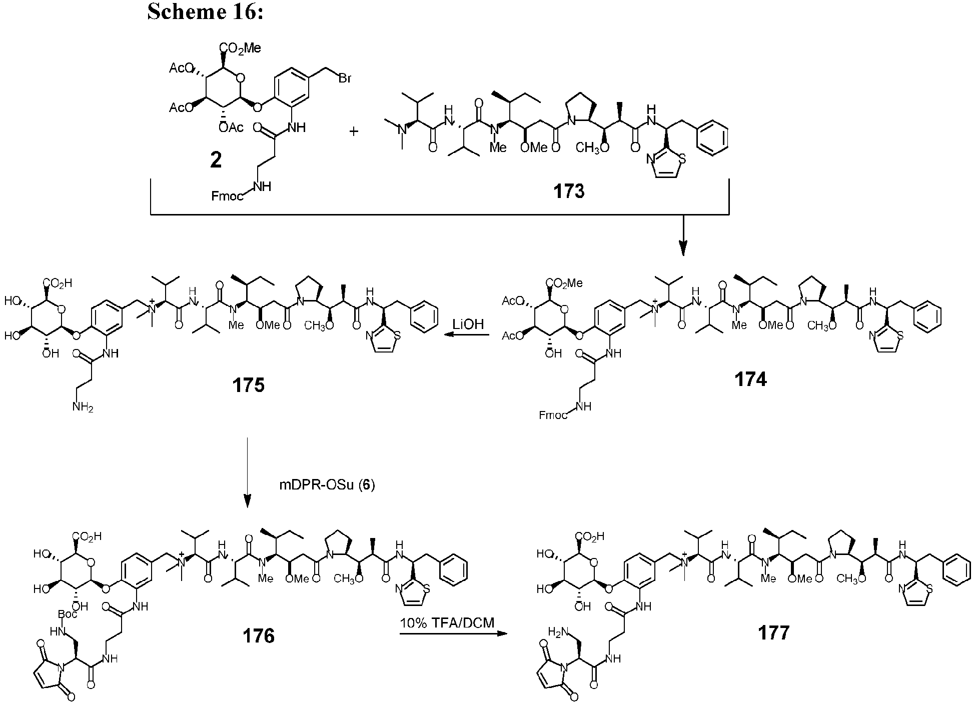

Scheme 16 shows an exemplary synthesis of a drug linker compound for preparation of Ligand Drug Conjugates having the formula of L_{b}'-A₁(BU)-Aₒ-Y(W)-D⁺ wherein L_{b}' is a M¹ moiety, Y is a PAB self-immolative (SI) moiety, W is a carbohydrate (Su) moiety bonded to SI through a glycosidic linkage wherein said linkage is cleavable by a glycosidase, and D⁺ is quaternized Dolastatin 10. In that drug-linker embodiment of formula L_{b}'-Lₒ-D⁺ the L_{b}'-A₁(BU)- structure represents an exemplary L_{SS} moiety. Compound **177** is analogous to Compound **8** of Scheme 1 in which quaternized auristatin E is replaced by quaternized Dolastatin 10. Ligand Drug Conjugates prepared from Compound **177** release free dolastatin 10, which is another member of the auristatin class of compounds and which contains a tertiary amine functional group, upon action by β-glucuronidase.

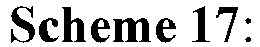

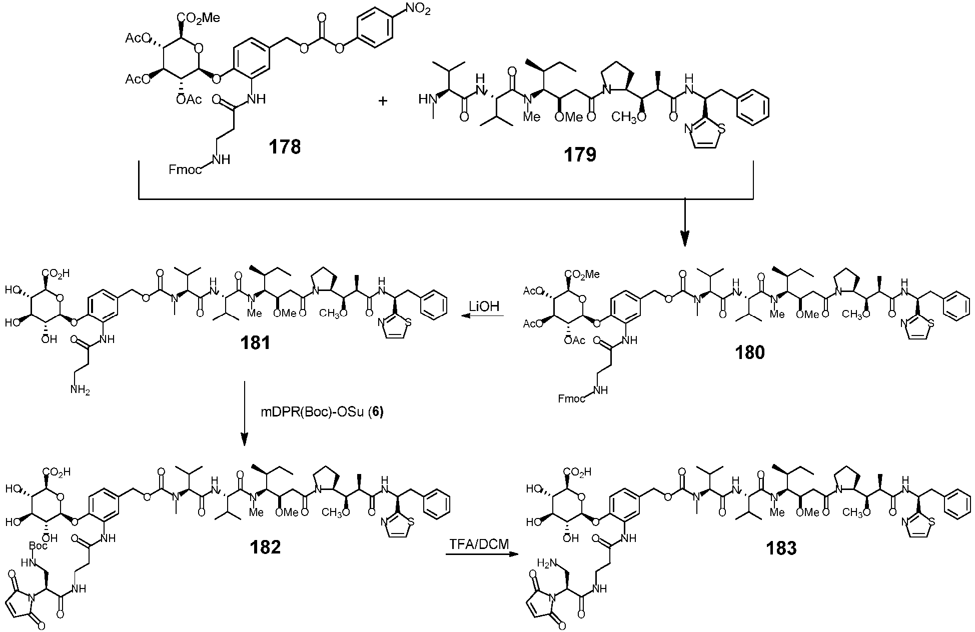

Scheme 17 shows an exemplary synthesis of a drug-linker compound for preparation of Ligand Drug Conjugates in which monomethyl dolastatin 10 is conjugated through a carbamate functional group to a PAB moiety. A carbamate-linked Ligand Drug Conjugate is unsuitable for a tertiary-amine containing drug in which that amine functional group is the point of attachment to a PAB moiety. Conjugates from Compound **183** release a secondary amine-containing free drug (i.e., monomethyl dolastatin 10) and conjugates from Compound **177** release the parent tertiary-containing free drug (i.e., dolastatin 10) though the same enzymatic processing by β-glucuronidase.

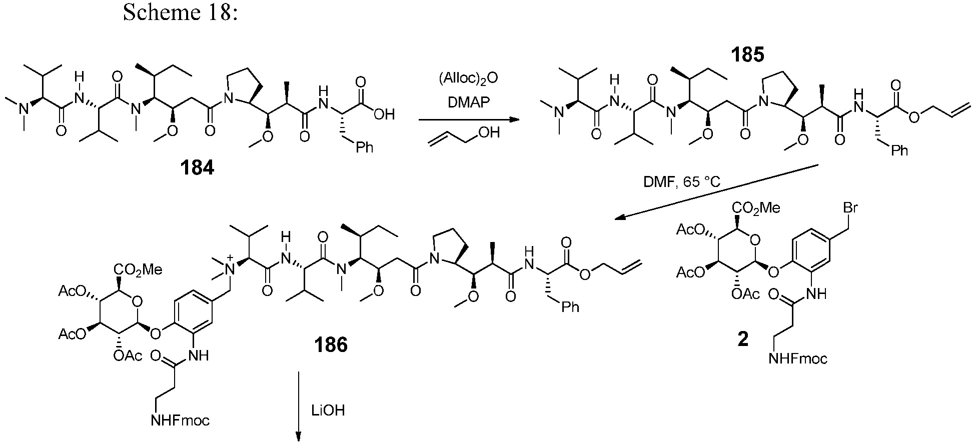

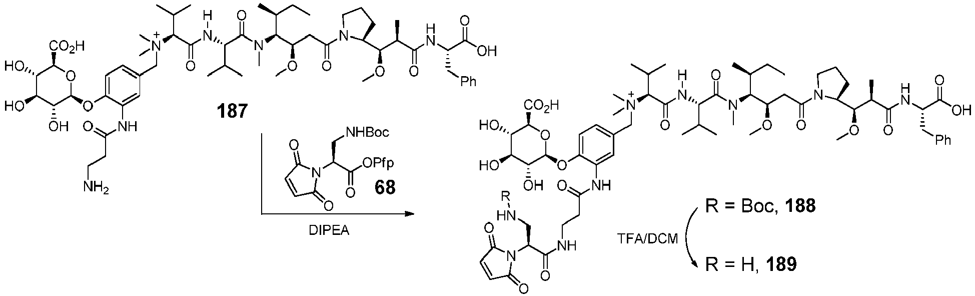

Scheme 18 shows an exemplary synthesis of a drug linker compound for preparation of Ligand Drug Conjugates having the formula of L_{b}'-A₁(BU)-Aₒ-Y(W)-D⁺ wherein L_{b}' is a M¹ moiety, Y is a PAB self-immolative (SI) moiety, W is a carbohydrate (Su) moiety bonded to SI through a glycosidic linkage wherein said linkage is cleavable by a glycosidase, and D⁺ is quaternized Auristatin F. In that drug-linker embodiment of formula L_{b}'-Lₒ-D⁺ the L_{b}'-A₁(BU)- structure represents an exemplary L_{SS} moiety. Compound **189** is analogous to Compound **8,** Compound **14** and Compound **17** of Schemes 1, 4 and 17, respectively, in which quaternized auristatin E or quaternized dolastatin 10, are replaced by quaternized MMAF. MMAF is another tertiary amine-containing drug of the auristatin class of compounds.

Drug-linker compounds replacing Auristatin E, Dolastatin 10 or Auristatin F with compounds of formula **D_{A}, D_{B}, D_{C}, D_{E}, D_{E-1}, D_{E-2}**, **D_{F}, D_{F-1},** or **D**_{**E/F**-3} are prepared in similar fashion to that exemplified in Scheme 1, Scheme 2, Scheme 5, Scheme 16 or Scheme 18 as appropriate.

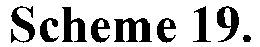

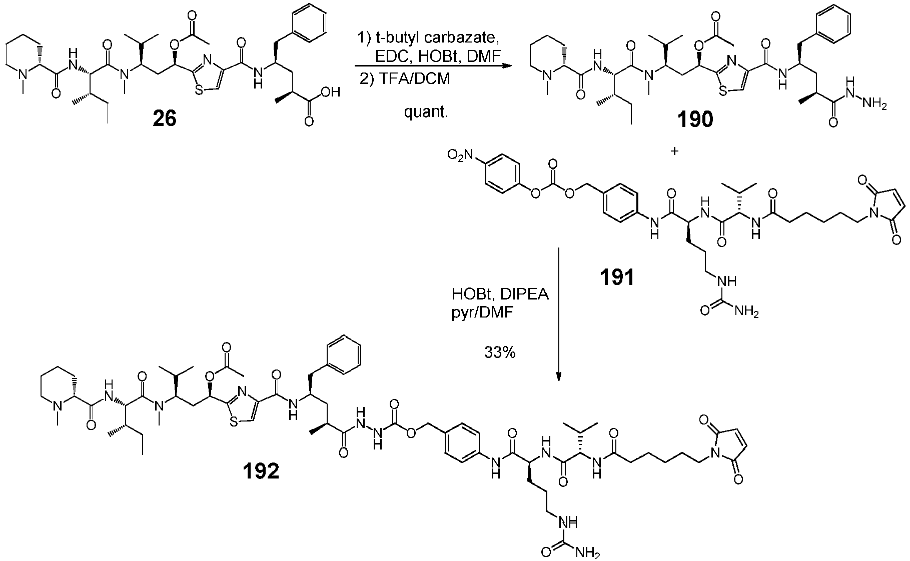

Scheme 19 shows an exemplary synthesis of a Drug Linker compound for preparation of Ligand Drug Conjugates having a non-quaternized tubulysin Drug Unit, wherein the Tubulysin M Drug Unit is attached through is C-terminal component to a Linker Unit by the hydrazine functional group of the Stretcher Unit. A LDC from Compound **172** represents a cleavable conjugate comprised of the moiety -W-Y-, wherein W is the dipeptide -Valine-Citrulline- and is of the general formula M¹-A-W-Y-D or M¹-A-W₂-W₁- PABC-D wherein M¹ is the maleimide moiety, the Stretcher unit A is an alkylene, W is a peptide cleavable unit of formula -W₂-W₁-, wherein W₁ is citrulline and W₂ is valine, PABC is the p-aminobenyloxycarbonyl moiety as the self-immolative Spacer unit Y, the stretcher unit A is an alkylene and the non-quaternized Drug unit D is TubM-NHNH-. A Ligand Drug Conjugate from that Drug-Linker compound is of the general formula L-S-M²-A-W-Y-D or L-S-M²-A-W₂-W₁-PABC-D, wherein M² is a succinimide moiety resulting from conjugate addition of a targeting moiety thiol to M¹ of the Drug Linker compound. An active drug moiety that is released from context-specific proteolysis at W has the structure of TubM-NH-NH₂-.

### EXAMPLES

Any examples falling outside the scope of the appended claims are provided to assist in better understanding the claimed invention.

### General: Chemistry

All commercially available anhydrous solvents were used without further purification. Analytical thin layer chromatography was performed on silica gel 60 F254 aluminum sheets (EMD Chemicals, Gibbstown, NJ). Radial chromatography was performed on Chromatotron apparatus (Harris Research, Palo Alto, CA). Column chromatography was performed on a Biotage Isolera One^{™} flash purification system (Charlotte, NC). Analytical HPLC was performed on a Varian ProStar 210^{™} solvent delivery system configured with a Varian ProStar 330 PDA detector. Samples were eluted over a C12 Phenomenex Synergi^{™} 2.0 × 150 mm, 4 µm, 80 Ǻ reverse-phase column. The acidic mobile phase consisted of acetonitrile and water both containing either 0.05% trifluoroacetic acid or 0.1% formic acid (denoted for each compound). Compounds were eluted with a linear gradient of acidic acetonitrile from 5% at 1 min post injection, to 95% at 11 min, followed by isocratic 95% acetonitrile to 15 min (flow rate = 1.0 mL/min). LC-MS was performed on two different systems. LC-MS system 1 consisted of a ZMD Micromass mass spectrometer interfaced to an HP Agilent 1100 HPLC instrument equipped with a C12 Phenomenex Synergi 2.0 × 150 mm, 4 µm, 80 Å reverse phase column. The acidic eluent consisted of a linear gradient of acetonitrile from 5% to 95% in 0.1% aqueous formic acid over 10 min, followed by isocratic 95% acetonitrile for 5 min (flow rate = 0.4 mL/min). LC-MS system 2 consisted of a Waters Xevo G2^{™} ToF mass spectrometer interfaced to a Waters 2695 Separations Module with a Waters 2996 Photodiode Array Detector; the column, mobile phases, gradient, and flow rate were same as for LC-MS system 1. UPLC-MS system 1 consisted of a Waters SQ mass detector interfaced to an Acquity^{™} Ultra Performance LC equipped with an Acquity UPLC BEH C18 2.1 × 50 mm, 1.7µm reverse phase column. The acidic mobile phase (0.1% formic acid) consisted of a gradient of 3% acetonitrile/97% water to 100% acetonitrile (flow rate = 0.5 mL/min). UPLC-MS system 2 consisted of a Waters Xevo G2 ToF mass spectrometer interfaced to a Waters Acquity H-Class Ultra Performance LC equipped with an Acquity UPLC BEH C18 2.1 × 50 mm, 1.7 µm reverse phase column. The acidic mobile phase (0.1% formic acid) consisted of a gradient of 3% acetonitrile/97% water to 100% acetonitrile (flow rate = 0.7 mL/min). Preparative HPLC was carried out on a Varian ProStar 210 solvent delivery system configured with a Varian ProStar 330 PDA detector. Products were purified over a C12 Phenomenex Synergi 10.0 × 250 mm, 4 µm, 80 Å reverse phase column eluting with 0.1% trifluoroacetic acid in water (solvent A) and 0.1% trifluoroacetic acid in acetonitrile (solvent B). The purification methods generally consisted of linear gradients of solvent A to solvent B, ramping from 90% aqueous solvent A to 10% solvent A. The flow rate was 4.6 mL/min with monitoring at 254 nm. NMR spectral data were collected on a Varian Mercury 400 MHz spectrometer. Coupling constants (J) are reported in hertz.

### General: Biology

***In vitro* assays.** Cells cultured in log-phase growth were seeded for 24 h in 96-well plates containing 150 µL RPMI 1640 supplemented with 20% FBS. Serial dilutions of antibody-drug conjugates in cell culture media were prepared at 4x working concentrations; 50 µL of each dilution was added to the 96-well plates. Following addition of ADC, cells were incubated with test articles for 4 d at 37 °C. After 96 h, growth inhibition was assessed by CellTiter-Glo^{™} (Promega, Madison, WI) and luminescence was measured on a plate reader. The IC₅₀ value, determined in triplicate, is defined here as the concentration that results in a 50% reduction in cell growth relative to untreated controls.

***In vivo* xenograft models.** All experiments were conducted in concordance with the Animal Care and Use Committee in a facility fully accredited by the Association for Assessment and Accreditation of Laboratory Animal Care. Efficacy experiments were conducted in an L540cy Hodgkin's lymphoma xenograft model. L540cy tumor cells, as a cell suspension, were implanted sub-cutaneous in immune-compromised SCID mice. Upon tumor engraftment, mice were randomized to study groups when the average tumor volume reached about 100 mm³. The ADC or controls were dosed once via intraperitoneal injection. Tumor volume as a function of time was determined using the formula (L × W²)/2. Animals were euthanized when tumor volumes reached 1000 mm³. Mice showing durable regressions were terminated around day 100 post implant.

***Ex vivo* stability experiments.** Assessing drug loading of humanized ADCs in rodent plasma samples by the reversed-phase LC-MS was achieved by first isolating the ADCs with IgSelect^{™} resin (GE Healthcare), which selectively binds to the human Fc domain. ADCs were prepared with 8 drugs per antibody of either MDPR-glucuronidequaternary amine-AE **8** or mDPR-Val-Cit-PAB-quaternary amine-AE (mDPR-vcPAB4-AE) **14** using fully reduced cAC10. The ADCs (1.0 mg/mL) were incubated in sterile rat and mouse plasma for 10 days at 37 °C. At eight time points during the incubation, a 50 µL aliquot of each ADC was removed and frozen at -80 °C. Upon completion of the time course, ADCs were purified from each sample and analyzed by reversed-phase HPLC in line with a TOF mass spectrometer to determine the drug : antibody ratio.

**Enzymatic drug release experiments.** Fifteen µL of 10 mM drug-linker stock of **8** in DMA was added to 27 µL of DMSO and quenched with N-acetylcysteine (30 µL of 100 mM stock in PBS) in an Eppendorf tube. 48 µL of PBS was added and the tube was allowed to sit at room temperature for 1 hour. Enzyme stocks were prepared concurrently by dissolving β-glucuronidase (from bovine liver, ≥1,000,000 units/g solid) in 100 mM NaOAc to a final concentration of 1 or 2 mg/mL whereupon 38 µL of the quenched linker solution was combined with 170 µL of fresh enzyme stock and 132 µL of 100 mM NaOAc then incubated at 37 ^{O}C. 25 µL were taken at each time point, added to 150 µL MeOH, sealed and stored at -80 ^{O}C until all time points were taken. Samples were then centrifuged at high speed for 20 minutes and analyzed by UPLC-MS, counting ions for intact quenched linker and released drug.

**Intracellular drug release.** TF1α, L428, and HCT-15 cells were used for the study. Cells that had been cultured under normal conditions were harvested and washed into fresh medium at ~5 × 10⁵ cells/mL. For each treatment condition, 5-15×10⁶ cells were plated in T25 or T150 flasks. cOKT9-5023 (8-drugs/Ab) was added at 1 µg/mL to cultures and they were incubated at 37 °C, 5% CO₂ for 24 hr. For suspension cells, a known volume of cell culture was harvested by centrifugation (500×g, 5 min, 4 °C), and an aliquot of the medium was removed and stored for mass spectral analysis (-20 °C). For adherent cells the medium was carefully removed (an aliquot stored for mass spectral analysis) and the cells lifted with trypsin-EDTA followed by centrifugation (500×g, 5 min, 4 °C) to collect the cell pellet. For both suspension and adherent cells, the harvested cell pellets were resuspended in ice cold PBS and an aliquot was removed for enumeration and determination of average cell diameters using a Vi-Cell XR2.03 cell viability analyzer (Beckman Coulter, Fullerton, CA). The remaining cell suspensions were pelleted (500×g, 5 min) and then washed once more with 1 mL of ice cold PBS. The resulting pellets were stored at -20 °C.

Untreated samples were generated using untreated cell pellets and culture medium for use in calibration curves. Untreated cell pellets were spiked with 4 µL of 25X standard analyte. Both spiked and treated cell pellets were suspended by vortexing in 400 µL of MeOH containing internal standard and placed at -20 °C for 15 min. The samples were then centrifuged at 16,000×g and the supernatants were prepared for solid phase extraction. Untreated culture medium samples (0.5 mL) were spiked with 4 µL of 25X standard. Both spiked and treated culture media were mixed with 10 µL of internal standard and prepared for solid phase extraction. Samples were subjected to solid phase extraction and the recovered drug-containing solutions were dried and reconstituted for quantification by LC-MS/MS. To derive an equation for the quantitation of released drug in the experimental unknown samples, the peak area for each drug standard was divided by the peak area obtained for the internal standard. The resultant peak area ratios were plotted as a function of the standard spiked amounts and the data points were fitted to a curve using linear regression. The peak area ratios obtained for released drug to internal standard in the experimental samples were converted to drug amounts using the derived equation.

### Example 1: (2S,3R,4S,5S,6S)-2-(2-(3-((((9H-fluoren-9-yl)methoxy)carbonyl)amino)propanamido)-4-(bromomethyl)phenoxy)-6-(methoxycarbonyl)tetrahydro-2H-pyran-3,4,5-triyl triacetate (2):

A flame dried flask was charged with known *(*Bioconjugate Chem. 2006, 17, 831-840) glucuronide linker fragment **(1, 210** mg, 281 µmol) in 4.5 mL anhydrous THF. The solution was stirred at room temperature under N₂. Triphenylphosphine (111 mg, 421.5 µmol) and N-bromosuccinimide (75 mg, 421.5 µmol) were added sequentially and the solution was stirred for 2 hours. The reaction was condensed under reduced pressure and purified over silica via a Biotage column (Hexanes/EtOAc, 30%-50%-70%) to provide **2** (222 mg, 97%). Analytical UPLC-MS (system 1): *t*ᵣ = 2.36 min, *m*/*z* (ES+) found 811.34.

### Example 2: (S)-N-(3-(3-((((9H-fluoren-9-yl)methoxy)carbonyl)amino)propanamido)-4-(((2S,3R,4S,5S,6S)-3,4,5-triacetoxy-6-(methoxycarbonyl)tetrahydro-2H-pyran-2-yl)oxy)benzyl)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4 yl)(methyl)amino)-3-methyl-1-oxobutan-2-yl)amino)-N,N,3-trimethyl-1-oxobutan-2-aminium (4):

A pressure vessel was charged with brominated Stretcher-Glucuronide unit intermediate **(2,** 111 mg, 137 µmol) of Example 1and auristatin E **(3,** 100 mg, 137 µmol) in anhydrous 2-butanone (4.2 mL). The reaction vessel was flushed with N₂ and sealed. The reaction was then stirred and heated to 80 °C for 12 hours. The resulting mixture was cooled, condensed under reduced pressure, and purified over silica via a Biotage column (CH₂Cl₂/MeOH, 2%-20%-100%) to provide **4** (113 mg, 56%). Analytical UPLC-MS (system 1): *t*ᵣ = 2.02 min, *m*/*z* (ES+) found 1462.53.

Example 3: *(S)-N-(3-(3-aminopropanamido)-4-(((2S,3R,4S,5S,6S)-6-carboxy-3,4,5-trihydroxytetrahydro-2H-pyran-2-yl)oxy)benzyl)-1-(((S)-1-(((3R,4S,5S)-1-((S))-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxobutan-2-aminium **(5):*** A flask was charged with glucuronide-AE **(4,** 113 mg, 77 µmol) in THF (1.3 mL) and MeOH (1.3 mL). This solution was stirred under N₂ and cooled to 0 °C. LiOH · H₂O (19 mg, 462 µmol) was solubilized in H₂O (1.3 mL) then added dropwise. The reaction was allowed to warm to room temperature and stirred for 1 hour. The reaction was then quenched with acetic acid (26 µL, 462 µmol) and condensed under reduced pressure. The residue was taken up in minimal DMSO and purified by preparative LC to provide **5** (34 mg, 40%). Analytical UPLC-MS (system 1): *t*ᵣ = 1.20 min, *m*/*z* (ES+) found 1100.51.

### Example 4: (S)-N-(3-(3-((S)-3-((tert-butoxycarbonyl)amino)-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-triimethyl-1-oxobutan-2-aminium (7):

A flask was charged with deprotected glucuronide-AE **(5,** 34 mg, 31 µmol) in anhydrous DMF (0.31 mL). mDPR(Boc)-OSu **(6,** 14 mg, 37 µmol) was added under N₂. N,N-Diisopropylethylamine (27 µL, 155 µmol) was added and the reaction was stirred at room temperature for 3 hours. The reaction was then quenched with acetic acid (27 µL) and purified by preparative LC to provide **7** (29 mg, 68%). Analytical UPLC-MS (system 1): *t*ᵣ = 1.52 min, *m*/*z* (ES+) found 1366.40.

### Example 5: (S)-N-(3-(3-((S)-3-amino-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1yl)-3-methoxy-5-methyl-1-oxoheptan-4-yl)(methyl)amino)-3-methyl-1-oxobutan-2-yl)amino)-N,N,3-trimethyl-1-oxobutan-2-aminium (8):

A flask containing mDPR(Boc)-Glucuronide-AE (7, 29 mg, 21 µmol) was cooled to 0 °C under N₂. A solution of 10% TFA in CH₂Cl₂ (1.1 mL) was added dropwise and stirred for 4 hours. The reaction was then taken up in DMSO, condensed under reduced pressure, and purified by preparative LC to yield **8** (20 mg, 75%). Analytical UPLC-MS: *t*ᵣ = 1.23 min, *m*/*z* (ES+) found 1266.39. Analytical HPLC-MS (system 2): *t*ᵣ = 9.53 min, *m*/*z* (ES+) found 1266.70.

### Example 6: (9H-fluoren-9-yl)methyl ((S)-1-(((S)-1-((4-(bromomethyl)phenyl)amino)-1-oxo-5-ureidopentan-2-yl)amino)-3-methyl-1-oxobutan-2-yl)carbamate (10):

Dipeptide Fmoc-Val-Cit-PAB **(9,** 75 mg, 125 µmol) was added to a flame dried flask under N₂ and cooled to 0 °C. A solution of 33% HBr in acetic acid (1.3 mL) was added dropwise. The solution was allowed to warm to room temperature and stirred for 2 hours. Remaining HBr was then blown off via a stream of N₂ and the reaction was further condensed under reduced pressure. The resulting residue was taken up in THF and recondensed 3 times then carried forward without further purification. Analytical UPLC-MS (system 1): *t*ᵣ = 2.19 min, *m*/*z* (ES+) found 664.47.

### Example 7: (S))-N-(4-((S))-2-((S)-2-((((9H-fluoren-9-yl)methoxy)carbonyl)amino)-3-methylbutanamido)-5-ureidopentanamido)benzyl)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxobutan-2-aminium (11):

To a pressure vessel charged with crude brominated dipeptide **(10,** 48 mg, 72 µmol) was added auristatin E **(3,** 30 mg, 41 µmol) and anhydrous 2-butanone (2.2 mL). The vessel was flushed with N₂ and sealed. The reaction was then heated to 80 °C with stirring for 3 hours. Observing no reaction by LC-MS, N,N-Diisopropylethylamine (7 µL, 41 µmol) was added and the reaction was resealed for 12 hours. The reaction mixture was condensed under reduced pressure and purified via preparative LC to provide **11** (13 mg, 24%). Analytical UPLC-MS (system 1): *t*ᵣ = 1.88 min, *m*/*z* (ES+) found 1315.57.

### Example 8: (S)-N-(4-((S)-2-((S)-2-amino-3-methylbutanamido)-5-ureidopentanamido)benzyl)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxobutan-2-aminium (12):

Fmoc-Val-Cit-PAB-AE **(11,** 13 mg, 10 µmol) was added to a flask with anhydrous DMF (0.4 mL). Piperidine (0.1 mL) was added under N₂. The reaction was stirred at room temperature for 30 minutes. The reaction was directly purified via preparative LC to provide **12** (8 mg, 74%). Analytical UPLC-MS (system 1): *t*ᵣ = 1.27 min, *m*/*z* (ES+) found 1093.62.

### Example 9: (S))-N-(4-((7S,10S,13S)-7-(2,5-dioxo-2,5-dihydro-1H-pyrrol-1-yl)-10-isopropyl-2,2-dimethyl-4,8,11-trioxo-13-(3-ureidopropyl)-3-oxa-5,9,12-triazatetradecanamido)benzyl)-1-(((S))-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxobutan-2-aminium (13):

A flask was charged with Val-Cit-PAB-AE **(12, 8** mg, 7.3 µmol) in anhydrous DMF (146 µL). mDPR(Boc)-OSu **(6, 3.3** mg, 8.8 µmol) was added under N₂. N,N-Diisopropylethylamine (6 µL, 35 µmol) was added and the reaction was stirred at room temperature for 3 hours. The reaction was then quenched with acetic acid (6 µL) and purified by preparative LC to provide **13** (2 mg, 17%). Analytical UPLC-MS (system 1): *t*ᵣ = 1.66 min, *m*/*z* (ES+) found 1359.51.

### Example 10: (S)-N-(4-((S)-2-((S)-2-((S)-3-amino-2-(2,5-dioxo-2,5-dihydro-1H-pyrrol-1yl)propanamido)-3-methylbutanamido)-5-ureidopentanamido)benzyl)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxobutan-2-aminium (14):

A flask containing mDPR(Boc)-Val-Cit-PAB-AE **(13,** 2 mg, 1.5 µmol) was cooled to 0 °C under N₂. A solution of 10% TFA in CH₂Cl₂ (294 µL) was added dropwise and stirred for 3 hours. The reaction was then taken up in DMSO, condensed under reduced pressure, and purified by preparative LC to yield **14** (2.2 mg, 99%). Analytical UPLC-MS (system 1): *t*ᵣ = 1.30 min, *m*/*z* (ES+) found 1259.69. Analytical HPLC-MS (system 2): *t*ᵣ = 9.22 min, *m*/*z* (ES+) found 1259.78.

### Example 11: N-(3-(3-((((9H-fluoren-9-yl)methoxy)carbonyl)amino)-propanamido)-4-(((2S,3R,4S,5S,6S)-3,4,5-triacetoxy-6-(methoxycarbonyl)tetrahydro-2H-pyran-2-yl)oxy)benzyl)-N-methyl-3-(methyl(2-(9-methylphenazine-1-carboxamido)ethyl)amino)-N-(2-(9-methylphenazine-1-carboxamido)ethyl)propan-1-aminium (16):

Brominated Stretcher-Glucuronide unit intermediate **2** (25 mg, 30 µmol) of Example 1 and symmetrical phenazine dimer **15** (J. Med. Chem., 2001, 44, 1407) (19 mg, 30 µmol) were dissolved in anhydrous butanone (0.48 mL) and anhydrous dimethylformamide (0.48 mL). The reaction was purged with nitrogen and heated to 80 °C in a sealed tube for 18 h. LC/MS analysis revealed product in the presence of starting material and bis-alkylated byproduct. The crude material was then concentrated and purified by preparative HPLC to provide quaternary amine **16** (8.5 mg, 21%). Analytical HPLC-MS (system 1): *t*ᵣ = 12.52 min, *m*/*z* (ES+) found 1359.19.

### Example 12: N-(3-(3-aminopropanamido)-4-(((2S,3R,4S,5S,6S)-6-carboxy-3,4,5-trihydroxytetrahydro-2H-pyran-2-yl)oxy)benzyl)-N-methyl-3-(methyl(2-(9-methylphenazine-1-carboxamido)ethyl)amino)-N-(2-(9-methylphenazine-1-carboxamido)ethyl)propan-1-aminium (17):

A flask was charged with quaternary amine linker **16** (8.5 mg, 6.0 µmol) dissolved in methanol (0.2 mL) and tetrahydrofuran (0.2 mL), then cooled under nitrogen to 0 °C in an ice bath. Lithium hydroxide monohydrate (0.75 mg, 18 µmol) was dissolved in water (0.1 mL) and the solution added dropwise to the reaction flask. Additional water (0.2 mL), methanol (0.2 mL), and tetrahydrofuran (0.2 mL) were added. The reaction was then stirred for 1.5 hours at 0 °C, then additional lithium hydroxide (0.75 mg, 18 µmol) was added in 0.1 mL water to the reaction. At 3 hours, the reaction was complete by LC, then quenched with glacial acetic acid (2.1 µL) and concentrated by rotary evaporation. The crude material was purified by preparative HPLC to provide fully deprotected quaternary amine linker **17** (3.7 mg, 62%). Analytical HPLC-MS (system 1): *t*ᵣ = 10.26 min, *m*/*z* (ES+) found 997.45.

### Example 13: N-(4-(((2S,3R,4S,5S,6S)-6-carboxy-3,4,5-trihydroxytetrahydro-2H-pyran-2-yl)oxy)-3-(3-(6-(2,5-dioxo-2,5-dihydro-1H-pyrrol-1-yl)hexanamido)propanamido)benzyl)-N-methyl-3-(methyl(2-(9-methylphenazine-1-carboxamido)ethyl)amino)-N-(2-(9-methylphenazine-1-carboxamido)ethyl)propan-1-aminium (19):

To a stirred solution of linker **17** (3.7 mg, 3.6 µmol) in anhydrous dimethylformamide (0.18 mL) under nitrogen was added maleimidocaproyl-succinimide ester **18** (1.2 mg, 4.0 µmol) as a solution in anhydrous dimethylformamide (0.18 mL), followed by diisopropylethylamide (3.8 µL). After stirring the reaction for 1 hour at room temperature under nitrogen, additional **18** (0.6 mg) was added in 90 µL dimethylformamide, followed by 90 µL dimethylsulfoxide to facilitate dissolution of all reactants. The reaction was stirred at room temperature under nitrogen for an additional two hours, then purified by multiple preparative HPLC injections to remove residual **18** from the product, quaternary amine-phenazine dimer linker **19** (2.1 mg, 45%). Analytical HPLC-MS (system 1): *t*ᵣ = 10.76 min, *m*/*z* (ES+) found 1190.35.

### Example 14: (S)-2-((S)-2-((tert-butoxycarbonyl)amino)-3-methylbutanamido)-propanoic acid (22):

A flask was charged with Boc-Val-OSu **(20,** 1.0 g, 3.18 mmol) and H-Ala-OH **(21, 312** mg, 3.5 mmol) in anhydrous dimethylformamide (10.6 mL). N,N-diisopropylethylamine (1.1 mL, 6.4 mmol) was added and the solution was stirred under N₂ at 50 ^{O}C for 12 hours. The reaction was taken up in DMSO and purified by preparative HPLC to yield **22** (808 mg, 88%). Analytical UPLC-MS (system 1): *t*ᵣ = 1.38 min, *m*/*z* (ES+) found 289.60.

### Example 15: tert-butyl ((S)-1-(((S)-1-((4-(hydroxymethyl)phenyl)amino)-1-oxopropan-2yl)amino)-3-methyl-1-oxobutan-2-yl)carbamate (24):

A flame-dried flask was charged with dipeptide **22** (808 mg, 2.8 mmol) and 4-aminobenzoic alcohol **23** (345 mg, 2.8 mmol) in anhydrous dichloromethane (14 mL). EEDQ (762 mg, 3.1 mmol) was added as a solid and stirred under nitrogen at room temperature for 12 h. The reaction was then condensed and purified over silica via a Biotage column (CH₂Cl₂/MeOH, 0%-10%) to provide **24** (660 mg, 60%). Analytical UPLC-MS (system 1): *t*ᵣ = 1.51 min, *m*/*z* (ES+) found 394.51.

### Example 16: tert-butyl ((S)-1-(((S)-1-((4-(bromomethyl)phenyl)amino)-1-oxopropan-2-yl)amino)-3-methyl-1-oxobutan-2-yl)carbamate (25):

A flask containing Boc-Val-Ala-PABA-OH **(24, 100** mg, 254 µmol), N-bromosuccinimide (68 mg, 381 µmol), and triphenylphosphine (100 mg, 381 µmol) was flushed with nitrogen. The reaction was taken up in THF (4 mL) and stirred for 12 hours. The reaction was condensed and purified over silica via a Biotage column (Hexanes/EtOAc, 10%-100%) to provide **25** (94 mg, 81%). Analytical UPLC-MS (system 1): *t*ᵣ = 2.09 min, *m*/*z* (ES+) found 456.10.

### Example 17: (2S,4R)-tert-butyl 4-(2-((1R,3R)-1-acetoxy-3-((2S,3S)-N,3-dimethyl-2-((R)-1-methylpiperidine-2-carboxamido)pentanamido)-4-methylpentyl)thiazole-4-carboxamido)-2-methyl-5-phenylpentanoate (27):

A flame dried flask was charged with Tubulysin M **(26,** 10 mg, 14 µmol) in anhydrous DCM (0.7 mL) and t-butanol (0.7 mL). Diisopropylcarbodiimide (3.2 µL, 21 µmol) and DMAP (0.08 mg, 0.7 µmol) were added and the reaction was stirred at room temperature for 48 hours. The reaction was condensed, taken up in DMSO, and purified by preparative HPLC to yield **27** (3.5 mg, 32%). Analytical UPLC-MS (system 1): *t*ᵣ = 1.35 min, *m*/*z* (ES+) found 784.56.

### Example 18: (2R)-2-(((2S,3S)-1-(((1R,3R)-1-acetoxy-1-(4-(((2R,4S)-5-(tert-butoxy)-4-methyl-5-oxo-1-phenylpentan-2-yl)carbamoyl)thiazol-2-yl)-4-methylpentan-3-yl)(methyl)amino)-3-methyl-1-oxopentan-2-yl)carbamoyl)-1-(4-((S))-2-((S))-2-((tert-butoxycarbonyl)amino)-3-methylbutanamido)propanamido)benzyl)-1-methylpiperidin-1-ium (28) :

A pressure vessel was charged with Boc-Val-Ala-PAB-Br **(25, 3.5** mg, 7.7 µmol) and protected Tubulysin M (4.0 mg, 5.1 µmol) in anhydrous butanone (0.765 mL). N,N-diisopropylethylamine was added (1.8 µL, 10 µmol) and the reaction was flushed with nitrogen. The vessel was sealed and allowed to stir at 80 ^{O}C for 12 hours. The reaction was condensed, taken up in DMSO, and purified by preparative HPLC to yield **28** (3.5 mg, 58%). Analytical UPLC-MS (system 1): *t*ᵣ = 1.51 min, *m*/*z* (ES+) found 1159.58.

### Example 19: (2R)-2-(((2S,3S)-1-(((1R,3R)-1-acetoxy-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(4-((S))-2-((S))-2-amino-3-methylbutanamido)propanamido)benzyl)-1-methylpiperidin-1-ium (29):

A flask containing Boc-Val-Ala-PAB-TubM-OtBu **(28, 3.5** mg, 3 µmol) was cooled to 0 °C under nitrogen. A solution of 10% TFA in CH₂Cl₂ (0.3 mL) was added dropwise and stirred for 4 hours. The reaction was condensed, taken up in DMSO, and purified by preparative HPLC to yield **29** (1.9 mg, 63%). Analytical UPLC-MS (system 1): *t*ᵣ = 1.05 min, *m*/*z* (ES+) found 1003.60.

### Example 20: (2R)-2-(((2S,3S)-1-(((1R,3R)-1-acetoxy-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(4-((S))-2-((S))-2-(6-(2,5-dioxo-2,5-dihydro-1H-pyrrol-1-yl)hexanamido)-3-methylbutanamido)propanamido)benzyl)-1-methylpiperidin-1-ium (30):

MC-OSu **(18, 0.6** mg, 2 µmol) was taken up in anhydrous dimethylformamide (0.2 mL) and added to a flask containing Val-Ala-PAB-TubM **(29, 1.9** mg, 2 µmol). N,N-diisopropylethylamine (1.0 mg, 8 µmol) was added and the reaction was stirred under nitrogen for 3 hours. The reaction was taken up in DMSO, and purified by preparative HPLC to yield quaternary amine tubulysin linker **30** (1.2 mg, 53%). Analytical UPLC-MS (system 1): *t*ᵣ = 1.25 min, *m*/*z* (ES+) found 1196.45.

### Example 21: (2R)-2-(((2S,3S)-1-(((1R,3R)-1-acetoxy-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(4-((7S,10S,13S)-7-(2,5-dioxo-2,5-dihydro-1H-pyrrol-1-yl)-10-isopropyl-2,2,13-trimethyl-4,8,11-trioxo-3-oxa-5,9,12-triazatetradecanamido)-benzyl)-1-methylpiperidin-1-ium (31):

MDPR(Boc)-OSu **(6,** 1.3 mg, 3.5 µmol) was taken up in anhydrous dimethylformamide (0.3 mL) and added to a flask containing Val-Ala-PAB-TubM **(29,** 3.2 mg, 3.2 µmol). N,N-diisopropylethylamine (1.6 mg, 13 µmol) was added and the reaction was stirred under nitrogen for 3 hours. The reaction was taken up in DMSO, and purified by preparative HPLC to yield **31** (2.0 mg, 49%). Analytical UPLC-MS (system 2): *t*ᵣ = 1.35 min, *m*/*z* (ES+) found 1269.76.

### Example 22: (2R)-2-(((2S,3S)-1-(((1R,3R)-1-acetoxy-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(4-((S))-2-((S))-2-((S)-3-amino-2-(2,5-dioxo-2,5-dihydro-1H-pyrrol-1-yl)propanamido)-3-methylbutanamido)propanamido)benzyl)-1-methylpiperidin-1-ium (32):

A flask containing MDPR(Boc)-Val-Ala-PAB-TubM **(31,** 2 mg, 1.6 µmol) was cooled to 0 °C under nitrogen. A solution of 10% TFA in CH₂Cl₂ (1.6 mL) was added dropwise and stirred for 4 hours. The reaction was condensed, taken up in DMSO, and purified by preparative HPLC to yield **32** (1.0 mg, 54%). Analytical UPLC-MS (system 2): *t*ᵣ = 1.02 min, *m*/*z* (ES+) found 1169.72.

### Example 23: 2-(3-(3-((((9H-fluoren-9-yl)methoxy)carbonyl)amino)propanamido)-4-(((3R,4S,5S,6S)-3,4,5-triacetoxy-6-(methoxycarbonyl)tetrahydro-2H-pyran-2-yl)oxy)benzyl)-2-(4-(4,5-dimethoxy-2-(quinoline-3-carboxamido)benzamido)phenethyl)-6,7-dimethoxy-1,2,3,4-tetrahydroisoquinolin-2-ium (34):

A pressure vessel was charged brominated Stretcher-Glucuronide unit intermediate **(2,** 78 mg, 96 µmol) of Example 1 and Tariquidar **(33,** 62 mg, 96 µmol) in anhydrous 2-butanone (2.9 mL). The reaction vessel was then purged with nitrogen and sealed. The reaction was then stirred and heated to 80 °C for 4 hours, at which time LC/MS revealed conversion to quaternized product. The resulting was cooled, condensed under reduced pressure, and purified by preparative HPLC to provide **34** (123 mg, 90%). Analytical UPLC-MS (system 1): *t*ᵣ = 2.05 min, *m*/*z* (ES+) found 1377.74. Analytical HPLC-MS (system 2): *t*ᵣ = 11.23 min, *m*/*z* (ES+) found 1377.47.

### Example 24: 2-(3-(3-aminopropanamido)-4-(((3R,4S,5S,6S)-6-carboxy-3,4,5-trihydroxytetrahydro-2H-pyran-2-yl)oxy)benzyl)-2-(4-(4,5-dimethoxy-2-(quinoline-3-carboxamido)benzamido)phenethyl)-6,7-dimethoxy-1,2,3,4-tetrahydroisoquinolin-2-ium (35):

A flask was charged with quaternary amine linker **34** (123 mg, 83 µmol) dissolved in methanol (1.4 mL) and tetrahydrofuran (1.4 mL), then cooled under nitrogen to 0 °C in an ice bath. Lithium hydroxide monohydrate (21 mg, 500 µmol) was dissolved in water (1.4 mL) and the solution added dropwise to the reaction flask. The reaction was then stirred for 2 hours at 0 °C, then additional lithium hydroxide (7 mg, 170 µmol) was added in 0.45 mL water to the reaction. At 3 hours, the reaction was complete by LC, quenched with glacial acetic acid (28 µL) and concentrated by rotary evaporation. The crude material was purified by preparative HPLC to provide fully deprotected quaternary amine linker **35** (71 mg, 85%). Analytical UPLC-MS (system 1): *t*ᵣ = 1.29 min, *m*/*z* (ES+) found 1015.74.

### Example 25: 2-(3-(3-((S)-3-amino-2-(2,5-dioxo-2,5-dihydro-1H-pyrrol-1-yl)propanamido)propanamido)-4-(((3R,4S,5S,6S)-6-carboxy-3,4,5-trihydroxytetrahydro-2H-pyran-2-yl)oxy)benzyl)-2-(4-(4,5-dimethoxy-2-(quinoline-3-carboxamido)benzamido)-phenethyl)-6,7-dimethoxy-1,2,3,4-tetrahydroisoquinolin-2-ium (36):

A solution of MDPR(Boc)-OSu **6** (20 mg, 52 µmol) in anhydrous dimethylformamide (0.9 mL) was added to a flask containing deprotected glucuronide-Tariquidar **35** (40 mg, 35 µmol). N,N-Diisopropylethylamine (30 µL) was added and the reaction was stirred at room temperature under nitrogen for 3 hours, at which time LC-MS revealed conversion to product. The crude reaction product was purified by preparative HPLC to provide the coupled product, MDPR(Boc)-glucuronide-Tariquidar (30 mg, 67%). Analytical HPLC-MS (system 2): *t*ᵣ = 9.99 min, *m*/*z* (ES+) found 1281.45. A flask containing mDPR(Boc)-glucuronide-Tariquidar (28 mg, 20 µmol) was cooled to 0 °C under nitrogen. A solution of 10% TFA in CH₂Cl₂ (2 mL) was added dropwise and stirred for 1 hour at room temperature, at which time LC-MS indicated complete deprotection. The reaction was diluted in 2 mL DMSO, concentrated by rotary evaporation and purified by preparative HPLC to yield **36** (20 mg, quant.). Analytical UPLC-MS (system 1): *t*ᵣ = 1.30 min, *m*/*z* (ES+) found 1181.64. Analytical HPLC-MS (system 2): *t*ᵣ = 8.95 min, *m*/*z* (ES+) found 1181.41.

### Example 26: General procedure for preparing tubuvaline ether intermediates

Etherification: A flame-dried flask is charged with the Boc-protected tubuvaline (J. Org. Chem., 2008, 73, 4362-4369) intermediate **41** in anhydrous tetrahydrofuran (50 mM), to which was added 18-crown-6 (2.0 equivalents) and cooled to -78°C. Potassium hexamethyldisilazide (1.5 equivalents) as a 1 M solution in tetrahydrofuran is added dropwise and the reaction is then stirred for 1 hour at -78°C under nitrogen. Iodoalkane (2-5 equivalents) is then added and the reaction slowly warmed to room temperature and followed by UPLC/MS. Once the starting material is consumed, the reaction is cooled on ice and quenched with saturated ammonium chloride and diluted in dichloromethane (10 volumes). The organic layer is washed with 0.1 M HCl and the resulting aqueous phase is extracted twice with dichloromethane. The combined organics are then dried over sodium sulfate, filtered, and concentrated to dryness. Purification of the crude BOC-protected O-alkylated ethyl ester products (BOC-Tuv(O-ether)-OEt) is achieved by flash chromatography over silica gel or preparative HPLC. Compounds **42, 43** and **44** (Scheme 7) were prepared to exemplify the general procedure.

Ethyl 2-((1R,3R)-3-((tert-butoxycarbonyl)(methyl)amino)-1-methoxy-4-methylpentyl)thiazole-4-carboxylate **(42):** UPLC-MS (system 2): *t*ᵣ = 1.62 min, *m*/*z* (ES+) calculated 401.21, found 401.28.

Ethyl 2-((1R,3R)-3-((tert-butoxycarbonyl)(methyl)amino)-1-ethoxy-4-methylpentyl)thiazole-4-carboxylate **(43):** Tubuvaline intermediate 41 (170 mg, 440 µmol) was O-ethylated as described above with iodoethane (89 µl, 880 µmol) to provide the title compound after silica gel purification eluting methanol and dichloromethane mixtures. UPLC-MS (system 2): *t*ᵣ = 1.66 min, *m*/*z* (ES+) calculated 415.23 (M+H)⁺, found 415.29

Ethyl 2-((1R,3R)-3-((tert-butoxycarbonyl)(methyl)amino)-4-methyl-1-propoxypentyl)thiazole-4-carboxylate **(44):** UPLC-MS (system 2): *t*ᵣ = 1.77 min, *m*/*z* (ES+) calculated 428.23 (M+H)⁺, found 451.30 (M+Na)⁺. ¹H NMR (1:1 mix of rotamers, CDCl₃) δ (ppm) 0.91 (m, 9H), 1.40 (t, *J* = 7.0 Hz, 3H), 1.47 (two s due to rotamers, 9H), 1.64 (m, 3H), 1.87 (m, 2H), 2.74 (m, 3H), 3.42 (m, 2H), 4.10 (m, 1H), 4.42 (q, *J* = 7.0 Hz, 2H), 4.50 (m, 1H), 8.14 (two s due to rotamers, 1H).

Deprotection: Saponification of the purified O-alkylated products (BOC-Tuv(O-ether)-OEt) to provide the corresponding tubuvaline ether free acid compounds (BOC-Tuv(O-ether)-OH) is carried out at 20 mM reaction concentration using a 1:1:1 solvent mixture of tetrahydrofuran:methanol:water mixture as follows. The O-alkylated tubuvaline intermediates are dissolved in 1 volume each tetrahydrofuran and methanol. The mixture is then cooled in an ice bath at 0°C. Lithium hydroxide monohydrate (2-3 equivalents) is dissolved in 1 volume of distilled water and added dropwise to the reaction flask, with stirring at 0°C. The reaction is then allowed to warm up to room temperature and monitored by UPLC/MS. Once the starting material is converted to free acid, the reaction is quenched with glacial acetic acid (2-3 equivalents) and concentrated by rotary evaporation. The crude carboxylic acids are then purified by preparative HPLC. Compounds **45, 46** and **47** (Scheme 7) were prepared from Compounds **42, 43** and **44,** respectively, to exemplify the general procedure.

Ethyl 2-((1R,3R)-3-((tert-butoxycarbonyl)(methyl)amino)-1-methoxy-4-methylpentyl)thiazole-4-carboxylate **(45):** UPLC-MS (system 1): *t*ᵣ = 1.47 min, *m*/*z* (ES+) calculated 373.18, found 373.41. ¹H NMR (1:1 mix of rotamers, CDCl₃) δ (ppm) 0.87 (dd, *J* = 6.7, 2.0 Hz, 3H), 0.96 (dd, *J* = 6.7, 1.2 Hz, 3H), 1.49 (two s due to rotamers, 9H), 1.67 (m, 1H), 1.85 (m, 1H), 2.01 (m, 1H), 2.70 (m, 3H), 3.41 (s, 3H), 4.12 (m, 1H), 4.36 (first rotamer, dd, *J* = 10.5, 2.3 Hz, 0.5H), 4.48 (second rotamer, d, *J* = 8.6 Hz, 0.5H), 8.28 (two s due to rotamers, 1H).

Ethyl 2-((1R,3R)-3-((tert-butoxycarbonyl)(methyl)amino)-1-ethoxy-4-methylpentyl)thiazole-4-carboxylate **(46):** UPLC-MS (system 2): *t*ᵣ = 1.48 min, m/z (ES+) calculated 387.20 (M+H)⁺, found 387.26. ¹H NMR (CDCl₃) δ (ppm) 0.88 (dd, *J* = 6.7, 2.0 Hz, 3H), 0.96 (d, *J* = 6.6 Hz, 3H), 1.49 (two s from rotamers, 9H), 1.68 (m, 1H), 1.86 (m, 1H), 2.00 (m, 1H), 2.69 (m, 3H), 3.53 (m, 2H), 4.09 (m, 1H), 4.43 (first rotamer, dd, *J* = 10.2, 2.7 Hz, 0.5H), 4.54 (second rotamer, d, *J* = 7.0 Hz, 0.5H), 8.24 (two s due to rotamers, 1H).

Ethyl 2-((1R,3R)-3-((tert-butoxycarbonyl)(methyl)amino)-4-methyl-1-propoxypentyl)thiazole-4-carboxylate **(47):** UPLC-MS (system 2): *t*ᵣ = 1.58 min, *m*/*z* (ES+) calculated 401.21 (M+H)⁺, found 401.28 (M+Na)⁺.

### Example 27: General procedure for introduction of the tubuphenylalanine component by amide coupling.

Peptide Coupling: The O-alkylated tubuvaline free acids (BOC-Tuv(O-ether)-OH) of Example 26 are pre-activated by dissolution in anhydrous dimethylformamide (25-50 mM) and addition of HATU (2.4 equivalents) and DIPEA (5 equivalents). The reaction mixture is then stirred under nitrogen at room temperature for 10 minutes. The activated acid is then added to tubuphenylalanine allyl ester **40** (Org. Lett., 2007, 9, 1605-1607) and the reaction is then stirred at an ambient temperature under nitrogen, with progress monitored by UPLC/MS. Upon reaction completion, glacial acetic acid (14 equivalents) is then added and the product (BOC-Tuv(O-ether)-Tup-O-allyl) is purified by preparative HPLC. Compounds **48, 49** and **50** (Scheme 7) were prepared from Compounds **45, 46** and **47,** respectively, to exemplify the general procedure.

(2S,4R)-allyl 4-(2-((1R,3R)-3-((tert-butoxycarbonyl)(methyl)amino)-1-methoxy-4-methylpentyl)thiazole-4-carboxamido)-2-methyl-5-phenylpentanoate **(48):** UPLC-MS (system 1): *t*ᵣ = 1.96 min, *m*/*z* (ES+) calculated 602.33 (M+H)⁺, found 602.26.

(2S,4R)-allyl 4-(2-((1R,3R)-3-((tert-butoxycarbonyl)(methyl)amino)-1-ethoxy-4-methylpentyl)thiazole-4-carboxamido)-2-methyl-5-phenylpentanoate **(49):** UPLC-MS (system 2): *t*ᵣ = 1.84 min, *m*/*z* (ES+) calculated 616.34 (M+H)⁺, found 616.43.

(2S,4R)-allyl 4-(2-((1R,3R)-3-((tert-butoxycarbonyl)(methyl)amino)-4-methyl-1-propoxypentyl)thiazole-4-carboxamido)-2-methyl-5-phenylpentanoate **(50):** UPLC-MS (system 2): *t*ᵣ = 2.00 min, *m*/*z* (ES+) calculated 630.36 (M+H)⁺, found 630.45. ¹H NMR (CDCl₃) δ (ppm) 0.94 (m, 9H), 1.19 (d, J = 7.4 Hz, 3H), 1.49 (s, 9H), 1.64 (m, 5H), 1.84 (m, 1H), 2.03 (m, 2H), 2.63 (m, 1H), 2.73 (m, 3H), 2.93 (m, 2H), 3.41 (m, 2H), 4.07 (m, 2H), 4.29 (m, 1H), 4.41 (m, 2H), 4.55 (m, 2H), 5.25 (m, 2H), 5.88 (m, 1H), 7.24 (m, 5H), 8.05 (two s from rotamers, 1H).

Deprotection: The purified O-alkylated tubuvaline-tubuphenylalanine di-peptide (BOC-Tuv(O-ether)-Tup-O-allyl) products are deprotected to reveal the tubuvaline secondary amine functional group under acidic conditions with 10% TFA in dichloromethane (25 mM). Specifically, the starting material is dissolved in anhydrous dichloromethane (9 volumes) and stirred under nitrogen at 0°C. Trifluoroacetic acid (1 volume) is then added dropwise to the stirred solution. The reaction is warmed slowly to room temperature and monitored by UPLC/MS. Upon completion, the reaction is concentrated by rotary evaporation and pumped down on a vacuum line overnight. The free amines (NH(CH₃)-Tuv(O-ether)-Tup-O-allyl) are carried forward without further purification. Compounds **51, 52** and **53** (Scheme 7) were prepared from Compounds **48, 49** and **50,** respectively, to exemplify the general procedure.

(2S,4R)-allyl 4-(2-((1R,3R)-1-methoxy-4-methyl-3-(methylamino)pentyl)thiazole-4-carboxamido)-2-methyl-5-phenylpentanoate **(51):** UPLC-MS (system 1): *t*ᵣ = 1.17 min, *m*/*z* (ES+) calculated 524.26 (M+Na)⁺, found 524.27.

(2S,4R)-allyl 4-(2-((1R,3R)-1-ethoxy-4-methyl-3-(methylamino)pentyl)thiazole-4-carboxamido)-2-methyl-5-phenylpentanoate **(52):** UPLC-MS (system 2): *t*ᵣ = 1.11 min, *m*/*z* (ES+) calculated 516.29 (M+H)⁺, found 516.37.

(2S,4R)-allyl 2-methyl-4-(2-((1R,3R)-4-methyl-3-(methylamino)-1-propoxypentyl)thiazole-4-carboxamido)-5-phenylpentanoate **(53):** UPLC-MS (system 2): *t*ᵣ = 1.16 min, *m*/*z* (ES+) calculated 530.31 (M+H)⁺, found 530.40.

Analogous dipeptides are prepared by replacing the suitably protected tubuphenylalanine of Compound **40** with other amino acid or amine-containing acid inputs, suitably protected and as indicated for Scheme 7, including 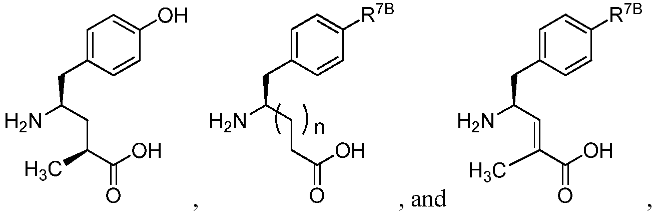 wherein R^{7B} is an O-linked substituent, preferably-OH, the subscript n is 0, 1 or 2, preferably 0 or 1; and further including NH₂-CH₂-CO₂H, NH₂-CH₂CH₂CO₂H and NH₂-CH₂CH₂CH₂CO₂H.

### Example 28: General procedure for the amide coupling of O-alkylated tubuvaline-tubuphenylalanine alkyl ester dipeptides with Fmocprotected L-isoleucine.

FMOC-L-Isoleucine (1.3-2 equivalents) is dissolved in anhydrous dimethylformamide (50-200 mM) and pre-activated with HATU (1.5-2 equivalents) and DIPEA (2 equivalents); the mixture is stirred for 10 minutes at room temperature under nitrogen. The activated acid is then added to the NH₂-Tuv(O-ether)-Tup-O-allyl dipeptides prepared according to Example 27; the reaction is stirred at room temperature under nitrogen and monitored by UPLC/MS. Once the reaction stops progressing or reaches completion, glacial acetic acid (13 equivalents) is added and the resulting FMOC-Ile-Tuv(O-ether)-TupO-allyl tripeptide products are purified by prep HPLC. Compounds **54, 55** and **56** (Scheme 7) were prepared from Compounds **51, 52** and **53,** respectively, to exemplify the general procedure.

(2S,4R)-allyl 4-(2-((5S,8R,10R)-5-((S)-sec-butyl)-1-(9H-fluoren-9-yl)-8-isopropyl-7-methyl-3,6-dioxo-2,11-dioxa-4,7-diazadodecan-10-yl)thiazole-4-carboxamido)-2-methyl-5-phenylpentanoate **(54):** UPLC-MS (system 1): *t*ᵣ = 2.07 min, *m*/*z* (ES+) calculated 837.43 (M+H)⁺, found 837.20

(2S,4R)-allyl 4-(2-((5S,8R,10R)-5-((S)-sec-butyl)-1-(9H-fluoren-9-yl)-8-isopropyl-7-methyl-3,6-dioxo-2,11-dioxa-4,7-diazatridecan-10-yl)thiazole-4-carboxamido)-2-methyl-5-phenylpentanoate **(55):** UPLC-MS (system 2): *t*ᵣ = 1.95 min, *m*/*z* (ES+) calculated 851.44 (M+H)⁺, found 851.54.

(2S,4R)-allyl 4-(2-((5S,8R,10R)-5-((S)-sec-butyl)-1-(9H-fluoren-9-yl)-8-isopropyl-7-methyl-3,6-dioxo-2,11-dioxa-4,7-diazatetradecan-10-yl)thiazole-4-carboxamido)-2-methyl-5-phenylpentanoate **(56):** UPLC-MS (system 2): *t*ᵣ = 2.25 min, *m*/*z* (ES+) calculated 865.46 (M+H)⁺, found 865.65.

Analogous tri-peptides are prepared according to the general procedure of Examples 26-28 by replacing the suitably protected tubuphenylalanine of Compound **40** in Example 26 with other amino acid or amine-containing acid inputs, suitably protected and as indicated for Scheme 7. Alternatively or in addition to replacing the suitably protected tubuphenylalanine other tripeptides are obtained by replacing FMOC-L-Isoleucine with other suitably protected amino acids having a hydrophobic side chain such as FMOC-L-leucine

### Example 29: General procedure for amide coupling of isoleucine-tubuvaline(O-ether)-tubuphenylalanine allyl ester tripeptides with tertiary amine-containing acids for introduction of the tubulysin N-terminal component.

Deprotection: The FMOC-tripeptides (FMOC-Ile-Tuv(O-ether)-TupO-allyl) prepared according to Example 28 are deprotected by removal of the FMOC protecting group using 20% piperidine in dimethylformamide (20 mM), with stirring under nitrogen at room temperature. Once complete deprotection is achieved, as monitored by UPLC/MS, the reaction mixture is concentrated by rotary evaporation. The crude product is then purified by preparative HPLC to provide the free amine tripeptides (NH₂-Ile-Tuv(O-ether)-TupO-allyl). Compounds **57, 58** and **59** (Scheme 7) were prepared from Compounds **54, 55** and **56,** respectively, to exemplify the general procedure.

(2S,4R)-allyl 4-(2-((1R,3R)-3-((2S,3S)-2-amino-N,3-dimethylpentanamido)-1-methoxy-4-methylpentyl)thiazole-4-carboxamido)-2-methyl-5-phenylpentanoate **(57):** . UPLC-MS (system 1): *t*ᵣ = 1.30 min, *m*/*z* (ES+) calculated 637.34 (M+Na)⁺, found 637.57.

(2S,4R)-allyl 4-(2-((1R,3R)-3-((2S,3S)-2-amino-N,3-dimethylpentanamido)-1-ethoxy-4-methylpentyl)thiazole-4-carboxamido)-2-methyl-5-phenylpentanoate **(58):** UPLC-MS (system 2): *t*ᵣ = 1.18 min, *m*/*z* (ES+) calculated 629.38 (M+H)⁺, found 629.45.

(2S,4R)-allyl 4-(2-((1R,3R)-3-((2S,3S)-2-amino-N,3-dimethylpentanamido)-4-methyl-1-propoxypentyl)thiazole-4-carboxamido)-2-methyl-5-phenylpentanoate **(59):** UPLC-MS (system 2): *t*ᵣ = 1.29 min, *m*/*z* (ES+) calculated 643.39 (M+H)⁺, found 643.55.

Peptide Coupling: (R)-N-methyl-pipecolic acid (D-Mep) **60** (1.5-2 equivalents) is dissolved in anhydrous dimethylformamide (25-50 mM) and pre-activated with HATU (2 equivalents) and DIPEA (4 equivalents); the reaction mixture is stirred for 10 minutes at room temperature under nitrogen. The activated acid is then added to the NH₂-Ile-Tuv(O-ether)-TupO-allyl tripeptides obtained from FMOC deprotection; the reaction was stirred at room temperature under nitrogen and monitored by UPLC/MS. Upon reaction completion, glacial acetic acid (14 equivalents) is then added and the tetra-peptide products (**D**-Mep-Ile-Tuv(O-ether)-TupO-allyl) are purified by preparative HPLC. Compounds **61, 62** and **63** (Scheme 7) were prepared from Compounds **57, 58** and **59,** respectively, to exemplify the general procedure.

(2S,4R)-allyl 4-(2-((1R,3R)-3-((2S,3S)-N,3-dimethyl-2-((R)-1-methylpiperidine-2-carboxamido)pentanamido)-1-methoxy-4-methylpentyl)thiazole-4-carboxamido)-2-methyl-5-phenylpentanoate **(61):** UPLC-MS (system 1): *t*ᵣ = 1.29 min, *m*/*z* (ES+) calculated 762.42 (M+Na)⁺, found 762.32.

(2S,4R)-allyl 4-(2-((1R,3R)-3-((2S,3S)-N,3-dimethyl-2-((R)-1-methylpiperidine-2-carboxamido)pentanamido)-1-ethoxy-4-methylpentyl)thiazole-4-carboxamido)-2-methyl-5-phenylpentanoate **(62):** UPLC-MS (system 2): *t*ᵣ = 1.25 min, *m*/*z* (ES+) calculated 754.46 (M+H)⁺, found 754.55.

(2S,4R)-allyl 4-(2-((1R,3R)-3-((2S,3S)-N,3-dimethyl-2-((R)-1-methylpiperidine-2-carboxamido)pentanamido)-4-methyl-1-propoxypentyl)thiazole-4-carboxamido)-2-methyl-5-phenylpentanoate **(63):** UPLC-MS (system 2): *t*ᵣ = 1.36 min, *m*/*z* (ES+) calculated 768.48 (M+H)⁺, found 768.55.

To obtain the corresponding tubulysin ethers as free drug (D-Mep-Ile-Tuv(O-ether)-TupOH), the allyl ester-protected tubulysin ether tetrapeptides are deprotected to provide tubulysin ethers having a free tubuphenylalanine acid moiety by dissolution in anhydrous dichloromethane (20 mM) and treatment with palladium tetrakis(triphenylphosphine) (0.1 equiv.), triphenylphosphine (0.2 equivalents), and anhydrous pyrrolidine (8 equivalents), and the reaction is stirred at an ambient temperature under nitrogen. Once UPLC/MS reveals conversion to the product free acid, the reaction is quenched with glacial acetic acid (22 equivalents), diluted with acetonitrile and dimethylformamide, and then concentrated by rotary evaporation. The crude tubulysin ether is then purified by preparative HPLC. Compounds **64, 65** and **66** (Scheme 7) were prepared from Compounds **61, 62** and **63,** respectively, to exemplify the general procedure.

(2S,4R)-4-(2-((1R,3R)-3-((2S,3S)-N,3-dimethyl-2-((R)-1-methylpiperidine-2-carboxamido)pentanamido)-1-methoxy-4-methylpentyl)thiazole-4-carboxamido)-2-methyl-5-phenylpentanoic acid **(64):** UPLC-MS (system 2): *t*ᵣ = 1.05 min, *m*/*z* (ES+) calculated 700.41 (M+H)⁺, found 700.50.

(2S,4R)-4-(2-((1R,3R)-3-((2S,3S)-N,3-dimethyl-2-((R)-1-methylpiperidine-2-carboxamido)pentanamido)-1-ethoxy-4-methylpentyl)thiazole-4-carboxamido)-2-methyl-5-phenylpentanoic acid **(65):** UPLC-MS (system 2): *t*ᵣ = 1.09 min, *m*/*z* (ES+) calculated 714.43 (M+H)⁺, found 714.51

(2S,4R)-4-(2-((1R,3R)-3-((2S,3S)-N,3-dimethyl-2-((R)-1-methylpiperidine-2-carboxamido)pentanamido)-4-methyl-1-propoxypentyl)thiazole-4-carboxamido)-2-methyl-5-phenylpentanoic acid **(66):** UPLC-MS (system 2): *t*ᵣ = 1.19 min, *m*/*z* (ES+) calculated 728.44 (M+H)⁺, found 728.54.

Additional tubulysin ethers, including those having variation(s) in amino acid or amine-containing acid inputs replacing the tubuphenylalanine (Compound **40)** input of Example 27 as shown therein, and/or the FMOC- amino acid (FMOC-Ile) of Example 28 are prepared by replacing D-Mep with other N-alkylated pipecolic acid inputs or with an N-alkylated azetidinyl-2-carboxylic acid, N-alkylated-D-proline or N,N-di-alkyl amino acid such as N,N-dimethylglycine.

### Example 30: Preparation of the L_{SS} precursor (S) perfluorophenyl 3-((tert-butoxycarbonyl)amino)-2-(2,5-dioxo-2,5-dihydro-1H-pyrrol-1-yl)propanoate (68).

A flask charged with mDPR(Boc)-OH **(67)** (Nature Biotech, 2014, 32, 1059-1062) **67** (500 mg, 1.76 mmol), to which pentafluorophenol (324 mg, 1.76 mmol) was added a solution in DMF (8.8 mL) followed by 1-Ethyl-3-(3-dimethylaminopropyl)carbodiimide (371 mg, 1.93 mmol) as a solid. The reaction was stirred for 1 hour at room temperature then quenched with 50 mL saturated NH₄Cl in H₂O and 50 mL H₂O. The aqueous layer was extracted with DCM twice, the organics were then washed with brine, dried over NaSO₄, and condensed under reduced pressure to provide the titled compound (589 mg, 74%). Analytical UPLC-MS (system 2): *t*ᵣ = 1.51 min, *m*/*z* (ES+) calculated 473.07 (M+Na)⁺, found 473.14.

Compound **68,** abbreviated as mDPR(BOC)-OPFF is an exemplary L_{SS} precursor (i.e., a type of L_{b}'-containing moiety) of formula M¹-A'(BU)-COOH in activated form for the preparation of Drug-Linker compounds, including those of general formula M¹-A₁(BU)-A_{O}-W-Y-D⁺ and M¹-A(BU)-Y(W)-D⁺ wherein M¹ is a maleimide moiety, A' is a Stretcher sub(unit) precursor, A₁ and A_{O} are subunits of Stretcher unit A, BU is a Basic unit such as an aminoalkyl moiety, Y is a self-immolative Spacer Unit and - Y(W)- is a Glucuronide moiety and is comprised for example of a glucuronic acid residue as the carbohydrate moiety and a PAB residue as the self immolative moiety; and D⁺ is a quaternized tubulysin.

### Example 31: General procedure for preparing Drug-Linker compound intermediates incorporating a glucuronic acid release mechanism from tubulysin (O-ether)-allyl esters.

Exemplary Drug-Linker compound intermediates of formula A'-Y_{y}(W'_{w'})-D⁺ wherein the subscripts w' and y are each 1, A' is Stretcher unit precursor, D⁺ is a quaternized tubulysin (O-ether) Drug Unit and -Y(W')- is a Glucuronide unit are prepared from tubulysin (O-ether)-allyl esters of Example 29 in the following manner. The products so obtained are represented by the general formula of FMOC-GlucQ-Tub(O-ether)-O-allyl.

Linker unit elaboration: A pressure vessel is charged with D-Mep-Ile-Tuv(O-ether)-Tup-O-allyl (1 equivalent) and the brominated Stretcher-Glucuronide unit intermediate **2** (1.5 equivalents) of Example 1 in anhydrous 2-butanone (50 mM). The reaction vessel is flushed with N₂ and sealed. The reaction is then stirred and heated to 60 °C for 18 hours. The resulting mixture is cooled, condensed to residue under reduced pressure, then carried forward crude or taken up in minimal DMSO to be purified by preparative HPLC to provide the title compounds . Compounds **69, 70** and **71** (Scheme 8) were prepared from Compounds **61, 62** and **63,** respectively, and Compound **2** to exemplify the general procedure.

(2R)-1-(3-(3-((((9H-fluoren-9-yl)methoxy)carbonyl)amino)propanamido)-4-(((2S,3R,4S,5S,6S)-3,4,5-triacetoxy-6-(methoxycarbonyl)tetrahydro-2H-pyran-2-yl)oxy)benzyl)-2-(((2S,3S)-1-(((1R,3R)-1-(4-(((2R,4S)-5-(allyloxy)-4-methyl-5-oxo-1-phenylpentan-2-yl)carbamoyl)thiazol-2-yl)-1-methoxy-4-methylpentan-3-yl)(methyl)amino)-3-methyl-1-oxopentan-2-yl)carbamoyl)-1-methylpiperidin-1-ium **(69):** Analytical UPLC-MS (system 1): *t*ᵣ = 1.61 min, *m*/*z* (ES+) calculated 1470.68 (M)⁺, found 1471.68.

(2R)-1-(3-(3-((((9H-fluoren-9-yl)methoxy)carbonyl)amino)propanamido)-4-(((2S,3R,4S,5S,6S)-3,4,5-triacetoxy-6-(methoxycarbonyl)tetrahydro-2H-pyran-2-yl)oxy)benzyl)-2-(((2S,3S)-1-(((1R,3R)-1-(4-(((2R,4S)-5-(allyloxy)-4-methyl-5-oxo-1-phenylpentan-2-yl)carbamoyl)thiazol-2-yl)-1-ethoxy-4-methylpentan-3-yl)(methyl)amino)-3-methyl-1-oxopentan-2-yl)carbamoyl)-1-methylpiperidin-1-ium **(70):** Analytical UPLC-MS (system 2): *t*ᵣ = 1.49 min, *m*/*z* (ES+) calculated 1484.69 (M)⁺, found 1484.84.

(2R)-1-(3-(3-((((9H-fluoren-9-yl)methoxy)carbonyl)amino)propanamido)-4-(((2S,3R,4S,5S,6S)-3,4,5-triacetoxy-6-(methoxycarbonyl)tetrahydro-2H-pyran-2-yl)oxy)benzyl)-2-(((2S,3S)-1-(((1R,3R)-1-(4-(((2R,4S)-5-(allyloxy)-4-methyl-5-oxo-1-phenylpentan-2-yl)carbamoyl)thiazol-2-yl)-4-methyl-1-propoxypentan-3-yl)(methyl)amino)-3-methyl-1-oxopentan-2-yl)carbamoyl)-1-methylpiperidin-1-ium **(71):** Analytical UPLC-MS (system 2): *tᵣ* = 1.47 min, *m*/*z* (ES+) calculated 1498.71 (M)⁺, found 1498.85.

Deprotection: The FMOC-GlucQ-Tub(O-ether)-O-allyl products are globally deprotected in THF and MeOH cooled to 0°C. LiOH•H₂O (6.0 equivalents) to which H₂O is added dropwise (1:1:1 THF:MeOH:H₂O, 50 mM end concentration) whereupon the reaction is allowed to warm to room temperature and stir overnight. THF and MeOH are removed under reduced pressure, and the resulting precipitate was resolubilized using minimal DMSO and the mixture was purified by preparative HPLC. The globally deprotected products are abbreviated as H-GlucQ-Tub(O-ether)-OH. Compounds **72, 73** and **74** (Scheme 8) were prepared from Compounds **69, 70** and **71,** respectively, to exemplify the general procedure.

(2R)-1-(3-(3-aminopropanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-1-methoxy-4-methylpentan-3-yl)(methyl)amino)-3-methyl-1-oxopentan-2-yl)carbamoyl)-1-methylpiperidin-1-ium **(72):** Fmoc-GlucQ-Tub(OMe)-OAllyl 69 (17 mg, 12 µmol) was deprotected as above to provide 72 (4.3 mg, 34%). Analytical UPLC-MS (system 1): *tᵣ* = 1.08 min, m/z (ES+) calculated 1068.53 (M)+, found 1068.66.

(2R)-1-(3-(3-aminopropanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-1-ethoxy-4-methylpentan-3-yl)(methyl)amino)-3-methyl-1-oxopentan-2-yl)carbamoyl)-1-methylpiperidin-1-ium **(73):** Analytical UPLC-MS (system 2): *tᵣ* = 0.95 min, m/z (ES+) calculated 1082.55 (M)+, found 1082.68.

(2R)-1-(3-(3-aminopropanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-4-methyl-1-propoxypentan-3-yl)(methyl)amino)-3-methyl-1-oxopentan-2-yl)carbamoyl)-1-methylpiperidin-1-ium **(74):** Analytical UPLC-MS (system 2): *tᵣ* = 0.98 min, m/z (ES+) calculated 1096.56 (M)+, found 1096.67.

### Example 32: General procedure for preparation of Drug Linker compounds by amide coupling of an L_{SS}-Stretcher subunit precursor with a drug-linker intermediate comprised of a Glucuronide unit and quaternized tubulysin (O-ether) Drug unit.

Exemplary Drug-Linker compounds of formula L_{b}'-Aₐ-Y_{y}(W'_{w'})-D⁺ wherein the subscripts a, y and w' are each 1 and in which L_{b}'-A- has the formula of M¹-A₁(BU)-Ao-, wherein M¹-A₁(BU)- represents a precursor of a self-stabilizing (L_{SS}), which is one type of a L_{b}'-containing moiety in a Ligand Drug Conjugate, wherein A₁ is a subunit of a Stretcher unit A with Ao as the other subunit, D⁺ is a quaternized tubulysin (O-ether) Drug Unit and -Y(W')- is a Glucuronide unit, are prepared from H-GlucQ-Tub(O-ether)-OH compounds in the following manner and are abbreviated as mDPR-GlucQ-Tub(O-ether)-OH in which mDPR- is a M¹-A¹(BU)-A_{O}- moiety.

Linker unit elaboration: A flask is charged with the amine H-GlucQ-Tub(O-ether)-OH of Example 31 to which mDPR(Boc)-OPFP **(68,** 1.2 equivalents) of Example 30 is added as a solution in DMF (10 mM). N,N-Diisopropylethylamine (4.0 equivalents) and the reaction was stirred at room temperature for 3 hours. The reaction was quenched with AcOH (4.0 equivalents) then diluted in DMSO (1 volume) and purified by preparative HPLC. Compounds **75, 76** and 77 (Scheme 8) were prepared from Compounds **72, 73** and **74,** respectively, to exemplify the general procedure.

(2R)-1-(3-(3-((S)-3-((tert-butoxycarbonyl)amino)-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-1-methoxy-4-methylpentan-3-yl)(methyl)amino)-3-methyl-1-oxopentan-2-yl)carbamoyl)-1-methylpiperidin-1-ium (75): Analytical UPLC-MS (system 1): t*ᵣ* = 1.22 min, m/z (ES+) calculated 1334.62 (M)+, found 1334.68

(2R)-1-(3-(3-((S)-3-((tert-butoxycarbonyl)amino)-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-1-ethoxy-4-methylpentan-3-yl)(methyl)amino)-3-methyl-1-oxopentan-2-yl)carbamoyl)-1-methylpiperidin-1-ium (76): Analytical UPLC-MS (system 2): *t*ᵣ = 1.19 min, *m*/*z* (ES+) calculated 1348.64 (M)⁺, found 1348.79.

(2R)-1-(3-(3-((S)-3-((tert-butoxycarbonyl)amino)-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-4-methyl-1-propoxypentan-3-yl)(methyl)amino)-3-methyl-1-oxopentan-2-yl)carbamoyl)-1-methylpiperidin-1-ium (77): Analytical UPLC-MS (system 2): *t*ᵣ = 1.24 min, *m*/*z* (ES+) calculated 1362.65 (M)⁺, found 1362.78.

Deprotection: The products from amide bond formation are deprotected to remove the BOC protecting group in the following manner. A flask is charged with mDPR(BOC)-GlucQ-Tub(O-ether)-OH and cooled to 0°C. A 10% solution of TFA in DCM (50 mM) is added and the reaction is allowed to warm to room temperature while stirring for 1 hour. The reaction is then diluted with DMSO (1 volume), DCM removed via reduced pressure, then purified by preparative HPLC. Compounds **78, 79** and **80** (Scheme 8) were prepared from Compounds **75, 76** and **77,** respectively, to exemplify the general procedure.

(2R)-1-(3-(3-((S)-3-amino-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-1-methoxy-4-methylpentan-3-yl)(methyl)amino)-3-methyl-1-oxopentan-2-yl)carbamoyl)-1-methylpiperidin-1-ium **(78):** Analytical UPLC-MS (system 2): *tᵣ* = 1.09 min, *m*/*z* (ES+) calculated 1234.57 (M)⁺, found 1234.65.

(2R)-1-(3-(3-((S)-3-amino-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-1-ethoxy-4-methylpentan-3-yl)(methyl)amino)-3-methyl-1-oxopentan-2-yl)carbamoyl)-1-methylpiperidin-1-ium **(79):** Analytical UPLC-MS (system 2): *tᵣ =* 0.95 min, m/z (ES+) calculated 1248.59 (M)+, found 1248.72.

(2R)-1-(3-(3-((S)-3-amino-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-2-(((2S,3S)-1-(((1R,3R)-1-(4-(((2R,4S)-4-carboxy-1-phenylpentan-2-yl)carbamoyl)thiazol-2-yl)-4-methyl-1-propoxypentan-3-yl)(methyl)amino)-3-methyl-1-oxopentan-2-yl)carbamoyl)-1-methylpiperidin-1-ium **(80):** Analytical UPLC-MS (system 2): *t*ᵣ = 1.03 min, *m*/*z* (ES+) calculated 1262.60 (M)⁺, found 1262.73.

### Example 33: Preparation of isoleucine-tubuvaline(OAc)-tubuphenylalanine allyl ester

Saponification: A flask was charged with BOC-Tuv(OAc)-Tup-OEt **(90,** 50 mg, 81 µmol) in THF (1.35 mL) and MeOH (1.35 mL) and cooled to 0 °C. LiOH · H₂O (27 mg, 647 µmol) was solubilized in H₂O (1.35 mL) then added dropwise. The reaction was allowed to warm to room temperature and stirred for 3 hours. The reaction was then quenched with acetic acid (37 µL, 647 µmol) and condensed under reduced pressure. The residue was taken up in DMSO and purified by preparative HPLC to provide (2S,4R)-4-(2-((1R,3R)-3-((tert-butoxycarbonyl)(methyl)amino)-1-hydroxy-4-methylpentyl)thiazole-4-carboxamido)-2-methyl-5-phenylpentanoic acid **(91)** (44 mg, quant.) (Scheme 9). Analytical UPLC-MS (system 2): *t*ᵣ = 1.53 min, *m*/*z* (ES+) calculated 548.28 (M+H)⁺, found 548.24.

Acetylation and allyl ester deprotection: The BOC-Tuv(OH)-Tup-OH **(91,** 44 mg, 81 µmol) product was solubilized in anhydrous pyridine (1.6 mL) and stirred at room temperature under N₂. Acetic anhydride (15.4 µL, 162 µmol) was added dropwise. 1.0 additional equivalent of acetic anhydride was added after one hour of stirring and the reaction was complete by LCMS after 2 hours. The reaction was concentrated to dryness under reduced pressure then resolubilized in anhydrous allyl alcohol (1.6 mL). Di-allyl pyrocarbonate (54 µL, 325 µmol) was added followed by solid DMAP (3.0 mg, 24 µmol). The reaction was allowed to stir at room temperature and monitored by LCMS, additional di-allyl pyrocarbonate added as needed to push reaction to completion (4.0 additional equivalents). The reaction was then taken up in DMSO, condensed under reduced pressure, and purified by preparative HPLC to provide (2S,4R)-allyl 4-(2-((1R,3R)-1-acetoxy-3-((tert-butoxycarbonyl)(methyl)amino)-4-methylpentyl)thiazole-4-carboxamido)-2-methyl-5-phenylpentanoate **(92)** (Scheme 9) (33 mg, 65%). Analytical UPLC-MS (system 2): *t*ᵣ = 1.75 min, *m*/*z* (ES+) calculated 630.32 (M+H)⁺, found 630.42.

Di-peptide deprotection: A flask charged with BOC-Tuv(OAc)-Tup-O-allyl **(92, 33** mg, 21 µmol) was cooled to 0 °C under N₂. A solution of 10% TFA in CH₂Cl₂ (0.52 mL) was added dropwise and stirred for 4 hours. The reaction was concentrated under reduced pressure, resolubilized in DCM, and condensed 3 times to remove TFA to provide crude (2S,4R)-allyl 4-(2-((1R,3R)-1-acetoxy-4-methyl-3-(methylamino)pentyl)thiazole-4-carboxamido)-2-methyl-5-phenylpentanoate **(93).** Analytical UPLC-MS (system 2): *t*ᵣ = 1.03 min, *m*/*z* (ES+) calculated 530.27 (M+H)⁺, found 530.36. Compound **93** (Scheme 9) was taken forward without further purification.

Peptide Coupling: To a flask charged with H-Tuv(OAc)-Tup-O-Allyl **(93,** 28 mg, 53 µmol) was added BOC-L-Ile-OH (15 mg, 63 µmol) and HATU (40 mg, 106 µmol) as solids followed by DMF (1.0 mL). *N,N*-Diisopropylethylamine (37 µL, 211 µmol) was added and the reaction was stirred at room temperature for 48 hours. The reaction was then taken up in DMSO, condensed under reduced pressure, and purified by preparative HPLC to provide (2S,4R)-allyl 4-(2-((6S,9R,11R)-6-((S)-sec-butyl)-9-isopropyl-2,2,8-trimethyl-4,7,13-trioxo-3,12-dioxa-5,8-diazatetradecan-11-yl)thiazole-4-carboxamido)-2-methyl-5-phenylpentanoate **(94)** (Scheme 9) (19 mg, 49%). Analytical UPLC-MS (system 2): *t*ᵣ = 1.72 min, *m*/*z* (ES+) calculated 743.41 (M+H)⁺, found 743.51.

Tri-peptide deprotection: A flask charged with BOC-Ile-Tuv(OAc)-Tup-Oallyl **(94,** 19 mg, 26 µmol) was cooled to 0 °C under N₂. A solution of 10% TFA in CH₂Cl₂ (0.52 mL) was added dropwise and stirred for 4 hours. The reaction was concentrated under reduced pressure, resolubilized in DCM, and condensed 3 times to remove TFA to provide crude (2S,4R)-allyl 4-(2-((1R,3R)-1-acetoxy-3-((2S,3S)-2-amino-N,3-dimethylpentanamido)-4-methylpentyl)thiazole-4-carboxamido)-2-methyl-5-phenylpentanoate **(95).** Analytical UPLC-MS (system 2): *t*ᵣ = 1.18 min, *m*/*z* (ES+) calculated 643.36 (M+H)⁺, found 643.42. Compound **95** (Scheme 9), abbreviated as Ile-Tuv(OAc)-Tup-O-allyl (isoleucine-tubuvaline(OAc)-tubuphenylalanine allyl ester), was carried forward without further purification.

### Example 34. Preparation of a quaternized nitrogen heterocycloalkyl corresponding to the N-terminal moiety of free tubulysin drug.

N-terminal precursor (D-Mep): H-Pip-OtBu (**96**, 500 mg, 2.70 mmol) was taken up in MeOH (4.50 mL), AcOH (4.50 mL) and 37% CHzO in H₂O (4.50 mL) and stirred for 20 minutes. NaBH₃CN (509 mg, 8.10 mmol) added slowly as a solid to vigorous bubbling, stir for 30 minutes. The reaction was then poured into 200 mL saturated NaHCO₃ solution and extracted 3x with 200 mL DCM. The organic layers were washed with brine, dried over Na₂SO₄, and condensed under reduced pressure to provide (R)-tert-butyl 1-methylpiperidine-2-carboxylate **(97)** (516 mg, 96%). Analytical UPLC-MS (system 2): tr = 0.53 min, m/z (ES+) calculated 200.17 (M+H)+, found 200.21. Compound **97** (Scheme 10), referred to as BOC-D-Mep-OtBu, was carried forward without further purification for condensation with the brominated Stretcher-Glucuronide unit intermediate **2** of Example 1 in the following manner.

Quaternization: A pressure vessel was charged with brominated Stretcher-Glucuronide unit intermediate **(2,** 104 mg, 128 µmol) and **D**-Mep-OtBu **(97, 34** mg, 171 µmol) in anhydrous 2-butanone (1.71 mL). The reaction vessel was flushed with N₂ and sealed. The reaction was then stirred and heated to 60 °C for 12 hours. The resulting mixture was cooled, condensed under reduced pressure, taken up in minimal DMSO and purified by preparative HPLC to provide (2R)-1-(3-(3-((((9H-fluoren-9-yl)methoxy)carbonyl)amino)propanamido)-4-(((2S,3R,4S,5S,6S)-3,4,5-triacetoxy-6-(methoxycarbonyl)tetrahydro-2H-pyran-2-yl)oxy)benzyl)-2-(tert-butoxycarbonyl)-1-methylpiperidin-1-ium **(98)** (97 mg, 82%). Analytical UPLC-MS (system 2): tᵣ = 1.32 min, m/z (ES+) calculated 930.40 (M)⁺, found 930.49. The protecting groups of carbohydrate moiety were then removed followed by reprotected as the allyl ester to provide Compound 99 (Scheme 10) in the following manner.

Glycoside deprotection: To a flame dried flask charged with Compound **98** (97 mg, 104 µmol) having acetate and methyl ester protecting groups in anhydrous allyl alcohol (2.09 mL) under N₂ was added Ti(OC₂H₅)₄ (87 µL, 417 µmol), and the resulting reaction mixture was heated to 80 °C with stirring for 2 hours. The reaction was then cooled to room temperature and poured into 50 mL 1M HCl. After resting for 45 minutes, the HCl was extracted 3x with 50 mL DCM. Resulting organics were washed with brine, dried over Na₂SO₄, condensed, and purified by preparative HPLC to provide (2R)-1-(3-(3-((((9H-fluoren-9-yl)methoxy)carbonyl)amino)-propanamido)-4-(((2S,3R,4S,5S,6S)-6-((allyloxy)carbonyl)-3,4,5-trihydroxytetrahydro-2H-pyran-2-yl)oxy)benzyl)-2-(tert-butoxycarbonyl)-1-methylpiperidin-1-ium **(99)** (42 mg, 48%). Analytical UPLC-MS (system 2): *t*ᵣ = 1.18 min, *m*/*z* (ES+) calculated 830.39 (M)⁺, found 830.49.

Example 35. *Preparation of a Drug-Linker compound intermediate by amide coupling of an tubulysin tripeptide intermediate having a tubuvaline-(O-Acyl) moiety to a quaternized nitrogen heterocycloalkyl corresponding to that of free tubulysin drug.* Compound **99** (Scheme 10), abbreviated as FMOC-Gluc(O-Allyl)Q-D-Mep-OtBu, of Example 34 was deprotected by removal of the t-butyl ester protecting group to provide Compound **100,** which was then coupled to the isoleucine-tubuvaline(OAc)-tubuphenylalanine allyl ester (Compound 95) of Example 33 by peptide bond formation through the following methods.

N-terminal (**D**-Mep) deprotection: A flask containing FMOC-Gluc(O-Allyl)Q-D-Mep-OtBu **(99,** 42 mg, 50 µmol) was cooled to 0 °C under N₂. A solution of 30% TFA in CH₂Cl₂ (2.5 mL) was added dropwise and stirred for 18 hours. The reaction was concentrated under reduced pressure, taken up in minimal DMSO and purified by preparative HPLC to provide 25 mg (64%) (2R)-1-(3-(3-((((9H-fluoren-9-yl)methoxy)carbonyl)amino)propanamido)-4-(((2S,3R,4S,5S,6S)-6-((allyloxy)carbonyl)-3,4,5-trihydroxytetrahydro-2H-pyran-2-yl)oxy)benzyl)-2-carboxy-1-methylpiperidin-1-ium **(100).** Analytical UPLC-MS (system 2): *t*ᵣ = 1.05 min, *m*/*z* (ES+) calculated 774.32 (M)⁺, found 774.42.

Peptide coupling: Compound **100** (Scheme 10), abbreviated as FMOC-Gluc(O-Allyl)Q-D-Mep-OH, was condensed with tripeptide **95** by adding Compound **100** (28 mg, 36 µmol) and HATU (27 mg, 72 µmol) as solids to a flask charged with H-Ile-Tuv(OAc)-Tup-O-Allyl **(95,** 23 mg, 36 µmol) followed by DMF (0.714 mL). N,N-Diisopropylethylamine (25 µL, 143 µmol) was then added and the reaction was stirred at room temperature for 1 hour. The reaction was then taken up in DMSO and purified by preparative LC to provide 23 mg (46%) (2R)-1-(3-(3-((((9H-fluoren-9-yl)methoxy)carbonyl)amino)propanamido)-4-(((2S,3R,4S,5S,6S)-6-((allyloxy)carbonyl)-3,4,5-trihydroxytetrahydro-2H-pyran-2-yl)oxy)benzyl)-2-(((2S,3S)-1-(((1R,3R)-1-acetoxy-1-(4-(((2R,4S)-5-(allyloxy)-4-methyl-5-oxo-1-phenylpentan-2-yl)carbamoyl)thiazol-2-yl)-4-methylpentan-3-yl)(methyl)amino)-3-methyl-1-oxopentan-2-yl)carbamoyl)-1-methylpiperidin-1-ium **(101).** Analytical UPLC-MS (system 2): *t*ᵣ = 1.39 min, *m*/*z* (ES+) calculated 1398.66 (M)⁺, found 1398.81.

Glycoside and Tup deprotection: The FMOC and allyl protecting groups of FMOC-Gluc(O-Allyl)Q-Mep-Ile-Tuv(OAc)-Tup-O-Allyl product (i.e., Compound **101),** further abbreviated as Fmoc-Gluc(O-Allyl)Q-TubM-OAllyl, while retaining the tubuvaline acetate, was accomplished in the following manner. FMOC-Gluc(O-Allyl)Q-TubM-OAllyl **(101,** 21 mg, 15 µmol) was taken up in DCM (1.5 mL) stirring under N₂. Pd(PPh₃)₄ (3.5 mg, 3.1 µmol) and PPh₃ (1.6 mg, 6.1 µmol) were added as solids followed by pyrrolidine (20.1 µL, 245 µmol). The reaction was stirred to 2 hours at room temperature then taken up in 1 mL DMSO, condensed under reduced pressure, and purified by preparative LC to provide 13 mg (79%) of (2R)-2-(((2S,3S)-1-(((1R,3R)-1-acetoxy-1 -(4-(((2R,4S)-4-carboxy-1 -phenylpentan-2-yl)carbamoyl)thiazol-2-yl)-4-methylpentan-3-yl)(methyl)amino)-3-methyl-1-oxopentan-2-yl)carbamoyl)-1-(3-(3-aminopropanamido)-4-(((2S,3R,4S,5S,6S)-6-carboxy-3,4,5-trihydroxytetrahydro-2H-pyran-2-yl)oxy)benzyl)-1-methylpiperidin-1-ium **(102).** Analytical UPLC-MS (system 2): *t*ᵣ = 0.94 min, *m*/*z* (ES+) calculated 1096.53 (M)⁺, found 1096.65.

Compound **102,** abbreviated as H-GlucQ-TubM-OH, is an exemplary Drug-Linker compound intermediate of formula A'-Y_{y}(W'_{w'})-D⁺ wherein the subscripts y and w' are each 1 and in which A' is a Stretcher (sub)unit precursor, -Y(W)- is a Glucuronide unit, which is comprised of a PAB Spacer unit capable of self-immolation to release tubulysin Drug (D) and a glucuronic acid moiety, and D⁺ is quaternized tubulysin M. To prepare analogous Drug Linker compound intermediates having alternative quaternized tubulysin containing a tubuvaline (O-acyl) component inputs in which the acetate moiety of BOC-Tuv(O-Ac)-Tup(OEt) **(90)** is replaced with a compound of general formula BOC-Tuv(O-Acyl)-Tup(OEt) in Example 33 to prepare H- Ile-Tuv(O-Acyl)-Tup-O-allyl, corresponding to Compound **95.** That intermediate is then carried forward in accordance with Examples 34 and 35 to provide compounds of general formula H-GlucQ-D-Mep-Ile-Tuv(O-Acyl)-Tup-OH, further abbreviated as H-GlucQ-Tub(O-Acyl)-OH, which corresponds to the Drug-Linker compound intermediate of Compound **102.**

Other variations are, or further include, replacing the suitably protected 6-membered tertiary amine-containing heterocycloalkyl, BOC-D-Mep-OtBu (Compound **97),** of Scheme 10 with another suitably protected 5- or 6-membered tertiary amine-containing heterocycle such as BOC-N-ethyl-pipecolic acid t-butyl ester, BOC-N-methyl-proline t-butyl ester or BOC-N-methyl-azetidinyl-2-carboxylic acid t-butyl ester to provide compounds analogous to Compound **99** and Compound **102,** respectively, of Scheme 10 in which the quaternized **D**-Mep moiety of FMOC-Gluc(O-Allyl)Q-D-Mep-OtBu and H-GlucQ-D-Mep-Ile-Tuv(O-Ac)-Tup OH, or more generally of H-GlucQ-D-Mep-Ile-Tuv(O-Acyl)-Tup-OH, is replaced by another quaternized tertiary amine-containing heterocycloalkyl. Additional variations of Drug-Linker compound intermediates in which the heterocycloalkyl is replaced with a tertiary amine containing carboxylic acid are also obtained through Scheme 10 to provide N-terminal ring-opened variants of quaternized tubulysins drug-linker compounds.

### Example 36: Preparation of Drug Linker compounds by amide coupling of an L_{SS}-Stretcher subunit precursor with a drug-linker intermediate comprised of a Glucuronide unit and quaternized tubulysin (O-acyl) Drug unit.

L_{SS} (mDPR) precursor coupling: A flask was charged with H-GlucQ-TubM-OH **(102,** 13.1 mg, 11.9 µmol) from Example 35 in anhydrous DMF (0.595 mL) to which mDPR(BOC)-OSu **(6,** 4.6 mg, 11.9 µmol) was added under N₂. N,N-Diisopropylethylamine (8.3 µL, 47.8 µmol) was added and the reaction was stirred at room temperature for 3 hours. The reaction was then quenched with acetic acid (8.3 µL) and purified by preparative HPLC to provide 5.2 mg (33%) (2R)-2-(((2S,3S)-1-(((1R,3R)-1-acetoxy-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(3-(3-((S)-3-((tert-butoxycarbonyl)amino)-2-(2,5-dioxo-2,5 -dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-1-methylpiperidin-1-ium **(103).** Analytical UPLC-MS (system 2): *t*ᵣ = 1.20 min, *m*/*z* (ES+) calculated 1362.62 (M)⁺, found 1362.75.

L_{SS} precursor deprotection: The BOC protecting group on mDPR(BOC)-GlucQ-TubM-OH **(103)** is removed as follows to prepare Drug-Linker compounds suitable for preparing Ligand-Drug Conjugates by condensing with a thiol containing targeting moiety.

A flask charged with mDPR(Boc)-GlucQ-TubM-OH **(103,** 5.2 mg, 3.8 µmol) was cooled to 0 °C under N₂. A solution of 10% TFA in CH₂Cl₂ (0.84 mL) was added dropwise and stirred for 4 hours. The reaction was then taken up in DMSO, condensed under reduced pressure, and purified by preparative HPLC to provide 4.8 mg (81%) of 2R)-2-(((2S,3S)-1-(((1R,3R)-1-acetoxy-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(3-(3-((S)-3-amino-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-1-methylpiperidin-1-ium **(104).** Analytical UPLC-MS (system 2): *t*ᵣ = 0.95 min, *m*/*z* (ES+) calculated 1262.56 (M)⁺, found 1262.68.

Also prepared by Example 36 are Drug-Linker compounds having variations in the quaternized tubulysin N-terminal residue in which quaternized D-Mep moiety in Tubulysin M is replaced with another quaternized 6-membered tertiary amine-containing heterocycloalkyl. Additionally prepared by Example 36 are other Drug-Linker compounds obtained from carboxylic acid substituted 4- or 5-membered tertiary amine-containing heterocycloalkyls. Other variations replace the quaternized 6-membered heterocycloalkyl of D-Mep with acyclic versions through use in Example 33 of suitably protected tertiary amine- containing acids to provide N-terminal ring-opened version of quaternized tubulysin Drug Linker compounds. Still other variations replace the acetate moiety of the tubuvaline component as described in Example 33-35 for Drug-Linker compound intermediates with another acyloxy group to provide analogous Drug-Linker compounds. Further variations incorporate both changes, i.e., variations in the N-terminal moiety and the tubuvaline acyloxy moiety.

The compounds prepared by Example 36 are exemplary Drug-Linker Compounds of Formula M¹-A₁(BU)-A_{O}-Y(W)-D⁺ or Lb'-A_{O}-Y(W)-D⁺ in which L_{b}' is comprised of a maleimide moiety (M¹), a Basic unit (BU) and a subunit (A₁) of Stretcher unit and Ao is another subunit of the Stretcher unit, wherein L_{b}' is a precursor of L_{SS} in a Ligand Drug Conjugate prepared from the Drug-Linker compound.

### Example 37: General procedure for preparing Drug-Linker compound intermediates incorporating a peptide release mechanism having a quaternized tubulysin(O-acyl) Drug unit.

Exemplary Drug-Linker compound intermediates of formula A'-W_{w}-Y_{y}-D⁺ are prepared as follows wherein the subscripts w and y are each one and in which A' is Stretcher unit precursor, D⁺ is a quaternized tubulysin Drug Unit and -W-Y- is a peptide Cleavable unit (W) covalently attached to a self-immolative Spacer unit (Y) wherein W provides for a protease recognition site, the action of which releases free tubulysin drug from a Ligand Drug Conjugate (LDC), wherein the LDC is comprised of that A'-W_{w}-Y_{y}-D⁺ as the -L_{O}-D⁺ moiety. In general a suitably protected peptide-Spacer unit intermediate of formula BOC-W₂-W₁-PAB-Br, wherein W₁ and W₂ are amino acid subunits of W and PAB is the self-immolative Spacer unit Y intermediate, is first prepared by peptide bond coupling of BOC protected W₂ to H-W₁-PAB-OH followed by bromination of the benzylic carbon to provide the peptide-Spacer unit intermediate BOC-W₂-W₁-PAB-Br. A tubulysin, suitably protected at the C-terminal component, as for example as the allyl ester, is then condensed with the peptide-Spacer unit intermediate for quaternization of its tertiary-amine-containing component to provide after global deprotection a Drug Linker compound intermediate of formula H-W-Y-D⁺ (i.e., H-W₂-W₁-PAB-D⁺). The product of that reaction sequence is exemplified by the following preparation of H-Val-Glu-PAB4-TubM-OH (Scheme 11), wherein the dipeptide residue -Val-Glu- corresponds to -W₂-W₁.

Spacer unit peptide coupling: A flask charged with FMOC-Glu(OtBu)-OH **(105,** 2.0 g, 4.7 mmol), H-PAB-OH **(23,** 579 mg, 4.7 mmol), and Cl₂CH₂ (25 mL) was stirred at room temperature. EEDQ (1.40 g, 5.6 mmol) was added as a solid and the mixture was stirred overnight. Product was eluted from a 4 mm chromatotron plate with EtOAc, product containing fractions were condensed. The resulting residue was taken up in 20% piperidine in DCM, stirred for 15 minutes, then condensed to an oil. The oil was dissolved in DCM and eluted from a 2 mm chromatotron plate using 10%-20% MeOH in DCM gradient to provide 860 mg (60%) of (S)-tert-butyl 4-amino-5-((4-(hydroxymethyl)phenyl)amino)-5-oxopentanoate **(106).** Analytical UPLC-MS (system 2): *t*ᵣ = 0.65 min, *m*/*z* (ES+) calculated 309.18 (M+H)⁺, found 309.24.

Cleavable unit peptide coupling: To a flask charged with H-Glu(OtBu)-PAB-OH (106, 860 mg, 2.78 mmol) in DMF (10 mL) was added BOC-Val-OSu **(20)** (1.13 g, 3.60 mmol) and DIPEA (0.75 mL). After 30 minutes the reaction mixture was poured into 100 mL EtOAc and washed with H₂O 3x, brine 1x, and dried over Na₂SO₄. The solution was dried under reduced pressure, solubilized in 50 mL EtOAc, and precipitated by 10% EtOAc in hexanes (50 mL). The solids were collected and dried to provide 0.97 g (70%) of (S)-tert-butyl 4-((S)-2-((tert-butoxycarbonyl)amino)-3-methylbutanamido)-5-((4-(hydroxymethyl)phenyl)amino)-5-oxopentanoate **(107).** Analytical UPLC-MS (system 2): *t*ᵣ = 1.37 min, *m*/*z* (ES+) calculated 508.30 (M+H)⁺, found 508.38.

Spacer Unit functionalization: A flask charged with BOC-Val-Glu(OtBu)-PAB-OH **(107,** 200 mg, 394 µmol), N-bromosuccinimide (105 mg, 591 µmol), and triphenylphosphine (155 mg, 591 µmol) was flushed with N₂. The reaction was taken up in THF (4 mL) and stirred for 12 hours. The reaction was condensed and purified over silica via a Biotage column (Hexanes/EtOAc, 10%-100%) to provide 210 mg (93%) of (S)-tert-butyl 5-((4-(bromomethyl)phenyl)amino)-4-((S)-2-((tert-butoxycarbonyl)amino)-3-methylbutanamido)-5-oxopentanoate **(108).** Analytical UPLC-MS (system 2): *t*ᵣ = 1.56 min, *m*/*z* (ES+) calculated 570.22 (M+H)⁺, found 570.30.

Quaternization: A flask charged with BOC-Val-Glu(OtBu)-PAB-Br **(108,** 40 mg, 70 µmol) and TubM-OAllyl (Org. Lett., 2007, 9, 1605-1607) **(109,** 45 mg, 59 µmol) was flushed with N₂. Butanone (1.17 mL) was added and the reaction was heated to 60 ^{O}C while stirring. After 18 hours the reaction was condensed to dryness, taken up in minimal DCM, and purified via Biotage (0-20% DCM/MeOH) to provide 62 mg (85%) of (2R)-2-(((2S,3S)-1-(((1R,3R)-1-acetoxy-1-(4-(((2R,4S)-5-(allyloxy)-4-methyl-5-oxo-1-phenylpentan-2-yl)carbamoyl)thiazol-2-yl)-4-methylpentan-3-yl)(methyl)amino)-3-methyl-1-oxopentan-2-yl)carbamoyl)-1-(4-((S)-5-(tert-butoxy)-2-((S)-2-((tert-butoxycarbonyl)amino)-3-methylbutanamido)-5-oxopentanamido)benzyl)-1-methylpiperidin-1-ium **(110).** Analytical UPLC-MS (system 2): *t*ᵣ = 1.47 min, *m*/*z* (ES+) calculated 1257.72 (M)⁺, found 1257.85.

Deprotection: A flask charged with BOC-Val-Glu(OtBu)-PABQ-TubM-OAllyl **(110,** 42 mg, 50 µmol) was cooled to 0 °C under N₂. A solution of 30% TFA in CH₂Cl₂ (0.99 mL) was added dropwise and stirred for 18 hours. The reaction was concentrated under reduced pressure, taken up in DCM and recondensed 3 times. The residue was then taken up in DCM (0.98 mL) to which Pd(PPh₃)₄ (5.7 mg, 4.9 µmol) and PPh₃ (2.6 mg, 9.8 µmol) were added as solids followed by pyrrolidine (32 µL, 392 µmol). After 1 hour the reaction was taken up in minimal DMSO, condensed, and purified by preparative HPLC to provide 47 mg (90%) of (2R)-2-(((2S,3S)-1-(((1R,3R)-1-acetoay-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(4-((S)-2-((S)-2-amino-3-methylbutanamido)-4-carboxybutanamido)benzyl)-1-methylpiperidin-1-ium **(111).** Analytical UPLC-MS (system 2): *t*ᵣ = 0.95 min, *m*/*z* (ES+) calculated 1061.57 (M)⁺, found 1061.69.

Other Drug-Linker compound intermediates analogous to Compound **111** are prepared by replacing the FMOC-Glu(Ot-Bu)-OH **(105)** and BOC-Val-OSu **(20)** inputs for W2 and W1 respectively with other appropriate amino acid inputs so that the prepared Drug-Linker compound intermediates of formula H-W₂-W₁-PAB-D⁺ have the structure of 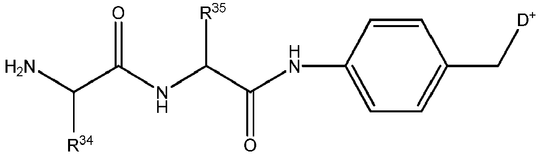 wherein R³⁴ is benzyl, methyl, isopropyl, isobutyl, sec-butyl, -CH(OH)CH₃ or has the structure of 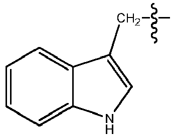 and R³⁵ is methyl, -(CH₂)₄-NH₂, -(CH₂)₃NH(C=O)NH₂, (CH₂)₃NH(C=NH)NH₂, or - (CH₂)₂CO₂H, wherein the wavy line at the dipeptide N-terminal indicates covalent binding to A or to L_{b} or L_{b}' and the wavy line at the dipeptide C-terminal indicates covalent binding to J of an SI moiety.

### Example 38: General procedure for preparing Drug-Linker compounds by introducing a L_{SS} precursor moiety into Drug-Linker compound intermediates incorporating a peptide release mechanism having a quaternized tubulysin(O-acyl) Drug unit.

Drug Linker compounds of formula L_{b}'-Y-W-D⁺ wherein L_{b}'- is M¹-A(BU)- and -Y-W-D⁺ corresponds to Drug Linker compound intermediates of Example 37, are exemplified by Scheme 12. Exemplification is provided as follows for the synthesis of the Drug-Linker compound mDPR-Val-Glu-PABQ-TubM-OH **(113)** whose generalized formula is M¹-A(BU)-W₂-W₁-PABQ-TubM-OH.

L_{SS}(mDPR) coupling: A flask was charged with H-ValGluPAB4-TubM (111, 22.5 mg, 21.2 µmol) in anhydrous DMF (0.420 mL), to which mDPR(Boc)-OSu **(6, 8.9** mg, 23.3 µmol) was added as a solid under N₂. N,N-Diisopropylethylamine (14.8 µL, 84.7 µmol) was added and the reaction was stirred at room temperature for 3 hours. The reaction was then quenched with acetic acid (14.8 µL) and purified by preparative HPLC to provide 11.5 mg (40%) of (2R)-2-(((2S,3S)-1-(((1R,3R)-1-acetoxy-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(4-((7S,10S,13S)-13-(2-carboxyethyl)-7-(2,5-dioxo-2,5-dihydro-1H-pyrrol-1-yl)-10-isopropyl-2,2-dimethyl-4,8,11-trioxo-3-oxa-5,9,12-triazatetradecanamido)benzyl)-1-methylpiperidin-1-ium **(112)** (11.5 mg, 40%). Analytical UPLC-MS (system 2): *t*ᵣ = 1.31 min, *m*/*z* (ES+) calculated 1327.66 (M)⁺, found 1327.94.

Deprotection: A flask charged with mDPR(Boc)-ValGluPAB4-TubM **(112,** 11.5 mg, 8.6 µmol) was cooled to 0 °C under N₂. A solution of 10% TFA in CH₂Cl₂ (0.86 mL) was added dropwise and stirred for 2 hours. The reaction was then taken up in DMSO, condensed under reduced pressure, and purified by preparative HPLC to provide 9.9 mg (93%) of (2R)-2-(((2S,3S)-1-(((1R,3R)-1-acetoxy-1-(4-(((2R,4S)-4-carboxy-1-phenylpentan-2-yl)carbamoyl)thiazol-2-yl)-4-methylpentan-3-yl)(methyl)amino)-3-methyl-1-oxopentan-2-yl)carbamoyl)-1-(4-((S)-2-((S)-2-((S)-3-amino-2-(2,5-dioxo-2,5-dihydro-1H-pyrrol-1-yl)propanamido)-3-methylbutanamido)-4-carboxybutanamido)benzyl)-1-methylpiperidin-1-ium **(113).** Analytical UPLC-MS (system 2): *t*ᵣ = 0.99 min, *m*/*z* (ES+) calculated 1227.61 (M)⁺, found 1227.83.

### Example 39: Preparation of tubulysin intermediate compounds having alternative acyloxy moieties in substitution of acetate in the tubuvaline component.

The preparation of the title compounds proceeds from BOC-protected des-acetyl tubuvaline compound through peptide coupling to tubuphenylalanine as its allyl ester. The acyl moiety is then reintroduced using the appropriate acid anhydride (Method 1), as exemplified for propionyloxy and butyryloxy, or acid chloride (Method 2) as exemplified for isobutyric acid chloride for replacement of the tubuvaline acetate moiety (i.e., isobutyryloxy replaces acetoxy). Alternatively, the tubuvaline hydroxyl is esterified through DCC activation of an aliphatic carboxylic acid (Method 3), as exemplified for dimethylbutyryloxy and isovaleryl replacement of the tubuvaline acetate moiety. The compounds obtained have general formula BOC-Tuv(O-acyl)-Tup-O-allyl as shown in Scheme 13. The BOC protecting group is the removed to allow for peptide coupling to introduce the N-terminal component to provide the Tubulysin M analogs.

Tuv hydrolysis: A flask was charged with tubuvaline acetate (Org. Lett., 2007, 9, 1605-1607) **122** (225 mg, 560 µmol) dissolved in methanol (5 ml) and tetrahydrofuran (5 ml), then cooled under nitrogen to 0 °C in an ice bath. Lithium hydroxide monohydrate (71 mg, 1680 µmol) was dissolved in water (5 ml) and the solution added dropwise to the reaction flask. The reaction was then stirred at room temperature until UPLC/MS revealed complete conversion to product. The material was then diluted with dichloromethane and washed with 0.1 M HCl. The aqueous layer was extracted twice with dichloromethane, then the combined organics were dried over sodium sulfated, filtered, and concentrated to provide 200 mg (quant.) of the free acid compound 2-((1R,3R)-3-((tert-butoxycarbonyl)(methyl)amino)-1-hydroxy-4-methylpentyl)thiazole-4-carboxylic acid (**123**) Analytical UPLC-MS (system 1): *t*ᵣ = 1.33 min, *m*/*z* (ES+) calculated 359.17 (M+H)⁺, found 359.14.

Peptide Coupling: Tubuvaline free acid Compound **123** (200 mg, 560 µmol) was pre-activated by dissolution in anhydrous dimethylformamide (5.4 mL, 100 mM) and addition of HATU (250 mg, 670 µmol) and DIPEA (0.59 ml, 3.36 mmol); the mixture was then stirred under nitrogen at room temperature for 10 minutes. The activated acid was then added to tubuphenylalanine allyl ester **40** (Org. Lett., 2007, 9, 1605-1607) and the reaction was then stirred at an ambient temperature under nitrogen, with progress monitored by UPLC/MS. Upon reaction completion, glacial acetic acid (14 equivalents) was then added and the product was purified by preparative HPLC to provide 272 mg (83%) of the Tuv(OH)-Tup-Oallyl dipeptide (2S,4R)-allyl 4-(2-((1R,3R)-3-((tert-butoxycarbonyl)(methyl)amino)-1-hydroay-4-methylpentyl)thiazole-4-carboxamido)-2-methyl-5-phenylpentanoate (**124**). Analytical UPLC-MS (system 1): *t*ᵣ = 1.84 min, *m*/*z* (ES+) calculated 588.31 (M+H)⁺, found 588.29.

Re-acylation (Method 1): Tuv(OH)-Tup-Oallyl dipeptide **124** (26 mg, 44 µmol) was dissolved in anhydrous pyridine (1.8 ml, 25 mM) and stirred under a nitrogen atmosphere at room temperature. Propionic anhydride **125** (113 µl, 20 equivalents) was added dropwise and the reaction was then monitored by UPLC/MS. Additional propionic anhydride (20 equivalents) was added to achieve conversion to product. The material was the diluted with dichloromethane and washed with 0.1 M HCl. The aqueous layer was extracted twice with dichloromethane, then the combined organics were dried over sodium sulfate, filtered, and concentrated to provide the crude product, which was subsequently purified by preparative HPLC to provide 17 mg (61%) of the BOC-Tuv(propionyloxy)-Tup-O-allyl product (2S,4R)-allyl 4-(2-((1R,3R)-3-((tert-butoxycarbonyl)(methyl)amino)-4-methyl-1-(propionyloxy)pentyl)thiazole-4-carboxamido)-2-methyl-5-phenylpentanoate (**127**). Analytical UPLC-MS (system 1): *t*ᵣ = 1.99 min, *m*/*z* (ES+) calculated 644.34 (M+H)⁺, found 644.26.

Tuv(OH)-Tup-Oallyl dipeptide **124** (27 mg, 46 µmol) was dissolved in anhydrous pyridine (0.9 ml, 50 mM) and stirred under a nitrogen atmosphere at room temperature. Butyric anhydride **126** (225 µl, 30 equivalents) was added dropwise and the reaction was then monitored by UPLC/MS. Additional butyric anhydride (40 equivalents) was added in three portions to achieve conversion to product. The material was the diluted with dichloromethane and washed with 0.1 M HCl. The aqueous layer was extracted twice with dichloromethane, then the combined organics were dried over sodium sulfated, filtered, and concentrated to provide the crude product, which was subsequently purified by preparative HPLC to provide 24 mg (80%) of the BOC-Tuv(butyryloxy)-Tup-O-allyl product (2S,4R)-allyl 4-(2-((1R,3R)-3-((tert-butoxycarbonyl)(methyl)amino)-1-(butyryloxy)-4-methylpentyl)thiazole-4-carboxamido)-2-methyl-5-phenylpentanoate (**128**). Analytical UPLC-MS (system 1): *t*ᵣ = 2.13 min, *m*/*z* (ES+) calculated 658.35 (M+H)⁺, found 658.23.

Re-acylation (Method 2): Tuv(OH)-Tup-Oallyl dipeptide **124** (26 mg, 44 µmol) was dissolved in anhydrous pyridine (1.8 ml, 25 mM) and stirred under a nitrogen atmosphere at room temperature. Isobutyryl chloride **129** (93 µl, 20 equivalents) was added dropwise and the reaction was then monitored by UPLC/MS. Upon conversion to product, the material was then diluted with dichloromethane and washed with 0.1 M HCl. The aqueous layer was extracted twice with dichloromethane, then the combined organics were dried over sodium sulfated, filtered, and concentrated to provide the crude product, which was subsequently purified by preparative HPLC to provide 29 mg (quant.) of the BOC-Tuv(Isobutyryloxy)-Tup-O-allyl product (2S,4R)-allyl 4-(2-((1R,3R)-3-((tert-butoxycarbonyl)(methyl)amino)-1-(isobutyryloxy)-4-methylpentyl)thiazole-4-carboxamido)-2-methyl-5-phenylpentanoate (**130**). Analytical UPLC-MS (system 1): *t*ᵣ = 2.13 min, *m*/*z* (ES+) calculated 658.35 (M+H)⁺, found 658.33.

Re-acylation (Method 3): A flask was charged with isovaleric acid **131** (94 µl, 851 µmol) dissolved in anhydrous dichloromethane (5.6 ml, 15 mM) and the solution was stirred at 0 C under a nitrogen atmosphere. DMAP (10 mg, 85 µmol) was then added, followed by DCC (88 mg, 425 µmol), and the reaction was allowed to warm to room temperature over 2 hours. The resulting activated acid was then added to Tuv(OH)-Tup-Oallyl dipeptide **124** (50 mg, 85 µmol) and the reaction was stirred overnight, at which time UPLC/MS revealed conversion to product. The reaction was then diluted with dichloromethane and washed with 0.1 M HCl. The aqueous layer was extracted twice with dichloromethane, then the combined organics were dried over sodium sulfated, filtered, and concentrated to provide the crude product, which was subsequently purified by preparative HPLC to provide 52 mg (91%) of the BOC-Tuv(isovalyryloxy)-Tup-Oallyl product (2S,4R)-allyl 4-(2-((1R,3R)-3-((tert-butoxycarbonyl)(methyl)amino)-4-methyl-1-((3-methylbutanoyl)oxy)pentyl)thiazole-4-carboxamido)-2-methyl-5-phenylpentanoate (**133**) Analytical UPLC-MS (system 2): *t*ᵣ = 1.91 min, *m*/*z* (ES+) calculated 672.37 (M+H)⁺, found 672.46.

A flask was charged with gem-dimethylbutyric acid **132** (98 µl, 766 µmol) dissolved in anhydrous dichloromethane (5.1 ml, 15 mM) and the solution was stirred at 0 C under a nitrogen atmosphere. DMAP (9 mg, 77 µmol) was then added, followed by DCC (79 mg, 383 µmol), and the reaction was allowed to warm to room temperature over 2 hours. The resulting activated acid was then added to Tuv(OH)-Tup-Oallyl dipeptide **124** (45 mg, 77 µmol) and the reaction was stirred overnight, at which time UPLC/MS revealed conversion to product. The reaction was then diluted with dichloromethane and washed with 0.1 M HCl. The aqueous layer was extracted twice with dichloromethane, then the combined organics were dried over sodium sulfated, filtered, and concentrated to provide the crude product, which was subsequently purified by preparative HPLC to provide 49 mg (93%) of the BOC-Tuv(dimethylbutyryloxy)-Tup-O-allyl product (2S,4R)-allyl4-(2-((1R,3R)-3-((tert-butoxycarbonyl)(methyl)amino)-1-((3,3-dimethylbutanoyl)oxy)-4-methylpentyl)thiazole-4-carboxamido)-2-methyl-5-phenylpentanoate (**134**). Analytical UPLC-MS (system 2): *t*ᵣ = 1.88 min, *m*/*z* (ES+) calculated 686.39 (M+H)⁺, found 686.47.

Deprotection: The BOC-Tuv(O-acyl)-Tup-O-allyl intermediates prepared by Method 1, 2 or 3 were deprotected under acidic conditions with 10% TFA in dichloromethane (25mM) to reveal the secondary amine functional group as follows. BOC-protected intermediate is dissolved in anhydrous dichloromethane (9 volumes) and stirred under nitrogen at 0°C. Trifluoroacetic acid (1 volume) is then added dropwise to the stirred solution. The reaction was warmed slowly to room temperature and monitored by UPLC/MS. Upon completion, the reaction was concentrated by rotary evaporation and pumped down on a vacuum line overnight. The free amines of general formula H-Tuv(O-acyl)-Tup-O-allyl so obtained are carried forward for introduction of the N-terminal component without further purification.

The H-Tuv(O-acyl)-Tup-O-allyl compounds that were prepared (Compounds **135-139**) are shown in Scheme 14 and listed below.
(2S,4R)-allyl 2-methyl-4-(2-((1R,3R)-4-methyl-3-(methylamino)-1-(propionyloxy)pentyl)thiazole-4-carboxamido)-5-phenylpentanoate (**135**): UPLC-MS (system 1): *t*ᵣ = 1.24 min, *m*/*z* (ES+) calculated 544.29 (M+H)⁺, found 544.25
(2S,4R)-allyl 4-(2-((1R,3R)-1-(butyryloxy)-4-methyl-3-(methylamino)pentyl)thiazole-4-carboxamido)-2-methyl-5-phenylpentanoate (**136**): UPLC-MS (system 2): *t*ᵣ = 1.20 min, *m*/*z* (ES+) calculated 558.30 (M+H)⁺, found 558.38
(2S,4R)-allyl 4-(2-((1R,3R)-1-(isobutyryloxy)-4-methyl-3-(methylamino)pentyl)thiazole-4-carboxamido)-2-methyl-5-phenylpentanoate (**137**): UPLC-MS (system 1): *t*ᵣ = 1.30 min, *m*/*z* (ES+) calculated 558.30 (M+H)⁺, found 557.93.
(2S,4R)-allyl 2-methyl-4-(2-((1R,3R)-4-methyl-3-(methylamino)-1-((3-methylbutanoyl)oxy)pentyl)thiazole-4-carboxamido)-5-phenylpentanoate (**138**): . UPLC-MS (system 2): *t*ᵣ = 1.23 min, *m*/*z* (ES+) calculated 572.32 (M+H)⁺, found 572.40.
(2S,4R)-allyl 4-(2-((1R,3R)-1-((3,3-dimethylbutanoyl)oxy)-4-methyl-3-(methylamino)pentyl)thiazole-4-carboxamido)-2-methyl-5-phenylpentanoate (**139**): UPLC-MS (system 2): *t*ᵣ = 1.27 min, *m*/*z* (ES+) calculated 586.33 (M+H)⁺, found 586.42.

### Example 40: General procedure for amide coupling of isoleucine with tubuvaline(O-acyl)-tubuphenylalanine allyl ester di-peptide

Fmoc-L-Isoleucine (4 equivalents) is dissolved in anhydrous dimethylformamide (50 mM) and pre-activated with HATU (4 equivalents) and DIPEA (8 equivalents); the mixture is stirred for 10 minutes at room temperature under nitrogen. The activated acid is then added to the dipeptides of general formula H-Tuv(O-acyl)-Tup-O-allyl; the reaction is stirred at room temperature under nitrogen and monitored by UPLC/MS. Once the reaction stops progressing or reaches completion, glacial acetic acid (13 equivalents) is added and the reaction product is purified by prep HPLC. The FMOC protecting group is then removed by treating the Fmoc-Ile-Tuv(O-Acyl)-Tup-O-allyl tripeptides with 20% piperidine in dimethylformamide (20 mM), with stirring under nitrogen at room temperature. Once complete deprotection had been achieved, as monitored by UPLC/MS, the reaction mixture was concentrated by rotary evaporation. The crude product of general formula H-Ile-Tuv(O-Acyl)-Tup-O-allyl was then purified by preparative HPLC to provide free amine tripeptides.

Compounds **140-144** of general formula FMOC-Ile-Tuv(O-acyl)-Tup-O-allyl shown in Scheme 14 and listed below were prepared in accordance with the above peptide coupling procedure.

(2S,4R)-allyl 4-(2-((5 S,8R, 10R)-5-((S)-sec-butyl)-1-(9H-fluoren-9-yl)-8-isopropyl-7-methyl-3,6,12-trioxo-2,11 -dioxa-4,7-diazatetradecan-10-yl)thiazole-4-carboxamido)-2-methyl-5 - phenylpentanoate (**140**): UPLC-MS (system 1): *t*ᵣ = 2.11 min, *m*/*z* (ES+) calculated 879.44 (M+H)⁺, found 879.60.

(2S,4R)-allyl 4-(2-((5S,8R, 10R)-5-((S)-sec-butyl)-1 -(9H-fluoren-9-yl)-8-isopropyl-7-methyl-3,6,12-trioxo-2,11 -dioxa-4,7-diazapentadecan-10-yl)thiazole-4-carboxamido)-2-methyl-5 - phenylpentanoate (**141**): UPLC-MS (system 2): *t*ᵣ = 1.94 min, *m*/*z* (ES+) calculated 893.45 (M+H)⁺, found 893.56.

(2S,4R)-allyl 4-(2-((5 S,8R, 10R)-5-((S)-sec-butyl)-1-(9H-fluoren-9-yl)-8-isopropyl-7,14-dimethyl-3,6,12-trioxo-2,11-dioxa-4,7-diazapentadecan-10-yl)thiazole-4-carboxamido)-2-methyl-5-phenylpentanoate (**143**): UPLC-MS (system 2): *t*ᵣ = 2.06 min, *m*/*z* (ES+) calculated 907.47 (M+H)⁺, found 907.58.

(2S,4R)-allyl 4-(2-((5 S,8R, 10R)-5-((S)-sec-butyl)-1-(9H-fluoren-9-yl)-8-isopropyl-7,14,14-trimethyl-3,6,12-trioxo-2,11-dioxa-4,7-diazapentadecan-10-yl)thiazole-4-carboxamido)-2-methyl-5-phenylpentanoate (**144**): UPLC-MS (system 2): *t*ᵣ = 2.50 min, *m*/*z* (ES+) calculated 921.49 (M+H)⁺, found 921.59

Compounds **145-149** of general formula H-Ile-Tuv(O-acyl)-Tup-O-allyl shown in Scheme 14 and listed below were prepared in accordance with the above FMOC deprotection procedure from Compounds **140-144,** respectively.

(2S,4R)-allyl4-(2-((1R,3R)-3-((2S,3S)-2-amino-N,3-dimethylpentanamido)-4-methyl-1-(propionyloxy)pentyl)thiazole-4-carboxamido)-2-methyl-5-phenylpentanoate (1**4**5): UPLC-MS (system 1): *t*ᵣ = 1.29 min, *m*/*z* (ES+) calculated 657.37 (M+H)⁺, found 658.04.

(2S,4R)-allyl 4-(2-((1R,3R)-3-((2S,3S)-2-amino-N,3-dimethylpentanamido)-1-(butyryloxy)-4-methylpentyl)thiazole-4-carboxamido)-2-methyl-5-phenylpentanoate (**146**): UPLC-MS (system 2): *t*ᵣ = 1.24 min, *m*/*z* (ES+) calculated 671.39 (M+H)⁺, found 671.48.

(2S,4R)-allyl 4-(2-((1R,3R)-3-((2S,3S)-2-amino-N,3-dimethylpentanamido)-1-(isobutyryloxy)-4-methylpentyl)thiazole-4-carboxamido)-2-methyl-5-phenylpentanoate (**147**): UPLC-MS (system 1): *t*ᵣ = 1.33 min, *m*/*z* (ES+) calculated 671.39 (M+H)⁺, found 671.33

(2S,4R)-allyl 4-(2-((1R,3R)-3-((2S,3S)-2-amino-N,3-dimethylpentanamido)-4-methyl-1-((3-methylbutanoyl)oxy)pentyl)thiazole-4-carboxamido)-2-methyl-5-phenylpentanoate (148): UPLC-MS (system 2): *t*ᵣ = 1.31 min, *m*/*z* (ES+) calculated 685.40 (M+H)⁺, found 685.49.

(2S,4R)-allyl 4-(2-((1R,3R)-3 -((2S,3 S)-2-amino-N,3-dimethylpentanamido)-1-((3,3 - dimethylbutanoyl)oxy)-4-methylpentyl)thiazole-4-carboxamido)-2-methyl-5-phenylpentanoate (**149**): UPLC-MS (system 2): *t*ᵣ = 1.30 min, *m*/*z* (ES+) calculated 699.42 (M+H)⁺, found 699.51.

Other tripeptides incorporating a tubuvaline-(O-acyl) component are similarly prepared by replacing FMOC-Ile with other aliphatic amino acids (e.g., FMOC-Leu) or aliphatic amine-containing acids.

### Example 41: General procedure for amide coupling of isoleucinetubuvaline(O-O-acyl)-tubuphenylalanine allyl ester tripeptide with tertiary amine-containing acids for introduction of the tubulysin N-terminal component.

(R)-*N*-methyl-pipecolic acid (D-Mep) **60** (2 equivalents) is dissolved in anhydrous dimethylformamide (20-25 mM) and pre-activated with HATU (2 equivalents) and DIPEA (4 equivalents); the mixture is stirred for 10 minutes at room temperature under nitrogen. The activated acid is then added to the tripeptide of Example 40 having the general formula of H-Ile-Tuv(O-Acyl)-Tup-O-allyl; the reaction was stirred at room temperature under nitrogen and monitored by UPLC/MS. Upon reaction completion, glacial acetic acid (14 equivalents) is then added and the tetrapeptide product of general formula D-Mep- Ile-Tuv(O-Acyl)-Tup-O-allyl was purified by preparative HPLC. The allyl protecting group is then removed without loss of the tubuvaline acyl moiety by dissolving the allyl ester-protected tubulysin tetrapeptide (**150-154**) in anhydrous dichloromethane (20 mM) treated with palladium tetrakis-(triphenylphosphine) (0.1 equiv.), triphenylphosphine (0.2 equivalents), and anhydrous pyrrolidine (8 equivalents), and the reaction is stirred at an ambient temperature under nitrogen. Once UPLC/MS reveals conversion to the product free acid, the reaction is quenched with glacial acetic acid (22 equivalents), diluted with acetonitrile and dimethylformamide, and then concentrated by rotary evaporation. The crude tubulysin compounds of general formula D-Mep-Ile-Tuv(O-Acyl)-Tup-O-allyl are then purified by preparative HPLC.

Compounds **150-154** of general formula **D**-Mep-Ile-Tuv(O-acyl)-Tup-O-allyl shown in Scheme 14 and listed below were prepared in accordance with the above peptide coupling procedure from Compounds **145-149,** respectively.

(2S,4R)-allyl 4-(2-((1R,3R)-3-((2S,3S)-N,3-dimethyl-2-((R)-1-methylpiperidine-2-carboxamido)pentanamido)-4-methyl-1-(propionyloxy)pentyl)thiazole-4-carboxamido)-2-methyl-5-phenylpentanoate (**150**): UPLC-MS (system 1): *t*ᵣ = 1.33 min, *m*/*z* (ES+) calculated 782.45 (M+H)⁺, found 781.82.

(2S,4R)-allyl 4-(2-((1R,3R)-1-(butyryloxy)-3-((2S,3S)-N,3-dimethyl-2-((R)-1-methylpiperidine-2-carboxamido)pentanamido)-4-methylpentyl)thiazole-4-carboxamido)-2-methyl-5-phenylpentanoate (**151**): UPLC-MS (system 2): *t*ᵣ = 1.31 min, *m*/*z* (ES+) calculated 796.47 (M+H)⁺, found 796.57.

(2S,4R)-allyl 4-(2-((1R,3R)-3-((2S,3S)-N,3-dimethyl-2-((R)-1-methylpiperidine-2-carboxamido)pentanamido)-1-(isobutyryloxy)-4-methylpentyl)thiazole-4-carboxamido)-2-methyl-5-phenylpentanoate (**152**): UPLC-MS (system 1): *t*ᵣ = 1.37 min, *m*/*z* (ES+) calculated 796.47 (M+H)⁺, found 795.78.

(2S,4R)-allyl 4-(2-((1R,3R)-3-((2S,3S)-N,3-dimethyl-2-((R)-1-methylpiperidine-2-carboxamido)pentanamido)-4-methyl-1-((3-methylbutanoyl)oxy)pentyl)thiazole-4-carboxamido)-2-methyl-5-phenylpentanoate (153): UPLC-MS (system 2): *t*ᵣ = 1.35 min, *m*/*z* (ES+) calculated 810.49 (M+H)⁺, found 810.59.

(2S,4R)-allyl 4-(2-((1R,3R)-3-((2S,3S)-N,3-dimethyl-2-((R)-1-methylpiperidine-2-carboxamido)pentanamido)-1-((3,3-dimethylbutanoyl)oxy)-4-methylpentyl)thiazole-4-carboxamido)-2-methyl-5-phenylpentanoate (**154**): UPLC-MS (system 2): *t*ᵣ = 1.38 min, *m*/*z* (ES+) calculated 824.50 (M+H)⁺, found 824.60.

Compounds **155-159** of general formula **D**-Mep-Ile-Tuv(O-acyl)-Tup-OH shown in Scheme 14 and listed below were prepared in accordance with the above allyl deprotection procedure from Compounds **150-154,** respectively.

(2S,4R)-4-(2-((1R,3R)-3-((2S,3S)-N,3-dimethyl-2-((R)-1-methylpiperidine-2-carboxamido)pentanamido)-4-methyl-1-(propionyloxy)pentyl)thiazole-4-carboxamido)-2-methyl-5-phenylpentanoic acid (**155**): UPLC-MS (system 2): *t*ᵣ = 1.11 min, *m*/*z* (ES+) calculated 742.42 (M+H)⁺, found 742.51.

(2S,4R)-4-(2-((1R,3R)-1-(butyryloxy)-3-((2S,3S)-N,3-dimethyl-2-((R)-1-methylpiperidine-2-carboxamido)pentanamido)-4-methylpentyl)thiazole-4-carboxamido)-2-methyl-5-phenylpentanoic acid (**156**): UPLC-MS (system 2): *t*ᵣ = 1.16 min, *m*/*z* (ES+) calculated 756.44 (M+H)⁺, found 756.54.

(2S,4R)-4-(2-((1R,3R)-3-((2S,3S)-N,3-dimethyl-2-((R)-1-methylpiperidine-2-carboxamido)pentanamido)-1-(isobutyryloxy)-4-methylpentyl)thiazole-4-carboxamido)-2-methyl-5-phenylpentanoic acid (**157**): UPLC-MS (system 1): *t*ᵣ = 1.23 min, *m*/*z* (ES+) calculated 756.44 (M+H)⁺, found 756.82.

(2S,4R)-4-(2-((1R,3R)-3-((2S,3S)-N,3-dimethyl-2-((R)-1-methylpiperidine-2-carboxamido)pentanamido)-4-methyl-1-((3-methylbutanoyl)oxy)pentyl)thiazole-4-carboxamido)-2-methyl-5-phenylpentanoic acid (**158**): UPLC-MS (system 2): *tᵣ =* 1.22 min, *m*/*z* (ES+) calculated 770.45 (M+H)⁺, found 770.55.

(2S,4R)-4-(2-((1R,3R)-3-((2S,3S)-N,3-dimethyl-2-((R)-1-methylpiperidine-2-carboxamido)pentanamido)-1-((3,3-dimethylbutanoyl)oxy)-4-methylpentyl)thiazole-4-carboxamido)-2-methyl-5-phenylpentanoic acid (**159**): UPLC-MS (system 2): *t*ᵣ = 1.23 min, *m*/*z* (ES+) calculated 784.47 (M+H)⁺, found 784.57.

Additional tubulysin O-acyl compounds, including those having variation(S) in amino acid or amine-containing acid inputs replacing the tubuphenylalanine moiety (Compound **40**) of Example 39, as shown in Example 27, and/or the FMOC-amino acid input (FMOC-Ile) of Example 40, are prepared by replacing **D**-Mep with other N-alkylated pipecolic acid inputs or with an N-alkylated azetidinyl-2-carboxylic acid, N-alkylated-D-proline or N,N-dialkyl amino acid such as N,N-dimethylglycine.

### Example 42: Preparation of a Drug-Linker Compound having an Non-cleavable tubulysin drug unit with N-terminal attachment to a Stretcher unit.

A Drug-Linker compound of general formula M¹-A-D, wherein M¹ is a maleimide moiety A is a Stretcher Unit and D is a tubulysin with N-terminal attachment to the M¹-A Linker Unit precursor was prepared as shown in Scheme 15. In that Scheme Compound **172** exemplifies a Drug-Linker compound, whose preparation is described below, wherein the Drug Unit is desmethyl-Tubulysin M (O-CH₃) (**170**) in which the O-linked acetate substituent of the tubuvaline component has been replaced with -OCH₃. That Drug-Linker compound provides a Ligand Drug Conjugate (LDC) through attachment of a Ligand unit through a thiol functional group from a targeting moiety that is resistant to release of free drug. For a LDC derived from Compound **172** and an antibody targeting moiety the active moiety released is typically represented by the general structure of Cys-M²-A-D, wherein M² is a succinimide moiety resulting from Michael addition of a cysteine thiol to the maleimide moiety of the M¹-A-D Drug-Linker compound. The active moiety eventually released from such LDCs can be considered a Tubulysin M (O-ether) in which the methyl substituent of the D-Mep moiety has been replaced with thiol a substituted alkyl moiety.

Des-methyl-Tub(OMe)-O-allyl: (R)-*N*-BOC-pipecolic acid **60** (6 mg, 24 µmol) was dissolved in anhydrous dimethylformamide (0.46 ml, 25 mM) and pre-activated with HATU (9 mg, 24 µmol) and DIPEA (9 µl, 48 µmol); the mixture was stirred for 10 minutes at room temperature under nitrogen. The activated acid was then added to the H-Ile-Tuv(OMe)-Tup-O-allyl tripeptide 57 (10 mg, 12 µmol) of Example 28; the reaction was stirred at room temperature under nitrogen and monitored by UPLC/MS. The reaction was then quenched with acetic acid and purified by preparative HPLC to provide 12 mg (quant.) (R)-tert-butyl 2-(((2S,3S)-1-(((1R,3R)-1-(4-(((2R,4S)-5-(allyloxy)-4-methyl-5-oxo-1-phenylpentan-2-yl)carbamoyl)thiazol-2-yl)-1 -methoxy-4-methylpentan-3-yl)(methyl)amino)-3-methyl-l-oxopentan-2-yl)carbamoyl)piperidine-1-carboxylate (**168**). Analytical UPLC-MS (system 1): *t*ᵣ = 1.99 min, *m*/*z* (ES+) calculated 847.47 (M+Na)⁺, found 847.87.

Deprotection: BOC-Tub(OMe)-OAllyl (**168**, 12 mg, 15 µmol) was taken up in DCM (0.75 ml) stirring under N₂. Pd(PPh₃)₄ (1.7 mg, 1.5 µmol) and PPh₃ (0.8 mg, 3 µmol) were added as solids followed by pyrrolidine (11 µl, 120 µmol). The reaction was stirred to 2 hours at room temperature, then taken up in DMSO, condensed under reduced pressure, and purified by preparative HPLC to provide 9 mg (76%) of (2S,4R)-4-(2-((1R,3R)-3-((2S,3S)-2-((R)-1-(tert-butoxycarbonyl)piperidine-2-carboxamido)-N,3-dimethylpentanamido)-1-methoxy-4-methylpentyl)thiazole-4-carboxamido)-2-methyl-5-phenylpentanoic acid (**169**): Analytical UPLC-MS (system 1): *t*ᵣ = 1.80 min, *m*/*z* (ES+) calculated 786.45 (M+H)⁺, found 786.67

N-terminal deprotection: A flask charged with BOC-TubOMe-OH (**169**, 9 mg, 11 µmol) was cooled to 0 °C under N₂. A solution of 10% TFA in CH₂Cl₂ (0.5 mL) was added dropwise and stirred for 4 hours. The reaction was then taken up in DMSO, condensed under reduced pressure, and purified by preparative HPLC to provide (5 mg) 63% of (2S,4R)-4-(2-((1R,3R)-3-((2S,3S)-N,3-dimethyl-2-((R)-piperidine-2-carboxamido)pentanamido)-1-methoxy-4-methylpentyl)thiazole-4-carboxamido)-2-methyl-5-phenylpentanoic acid (**170**). Analytical UPLC-MS (system 1): *tᵣ* = 1.15 min, *m*/*z* (ES+) calculated 686.40 (M+H)⁺, found 685.59.

L_{b}'-A- coupling: Maleimidocaproic acid **171** (2 mg, 9.5 µmol) was dissolved in anhydrous dimethylformamide (0.5 ml, 20 mM) and pre-activated with HATU (2.3 mg, 6 µmol) and DIPEA (5 µl, 29 µmol); the mixture was stirred for 10 minutes at room temperature under nitrogen. The activated acid was then added to the tubulysin methyl ether **170** (5 mg, 7.3 µmol); the reaction was stirred at room temperature under nitrogen and monitored by UPLC/MS. The reaction was then quenched with acetic acid and purified by preparative HPLC to provide 7 mg (quant.) of (2S,4R)-4-(2-((1R,3R)-3-((2S,3S)-2-((R)-1-(6-(2,5-dioxo-2,5-dihydro-1H-pyrrol-1-yl)hexanoyl)piperidine-2-carboxamido)-N,3-dimethylpentanamido)-1-methoxy-4-methylpentyl)thiazole-4-carboxamido)-2-methyl-5-phenylpentanoic acid (**172**). Analytical UPLC-MS (system 1): *t*ᵣ = 1.60 min, *m*/*z* (ES+) calculated 879.47 (M+H)⁺, found 879.11.

The quaternary amine conjugates as described herein conditionally release tubulysins having a tertiary amine-containing N-terminal component wherein the nitrogen atom of that component is directly substituted by an alkyl moiety (i.e., through a nitrogencarbon bond). In contrast the active drug moiety non-conditionally release released from a Ligand Drug Conjugate prepared from Drug-Linker compound **172** has a secondaryamine containing N-terminal component substituted to a alkylene through an amide functional group.

### Example 43: Preparation of a Drug-Linker compound having quaternized Dolastatin 10 incorporating a Glucuronide unit.

Quaternization: A pressure vessel was charged with brominated Stretcher-Glucuronide unit intermediate **2** (62 mg, 76 µmol) of Example 1 and dolastatin 10 (**173**, 45 mg, 51 µmol) in anhydrous 2-butanone (1.0 mL). The reaction vessel was flushed with N₂ and sealed. The reaction was then stirred and heated to 80 °C for 12 hours. The resulting mixture was cooled, condensed under reduced pressure, and carried forward without further purification to provide (S)-N-(3-(3-((((9H-fluoren-9-yl)methoxy)carbonyl)amino)-propanamido)-4-(((2S,3R,4S,5S,6S)-3,4,5-triacetoxy-6-(methoxycarbonyl)tetrahydro-2H-pyran-2-yl)oxy)benzyl)-1-(((S)-1-(((3R,4S,5S)-3-methoxy-1-((S)-2-((1R,2R)-1 -methoxy-2-methyl-3-oxo-3-(((S)-2-phenyl-1 -(thiazol-2-yl)ethyl)amino)propyl)pyrrolidin-1-yl)-5-methyl-1-oxoheptan-4-yl)(methyl)amino)-3-methyl-1-oxobutan-2-yl)amino)-N,N,3-trimethyl-1-oxobutan-2-aminium (**174**). Analytical UPLC-MS (system 2): *t*ᵣ = 1.42 min, *m*/*z* (ES+) calculated 1515.74 (M)⁺, found 1515.91.

Glycoside deprotection: A flask was charged with FMOC-GlucQ-D10 (**174**, 39 mg, 26 µmol) in THF (0.42 mL) and MeOH (0.42 mL). This solution was stirred under N₂ and cooled to 0 °C. LiOH·H₂O (8.6 mg, 206 µmol) was solubilized in H₂O (0.42 mL) then added dropwise. The reaction was allowed to warm to room temperature and stirred for 2 hours. The reaction was then quenched with acetic acid (12 µL, 206 µmol) and condensed under reduced pressure. The residue was taken up in minimal DMSO and purified by preparative HPLC to provide 7 mg (24%) (S)-N-(3-(3-aminopropanamido)-4-(((2S,3R,4S,5S,6S)-6-carboxy-3,4,5-trihydroxytetrahydro-2H-pyran-2-yl)oxy)benzyl)-1-(((S)-1-(((3R,4S,5S)-3-methoxy-1-((S)-2-((1R,2R)-1-methoxy-2-methyl-3-oxo-3-(((S)-2-phenyl-1-(thiazol-2-yl)ethyl)amino)propyl)pyrrolidin-1-yl)-5-methyl-1-oxoheptan-4-yl)(methyl)amino)-3-methyl-1-oxobutan-2-yl)amino)-N,N,3-trimethyl-1-oxobutan-2-aminium (**175**). Analytical UPLC-MS (system 2): *t*ᵣ = 0.90 min, *m*/*z* (ES+) calculated 1153.62 (M)⁺, found 1153.78.

L_{SS} (mDPR) coupling: A flask was charged with H-GlucQ-D10 (**175**, 7.0 mg, 6.0 µmol) in anhydrous DMF (0.62 mL). mDPR(BOC)-OSu (**6**, 2.3 mg, 6.1 µmol) was added under N₂. N,N-Diisopropylethylamine (4.2 µL, 24 µmol) was added and the reaction was stirred at room temperature for 3 hours. The reaction was then quenched with acetic acid (4.2 µL) and purified by preparative HPLC to provide 5.5 mg (64%) (S)-N-(3-(3-((S)-3-((tert-butoaycarbonyl)amino)-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzyl)-1-(((S)-1-(((3R,4S,5S)-3-methoxy-1-((S)-2-((1R,2R)-1-methoxy-2-methyl-3-oxo-3-(((S)-2-phenyl-1-(thiazol-2-yl)ethyl)amino)propyl)pyrrolidin-1-yl)-5-methyl-1-oxoheptan-4-yl)(methyl)amino)-3-methyl-1-oxobutan-2-yl)amino)-N,N,3-trimethyl-1-oxobutan-2-aminium (**176**). Analytical UPLC-MS (system 2): *t*ᵣ = 1.18 min, *m*/*z* (ES+) calculated 1419.71 (M)⁺, found 1419.87.

L_{SS} (mDPR) deprotection: A flask containing mDPR(BOC)-GlucQ-D10 (**176,** 5.5 mg, 3.9 µmol) was cooled to 0 °C under N₂. A solution of 10% TFA in CH₂Cl₂ (0.39 mL) was added dropwise and stirred for 4 hours. The reaction was then taken up in DMSO, condensed under reduced pressure, and purified by preparative HPLC to provide 5.1 mg (99%) of (S)-N-(3-(3-((S)-3-amino-2-(2,5-dioxo-2,5-dihydro-1H-pyrrol-1-yl)propanamido)propanamido)-4-(((2S,3R,4S,5S,6S)-6-carboxy-3,4,5-tnhydroxytetrahydro-2H-pyran-2-yl)oxy)benzyl)-1-(((S)-1-(((3R,4S,5S)-3-methoxy-1-((S)-2-((1R,2R)-1-methoxy-2-methyl-3-oxo-3-(((S)-2-phenyl-1-(thiazol-2-yl)ethyl)amino)propyl)pyrrolidin-1-yl)-5-methyl-1-oxoheptan-4-yl)(methyl)amino)-3-methyl-1 -oxobutan-2-yl)amino)-N,N,3-trimethyl-1-oxobutan-2-aminium (177). Analytical UPLC-MS: *t*ᵣ = 0.95 min, *m*/*z* (ES+) calculated 1319.66 (M)⁺, found 1319.81.

### Example 44: Preparation of a Drug-Linker compound having Dolastatin 10 with carbamate attachment to a Glucuronide unit

Carbamate Drug unit attachment: A flask was charged with FMOC-Gluc-PNP *(*Bioconjugate Chem., 2006, 17, 831-840.) (**178,** 60 mg, 66 µmol) and monomethyl dolastatin 10 (**179**, 46 mg, 60 µmol) in anhydrous DMF (0.96 mL) to which pyridine (0.24 mL) was added followed by HOBt (3.2 mg, 24 µmol). The reaction was allowed to stir at room temperature overnight, condensed under reduced pressure, the purified by flash chromatography (DCM/MeOH) to provide 54 mg (59%) (2S,3R,4S,5S,6S)-2-(2-(3-((((9H-fluoren-9-yl)methoay)carbonyl)amino)-Opropanamido)-4-((5S,8S,11S,12R)-11-((S)-sec-butyl)-5,8-diisopropyl-12-(2-((S)-2-((1R,2R)-1-methoxy-2-methyl-3-oxo-3-(((S)-2-phenyl-1-(thiazol-2-yl)ethyl)amino)propyl)-pyrrolidin-1-yl)-2-oxoethyl)-4,10-dimethyl-3,6,9-trioxo-2,13-dioxa-4,7,10-triazatetradecyl)-phenoxy)-6-(methoxycarbonyl)tetrahydro-2H-pyran-3,4,5-triyl triacetate (**180**). Analytical UPLC-MS (system 2): *t*ᵣ = 1.65 min, *m*/*z* (ES+) calculated 1545.71 (M+H)⁺, found 1545.98.

Glycoside deprotection: A flask was charged with Fmoc-Gluc-MMD10 (**180**, 54 mg, 35 µmol) in THF (0.78 mL) and MeOH (0.78 mL). This solution was stirred under N₂ and cooled to 0 °C. LiOH·H₂O (12 mg, 278 µmol) was solubilized in H₂O (0.78 mL) then added dropwise. The reaction was allowed to warm to room temperature and stirred for 2 hours. The reaction was then quenched with acetic acid (17 µL, 278 µmol) and condensed under reduced pressure. The residue was taken up in minimal DMSO and purified by preparative HPLC to provide 9.6 mg (23%) of (2S,3S,4S,5R,6S)-6-(2-(3-aminopropanamido)-4-((5S,8S,11S,12R)-11-((S)-sec-butyl)-5,8-diisopropyl-12-(2-((S)-2-((1R,2R)-1-methoxy-2-methyl-3-oxo-3-(((S)-2-phenyl-1-(thiazol-2-yl)ethyl)amino)propyl)pyrrolidin-1-yl)-2-oxoethyl)-4,10-dimethyl-3,6,9-trioxo-2,13-dioxa-4,7,10-triazatetradecyl)phenoxy)-3,4,5-trihydroxytetrahydro-2H-pyran-2-carboxylic acid (**181**). Analytical UPLC-MS (system 2): *t*ᵣ = 1.13 min, *m*/*z* (ES+) calculated 1183.60 (M+H)⁺, found 1183.70.

Glucuronide-Drug unit coupling: A flask was charged with H-Gluc-MMD10 (**181**, 9.6 mg, 8.1 µmol) in anhydrous DMF (0.81 mL). mDPR(Boc)-OSu (**6**, 3.4 mg, 8.9 µmol) was added under N₂. N,N-Diisopropylethylamine (5.7 µL, 32 µmol) was added and the reaction was stirred at room temperature for 3 hours. The reaction was then quenched with acetic acid (5.7 µL) and purified by preparative HPLC to provide 5.2 mg (44%) of (2S,3S,4S,5R,6S)-6-(2-(3-((S)-3-((tert-butoxycarbonyl)amino)-2-(2,5-dioxo-2,5-dihydro-1H-pyrroL1-yl)propanamido)propanamido)-4-((5S,8S,11S,12R)-11-((S)-sec-butyl)-5,8-diisopropyl-12-(2-((S)-2-((1R,2R)-1-methoxy-2-methyl-3-oxo-3-(((S)-2-phenyl-1-(thiazol-2-yl)ethyl)amino)propyl)pyrrolidin-1-yl)-2-oxoethyl)-4,10-dimethyl-3,6,9-trioxo-2,13-dioxa-4,7,10-triazatetradecyl)phenoxy)-3,4,5-trihydroxytetrahydro-2H-pyran-2-carboxylic acid (**182**). Analytical UPLC-MS (system 2): *t*ᵣ = 1.40 min, *m*/*z* (ES+) calculated 1449.69 (M+H)⁺, found 1449.80.

Deprotection: A flask containing mDPR(Boc)-Gluc-MMD10 (**182**, 5.6 mg, 3.9 µmol) was cooled to 0 °C under N₂. A solution of 10% TFA in CH₂Cl₂ (0.39 mL) was added dropwise and stirred for 4 hours. The reaction was then taken up in DMSO, condensed under reduced pressure, and purified by preparative HPLC to provide 5.2 mg (99%) of (2S,3S,4S,5R,6S)-6-(2-(3-((S)-3-amino-2-(2,5-dioxo-2,5-dihydro-1H-pyrrol-1-yl)propanamido)propanamido)-4-((5S,8S,11S,12R)-11-((S)-sec-butyl)-5,8-diisopropyl-12-(2-((S)-2-((1R,2R)-1-methoay-2-methyl-3-oxo-3-(((S)-2-phenyl-1-(thiazol-2-yl)ethyl)amino)propyl)pyrrolidin-1-yl)-2-oxoethyl)-4,10-dimethyl-3,6,9-trioxo-2,13-dioxa-4,7,10-triazatetradecyl)phenoxy)-3,4,5-trihydroxytetrahydro-2H-pyran-2-carboxylic acid (**183**). Analytical UPLC-MS (system 2): *t*ᵣ = 1.13 min, *m*/*z* (ES+) calculated 1349.64 (M+H)⁺, found 1349.75.

Scheme 17 for Example 44 represents an exemplary reaction sequence for preparing Drug-Linker compounds having carbamate attached Drug units. The Ligand Drug Conjugates obtainable from such carbamate Drug-Linker compound, which contain non-quaternized Drug Units, can be compared to the quaternized version whose synthesis of the requisite quaternized Drug Linker compounds is provided by generalization of Scheme 16. For adaptation to a carbamate Drug-Linker compound, an alkyl substituent must be removed from the tertiary amine function group of a tertiary amine-containing drug if its attachment as the Drug unit is to be through the nitrogen of that functional group. Release of free drug from a Ligand Drug Conjugate having that modification in the Drug unit will release the modified drug, which is expected to have different properties in comparison to the parent drug that may be undesirable.

### Example 45: Preparation of a Drug-Linker compound having quaternized Auristatin F incorporating a Glucuronide unit

Auristatin F C-terminal protection: A round bottom flask was charged with Auristatin F **184** (150 mg, 201 µmol) and dissolved in allyl alcohol (10 mL). The reaction was cooled to 0 °C and allyl pyrocarbonate (149 mg, 804 µmol) was added followed by DMAP (7.3 mg, 60 µmol). The reaction extruded CO₂ vigorously and slowed down after 15 min. The reaction was analyzed by UPLC after stirring at RT for 2 h and showed >90% conv. The reaction was concentrated in vacuo and the crude was purified by silica gel chromatography (0-25% MeOH). The fractions were concentrated to dryness to afford 111 mg (71%) of (S)-allyl 2-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-3-phenylpropanoate (**185**). Analytical UPLC-MS (system 2): *t*ᵣ = 1.1 min, *m*/*z* (ES+) calculated 786.54 (M+H)⁺, found 786.72.

Quaternization: A 20 mL flask containing AF-OAllyl **185** (111 mg, 141 µmol) was added brominated Stretcher-Glucuronide unit intermediate **2** (160 mg, 197 µmol) and dry DMF (5 mL). The solution was stirred at 65 °C for 16 h and the reaction was complete by UPLC. Reaction was dried, taken up in DMSO and purified by HPLC. The product fractions were concentrated to dryness to afford 85 mg (40 %) of (S)-N-(3-(3-((((9H-fluoren-9-yl)methoxy)carbonyl)amino)propanamido)-4-(((2S,3R,4S,5S,6S)-3,4,5-triacetoxy-6-(methoxycarbonyl)tetrahydro-2H-pyran-2-yl)oxy)benzyl)-1-(((S)-1-(((3R,4S,5 S)-1 -((S)-2-((1R,2R)-3 -(((S)-1 -(allyloxy)-1 -oxo-3 -phenylpropan-2-yl)amino)-1 - methoay-2-methyl-3-oxopropyl)pyrrolidin-1-yl)-3-methoay-5-methyl-1-oxoheptan-4-yl)(methyl)amino)-3-methyl-1-oxobutan-2-yl)amino)-N,N,3-trimethyl-1-oxobutan-2-aminium (**186**). Analytical UPLC-MS (system 2): *t*ᵣ = 1.45 min, *m*/*z* (ES+) calculated 1516.77 (M)⁺, found 1517.03.

Glycoside deprotection: A 20 mL vial containing protected GlucQ-AF-OAllyl **186** (85 mg, 56 µmol) was added THF (4 mL) and MeOH (4 mL). The reaction was cooled to 0 °C and a solution of LiOH (18 mg/mL in water) was added in one portion. The reaction was stirred at RT for 3 h, and the reaction was complete by UPLC. The solvent was removed in vacuo and the residue was taken up in DMSO/H₂O (1:1). HPLC prep followed by lyophilization afforded 42 mg (62 %) of (S)-N-(3-(3-aminopropanamido)-4-(((2S,3R,4S,5S,6S)-6-carboxy-3,4,5-trihydroxytetrahydro-2H-pyran-2-yl)oxy)benzyl)-1 -(((S)-1-(((3R,4S,5S)-1-((S)-2-((1R,2R)-3-(((S)-1-carboxy-2-phenylethyl)amino)-l-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxobutan-2-aminium (**187**). Analytical UPLC-MS (system 2): *t*ᵣ = 0.88 min, *m*/*z* (ES+) calculated 1114.63 (M)⁺, found 1114.83.

L_{SS} (mDPR) coupling: A 3 mL vial was charged with NH₂-GluQ-AF **187** (42 mg, 39 µmol) and dry DMF (1.6 mL). The solution was stirred and mDPR-(Boc)-OPfp **68** (19 mg, 42 µmol) was added. The reaction was stirred for 5 min at RT then DIPEA (13 µL, 77 µmol) was added. The reaction was stirred for 1 h at RT and the reaction was complete by UPLC. HPLC prep of the crude followed by lyophilization afforded 13 mg (24 %) of (S)-N-(3-(3-((S)-3-((tert-butoxycarbonyl)amino)-2-(2,5-dioxo-2,5-dihydro-1H-pyrrol-l-yl)propanamido)-propanamido)-4-(((2S,3R,4S,5S,6S)-6-carboxy-3,4,5-tnhydroxytetrahydro-2H-pyran-2-yl)oxy)benzyl)-1-(((S)-1-(((3R,4S,5S)-1-((S)-2-((1R,2R)-3-(((S)-1-carboxy-2-phenylethyl)amino)-1-methoxy-2-methyl-3-oxopropyl)pyrrolidin-1-yl)-3-methoay-5-methyl-1-oxoheptan-4-yl)(methyl)amino)-3-methyl-1-oxobutan-2-yl)aminoamino)-N,N,3-trimethyl-1-oxobutan-2-aminium (**188**). Analytical UPLC-MS (system 2): *t*ᵣ = 1.12 min, *m*/*z* (ES+) calculated 1380.72 (M)⁺, found 1380.87.

L_{SS} (mDPR)-Ao-Y(W)-D⁺ deprotection: A 20 mL vial was containing Boc-mDPR-GlucQ-AF (13 mg, 9 µmol) was cooled to 0 °C and a solution of 20% TFA in DCM (1.0 mL) was added. The reaction was allowed to slowly warm to RT and stirred for 4h, and the reaction was complete by UPLC. DMSO/0.1% TFA in H₂O was added to the reaction and then purified on HPLC to afford 1.5 mg (33 %) of (S)-N-(3-(3-((S)-3-amino-2-(2,5-dioxo-2,5-dihydro-1H-pyrrol-1-yl)propanamido)propanamido)-4-(((2S,3R,4S,5S,6S)-6-carboxy-3,4,5-trihydroxytetrahydro-2H-pyran-2-yl)oxy)benzy1)-1-(((S)-1-(((3R,4S,5S)-1-((S)-2-((1R,2R)-3-(((S)-1-carboxy-2-phenylethyl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-N,N,3-trimethyl-1-oxobutan-2-aminium (**189**). Analytical UPLC-MS (system 2): *t*ᵣ = 0.86 min, *m*/*z* (ES+) calculated 1280.67 (M)⁺, found 1280.80.

Scheme 18 for Example 45 and Scheme 16 for Example 43 along with Scheme 1 provides representative routes for preparing Drug-Linker compounds of general formula L_{b}'-Ao-Y(W)-D⁺, more specifically M₁-A₁-A_{O}-Y(W)-D⁺ wherein D⁺ is a quaternized auristatin (i.e., dolastatin 10, auristatin E, or auristatin F, respectively). Other Drug-Linker compounds of that general formula are prepared with additional auristatin compounds having a tertiary amine functional group, particularly when present as the functional group of an N-terminal amino acid component.

### Example 46: Preparation of a Drug-Linker Compound having a conditionally releasable tubulysin Drug unit with C-terminal attachment to a Stretcher unit.

Tubulysin M-hydrazide was synthesized as a free drug (**190**) and coupled to the Val-Cit-PAB-PNP activated Drug-Linker compound intermediate (**192**) as shown in Scheme 24. Briefly, tubulysin M (**26**) was coupled to tertiary-butyl carbazate through activation with 1-ethyl-3-(3-dimethylaminopropyl)carbodiimide in the presence of hydroxybenzotriazole. The coupled product was BOC-deprotected under standard conditions with trifluoroacetic acid in dichloromethane to afford the free drug, tubulysin M-hydrazide (1R,3R)-3-((2S,3S)-N,3-dimethyl-2-((R)-1-methylpiperidine-2-carboxamido)pentanamido)-1-(4-(((2R,4S)-5-hydrazinyl-4-methyl-5-oxo-1-phenylpentan-2-yl)carbamoyl)thiazol-2-yl)-4-methylpentyl acetate (**190**). A cleavable MC-Val-Cit-PABN-D Drug-Linker compound of general formula M¹-A-W-Y-D, wherein M¹ is a maleimide moiety, A is a alkylene as the Spacer Unit, W is -W₂-W₁-, wherein W₁ is citrulline and W₂ is valine, Y is a PAB moiety as the self-immolative Spacer Unit attached to the tubulysin Drug Unit through a hydrazide functional group, was prepared by reacting the hydrazide nitrogen with PNP-activated Drug-Linker compound intermediate 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate (**191**) in the presence of diisopropylethylamine, hydroxybenzotriazole, and pyridine as a co-solvent, affording Drug-Linker compound 4-((S)-2-((S)-2-(6-(2,5-dioxo-2,5-dihydro-1H-pyrrol-1-yl)hexanamido)-3-methylbutanamido)-5-ureidopentanamido)benzyl 2-((2S,4R)-4-(2-((1R,3R)-1-acetoxy-3-((2S,3S)-N,3-dimethyl-2-((R)-1-methylpiperidine-2-carboxamido)pentanamido)-4-methylpentyl)thiazole-4-carboxamido)-2-methyl-5-phenylpentanoyl)hydrazinecarboxylate (**192**) in 33% yield from the Tubulysin M-hydrazide (**190**).

Conditional proteolysis of a Ligand Drug Conjugate prepared from Drug-Linker compound **192** releases Compound **190** as the active drug moiety in which the C-terminal residue remains modified as its hydrazide. The quaternary amine conjugates as described herein release tubulysins having a free carboxylic acid functional group at their C-termini.

### Example 47: Assessment of de-acylation of tubuvaline (O-acyl) component of Ligand Drug Conjugates having a quaternized Tub(O-acyl) Drug Unit.

In a 2.5 µm polypropylene 96-well filter plate, 1 ml PBS was added to each well. A 200 µl slurry of mAbSelect^{™} Protein A resin was applied to each well, and the aqueous component was vacuumed away. A 20 µl aliquot of each thawed plasma sample was applied to the resin and allowed to mix at 4 C for 1 hour. The flow-through was collected in a plate by centrifugation (500g, 3 minutes) and the resin was washed with 200 µl [2X] papain buffer (40mM KPO₄, 20mM EDTA, pH 7) via another centrifugation.

Papain enzyme was dissolved at 2.5 mg/ml in [1X] papain buffer (20 mM KPO₄, 10 mM EDTA, 2 mM cysteine, pH 7) and incubated at 37 C for 15 minutes. A 200 µl aliquot of papain was added to each well with resin. The plate was sealed and incubated for 2 hours at 37 C. The flow-through was recovered via centrifugation (500g, 3 minutes). The resin was washed with 100 µl [1X] papain buffer and combined with the flow-through. The samples were transferred to Eppendorf tubes and 1000 µl of ice cold MeOH was added to each tube and incubated on ice for 15 minutes. The samples were precipitated by centrifugation at 16,000g for 5 minutes at 4 C, and the supernatants were transferred to a 96-well plate.

An eight-point standard curve ranging from 1-10,000 nM for each drug was prepared in 50% MeOH. A 20 µL aliquot of each standard was added to the 96-well plate that also contained the samples. A 300 µL aliquot of 20 mM KPO₄, 10 mM EDTA, 2 mM cysteine (1X papain buffer) and 1000 µL MeOH were added to each standard well. The plate was evaporated under nitrogen to dryness.

The plate was reconstituted with 70 µl 20% acetonitrile + 0.1% formic acid and a 50 µL aliquot was injected onto a Waters Acquity LC coupled to a Quattro Premier triple quadrupole mass spectrometer. The mass spectrometer measures the transition of the parent ions to the MS/MS fragment ions, resulting in a peak area for each analyte in the sample and standard. The standard peak areas were plotted as a function of drug concentration and the peak areas measured from the samples were quantified using the equation of the line determined by the standard curve.

### Example 47. In vitro cytotoxicity of a Ligand Drug Conjugate having a quaternized tubulysin as the D⁺ moiety.

An LDC composition, which targets lymphoma cells, was obtained from Drug Linker compound **30** of Scheme 5 and monoclonal antibody cAC10, wherein the composition predominately contains LDCs having 8 D⁺ units wherein D⁺ is quaternized tubulysin M. That predominate LDC is represented by the following structure 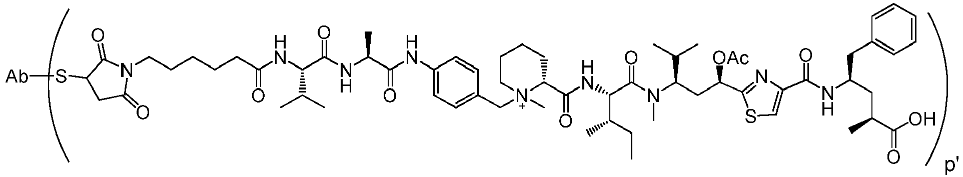 wherein Ab is cAC10 and p' is 8 and S is a sulfur atom of an antibody cysteine residue. Cytotoxicity of that LDC, which is comprised a monoclonal antibody targeting CD30 antibody Ligand unit, a dipeptide Cleavable unit and quaternized tubulysin M, towards a panel of CD30⁺ lymphoma cell lines was compared against a control conjugate wherein the cAC10 antibody is replaced by a control antibody (h2H12) that targets CD33. Cytotoxicity of the cAC10-D⁺ conjugate occurs after its cellular internalization into CD30+ cells and proteolytic cleavage of the Val-Ala dipeptide, which is the cleavable unit of the LDC, to release free tubulysin M. The results of that comparison are provided in Table 2 in which IC₅₀'s for cell growth inhibition are provided in units of ng/mL.

**Table 2. In vitro Cytotoxicity of a Quaternized Tubulysin M Ligand Drug Conjugate targeted to CD30⁺ lymphoma cells**

| Ab | Karpas-299 | L540cy | KM-H2 | L428 | L540cy parental | L540cy-BVR | Del parental | DELI BVR |
|---|---|---|---|---|---|---|---|---|
| cAC10 | 0.07 | 1 | 0.3 | 0.2 | 2 | 2 | 0.3 | 0.6 |
| h2H12 | >10,000 | >10,000 | >10,000 | >10,000 | >10,000 | >10,000 | >10,000 | >10,000 |

Notably KM-2, L428, L540cy-BVR and Del/BYR are multi-drug resistant cell lines. An LDC composition was prepared in which the methyl substituent from the Mep residue of tubulysin M was removed and the resulting secondary amine was linked through a carbamate to a PAB self-immolating moiety that is activated by cathepsin cleavage of a valine-citrulline (vc) cleavable unit. That LDC composition, which was prepared from Drug Linker compound **172** as described in Example 53, was found to be inactive, which confirms the importance of the tertiary amine pharmacophore for the cytotoxic activity of tubulysins.

The carboxylic acid moiety in the Tup/Tut residue was also found to be intolerant to modification when used an alternative site of conjugation. Thus, when that carboxylic acid was converted to a hydrazide to allow for attachment of tubulysin M through its Tup residue to the same PAB self-immolating moiety (also through the carbamate functional group) the conjugate prepared from Drug Linker compound MC-Val-Cit-PABC-NHNH-Tub M, abbreviated in Table 3 as vcPABN (hydrazide), was found to have lost significant activity when compared to the conjugate prepared from the quaternized drug linker compound MC-Val-Ala-PAB4-TubM abbreviated as vaPAB4 . That result confirms the importance of the free carboxylic acid for cytotoxicity of Tubulysin M (since the released drug is modified as the hydrazide at that position) and is summarized in Table 3 for cOKT9 conjugates, which target the CD71 antigen. In Table 3, cOKT9-vaPAB4 (having predominate 8 load), which releases fee tubulysin M, corresponds in structure to the cAC10 conjugate of Table 2 in which the cAC10 targeting moiety is replaced cOKT9. cOKT9-vcPABN (hydrazide) having DAR of 6.7 of Table 3 is the carbamate linked tubulysin construct corresponding to tubulysin M in which the tubulysin released is modified at the Tup residue as described above. The prominent LDC in both compositions has 8 drugs (or 8 quaternized drug units) bound per antibody.

**Table 3. In vitro Cytotoxicity of a quaternized tubulysin Ligand Drug Conjugate targeting C71⁺ lymphoma cells in comparison to non-quaternized tubulysin Ligand Drug Conjugate having attachment to the C-terminal component**

| cOKT9 ADC | HCT-116 | L428 | KM-H2 | L548cy | MM.1R | HL60cy |
|---|---|---|---|---|---|---|
| vaPAB4 | 15 (7) | 83 (29) | 4 (0) | 6(7) | 4 (2) | 28(0) |
| vcPABN (hydrazide) | 302 (1) | 284(16) | 56 (0) | 117 (7) | 19(4) | 85 (0) |

The parenthetic values indicate the percentages of viable cells remaining at the bottom of the dose-response curve. Copy numbers of CD71 for HT-116, L-427, KH-H2, L540cy, MM.1R and HL60cy cell lines that were tested are 5K, 35K, 121K, 33K, 32K and 228K, respectively. The results of Table 3 are confirmed by Example 64 in which the cOKT9-hydrazide ADC having the drug linker moiety MC-Val-Cit-PABN-TubM (**192**) is compared against the cOKT9 ADC prepared from Drug Linker compound MC-Val-Ala-PAB4-TubM (**30**)

The results of Table 2 and Table 3 indicate that potent and immunologically specific Ligand Drug Conjugates are obtained when a tubulysin is incorporated into such constructs as a quaternized drug unit and that such conjugates are active against multiresistant cell lines. Furthermore, it was surprisingly found that the quaternized conjugate obtained from compound **30** had the ability to kill cells not expressing CD30 antigen when those cells were co-cultured with CD30+ cells that are targeted by the conjugate. Thus, the LDC composition exhibited an IC₅₀ of 6 ng/mL against a co-culture of L540cy:U268Luc⁺ cells, with 0% viable cells remaining at the bottom of the dose-response curve. L540cy are CD30⁺ and U266 are CD30⁻

### Example 48. In vitro cytotoxicity of Ligand Drug Conjugates having a quaternized auristatin as the D⁺ moiety.

Cytotoxicity of the LDC comprised of the monoclonal antibody cAC10, which targets CD30, and quaternized auristatin E towards a panel of CD30⁺ lymphoma cell lines was compared against a control conjugate wherein the cAC10 antibody is replaced by a control antibody (hBU12) that targets CD19. Control cells having no detectable CD30⁺ antigen, but having C19 antigen, are also used. Cytotoxicity of the cAC10-D⁺ conjugate occurs after its cellular internalization into CD30⁺ cells and cathepsin cleavage of the Val-Cit dipeptide, which is the cleavable unit of the LDC, for conjugates designated in Table 4 as vcPAB4-AE, or glucuronidase cleavage of the carbohydrate glycoside bond, which is the cleavage site of cleavable units for cAC10 conjugates designated as gluc4-AE, to release free auristatin E. Likewise, cytotoxicity occurs for the analogous hBU12 conjugates when these conjugates are internalized into CD19⁺ cells having sufficient copy number of the relevant antigen. The results of those comparisons are provided in Table 4 in which IC₅₀'s for cell growth inhibition is provided in units of ng/mL.

The LDC compositions designated as vcPAB4-AE in Table 4 were obtained from compound **14** of Scheme 2 and monoclonal antibody cAC10, which targets CD30⁺ lymphoma cells, or monoclonal antibody hBU12, which targets C19⁺ cells, wherein the composition predominately contains LDCs having 8 D⁺ units, wherein D⁺ is quaternized auristatin E. That predominate LDC, after controlled hydrolysis of the succinimide ring system, is represented by the following structure: 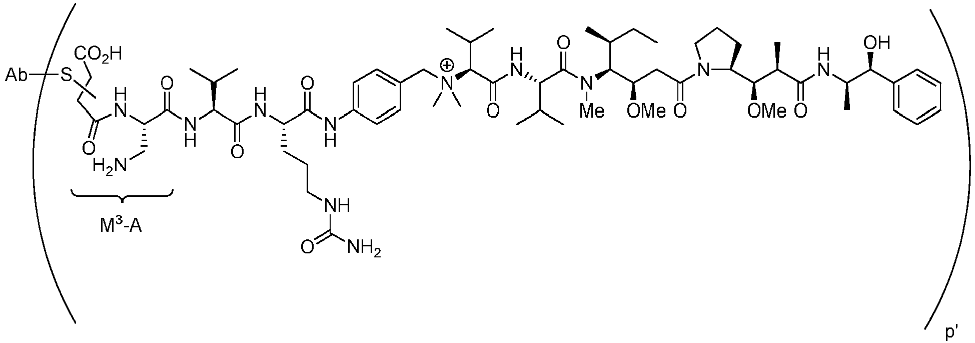 wherein the Ab-S- moiety of cAC10 or hBU12 is bonded to the carbon α or β to the M³ carboxylic acid. M³-A indicated in the above structure represents a selfstabilized (L_{S}) linker moiety, which is bonded to the Val-Cit dipeptide that serves as the cleavable unit.

The LDC compositions designated as gluc4-AE in Table 4 were obtained from compound **8** of Scheme 1 and monoclonal antibody cAC10, which targets CD30⁺ lymphoma cells and monoclonal antibody hBU12, which targets C19⁺ cells wherein D⁺ is quaternized auristatin E. The predominate LDC of the composition, after controlled hydrolysis of the succinimide ring system, is represented by the following structure: 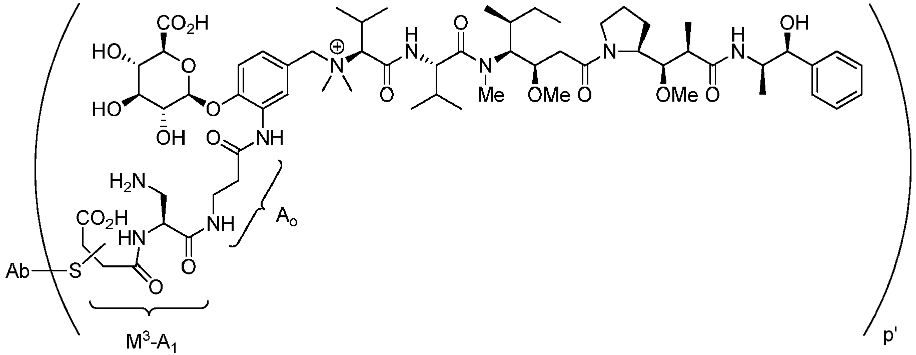 wherein the Ab-S- moiety of cAC10 or hBU12 is bonded to the carbon α or β to the M³ carboxylic acid. M³-A₁ indicated in the above structure represents a selfstabilized (L_{S}) linker moiety, which is bonded to the Aₒ subunit of A.

Both vcPAB4-AE and gluc4-AE composition predominately contain LDCs having 8 D⁺ units per antibody (i.e., p' is 8).

**Table 4. In vitro Cytotoxicity of a Quaternized Auristatin Ligand Drug Conjugates targeting CD30⁺ lymphoma cells or C19⁺ lymphoma cells.**

| ADC | -Linker Description | **Karpas-299** | **L428** | **L540cy parental** | **Ramos** | **RL** |
|---|---|---|---|---|---|---|
| cAC10-**8** | gluc4-AE | 0.7 (0) | 7 (32) | 2 (3) | >10,000 (0) | >10,000 (77) |
| cAC10-**14** | vcPAB4-AE | 0.4 (4) | 4.6 (24) | 1.5 (3) | 6173 (0) | >10,000 (52) |
| hBU12-**8** | gluc4-AE | >10,000 (63) | >10,000 (100) | >10,000 (100) | 1.3 (0) | >10,000 (76) |
| hBU12-**14** | vcPAB4-AE | 3903 (10) | 8614 (41) | 5080 (44) | 0.7 (0) | 2110 (21) |

Karpas 299 are CD30⁺ (copy number 238K) and have low amounts of CD19 (copy number 10K), L-428 are CD30⁺ (copy number 77K) and have no detectable C19 antigen, and L540cy are CD30⁺ (copy number 433K) and have low amounts of CD19 (copy number 23K). On the other hand, Ramos and RL have no detectable CD30 and have differing levels of CD19 (32K vs. 18K, respectively). The parenthetic values indicate the percentages of viable cells remaining at the bottom of the dose-response curve.

The results of Table 4 indicate that potent and immunologically specific LDCs are obtained when a tertiary amine-containing auristatin is incorporated into such constructs as a quaternized drug unit.

### Example 49. In vitro Cytotoxicity of Tubulysin (O-ether) compounds as free drugs.

Cells were treated for 96 hours with tubulysin ether free acid compounds of general formula Tub(O-Et)-OH (**64-66**), or are treated with tubulysin M (**26**), then assessed for viability as described in the methods. The results are summarized in Table 5 with IC₅₀ values given in nM concentration units.

**Table 5. In vitro Cytotoxicity of Tubulysin (O-ether) compounds as free drugs in comparison to Tubulysin M**

| compd | Description | **Karpas29 9,** ALCL | **L540cy,** HL | **L428,** MDR+ HL | **HL60,** AML | **HL60/RV,** MDR+ AML |
|---|---|---|---|---|---|---|
| **26** | TubM | 0.17 | 0.14 | 0.11 | 0.3 | 2.2 |
| **64** | Tub(OMe)-OH | 0.19 | 0.12 | 0.5 | 0.13 | 29 |
| **65** | Tub(OEt)-OH | 0.05 | 0.05 | 0.14 | 0.05 | 5 |
| **66** | Tub(OPr)-OH | 0.15 | 0.13 | 0.4 | 0.17 | 14 |

All four test articles were highly potent on all cell lines tested. The tubulysin ethyl ether compound Tub(OEt) (**62**) trended more potent than the methyl (**61**) and propyl (**63**) analogs. The ethyl ether and tubulysin M compounds maintained the highest level of potency in the context of MDR+ L428 and HL60/RV cell lines.

### Example 50. In vitro Cytotoxicity of Tubulysin (O-Acyl) compounds as free drugs.

Cells were treated for 96 hours with tubulysin esters **155-159** (i.e., Tub(O-ester)-OH) or tubulysin M (**26**), then assessed for viability as described in the methods. The results are summarized in Table 6 with IC₅₀ values given in nM concentrations. Tubulysin esters **155-158** performed comparably to tubulysin M, with similar potencies across of the cell lines. The most hindered tubulysin O-acyl compound (**159**) displayed significantly impaired potency relative to tubulysin M, with potency losses ranging from 5-47-fold relative to tubulysin M.

**Table 6. In vitro Cytotoxicity of other Tubulysin (O-acyl) compounds as free drugs in comparison to Tubulysin M**

| compd | Description | **Karpas299,** ALCL | **L540cy,** HL | **L428,** MDR+ HL | **HL60,** AML | **HL60/RV,** MDR+ AML |
|---|---|---|---|---|---|---|
| **26** | TubM | 0.04 | 0.08 | 0.04 | 0.15 | 1 |
| **155** | Tub(propionate) | 0.02 | 0.06 | 0.04 | 0.1 | 1 |
| **156** | Tub(isobutyrate) | 0.02 | 0.1 | 0.12 | 0.1 | 6 |
| **157** | Tub(butyrate) | 0.011 | 0.04 | 0.05 | 0.08 | 3 |
| **158** | Tub(isovalerate) | 0.02 | 0.06 | 0.11 | 0.04 | 6 |
| **159** | Tub(3,3-dimethybutyrate) | 0.18 | 0.7 | 0.93 | 0.7 | 47 |

### Example 51. In vitro Cytotoxicity of other Ligand Drug Conjugates having quaternized Tubulysin (O-ether) Drug Units targeting CD30⁺ lymphoma cells

Anti-CD30 Ligand Drug Conjugates bearing quaternized tubulysin ethers (i.e., Tub(O-ether compounds) were compared with the corresponding tubulysin M conjugate each having 8 quaternized Drug Units/antibody Ligand Unit and having conditional glucuronidase Linker Units. Cells were treated with the cAC10 (anti-CD30) conjugates for 96 h and then assessed for viability. The IC₅₀s are shown in Table 7 given in ng/mL concentration units. The conjugate prepared from tubulysin ethyl ether Drug Linker **79** was consistently more potent than the methyl (**78**) or propyl (**80**) analogs. With the exception of L428, the conjugate prepared from the tubulysin ethyl ether Drug-Linker **79** performed similarly to that prepared from the tubulysin M Drug Drug-Linker **104.** All LDCs were inactive (no effect at 1000 ng/ml) on a CD30-negative Ramos NHL cell line, indicating a high degree of immunological specificity.

**Table 7. In vitro Cytotoxicity of Ligand Drug Conjugates targeting CD30⁺ lymphoma cells having quaternized Tubulysin (O-ether) Drug Units and conditional glucuronidase release Linker units**

| **Drug-Linker** | Drug-Linker Description | **Karpas299** ALCL | **L540cy** HL | **L428** MDR+ HL | **DEL** ALCL | **DEL/BVR** MDR+ ALCL |
|---|---|---|---|---|---|---|
| **104** | mDPR-gluc4-TubM | 0.6 | 4 | 0.5 | 2 | 3 |
| **78** | mDPR-gluc4-Tub(OMe) | 3 | 10 | >1000 | 2 | 20 |
| **79** | mDPR-gluc4-Tub(OEt) | 1 | 5 | 4 | 2 | 5 |
| **80** | mDPR-gluc4-Tub(OPr) | 2 | 6 | 117 | 2 | 10 |

### Example 52. In vitro Cytotoxicity of Ligand Drug Conjugates having quaternized Tubulysin M Drug units targeting CD30⁺ lymphoma cells or CD33⁺ Leukemia cells.

The tubulysin quaternary amine Drug Linker compound MC-Val-Ala-PAB4-TubM **(30)** was conjugated to chimeric AC10 and humanized 2H12, which are anti-CD30 and anti-CD33 antibodies, respectively, at 8 quaternized Drugs units per antibody. The conjugates were tested on a panel of CD30-positive and CD33-positive lymphoma and acute myeloid leukemia (AML) cell lines, respectively. The results (in ng/mL) for the CD30-positive cell lines are shown in Table 8. The CD30-binding conjugate, cAC10-**30**, was highly potent across all of the cell lines; whereas, the non-binding control h2H12-**30** in this context was inactive, with IC₅₀s > 1000 ng/ml.

**Table 8. In vitro Cytotoxicity of Ligand Drug Conjugates targeting CD30⁺ Lymphoma cells having quaternized Tubulysin M Drug units with varying Linker unit hydrophobicity.**

| **ADC** | **Karpas299, ALCL** CD30+ CD33- | **L-428, HL** CD30+ CD33- | **L540cy, HL** CD30+ CD33- | **KMH-2, HL** CD30+ CD33- | **DEL, ALCL** CD30+ CD33- |
|---|---|---|---|---|---|
| cAC10-**30** | 0.07 | 0.2 | 1 | 0.3 | 0.3 |
| h2H12-**30** | >1000 | >1000 | >1000 | >1000 | >1000 |

The results for the CD33-expressing AML cell line panel are shown in Table 9. The CD33-binding conjugate, h2H12-**30**, was potent across all of the cell lines with IC50s ranging from 0.9 to 18 ng/ml. The conjugate was inactive on CD33-negative L540cy, indicating a high degree of immunological specificity for this ADC.

**Table 9. In vitro Cytotoxicity of Ligand Drug Conjugates targeting CD33⁺ Leukemia cells having quaternized Tubulysin M Drug units with varying Linker unit hydrophobicity.**

| **ADC** | **HL60cy, AML** CD33+ | **KG-1, AML** CD33+ | **TF-1a, AML** CD33+ | **THP-1, AML** CD33+ | **HNT-34, AML** CD33+ | **HEL9217, AML** CD33+ | **L540cy, HL** CD33- |
|---|---|---|---|---|---|---|---|
| h2H12-**30** | 2 | 18 | 2 | 0.9 | 4 | 1 | >1000 |

### Example 53. In vitro Cytotoxicity of Ligand Drug Conjugates targeting CD30⁺ lymphoma cells having quaternized Tubulysin MDrug units with varying Linker unit hydrophobicity.

Several hydrophilic linker Drug Linker compounds having quaternized tubulysin M were prepared and the Ligand Drug Conjugates targeting CD30+ cells derived therefrom at 8 drug unit/ cAC10 antibody Ligand Unit were evaluated *in vitro*; the results are shown in Table 10 (IC₅₀ values given in ng/mL units). The data indicate the conjugate having tubulysin M linked via a more hydrophilic -Val-Glu- dipeptide prepared from Drug Linker **113,** which provides for cathepsin conditional release of free Tubulysin M, and the conjugate prepared from Drug Linker **104** incorporating a hydrophilic Glucuronide Unit, which provide for conditional glucuronidase release of free Tubulysin M, are equipotent to the -Val-Ala- control conjugate prepared from Drug-Linker **32** for cathepsin conditional free drug release. All conjugates displayed a high degree of immunological specificity, with IC₅₀s > 1000 ng/ml on antigen-negative Hep3B hepatocellular carcinoma cells.

**Table 10. In vitro Cytotoxicity of Ligand Drug Conjugates having quaternized Tubulysin M targeting CD30⁺ lymphoma cells with varying hydrophobicity in the Linker Unit**

| **Drug-Linker** | Linker description | **L540cy,** HL | **L428,** MDR+ HL | **DEL,** ALCL | **DEL/BVR,** MDR+ ALCL | **Hep3B,** CD30-HCC |
|---|---|---|---|---|---|---|
| **32** | mDPR-ValAlaPAB4 | 2 | 1 | 0.3 | 4 | >1000 |
| **104** | mDPR-glucQ | 1 | 0.4 | 0.3 | 4 | >1000 |
| **113** | mDPR-ValGluPAB4 | 1 | 0.3 | 0.1 | 2 | >1000 |

### Example 54: In vitro cytotoxicities of Ligand Drug Conjugates having non-quaternized Drug units attached to Linker units through the N-terminal component of tubulysin-derived compounds

Prior to the present invention to conjugate to a tertiary amine-containing drug in which the amine nitrogen is to be used as the point of attachment required removal of an alkyl substituent from the tertiary amine. The secondary amine so obtained permits either re-alkylation with a Linker unit component to provide an entirely different tertiary amine functional group or acylation to provide an secondary amide functional group. In both approaches the release of the original parent drug is obviated. Furthermore, the active drug moiety that is released is a different tertiary amine-containing or an amide-containing compound that may have impaired biological activity compared the parent drug or is a secondary amine that may have poor release kinetics and/or impaired biological activity. To determine the outcome of applying the strategy of removing an alkyl substituent from the tertiary amine of a tubulysin to provide a point of attachment for a non-quaternized Drug unit des-methyl-tubulysin M and des-methyl-Tub(OCH₃)-OH (**170**) were prepared in accordance with the procedures of WO 2013/085925 and Example 42 (see Scheme 15), respectively. Compound **170** is analogous to that of des-methyl-tubulysin M by replacement of the acetate O-linked substituent of the tubuvaline component of desmethyl tubulysin M with the O-linked ether moiety -OCH₃. The Linker Unit having the general formula of M¹-A-, abbreviated as MC, in which M¹ is a maleimide moiety and the Stretcher unit A is an alkylene, is then attached to des-methyl-tubulysin M and des-methyl-Tub(OCH₃)-OH through an amide functional group to provide the Drug-Linker compounds **166** and **172,** respectively. When incorporated into Ligand Drug Conjugates the drug linker moieties of those two compounds will non-conditionally release an active drug moiety that either retains a Linker unit moiety (i.e., is an amide-containing compound) or a secondary amine as des-methyl Tubulysin M or desmethyl-Tub(OCH₃)-OH, which results from non-specific cleavage of the amide bond. As the latter type of release is expected to be slow, Drug-Linker compounds such as Compounds **166** and **172** are characterized as effectively non-cleavable.

Anti-CD30 cAC10 conjugates were prepared from Drug-Linker compounds **166** and **172,** which were obtained from secondary amine-containing tubulysins, at 8-drugs/mAb and compared to the tertiary amine-containing tubulysins tubulysin M and tubulysin (OCH₃)-OH, which were conjugated via the quaternary amine strategy of the present invention. The results as shown in Table 11 (IC₅₀ values in ng/mL units) indicate that cAC10 antibody Ligand Drug conjugates from Drug-Linker compounds **166** and **172** relying upon the strategy of N-dealkylation of a tertiary amine-containing compound to provide for non-quaternary amine attachment of a tubulysin Drug unit were inactive on lymphoma cell lines in comparison to the quaternary amine-linked comparators prepared from Drug-Linker compounds **104** (4° TubM) and **78** (4°Tub(OCH₃)-OH), which releases free drug that retains the tertiary amine functionality of the parent drug.

**Table 11. Comparison of Ligand Drug Conjugates from Non-Quaternized and quaternized Tubulysin Drug Units**

| **Drug-Linker** | Drug-Linker Description | Linker type | **Karpas299,** ALCL | **L540cy,** HL | **DEL,** ALCL |
|---|---|---|---|---|---|
| **104** | mDPR-gluc4-TubM | 4° amine | 0.6 | 4 | 2 |
| **166** | MC-TubM | Non-cleavable | >1000 | >1000 | >1000 |
| **78** | mDPR-gluc4-Tub(OMe) | 4° amine | 3 | 10 | 2 |
| **172** | MC-Tub(OMe) | Non-cleavable | >1000 | >1000 | >1000 |

### Example 55. In vivo cytotoxicity of a Ligand Drug Conjugate having a quaternized auristatin as the D⁺ moiety.

A L540cy Hodgkin lymphoma xenograft model was treated with a single dose i.p. of the composition of Table 4 having cAC10 as the targeting moiety and vcPAB4-AE as the linker-quaternized drug unit for which the predominate antibody Ligand Drug Conjugate has 4 D⁺ units per antibody (i.e., p' is 4). Comparison of that conjugate was made to another cAC10 conjugate, whose linker-drug construct is designated as vcPABC-MMAE (Figure 1). In that LDC a methyl substituent of the tertiary amine of auristatin E (AE) was removed to provide monomethyl auristatin E (MMAE) that was then conjugated through its secondary amine via a carbamate to a PAB self-immolating moiety activated by cathepsin cleavage of the same valine-citrulline cleavable unit. The results of that comparison are shown in Figure 1. In that Figure cAC10-**C** is the carbamate cAC10-vcPAB-MMAE conjugate, cAC10-vcPAB4-AE designated cAC10-**14** is the quaternized AE conjugate, and h00-**C** refers to h00-vcPAB-MMAE and h00-**14** to h00-vcPAB4-AE, wherein h00 is a universal nonbinding antibody used as a non-targeting control Ligand Unit. The results of Figure 1 demonstrates that the *in-vitro* cytotoxicity of the cAC10 quaternized AE conjugate, which targets the CD30⁺ Hodgkin lymphoma cells and releases free tertiary amine-containing drug (AE), translates *in vivo* in a dose-dependent fashion and is superior to the conjugate in which the corresponding secondary amine-containing drug (MMAE) is released. Results from administration of the corresponding conjugates that target an antigen not present on L540cy cells (monoclonal Ab is h00) are similar to sham treatment. Notably, at the higher dose (2 mg/Kg) tested the vcPAB4-AE antibody Ligand Drug Conjugate caused complete ablation of the tumor, which did not rebound during the remainder of the study.

Comparison was also made between the CD30 targeted antibody Ligand drug Conjugate incorporating a quaternized AE drug unit and a Glucuronide Unit for conditional auristatin drug release by a glucuronidase and a similar conjugate in which the non-quaternized MMAE drug unit is attached to the Glucuronide unit via a carbamate. Thus, the quaternized gluc4-AE antibody Ligand Drug Conjugate composition of Table 4, again with the predominate LDC having 4 D⁺ units per cAC10 antibody, with the corresponding gluc-MMAE composition. The gluc4-AE and gluc-MMAE linkers were also conjugated to h00, which as previously stated is a universal non-binding protein (and thus does not bind to L540cy Hodgkin lymphoma cells) to provide control LDCs. The results are shown in Figure 2. In that Figure 1 cAC10-glcu4-AE, designated cAC10-**8**, is the quaternized AE conjugate, cAC10-**B** is the carbamate-linked cAC10-gluc-MME conjugate, and h00-gluc4-AE, designated as h00-**8,** and h00-glu-MMAE, designated as h00-**B**, are the respective control conjugates. As with the cAC10-vcPAB4-AE conjugate, the *in vitro* result with the cAC10-gluc4-AE conjugate, which releases a free tertiaryamine containing auristatin (AE), has translated in a dose-dependent fashion *in vivo* and is superior to the corresponding conjugate (cAClO-gluc-MMAE) that releases the desmethyl version of that auristatin (MMAE). Furthermore, Figure 1 and Figure 2 shows that the *in vivo* effects of the quaternized conjugates are immunologically specific.

### Example 56. In vivo cytotoxicity of a Ligand Drug Conjugate having a quaternized tubulysin as the D⁺ moiety and peptide Cleavable unit

A cAC10 antibody Ligand Drug Conjugate, designated cAC10-**32**, having a quaternary amine-tubulysin M drug linker moiety incorporating the -Val-Ala- Cleavable unit prepared from Drug Linker compound **32** was evaluated in two CD30+ xenograft models, Karpas299 ALCL and L540cy Hodgkin lymphoma. The results are shown in Figures 5 and 6, respectively. In both experiments, tumor-bearing mice were administered a single dose i.p. of test article when the average tumor volume reached 100 mm³.

In the Karpas299 xenograft model (see Figure 5), the targeted, anti-CD30 cAC10 conjugate at 4-drugs/mAb induced a tumor growth delay following a 0.3 mg/kg dose; whereas, a higher dose of 1 mg/kg resulted in durable, complete regressions in 4 of 5 mice. In contrast, the h00 non-binding conjugate displayed no activity at the 1 mg/kg dose, indicating a high degree of immunological specificity.

Similar results were observed in the L540cy xenograft model (see Figure 6). The targeted, anti-CD30 conjugate loaded at 6-drugs/mAb induced a tumor growth delay following a 0.3 mg/kg dose; whereas, a higher dose of 1 mg/kg resulted in durable, complete regressions in 3 of 4 mice.

### Example 57. In vivo cytotoxicity of a Ligand Drug Conjugate having a quaternized tubulysin(O-ether) as the D⁺ moiety attached through a Glucuronide unit

Glucuronide-based quaternary amine drug-linkers were evaluated in the L540cy Hodgkin lymphoma xenograft model. Antibody Ligand Drug Conjugates (ADCs) prepared from Drug Linker compounds **104, 79** and **80** have quaternized tubulysin M and quaternized tubulysin ethers tubulysin (O-ethyl) and tubulysin (O-propyl) ether D⁺ drug units, respectively, that are attached by Glucuronide Units. Those ADCs, designated a cAC10-**104**, cAC10-**79** and cAC10-**80**, respectively, were also compared to cAC10-**32**, which has quaternized Tubulysin M as D+ and incorporate the dipeptide -Val-Ala- as the Cleavable unit. All conjugates were loaded at 4-drugs/mAb to minimize the effects of ADC PK. Mice bearing CD30+ L540cy xenografts were administered a single i.p. dose of test articles at 0.6 or 2 mg/kg when average tumor volume reached 100 mm³. As shown in Figure 9, all ADCs displayed significant tumor regressions through 30 days. At the lower dose level of 0.6 mg/kg of cAC 10-**32**, the tumors in 4/5 mice were increasing in size at day 36; whereas all of the mice had complete tumor regressions at that time after treatment with 0.6 mg/kg of cAC10-**104**. For antibody Ligand Drug Conjugates derived from Drug Linker compounds **79** and **80** having quaternized tubulysin ethers displayed, complete tumor regressions at both doses at study day 36 was observed.

### Example 58. In vivo cytotoxicity of a Ligand Drug Conjugate having alternative quaternized auristatins as the D⁺ moiety attached through a Glucuronide unit

Antibody Ligand drug Conjugates (ADCs) having glucuronide quaternary amine drug linker moieties were evaluated for targeted delivery of antimitotic dolastatin 10 and auristatin F and were prepared from Drug Linker compounds **177** and **189,** respectively. Those ADC are designated cAC10-**177** in Figure 11 and cAC10-**189** in Figure 10.

cAC10-**189** having a drug loading of 8 quaternized auristatin F Drug units per antibody was evaluated in the L540cy Hodgkin lymphoma xenograft model compared vs. sham treat animals at single i.p. doses of 0.5 mg/kg and 2 mg/Kg. In the same study the ADC, designated cAC 10-**78**, prepared from Drug Linker compound **78** in which the tubulysin ether Tub(OCH₃)-OH is quaternized was also tested. For cAC10-**78** the DAR was 6 quaternized tubulysin ethers per antibody. Both of the conjugates have the quaternized Drug Unit attached to identical Linker Units that incorporate a Glucuronide unit. The results are shown in Figure 10. Through day 20 all test articles showed tumor regression at both dose levels.

The antibody Ligand Drug Conjugate having quaternary amine dolastatin 10 as the D+ was assessed in the same L540cy xenograft model at single i.p. doses of 0.4 mg/kg and 0.8 mg/Kg relative to monomethyldolastatin 10, which was attached to the Glucuronide unit as through a carbamate functional group. The antibody Ligand Drug Conjugate (ADC) with quaternized dolastatin 10 prepared from Drug-Linker compound 177 having the Glucuronide unit is designated in Figure 11 as cAC10-**177**, whereas the ADC having the non-quaternized Drug unit attached to the same Glucuronide unit is designated cAC10-**183**. Both ADCs compared to respective their non-targeting controls h00-177 and h00-**183** induced durable, complete regressions at the lowest dose tested of 0.4 mg/kg, with 5/5 cures for the quaternary amine-linked dolastatin 10 (**177**) compared to 4/5 cures for carbamate-linked monomethyldolastatin 10 (**183**). At the highest dose tested (0.8 mg/kg), the non-binding h00 control bearing carbamate-linked dolastatin 10 exhibited a transient tumor regression compared to a brief tumor growth delay for the quaternary amine-linked dolastatin 10. Ultimately, both ADC constructs displayed immunological specificity.

### Example 59. Intracellular release of free tertiary amine-containing drug from a quaternized drug unit in a Ligand Drug Conjugate.

MDR cell lines that are CD71⁺ are exposed to an LDC (1 µg/mL) comprised of and antibody targeting CD71 (cOKT9) and a quaternized MDR inhibitor (Tariquidar^{™}). The LDC composition designated as cOKT9-gluc4-T was obtained from compound **36** of Scheme 6 and predominately contains LDCs having 8 D⁺ units wherein D⁺ is quaternized Tariquidar. The predominate LDC of the composition, after controlled hydrolysis of the succinimide ring system is represented by the structure of: 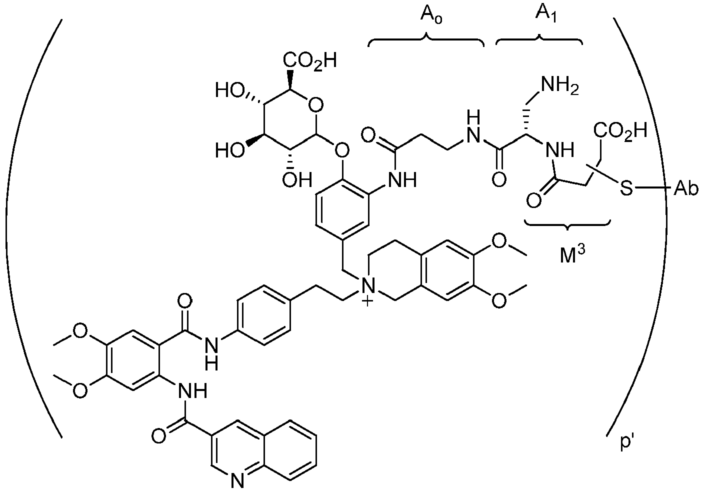 wherein the Ab-S- moiety is bonded to the carbon α or β to the M³ carboxylic acid; and p is 8.

The concentration of intracellularly delivered and retained drug released from the Ligand Drug Conjugate is shown in Table 12 and is compared to the amount of intracellular drug (i.e., pgp-bound drug) that results from treating the cells with 50 nM of free drug. With LDC treatment, the parenthetic values are extracellular concentrations of the MDR inhibitor that have leaked back out from the targeted cell while the parenthetic values for free drug treatment represent the amount of free drug that remains outside the cell.

**Table 12: Intracellular drug delivery by an Ligand Drug Conjugate having a quaternized MDR inhibitor.**

| Treatment | Intracellular drug (extracellular drug) nM | | |
|---|---|---|---|
| | TF1a | L428 | HCT-15 |
| cOKT9-glu4-T @ 1 µg/mL | 1525 (14.8) | 1100 (5.8) | 2368 (9.4) |
| Free drug @ 50 nM | 3575 (43.8) | 4846 (47.0) | 6649 (44.3) |

The results of Table 12 indicate that the MDR inhibitor Tariquidar has been efficiently delivered intracellularly into MDR cells. When analyzed in terms of pmol drug released from 10⁶ cells the cOKT9-glu4-T composition delivers 50-80% of its drug load to the targeted cells.

### Example 60. Kinetics of enzymatic drug release from a quaternized drug unit

N-acetyl cysteine (NAC) versions of the carbamate cAC10-gluc-MMAE conjugate and the quaternized cAC10-gluc4-AE Ligand Drug Conjugates described in Example 53 (i.e., cAC10 is replaced with NAC, wherein p' for the corresponding structure is 1) were subjected to 0.5 mg/mL or 1 mg/mL glucuronidase and compared to control having no glucuronidase present. The results of Figure 4 indicated that release kinetics of AE from the quaternized drug unit of the NAC-gluc4-AE conjugate is comparable for MMAE release kinetics from the NAC-gluc-MMAE conjugate, with complete release of AE occurring within 3.5-4.5 hrs depending on enzyme concentration. In Figure 4 NAC-**8** is the conjugate having a quaternized drug unit that releases free tertiary amine-containing drug (AE) and NAC-**B** is the NAC-gluc-MMAE carbamate conjugate that release and the corresponding desmethyl auristatin (MMAE).

### Example 61: Ex vivo stability of Ligand Drug Conjugates having a quaternized auristatin drug unit.

Integrity losses of Ligand Drug Conjugate compositions cAC10-vcPAB4-AE and cAC10-gluc4-AE of Example 55 after incubation in mouse and rat plasma are shown in Figure 3. In Figure 3 the gluc4-AE conjugate is designated cAC10-**8** is and the vcPAB4-AE conjugate is designated cAC10-**14**. The results indicate both types of quaternized drug-linker constructs have good stability and do not prematurely release free tertiary amine-containing drug. Quaternized tubulysin and conjugates and model systems of quaternized tarquidar and phenazine dimer conjugates in which N-acetyl cysteine is present in lieu of a targeting antibody were also subjected to the same experimental conditions. In those cases as well there was no premature release of free drug.

### Example 62. In vivo stability of Ligand Drug Conjugates having a quaternized tubulysin(O-acyl) Drug unit.

Plasma samples from the L540cy xenograft study of Example 56, Figure 6, which studied targeted cytotoxicity of ADC cAC 10-**32**, were collected from all mice 4 and 10 days post-dose (study days 11 and 17, respectively) to assess the extent of tubulysin M deacetylation. Loss of the tubuvaline acetate is known to result in the de-activation of tubulysin. For example, deacetylation of tubulysin U, forming tubulysin V, resulted in a >100-fold loss of potency (J. Med. Chem., 2009, 52, 238-240). The plasma samples from the L540cy xenograft of Example 56 at 4 and 10 days post dosing were processed to capture ADC, drug was released enzymatically, and the extent of deacetylation quantified by mass spectrometry. Four days after injection (Figure 7), the conjugated tubulysin drug warhead was >40% deacetylated across all three dose levels. At ten days post dose (Figure 8), the extent of deacetylation was >70% across all dose levels. Those finding suggest that the circulating ADC is undergoing a loss of warhead potency as a function of time, perhaps attenuating *in vivo* activity.

### Example 63: Pharmacokinetic (PK) evaluation of Ligand Drug Conjugates having a quaternized tubulysin as the Drug unit D⁺.

Pharmacokinetic (PK) experiments were performed using radiolabeled antibody or ADC. PK test articles were radiolabeled using the following procedure. To a solution of antibody or ADC in PBS supplemented with an additional 50 mM potassium phosphate (pH 8.0) and 50 mM sodium chloride was added 55 µCi N10 succinimidyl propionate, [propionate-2,3-3H]- (Moravek Biochemicals, Cat. No.: MT 919, 80 Ci/mmol, 1 mCi/mL, 9:1 hexane:ethyl acetate solution) per mg of antibody or ADC. The resulting mixture was vortexed and left at room temperature for 2 hours. The mixture was centrifuged at 4,000g for 5 minutes and the lower aqueous layer was removed and split into Amicon Ultra-15 Centrifugal Filter Units (Millipore, Cat. No.: UFC903024, 30 kDa MWCO). Unconjugated radioactivity was removed by 4 rounds of dilution and centrifugation at 4,000g. The resulting products were filtered through sterile 0.22 µm Ultrafree-MC Centrifugal Filter Units (Millipore, Cat. No.: UFC30GV0S) and the final antibody or ADC concentration was measured spectrophotometrically. The specific activity (µCi/mg) of each product was determined by liquid scintillation counting.

The pharmacokinetic properties of the unconjugated antibody or ADC were examined in several experiments. In each experiment, 1 mg of radiolabeled antibody or ADC per kg of animal weight were injected via the tail vein. Each test article was dosed once in replicate animals. Blood was drawn into K2EDTA tubes via the saphenous vein or by cardiac puncture for terminal bleeds at various time points. Plasma was isolated by centrifugation for 10 minutes at 10,000g. A 10-20 µl of sample of plasma from each time point was added to 4 ml Ecoscint-A liquid scintillation cocktail (National Diagnostics) and the total radioactivity was measured by liquid scintillation counting. The resulting disintegrations per minute values were converted to µCi and the specific activity of the radiolabeled test articles was used to calculate the concentration of antibody or ADC remaining in the plasma at each time point.

Figure 12 shows the exposure profiles for conjugates prepared from the Drug-Linker Compound mDPR-Val-Ala-PAB4-TubM **(32)** and the hydrophilic linker analogs **113** described in Example 51. The ADC with humanized IgG Ligand unit and DAR 4 of quaternized Tubulysin M prepared from Drug-Linker compound **32,** which has a -Val-Ala- dipeptide Cleavable unit and is designated as hIgG-**32**(4), had a clearance profile identical to that of unmodified antibody; however, the ADC at DAR of 8, designated as hIgG-**32**(8) the ADC was cleared from circulation much more rapidly. The corresponding ADC with DAR 8 prepared from Drug Linker compound **113** designated hIgG-**113**(8) in which the alanine residue in the Cleavable unit is substituted with glutamate did not result in an increase in exposure. With hIgG ADC designated hIgG-**104**(8) prepared from Drug Linker Compound **104** in which the hydrophobic dipeptide Cleavable unit of hIgG-**32**(8) is replaced with the more polar Glucuronide Unit a slight improvement in clearance was observed

Figure 13 contains the PK exposures for DAR 8 h00 conjugates having the glucuronide quaternary amine-linked tubulysin ether compound Tub(O-Pr)-OH of prepared from Drug Linker compound (**80**). That h00 antibody Ligand Drug Conjugate bearing **8** copies of the drug linker moiety derived from mDPR-gluc4-Tub(OPr)-OH **80** was cleared from circulation much more rapidly than the unmodified antibody.

Figure 14 shows the clearance properties of DAR 8 h00 antibody Ligand Drug Conjugates (h00 ADCs) having drug-linker moieties comprised of a glucuronide Unit and auristatin E or dolastatin 10 cytotoxin as quaternized D⁺ Drug Units and the equivalent Conjugates having non-quaternized Drug unit carbamate-linked Drug Units in which a methyl group has been removed from AE and dolastatin 10 (i.e., conjugates of non-quaternized Drug Units MMAE and monomethyl-dolastatin 10). The h00 ADC with glucuronide quaternary amine-auristatin E drug linker moiety prepared from Drug Linker compound **8** provided PK properties identical to that of unmodified antibody through 7 days. However, the h00 ADC conjugate having the non-quaternized carbamate-linked monomethylauristatin E designated h00-**B** caused accelerated ADC clearance. In contrast, the h00-ADC having quaternized dolastatin 10 prepared from Drug Linker compound **177** and designated as h00-**177**, and the h00-ADC having non-quaternized monomethydolastatin 10 attached to the Linker Unit through a carbamate functional group, which is prepared from the carbamate Drug Linker compound **183** and designated h00-**183**, were cleared more rapidly than naked antibody and had similar exposures.

### Example 64: In vitro comparison between in vitro cytotoxicity of tubulysin M-hydrazide and tubulysin M Drug hydrazide as free drugs.

A C-terminal strategy to conjugate tubulysins as the hydrazide analogs has been described (Cancer Res., 2008, 68, 9839-9844) previously for tubulysin B. This approach is an alternative to the quaternary amine strategy described herein and the non-cleavable N-terminal conjugation strategy of Example 54 , and involves derivitization of the tubulysin C-terminal component (*cf.* Example 47). The tubulysin M-hydrazide (**190**) was evaluated *in vitro* as free drug relative to unmodified tubulysin M (**26**) across a panel of cell lines; the cells were treated for 96 hours and the results are shown in Table 11 (IC₅₀ values in nM concentration).

**Table 13. Comparison of In vitro Cytotoxicity of between Tubulysin M and Tubulysin M hydrazide as free drugs**

| **free drug** | Description | **L428,** MDR+ HL | **786-O,** MDR+ RCC | **HL60,** AML | **HEL9217,** MDR+ AML |
|---|---|---|---|---|---|
| **26** | TubM | 0.04 nM | 0.3 | 0.2 | 0.4 |
| **190** | TubM-hydrazide | 1 | 4 | 0.8 | 4 |

The tubulysin M-hydrazide was consistently less potent than the parent free drug, tubulysin M, in all the cell lines tested. The loss in potency of the hydrazide derivative ranged from 4- to 25-fold across the four cell lines.

### Example 63. In vitro comparison of tubulysin M as a non-quaternized Val-Cit-PABN carbamate hydrazide drug linker moiety compared to quaternized Val-Ala-PAB4 tubulysin M.

The Val-Ala quaternary amine-tubulysin M Drug Linker compound MC-vaPAB4-TubM (**30**) and the carbamate-linked tubulysin M-hydrazide Drug Linker compound mDPR-Val-Cit-PABN (**192**) were conjugated to cOKT-9, an anti-transferrin receptor IgG. Briefly, cOKT-9 was fully reduced with TCEP to reveal 8 conjugatable cysteine residues. The resulting thiols were alkylated with the maleimido-containing Drug Linker compounds **30** and **192,** added in excess. The cOKT-9 conjugates were found to be loaded at 8 and 6.7 drugs/mAb for linkers **30** and **192,** respectively. A panel of cancer cell lines were treated with the resulting conjugates for 96 hours and assessed for viability. The results are shown in Table 1 with IC₅₀s given in ng/ml units.

**Table 14. In vitro Cytotoxicity of a quaternized tubulysin Ligand Drug Conjugate targeting transferrin receptor in comparison to non-quaternized tubulysin Ligand Drug Conjugate having attachment to the C-terminal component.**

| **drug-linker compound** | DAR | drug-linker description | **L540cy,** HL | **L428,** MDR+ HL | **KM-H2,** MDR+ HL | **HL60,** AML | **HCT-116,** CRC |
|---|---|---|---|---|---|---|---|
| **30** | 8 | MC-vaPAB4-TubM | 1 | 0.4 | <0.26 | 6 | 1 |
| **192** | 6.7 | mDPR-vcPABN-TubM (hydrazide) | 117 | 284 | 56 | 85 | 302 |

The conjugate bearing the TubM-hydrazide (**192**) was significantly less potent than the quaternary amine-linked TubM (**30**) comparator. The derivitized tubulysin ranged from 1-3 logs less potent than the parent tubulysin M.

### Example 65: In vitro evaluation of auristatin F linked via glucuronide quaternary-amine linker.

The cOKT9 antibody Ligand Drug Conjugate (ADC) prepared from the glucuronide quaternary amine-auristatin F Drug Linker compound **189** was prepared as described and conjugated at 8-drugs/Ab to cOKT-9, an anti-transferrin receptor antibody. The resulting ADC was tested on a panel of cancer cell lines for comparison to the corresponding ADC prepared from the carbamate-Drug Linker compound MC-Val-Cit-PABC-MMAF **193** (Bioconjugate Chem., 2006, 17, 114-124). The results are shown in Table 13.

**Table 15. Cytotoxicity of a quaternized auristatin F Ligand Drug Conjugate targeting transferrin receptor in comparison to a corresponding non-quaternized auristatin Ligand Drug Conjugate**

| **drug-linker compd** | DAR | drug-linker description | **L540cy,** HL | **L428,** MDR+ HL | **786-O,** MDR+ RCC | **HL60,** AML | **HL60/RV,** MDR+ AML |
|---|---|---|---|---|---|---|---|
| **193** | 8 | MC-ValCitPABC-MMAF | 2 | 0.2 | 2 | 33 | 15 |
| **189** | 8 | mDPR-gluc4-AF | 3 | 0.3 | 3 | 98 | 38 |

The conjugate having auristatin F as a quaternary-amine Drug Unit prepared from Drug Linker Compound **189,** which has a Glucuronide Unit and the self-stabilizing LSS precursor mDPR, provides similar cytotoxicity as shown in Table 14 (IC₅₀ values in ng/mL units) to an ADC prepared from the Val-Cit PAB carbamate (vcPABC)-MMAF Drug-Linker compound **193** on most of the cell lines. In contrast to Compound **189** that Drug Linker compound has a Linker unit that does not contain a L_{SS} precursor and is comprised of a dipeptide cleavable unit. In the AML cell lines HL60 and HL60/RV there was a modest decrease (2- to 3-fold) in potency observed relative to the Val-Cit-PABC carbamate control obtained from **193.**

### Example 66: Cytotoxicity of quaternized Phenazine dimer Ligand Drug Conjugates targeting cancer cells.

The phenazine dimer quaternary amine Drug-Linker Compound **19** was conjugated to chimeric AC10 and humanized 1F6 antibodies,which are anti-CD30 and anti-CD70 antibodies, respectively, at DAR of 4-drugs per antibody. The conjugates were tested on two CD30+ lymphoma cell lines, Karpas299 and L540cy, and two CD70-positive renal cell carcinoma cell lines, 786-O and Caki-1. The data are shown in Table 16 (IC₅₀ values in ng/mL units).

**Table 16. Cytotoxicity of quaternized Phenazine dimer Ligand Drug Conjugates targeting CD30⁺ or CD70⁺ cancer cells**

| **ADC** | **Karpas299, ALCL** CD30+ CD70- | **L540cy, HL** CD30+ CD70+ | **786-O, RCC** CD30-CD70+ | **Caki-1, RCC** CD30-CD70+ |
|---|---|---|---|---|
| cAC10-**19** | 18 | >10,000 | 530 | 2200 |
| h1F6-**19** | 6400 | >10,000 | 22 | 68 |

The anti-CD30 binding conjugate, cAC10-**19**, was potent on the Karpas299 ALCL line; whereas its control, h1F6-**19**, which does not recognized the CD30 antigen, was inactive, indicating a high degree of immunological specificity. Both the cAC10-**19** and h1F6-**19** conjugates were inactive on CD30⁺/CD70⁺ L540cy cells. On the CD30 negative /CD70 positive RCC cells the CD70 antigen-specific conjugate, h1F6-**19**, was significantly more potent than its control conjugate cAC10-**19**, which does not recognize the CD70 antigen.

## Claims

1. A Ligand Drug Conjugate (LDC) composition for use in treating a cancer or an autoimmune disease, wherein the LDC composition is represented by the structure of Formula 1: 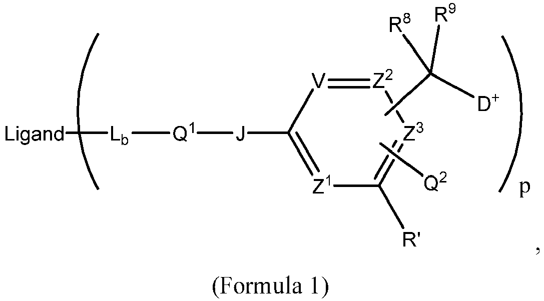 wherein
"Ligand" is a Ligand Unit (L), wherein L is capable of selectively binding to a target moiety, and L is an antibody or fragment thereof to define an antibody drug conjugate (ADC), wherein the target moiety is an antigen capable of selectively binding to the ADC, and wherein the antigen is an extracellularly accessible cell-surface protein, glycoprotein or carbohydrate displayed preferentially by abnormal cells in comparison to normal cells, wherein the abnormal and normal cells are cells in a mammal, wherein the abnormal cells are hyper-proliferating cells;
L_{b} is a primary linker;
Q¹ is Aₐ-W_{w}, wherein A is an optional Stretcher unit so that subscript a is 0 when A is absent or 1 when A is present and comprises two, three or four subunits;
Q² is W'_{w'}-E-, wherein Q², when present, is bonded to V, Z¹, Z², or Z³;
W_{w} and W'_{w'} are cleavable units, wherein
W_{w} of Q¹ is capable of selective cleavage by an intracellular or regulatory protease in comparison to serum proteases, or by glutathione through disulfide exchange, or is more reactive to hydrolysis under more acidic conditions present in lysosomes in comparison to physiological pH of serum,
W'_{w'}-E of Q² provides a glycosidic bond cleavable by a glycosidase located intracellularly, and
subscript w is 0 or 1 so that W_{w} is absent when w is 0 or W_{w} is present when w is 1, and subscript w' is 0 or 1, wherein W'w-E is absent when w' is 0 or W'_{w'}-E is present when w' is 1, and wherein w + w' is 1 so that one and only one of W_{w}, W'_{w'}-E is present;
V, Z¹, Z², and Z³ are =N- or =C(R²⁴)-, wherein R²⁴ is hydrogen, optionally substituted alkyl, alkenyl or alkynyl, or halogen, -NO₂, -CN, other electron withdrawing group, an electron donating group, -Q², or -C(R⁸)(R⁹)-D⁺, wherein at least one of V, Z¹, Z² and Z³ is =C(R²⁴)- when w is 1, and at least two of V, Z¹, Z², and Z³ are =C(R²⁴)- when w' is 1;
provided that when w is 1, Q² is absent, one and only one R²⁴ is -C(R⁸)(R⁹)-D⁺ , so that C(R⁸)(R⁹)-D⁺ is bonded to one of V, Z¹, Z², Z³ when that variable group is =C(R²⁴)-, and the Q¹-J- and -C(R⁸)(R⁹)-D⁺ substituents are *ortho* or *para* to each other,
provided that when w' is 1, one and only one R²⁴ is -C(R⁸)(R⁹)-D⁺, so that -C(R⁸)(R⁹)-D⁺ is bonded to one of V, Z¹, Z², Z³ when that variable group is =C(R²⁴)-, and one and only one R²⁴ is Q² so that Q² is bonded to another one of V, Z¹, Z², Z³ when that variable group is =C(R²⁴)-, and the Q² and -C(R⁸)(R⁹)-D⁺ substituents are *ortho* or *para* to each other;
R⁸ and R⁹ independently are hydrogen, or optionally substituted alkyl, alkenyl, alkynyl, aryl, or heteroaryl;
R' is hydrogen, halogen, -NO₂, -CN, other electron withdrawing group, or an electron donating group;
E and J independently are -O-, -S-, or -N(R³³)-, wherein R³³ is hydrogen or optionally substituted alkyl;
D⁺ represents a structure of a quaternized tertiary amine-containing drug; and
subscript p is an average drug loading having a number ranging from 1 to 24; and
wherein said cleavage by an intracellular or regulatory protease, by glutathione through disulfide exchange, by acid hydrolysis, or by a glycosidase results in release of tertiary amine containing drug (D) from a Ligand Drug Conjugate compound of the composition.

2. The composition for use of claim 1, wherein the structure of the composition is represented by the structure of Formula 2A or Formula 2B:

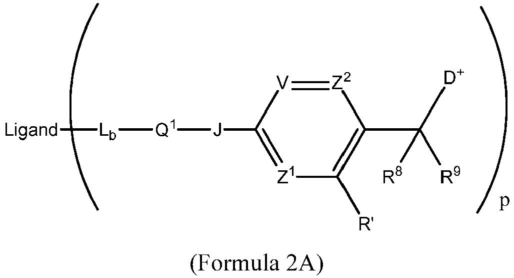

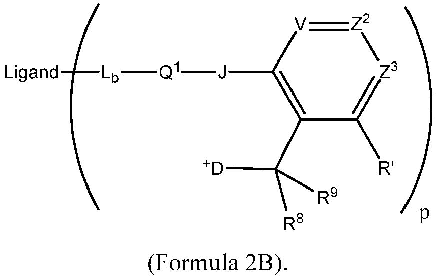

3. The composition for use of claim 1, wherein the structure of the composition is represented by the structure of Formula 3A, Formula 3B, Formula 3C, Formula 3D, Formula 3E, or Formula 3F:

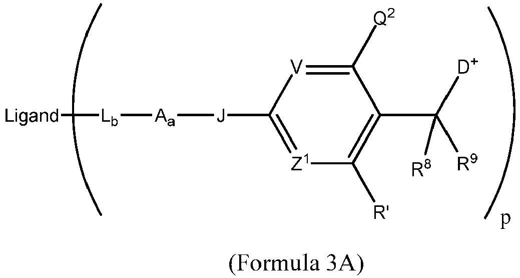

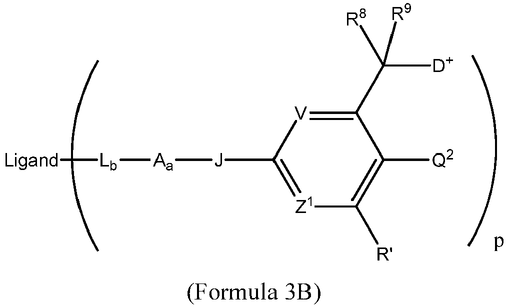

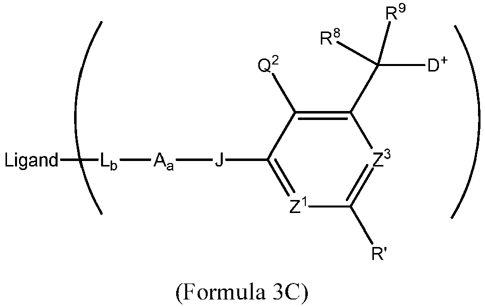

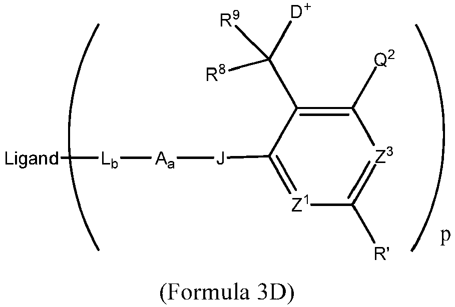

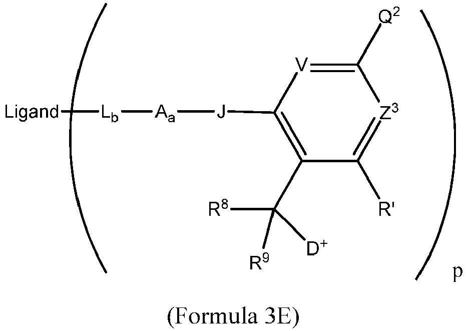

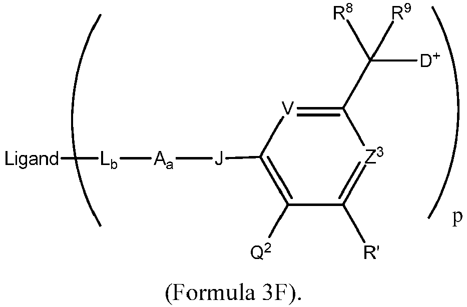

4. The composition for use of claim 2, wherein W of Q¹ comprises a peptide moiety having a peptide bond to J that is selectively cleavable by an intracellular or regulatory protease in comparison to serum proteases, wherein action of the regulatory protease on W causes release of tertiary amine-containing drug (D) from a Ligand Drug Conjugate compound of the composition, particularly wherein the peptide moiety of W comprises a dipeptide moiety having the structure of Formula 6: 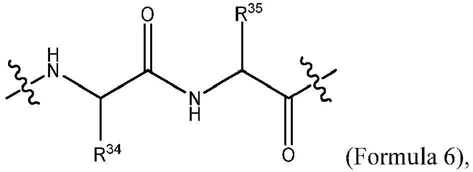
wherein R³⁴ is benzyl, methyl, isopropyl, isobutyl, sec-butyl, -CH(OH)CH₃ or has the structure of 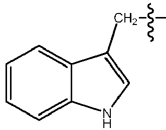 and R³⁵ is methyl, -(CH₂)₄-NH₂, -(CH₂)₃NH(C=O)NH₂, -(CH₂)₃NH(C=NH)NH₂, or -(CH₂)₂CO₂H,
wherein the wavy bond to the dipeptide's C-terminus indicates covalent bonding to J of the arylene moiety of Formula 2A or 2B and the wavy bond to the dipeptide's N-terminus indicates covalent bonding to the remainder of W, if any such remainder is present, or to A, or a subunit thereof, when a is 1 or to L_{b} when subscript a is 0, and wherein the dipeptide's bond to J is cleavable by an intracellular or regulatory protease,
particularly wherein the structure of the composition is represented by the structure of Formula 9: 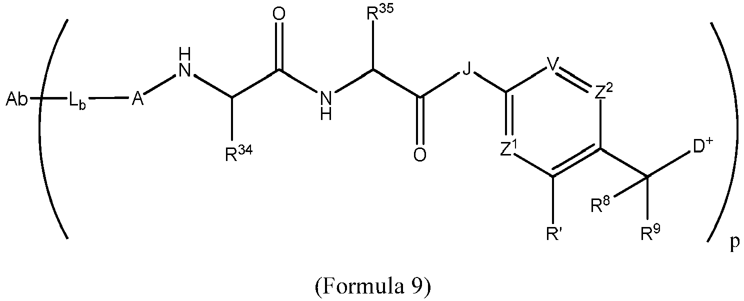
wherein Ab is an antibody Ligand Unit;
R' is hydrogen or an electron donating group;
R⁸ is hydrogen;
R⁹ is hydrogen, optionally substituted C₁-C₆ alkyl, or optionally substituted phenyl;
R³⁴ is methyl, isopropyl, or -CH(OH)CH₃;
R³⁵ is methyl, -(CH₂)₃NH(C=O)NH₂, or -(CH₂)₂CO₂H;
J is -N(R³³)-, wherein R³³ is hydrogen or methyl; and
V and Z¹ independently are =CH- or =N-,
particularly wherein the structure of the composition is represented by the structure of Formula 10: 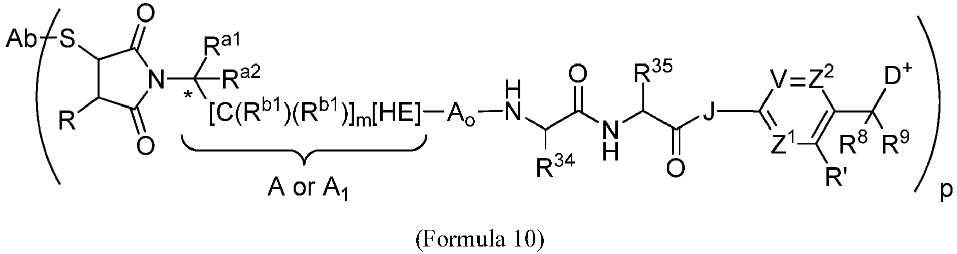 wherein
S is a sulfur atom of an antibody Ligand Unit (Ab-);
the asterisk (*) designates chirality or absence thereof at the indicated carbon;
Aₒ is an optional subunit of A, wherein -[C(R^{b1})(R^{b1})]ₘ-[HE]- is A when Aₒ is absent and is A₁ when Aₒ is present so A becomes -A₁-Aₒ-;
R is -H;
R^{a1} is -H or a Basic Unit (BU), wherein BU has the structure of -CH₂-N(R²²)(R²³), wherein R²² and R²³ independently are hydrogen, methyl, or ethyl or both together with the nitrogen atom to which they are attached comprise a 5- or 6-membered heterocycloalkyl;
R^{a2} is hydrogen;
subscript m is an integer ranging from 0 to 5 when HE is present or from 1 to 5 when HE is absent;
each R^{b1} independently is hydrogen or optionally substituted C₁-C₆ alkyl;
HE is absent or is -C(=O)-;
R³⁴ is methyl, isopropyl, or -CH(OH)CH₃;
R³⁵ is -(CH₂)₃NH(C=O)NH₂, or -(CH₂)₂CO₂H;
J is -NH-;
V, Z¹, and Z² are =CH-;
R' is hydrogen or an electron donating group;
R⁸ is hydrogen; and
R⁹ is hydrogen or methyl.

5. The composition for use of claim 3, wherein W' of Q² is a glycoside-bonded carbohydrate, wherein the glycoside bond W'w-E of Q² provides for a cleavage site for a glycosidase located intracellularly wherein action of the glycosidase on W'_{w'}-E causes release of tertiary amine-containing drug (D) from a Ligand Drug Conjugate compound of the composition,
particularly wherein W' in Q² is a carbohydrate moiety that is glycoside-bonded to E, wherein the glycosidic bond is cleavable by a glycosidase located intracellularly, and wherein W'_{w'}-E has the structure of Formula 7: 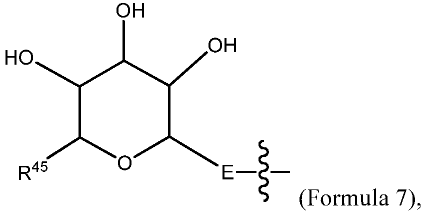
wherein the wavy line represents E bonded to one of V, Z¹, Z², or Z³ when that variable group is =C(R²⁴), wherein E is -O-, -S-, or -N(R³³)-, wherein R³³ is hydrogen or methyl; and
wherein R⁴⁵ is -CH₂OH or -CO₂H, particularly wherein the structure of the composition is represented by the structure of Formula 11: 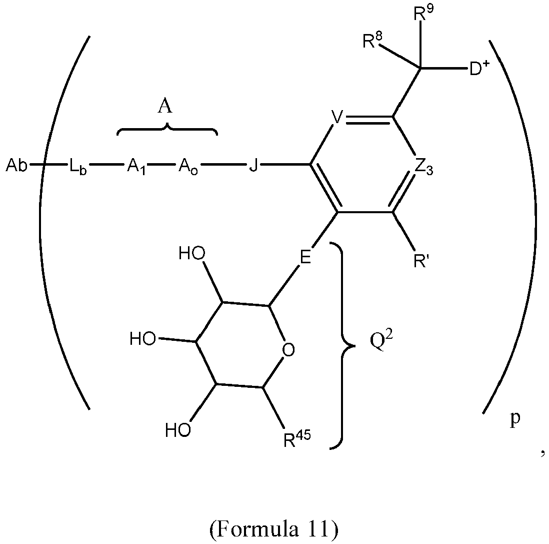
wherein
Ab is an antibody Ligand Unit;
A₁ and Aₒ are independently selected subunits of A, wherein Aₒ is an optional subunit of A so that A₁ becomes A when Aₒ is absent and A is -A₁-Aₒ- when Aₒ is present;
E is -O- or -NH-;
J is -N(R³³)-, wherein R³³ is hydrogen or methyl;
V and Z³ independently are =CH- or =N-;
R' is hydrogen or an electron withdrawing group;
R⁸ is hydrogen;
R⁹ is hydrogen, optionally substituted C₁-C₆ alkyl or optionally substituted phenyl;
R⁴⁵ is -CO₂H; and
subscript p is a number ranging from 1 to 8,
particularly wherein the structure of the composition is represented by the structure of Formula 12: 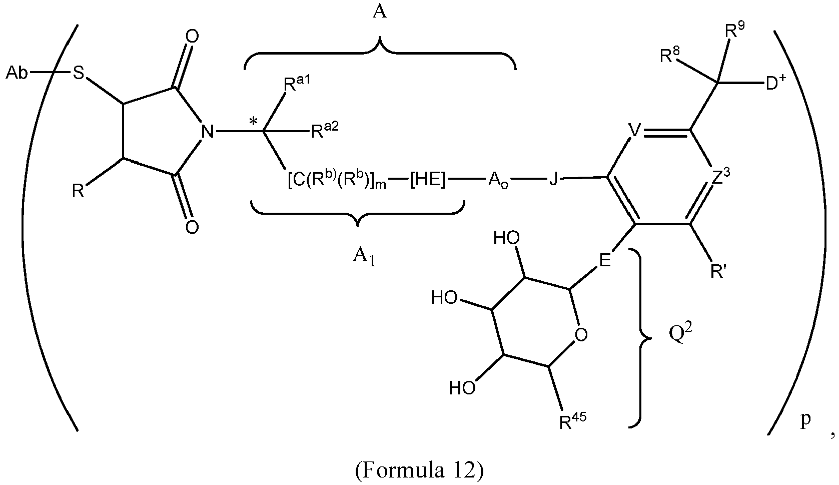 wherein
S is a sulfur atom of antibody Ligand Unit Ab;
the asterisk (*) designates chirality or absence thereof at the indicated carbon, that is predominantly in the same absolute configuration as the alpha carbon of an **L**-amino acid when that indicated carbon has chirality;
A₁ and Aₒ are independently selected subunits of A, wherein Aₒ is an optional subunit of A, wherein -[C(R^{b1})(R^{b1})]ₘ-[HE]- is A when Aₒ is absent and is A₁ when Aₒ is present so that A becomes A₁-Aₒ; wherein Aₒ when present corresponds in structure to an amine-containing acid bonded to J through the C-terminal carbonyl of the amine-containing acid;
R is hydrogen;
R' is hydrogen or an electron withdrawing group;
R^{a1} is hydrogen or R^{a1} is a Basic Unit (BU) that participates in base-assisted hydrolysis of the succinimide ring, wherein BU has the structure of -CH₂-N(R²²)(R²³), or an acid addition salt thereof, wherein R²² and R²³ independently are hydrogen, methyl or ethyl or both together with the nitrogen atom to which they are attached comprise a 5- or 6-membered heterocycloalkyl;
R^{a2} is hydrogen;
subscript m is an integer ranging from 0 to 5 when HE is present or 1 to 5 when HE is absent;
each R^{b1} independently is hydrogen or optionally substituted C₁-C₆ alkyl;
HE is absent or is -C(=O)-;
R⁴⁵ is -CO₂H;
E is -O-;
J is -NH-;
V and Z³ are =CH-;
R⁸ is hydrogen;
R⁹ is hydrogen or methyl; and
subscript p is a number ranging from 1 to 8,
or wherein Aₒ, when present, has the structure of Formula 13 or Formula 14: 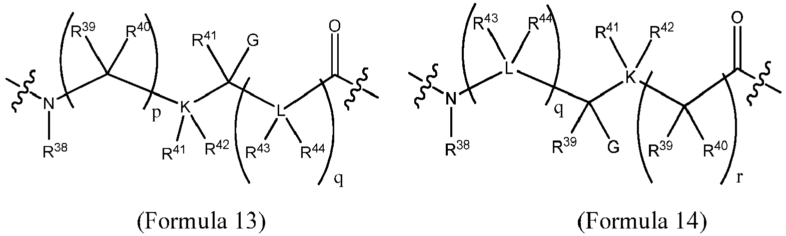
wherein the wavy line to the carbonyl moiety of either structure represents the point of attachment of Aₒ to W and wherein the wavy line to the amino moiety of either structure represents the point of attachment of Aₒ to A₁,
wherein K and L independently are C, N, O or S, provided that when K or L is O or S, R⁴¹ and R⁴² to K or R⁴³ and R⁴⁴ to L are absent, and when K or L are N, one of R⁴¹, R⁴² to K or one of R⁴³, R⁴⁴ to L are absent, and provided that no two adjacent L are independently selected as N, O, or S;
wherein q is an integer ranging from 0 to 12, and r is an integer ranging from 1 to 12;
wherein G is hydrogen, optionally substituted C₁-C₆ alkyl, -OH, -OR^{G}, -CO₂H, CO₂R^{G}, wherein R^{G} is C₁-C₆ alkyl, aryl or heteroaryl, optionally substituted, a suitable protecting group, -NH₂, or -N(R^{G})(R^{G}), wherein R^{G} independently selected is as previously defined or both R^{G} together with the nitrogen to which they are attached comprises a 5- or 6-membered heterocycloalkyl or both R^{G} together form a suitable protecting group;
wherein R³⁸ is hydrogen or optionally substituted C₁-C₆ alkyl; R³⁹-R⁴⁴ independently are hydrogen, optionally substituted C₁-C₆ alkyl, optionally substituted, or optionally substituted heteroaryl, or both R³⁹, R⁴⁰ together with the carbon to which they are attached comprise a C₃-C₆ cycloalkyl, or R⁴¹, R⁴² together with K to which they are attached when K is C, or R⁴³, R⁴⁴ together with L to which they are attached when L is C, comprise a C₃-C₆ cycloalkyl, or R⁴⁰ and R⁴¹, or R⁴⁰ and R⁴³, or R⁴¹ and R⁴³ to together with the carbon or heteroatom to which they are attached and atoms intervening between those carbon and/or heteroatoms comprise a 5- or 6-membered cycloalkyl or heterocycloalkyl,
or wherein Aₒ has a structure corresponding to alpha-amino, beta-amino or another amine-containing acid.

6. The composition for use of any one of claims 1-5, wherein subscript a is 1;
wherein -L_{b}-Aₐ in Formula 1 is -L_{b}-A when Aₒ is absent or -L_{b}-A is -L_{b}-A₁-Aₒ when Aₒ is present so that A becomes A₁-Aₒ
wherein L_{b}-Aₐ- has the structure of Formula 8:
wherein 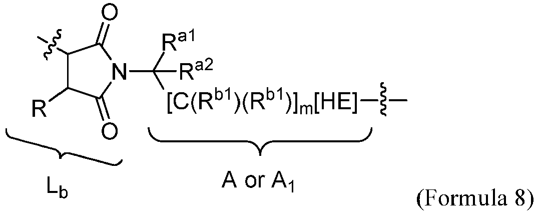
the -[C(R^{b1})(R^{b1})]ₘ-[HE]- moiety is A or A₁;
R and R^{a2} independently are hydrogen or methyl;
R^{a1} is hydrogen, methyl, ethyl or R^{a1} is a Basic Unit (BU) that participates in base-assisted hydrolysis of the succinimide ring;
HE is an optional Hydrolysis Enhancer (HE) Unit;
subscript m is an integer ranging from 0 to 6;
each R^{b1} independently is hydrogen, optionally substituted C₁-C₆ alkyl, optionally substituted aryl or optionally substituted heteroaryl, or two R^{b1} together with the carbon(s) to which they are attached comprise a C₃-C₆ cycloalkyl, or one R^{b1} and HE together with the carbon to which they are attached comprise a 5 or 6-membered cycloalkyl or a 5- or 6-membered heterocycloalkyl and the other R^{b1} is hydrogen, optionally substituted C₁-C₆ alkyl, optionally substituted aryl, or optionally substituted heteroaryl;
BU has the structure of -[C(R¹)(R¹)]-[C(R²)(R²)]ₙ-N(R²²)(R²³),
wherein subscript n is 0, 1, 2, or 3;
each R¹ independently is hydrogen or C₁-C₄ alkyl or two R¹ together with the carbon to which they are attached comprise a C₃-C₆ cycloalkyl, and each R² independently is hydrogen, optionally substituted C₁-C₆ alkyl, optionally substituted aryl, or optionally substituted heteroaryl, or two R² together with the carbon(s) to which they are attached and any intervening carbons define a C₃-C₆ cycloalkyl, or one R¹ and one R² together with the carbons to which they are attached and any intervening carbons comprise a 5- or 6-membered cycloalkyl and the remaining R¹ and R² are as defined;
R²² and R²³ independently are hydrogen, or optionally substituted C₁-C₆ alkyl, or together with the nitrogen to which they are attached comprise a 5- or 6-membered heterocycloalkyl; and
wherein the wavy line to the succinimide ring of L_{b} indicates covalent bonding of sulfur derived from a sulfhydryl group of a targeting moiety and the other wavy line indicates covalent bonding of A (or A₁) to the remainder of the composition structure.

7. The composition for use of claim 1, wherein the Ligand Drug Conjugate compounds of the composition are represented by the structures of Formula 16A and Formula 16B: 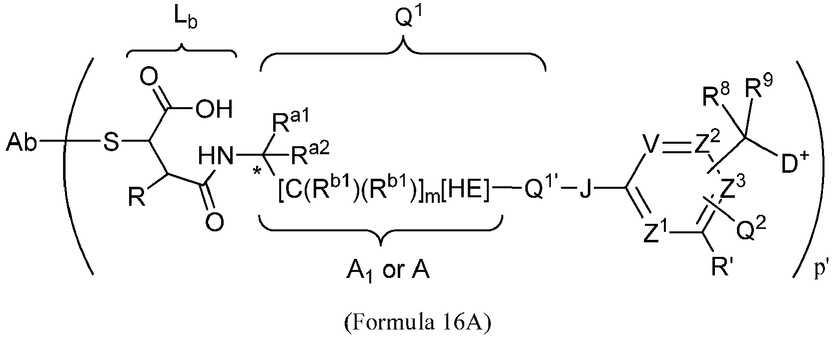 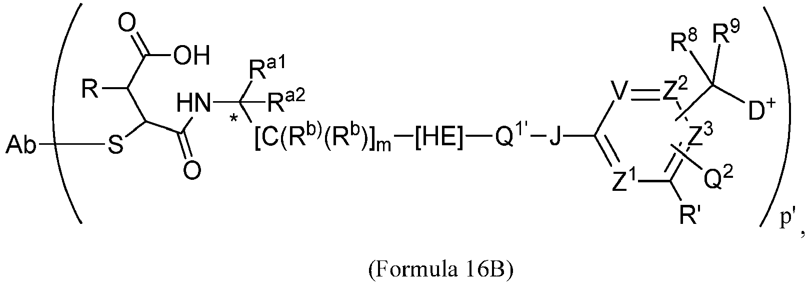
wherein Ab is an antibody Ligand Unit;
S is a sulfur atom of the antibody Ligand Unit;
the asterisk (*) designates chirality or absence thereof at the indicated carbon;
Q^{1'} is Aₒ-W_{w}, wherein Aₒ is an optional subunit of A so that -Q^{1'}- is -W_{w}- when Aₒ is absent, or is -Aₒ-W_{W}- when Aₒ is present,
wherein -[C(R^{b})(R^{b})]ₘ-[HE]- becomes A when Aₒ is absent or is A₁ when Aₒ is present, and
wherein subscript w is 1 when Q² is absent or w is 0 so that W is absent and Q² is present;
R is hydrogen;
R^{a1} is BU, wherein BU has the structure of -CH₂-N(R²²)(R²³), or an acid addition salt thereof, wherein R²² and R²³ independently are hydrogen or methyl or both together with the nitrogen atom to which they are attached comprise a 5- or 6-membered heterocycloalkyl;
R^{a2} is hydrogen;
subscript m is an integer ranging from 0 to 5 when HE is present or from 1 to 5 when HE is absent;
each R^{b1} independently is hydrogen or optionally substituted C₁-C₆ alkyl;
HE is absent or is -C(=O)-;
J is -O- or -NH-;
Q² when present is W'_{w'}-E, wherein E is -O- or -NH-; and
subscript p' is an integer ranging from 1 to 24.

8. The composition for use of claim 7, wherein the Ligand Drug Conjugate compounds of the composition have the structures of Formula 17A and Formula 17B: 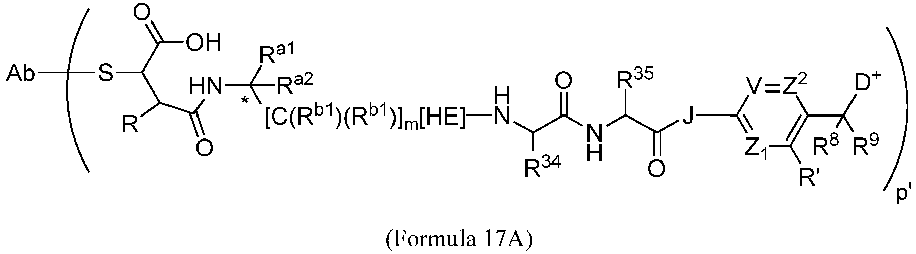 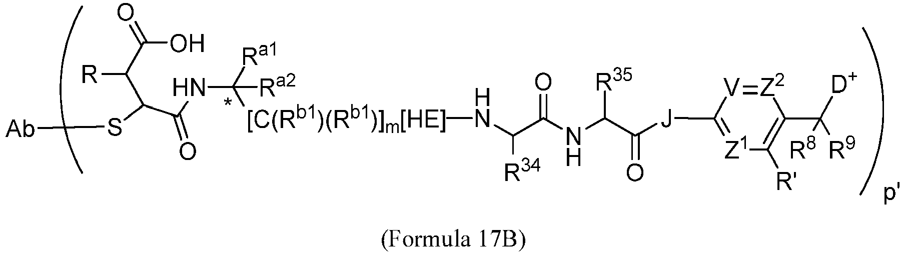
wherein J is -NH-;
one of V or Z¹ is =C(R²⁴)-, wherein R²⁴ is hydrogen, -Cl or -NO₂, and the others of V, Z¹, and Z² are =CH-;
R⁸ is hydrogen;
R⁹ is hydrogen or methyl; and
subscript p' is an integer ranging from 1 to 8.

9. The composition for use of claim 7, wherein the Ligand Drug Conjugate compounds of the composition have the structures of Formula 18A and Formula 18B: 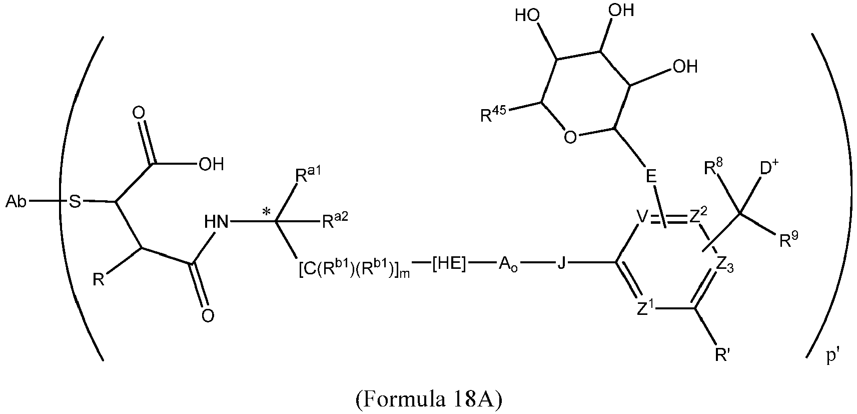 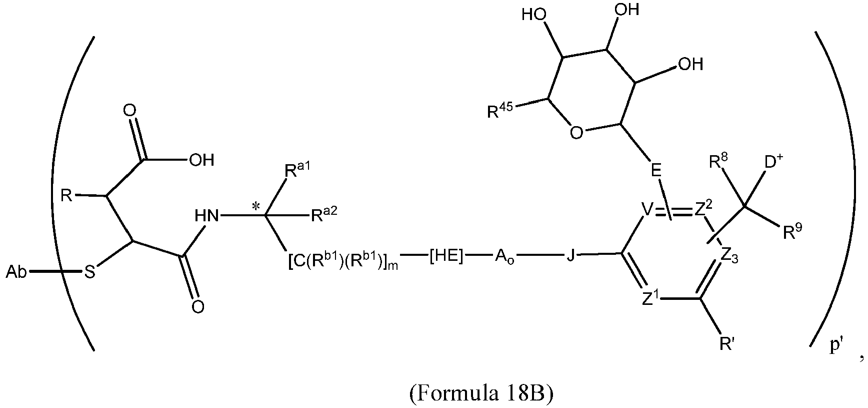
wherein Aₒ is an optional subunit of A, wherein the -[C(R^{b1})(R^{b1})]ₘ-[HE]- moiety is A when Aₒ is absent or the moiety is A₁ when Aₒ is present so that A becomes -A₁-Aₒ-;
R is -H;
R^{a1} is a Basic Unit (BU), wherein BU has the structure of -CH₂-N(R²²)(R²¹), or an acid addition salt thereof, wherein R²² and R²³ independently are hydrogen or methyl or both together with the nitrogen atom to which they are attached comprise a 5- or 6-membered heterocycloalkyl;
R^{a2} is hydrogen;
subscript m is an integer ranging from 0 to 5 when HE is present or 1 to 5 when HE is absent;
each R^{b1} independently is hydrogen or optionally substituted C₁-C₆ alkyl;
HE is absent or is -C(=O)-; and
R⁸ is hydrogen; and
R⁹ is hydrogen, optionally substituted C₁-C₆ alkyl or optionally substituted phenyl;
particularly wherein the Ligand Drug Conjugate compounds of the composition have the structures of Formula 19A and Formula 19B: 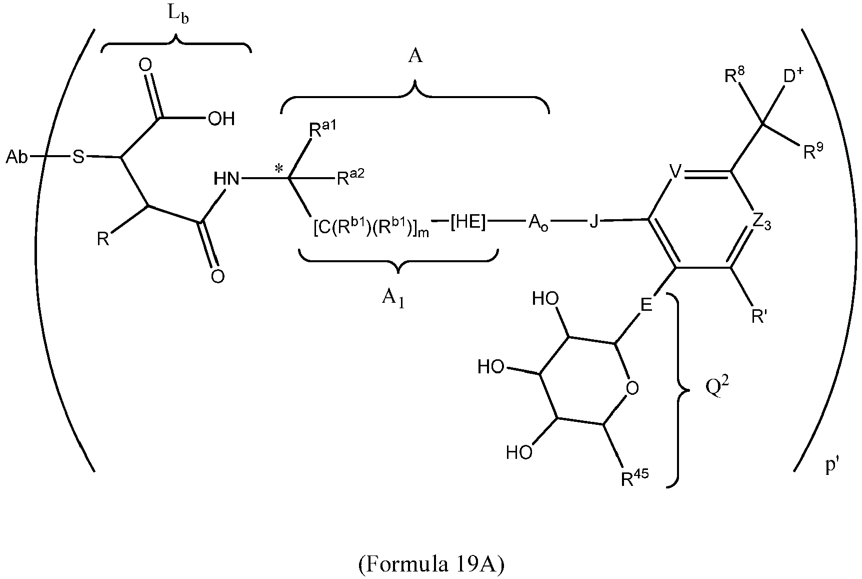 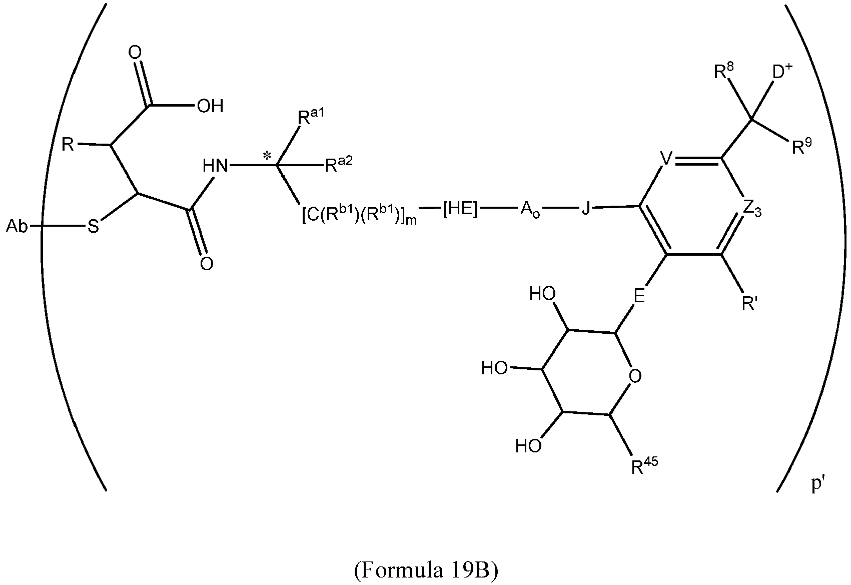
wherein
R is hydrogen;
R^{a1} is a Basic Unit (BU), wherein BU has the structure of -CH₂-N(R²²)(R²¹), or an acid addition salt thereof, wherein R²² and R²³ independently are hydrogen or methyl or both together with the nitrogen atom to which they are attached comprise a 5- or 6-membered heterocycloalkyl;
R^{a2} is hydrogen;
subscript m is an integer ranging from 0 to 5 when HE is present or 1 to 5 when HE is absent;
each R^{b1} independently is hydrogen or optionally substituted C₁-C₆ alkyl;
HE is absent or is -C(=O)-;
R⁴⁵ is -CO₂H;
E is -O-;
J is -NH-;
V and Z³ are =CH-;
R⁸ is hydrogen;
R⁹ is hydrogen or methyl; and
the indicated (*) carbon is predominantly in the same absolute configuration as the alpha carbon of an L-amino acid when that indicated carbon has chirality.

10. The composition for use of any one of claims 1-9, wherein -D⁺ is a quaternized tertiary amine-containing tubulin disrupting agent,
particularly wherein the quaternized tubulin disrupting Drug Unit -D⁺ is a quaternized tubulysin Drug Unit,
particularly wherein the quaternized tubulysin Drug Unit -D⁺ has the structure of Formula D_{G-1}' or Formula D_{H-1}': 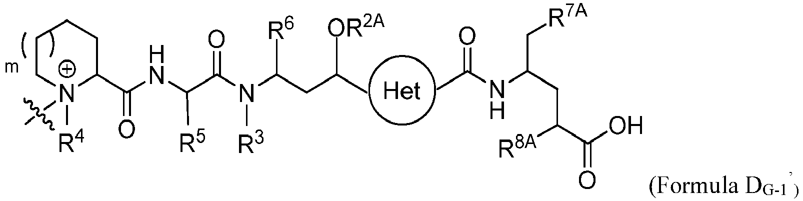 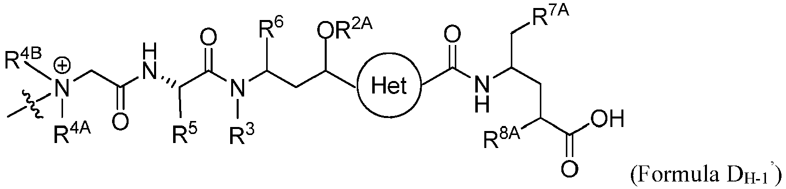
wherein the circle represents a 5-membered nitrogen-heteroaryl and wherein the indicated required substituents to that heteroaryl are in a 1,3-relationship with each other with optional substitution at the remaining positions;
R^{2A} is hydrogen or optionally substituted alkyl or R^{2A} along with the oxygen atom to which it is attached defines an O-linked substituent other than -OH;
R³ is hydrogen or optionally substituted alkyl;
R⁴, R^{4A}, R^{4B}, R⁵, and R⁶ are optionally substituted alkyl, independently selected;
R^{7A} is optionally substituted aryl or optionally substituted heteroaryl;
R^{8A} is hydrogen or optionally substituted alkyl; and
m is 0 or 1,
wherein the wavy line indicates covalent bonding of D⁺ to the remainder of the composition structure.

11. The composition for use of claim 10, wherein the quaternized tubulysin Drug Unit -D⁺ has the structure of: 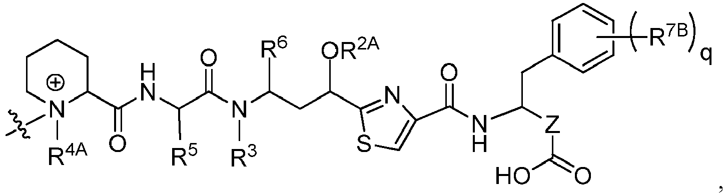
wherein Z is an optionally substituted C₁-C₄ alkylene or an optionally substituted C₂-C₄ alkenylene;
subscript q, indicating the number of R^{7B} substituents, is 1, 2, or 3;
wherein each R^{7B} is independently selected from the group consisting of hydrogen and an O-linked substituent; and
wherein the wavy line indicates covalent bonding of D⁺ to the remainder of the composition structure,
or wherein the quaternized tubulysin Drug Unit -D⁺ has the structure of: 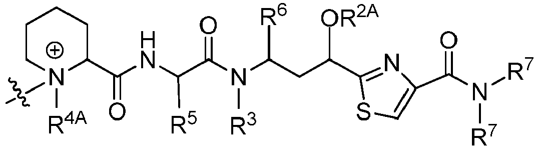
wherein R^{2A} is hydrogen or optionally substituted C₁-C₆ alkyl or R^{2A} along with the oxygen atom to which it is attached defines an O-linked substituent other than -OH;
R³ is optionally substituted C₁-C₆ alkyl;
R⁵ and R⁶ are the side chain residues of natural hydrophobic amino acids;
-N(R⁷)(R⁷) is -NH(C₁-C₆ alkyl) or -NH-N(C₁-C₆ alkyl)₂, wherein one and only one C₁-C₆ alkyl is optionally substituted by -CO₂H, or an ester thereof, or by an optionally substituted phenyl; and
wherein the wavy line indicates covalent bonding of D⁺ to the remainder of the composition structure,
particularly wherein -N(R⁷)(R⁷) is selected from the group consisting of -NH(CH₃), -NHCH₂CH₂Ph, and -NHCH₂-CO₂H, -NHCH₂CH₂CO₂H, and -NHCH₂CH₂CH₂CO₂H,
particularly wherein the O-linked substituent -OR^{2A} is other than -OH so that R^{2A} is not hydrogen,
particularly wherein R^{2A} is -CH₂CH₃.

12. The composition for use of claim 11, wherein the quaternized tubulysin Drug Unit -D⁺ has the structure of: 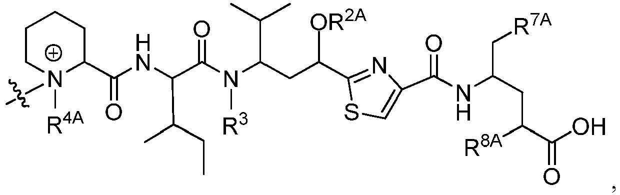 particularly wherein the quaternized tubulysin Drug Unit -D⁺ has the structure of: 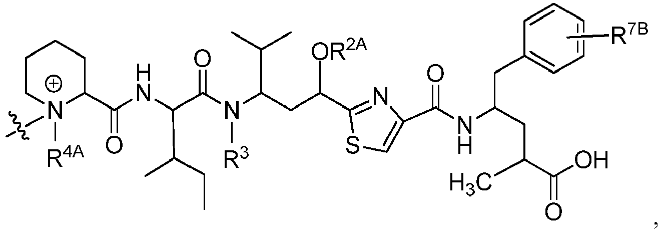
wherein R^{4A} is methyl;
R³ is H, methyl, ethyl, propyl, -CH₂-OC(O)R^{3A}, -CH₂CH(R^{3B})C(O)R^{3A}, or -CH(R^{3B})C(O)NHR^{3A}, wherein R^{3A} is C₁-C₆ alkyl and R^{3B} is H or C₁-C₆ alkyl, independently selected from R^{3A}; and
-OR^{2A} is an O-linked substituent selected from the group consisting of -OR^{2B}, -OC(O)R^{2B} and -OC(O)N(R^{2B})(R^{2C}), wherein R^{2B} and R^{2C} are independently selected from the group consisting of H, C₁-C₆ alkyl, and C₂-C₆ alkenyl; and
R^{7B} is hydrogen or -OH,
particularly wherein R^{2A} is -CH₂CH₃.

13. The composition for use of claim 10, wherein the quaternized tubulysin Drug Unit -D⁺ has the structure of: 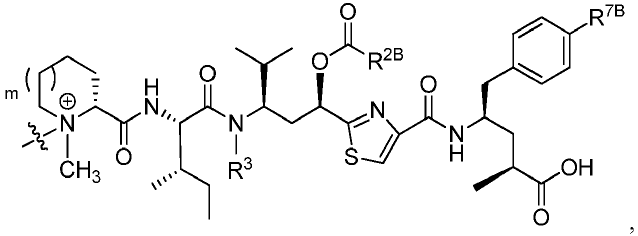
wherein subscript m is 1;
R^{2B} is -CH₃, -CH₂CH₃, -CH(CH₃)₂, -C(CH₃)₃, or -CH=CH₂;
R³ is methyl, ethyl, or propyl; and
R^{7B} is hydrogen or -OH;
or wherein the quaternized tubulysin Drug Unit -D⁺ has the structure of: 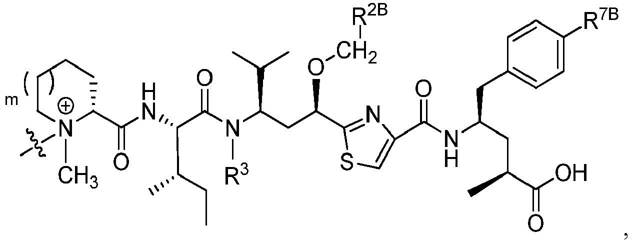
wherein subscript m is 1;
R^{2B} is -H, -CH₃, -CH₂CH₃, or -OCH₃;
R³ is methyl, ethyl or propyl; and
R^{7B} is hydrogen or -OH,
or wherein the quaternized tubulysin Drug Unit -D⁺ has the structure of: 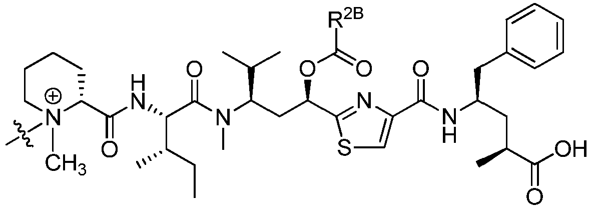 or 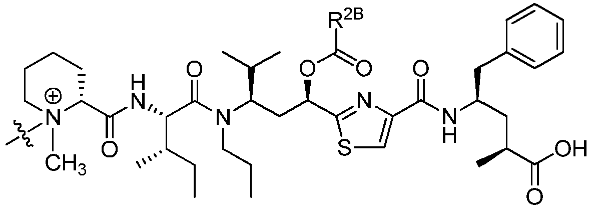
wherein R^{2B} is -CH₃, -CH₂CH₃, -CH₂CH₂CH₃, -CH(CH₃)₂, -CH₂CH(CH₃)₂, - CH₂C(CH₃)₃,
or 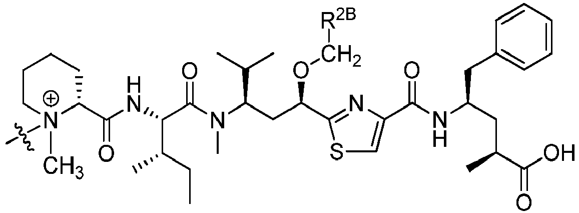 or 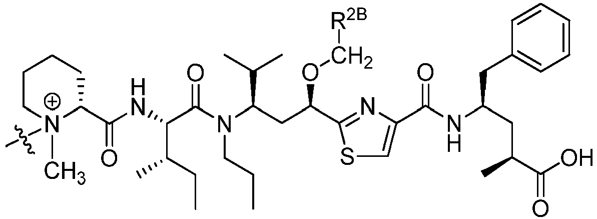
wherein R^{2B} is hydrogen, methyl, or -OCH₃ so that -CH₂R^{2B} is a methyl, ethyl, or methoxymethyl ether substituent,
particularly wherein R^{2B} is methyl.

14. The composition for use of claim 1, wherein the composition is represented by the structure of: 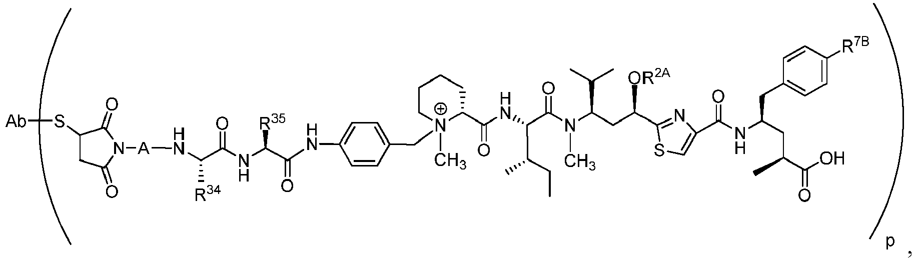
wherein Ab is an antibody Ligand Unit;
S is a sulfur atom of the antibody Ligand Unit;
R³⁴ is isopropyl and R³⁵ is -CH₃, isopropyl, -CH₂CH₂CH₂NH(C=O)NH₂, or -CH₂CH₂CO₂H;
R^{7B} is hydrogen or -OH;
R^{2A} is C₁-C₆ alkyl, C₂-C₆ alkenyl, -OCH₂OR^{2B}, -C(=O)R^{2B}, or -C(=O)NHR^{2B}, wherein R^{2B} is hydrogen or C₁-C₆ alkyl; and
subscript p is a number ranging from 1 to 8,
particularly wherein
R³⁴ is isopropyl and R³⁵ is -CH₃ or -CH₂CH₂CH₂NH(C=O)NH₂;
R^{7B} is hydrogen or -OH; and
R^{2A} is C₁-C₄ alkyl, -C(=O)R^{2B}, or -C(=O)NHR^{2B}, wherein R^{2B} is C₁-C₄ alkyl, particularly wherein A is -CH₂(CH₂)₄(C=O)- or -CH₂(CH₂)₄(C=O)NHCH₂CH₂(C=O)-, particularly wherein the structure of the composition is represented by the structure of: 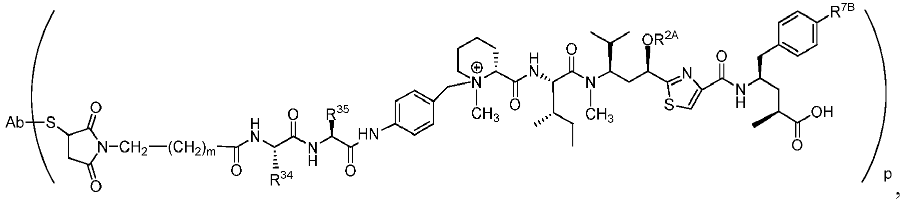
wherein Ab is an antibody Ligand Unit;
S is a sulfur atom of the antibody Ligand Unit;
subscript p is a number ranging from 1 to 8; and
subscript m is 4,
particularly wherein R^{2A} is -C(O)CH₃, methyl, ethyl, or propyl.

15. The composition for use of claim 1, wherein the composition is represented by the structure of: 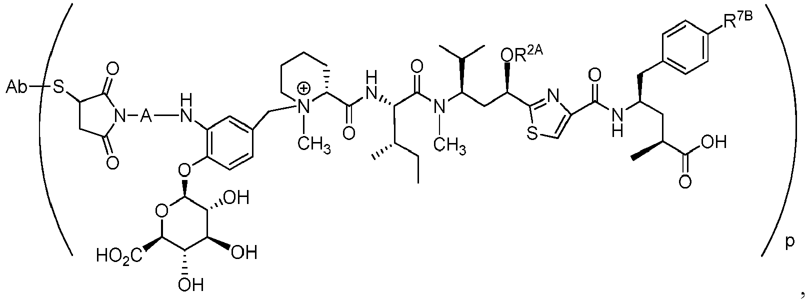
wherein Ab is an antibody Ligand Unit;
S is a sulfur atom of the antibody Ligand Unit;
R^{7B} is hydrogen or -OH;
R^{2A} is C₁-C₆ alkyl, -OCH₂OR^{2B}, -C(=O)R^{2B}, or -C(=O)NHR^{2B}, wherein R^{2B} is C₁-C₆ alkyl or C₂-C₆ alkenyl; and
subscript p is a number ranging from 1 to 8,
particularly wherein
R^{7B} is hydrogen or -OH; and
R^{2A} is C₁-C₄ alkyl, -C(=O)R^{2B} or -C(=O)NHR^{2B}, wherein R^{2B} is C₁-C₄ alkyl, particularly wherein A is -CH₂(CH₂)₄(C=O)- or -CH₂(CH₂)₄(C=O)NHCH₂CH₂(C=O)-, particularly wherein the structure of the composition is represented by the structure of: 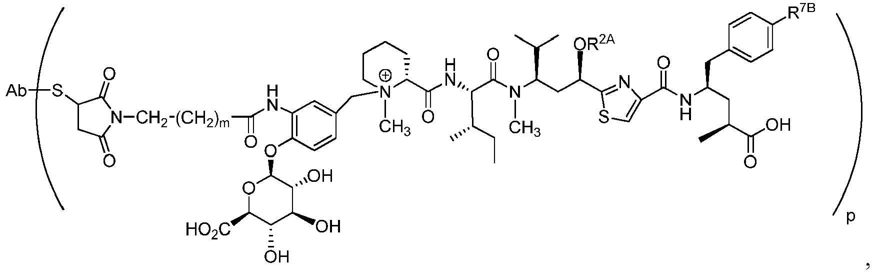
wherein Ab is an antibody Ligand Unit;
S is a sulfur atom of the antibody Ligand Unit;
subscript p is a number ranging from 1 to 8; and
subscript m is 4,
particularly wherein R^{2A} is -C(O)CH₃, methyl, ethyl, or propyl.

16. The composition for use of claim 1, wherein each Ligand Drug Conjugate compound of the composition is represented by the structure of: 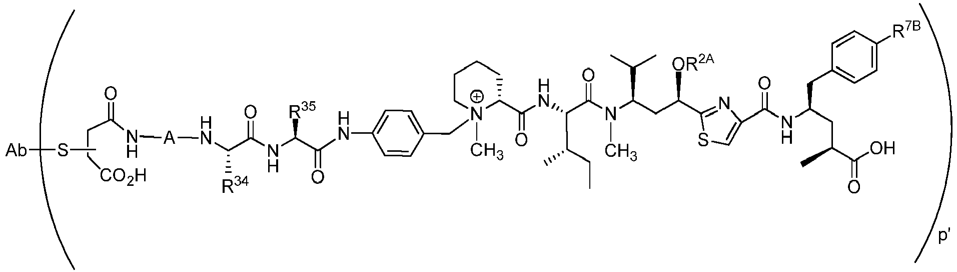
wherein Ab is an antibody Ligand Unit;
S is a sulfur atom of the antibody Ligand Unit;
the Ab-S- moiety is bonded to the carbon α or β to the carboxylic acid;
R³⁴ is isopropyl and R³⁵ is -CH₃ or -CH₂CH₂CH₂NH(C=O)NH₂;
R^{7B} is hydrogen or -OH;
R^{2A} is C₁-C₄ alkyl, -C(=O)R^{2B}, or -C(=O)NHR^{2B}, wherein R^{2B} is C₁-C₄ alkyl; and
subscript p' is an integer ranging from 1 to 8,
particularly wherein A is -CH₂(CH₂)₄(C=O)- or -CH₂(CH₂)₄(C=O)NHCH₂CH₂(C=O)-, particularly wherein each Ligand Drug Conjugate compound of the composition is represented by the structure of: 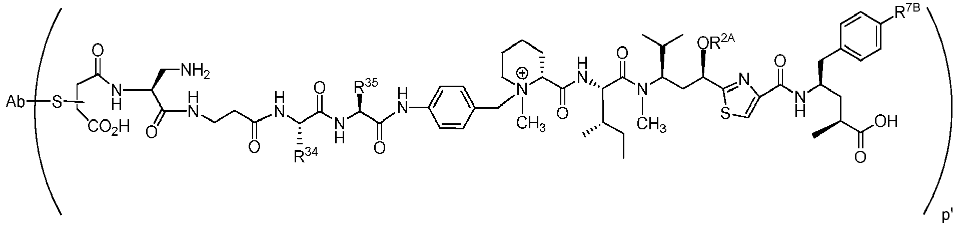 or 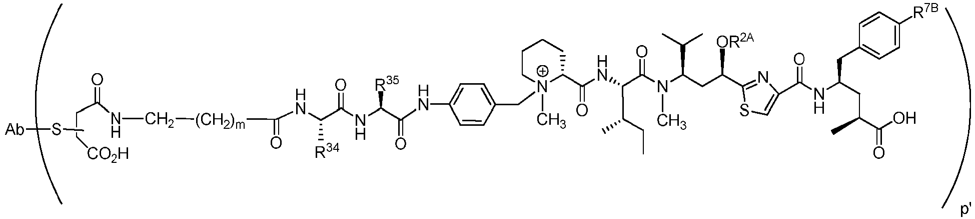
wherein Ab is an antibody Ligand Unit;
S is a sulfur atom of the antibody Ligand Unit;
the Ab-S- moiety is bonded to the carbon α or β to the carboxylic acid;
subscript p' is an integer ranging from 1 to 8; and
subscript m is 4,
particularly wherein R^{2A} is -C(O)CH₃, methyl, ethyl, or propyl.

17. The composition for use of claim 1, wherein each Ligand Drug Conjugate compound of the composition is represented by the structure of: 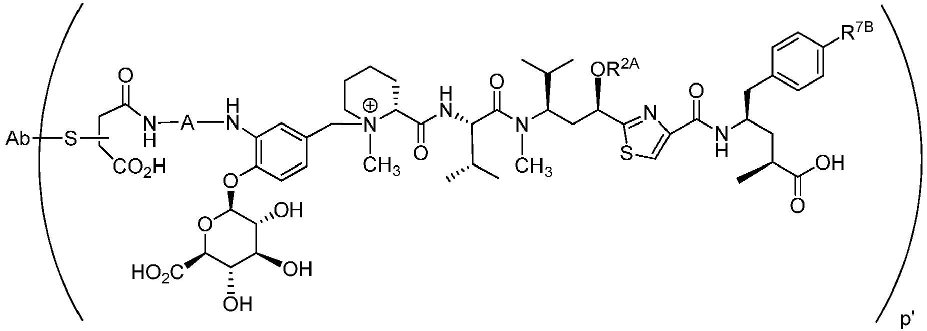 wherein
Ab is an antibody Ligand Unit;
S is a sulfur atom of the antibody Ligand Unit;
the Ab-S- moiety is bonded to the carbon α or β to the carboxylic acid;
R^{7B} is hydrogen or -OH;
R^{2A} is C₁-C₄ alkyl, -C(=O)R^{2B}, or -C(=O)NHR^{2B}, wherein R^{2B} is C₁-C₄ alkyl;
and subscript p' is an integer ranging from 1 to 8,
particularly wherein A is -CH₂(CH₂)₄(C=O)- or -CH₂(CH₂)₄(C=O)NHCH₂CH₂(C=O)-, particularly wherein each Ligand Drug Conjugate compound of the composition is represented by the structure of: 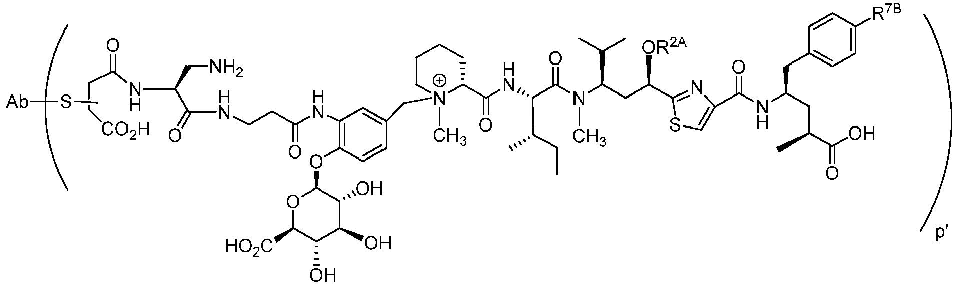 or 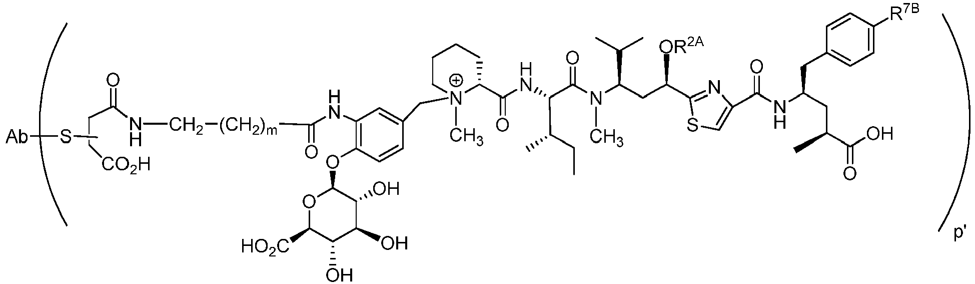
wherein Ab is an antibody Ligand Unit;
S is a sulfur atom of the antibody Ligand Unit;
the Ab-S- moiety is bonded to the carbon α or β to thecarboxylic acid;
subscript p' is an integer ranging from 1 to 8; and
subscript m is 4,
particularly wherein R^{2A} is -C(O)CH₃, methyl, ethyl, or propyl.

18. The composition for use of claim 1, wherein -D⁺ is a quaternized auristatin Drug Unit or a quaternized dolastatin Drug Unit,
particularly wherein the quaternized dolastatin Drug Unit is quaternized dolastatin 10 or quaternized dolastatin 15,
particularly wherein the quaternized auristatin Drug unit -D⁺ has the structure of D_{E}' or D_{F}': 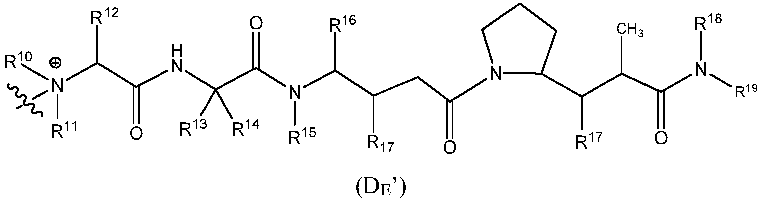 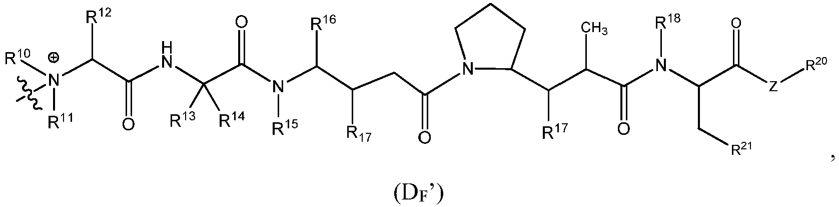
wherein R¹⁰ and R¹¹ are independently C₁-C₈ alkyl;
R¹² is hydrogen, C₁-C₈ alkyl, C₃-C₈ cycloalkyl, aryl, -X¹-aryl, -X¹-(C₃-C₈ cycloalkyl), C₃-C₈ heterocycle, or -X¹-(C₃-C₈ heterocycle);
R¹³ is hydrogen, C₁-C₈ alkyl, C₃-C₈ cycloalkyl, aryl, -X¹-aryl, -X¹-(C₃-C₈ cycloalkyl), C₃-C₈ heterocycle, or -X¹-(C₃-C₈ heterocycle);
R¹⁴ is hydrogen or methyl;
or R¹³ and R¹⁴ taken together with the carbon to which they are attached comprise a C₃-C₈ cycloalkyl;
R¹⁵ is hydrogen or C₁-C₈ alkyl;
R¹⁶ is hydrogen, C₁-C₈ alkyl, C₃-C₈ cycloalkyl, aryl, -X¹-aryl, -X¹-(C₃-C₈ cycloalkyl), C₃-C₈ heterocycle, or -X¹-(C₃-C₈ heterocycle);
R¹⁷ independently are hydrogen, -OH, C₁-C₈ alkyl, C₃-C₈ cycloalkyl, or O-(C₁-C₈ alkyl);
R¹⁸ independently are hydrogen or C₁-C₈ alkyl;
R¹⁹ is -C(R^{19A})₂-C(R^{19A})₂-aryl, -C(R¹⁹A)₂-C(R¹⁹A)₂-(C₃-C₈ heterocycle), or -C(R^{19A})₂-C(R^{19A})₂-(C₃-C₈ cycloalkyl);
R²¹ is aryl or C₃-C₈ heterocycle; wherein R^{19A} is hydrogen, C₁-C₈ alkyl, or -OH;
R²⁰ is hydrogen, C₁-C₂₀ alkyl, aryl, C₃-C₈ heterocycle, -(R⁴⁷O)ₘ-R⁴⁸, or -(R⁴⁷O)ₘ-CH(R⁴⁹)₂;
subscript m is an integer ranging from 1-1000;
R⁴⁷ is C₂-C₈ alkyl;
R⁴⁸ is hydrogen or C₁-C₈ alkyl;
R⁴⁹ independently are -COOH, -(CH₂)ₙ-N(R⁵⁰)₂, -(CH₂)ₙ-SO₃H, or -(CH₂)ₙ-SO₃-C₁-C₈ alkyl;
R⁵⁰ independently are C₁-C₈ alkyl or -(CH₂)ₙ-COOH;
Z is O, S, NH, or NR⁴⁶, wherein R⁴⁶ is C₁-C₈ alkyl;
X¹ is C₁-C₁₀ alkylene; and
subscript n is an integer ranging from 0 to 6;
wherein the wavy line indicates covalent bonding of D⁺ to the remainder of the composition structure,
particularly wherein each Ligand Drug Conjugate compound of the composition is represented by the structure of: 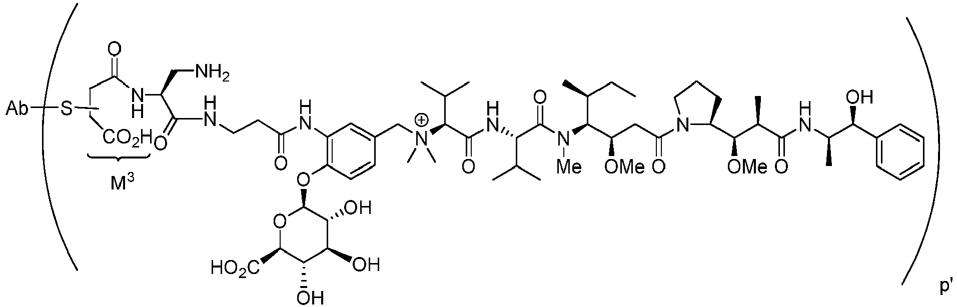 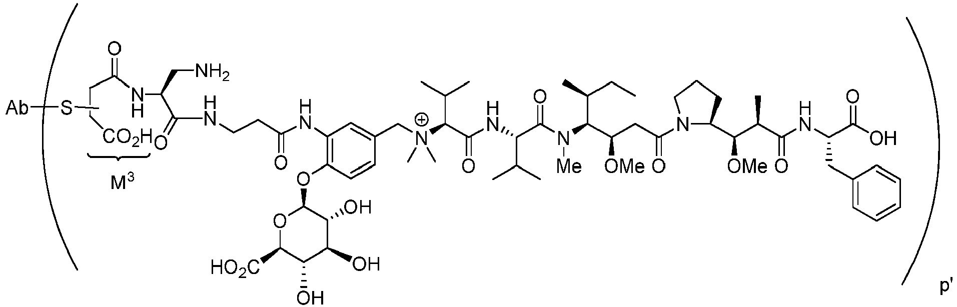 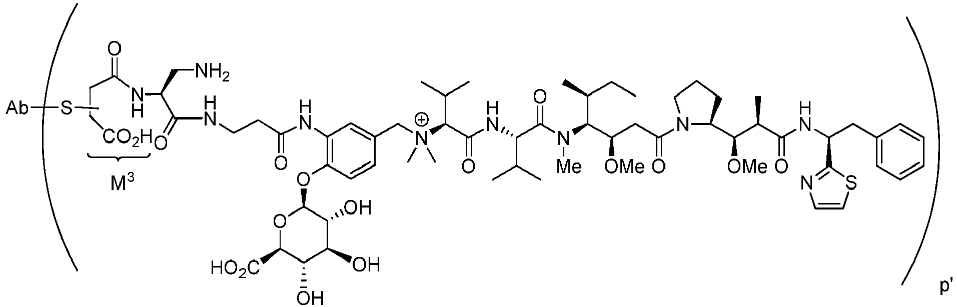 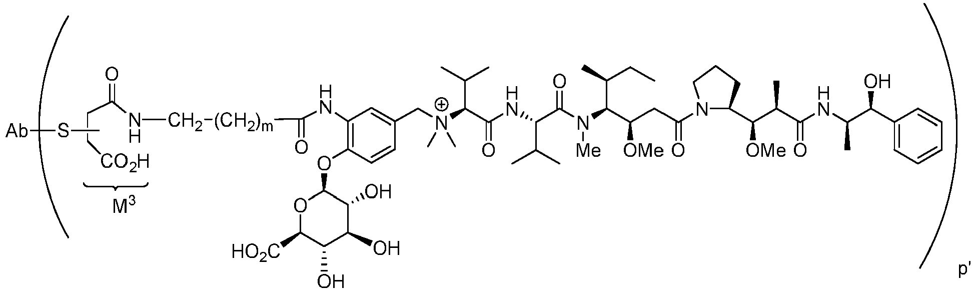 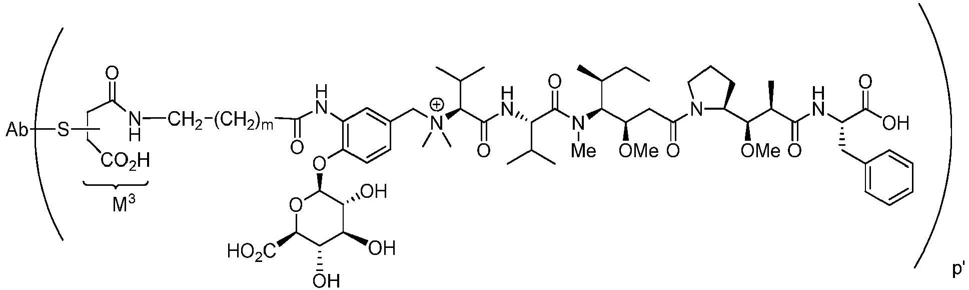 or 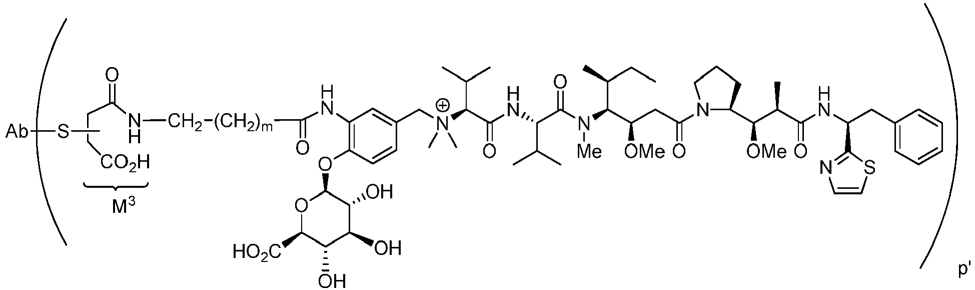
wherein Ab is an antibody Ligand Unit;
S is a sulfur atom of the antibody Ligand Unit;
the Ab-S- moiety is bonded to the carbon α or β to thecarboxylic acid; and
subscript p' is an integer ranging from 1 to 8.

19. The composition for use of any one of claims 1-18, wherein the use is treatment of a cancer.

20. The composition for use of claim 19, wherein the cancer is a solid tumor, a blood-borne cancer, an acute leukemia, a chronic leukemia, or a lymphoma.

21. The composition for use of claim 20, wherein the cancer is a solid tumor selected from the group consisting of fibrosarcoma, myxosarcoma, liposarcoma, chondrosarcoma, osteogenic sarcoma, chordoma, angio sarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

22. The composition for use of claim 20, wherein the cancer is a blood-borne cancer selected from the group consisting of acute lymphoblastic leukemia, acute lymphoblastic B-cell leukemia, acute lymphoblastic T-cell leukemia, acute myeloblastic leukemia, acute promyelocytic leukemia, acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia, chronic lymphocytic leukemia, hairy cell leukemia, and multiple myeloma.

23. The composition for use of claim 20, wherein the cancer is a lymphoma selected from the group consisting of Hodgkin's disease, non-Hodgkin's Lymphoma, multiple myeloma, Waldenstrom's macroglobulinemia, heavy chain disease, and polycythemia vera.

24. The composition for use of any one of claims 1-18, wherein the use is treatment of an autoimmune disease.

## Patentansprüche

1. Ligandenarzneimittelkonjugat-Zusammensetzung (LDC-Zusammensetzung) zur Verwendung bei der Behandlung eines Krebses oder einer Autoimmunerkrankung, wobei die LDC-Zusammensetzung durch die Struktur der Formel 1 dargestellt wird: 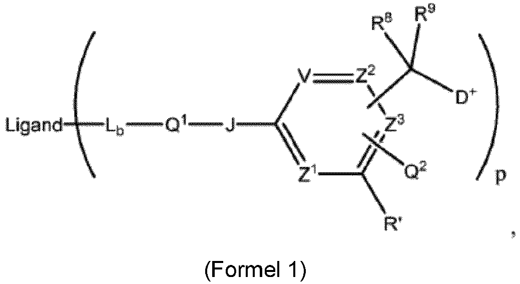 wobei
"Ligand" ist Ligandeneinheit (L) ist, wobei L in der Lage ist, selektiv an eine Zieleinheit zu binden, und L ein Antikörper oder ein Fragment davon ist, um ein Antikörperarzneimittelkonjugat (ADC) zu definieren, wobei die Zieleinheit ein Antigen ist, das in der Lage ist, selektiv an das ADC zu binden, und wobei das Antigen ein extrazellulär zugängliches Zelloberflächenprotein, Glykoprotein oder Kohlenhydrat ist, das bevorzugt von abnormalen Zellen im Vergleich zu normalen Zellen präsentiert wird, wobei die abnormalen und normalen Zellen Zellen in einem Säugetier sind, wobei die abnormalen Zellen hyperproliferierende Zellen sind;
L_{b} ein primärer Linker ist;
Q¹ Aₐ-W_{w} ist, wobei A eine optionale Strecher-Einheit ist, so dass der Index a 0 ist, wenn A abwesend ist, oder 1 ist, wenn A vorhanden ist, und zwei, drei oder vier Untereinheiten umfasst;
Q² W'_{w'}-E- ist, wobei Q², falls vorhanden, an V, Z¹, Z² oder Z³ gebunden ist;
W_{w} und W'_{w'} spaltbare Einheiten sind, wobei
W_{w} von Q¹ zur selektiven Spaltung durch eine intrazelluläre oder regulatorische Protease im Vergleich zu Serumproteasen oder durch Glutathion durch Disulfidaustausch fähig ist, oder gegenüber Hydrolyse unter saureren Bedingungen, die im Vergleich zum physiologischen pH-Wert des Serums in Lysosomen vorhanden sind, reaktiver ist,
W'_{w'}-E von Q² eine glykosidische Bindung bereitstellt, die durch eine intrazellulär gelegene Glykosidase spaltbar ist, und
der Index w 0 oder 1 ist, so dass W_{w} abwesend ist, wenn w 0 ist, oder W_{w} vorhanden ist, wenn w 1 ist, und der Index w' 0 oder 1 ist, wobei W'_{w'}-E abwesend ist, wenn w' 0 ist, oder W'_{w'}-E vorhanden ist, wenn w' 1 ist, und wobei w + w' 1 ist, so dass genau eines von W_{w}, W'_{w'}-E vorhanden ist;
V, Z¹, Z² und Z³ =N- oder =C(R²⁴)- sind, wobei R²⁴ Wasserstoff, optional substituiertes Alkyl, Alkenyl oder Alkinyl oder Halogen, -NO₂, -CN, eine andere elektronenziehende Gruppe eine elektronenabgebende Gruppe, -Q² oder -C(R⁸)(R⁹)-D⁺ ist, wobei mindestens eines von V, Z¹, Z² und Z³ =C(R²⁴)- ist, wenn w 1 ist, und mindestens zwei von V, Z¹, Z² und Z³ =C(R²⁴)- sind, wenn w' 1 ist;
vorausgesetzt, dass, wenn w 1 ist, Q² abwesend ist, und genau ein R²⁴ -C(R⁸)(R⁹)-D⁺ ist, so dass C(R⁸)(R⁹)-D⁺ an eines von V, Z¹, Z² und Z³ gebunden ist, wenn diese variable Gruppe =C(R²⁴)-ist, und die Substituenten Q¹-J- und -C(R⁸)(R⁹)-D⁺- ortho- oder para zueinander sind,
vorausgesetzt, dass, wenn w' 1 ist, genau ein R²⁴ -C(R⁸)(R⁹)-D⁺ ist, so dass -C(R⁸)(R⁹)-D⁺ an eines von V, Z¹, Z², Z³ gebunden ist, wenn diese variable Gruppe =C(R²⁴)- ist, und genau ein R²⁴ Q² ist, so dass Q² an ein anderes von V, Z¹, Z², Z³ gebunden ist, wenn diese variable Gruppe =C(R²⁴)-ist, und die Substituenten Q² und -C(R⁸)(R⁹)-D⁺ zueinander ortho oder para sind;
R⁸ und R⁹ unabhängig voneinander Wasserstoff oder optional substituiertes Alkyl, Alkenyl, Alkinyl, Aryl oder Heteroaryl sind;
R' Wasserstoff, Halogen, -NO₂, -CN, eine andere elektronenziehende Gruppe oder eine elektronenabgebende Gruppe ist;
E und J unabhängig voneinander -O-, -S- oder -N(R³³)- sind, wobei R³³ Wasserstoff oder optional substituiertes Alkyl ist;
D⁺ eine Struktur eines quaternisierten, tertiäres Amin enthaltenden Arzneimittels darstellt; und
der Index p eine durchschnittliche Arzneimittelbeladung mit einer Zahl im Bereich von 1 bis 24 ist; und
wobei die Spaltung durch eine intrazelluläre oder regulatorische Protease, durch Glutathion durch Disulfidaustausch, durch Säurehydrolyse oder durch eine Glycosidase zur Freisetzung des tertiäres Amin enthaltenden Arzneimittels (D) aus einer Ligandenarzneimittelkonjugatverbindung der Zusammensetzung führt.

2. Zusammensetzung zur Verwendung nach Anspruch 1, wobei die Struktur der Zusammensetzung durch die Struktur der Formel 2A oder Formel 2B dargestellt wird:

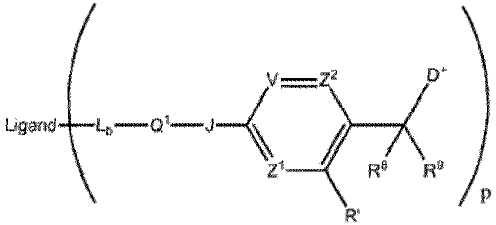

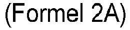

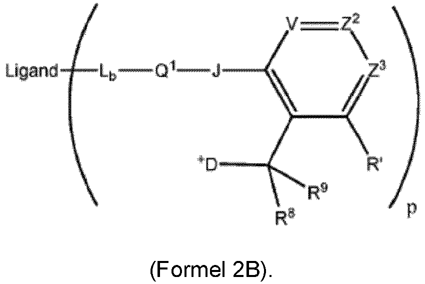

3. Zusammensetzung zur Verwendung nach Anspruch 1, wobei die Struktur der Zusammensetzung durch die Struktur der Formel 3A, Formel 3B, Formel 3C, Formel 3D, Formel 3E oder Formel 3F dargestellt wird:

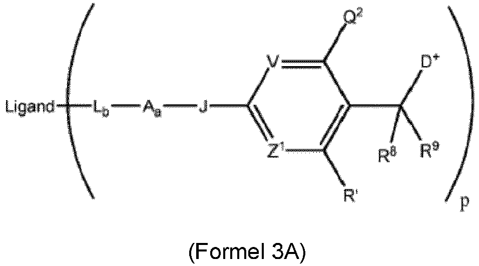

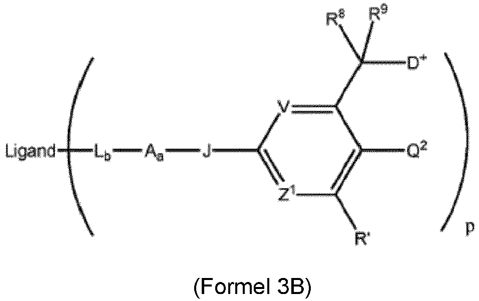

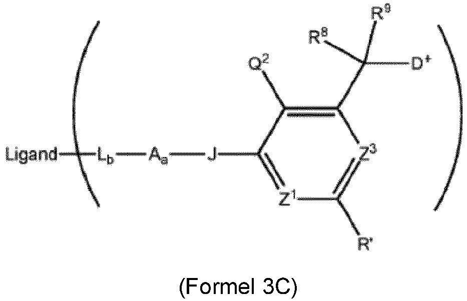

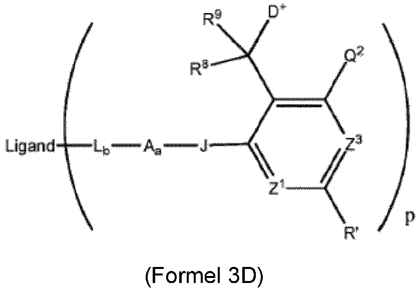

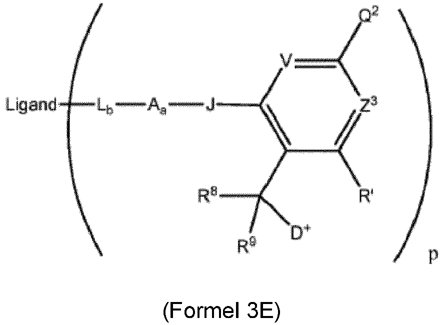

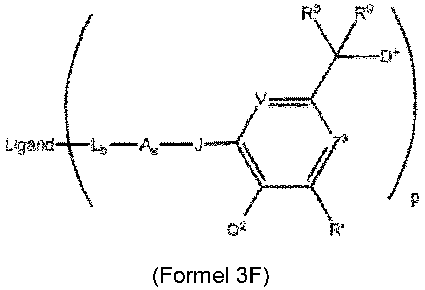

4. Zusammensetzung zur Verwendung nach Anspruch 2, wobei W von Q¹ eine Peptideinheit mit einer Peptidbindung an J umfasst, die selektiv durch eine intrazelluläre oder regulatorische Protease im Vergleich zu Serumproteasen spaltbar ist, wobei die Wirkung der regulatorischen Protease auf W eine Freisetzung von tertiärem Amin enthaltenden Arzneimittel (D) aus einer Ligandenarzneimittelkonjugatverbindung der Zusammensetzung bewirkt, insbesondere wobei die Peptideinheit von Weine Dipeptideinheit mit der Struktur der Formel 6 umfasst: 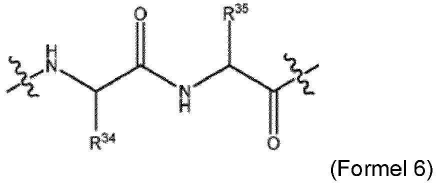 wobei R³⁴ Benzyl, Methyl, Isopropyl, Isobutyl, sec-Butyl, -CH(OH)CH₃ ist oder die Struktur 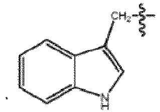 aufweist und R³⁵ Methyl, -(CH₂)4-NH₂, -(CH₂)₃NH(C=O)NH₂, -(CH₂)₃NH(C=NH)NH₂ oder -(CH₂)2CO₂H ist,
wobei die wellenförmige Bindung am C-Terminus des Dipeptids eine kovalente Bindung an J derAryleneinheit der Formel 2Aoder 2B anzeigt und die wellenförmige Bindung am N-Terminus des Dipeptids eine kovalente Bindung an den Rest von W anzeigt, falls ein solcher Rest vorhanden ist, oder an A oder eine Untereinheit davon, wenn a 1 ist, oder an L_{b}, wenn der Index a 0 ist, und wobei die Bindung des Dipeptids an J durch eine intrazelluläre oder regulatorische Protease spaltbar ist,
insbesondere wobei die Struktur der Zusammensetzung durch die Struktur der Formel 9 dargestellt wird: 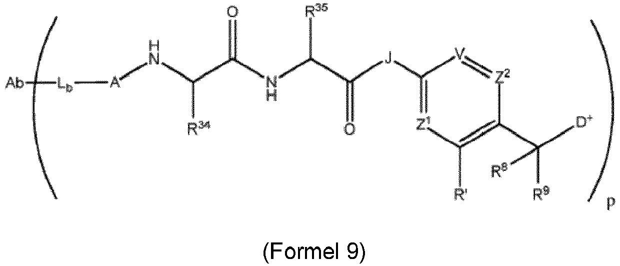
wobei Ab eine Antikörperligandeneinheit ist;
R' Wasserstoff oder eine elektronenabgebende Gruppe ist;
R⁸ Wasserstoff ist;
R⁹ Wasserstoff, optional substituiertes C₁-C₆-Alkyl oder optional substituiertes Phenyl ist;
R³⁴ Methyl, Isopropyl oder -CH(OH)CH₃ ist;
R³⁵ Methyl, -(CH₂)₃NH(C=O)NH₂, oder-(CH₂)₂CO₂H ist;
J -N(R³³)- ist, wobei R³³ Wasserstoff oder Methyl ist; und
V und Z¹ unabhängig voneinander =CH- oder =N- sind,
insbesondere wobei die Struktur der Zusammensetzung durch die Struktur der Formel 10 dargestellt wird: 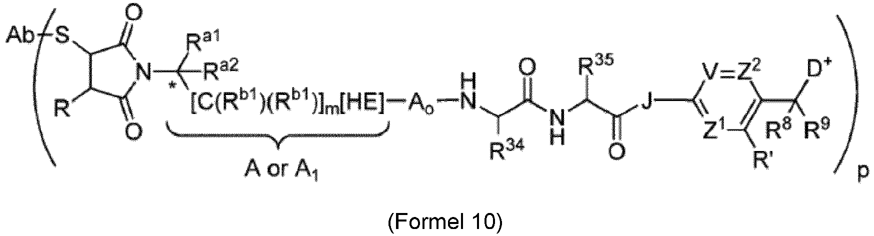 wobei
S ein Schwefelatom einer Antikörperligandeneinheit (Ab-) ist;
das Sternchen (*) die Chiralität oder deren Abwesenheit an dem angegebenen Kohlenstoff bezeichnet;
Aₒ eine optionale Untereinheit von A ist, wobei -[C(R^{b1})(R^{b1})]ₘ-[HE]- A ist, wenn Aₒ abwesend ist, und A, ist, wenn Aₒ vorhanden ist, sodass A zu -A₁-Aₒ- wird;
R -H ist;
R^{a1} -H ist oder eine basische Einheit (BU) ist, wobei BU die Struktur -CH₂-N(R²²)(R²³) aufweist, wobei R²² und R²³ unabhängig voneinander Wasserstoff, Methyl oder Ethyl sind oder beide zusammen mit dem Stickstoffatom, an das sie gebunden sind, ein 5- oder 6-gliedriges Heterocycloalkyl umfassen;
R^{a2} Wasserstoff ist;
der Index m eine ganze Zahl im Bereich von 0 bis 5 ist, wenn HE vorhanden ist, oder von 1 bis 5, wenn HE abwesend ist;
jedes R^{b1} unabhängig Wasserstoff oder optional substituiertes C₁-C₆-Alkyl ist;
HE abwesend ist oder -C(=O)- ist;
R³⁴ Methyl, Isopropyl oder -CH(OH)CH₃ ist;
R³⁵ -(CH₂)₃NH(C=O)NH₂, oder -(CH₂)2CO₂H ist;
J -NH- ist;
V, Z¹ und Z² =CH- sind;
R' Wasserstoff oder eine elektronenabgebende Gruppe ist;
R⁸ Wasserstoff ist; und
R⁹ Wasserstoff oder Methyl ist.

5. Zusammensetzung zur Verwendung nach Anspruch 3, wobei W' von Q² ein glykosidgebundenes Kohlenhydrat ist, wobei die Glykosidbindung W'_{w'}-E von Q² eine Spaltstelle für eine intrazellulär gelegene Glykosidase bereitstellt, wobei die Wirkung der Glykosidase auf W'_{w'}-E eine Freisetzung von tertiärem Amin enthaltenden Arzneimittel (D) aus einer Ligandenarzneimittelkonjugatverbindung der Zusammensetzung bewirkt,
insbesondere wobei W' in Q² eine Kohlenhydrateinheit ist, die an E glykosidgebunden ist,
wobei die glykosidische Bindung durch eine intrazellulär gelegene Glykosidase spaltbar ist, und wobei W'_{w'}-E die Struktur der Formel 7 aufweist: 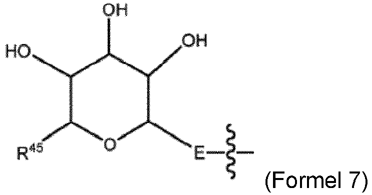
wobei die Wellenlinie E darstellt, das an eines von V, Z¹, Z² oder Z³ gebunden ist, wenn diese variable Gruppe =C(R²⁴) ist, wobei E -O-, -S- oder -N(R³³)- ist, wobei R³³ Wasserstoff oder Methyl ist; und
wobei R⁴⁵ -CH₂OH oder -CO₂H ist,
insbesondere wobei die Struktur der Zusammensetzung durch die Struktur der Formel 11 dargestellt wird: 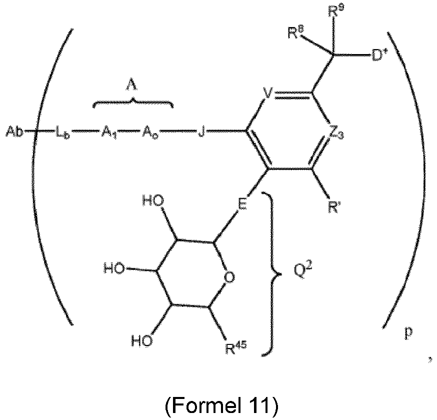 wobei
Ab eine Antikörperligandeneinheit ist;
A₁ und Aₒ unabhängig voneinander ausgewählte Untereinheiten von A sind, wobei Aₒ eine optionale Untereinheit von A ist, sodass A₁ zu A wird, wenn Aₒ abwesend ist, und A -A₁-Aₒ- ist, wenn Aₒ vorhanden ist;
E -O- oder -NH- ist;
J -N(R³³)- ist, wobei R³³ Wasserstoff oder Methyl ist;
V und Z³ sind unabhängig voneinander =CH- oder =N- sind;
R' Wasserstoff oder eine elektronenziehende Gruppe ist;
R⁸ ist Wasserstoff ist;
R⁹ Wasserstoff, gegebenenfalls substituiertes C₁-C₆-Alkyl oder optional substituiertes Phenyl ist;
R⁴⁵ ist -CO₂H ist; und
der Index p eine Zahl im Bereich von 1 bis 8 ist,
insbesondere wobei die Struktur der Zusammensetzung insbesondere durch die Struktur der Formel12 dargestellt wird: 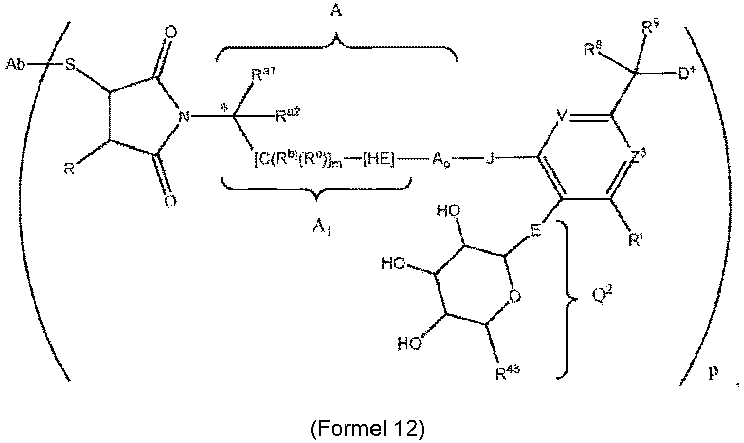 wobei
S ein Schwefelatom der Antikörperligandeneinheit Ab ist;
das Sternchen (*) die Chiralität oder deren Abwesenheit an dem angezeigten Kohlenstoff angibt, der überwiegend in der gleichen absoluten Konfiguration wie das Alpha-Kohlenstoffatom einer L-Aminasäure vorliegt, wenn der angezeigte Kohlenstoff Chiralität aufweist;
A₁ und Aₒ unabhängig voneinander ausgewählte Untereinheiten von A sind, wobei Aₒ eine optionale Untereinheit von A ist, wobei -[C(R^{b1})(R^{b1})]ₘ-[HE]- A ist, wenn Aₒ abwesend ist, und A₁ ist, wenn Aₒ vorhanden ist, sodass A zu A₁-Aₒ wird; wobei Aₒ, wenn es vorhanden ist, in seiner Struktur einer aminhaltigen Säure entspricht, die über das C-terminale Carbonyl der aminhaltigen Säure an J gebunden ist;
R Wasserstoff ist;
R' Wasserstoff oder eine elektronenziehende Gruppe ist;
R^{a1} Wasserstoff ist oder R^{a1} ist eine basische Einheit (BU) ist, die an der basenunterstützten Hydrolyse des Succinimidrings teilnimmt, wobei BU die Struktur -CH₂-N(R²²)(R²³) hat, oder ein Säureadditionssalz davon ist, wobei R²² und R²³ unabhängig voneinander Wasserstoff, Methyl oder Ethyl oder beide zusammen mit dem Stickstoffatom, an das sie gebunden sind, ein 5- oder 6-gliedriges Heterocycloalkyl umfassen;
R^{a2} Wasserstoff ist;
der Index m eine ganze Zahl im Bereich von 0 bis 5 ist, wenn HE vorhanden ist, oder 1 bis 5, wenn HE abwesend ist;
jedes R^{b1} unabhängig voneinander Wasserstoff oder optional substituiertes C₁-C₆-Alkyl ist;
HE abwesend oder -C(=O)- ist;
R⁴⁵ -CO₂H ist;
E -0- ist;
J -NH- ist;
V und Z³ =CH- sind;
R⁸ Wasserstoff ist;
R⁹ Wasserstoff oder Methyl ist; und
der Index p eine Zahl im Bereich von 1 bis 8 ist,
oder wobei Aₒ, falls vorhanden, die Struktur der Formel 13 oder der Formel 14 aufweist: 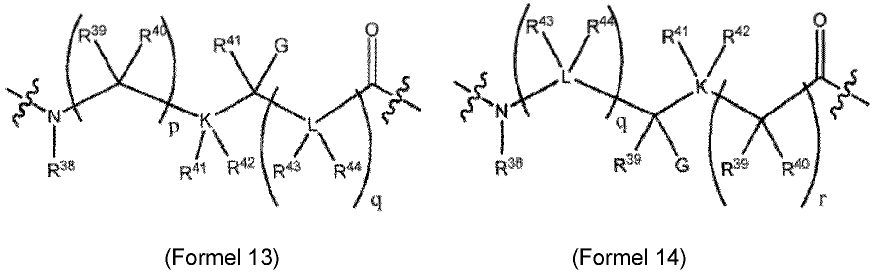
wobei die Wellenlinie zur Carbonylgruppe jeder der beiden Strukturen den Punkt der Bindung von Aₒ an W darstellt, und wobei die Wellenlinie zur Aminogruppe jeder der beiden Strukturen den Punkt der Bindung von Aₒ an A₁ darstellt,
wobei K und L unabhängig voneinander C, N, O oder S sind, vorausgesetzt, dass, wenn K oder L O oder S ist, R⁴¹ und R⁴² bis K oder R⁴³ und R⁴⁴ bis L abwesend ist, und wenn K oder L N sind, einer von R⁴¹, R⁴² bis K oder einer von R⁴³, R⁴⁴ bis L abwesend ist, und vorausgesetzt, dass keine zwei benachbarten L unabhängig voneinander als N, O oder S ausgewählt sind;
wobei q eine ganze Zahl im Bereich von 0 bis 12 ist und r eine ganze Zahl im Bereich von 1 bis 12 ist;
wobei G Wasserstoff, optional substituiertes C₁-C₆-Alkyl, -OH, -OR^{G}, -CO₂H, CO₂R^{G} ist, wobei R^{G} C₁-C₆-Alkyl, Aryl oder Heteroaryl, optional substituiert, eine geeignete Schutzgruppe, -NH₂ oder - N(R^{G})(R^{G}) ist, wobei R^{G} unabhängig ausgewählt ist, wie oben definiert, oder beide R^{G} zusammen mit dem Stickstoff, an den sie gebunden sind, ein 5- oder 6-gliedriges Heterocycloalkyl umfassen oder beide R^{G} zusammen eine geeignete Schutzgruppe bilden;
wobei R³⁸ Wasserstoff oder optional substituiertes C₁-C₆-Alkyl ist; R³⁹-R⁴⁴ unabhängig voneinander Wasserstoff, optional substituiertes C₁-C₆-Alkyl, optional substituiert, oder optional substituiertes Heteroaryl sind, oder beide R³⁹, R⁴⁰ zusammen mit dem Kohlenstoff, an den sie gebunden sind, ein C₃-C₆-Cycloalkyl umfassen, oder R⁴¹, R⁴² zusammen mit K, an das sie gebunden sind, wenn K C ist, oder R⁴³, R⁴⁴ zusammen mit L, an das sie gebunden sind, wenn L C ist, ein C₃-C₆-Cycloalkyl umfassen, oder R⁴⁰ und R⁴¹ oder R⁴⁰ und R⁴³ oder R⁴¹ und R⁴³ zusammen mit dem Kohlenstoffatom oder Heteroatom, an das sie gebunden sind, und Atomen, die zwischen diesen Kohlenstoff- und/oder Heteroatomen liegen, ein 5- oder 6-gliedriges Cycloalkyl oder Heterocycloalkyl umfassen,
oder wobei Aₒ eine Struktur aufweist, die einer alpha-Amino-, beta-Amino- oder einer anderen aminhaltigen Säure entspricht.

6. Zusammensetzung zur Verwendung nach einem der Ansprüche 1 bis 5, wobei der Index a 1 ist;
wobei -L_{b}-A a in Formel 1 -L_{b}-A ist, wenn Aₒ abwesend ist, oder -L_{b}-A -Lb-A1-Aₒ ist, wenn Aₒ vorhanden ist, sodass A zu A₁-Aₒ wird,
wobei L_{b} -Aₐ- die Struktur der Formel 8 hat: 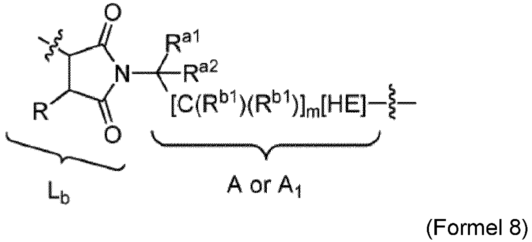
wobei
die -[C(R^{b1} )(R^{b1})]ₘ-[HE]-Einheit A oder A 1 ist;
R und R^{a2} unabhängig voneinander Wasserstoff oder Methyl sind;
R^{a1} Wasserstoff, Methyl, Ethyl ist oder R^{a1} eine basische Einheit (BU) ist, die an der basenunterstützten Hydrolyse des Succinimidrings beteiligt ist;
HE eine optionale Hydrolyseverstärker-Einheit (HE-Einheit, Hydrolysis Enhancer- HE) ist;
der Index m eine ganze Zahl im Bereich von 0 bis 6 ist;
jedes R^{b1} unabhängig Wasserstoff, optional substituiertes C₁-C₆ -Alkyl, optional substituiertes Aryl oder optional substituiertes Heteroaryl ist, oder zwei R^{b1} zusammen mit dem/den Kohlenstoffatom(en), an das/die sie gebunden sind, ein C₃-C₆-Cycloalkyl umfassen oder ein R^{b1} und HE zusammen mit dem Kohlenstoffatom, an das sie gebunden sind, ein 5- oder 6-gliedriges Cycloalkyl oder ein 5- oder 6-gliedriges Heterocycloalkyl umfassen und das andere R^{b1} Wasserstoff, optional substituiertes C₁-C₆-Alkyl, optional substituiertes Aryl oder optional substituiertes Heteroaryl ist;
BU die Struktur -[C(R¹)(R¹)]-[C(R² )(R²)]ₙ-N(R²²)(R²³) aufweist,
wobei der Index n 0, 1, 2 oder 3 ist;
jedes R¹ unabhängig Wasserstoff oder C₁-C₄-Alkyl ist oder zwei R¹ zusammen mit dem Kohlenstoffatom, an das sie gebunden sind, ein C₃-C₆-Cycloalkyl umfassen, und jedes R² unabhängig Wasserstoff, optional substituiertes C₁-C₆-Alkyl, optional substituiertes Aryl oder optional substituiertes Heteroaryl ist, oder zwei R² zusammen mit dem/den Kohlenstoffatom(en), an das/die sie gebunden sind, und allen dazwischenliegenden Kohlenstoffatomen ein C₃-C₆-Cycloalkyl definieren, oder ein R¹ und ein R² zusammen mit den Kohlenstoffatomen, an die sie gebunden sind, und allen dazwischenliegenden Kohlenstoffatome ein 5- oder 6-gliedriges Cycloalkyl umfassen und die verbleibenden R¹ und R² wie zuvor definiert sind;
R²² und R²³ unabhängig voneinander Wasserstoff oder optional substituiertes C₁-C₆-Alkyl sind oder zusammen mit dem Stickstoffatom, an das sie gebunden sind, ein 5- oder 6-gliedriges Heterocycloalkyl umfassen; und
wobei die Wellenlinie zum Succinimidring von L_{b} eine kovalente Bindung von Schwefel anzeigt, der von einer Sulfhydrylgruppe einer Zieleinheit abgeleitet ist, und die andere Wellenlinie eine kovalente Bindung von A (oder A₁ ) an den Rest der Zusammensetzungsstruktur anzeigt.

7. Zusammensetzung zur Verwendung nach Anspruch 1, wobei die Ligandenarzneimittelkonjugatverbindungen der Zusammensetzung durch die Strukturen der Formel 16A und der Formel 16B dargestellt sind: 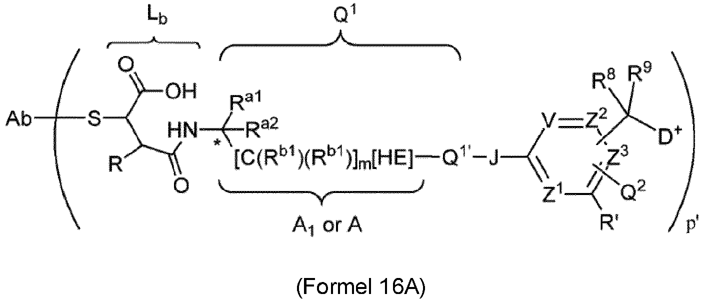 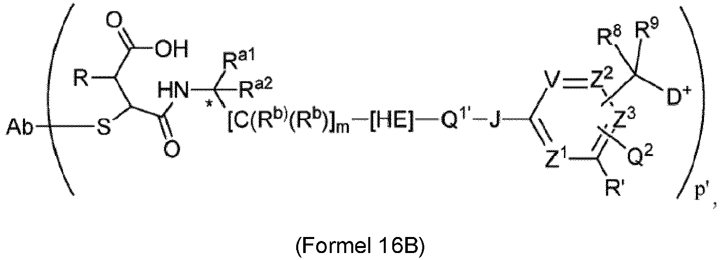
wobei Ab eine Antikörperligandeneinheit ist;
S ein Schwefelatom der Antikörperligandeneinheit ist;
das Sternchen (*) die Chiralität oder deren Abwesenheit am angezeigten Kohlenstoffatom angibt;
Q^{1'} Aₒ-W_{w} ist, wobei Aₒ eine optionale Untereinheit von A ist, sodass -Q^{1'}- -W_{w}- ist, wenn Aₒ abwesend ist, oder -Aₒ-W_{w}- ist, wenn Aₒ vorhanden ist,
wobei -[C(R^{b})(R^{b})]ₘ-[HE]- zu A wird, wenn Aₒ abwesend ist, oder A₁ ist, wenn Aₒ vorhanden ist, und
wobei der Index w 1 ist, wenn Q² abwesend ist, oder w 0 ist, sodass W abwesend ist und Q² vorhanden ist;
R Wasserstoff ist;
R^{a1} BU ist, wobei BU die Struktur -CH₂-N(R²²)(R²³) aufweist, oder eines Säureadditionssalzes davon, wobei R²² und R²³ unabhängig voneinander Wasserstoff oder Methyl sind oder beide zusammen mit dem Stickstoffatom, an das sie gebunden sind, ein 5- oder 6-gliedriges Heterocycloalkyl umfassen;
R^{a2} Wasserstoff ist;
der Index m eine ganze Zahl im Bereich von 0 bis 5 ist, wenn HE vorhanden ist, oder von 1 bis 5, wenn HE abwesend ist;
jedes R^{b1} unabhängig Wasserstoff oder optional substituiertes C₁-C₆-Alkyl ist;
HE abwesend ist oder -C(=O)- ist;
J -O- oder -NH- ist;
Q², wenn vorhanden, W'_{w'}-E ist, wobei E -O- oder -NH- ist; und
der Index p' eine ganze Zahl im Bereich von 1 bis 8 ist.

8. Zusammensetzung zur Verwendung nach Anspruch 7, wobei die Ligandenarzneimittelkonjugatverbindungen der Zusammensetzung die Strukturen der Formel 17A und der Formel 17B aufweisen: 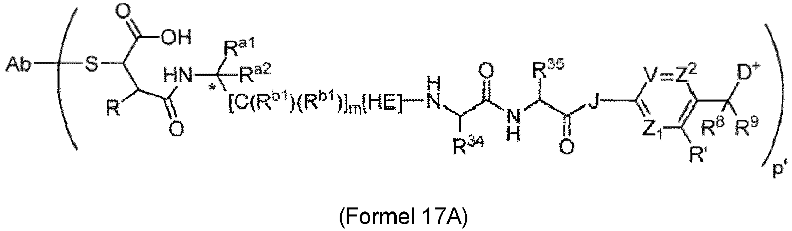 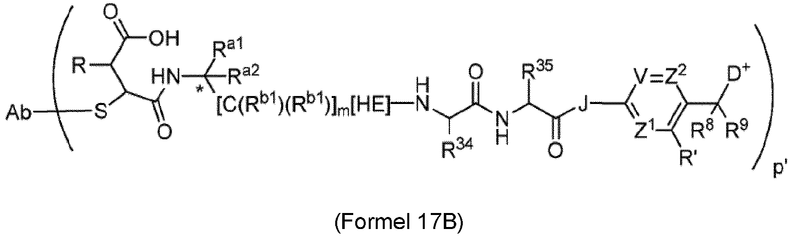
wobei J -NH- ist;
eines von V oder Z¹ =C(R²⁴)- ist, wobei R²⁴ Wasserstoff, -C1 oder -NO₂ ist und die anderen von V, Z¹ und Z² =CH- sind;
R⁸ Wasserstoff ist;
R⁹ Wasserstoff oder Methyl ist; und
der Index p' eine ganze Zahl im Bereich von 1 bis 8 ist.

9. Zusammensetzung zur Verwendung nach Anspruch 7, wobei die Ligandenarzneimittelkonjugatverbindungen der Zusammensetzung die Strukturen der Formel 18A und der Formel 18B aufweisen: 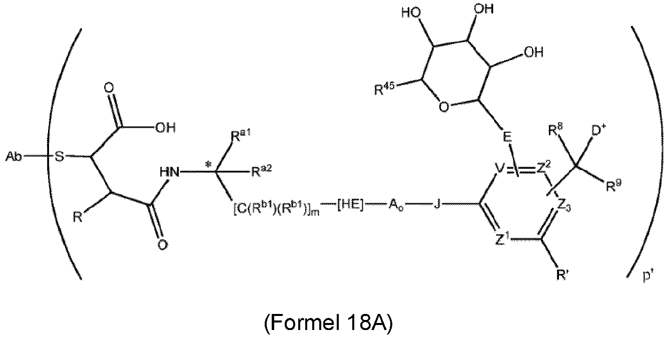 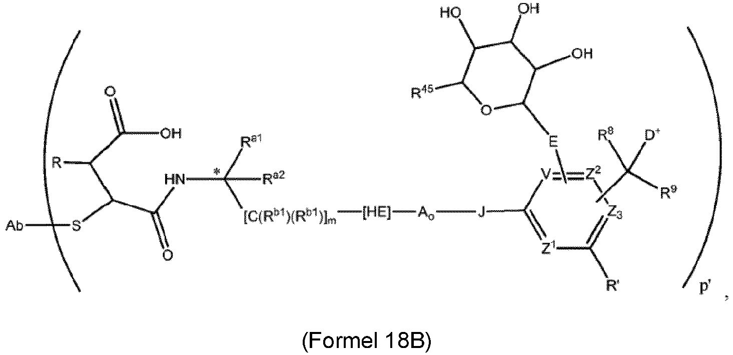
wobei Aₒ eine optionale Untereinheit von A ist, wobei die -[C(R^{b1})(R^{b1})]ₘ-[HE]-Einheit A ist, wenn Aₒ abwesend ist, oder die Einheit A₁ ist, wenn Aₒ vorhanden ist, sodass A zu A₁-Aₒ- wird;
R -H ist;
R^{a1} eine basische Einheit (BU) ist, wobei BU die Struktur -CH₂-N(R²²)(R²³) aufweist, oder eines Säureadditionssalzes davon, wobei R²² und R²³ unabhängig voneinander Wasserstoff oder Methyl sind oder beide zusammen mit dem Stickstoffatom, an das sie gebunden sind, ein 5- oder 6-gliedriges Heterocycloalkyl umfassen;
R^{a2} Wasserstoff ist;
der Index m eine ganze Zahl im Bereich von 0 bis 5 ist, wenn HE vorhanden ist, oder von 1 bis 5, wenn HE abwesend ist;
jedes R^{b1} unabhängig Wasserstoff oder optional substituiertes C₁-C₆-Alkyl ist;
HE abwesend ist oder -C(=O)- ist; und
R⁸ Wasserstoff ist; und
R⁹ Wasserstoff, optional substituiertes C₁-C₆-Alkyl oder optional substituiertes Phenyl ist;
insbesondere wobei die Ligandenarzneimittelkonjugatverbindungen der Zusammensetzung die Strukturen der Formel 19A und der Formel 19B aufweisen: 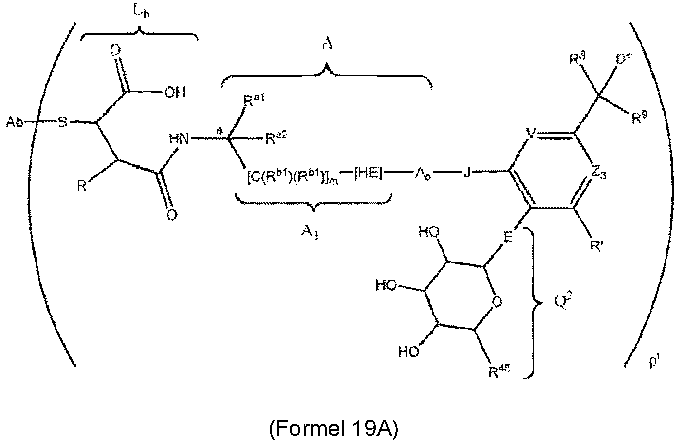 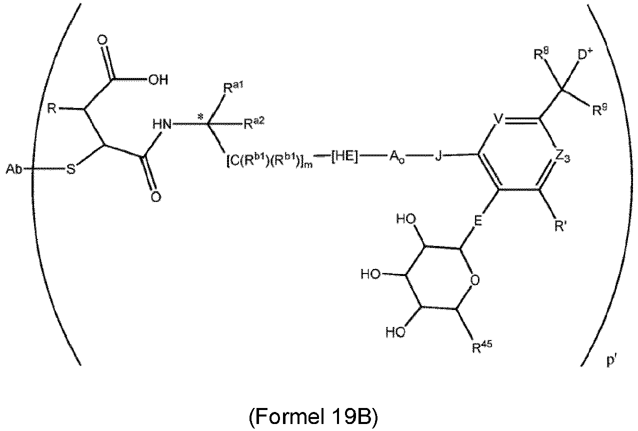
wobei
R Wasserstoff ist;
R^{a1} eine basische Einheit (BU) ist, wobei BU die Struktur -CH₂-N(R²²)(R²³) aufweist, oder eines Säureadditionssalzes davon, wobei R²² und R²³ unabhängig voneinander Wasserstoff oder Methyl sind oder beide zusammen mit dem Stickstoffatom, an das sie gebunden sind, ein 5- oder 6-gliedriges Heterocycloalkyl umfassen;
R^{a2} Wasserstoff ist;
der Index m eine ganze Zahl im Bereich von 0 bis 5 ist, wenn HE vorhanden ist, oder von 1 bis 5, wenn HE abwesend ist;
jedes R^{b1} unabhängig Wasserstoff oder optional substituiertes C₁-C₆-Alkyl ist;
HE abwesend ist oder -C(=O)- ist;
R⁴⁵ -CO₂H ist;
E -O- ist;
J -NH- ist;
V und Z³ =CH- sind;
R⁸ Wasserstoff ist;
R⁹ Wasserstoff oder Methyl ist; und
das mit (*) gekennzeichnete Kohlenstoffatom vorwiegend in der gleichen absoluten Konfiguration vorliegt wie das Alpha-Kohlenstoffatom einer L-Aminosäure, wenn das gekennzeichnete Kohlenstoffatom Chiralität aufweist.

10. Zusammensetzung zur Verwendung nach einem der Ansprüche 1 bis 9, wobei -D⁺ ein quaternisiertes tertiäres aminhaltiges Tubulin-Disruptionsmittel ist,
insbesondere wobei die quaternisierte Tubulin-störende Arzneimitteleinheit -D⁺ eine quaternisierte Tubulysin-Arzneimitteleinheit ist,
insbesondere wobei die quaternisierte Tubulysin-Arzneimitteleinheit -D⁺ die Struktur der Formel D_{G-1'} oder der Formel D_{H-1'} aufweist: 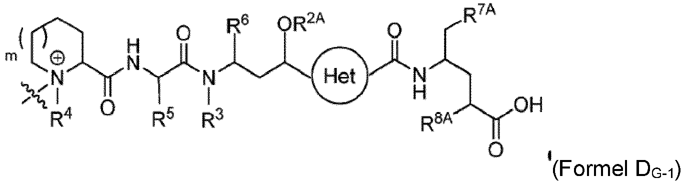 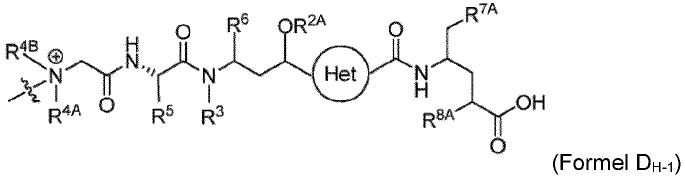
wobei der Kreis ein 5-gliedriges Stickstoff-Heteroaryl darstellt und wobei die angegebenen erforderlichen Substituenten zu diesem Heteroaryl in einer 1,3-Beziehung zueinander stehen, bei optionaler Substitution an den verbleibenden Positionen;
R^{2A} Wasserstoff oder optional substituiertes Alkyl ist oder R^{2A} zusammen mit dem Sauerstoffatom, an das es gebunden ist, einen von -OH unterschiedlichen O-verknüpften Substituenten definiert;
R³ Wasserstoff oder optional substituiertes Alkyl ist;
R⁴, R^{4A} R^{4B}, R⁵ und R⁶ optional substituiertes Alkyl sind, unabhängig ausgewählt;
R^{7A} optional substituiertes Aryl oder optional substituiertes Heteroaryl ist;
R^{8A} Wasserstoff oder optional substituiertes Alkyl ist; und
m 0 oder 1 ist,
wobei die Wellenlinie die kovalente Bindung von D⁺ an den Rest der Zusammensetzungsstruktur anzeigt.

11. Zusammensetzung zur Verwendung nach Anspruch 10, wobei die quaternisierte Tubulysin-Arzneimitteleinheit -D⁺ die Struktur aufweist: 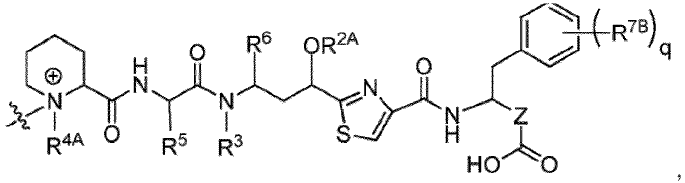
wobei Z ein optional substituiertes C₁-C₄-Alkylen oder ein optional substituiertes C₂-C₄ - Alkenylen ist;
der Index q, der die Anzahl der R^{7B}-Substituenten angibt, 1, 2 oder 3 ist;
wobei jedes R^{7B} unabhängig aus der Gruppe ausgewählt ist, die aus Wasserstoff und einem O-verknüpften Substituenten besteht; und
wobei die Wellenlinie die kovalente Bindung von D⁺ an den Rest der Zusammensetzungsstruktur anzeigt,
oder wobei die quaternisierte Tubulysin-Arzneimitteleinheit -D⁺ die folgende Struktur aufweist: 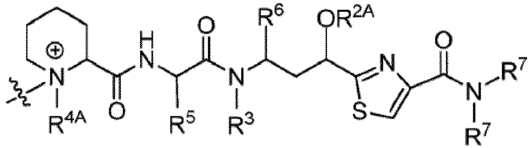
wobei R^{2A} Wasserstoff oder optional substituiertes C₁-C₆-Alkyl ist oder R^{2A} zusammen mit dem Sauerstoffatom, an das es gebunden ist, einen von -OH unterschiedlichen O-verknüpften Substituenten definiert;
R³ optional substituiertes C₁-C₆-Alkyl ist;
R⁵ und R⁶ die Seitenkettenreste natürlicher hydrophober Aminosäuren sind;
-N(R⁷)(R⁷) -NH(C₁-C₆-Alkyl) oder -NH-N(C₁-C₆-Alkyl)₂ ist, wobei optional genau ein C₁-C₆-Alkyl substituiert ist durch -CO₂H oder einen Ester davon oder durch ein optional substituiertes Phenyl; und
wobei die Wellenlinie die kovalente Bindung von D⁺ an den Rest der Zusammensetzungsstruktur anzeigt,
insbesondere wobei -N(R⁷)(R⁷) ausgewählt ist aus der Gruppe bestehend aus -NH(CH₃), - NHCH₂CH₂Ph und -NHCH₂-CO₂H, -NHCH₂CH₂CO₂H und -NHCH₂CH₂CH₂CO₂H,
insbesondere wobei der O-verknüpfte Substituent -OR^{2A} von -OH verschieden ist, sodass R^{2A} nicht Wasserstoff ist,
insbesondere wobei R^{2A} -CH₂CH₃ ist.

12. Zusammensetzung zur Verwendung nach Anspruch 11, wobei die quaternisierte Tubulysin-Arzneimitteleinheit -D⁺ die folgende Struktur aufweist: 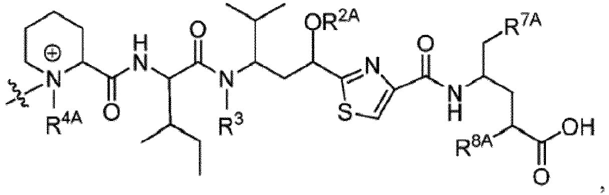 insbesondere wobei die quaternisierte Tubulysin-Arzneimitteleinheit -D⁺ die folgende Struktur aufweist: 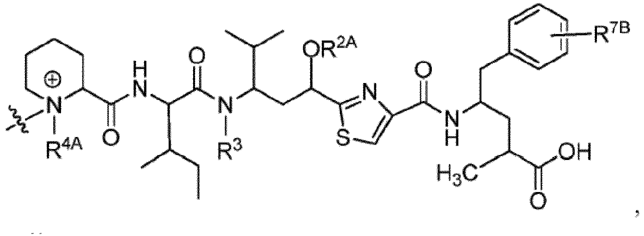
wobei R^{4A} Methyl ist;
R³ H, Methyl, Ethyl, Propyl, -CH₂-OC(O)R^{3A}, -CH₂CH(R^{3B})C(O)R^{3A} oder -CH(R^{3B})C(O)NHR^{3A} ist, wobei R^{3A} C₁-C₆-Alkyl ist und R^{3B} H oder C₁-C₆-Alkyl ist, unabhängig ausgewählt aus R^{3A} ; und
-OR^{2A} ein O-verknüpfter Substituent ist, ausgewählt aus der Gruppe bestehend aus -OR^{2B}, - C(O)R^{2B} und -OC(O)N(R^{2B})(R^{2C}), wobei R^{2B} und R^{2C} unabhängig ausgewählt sind aus der Gruppe bestehend aus H, C₁-C₆-Alkyl und C₂-C₆-Alkenyl; und
R^{7B} Wasserstoff oder -OH ist,
insbesondere wobei R^{2A} -CH₂CH₃ ist.

13. Zusammensetzung zur Verwendung nach Anspruch 10, wobei die quaternisierte Tubulysin-Arzneimitteleinheit -D⁺ die folgende Struktur aufweist: 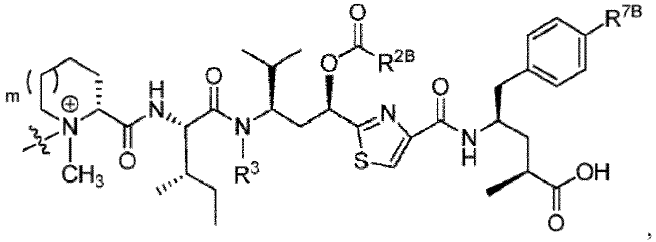
wobei der Index m 1 ist;
R^{2B}-CH₃, -CH₂CH₃, -CH(CH₃)₂, -C(CH₃)₃ oder-CH=CH 2 ist;
R³ Methyl, Ethyl oder Propyl ist; und
R^{7B} Wasserstoff oder -OH ist;
oder wobei die quaternisierte Tubulysin-Arzneimitteleinheit -D⁺ die folgende Struktur aufweist: 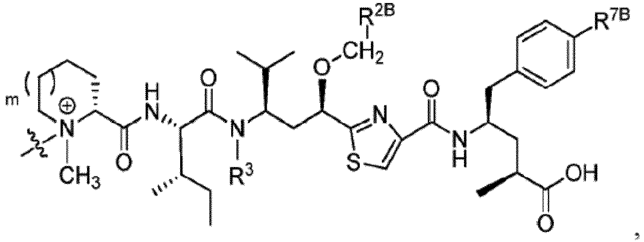
wobei der Index m 1 ist;
R^{2B} -H, -CH₃ , -CH₂CH₃ oder -OCH₃ ist;
R³ Methyl, Ethyl oder Propyl ist; und
R^{7B} Wasserstoff oder -OH ist,
oder wobei die quaternisierte Tubulysin-Arzneimitteleinheit -D⁺ die folgende Struktur aufweist: 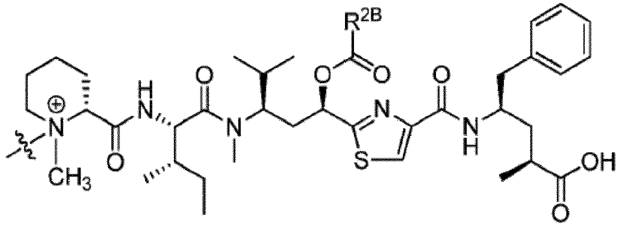 oder 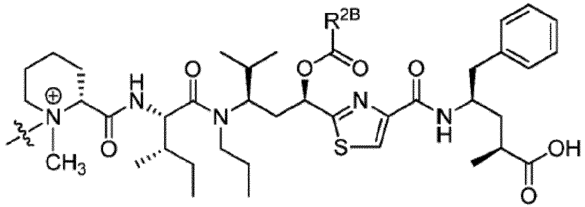
wobei R^{2B} -CH₃, -CH₂CH₃, -CH₂CH₂CH₃, -CH(CH₃)₂, -CH₂CH(CH₃)₂, -CH₂C(CH₃)₃ ist,
oder 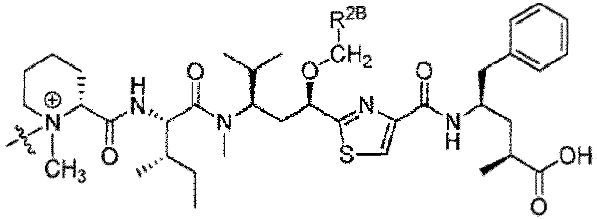 oder 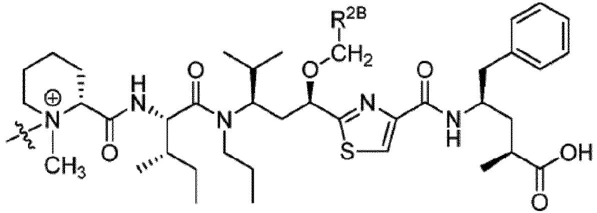
wobei R^{2B} Wasserstoff, Methyl oder -OCH₃ ist, sodass -CH₂R^{2B} ein Methyl, Ethyl- oder Methoxymethylether-Substituent ist,
insbesondere wobei R^{2B} Methyl ist.

14. Zusammensetzung zur Verwendung nach Anspruch 1, wobei die Zusammensetzung durch die folgende Struktur dargestellt wird: 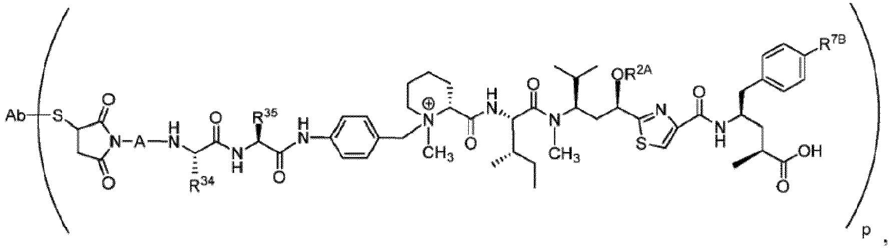
wobei Ab eine Antikörperligandeneinheit ist;
S ein Schwefelatom der Antikörperligandeneinheit ist;
R³⁴ Isopropyl ist und R³⁵ -CH₃, Isopropyl, -CH₂CH₂CH₂NH(C=O)NH₂ oder -CH₂CH₂CO₂H ist;
R^{7B} Wasserstoff oder -OH ist;
R^{2A} C₁-C₆-Alkyl, C₂-C6-Alkenyl, -OCH₂OR^{2B}, -C(=O)R^{2B} oder -C(=O)NHR^{2B} ist, wobei R^{2B} Wasserstoff oder C₁-C₆-Alkyl ist; und
der Index p eine Zahl im Bereich von 1 bis 8 ist,
insbesondere wobei
R³⁴ Isopropyl ist und R³⁵ -CH₃ oder -CH₂CH₂CH₂ NH(C=O)NH₂ ist;
R^{7B} Wasserstoff oder -OH ist; und
R^{2A} C₁-C₄-Alkyl, -C(=O)R^{2B} oder -C(=O)NHR^{2B} ist, wobei R^{2B} C₁-C₄-Alkyl ist,
insbesondere wobei A -CH₂(CH₂)₄(C=O)- oder -CH₂(CH₂)₄(C=O)NHCH₂CH₂(C=O)- ist,
insbesondere wobei die Struktur der Zusammensetzung durch die folgende Struktur dargestellt wird: 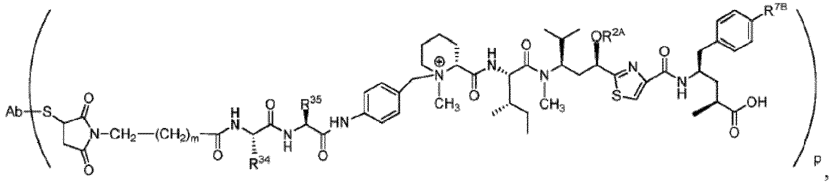
wobei Ab eine Antikörperligandeneinheit ist;
S ein Schwefelatom der Antikörperligandeneinheit ist;
der Index p eine Zahl im Bereich von 1 bis 8 ist; und
der Index m 4 ist,
insbesondere wobei R^{2A} -C(O)CH₃, Methyl, Ethyl oder Propyl ist.

15. Zusammensetzung zur Verwendung nach Anspruch 1, wobei die Zusammensetzung durch die folgende Struktur dargestellt wird: 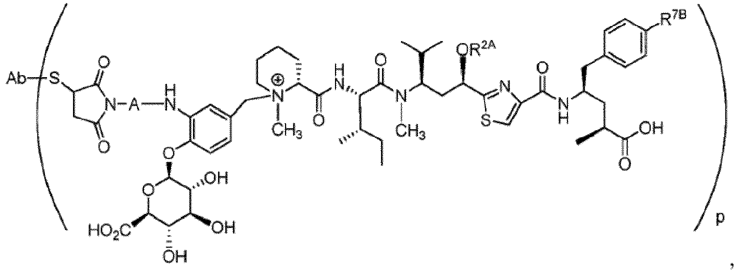
wobei Ab eine Antikörperligandeneinheit ist;
S ein Schwefelatom der Antikörperligandeneinheit ist;
R^{7B} Wasserstoff oder -OH ist;
R^{2A} C₁-C⁶-Alkyl, -OCH₂OR^{2B}, -C(=O)R^{2B} oder -C(=O)NHR^{2B} ist, wobei R^{2B} C₁-C₆-Alkyl oder C₂-C₆-Alkenyl ist; und
der Index p eine Zahl im Bereich von 1 bis 8 ist,
insbesondere wobei
R^{7B} Wasserstoff oder -OH ist; und
R^{2A} C₁-C₄-Alkyl, -C(=O)R^{2B} oder -C(=O)NHR^{2B} ist, wobei R^{2B} C₁-C₄-Alkyl ist,
insbesondere wobei A -CH₂(CH₂)₄(C=O)- oder -CH₂(CH₂)₄(C=O)NHCH₂CH₂(C=O)- ist,
insbesondere wobei die Struktur der Zusammensetzung durch die folgende Struktur dargestellt wird: 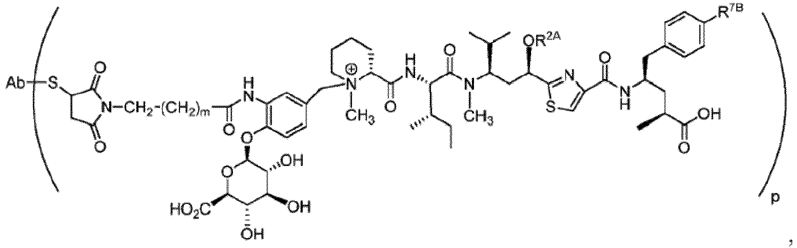
wobei Ab eine Antikörperligandeneinheit ist;
S ein Schwefelatom der Antikörperligandeneinheit ist;
der Index p eine Zahl im Bereich von 1 bis 8 ist; und
der Index m 4 ist,
insbesondere wobei R^{2A} -C(O)CH₃, Methyl, Ethyl oder Propyl ist.

16. Zusammensetzung zur Verwendung nach Anspruch 1, wobei jede Ligandenarzneimittelkonjugatverbindung der Zusammensetzung durch die folgende Struktur dargestellt wird: 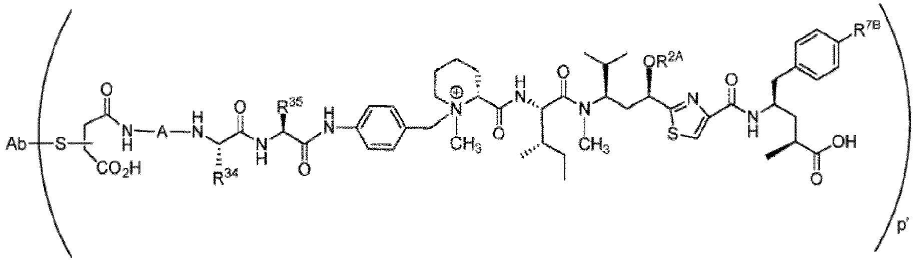
wobei Ab eine Antikörperligandeneinheit ist;
S ein Schwefelatom der Antikörperligandeneinheit ist;
die Ab-S-Einheit an den Kohlenstoff α oder β an der Carbonsäure gebunden ist;
R³⁴ Isopropyl ist und R³⁵-CH₃ oder -CH₂CH₂CH₂NH(C=O)NH₂ ist;
R^{7B} Wasserstoff oder -OH ist;
R^{2A} C₁-C₄ -Alkyl, -C(=O)R^{2B} oder -C(=O)NHR^{2B} ist, wobei R^{2B} C₁-C₄-Alkyl ist; und
der Index p' eine ganze Zahl im Bereich von 1 bis 8 ist,
insbesondere wobei A -CH₂(CH₂)₄(C=O)- oder -CH₂(CH₂)₄(C=O)NHCH₂CH₂(C=O)- ist,
insbesondere wobei jede Ligandenarzneimittelkonjugatverbindung der Zusammensetzung durch die folgende Struktur dargestellt wird: 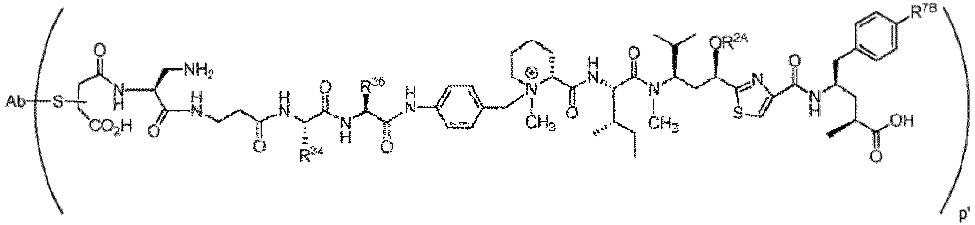 oder 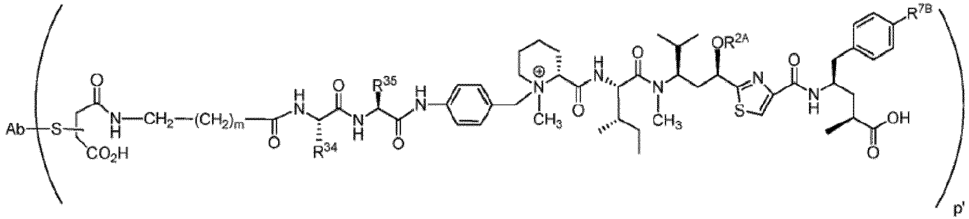
wobei Ab eine Antikörperligandeneinheit ist;
S ein Schwefelatom der Antikörperligandeneinheit ist;
die Ab-S-Einheit an den Kohlenstoff α oder β an der Carbonsäure gebunden ist;
der Index p' eine ganze Zahl im Bereich von 1 bis 8 ist; und
der Index m 4 ist,
insbesondere wobei R^{2A} -C(O)CH₃, Methyl, Ethyl oder Propyl ist.

17. Zusammensetzung zur Verwendung nach Anspruch 1, wobei jede Ligandenarzneimittelkonjugatverbindung der Zusammensetzung durch die folgende Struktur dargestellt wird: 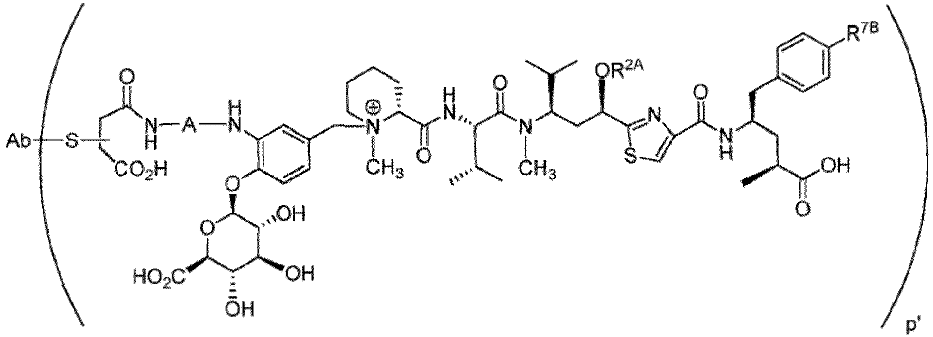 wobei
wobei Ab eine Antikörperligandeneinheit ist;
S ein Schwefelatom der Antikörperligandeneinheit ist;
die Ab-S-Einheit an den Kohlenstoff α oder β an der Carbonsäure gebunden ist;
R^{7B} Wasserstoff oder -OH ist;
R^{2A} C₁-C₄-Alkyl, -C(=O)R^{2B} oder -C(=O)NHR^{2B} ist, wobei R^{2B} C₁-C₄-Alkyl ist;
und der Index p' eine ganze Zahl im Bereich von 1 bis 8 ist,
insbesondere wobei A -CH₂(CH₂)₄(C=O)- oder -CH₂(CH₂)₄(C=O)NHCH₂CH₂(C=O)- ist,
insbesondere wobei jede Ligandenarzneimittelkonjugatverbindung der Zusammensetzung durch die folgende Struktur dargestellt wird: 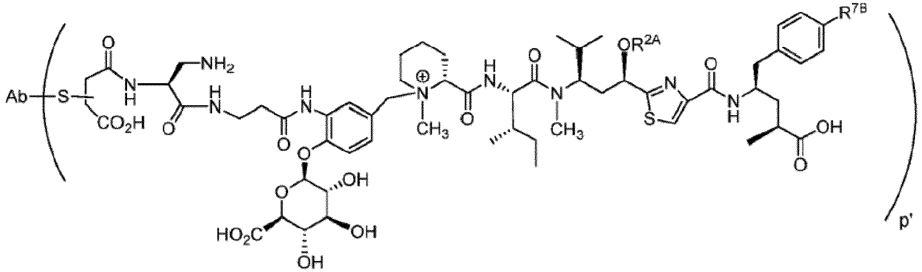 oder 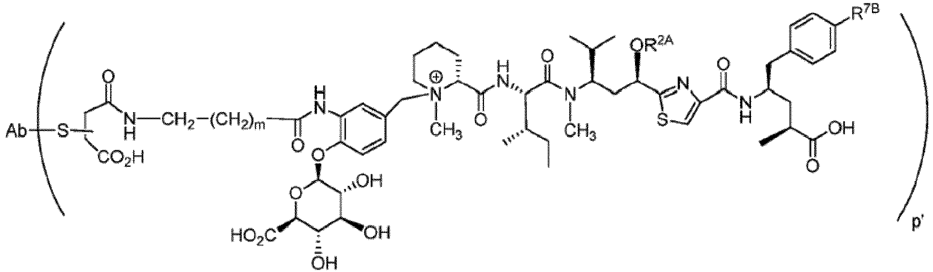
wobei Ab eine Antikörperligandeneinheit ist;
S ein Schwefelatom der Antikörperligandeneinheit ist;
die Ab-S-Einheit an den Kohlenstoff α oder β an der Carbonsäure gebunden ist;
der Index p' eine ganze Zahl im Bereich von 1 bis 8 ist; und
der Index m 4 ist,
insbesondere wobei R^{2A} -C(O)CH₃, Methyl, Ethyl oder Propyl ist.

18. Zusammensetzung zur Verwendung nach Anspruch 1, wobei -D⁺ eine quaternisierte Auristatin-Arzneimitteleinheit oder eine quaternisierte Dolastatin-Arzneimitteleinheit ist,
insbesondere wobei die quaternisierte Dolastatin-Arzneimitteleinheit quaternisiertes Dolastatin 10 oder quaternisiertes Dolastatin 15 ist,
insbesondere wobei die quaternisierte Auristatin-Arzneimitteleinheit -D⁺ die Struktur D_{E'} oder D_{F}' aufweist: 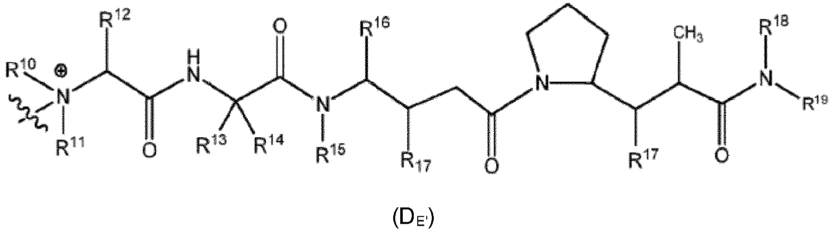 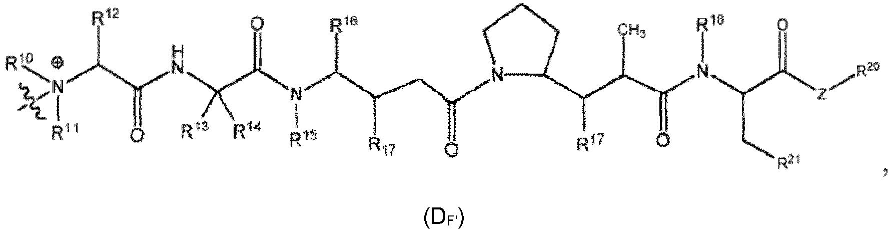
wobei R¹⁰ und R¹¹ unabhängig voneinander C₁-C₈-Alkyl sind;
R¹² Wasserstoff, C₁-C₈-Alkyl, C₃-C₈ -Cycloalkyl, Aryl, -X¹-Aryl, -X¹-(C₃-C₈-Cycloalkyl), C₃-C₈-Heterocyclus oder -X¹-(C₃-C₈-Heterocyclus) ist;
R¹³ Wasserstoff, C₁-C₈-Alkyl, C₃-C₈ -Cycloalkyl, Aryl, -X¹-Aryl, -X¹-(C₃-C₈-Cycloalkyl), C₃-C₈-Heterocyclus oder -X¹-(C₃-C₈-Heterocyclus) ist;
R¹⁴ Wasserstoff oder Methyl ist;
oder R¹³ und R¹⁴ zusammen mit dem Kohlenstoff, an das sie gebunden sind, ein C₃-C₈-Cycloalkyl umfassen;
R¹⁵ Wasserstoff oder C₁-C₈-Alkyl ist;
R₁₆ Wasserstoff, C₁-C₈-Alkyl, C₃-C₈-Cycloalkyl, Aryl, -X¹-Aryl, -X¹-(C₃-C₈-Cycloalkyl), C₃-C₈-Heterocyclus oder -X¹-(C₃-C₈-Heterocyclus) ist;
R¹⁷ unabhängig voneinander Wasserstoff, -OH, C₁-C₈-Alkyl, C₃-C₈-Cycloalkyl oder O-(C₁-C₈-Alkyl) sind;
R¹⁸ unabhängig voneinander Wasserstoff oder C₁-C₈-Alkyl sind;
R¹⁹ -C(R^{19A})₂-C(R^{19A})₂-Aryl, -C(R^{19A})₂-C(R^{19A})₂-(C₃-C₈-Heterocyclus) oder -C(R^{19A})₂-C(R^{19A})₂-(C₃-C₈-Cycloalkyl) ist;
R²¹ ein Aryl oder C₃-C₈-Heterocyclus ist; wobei R^{19A} Wasserstoff, C₁-C₈-Alkyl oder -OH ist;
R²⁰ Wasserstoff, C₁-C₂₀-Alkyl, Aryl, C₃-C₈-Heterocyclus, -(R⁴⁷O)ₘ-R⁴⁸ oder -(R⁴⁷O)ₘ-CH(R⁴⁹)₂ ist;
der Index m eine ganze Zahl im Bereich von 1 bis 1000 ist;
R⁴⁷ C₂-C₈-Alkyl ist;
R⁴⁸ Wasserstoff oder C₁-C₈-Alkyl ist;
R⁴⁹ unabhängig voneinander -COOH, -(CH₂)ₙ-N(R⁵⁰)₂ , -(CH₂)ₙ-SO₃H oder -(CH₂)ₙ-SO₃-C₁-C₈-Alkyl sind;
R⁵⁰ unabhängig voneinander C₁-C₈-Alkyl oder -(CH₂)ₙ-COOH sind;
Z, O, S, NH oder NR⁴⁶ ist, wobei R⁴⁶ C₁-C₈-Alkyl ist;
X¹ C₁-C₁₀-Alkylen ist; und
der Index n eine ganze Zahl im Bereich von 0 bis 6 ist;
wobei die Wellenlinie die kovalente Bindung von D⁺ an den Rest der Zusammensetzungsstruktur anzeigt,
insbesondere wobei jede Ligandenarzneimittelkonjugatverbindung der Zusammensetzung durch die folgende Struktur dargestellt wird: 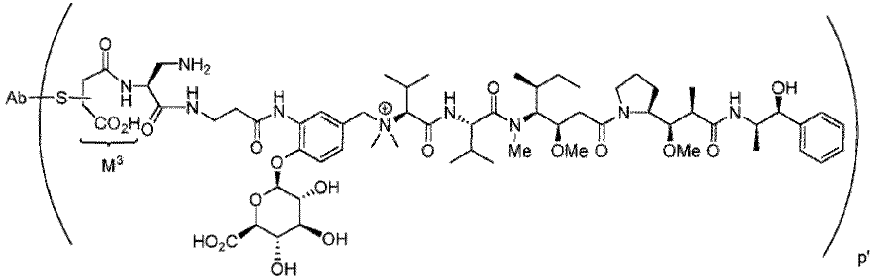 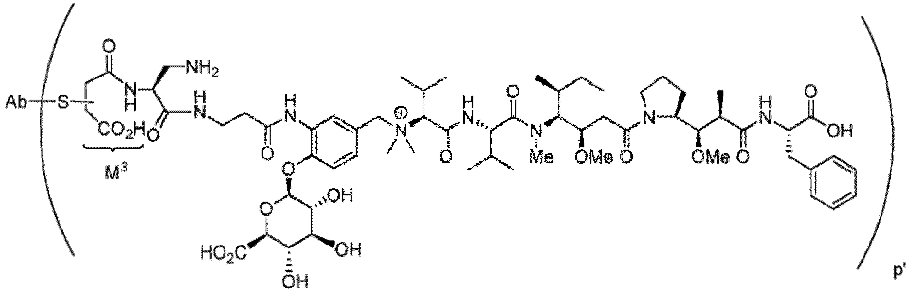 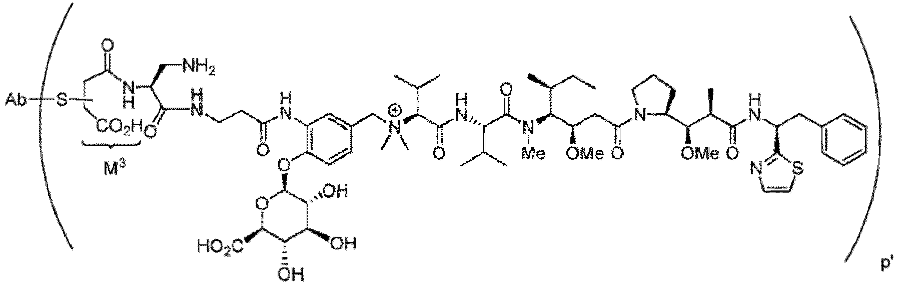 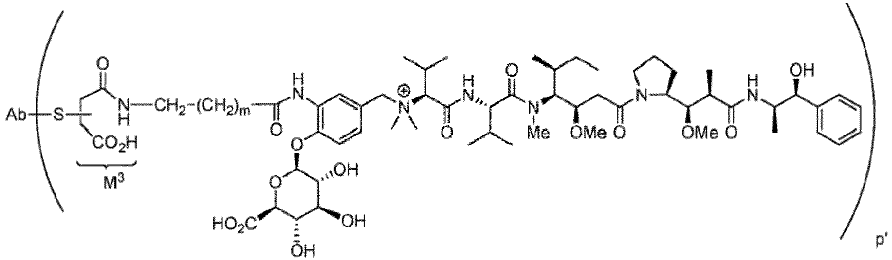 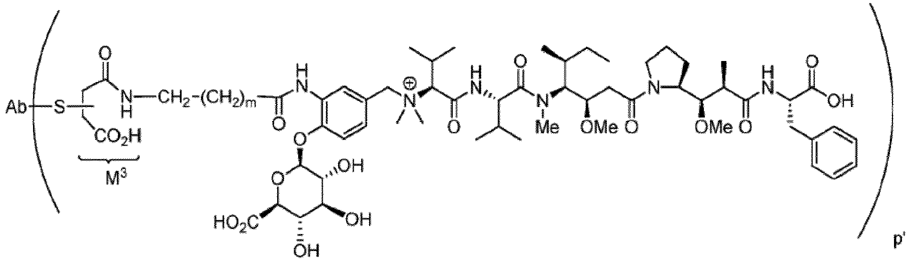 oder 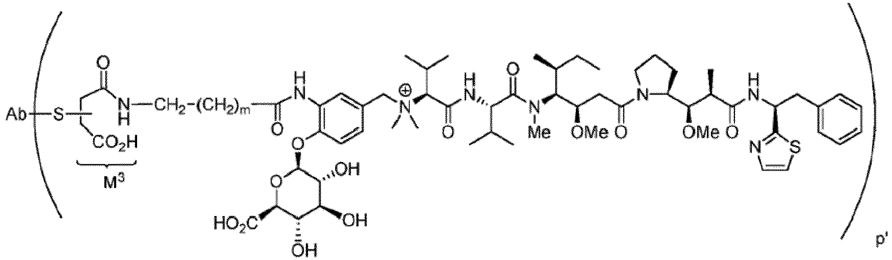
wobei Ab eine Antikörperligandeneinheit ist;
S ein Schwefelatom der Antikörperligandeneinheit ist;
die Ab-S-Einheit an den Kohlenstoff α oder β an der Carbonsäure gebunden ist; und
der Index p' eine ganze Zahl im Bereich von 1 bis 8 ist.

19. Zusammensetzung zur Verwendung nach einem der Ansprüche 1 bis 18, wobei die Verwendung die Behandlung eines Krebses ist.

20. Zusammensetzung zur Verwendung nach Anspruch 19, wobei der Krebs ein solider Tumor, ein durch Blut übertragener Krebs, eine akute Leukämie, eine chronische Leukämie oder ein Lymphom ist.

21. Zusammensetzung zur Verwendung nach Anspruch 20, wobei der solide Tumor ausgewählt ist aus der Gruppe bestehend aus Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiomyosarkom, Rhabdomyosarkom, Dickdarmkrebs, kolorektales Karzinom, Nierenkrebs, Bauchspeicheldrüsenkrebs, Knochenkrebs, Brustkrebs, Eierstockkrebs, Prostatakrebs, Speiseröhrenkrebs, Magenkrebs, Mundhöhlenkrebs, Nasenkrebs, Kehlkopfkrebs, Plattenepithelkarzinom, Basalzellkarzinom, Adenokarzinom, Schweißdrüsenkarzinom, Talgdrüsenkarzinom, Papillarkarzinom, papilläre Adenokarzinome, Zystadenokarzinom, medulläres Karzinom, Bronchialkarzinom, Nierenzellkarzinom, Hepatom, Gallengangskarzinom, Choriokarzinom, Seminom, Embryonalkarzinom, Wilms-Tumor, Gebärmutterhalskrebs, Hodenkrebs, kleinzelliges Lungenkarzinom, Blasenkarzinom, Lungenkrebs, Epithelkarzinom, Gliom, Glioblastoma multiforme, Astrozytom, Medulloblastom, Kraniopharyngiom, Ependymom, Pinealom, Hämangioblastom, Akustikusneurinom, Oligodendrogliom, Meningiom, Hautkrebs, Melanom, Neuroblastom und Retinoblastom.

22. Zusammensetzung zur Verwendung nach Anspruch 20, wobei der Krebs ein durch Blut übertragener Krebs ist, ausgewählt aus der Gruppe bestehend aus akuter lymphoblastischer Leukämie, akuter lymphoblastischer B-Zell-Leukämie, akuter lymphoblastischer T-Zell-Leukämie, akuter myeloblastischer Leukämie, akuter promyelozytischer Leukämie, akute monoblastische Leukämie, akute erythroleukämische Leukämie, akute megakaryoblastische Leukämie, akute myelomonozytäre Leukämie, akute nicht lymphozytäre Leukämie, akute undifferenzierte Leukämie, chronische myelozytäre Leukämie, chronische lymphozytäre Leukämie, Haarzellenleukämie und multiples Myelom.

23. Zusammensetzung zur Verwendung nach Anspruch 20, wobei das Lymphom ausgewählt ist aus der Gruppe bestehend aus Morbus Hodgkin, Non-Hodgkin-Lymphom, multiplem Myelom, Waldenstrom-Makroglobulinämie, Schwerkettenkrankheit und Polycythemia vera.

24. Zusammensetzung zur Verwendung nach einem der Ansprüche 1 bis 18, wobei die Verwendung die Behandlung einer Autoimmunerkrankung ist.

## Revendications

1. Composition de conjugué ligand-substance médicamenteuse (LDC) pour utilisation dans le traitement d'un cancer ou d'une maladie auto-immune, la composition de LDC étant représentée par la structure de formule 1 : 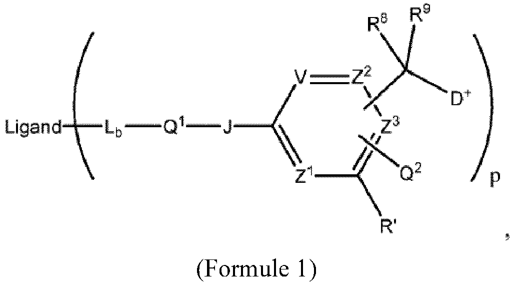 dans laquelle
« Ligand » est une unité ligand (L), L étant capable de se lier sélectivement à une entité cible, et L est un anticorps ou fragment d'anticorps pour définir un conjugué anticorps-substance médicamenteuse (ADC), l'entité cible étant un antigène capable de se lier sélectivement à l'ADC, et l'antigène étant une protéine, une glycoprotéine ou un glucide, de surface cellulaire accessible de façon extracellulaire, présenté(e) préférentiellement par des cellules anormales en comparaison de cellules normales, les cellules anormales et les cellules normales étant des cellules chez un mammifère, les cellules anormales étant des cellules hyper-proliférantes ;
L_{b} est un segment de liaison primaire ;
Q¹ est Aₐ-W_{w}, où A est une unité d'espacement optionnelle, de sorte que l'indice a est 0 lorsque A est absent ou 1 lorsque A est présent et comprend deux, trois ou quatre sous-unités ;
Q² est W'_{w'}-E-, Q², lorsqu'il est présent, étant lié à V, Z¹, Z², ou Z³ ;
W_{w} et W'_{w'} sont des unités clivables,
W_{w} de Q¹ étant capable de clivage sélectif par une protéase régulatrice ou intracellulaire en comparaison des protéases sériques, ou par échange de disulfures par l'intermédiaire du glutathion, ou étant plus réactif à l'hydrolyse dans des conditions plus acides présentes dans les lysosomes en comparaison du pH physiologique du sérum,
W'_{w'}-E de Q² fournit une liaison glycosidique clivable par une glycosidase localisée à l'intérieur de la cellule, et
l'indice w est 0 ou 1 de sorte que W_{w} est absent lorsque w est 0 ou W_{w} est présent lorsque w est 1, et l'indice w' est 0 ou 1, W'_{w'}-E étant absent lorsque w' est 0 ou W'_{w'}-E étant présent lorsque w' est 1, et la somme w + w' étant égale à 1 de sorte qu'un et seulement un de W_{w}, W'_{w'}-E est présent ;
V, Z¹, Z², et Z³ représentent =N- ou =C(R²⁴)-, R²⁴ étant un atome d'hydrogène ou d'halogène, un groupe alkyle, alcényle ou alcynyle éventuellement substitué, -NO₂, -CN, un autre groupe attirant les électrons, un groupe donneur d'électrons, -Q², ou -C(R⁸)(R⁹)-D⁺, au moins un parmi V, Z¹, Z² et Z³ étant =C(R²⁴)- lorsque w est 1, et au moins deux parmi V, Z¹, Z², et Z³ étant =C(R²⁴)- lorsque w' est 1 ;
étant entendu que lorsque w est 1, Q² est absent, un et seulement un R²⁴ est -C(R⁸)(R⁹)-D⁺, de sorte que C(R⁸)(R⁹)-D⁺ est lié à l'un de V, Z¹, Z², Z³ lorsque ce groupe variable est =C(R²⁴)-, et les substituants Q¹-J- et -C(R⁸)(R⁹)-D⁺ sont en position *ortho* ou *para* l'un par rapport à l'autre ;
étant entendu que lorsque w' est 1, un et seulement un R²⁴ est -C(R⁸)(R⁹)-D⁺, de sorte que -C(R⁸)(R⁹)-D⁺ est lié à l'un de V, Z¹, Z², Z³ lorsque ce groupe variable est =C(R²⁴)-, et un et seulement un R²⁴ est Q² de sorte que Q² est lié à un autre de V, Z¹, Z², Z³ lorsque ce groupe variable est =C(R²⁴)-, et les substituants Q² et -C(R⁸)(R⁹)-D⁺ sont en position *ortho* ou *para* l'un par rapport à l'autre ;
R⁸ et R⁹ représentent indépendamment un atome d'hydrogène ou un groupe alkyle, alcényle, alcynyle, aryle, ou hétéroaryle, éventuellement substitué ;
R' est un atome d'hydrogène ou d'halogène, un groupe -NO₂, -CN, un autre groupe attirant les électrons, ou un groupe donneur d'électrons ;
E et J représentent indépendamment -O-, -S-, ou -N(R³³)-, R³³ étant un atome d'hydrogène ou un groupe alkyle éventuellement substitué ;
D⁺ représente une structure d'une substance médicamenteuse quaternisée contenant une amine tertiaire ; et
l'indice p est une charge moyenne de substance médicamenteuse ayant un nombre se situant dans la plage de 1 à 24 ; et
ledit clivage par une protéase régulatrice ou intracellulaire, par échange de disulfures par l'intermédiaire du glutathion, par hydrolyse acide, ou par une glycosidase conduisant à la libération de substance médicamenteuse (D) contenant une amine tertiaire à partir d'un composé conjugué ligand-substance médicamenteuse de la composition.

2. Composition pour utilisation, selon la revendication 1, dans laquelle la structure de la composition est représentée par la structure de formule 2A ou de formule 2B :

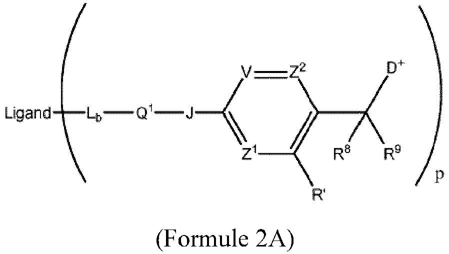

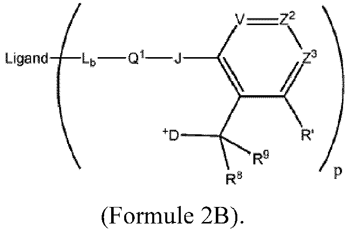

3. Composition pour utilisation, selon la revendication 1, dans laquelle la structure de la composition est représentée par la structure de formule 3A, formule 3B, formule 3C, formule 3D, formule 3E, ou formule 3F :

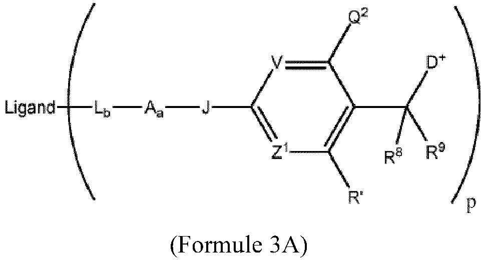

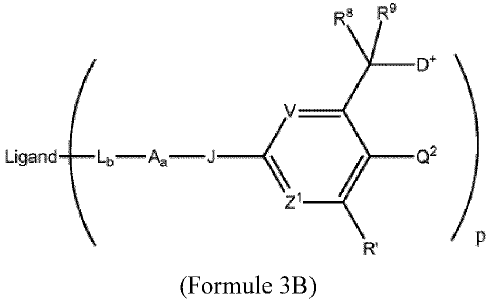

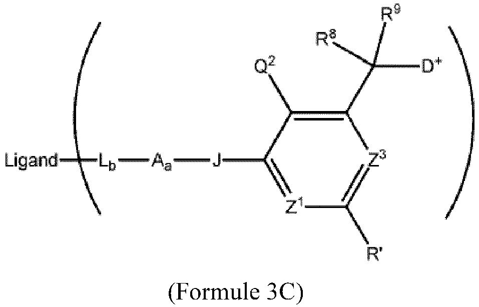

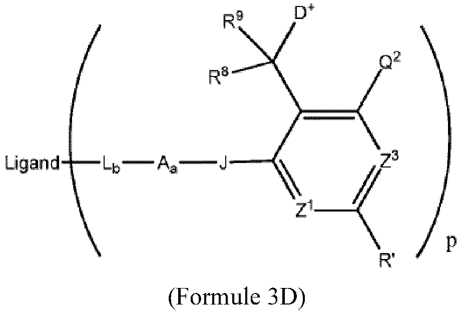

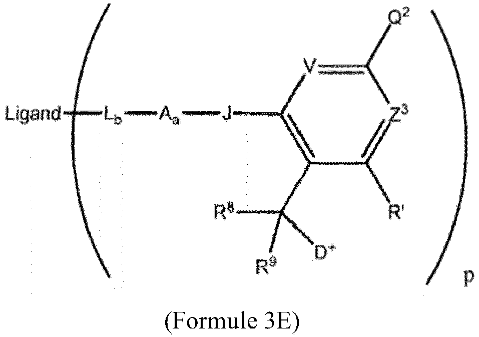

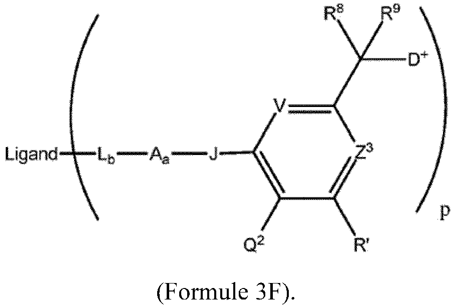

4. Composition pour utilisation, selon la revendication 2, dans laquelle W de Q¹ comprend un fragment peptidique comportant une liaison peptidique à J qui est sélectivement clivable par une protéase régulatrice ou intracellulaire en comparaison des protéases sériques, l'action de la protéase régulatrice sur W provoquant la libération de substance médicamenteuse (D) contenant une amine tertiaire à partir d'un composé conjugué ligand-substance médicamenteuse de la composition, en particulier dans laquelle le fragment peptidique de W comprend un fragment peptidique ayant la structure de formule 6 : 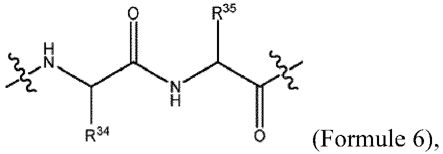
dans laquelle R³⁴ est un groupe benzyle, méthyle, isopropyle, isobutyle, sec-butyle, -CH(OH)CH₃ ou présente la structure de 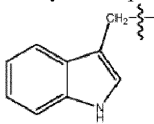 et R³⁵ est un groupe méthyle, -(CH₂)₄-NH₂, -(CH₂)₃NH(C=O)NH₂, -(CH₂)₃NH(C=NH)NH₂, ou -(CH₂)₂CO₂H,
dans laquelle le trait ondulé de liaison à l'extrémité C-terminale du dipeptide indique la liaison covalente à J du fragment arylène de la formule 2A ou 2B et le trait ondulé de liaison à l'extrémité N-terminale du dipeptide indique la liaison covalente au reste de W, si un tel reste est présent, ou à A, ou à une sous-unité de celui-ci, lorsque a est 1 ou à L_{b} lorsque l'indice a est 0, et dans laquelle la liaison du dipeptide à J est clivable par une protéase régulatrice ou intracellulaire,
en particulier la structure de la composition étant représentée par la structure de formule 9 : 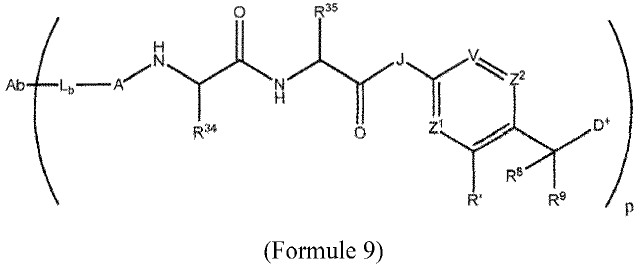
dans laquelle Ab est une unité ligand anticorps ;
R' est un atome d'hydrogène ou un groupe donneur d'électrons ;
R⁸ est un atome d'hydrogène ;
R⁹ est un atome d'hydrogène, un groupe alkyle en C₁-C₆ éventuellement substitué, ou phényle éventuellement substitué ;
R³⁴ est un groupe méthyle, isopropyle, ou -CH(OH)CH₃ ;
R³⁵ est un groupe méthyle, -(CH₂)₃NH(C=O)NH₂, ou -(CH₂)₂CO₂H ;
J est -N(R³³)-, R³³ étant un atome d'hydrogène ou un groupe méthyle ; et
V et Z¹ représentent indépendamment =CH- ou =N-,
en particulier la structure de la composition étant représentée par la structure de formule 10 : 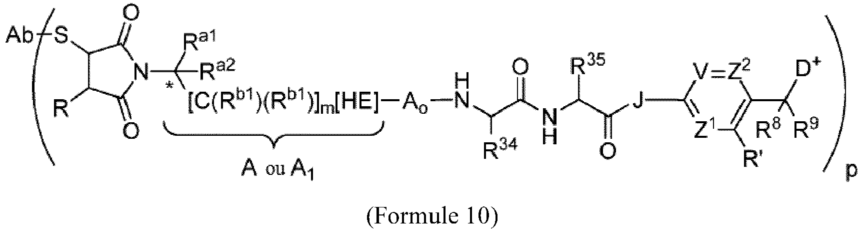
dans laquelle
S est un atome de soufre d'une unité ligand anticorps (Ab-) ;
l'astérisque (*) désigne la chiralité ou l'absence de celle-ci au niveau de l'atome de carbone indiqué ;
Aₒ est une sous-unité optionnelle de A, -[C(R^{b1})(R^{b1})]ₘ-[HE]- étant A lorsque Aₒ est absent et étant A₁ lorsque Aₒ est présent de sorte que A devient -A₁-Aₒ ;
R est -H ;
R^{a1} est -H ou une unité basique (BU), BU ayant la structure de -CH₂-N(R²²)R²³, R²² et R²³ représentant indépendamment un atome d'hydrogène, un groupe méthyle ou éthyle ou formant ensemble, conjointement avec l'atome d'azote auquel ils sont liés, un groupe hétérocycloalkyle à 5 ou 6 chaînons ;
R^{a2} est un atome d'hydrogène ;
l'indice m est un nombre entier se situant dans la plage de 0 à 5 lorsque HE est présent ou de 1 à 5 lorsque HE est absent ;
chaque R^{b1} indépendamment est un atome d'hydrogène ou un groupe alkyle en C₁-C₆ éventuellement substitué ;
HE est absent ou est -C(=O)- ;
R³⁴ est un groupe méthyle, isopropyle, ou -CH(OH)CH₃ ;
R³⁵ est -(CH₂)₃NH(C=O)NH₂, ou -(CH₂)2CO₂H ;
J est -NH- ;
V, Z¹, et Z² représentent =CH- ;
R' est un atome d'hydrogène ou un groupe donneur d'électrons ;
R⁸ est un atome d'hydrogène ; et
R⁹ est un atome d'hydrogène ou un groupe méthyle.

5. Composition pour utilisation, selon la revendication 3, dans laquelle W' de Q² est un glucide en liaison glycosidique, la liaison glycosidique W'_{w'}-E de Q² fournissant un site de clivage pour une glycosidase localisée à l'intérieur de la cellule, l'action de la glycosidase sur W'_{w'}-E provoquant la libération de substance médicamenteuse (D) contenant une amine tertiaire à partir d'un composé conjugué ligand-substance médicamenteuse de la composition,
en particulier dans laquelle W' dans Q² est un fragment glucidique qui est en liaison glycosidique à E, la liaison glycosidique étant clivable par une glycosidase localisée à l'intérieur de la cellule, et dans laquelle W'_{w'}-E a la structure de formule 7 : 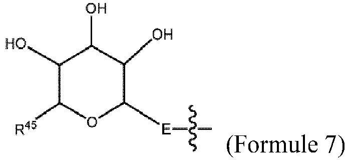
dans laquelle le trait ondulé représente E lié à l'un de V, Z¹, Z², ou Z³ lorsque ce groupe variable est =C(R²⁴), E étant -O-, -S-, ou -N(R³³)-, R³³ étant un atome d'hydrogène ou un groupe méthyle ; et
dans laquelle R⁴⁵ est -CH₂OH ou -CO₂H,
en particulier la structure de la composition étant représentée par la structure de formule 11 : 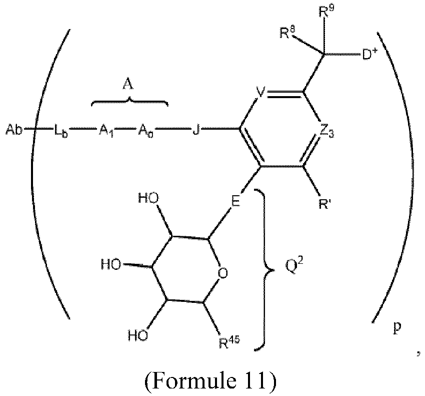
dans laquelle
A_{b} est une unité ligand anticorps ;
A₁ et Aₒ représentent indépendamment des sous-unités choisies de A, Aₒ étant une sous-unité optionnelle de A de sorte que A₁ devient A lorsque Aₒ est absent et A est -A₁-Aₒ- lorsque Aₒ est présent ;
E est -O- ou -NH- ;
J est -N(R³³)-, R³³ étant un atome d'hydrogène ou un groupe méthyle ;
V et Z³ représentent indépendamment =CH- ou =N- ;
R' est un atome d'hydrogène ou un groupe attirant les électrons ;
R⁸ est un atome d'hydrogène ;
R⁹ est un atome d'hydrogène, un groupe alkyle en C₁-C₆ éventuellement substitué ou phényle éventuellement substitué ;
R⁴⁵ est -CO₂H; et
l'indice p est un nombre se situant dans la plage de 1 à 8,
en particulier la structure de la composition étant représentée par la structure de formule 12 : 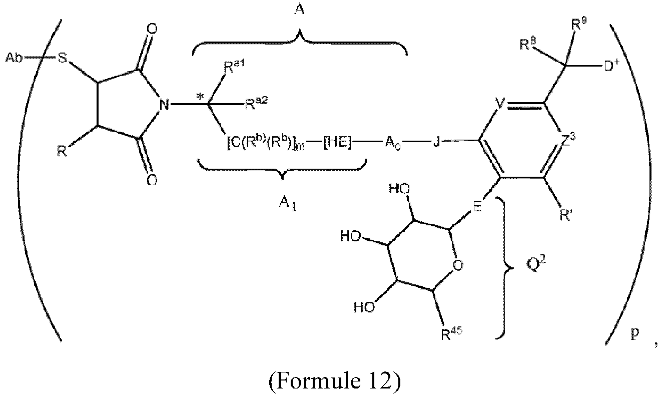
dans laquelle
S est un atome de soufre d'unité ligand anticorps Ab ;
l'astérisque (*) désigne une chiralité ou absence de celle-ci au niveau de l'atome de carbone indiqué, qui est majoritairement en la même configuration absolue que l'atome de carbone alpha d'un acide aminé L lorsque cet atome de carbone indiqué a une chiralité ;
A₁ et Aₒ représentent indépendamment des sous-unités choisies de A, Aₒ étant une sous-unité optionnelle de A, -[C(R^{b1})(R^{b1})]ₘ-[HE]- étant A lorsque Aₒ est absent et étant A₁ lorsque Aₒ est présent de sorte que A devient -A₁-Aₒ; Aₒ lorsqu'il est présent correspondant en structure à un acide contenant une amine, lié à J par le groupe carbonyle C-terminal de l'acide contenant une amine ;
R est un atome d'hydrogène ;
R' est un atome d'hydrogène ou un groupe attirant les électrons ;
R^{a1} est un atome d'hydrogène ou R^{a1} est une unité basique (BU) qui participe à l'hydrolyse assistée par base du cycle succinimide, BU ayant la structure de -CH₂-N(R²²)(R²³), ou un sel d'addition avec un acide de celle-ci, R²² et R²³ représentant indépendamment un atome d'hydrogène, un groupe méthyle ou éthyle ou formant ensemble, conjointement avec l'atome d'azote auquel ils sont liés, un groupe hétérocycloalkyle à 5 ou 6 chaînons ;
R^{a2} est un atome d'hydrogène ;
l'indice m est un nombre entier se situant dans la plage de 0 à 5 lorsque HE est présent ou de 1 à 5 lorsque HE est absent ;
chaque R^{b1} indépendamment est un atome d'hydrogène ou un groupe alkyle en C₁-C₆ éventuellement substitué ;
HE est absent ou est -C(=O)-;
R⁴⁵ est -CO₂H;
E est -O- ;
J est -NH- ;
V et Z³ représentent =CH- ;
R⁸ est un atome d'hydrogène ;
R⁹ est un atome d'hydrogène ou un groupe méthyle ; et
l'indice p est un nombre se situant dans la plage de 1 à 8,
ou dans laquelle Aₒ, lorsqu'il est présent, a la structure de formule 13 ou de formule 14 : 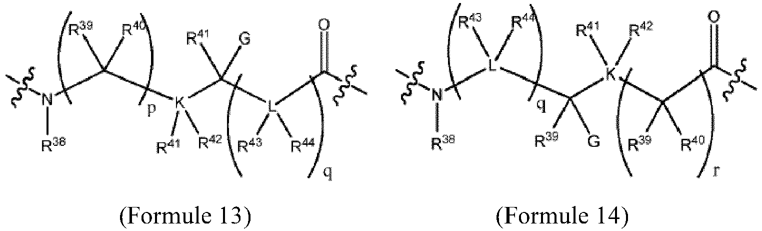
formules dans lesquelles le trait ondulé lié au groupe carbonyle de l'une ou l'autre structure représente le point d'attachement de Aₒ à W et dans lesquelles le trait ondulé lié au groupe amino de l'une ou l'autre structure représente le point d'attachement de Aₒ à A₁,
dans lesquelles K et L représentent indépendamment C, N, O ou S, étant entendu que lorsque K ou L est O ou S, R⁴¹ et R⁴² liés à K ou R⁴³ et R⁴⁴ liés à L sont absents, et lorsque K ou L est N, l'un de R⁴¹, R⁴² liés à K ou l'un de R⁴³, R⁴⁴ liés à L est absent, et étant entendu que deux L adjacents ne sont pas choisis indépendamment en tant que N, O ou S ;
dans lesquelles q est un nombre entier se situant dans la plage de 1 à 12, et r est un nombre entier se situant dans la plage de 1 à 12 ;
dans lesquelles G est un atome d'hydrogène, un groupe alkyle en C₁-C₆ éventuellement substitué, -OH, -OR^{G}, -CO₂H, CO₂R^{G}, R^{G} étant un groupe alkyle en C₁-C₆, aryle ou hétéroaryle, éventuellement substitué, un groupe protecteur approprié, -NH₂, ou -N(R^{G})(R^{G}), R^{G} choisi indépendamment étant tel que défini précédemment ou les deux R^{G} formant ensemble, conjointement avec l'atome d'azote auquel ils sont liés, un groupe hétérocycloalkyle à 5 ou 6 chaînons, ou les deux R^{G} formant ensemble un groupe protecteur approprié ;
dans lesquelles R³⁸ est un atome d'hydrogène ou un groupe alkyle en C₁-C₆ éventuellement substitué ; R³⁹-R⁴⁴ représentent indépendamment un atome d'hydrogène, un groupe alkyle en C₁-C₆ éventuellement substitué, éventuellement substitué, ou hétéroaryle éventuellement substitué, ou R³⁹, R⁴⁰ forment ensemble, conjointement avec l'atome de carbone auquel ils sont liés, un groupe cycloalkyle en C₃-C₆, ou R⁴¹, R⁴² forment ensemble, conjointement avec K auquel ils sont liés lorsque K est C, ou R⁴³, R⁴⁴ forment ensemble, conjointement avec L auquel ils sont liés lorsque L est C, un groupe cycloalkyle en C₃-C₆, ou R⁴⁰ et R⁴¹, ou R⁴⁰ et R⁴³, ou R⁴¹ et R⁴³ forment ensemble, conjointement avec l'atome de carbone ou l'hétéroatome auquel ils sont liés et les atomes intercalés entre ces atomes de carbone et/ou hétéroatomes, un groupe cycloalkyle ou hétérocycloalkyle à 5 ou 6 chaînons,
ou dans lesquelles Aₒ a une structure correspondant à un acide alpha-aminé, bêta-aminé ou un autre acide contenant une amine.

6. Composition pour utilisation, selon l'une quelconque des revendications 1-5, dans laquelle l'indice a est 1 ;
dans laquelle -L_{B}-Aₐ dans la formule 1 est -L_{b}-A lorsque Aₒ est absent ou -L_{b}-A est -L_{b}-A₁-Aₒ lorsque Aₒ est présent de sorte que A devient A₁-Aₒ
dans laquelle L_{b}-Aₐ- a la structure de formule 8 : 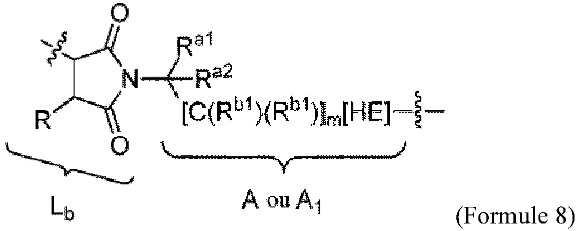
dans laquelle
le groupement -[C(R^{b1})(R^{b1})]ₘ-[HE]- est A ou A₁;
R et R^{a2} représentent indépendamment un atome d'hydrogène ou un groupe méthyle ;
R^{a1} est un atome d'hydrogène, un groupe méthyle ou éthyle ou R^{a1} est une unité basique (BU) qui participe à l'hydrolyse assistée par base du cycle succinimide :
HE est une unité activatrice d'hydrolyse (HE) ;
l'indice m est un nombre entier se situant dans la plage de 0 à 6 ;
chaque R^{b1} indépendamment est un atome d'hydrogène, un groupe alkyle en C₁-C₆ éventuellement substitué, aryle éventuellement substitué ou hétéroaryle éventuellement substitué, ou deux R^{b1} forment ensemble, conjointement avec l'atome/les atomes de carbone au(x)quel(s) ils sont liés, un groupe cycloalkyle en C₃-C₆, ou l'un des R^{b1} et HE forment ensemble, conjointement avec l'atome de carbone auquel ils sont liés, un groupe cycloalkyle à 5 ou 6 chaînons ou un groupe hétérocycloalkyle à 5 ou 6 chaînons et l'autre R^{b1} est un atome d'hydrogène, un groupe alkyle en C₁-C₆ éventuellement substitué, aryle éventuellement substitué, ou hétéroaryle éventuellement substitué ;
BU a la structure de -[C(R¹)(R¹)]-[C(R²)(R²)]ₙ-N(R²²)(R²³),
où l'indice n est 0, 1, 2, ou 3 ;
chaque R¹ indépendamment est un atome d'hydrogène ou un groupe alkyle en C₁-C₄ ou deux R¹ forment ensemble, conjointement avec l'atome de carbone auquel ils sont liés, un groupe cycloalkyle en C₃-C₆, et chaque R² indépendamment est un atome d'hydrogène, un groupe alkyle en C₁-C₆ éventuellement substitué, aryle éventuellement substitué ou hétéroaryle éventuellement substitué, ou deux R² forment ensemble, conjointement avec l'atome/les atomes de carbone au(x)quel(s) ils sont liés et tous atomes de carbone intercalés, un groupe cycloalkyle en C₃-C₆, ou un R¹ et un R² forment ensemble, conjointement avec les atomes de carbone auxquels ils sont liés et tous atomes de carbone intercalés, un groupe cycloalkyle à 5 ou 6 chaînons et les R¹ et R² restants sont tels que définis ;
R²² et R²³ représentent indépendamment un atome d'hydrogène ou un groupe alkyle en C₁-C₆ éventuellement substitué, ou forment ensemble, conjointement avec l'atome d'azote auquel ils sont liés, un groupe hétérocycloalkyle à 5 ou 6 chaînons ; et
dans laquelle le trait ondulé de liaison au cycle succinimide de L_{b} indique la liaison covalente du soufre dérivé d'un groupe sulfhydryle d'une entité de ciblage et l'autre trait ondulé indique la liaison covalente de A ( ou A₁) au reste de la structure de la composition.

7. Composition pour utilisation, selon la revendication 1, dans laquelle les composés conjugués ligand-substance médicamenteuse de la composition sont représentés par les structures de formule 16A et formule 16B : 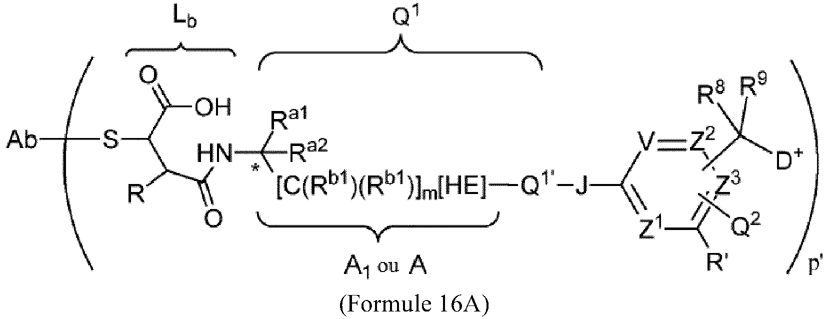 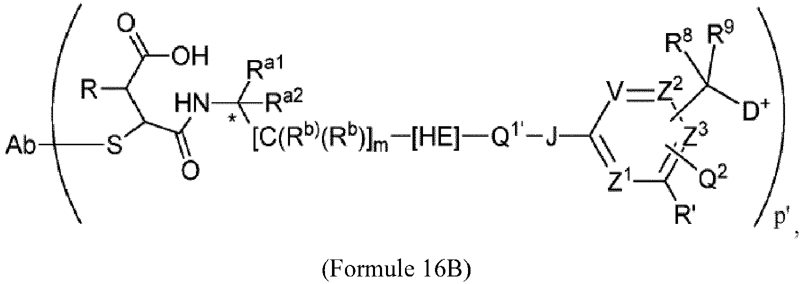
formules dans lesquelles Ab est une unité ligand anticorps ;
S est un atome de soufre de l'unité ligand anticorps ;
l'astérisque (*) désigne une chiralité ou absence de celle-ci au niveau de l'atome de carbone indiqué :
Q^{1'} est Aₒ-W_{w}, Aₒ étant une sous-unité optionnelle de A de sorte que -Q^{1'}-est -W_{w}- lorsque Aₒ est absent, ou est -Aₒ-W_{w}- lorsque Aₒ est présent,
dans lesquelles -[C(R^{b})(R^{b})]ₘ-[HE]- devient A lorsque Aₒ est absent ou est A₁ lorsque Aₒ est présent, et
dans lesquelles l'indice w est 1 lorsque Q² est absent ou w est 0 de sorte que W est absent et Q² est présent ;
R est un atome d'hydrogène ;
R^{a1} est BU, BU ayant la structure de -CH₂-N(R²²)R²³, ou un sel d'addition avec un acide de celle-ci, R²² et R²³ représentant indépendamment un atome d'hydrogène ou un groupe méthyle ou formant ensemble, conjointement avec l'atome d'azote auquel ils sont liés, un groupe hétérocycloalkyle à 5 ou 6 chaînons ;
R^{a2} est un atome d'hydrogène ;
l'indice m est un nombre entier se situant dans la plage de 0 à 5 lorsque HE est présent ou de 1 à 5 lorsque HE est absent ;
chaque R^{b1} indépendamment est un atome d'hydrogène ou un groupe alkyle en C₁-C₆ éventuellement substitué ;
HE est absent ou est -C(=O)-;
J est -O- ou -NH- ;
Q² lorsqu'il est présent est W'_{w'}-E, E étant -O- ou -NH- ; et
l'indice p' est un nombre entier se situant dans la plage de 1 à 24.

8. Composition pour utilisation, selon la revendication 7, dans laquelle les composés conjugués ligand-substance médicamenteuse de la composition ont les structures de formule 17A et formule 17B : 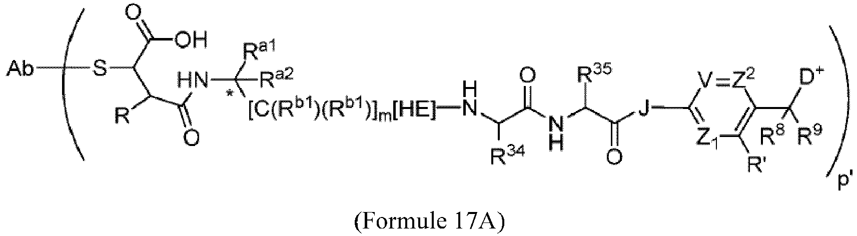 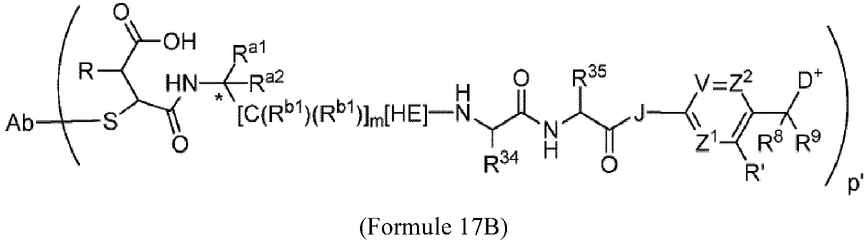
formules dans lesquelles J est -NH- ;
l'un de V ou Z¹ est =C(R²⁴)-, R²⁴ étant un atome d'hydrogène, -Cl ou -NO₂, et les autres de V, Z¹, et Z² représentant =CH- ;
R⁸ est un atome d'hydrogène ;
R⁹ est un atome d'hydrogène ou un groupe méthyle ; et
l'indice p' et un nombre entier se situant dans la plage de 1 à 8.

9. Composition pour utilisation, selon la revendication 7, dans laquelle les composés conjugués ligand-substance médicamenteuse de la composition ont les structures de formule 18A et formule 18B : 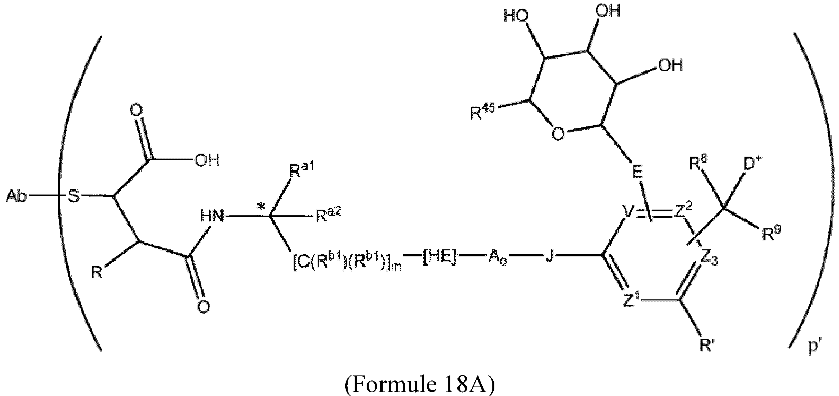 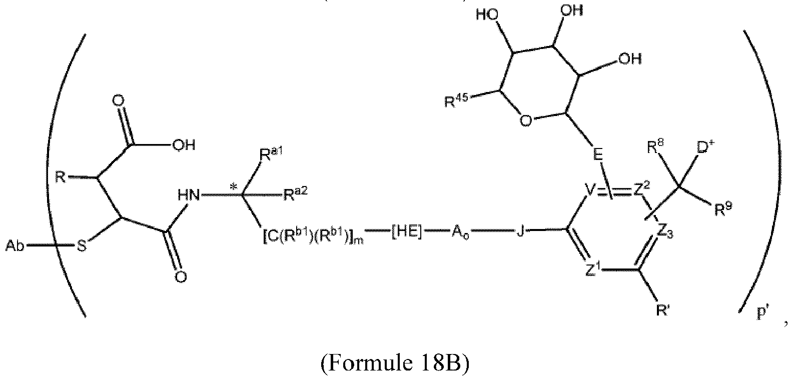
formules dans lesquelles Aₒ est une sous-unité optionnelle de A, le groupement -[C(R^{b1})(R^{b1})]ₘ-[HE]- étant A lorsque Aₒ est absent ou le groupement étant A₁ lorsque Aₒ est présent de sorte que A devient -A₁-Aₒ- ;
R est -H- ;
R^{al} est une unité basique (BU), BU ayant la structure de -CH₂-N(R²²)R²³, ou un sel d'addition avec un acide de celle-ci, R²² et R²³ représentant indépendamment un atome d'hydrogène ou un groupe méthyle ou formant ensemble, conjointement avec l'atome d'azote auquel ils sont liés, un groupe hétérocycloalkyle à 5 ou 6 chaînons ;
R^{a2} est un atome d'hydrogène ;
l'indice m est un nombre entier se situant dans la plage de 0 à 5 lorsque HE est présent ou de 1 à 5 lorsque HE est absent ;
chaque R^{b1} indépendamment est un atome d'hydrogène ou un groupe alkyle en C₁-C₆ éventuellement substitué ;
HE est absent ou est -C(=O)- ; et
R⁸ est un atome d'hydrogène ; et
R⁹ est un atome d'hydrogène, un groupe alkyle en C₁-C₆ éventuellement substitué ou phényle éventuellement substitué ;
en particulier les composés conjugués ligand-substance médicamenteuse de la composition ayant les structures de formule 19A et formule 19B : 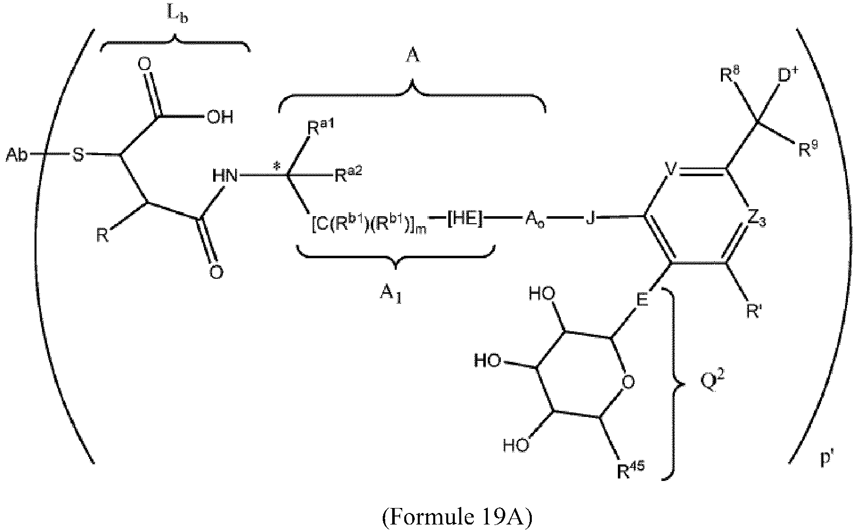 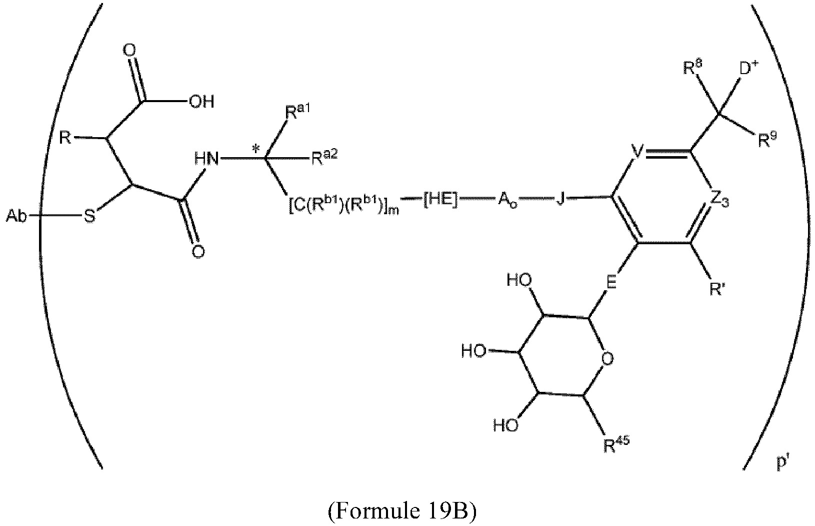
formules dans lesquelles
R est un atome d'hydrogène ;
R^{al} est une unité basique (BU), BU ayant la structure de -CH₂-N(R²²)R²³, ou un sel d'addition avec un acide de celle-ci, R²² et R²³ représentant indépendamment un atome d'hydrogène ou un groupe méthyle ou formant ensemble, conjointement avec l'atome d'azote auquel ils sont liés, un groupe hétérocycloalkyle à 5 ou 6 chaînons ;
R^{a2} est un atome d'hydrogène ;
l'indice m est un nombre entier se situant dans la plage de 0 à 5 lorsque HE est présent ou de 1 à 5 lorsque HE est absent ;
chaque R^{b1} indépendamment est un atome d'hydrogène ou un groupe alkyle en C₁-C₆ éventuellement substitué ;
HE est absent ou est -C(=O)- ;
R⁴⁵ est -CO₂H ;
E est -O- ;
J est -NH- ;
V et Z³ représentent =CH- ;
R⁸ est un atome d'hydrogène ;
R⁹ est un atome d'hydrogène ou un groupe méthyle ; et
l'atome de carbone indiqué (*) est majoritairement en la même configuration absolue que l'atome de carbone alpha d'un acide aminé L lorsque cet atome de carbone indiqué a une chiralité.

10. Composition pour utilisation, selon l'une quelconque des revendications 1-9, dans laquelle -D⁺ est un agent perturbateur de tubuline contenant une amine tertiaire, quaternisé,
en particulier dans laquelle l'unité substance médicamenteuse -D⁺ perturbatrice de tubuline, quaternisée, est une unité substance médicamenteuse tubulysine quaternisée,
en particulier dans laquelle l'unité substance médicamenteuse tubulysine quaternisée -D⁺ a la structure de formule D_{G}-₁' ou formule D_{H-1}' : 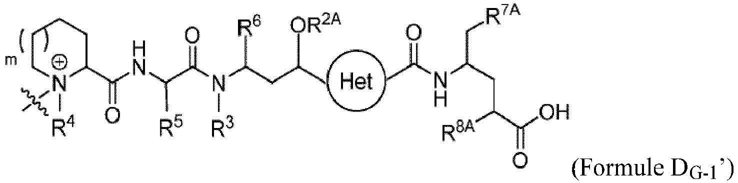 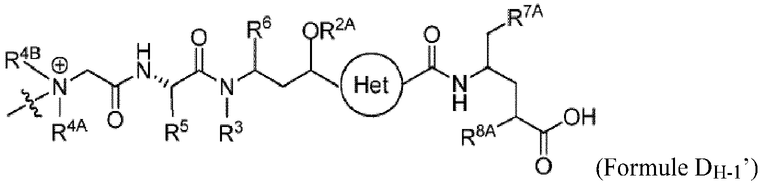
formules dans lesquelles le cercle représente un groupe hétéroaryle azoté à 5 chaînons et dans lesquelles les substituants requis indiqués sur ce groupe hétéroaryle sont en relation 1,3 entre eux avec substitution optionnelle en les positions restantes :
R^{2A} est un atome d'hydrogène ou un groupe alkyle éventuellement substitué ou R^{2A} forme conjointement avec l'atome d'oxygène auquel il est lié un substituant lié par O, différent de -OH ;
R³ est un atome d'hydrogène ou un groupe alkyle éventuellement substitué ;
R⁴, R^{4A}, R^{4B}, R⁵, et R⁶ représentent un groupe alkyle éventuellement substitué, choisi indépendamment ;
R^{7A} est un groupe aryle éventuellement substitué ou hétéroaryle éventuellement substitué ;
R^{8A} est un atome d'hydrogène ou un groupe alkyle éventuellement substitué ; et
m est 0 ou 1,
dans lesquelles le trait ondulé indique la liaison covalente de D⁺ au reste de la structure de la composition.

11. Composition pour utilisation, selon la revendication 10, dans laquelle l'unité substance médicamenteuse tubulysine quaternisée -D⁺ a la structure de : 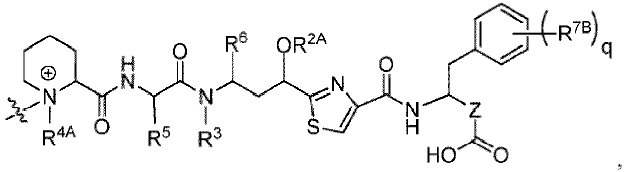
dans laquelle Z est un groupe alkylène en C₁-C₄ éventuellement substitué ou un groupe alcénylène en C₂-C₄ éventuellement substitué ;
l'indice q, indiquant le nombre de substituants R^{7B}, est 1, 2, ou 3 ;
dans laquelle chaque R^{7B} est choisi indépendamment dans le groupe constitué par un atome d'hydrogène et un substituant lié par O ; et
dans laquelle le trait ondulé indique la liaison covalente de D⁺ au reste de la structure de la composition,
ou dans laquelle l'unité substance médicamenteuse tubulysine quaternisée -D⁺ a la structure de : 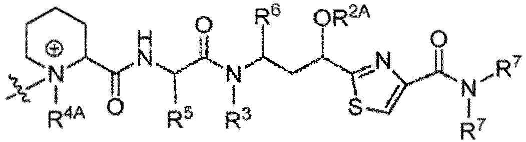
dans laquelle R^{2A} est un atome d'hydrogène ou un groupe alkyle en C₁-C₆ éventuellement substitué ou R^{2A} forme conjointement avec l'atome d'oxygène auquel il est fixé un substituant lié par O, différent de -OH ;
R³ est un groupe alkyle en C₁-C₆ éventuellement substitué ;
R⁵ et R⁶ représentent les résidus de chaînes latérales d'acides aminés hydrophobes naturels ;
-N(R⁷)(R⁷) est un groupe -NH(alkyle en C₁-C₆) ou -NH-N(alkyle en C₁-C₆)₂, un et seulement un groupe alkyle en C₁-C₆ étant éventuellement substitué par -CO₂H, ou un ester de ce dernier, ou par un groupe phényle éventuellement substitué ; et
dans laquelle le trait ondulé indique la liaison covalente de D⁺ au reste de la structure de la composition,
en particulier dans laquelle -N(R⁷)(R⁷) est choisi dans le groupe constitué par -NH(CH₃), -NHCH₂CH₂Ph, et -NHCH₂-CO₂H, -NHCH₂CH₂CO₂H, et -NHCH₂CH₂CH₂CO₂H,
en particulier dans laquelle le substituant lié par O, -OR^{2A}, est différent de -OH de sorte que R^{2A} n'est pas un atome d'hydrogène,
en particulier dans laquelle R^{2A} est -CH₂CH₃.

12. Composition pour utilisation, selon la revendication 11, dans laquelle l'unité substance médicamenteuse tubulysine quaternisée -D⁺ a la structure de : 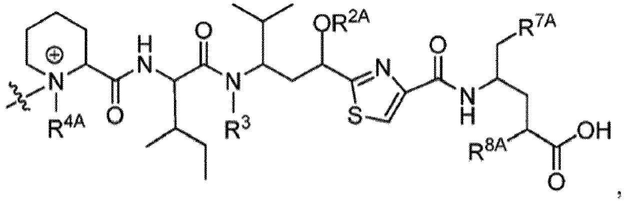
en particulier dans laquelle l'unité substance médicamenteuse tubulysine quaternisée -D⁺ a la structure de : 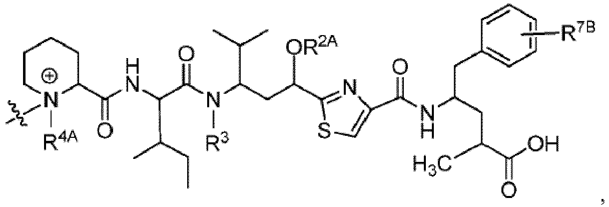
dans laquelle R^{4A} est un groupe méthyle ;
R³ est H, un groupe méthyle, éthyle, propyle, -CH₂-OC(O)R^{3A}, -CH₂CH(R^{3B})C(O)R^{3A}, ou -CH(R^{3B})C(O)NHR^{3A}, R^{3A} étant un groupe alkyle en C₁-C₆ et R^{3B} étant H ou un groupe alkyle en C₁-C₆, choisi indépendamment de R^{3A} ; et
-OR^{2A} est un substituant lié par O, choisi dans le groupe constitué par - OR^{2B}, -OC(O)R^{2B} et -OC(O)N(R^{2B})(R^{2C}), R^{2B} et R^{2C} étant choisis indépendamment dans le groupe constitué par H, les groupes alkyle en C₁-C₆, et alcényle en C₂-C₆; et
R^{7B} est un atome d'hydrogène ou -OH,
en particulier dans laquelle R^{2A} est -CH₂CH₃.

13. Composition pour utilisation, selon la revendication 10, dans laquelle l'unité substance médicamenteuse tubulysine quaternisée -D⁺ a la structure de : 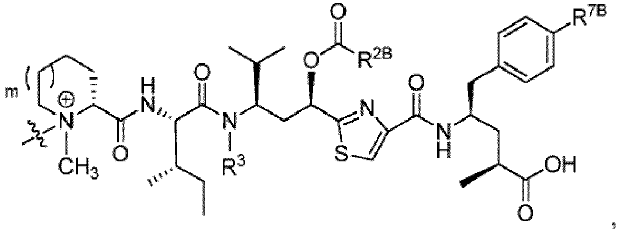
dans laquelle l'indice m est 1 ;
R^{2B} est -CH₃, -CH₂CH₃, -CH(CH₃)₂, -C(CH₃)₃, ou -CH=CH₂ ;
R³ est un groupe méthyle, éthyle, ou propyle ; et
R^{7B} est un atome d'hydrogène ou -OH ;
ou dans laquelle l'unité substance médicamenteuse tubulysine quaternisée -D⁺ a la structure de : 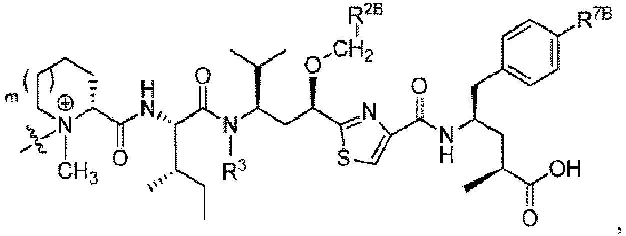
dans laquelle l'indice m est 1 ;
R^{2B} est -H, -CH₃, -CH₂CH₃, ou -OCH₃ ;
R³ est un groupe méthyle, éthyle ou propyle ; et
R^{7B} est un atome d'hydrogène ou -OH ;
ou dans laquelle l'unité substance médicamenteuse tubulysine quaternisée -D⁺ a la structure de : 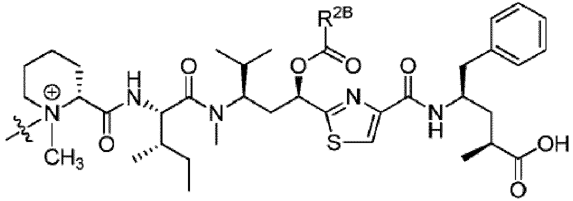 ou 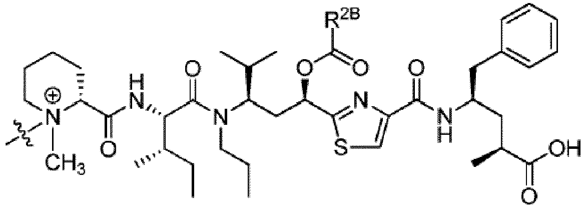
où R^{2B} est -CH₃, -CH₂CH₃, -CH₂CH₂CH₃, - CH(CH₃)₂, -CH₂CH(CH₃)₂, -CH₂C(CH₃)₃,
ou 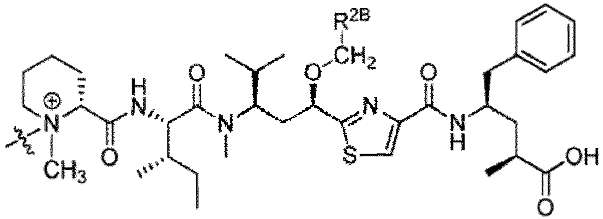 ou 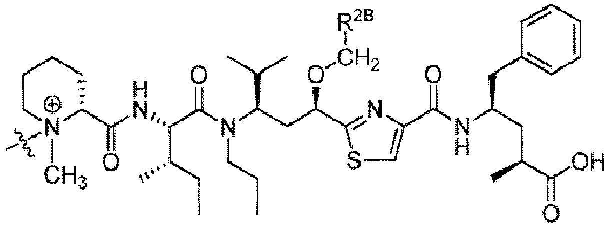
où R^{2B} est un atome d'hydrogène, un groupe méthyle, ou -OCH₃ de sorte que -CH₂R^{2B} est un substituant méthyle, éthyle ou éther méthoxyméthylique,
R^{2B} étant en particulier un groupe méthyle.

14. Composition pour utilisation, selon la revendication 1, la composition étant représentée par la structure de : 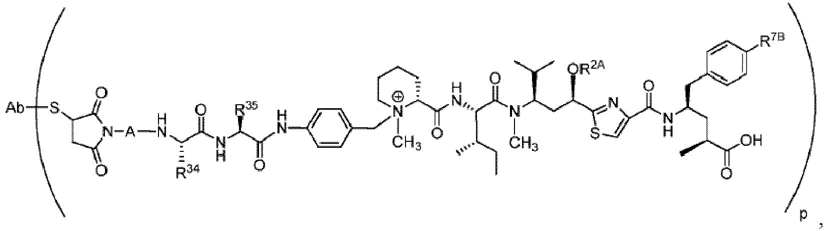
dans laquelle Ab est une unité ligand anticorps ;
S est un atome de soufre de l'unité ligand anticorps ;
R³⁴ est un groupe isopropyle et R³⁵ est un groupe -CH₃, isopropyle, -CH₂CH₂CH₂NH(C=O)NH₂, ou -CH₂CH₂CO₂H ;
R^{7B} est un atome d'hydrogène ou -OH ;
R^{2A} est un groupe alkyle en C₁-C₆, alcényle en C₂-C₆, - OCH₂OR^{2B}, -C(=O)R^{2B}, ou -C(=O)NHR^{2B}, R^{2B} étant un atome d'hydrogène ou un groupe alkyle en C₁-C₆ ; et
l'indice p est un nombre se situant dans la plage de 1 à 8,
en particulier dans laquelle
R³⁴ est un groupe isopropyle et R³⁵ est -CH₃ ou -CH₂CH₂CH₂NH(C=O)NH₂ ;
R^{7B} est un atome d'hydrogène ou -OH ; et
R^{2A} est un groupe alkyle en C₁-C₄, -C(=O)R^{2B}, ou -C(=O)NHR^{2B}, R^{2B} étant un groupe alkyle en C₁-C₄,
en particulier dans laquelle A est -CH₂(CH₂)₄(C=O)- ou -CH₂(CH₂)₄(C=O)NHCH₂CH₂(C=O)-,
la structure de la composition étant en particulier représentée par la structure de : 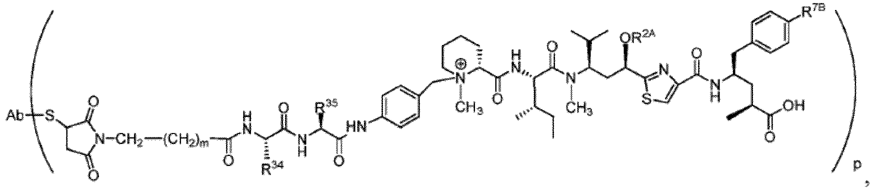
dans laquelle Ab est une unité ligand anticorps ;
S est un atome de soufre de l'unité ligand anticorps ;
l'indice p est un nombre se situant dans la plage de 1 à 8 ; et
l'indice m est 4,
en particulier dans laquelle R^{2A} est un groupe -C(O)CH₃, méthyle, éthyle, ou propyle.

15. Composition pour utilisation, selon la revendication 1, la composition étant représentée par la structure de : 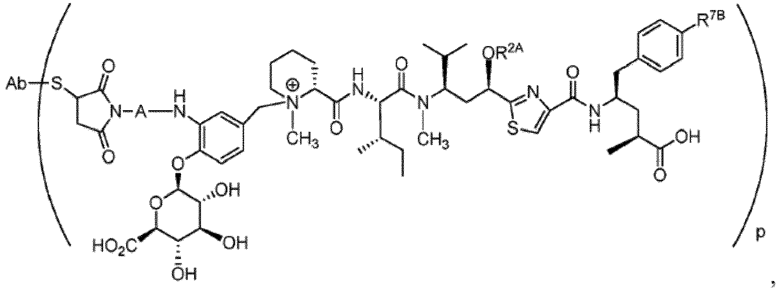
dans laquelle Ab est une unité ligand anticorps ;
S est un atome de soufre de l'unité ligand anticorps ;
R^{7B} est un atome d'hydrogène ou -OH ;
R^{2A} est un groupe alkyle en C₁-C₆, -OCH₂OR^{2B}, -C(=O)R^{2B}, ou -C(=O)NHR^{2B}, R^{2B} étant un groupe alkyle en C₁-C₆ ou alcényle en C₂-C₆ ; et
l'indice p est un nombre se situant dans la plage de 1 à 8,
en particulier dans laquelle
R^{7B} est un atome d'hydrogène ou -OH ; et
R^{2A} est un groupe alkyle en C₁-C₄, -C(=O)R^{2B}, ou -C(=O)NHR^{2B}, R^{2B} étant un groupe alkyle en C₁-C₄,
en particulier dans laquelle A est -CH₂(CH₂)₄(C=O)- ou -CH₂(CH₂)₄(C=O)NHCH₂CH₂(C=O)-,
la structure de la composition étant en particulier représentée par la structure de : 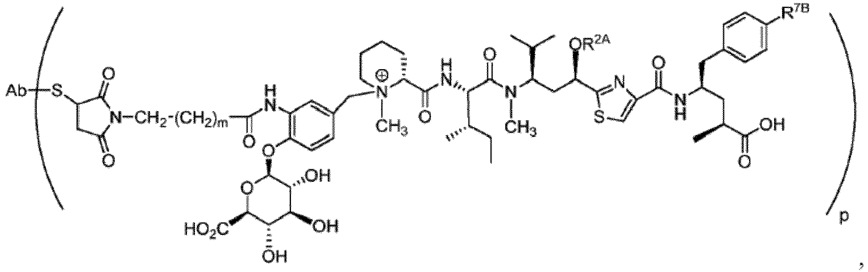
dans laquelle Ab est une unité ligand anticorps ;
S est un atome de soufre de l'unité ligand anticorps ;
l'indice p est un nombre se situant dans la plage de 1 à 8 ; et
l'indice m est 4,
en particulier dans laquelle R^{2A} est un groupe -C(O)CH₃, méthyle, éthyle, ou propyle.

16. Composition pour utilisation, selon la revendication 1, dans laquelle chaque composé conjugué ligand-substance médicamenteuse de la composition est représenté par la structure de : 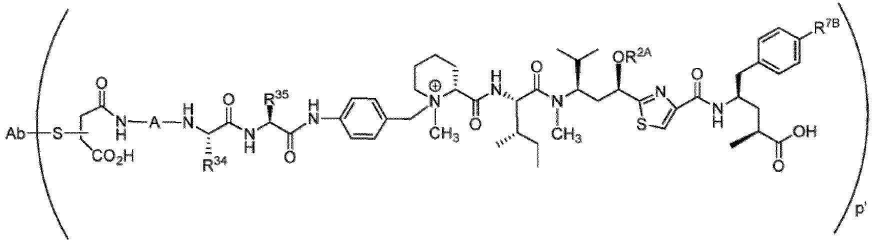
dans laquelle Ab est une unité ligand anticorps ;
S est un atome de soufre de l'unité ligand anticorps ;
le fragment Ab-S- est lié à l'atome de carbone α ou β de l'acide carboxylique ;
R³⁴ est un groupe isopropyle et R³⁵ est -CH₃ ou -CH₂CH₂CH₂NH(C=O)NH₂ ;
R^{7B} est un atome d'hydrogène ou -OH ;
R^{2A} est un groupe alkyle en C₁-C₆, -C(=O)R^{2B}, ou -C(=O)NHR^{2B}, R^{2B} étant un groupe alkyle en C₁-C₄ ; et
l'indice p' est un nombre entier se situant dans la plage de 1 à 8,
en particulier dans laquelle A est -CH₂(CH₂)₄(C=O)- ou -CH₂(CH₂)₄(C=O)NHCH₂CH₂(C=O)-,
le composé conjugué ligand-substance médicamenteuse de la composition étant en particulier représenté par la structure de : 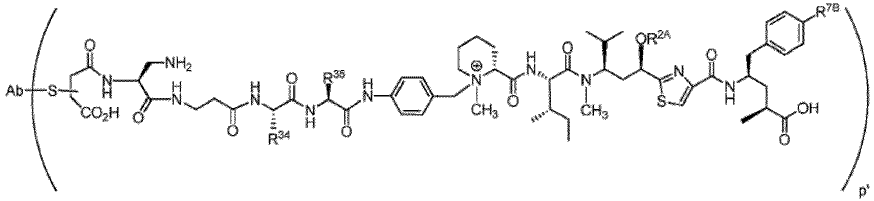 ou 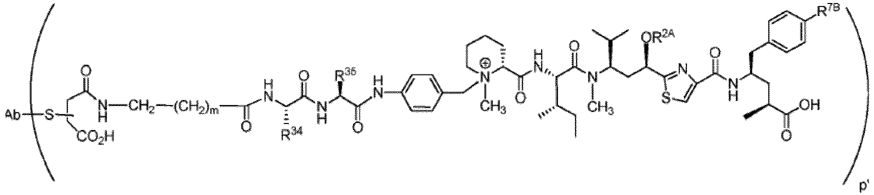
où Ab est une unité ligand anticorps ;
S est un atome de soufre de l'unité ligand anticorps ;
le fragment Ab-S- est lié à l'atome de carbone α ou β de l'acide carboxylique ;
l'indice p' est un nombre entier se situant dans la plage de 1 à 8 ; et
l'indice m est 4,
en particulier dans laquelle R^{2A} est -C(O)CH₃, un groupe méthyle, éthyle, ou propyle.

17. Composition pour utilisation, selon la revendication 1, dans laquelle chaque composé conjugué ligand-substance médicamenteuse de la composition est représenté par la structure de : 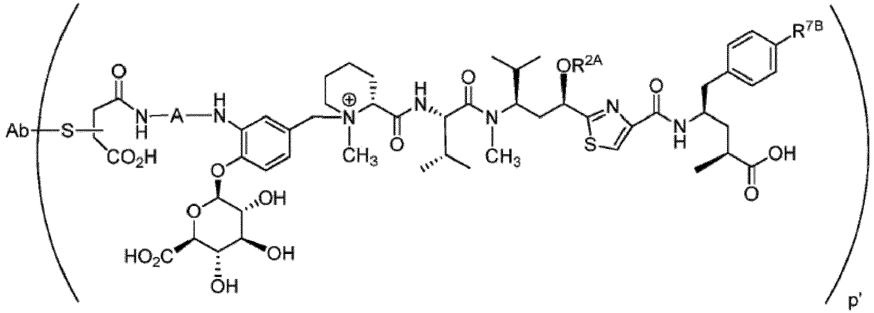 dans laquelle
Ab est une unité ligand anticorps ;
S est un atome de soufre de l'unité ligand anticorps ;
le fragment Ab-S- est lié à l'atome de carbone α ou β de l'acide carboxylique ;
R^{7B} est un atome d'hydrogène ou -OH ;
R^{2A} est un groupe alkyle en C₁-C₄, -C(=O)R^{2B}, ou -C(=O)NHR^{2B}, R^{2B} étant un groupe alkyle en C₁-C₄ ;
et l'indice p' est un nombre entier se situant dans la plage de 1 à 8,
en particulier dans laquelle A est -CH₂(CH₂)₄(C=O)- ou -CH₂(CH₂)₄(C=O)NHCH₂CH₂(C=O)-,
chaque composé conjugué ligand-substance médicamenteuse de la composition étant en particulier représenté par la structure de : 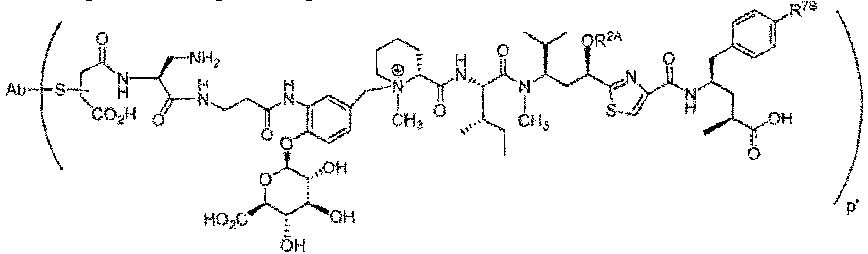 ou 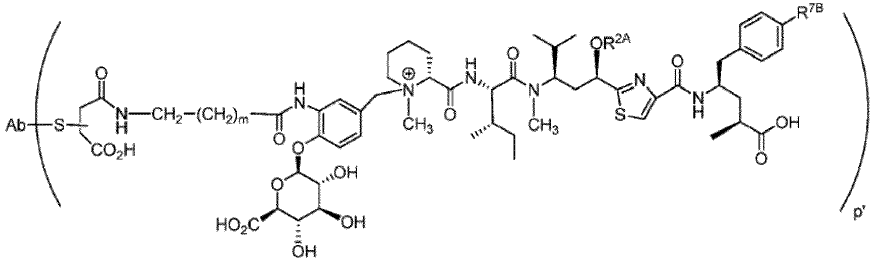
où Ab est une unité ligand anticorps ;
S est un atome de soufre de l'unité ligand anticorps ;
le fragment Ab-S- est lié à l'atome de carbone α ou β de l'acide carboxylique ;
l'indice p' est un nombre entier se situant dans la plage de 1 à 8 ; et
l'indice m est 4,
en particulier où R^{2A} est -C(O)CH₃, un groupe méthyle, éthyle, ou propyle.

18. Composition pour utilisation, selon la revendication 1, dans laquelle -D⁺ est une unité substance médicamenteuse auristatine quaternisée ou une unité substance médicamenteuse dolastatine quaternisée,
en particulier dans laquelle l'unité substance médicamenteuse dolastatine quaternisée est soit la dolastatine 10 quaternisée ou la dolastatine 15 quaternisée,
en particulier dans laquelle l'unité substance médicamenteuse auristatine quaternisée -D⁺ a la structure de D_{E}' ou D_{F}' : 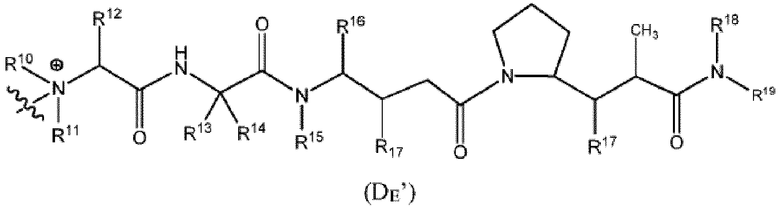 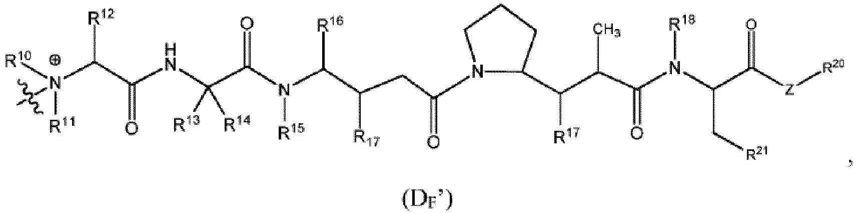
où R¹⁰ et R¹¹ représentent indépendamment un groupe alkyle en C₁-C₈;
R¹² est un atome d'hydrogène, un groupe alkyle en C₁-C₈, cycloalkyle en C₃-C₈, aryle, -X¹-aryle, -X1-(cycloalkyle en C₃-C₈), hétérocyclique en C₃-C₈, ou -X¹-(hétérocyclique en C₃-C₈) ;
R¹³ est un atome d'hydrogène, un groupe alkyle en C₁-C₈, cycloalkyle en C₃-C₈, aryle, -X¹-aryle, -X1-(cycloalkyle en C₃-C₈), hétérocyclique en C₃-C₈, ou -X¹-(hétérocyclique en C₃-C₈) ;
R¹⁴ est un atome d'hydrogène ou un groupe méthyle ;
ou R¹³ et R¹⁴ forment ensemble, conjointement avec l'atome de carbone auquel ils sont liés, un groupe cycloalkyle en C₃-C₈ ;
R¹⁵ est un atome d'hydrogène ou un groupe alkyle en C₁-C₈ ;
R¹⁶ est un atome d'hydrogène, un groupe alkyle en C₁-C₈, cycloalkyle en C₃-C₈, aryle, -X¹-aryle, -X1-(cycloalkyle en C₃-C₈), hétérocyclique en C₃-C₈, ou -X¹-(hétérocyclique en C₃-C₈) ;
R¹⁷ représente indépendamment un atome d'hydrogène, -OH, un groupe alkyle en C₁-C₈, cycloalkyle en C₃-C₈, ou O-(alkyle en C₁-C₈) ;
R¹⁸ représente indépendamment un atome d'hydrogène ou un groupe alkyle en C₁-C₈ ;
R¹⁹ est un groupe -C(R^{19A})₂-C(R^{19A})₂-aryle, -C(R^{19A})₂-C(R^{19A})₂-(hétérocyclique en C₃-C₈), ou -C(R^{19A})₂-C(R^{19A})₂-(cycloalkyle en C₃-C₈) ;
R²¹ est un groupe aryle ou hétérocyclique en C₃-C₈ ; R^{19A} étant un atome d'hydrogène, un groupe alkyle en C₁-C₈, ou -OH ;
R²⁰ est un atome d'hydrogène, un groupe alkyle en C₁-C₂₀, hétérocyclique en C₃-C₈, -(R⁴⁷O)ₘ-R⁴⁸, ou -(R⁴⁷O)ₘ-CH(R⁴⁹)₂ ;
l'indice m est un nombre entier se situant dans la plage de 1 à 1 000 ;
R⁴⁷ est un groupe alkyle en C₂-C₈ ;
R⁴⁸ est un atome d'hydrogène ou un groupe alkyle en C₁-C₈ ;
R⁴⁹ représente indépendamment -COOH, -(CH₂)ₙ-N(R⁵⁰)₂, -(CH₂)ₙ-SO₃H, ou -(CH₂)ₙ-SO₃-alkyle en C₁-C₈ ;
R⁵⁰ représente indépendamment un groupe alkyle en C₁-C₈ ou -(CH₂)ₙ-COOH ;
Z est O, S, NH, ou NR⁴⁶, R⁴⁶ étant un groupe alkyle en C₁-C₈ ;
X¹ et un groupe alkylène en C₁-C₁₀ ; et
l'indice n est un nombre entier se situant dans la plage de 0 à 6 :
le trait ondulé indiquant la liaison covalente de D⁺ au reste de la structure de la composition,
en particulier chaque composé conjugué ligand-substance médicamenteuse de la composition étant représenté par la structure de : 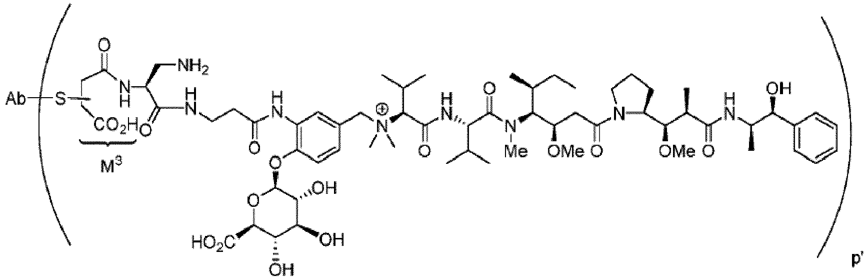 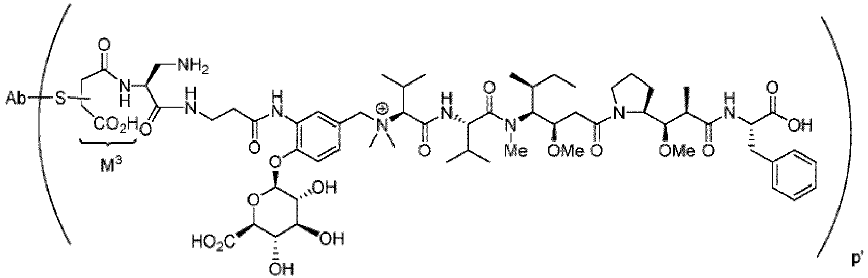 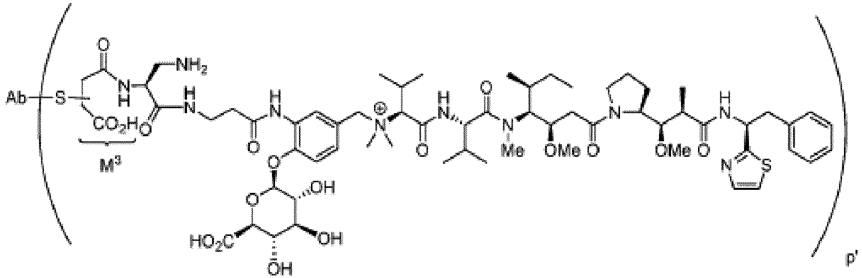 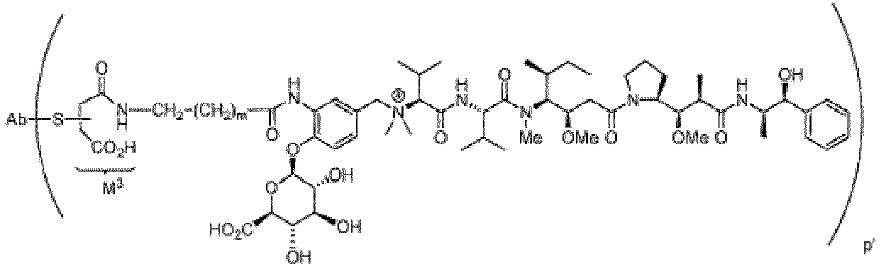 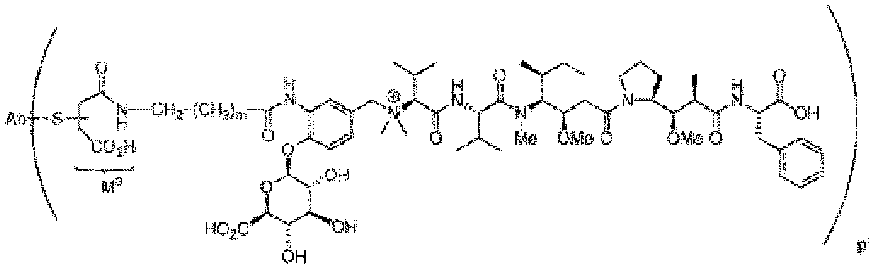 ou 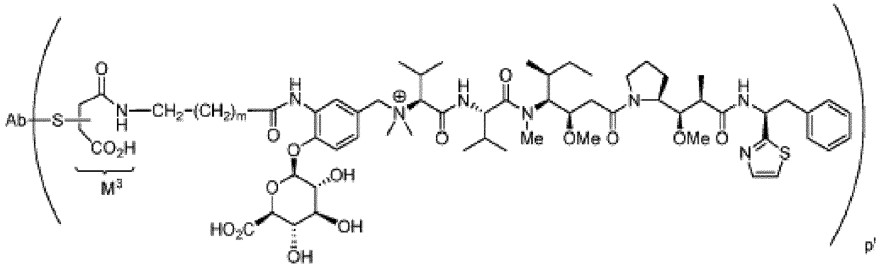
où Ab est une unité ligand anticorps ;
S est un atome de soufre de l'unité ligand anticorps ;
le fragment Ab-S- est lié à l'atome de carbone α ou β de l'acide carboxylique ; et
l'indice p' est un nombre entier se situant dans la plage de 1 à 8,

19. Composition pour utilisation, selon l'une quelconque des revendications 1-18, dans laquelle l'utilisation est le traitement d'un cancer.

20. Composition pour utilisation, selon la revendication 19, dans laquelle le cancer est une tumeur solide, un cancer hématologique, une leucémie aiguë, une leucémie chronique, ou un lymphome.

21. Composition pour utilisation, selon la revendication 20, dans laquelle le cancer est une tumeur solide choisie dans le groupe constitué par un fibrosarcome, myxosarcome, liposarcome, chondrosarcome, sarcome ostéogénique, chordome, angiosarcome, endothéliosarcome, lymphangiosarcome, lymphangio-endothéliosarcome, synoviome, mésothéliome, une tumeur d'Ewing, un leiomyosarcome, rhabdomyosarcome, cancer du côlon, cancer colorectal, cancer du rein, cancer du pancréas, cancer des os, cancer du sein, cancer de l'ovaire, cancer de la prostate, cancer de l'oesophage, cancer gastrique, cancer buccal, cancer nasal, cancer de la gorge, carcinome épidermoïde, carcinome basocellulaire, adénocarcinome, carcinome des glandes sudoripares, carcinome des glandes sébacées, carcinome papillaire, les adénocarcinomes papillaires, un cystadénocarcinome, carcinome médullaire, carcinome bronchogène, carcinome des cellules rénales, hépatome, carcinome des voies biliaires, choriocarcinome, seminome, carcinome embryonnaire, une tumeur de Wilms, un cancer du col utérin, cancer de l'utérus, cancer du testicule, cancer du poumon à petites cellules, cancer de la vessie, cancer du poumon, épithéliome, gliome, glioblastome multiforme, astrocytome, médulloblastome, craniopharyngiome, épendymome, pinéalome, hémangioblastome, neurinome de l'acoustique, oligodendrogliome, méningiome, cancer de la peau, mélanome, neuroblastome, et rétinoblastome.

22. Composition pour utilisation, selon la revendication 20, dans laquelle le cancer est un cancer hématologique choisi dans le groupe constitué par une leucémie aiguë lymphoblastique, leucémie aiguë lymphoblastique à lymphocytes B, leucémie aiguë lymphoblastique à lymphocytes T, leucémie aiguë myéloblastique, leucémie aiguë promyélocytaire, leucémie aiguë monoblastique, leucémie aiguë érythroblastique, leucémie aiguë à mégacaryoblastes, leucémie aiguë myélomonocytaire, leucémie aiguë non lymphocytaire, leucémie aiguë indifférenciée, leucémie myélocytaire chronique, leucémie lymphocytaire chronique, leucémie à cellules chevelues, et un myélome multiple.

23. Composition pour utilisation, selon la revendication 20, dans laquelle le cancer est un lymphome choisi dans le groupe constitué par la maladie de Hodgkin, un lymphome non hodgkinien, un myélome multiple, la macroglobulinémie de Waldenstrom, la maladie des chaînes lourdes, et la polycythémie vraie.

24. Composition pour utilisation, selon l'une quelconque des revendications 1-18, dans laquelle l'utilisation est un traitement d'une maladie auto-immune.
